(19) Europäisches Patentamt
European Patent Office
Office européen des brevets

(11) **EP 4 759 807 A1**

(12) **EUROPEAN PATENT APPLICATION**
published in accordance with Art. 153(4) EPC

(43) Date of publication:
**17.06.2026 Bulletin 2026/25**

(21) Application number: **24853694.8**

(22) Date of filing: **09.08.2024**

(51) International Patent Classification (IPC):
***C07D 403/14*** *(2006.01)* ***C07D 401/14*** *(2006.01)*
***C07D 487/04*** *(2006.01)* ***A61P 37/06*** *(2006.01)*
***A61P 35/00*** *(2006.01)* ***A61K 31/4192*** *(2006.01)*
***A61K 31/538*** *(2006.01)*

(52) Cooperative Patent Classification (CPC):
**A61K 31/4192; A61K 31/538; A61P 35/00; A61P 37/06; C07D 401/14; C07D 403/14; C07D 487/04**

(86) International application number:
**PCT/CN2024/111013**

(87) International publication number:
**WO 2025/036285 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**11.08.2023 CN 202311018650**
**16.08.2023 CN 202311038908**
**29.11.2023 CN 202311618951**
**26.01.2024 CN 202410120222**

(71) Applicant: **Shenzhen Zhongge Biological Technology Co., Ltd.**
**Futian District**
**Shenzhen, Guangdong 518017 (CN)**

(72) Inventors:
- **DU, Xinming**
  **Shenzhen, Guangdong 518017 (CN)**
- **MA, Junjun**
  **Shenzhen, Guangdong 518017 (CN)**
- **DU, Lifei**
  **Shenzhen, Guangdong 518017 (CN)**
- **CHEN, Zhaoqiang**
  **Shenzhen, Guangdong 518017 (CN)**
- **WANG, Shaohui**
  **Shenzhen, Guangdong 518017 (CN)**
- **CHEN, Bin**
  **Shenzhen, Guangdong 518017 (CN)**
- **ZHANG, Peiyu**
  **Shenzhen, Guangdong 518017 (CN)**

(74) Representative: **Snaith, James Michael**
**Kilburn & Strode LLP**
**Lacon London**
**84 Theobalds Road**
**London WC1X 8NL (GB)**

Remarks:
The complete document including Reference Table(s) and the Sequence Listing(s) can be downloaded from the EPO website

(54) **PROTEOLYSIS TARGETING CHIMERA COMPOUND FOR DEGRADING IRAK4 AND USE THEREOF**

(57) The present application relates to a proteolysis targeting chimera compound represented by formula (A) for degrading IRAK4 and a preparation method therefor, a pharmaceutical composition comprising said compound, and a use of the pharmaceutical composition for treating diseases, disorders or conditions associated with the kinase protein IRAK4.

EP 4 759 807 A1

AA

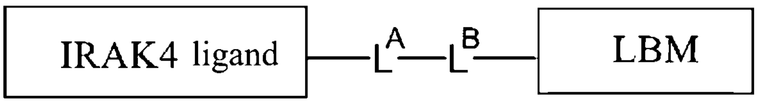

(A)

AA IRAK4 ligand

**Description**

Cross-Reference to Related Applications

The present application claims priority to Chinese patent application CN202311018650.4 filed on August 11, 2023, Chinese patent application CN202311038908.7 filed on August 16, 2023, Chinese patent application CN202311618951.0 filed on November 29, 2023, and Chinese patent application CN202410120222.0 filed on January 26, 2024, and their contents were incorporated into the present application by reference in their entirety and for all purposes.

Technical Field

The present application relates to a proteolysis targeting chimera (PROTAC) compound for degrading IRAK4 and a preparation method therefor, a pharmaceutical composition comprising said compound, and a use of the pharmaceutical composition for treating diseases, disorders or conditions associated with the kinase protein IRAK4.

Background Art

IRAK4 is a serine/threonine protein kinase and belongs to the interleukin-1 receptor-associated kinase family, which includes four isoforms: IRAK1, IRAK2, IRAK3 (or referred to as 'IRAKM') and IRAK4. IRAK1, IRAK2 and IRAK4 promote the release of inflammatory factors, while IRAK3 is involved in the antiinflammatory process. Among the four subtypes, the biological function of IRAK4 has been clearly elucidated. After TLR or IL-1R senses external signal stimulation, the Myddosome complex formed by IRAK4 activates the MAPK and NF-κB pathways, thereby leading to release of a variety of inflammatory factors.

Studies have confirmed that IRAK4 is highly expressed in a variety of tumor cells and inflammatory models, and the development of IRAK4-targeted inhibitor has become an important direction for the treatment of autoimmune diseases and tumors. IRAK4 has two functions, kinase activity and scaffold activity, and both functions play an important role in downstream signal regulation. Traditional small molecule IRAK4 kinase inhibitors cannot achieve the ideal therapeutic effect by only inhibiting the kinase activity of IRAK4, and there are subsequent problems such as target protein mutation-induced drug resistance.

The ubiquitin-proteasome pathway (UPP) is a key pathway for regulating key regulatory factor proteins and degrading misfolded or abnormal proteins. UPP plays a central role in multiple cellular processes, and if defective or unbalanced, contributes to the pathogenesis of a variety of diseases. The covalent linkage of ubiquitin to a specific protein substrate is achieved via the action of E3 ubiquitin ligases.

The proteolysis targeting chimera (PROTAC) technology is a new technology that has emerged in recent years. Since its introduction in 2001, this technology has attracted much attention. Currently, multiple drug developments based on this technology have entered the clinical research stage, and some of them have entered phase 2 clinical research. PROTAC is a heterogeneous bifunctional molecule, which consists of a small molecule inhibitor that can recognize the target protein at one end, a linker, and a ligand that can recognize the E3 ubiquitin ligase at the other end. Such bifunctional molecule recognizes the target protein in vivo and brings the target protein and the E3 ubiquitin ligase together to form a ternary complex, and then the target protein was ubiquitinated, thereby initiating a degradation pathway that depends on the ubiquitin-proteasome. Compared with traditional small molecule inhibitors, PROTAC technology achieves simultaneous inhibition of the two functions of IRAK4 by degrading the IRAK4 protein, which can effectively solve the problems of insufficient activity of small molecule inhibitors or target protein mutations.

There is a need to develop novel PROTAC compounds that degrade IRAK4 for treating diseases, disorders or conditions associated with the kinase protein IRAK4.

Summary of the Invention

The present disclosure provides a PROTAC compound targeting IRAK4, which is used for recruiting IRAK4 kinase to the E3 ubiquitin ligase for degradation. In other words, the bifunctional PROTAC compound of the present disclosure has utility as a modulator for targeted ubiquitination of IRAK4 kinase. The IRAK4 kinase is degraded and/or otherwise inhibited by the bifunctional compound as described herein. The inventors of the present application have shown that the compound of the present disclosure is able to effectively degrade IRAK4 kinase. The compound of the present disclosure can be used to treat diseases, disorders or conditions associated with kinase protein IRAK4. In addition, the compound of the present disclosure has better physicochemical properties (e.g., solubility, physical and/or chemical stability), improved pharmacokinetic properties (e.g., improved bioavailability, improved metabolic stability, suitable half-life and duration of action), improved safety (lower toxicity (e.g., reduced cardiotoxicity) and/or less side effects), less prone

to drug resistance and other superior properties.

In one aspect, the present disclosure provides a compound of formula (A) as defined below:

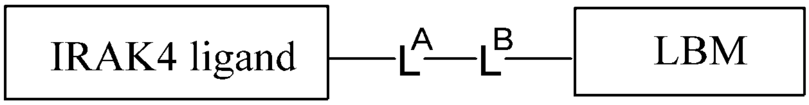

(A)

or a stereoisomer, a tautomer, a diastereomer, a racemate, a cis-trans isomer, an isotopically-labeled compound (preferably deuterated compound), an N-oxide, a metabolite, an ester, a prodrug, a crystal form, a hydrate, a solvate or a pharmaceutically acceptable salt thereof.

In another aspect, the present disclosure provides a method for targeted degradation of kinase protein IRAK4, comprising contacting the kinase protein IRAK4 with the compound of formula (A) of the present disclosure in the presence of an E3 ubiquitin ligase.

In another aspect, the present disclosure provides a pharmaceutical composition comprising the compound of formula (A) of the present disclosure or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, and a pharmaceutically acceptable excipient, a carrier or a diluent. The pharmaceutical composition is preferably a solid preparation, a liquid preparation or a transdermal preparation.

In another aspect, the present disclosure provides a use of the compound of formula (A) of the present disclosure or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, or the pharmaceutical composition of the present disclosure in the preparation of a medicament for treating a disease, disorder or condition associated with kinase protein IRAK4.

In another aspect, the present disclosure provides a method for treating a disease, disorder or condition associated with kinase protein IRAK4, comprising administering to an individual in need thereof a therapeutically effective amount of the compound of formula (A) of the present disclosure or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, or the pharmaceutical composition of the present disclosure.

In another aspect, the present disclosure provides a method for preparing the compound of formula (A) of the present disclosure.

Brief Description of the Drawings

Fig. 1 is the effect of positive reference and example 146 in test example 3 of the present application on the proteolysis level of IRAK4 in the spleen of Balb/c mice in vivo;

Fig. 2 is a data graph showing that positive reference and example 146 in test example 6 of the present application inhibited LPS-induced secretion of multiple cytokines by hPBMC.

Detailed Description of Embodiments

**<u>Definitions</u>**

Unless otherwise defined below, all technical and scientific terms used herein are intended to have the same meaning as commonly understood by those skilled in the art. References to technology used herein are intended to mean technology as commonly understood in the art, including those technological variations or replacement of equivalent technologies that would be apparent to those skilled in the art. Although the following terms are believed to be well understood by those skilled in the art, the following definitions are set forth to better explain the present disclosure.

The terms "include," "comprise," "have," "contain," or "involve," and other variations thereof herein, are inclusive or open-ended and do not exclude other unrecited elements or method steps (i.e., these terms also encompass the terms "consist essentially of" and "consist of").

As used herein, the term "hydrocarbyl" means a straight or branched saturated or unsaturated aliphatic hydrocarbyl. Hydrocarbyl includes alkyl, alkenyl and alkynyl. In some embodiments, the hydrocarbyl has 1 to 12, such

as 1 to 6 (e.g., 1, 2, 3, 4, 5 or 6) carbon atoms. For example, as used herein, the term "$C_{1-6}$ hydrocarbyl" refers to a straight or branched group having 1 to 6 carbon atoms, including "$C_{2-6}$ hydrocarbyl", "$C_{2-5}$ hydrocarbyl" and "$C_{1-4}$ hydrocarbyl". As used herein, the term "alkylene" refers to a group obtained by further losing 1 hydrogen atom from the "hydrocarbyl" as defined above.

As used herein, the term "alkane" means a straight or branched saturated aliphatic hydrocarbon.

As used herein, the term "alkyl" means a straight or branched monovalent saturated aliphatic hydrocarbyl, which can be regarded as a group obtained by losing 1 hydrogen atom from an alkane. In some embodiments, the alkyl has 1 to 12, such as 1 to 6 (e.g., 1, 2, 3, 4, 5 or 6) carbon atoms. For example, as used herein, the term "$C_{1-6}$ alkyl" refers to a straight or branched group having 1 to 6 carbon atoms, including "$C_{2-6}$ alkyl", "$C_{2-5}$ alkyl" and "$C_{1-4}$ alkyl". Examples of "$C_{1-6}$ alkyl" include methyl, ethyl, n-propyl, isopropyl, n-butyl, isobutyl, sec-butyl, tert-butyl, n-pentyl, and n-hexyl. The term "$C_{1-4}$ alkyl" refers to an alkyl having 1 to 4 carbon atoms (i.e., methyl, ethyl, n-propyl, isopropyl, n-butyl, isobutyl, sec-butyl, or tert-butyl).

As used herein, the term "alkylene" refers to a group obtained by further losing 1 hydrogen atom from the "alkyl" as defined above. In some embodiments, alkylene has 1 to 12 carbon atoms, preferably has 1, 2, 3, 4, 5, or 6 carbon atoms. For example, "$C_{1-6}$ alkylene", "$C_{2-6}$ alkylene", "$C_{2-5}$ alkylene", and "$C_{1-4}$ alkylene". Examples of the "$C_{1-6}$ alkylene" include methylene, ethylene, n-propylene, isopropylene, n-butylene, isobutylene, sec-butylene, tert-butylene, n-pentylene and n-hexylene. The term "$C_{1-4}$ alkylene" refers to an alkylene having 1 to 4 carbon atoms.

As used herein, the term "alkoxy" refers to -O-alkyl, wherein the alkyl is as defined above.

As used herein, the term "heteroalkyl" refers to an alkyl as defined above, wherein 1 or more, but not all, C atoms in the alkyl chain are replaced by a heteroatom or heteroatomic group selected from NR', O, C(O), S, S(O) and S(O)2, wherein R' is a suitable substituent, such as H, alkyl, etc. The heteroalkyl can be connected to the remainder of the molecule via a C atom or the heteroatom or heteroatomic group. Preferably, the heteroalkyl is connected to the remainder of the molecule via a C atom.

As used herein, the term "alkenyl" means a straight or branched monovalent aliphatic hydrocarbyl comprising one or more double bonds. In some embodiments, the alkenyl has 2, 3, 4, 5 or 6 carbon atoms ("$C_{2-6}$ alkenyl", e.g., "$C_{2-4}$ alkenyl"). The alkenyl is, for example, $-CH=CH_2$, $-CH_2CH=CH_2$, $-C(CH_3)=CH_2$, $-CH_2-CH=CH-CH_3$, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present disclosure contains an alkenyl, the compound can be present in the form of pure E (entgegen), the form of pure Z (zusammen) or the form of any mixture thereof. The term "alkenylene" is a corresponding divalent group, including, for example, "$C_{2-6}$ alkenylene", "$C_{2-4}$ alkenylene", etc., and specific examples thereof include, but are not limited to: $-CH=CH-$, $-CH_2CH=CH-$, $-C(CH_3)=CH-$, butenylene, pentenylene, hexenylene, cyclopentenylene, cyclohexenylene, etc.

As used herein, the term "alkynyl" means a straight or branched monovalent aliphatic hydrocarbyl comprising one or more triple bonds. In some embodiments, the alkynyl has 2, 3, 4, 5 or 6 carbon atoms ("$C_{2-6}$ alkynyl", e.g., "$C_{2-4}$ alkynyl"). The alkynyl is, for example, $-C\equiv CH$, $-CH_2C\equiv CH$, $-C\equiv C-CH_3$, $-CH_2-C\equiv C-CH_3$, 2-pentynyl, 3-pentynyl, 4-pentynyl, 2-hexynyl, 3-hexynyl, 4-hexynyl, 5-hexynyl, 3-methyl-2-butynyl and 2-methyl-3-pentynyl. The term "alkynylene" is a corresponding divalent group, including, for example, "$C_{2-6}$ alkynylene", "$C_{2-4}$ alkynylene", etc., and specific examples thereof include, but are not limited to: $- C\equiv C-$, $-CH_2C\equiv C-$, $-C\equiv C-CH_2-$, $-CH_2-C\equiv C-CH_2-$, pentynylene, hexynylene, etc.

As used herein, the term "fused" means that two or more cyclic structures share two adjacent atoms with each other.

As used herein, the term "bridge" or "bridged" means that two or more cyclic structures share two non-adjacent atoms with each other.

As used herein, the term "spiro" or "spiro linkage" means that two or more cyclic structures share 1 atom with each other.

As used herein, the terms "cyclohydrocarbyl", "hydrocarbon ring" and "cyclohydrocarbylene" refer to saturated (i.e., "cycloalkyl" and "cycloalkylene") or partially unsaturated (i.e., having one or more double bonds (i.e., "cycloalkenyl" and "cycloalkenylene") and/or triple bonds within the ring) monocyclic or polycyclic hydrocarbon rings having, for example, 3-12 (suitably having 3-10, 3-8, 3-7, 3-6, 4-6 or 5-6) ring carbon atoms, including, but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclobutenyl(ene) (ring), cyclopentenyl(ene) (ring), cyclohexenyl(ene) (ring), cycloheptenyl(ene) (ring), cyclooctenyl(ene) (ring), cyclononenyl(ene) (ring), etc. In some embodiments, cyclohydrocarbyl includes aryl-fused cyclohydrocarbyl, as long as the entire ring system is non-aromatic.

As used herein, the terms "cycloalkyl" and "cycloalkylene" refer to a saturated monocyclic or polycyclic (such as bicyclic) hydrocarbon ring (e.g., a monocyclic ring such as cyclopropyl, cyclobutyl, cyclopentyl, cyclohexyl, cycloheptyl, cyclooctyl, cyclononyl, or a bicyclic ring, including spiro, fused or bridged systems (such as bicyclo[1.1.1]pentyl, bicyclo[2.2.1]heptyl, bicyclo[3.2.1]octyl or bicyclo[5.2.0]nonyl, decalinyl, etc.). The cycloalkyl has 3-15 carbon atoms, suitably has 3-12, 3-10, 3-8, 3-7, 3-6, 4-6 or 5-6 carbon atoms. For example, the terms "$C_{3-6}$ cycloalkyl" and "$C_{3-6}$ cycloalkylene" refers to a saturated monocyclic or polycyclic (such as bicyclic) hydrocarbon ring having 3 to 6 ring-forming carbon atoms (for

example, cyclopropyl, cyclobutyl, cyclopentyl or cyclohexyl).

The terms "spiro cycloalkyl" and "spiro cycloalkylene" refer to polycyclic (such as bicyclic) "cycloalkyl" and "cycloalkylene" as defined above, wherein any two connected rings share one carbon atom. For example, "$C_{7-12}$ spiro cycloalkyl" and "$C_{7-12}$ spiro cycloalkylene" refer to a cyclic structure containing 7 to 12 (e.g., 5-12 or 7-11) carbon atoms and formed by at least two rings sharing one atom.

The terms "fused cycloalkyl" and "fused cycloalkylene" refer to polycyclic (such as bicyclic) "cycloalkyl" and "cycloalkylene" as defined above, wherein any two connected rings share two adjacent carbon atoms. For example, "$C_{4-10}$ fused cycloalkyl" and "$C_{4-10}$ fused cycloalkylene" refer to a fused ring containing 4 to 10 (e.g., 6-10 or 8-10) ring carbon atoms and formed by two or more rings sharing two adjacent carbon atoms.

The terms "bridged cycloalkyl" and "bridged cycloalkylene" refer to polycyclic (such as bicyclic) "cycloalkyl" and "cycloalkylene" as defined above, wherein any two connected rings share two carbon atoms that are not adjacent to each other. For example, "$C_{7-10}$ bridged cycloalkyl" and "$C_{7-10}$ bridged cycloalkylene" refer to a cyclic structure containing 7 to 12 (e.g., 6-10, 6-9, or 6-8) carbon atoms and formed by two rings that share two atoms that are not adjacent to each other.

As used herein, the terms "cycloalkenyl" and "cycloalkenylene" refer to a monocyclic or polycyclic (such as a bicyclic) fused hydrocarbon ring (e.g., a monocyclic ring such as cyclopropenyl, cyclobutenyl, cyclopentenyl, cyclopentadienyl, cyclohexenyl, cyclohexadienyl, cycloheptenyl, cyclooctenyl, cyclononenyl, or a bicyclic ring) having 1 or more double bonds within the ring. The cycloalkenyl and "cycloalkenylene" have 3 to 10 carbon atoms, suitably has 3-8, such as 3-7, 3-6, 4-6 or 5-6 carbon atoms.

As used herein, the terms "heterocyclyl", "heterocycle" and "heterocyclylene" refer to a saturated (i.e., "heterocycloalkyl" and "heterocycloalkylene") or partially unsaturated (e.g., having one or more double bonds within the ring (i.e., "heterocycloalkenyl" and "heterocycloalkenylene")) monocyclic or polycyclic (e.g., bicyclic) cyclic structure having 2, 3, 4, 5, 6, 7, 8 or 9 carbon atoms and 1 or more (e.g., 1, 2, 3 or 4) heteroatom-containing groups selected from O, S, S(=O), $S(=O)_2$ and NR' in the ring, wherein R' is as defined above. The heterocycle can be connected to the remainder of the molecule via any of the carbon atoms or the nitrogen atom (if present). In particular, a 3- to 12-membered heterocycle is a group having 3-12 (e.g., 3-10, 3-8, 3-7, 3-6, 4-11, 4-9, 4-7, 4-6, 5-12, 5-6, 6-10, 6-9, 6-8, 7-11, or 8-12) ring atoms , the ring atoms including carbon atom(s) and heteroatom(s). Examples that can be listed include, but are not limited to, oxiranyl, aziridinyl, azetidinyl, oxetanyl, tetrahydrofuranyl, tetrahydrothienyl, dioxolinyl, pyrrolidinyl, pyrrolidonyl, oxazolidine, thiazolidinyl, pyrazolidinyl, imidazolidinyl, pyrazolidinyl, pyrrolinyl, tetrahydropyranyl, piperidinyl, hexahydropyrimidinyl, triazinanyl, morpholinyl, dithianyl, thiomorpholinyl, piperazinyl, trithianyl, azacyclooctanyl, dihydropyrrolyl, dihydroimidazolyl, azacyclooctenyl.

As used herein, the heterocycle described above includes nitrogen-containing heterocycle, oxygen-containing heterocycle, and sulfur-containing heterocycle. For example, the "nitrogen-containing heterocycle" has at least one nitrogen atom, and may optionally have one or more (e.g., one, two, three or four) ring members selected from N, O, C=O, S, S=O and $S(=O)_2$. The nitrogen-containing heterocycle may be connected to the remainder of the molecule via the nitrogen atom. The nitrogen-containing heterocycle is preferably a saturated nitrogen-containing monocyclic ring. In particular, the 3- to 12-membered nitrogen-containing heterocycle is a group having 3-12 ring atoms, the ring atoms including carbon atom(s) and heteroatom(s) (at least one of which is a nitrogen atom), and includes but not limited to a three-membered nitrogen-containing heterocycle (such as aziridinyl), a four-membered nitrogen-containing heterocycle (such as azetidinyl), a five-membered nitrogen-containing heterocycle (such as pyrrolyl, pyrrolidinyl (pyrrolidine ring), pyrrolinyl, pyrrolidonyl, imidazolyl, imidazolidinyl, imidazolinyl, pyrazolyl, pyrazolinyl), a six-membered nitrogen-containing heterocycle (such as piperidinyl (piperidine ring),morpholinyl, thiomorpholinyl, piperazinyl), a seven-membered nitrogen-containing heterocycle, etc.

As used herein, the heterocycle described above includes monocyclic ring, fused ring, bridged ring and spiro ring, i.e., monocyclic heterocycle, bridged heterocycle, spiro heterocycle and fused heterocycle. The point of connection of bridged heterocycle, spiro heterocycle and fused heterocycle to other group may be on any ring in the structure.

As used herein, fused heterocycle refers to polycyclic (such as bicyclic) heterocycle as defined above, wherein any two connected rings share two adjacent atoms. Fused heterocycle includes, but not limited to, heterocyclyl fused heterocyclyl, heterocyclyl fused cycloalkyl, monoheterocyclyl fused monoheterocyclyl, monoheterocyclyl fused monocycloalkyl, such as 3- to 7-membered (mono) heterocyclyl fused 3- to 7-membered (mono) heterocyclyl, 3- to 7-membered (mono) heterocyclyl fused (mono) cycloalkyl, 3- to 7-membered (mono) heterocyclyl fused $C_{4-6}$ (mono) cycloalkyl. Preferably, the fused heterocycle is 6- to 10-membered, and more preferably 8- to 10-membered. Examples of fused heterocycle includes, but not limited to, pyrrolidinyl fused cyclopropyl, cyclopentyl fused aziridinyl, pyrrolidinyl fused cyclobutyl, pyrrolidinyl fused pyrrolidinyl, pyrrolidinyl fused piperidinyl, pyrrolidinyl fused piperazinyl, piperidinyl fused morpholinyl,

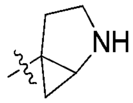, 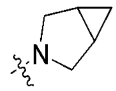, 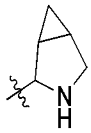, 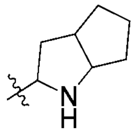, 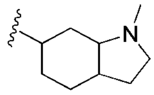, 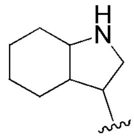,

or

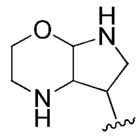.

In some embodiments, the fused heterocyclyl also includes heteroaryl fused heterocyclyl or heteroaryl fused cyclohydrocarbyl, and aryl fused heterocyclyl, as long as the entire ring system is non-aromatic. In some embodiments, the fused heterocyclyl includes 5- to 6-membered monocyclic heteroaryl fused $C_{5-6}$ monocyclic cyclohydrocarbyl, 5- to 6-membered monocyclic heteroaryl fused 5- to 6-membered monocyclic heterocyclyl, and phenyl fused 5- to 6-membered monocyclic heterocyclyl, such as pyrrolotetrahydropyridyl, pyrazolotetrahydropyridyl, and imidazotetrahydropyridyl.

As used herein, spiro heterocycle refers to polycyclic (such as bicyclic) heterocycle as defined above, wherein any two connected rings share one carbon atom. Preferably, the spiro heterocycle is 5- to 12-membered, and more preferably 7- to 11-membered. According to the number of shared spiro atoms, the spiro heterocycle is divided into monospiro heterocycle, dispiro heterocycle, or polyspiro heterocycle, and preferably refer to monospiro heterocycle or dispiro heterocycle, and more preferably 4-membered/4-membered, 3-membered/5-membered, 4-membered/5-membered, 4-membered/6-membered, 5-membered/5-membered, or 5-membered/6-membered monospiro heterocycle.

As used herein, bridged heterocycle or bridge heterocycle refers to polycyclic (such as bicyclic) heterocycle as defined above, wherein any two connected rings share two non-adjacent atoms. One or more rings of the bridge heterocycle may contain one or more double bonds, but none of the rings have a completely conjugated $\pi$ electron system. Preferably, the bridge heterocycle is 6- to 9-membered, and more preferably 6- to 8-membered. According to the number of member rings, the bridge heterocycle is divided into bicyclic, tricyclic, tetracyclic or polycyclic bridge heterocycle, and preferably refer to bicyclic, tricyclic or tetracyclic bridge heterocycle, and more preferably bicyclic or tricyclic bridge heterocycle.

As used herein, the term "aryl" refers to an all-carbon monocyclic or fused polycyclic aromatic group having a conjugated $\pi$ electron system. For example, as used herein, the term "$C_{6-10}$ aryl" means an aromatic group containing 6 to 10 carbon atoms, such as phenyl or naphthyl.

As used herein, the term "heteroaryl" refers to a monocyclic or polycyclic (e.g. bicyclic or tricyclic) aromatic ring system having 5 to 14 ring atoms, e.g. 5, 6, 7, 8, 9, 10, 11, 12, 13 or 14 ring atoms, in particular having 1, 2, 3, 4, 5, 6, 7, 8, 9, 10, 11, 12 or 13 carbon atoms and 1, 2, 3, 4 or 5 same or different heteroatoms independently selected from N, O, S and $S(O)_2$. 1 or more ring carbon atoms in the heteroaryl may be replaced by C(O). The heteroaryl may be benzo-fused. Examples of heteroaryl include, but are not limited to: pyridyl, pyridonyl, pyrimidinyl, pyrimidonyl, pyrazinyl, pyridazinyl, thiazolyl, thienyl, oxazolyl, furanyl, pyrrolyl, pyrazolyl, triazolyl, tetrazolyl, isoxazolyl, isothiazolyl, imidazolyl, triazinyl, oxadiazolyl, thiadiazolyl, benzothiazolyl, benzisothiazolyl, imidazopyridyl, quinolyl, indolyl, pyrrolopyridazinyl, benzofuranyl, benzothienyl, indazolyl, benzoxazolyl, benzisoxazolyl, quinazolinyl, pyrrolopyridyl, pyrazolopyrimidinyl, imidazopyridazinyl, pyrazolopyridyl, triazolopyridyl, isoquinolinyl, tetrahydroisoquinolinyl, benzimidazolyl, cinnolinyl, indolizinyl, phthalazinyl, isoindolyl, pteridinyl, purinyl, furazanyl, benzofurazanyl, quinoxalinyl, naphthyridinyl or furopyridyl.

As used herein, the term "halo" or "halogen" group is defined to include F, Cl, Br or I.

As used herein, the term "haloalkyl" refers to alkyl substituted with one or more (such as 1 to 3) the same or different halogen atoms, the alkyl is as defined herein. The terms "$C_{1-8}$ haloalkyl", "$C_{1-6}$ haloalkyl" and "$C_{1-4}$ haloalkyl" refer to haloalkyl having 1 to 8 carbon atoms, 1 to 6 carbon atoms and 1 to 4 carbon atoms, respectively, such as $-CF_3$, $-C_2F_5$, $-CHF_2$, $-CH_2F$, $-CH_2CF_3$, $-CH_2Cl$, $-CH_2CH_2CF_3$, etc.

As used herein, the term "haloalkenyl" refers to alkenyl substituted with one or more (such as 1 to 3) the same or different halogen atoms, the alkenyl is as defined herein. The terms "$C_{2-8}$ haloalkenyl", "$C_{2-6}$ haloalkenyl" and "$C_{2-4}$ haloalkenyl" refer to haloalkenyl having 2 to 8 carbon atoms, 2 to 6 carbon atoms and 2 to 4 carbon atoms, respectively.

The term "substituted" refers to replacement of one or more (e.g., one, two, three or four) hydrogens on the designated atom with a group selected from the indicated groups, provided that the normal valence of the designated atom in the present context is not exceeded and the substitution results in a stable compound. Combinations of substituents and/or variables are permissible only if such combinations result in stable compounds.

If a group is described as "optionally substituted with" or "optionally substituted", then the group can be: (1)

unsubstituted or (2) substituted. If a carbon of a group is described as being optionally substituted with one or more of the listed substituents, then one or more hydrogens on that carbon (to the extent of any hydrogen(s) present) may be replaced individually and/or collectively with independently selected optional substituents. If a nitrogen of a group is described as being optionally substituted with one or more of the listed substituents, then one or more hydrogens on that nitrogen (to the extent of any hydrogen(s) present) may each be replaced with independently selected optional substituents.

If substituents are described as being "independently selected from" a set, then each substituent is selected independently of the other. Thus, each substituent may be the same as or different from another (other) substituent(s).

As used herein, the term "one or more" means 1 or more than 1, such as 2, 3, 4, 5 or 10, where reasonable.

Unless otherwise specified, as used herein, the point of connection of a substituent may be at any suitable position of the substituent.

When a bond of a substituent is shown as a bond passing through a ring and connecting two atoms (a "floating bond"), such a substituent may be bonded to any ring-forming atom in the substitutable ring, unless otherwise specified. In cases where an available ring member is shown to carry a substitutable hydrogen atom, the substitutable hydrogen atom is substantially substituted (i.e., not present) when the floating bond is attached to the available ring member.

The present disclosure further includes all pharmaceutically acceptable isotopically labeled compounds which are identical to the compound of the present disclosure except that one or more atoms are replaced with an atom having the same atomic number but an atomic mass or mass number different from the atomic mass or mass number prevalent in nature. Examples of isotopes suitable for inclusion in the compound of the present disclosure include, but are not limited to, isotopes of hydrogen (e.g., deuterium (D,$^{2}H$), tritium (T,$^{3}H$)); isotopes of carbon (e.g., $^{11}C$, $^{13}C$, and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g. $^{15}O$, $^{17}O$ and $^{18}O$); isotopes of phosphorus (e.g. $^{32}P$); and isotopes of sulfur (e.g., $^{35}S$). Certain isotopically labeled compounds of the present disclosure (e.g., those incorporating radioactive isotopes) are useful in drug and/or substrate tissue distribution studies (e.g., assays). The radioactive isotopes tritium (i.e., $^{3}H$) and carbon-14 (i.e., $^{14}C$) are particularly useful for this purpose because of their ease of incorporation and ease of detection. Substitution with positron emitting isotopes (e.g., $^{11}C$, $^{18}F$, $^{15}O$, and $^{13}N$) may be used to examine substrate receptor occupancy in positron emission tomography (PET) studies. Isotopically-labeled compounds of the present disclosure can be prepared by procedures analogous to those described in the accompanying schemes and/or in the examples and preparations using an appropriate isotopically-labeled reagent in place of the non-labeled reagent previously employed. Pharmaceutically acceptable solvates of the present disclosure include those in which the crystallization solvent may be isotopically substituted, for example, $D_2O$, acetone-$d_6$ or DMSO-$d_6$. In some embodiments, the isotopically labeled compounds of the present disclosure are deuterated compounds.

The term "stereoisomer" refers to an isomer which is formed due to at least one asymmetric center and has the same chemical composition but differs in the spatial arrangement of atoms or groups. In compounds having one or more (e.g., 1, 2, 3 or 4) asymmetric centers, racemic mixtures, single enantiomers, diastereomeric mixtures and individual diastereomers may be produced. Specific individual molecules may also exist as geometric isomers (cis/trans). Similarly, the compounds of the present disclosure may exist as mixtures of two or more structurally different forms in rapid equilibrium (commonly referred to as tautomers). Representative examples of tautomers include keto-enol tautomers, phenol-keto tautomers, nitroso-oxime tautomers, imine-enamine tautomers, and the like. It is to be understood that the scope of the present application encompasses all such isomers in any proportion (e.g., 60%, 65%, 70%, 75%, 80%, 85%, 90%, 95%, 96%, 97%, 98%, 99%) or mixtures thereof.

"Diastereomers" refer to stereoisomers having two or more chiral centers and whose molecules are not mirror images of each other. Diastereomers have different physical properties, such as melting points, boiling points, spectral properties and reactivity. Mixtures of diastereomers can be separated by high-resolution analytical methods such as electrophoresis and chromatography.

"Enantiomers" refer to two stereoisomers of a compound that are non-superimposable mirror images of each other.

The term "chiral" refers to molecules having the property of non-superimposability of mirror image pairs, while the term "achiral" refers to molecules that are superimposable on their mirror image pairs.

The compounds of the present disclosure can be prepared in racemic form, or single enantiomer can be prepared by enantioselective synthesis or by resolution.

As used herein, the term "cis/trans isomer" or "geometric isomer" is caused by the fact that double bonds or single bonds of ring-forming carbon atoms cannot rotate freely. The compounds provided herein include all cis, trans, syn, anti, entgegen (E) and zusammen (Z) isomers and their corresponding mixtures.

The solid line (-), solid wedge (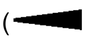) or dashed wedge () can be used herein to depict the chemical bonds of the compounds of the present disclosure. The use of solid lines to depict the bonds connected to asymmetric carbon atoms is intended to indicate that all possible stereoisomers at the carbon atoms (such as specific enantiomers and racemic mixtures) are included. The use of solid or dashed wedges to depict the bonds connected to asymmetric carbon atoms is intended to indicate the presence of the stereoisomers shown. When present in a racemic mixture, solid and

dashed wedges are used to define the relative stereochemistry rather than the absolute stereochemistry. Unless otherwise indicated, the compounds of the present disclosure are intended to exist in forms of stereoisomers, including cis and trans isomers, optical isomers (e.g., R- and S-enantiomers), diastereomers, geometric isomers, rotamers, conformers, atropisomers, and mixtures thereof. The compounds of the present disclosure may exhibit more than one type of isomerism, and consist of mixtures thereof (such as racemic mixtures and diastereomeric pairs).

It should also be understood that certain compounds of the present disclosure may exist in free form for treatment, or, where appropriate, in the form of pharmaceutically acceptable derivatives thereof. In the present disclosure, pharmaceutically acceptable derivatives include, but are not limited to, pharmaceutically acceptable salts, esters, solvates, metabolites or prodrugs, which, after administration to a patient in need thereof, can directly or indirectly provide the compounds of the present disclosure or their metabolites or residues. Therefore, when referring to "compounds of the present disclosure" herein, the above-mentioned various derivative forms of the compounds are also intended to be encompassed.

The term "pharmaceutically acceptable" means that the substance or composition must be chemically and/or toxicologically compatible with the other ingredients making up the preparation and/or the mammal to be treated therewith.

Pharmaceutically acceptable salts of the compounds of the present disclosure include acid addition salts and base addition salts thereof.

Suitable acid addition salts are formed from acids that form pharmaceutically acceptable salts. Examples include aspartate, benzoate, bicarbonate/carbonate, bisulfate/sulfate, fumarate, glucoheptonate, gluconate, glucuronate, hexafluorophosphate, hydrobromide/bromide, hydroiodide/iodide, maleate, malonate, methylsulfate, naphthylate, nicotinate, nitrate, orotate, oxalate, palmitate and other similar salts.

Suitable base addition salts are formed from bases that form pharmaceutically acceptable salts. Examples include aluminum salt, arginine salt, choline salt, diethylamine salt, lysine salt, magnesium salt, meglumine salt, potassium salt and other similar salts.

For a review of suitable salts, see Stahl and Wermuth, "Handbook of Pharmaceutical Salts: Properties, Selection, and Use" (Wiley-VCH, 2002). Methods for preparing pharmaceutically acceptable salts of the compounds of the present disclosure are known to those skilled in the art.

As used herein, the term "ester" means an ester derived from the compounds of the respective general formulae in the present application, including physiologically hydrolyzable esters (which can be hydrolyzed under physiological conditions to release the compounds of the present disclosure in the form of free acid or alcohol). The compounds of the present disclosure may themselves be esters.

The present disclosure encompasses all possible crystalline forms or polymorphs of the compounds of the present disclosure, which may be a single polymorph or a mixture of more than one polymorph in any ratio.

The compound of the present disclosure may exist in the form of solvate, preferably hydrate, in which the compound of the present disclosure comprises a polar solvent, such as in particular water, methanol or ethanol, as a structural component of the crystal lattice of the compound. The amount of the polar solvent, in particular water, may be present in a stoichiometric or non-stoichiometric ratio.

Those skilled in the art will appreciate that not all nitrogen-containing heterocycles are capable of forming N-oxides, as nitrogen requires an available lone pair of electrons to be oxidized to an oxide; Those skilled in the art will recognize nitrogen-containing heterocycles that are capable of forming N-oxides. Those skilled in the art will also recognize that tertiary amines are capable of forming N-oxides. Synthetic methods for preparing N-oxides of heterocycles and tertiary amines are well known to those skilled in the art and include oxidation of heterocycles and tertiary amines with peroxyacids such as peracetic acid and m-chloroperbenzoic acid (MCPBA), hydrogen peroxide, alkyl hydroperoxides such as tert-butyl hydroperoxide, sodium perborate, and dioxirane such as dimethyldioxirane. These methods for preparing N-oxides have been extensively described and reviewed in the literature, see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

Also included within the scope of the present disclosure are metabolites of the compounds of the present disclosure, i.e., substances formed in vivo upon administration of the compounds of the present disclosure. Such products may be produced, for example, by oxidation, reduction, hydrolysis, amidation, deamidation, esterification, defatting, enzymatic hydrolysis, etc. of the administered compound. Thus, the present disclosure includes metabolites of the compounds of the present disclosure, including compounds produced by a process of contacting the compounds of the present disclosure with a mammal for a period of time sufficient to produce metabolic products thereof.

The present disclosure further includes within its scope prodrugs of the compounds of the present disclosure, which are certain derivatives of the compounds of the present disclosure that may themselves have little or no pharmacological activity but are converted, for example by hydrolytic cleavage, into compounds of the present disclosure having the desired activity when administered into or onto the body. Typically such prodrugs will be functional group derivatives of the compounds that are readily converted into the desired therapeutically active compound in vivo.

Additional information on the use of prodrugs can be found in "Pro-drugs as Novel Delivery Systems," Vol. 14, ACS Symposium Series (T. Higuchi and V. Stella) and "Bioreversible Carriers in Drug Design," Pergamon Press, 1987 (E. B. Roche, ed., American Pharmaceutical Association). Prodrugs of the present disclosure can be prepared, for example, by replacing appropriate functional groups present in the compounds of the present disclosure with certain moieties known to those skilled in the art as "pro-moieties" (e.g., as described in "Design of Prodrugs," H. Bundgaard (Elsevier, 1985)).

The present disclosure also encompasses the compounds of the present disclosure containing protecting groups. During any of the processes for preparation of the compounds of the present disclosure, it may be necessary and/or desirable to protect sensitive or reactive groups on any of the molecules concerned, thereby forming chemically protected forms of the compounds of the present disclosure. This can be achieved by conventional protecting groups, for example, those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which references are incorporated herein by reference. The protecting groups may be removed at an appropriate subsequent stage using methods known in the art.

As used herein, the term "about" means within ±10%, preferably within ±5%, and more preferably within ±2% of the stated value.

**Compound**

In one aspect, the present disclosure provides a compound of formula (A):

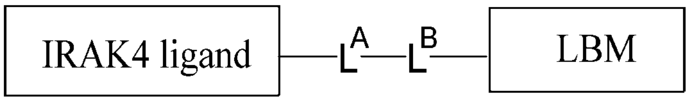


(A)

or a stereoisomer, a tautomer, a diastereomer, a racemate, a cis-trans isomer, an isotopically-labeled compound (preferably deuterated compound), an N-oxide, a metabolite, an ester, a prodrug, a crystal form, a hydrate, a solvate or a pharmaceutically acceptable salt thereof,

wherein:

the

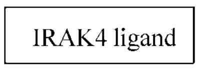


moiety is an IRAK4 ligand capable of binding to IRAK4;

the

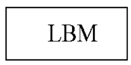


moiety is a ligase binding moiety,

$-L^A-L^B-$ is a divalent moiety that connects the

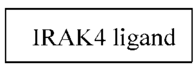


moiety to the

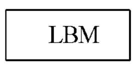


moiety.

In some embodiments, the present disclosure provides a compound of formula (A), wherein:

$L^A$ is selected from a bond and a straight or branched $C_{1-4}$ alkylene, and the $C_{1-4}$ alkylene is optionally substituted with one or more substituents independently selected from $C_{1-4}$ alkyl, $C_{1-4}$ haloalkyl, halogen, oxo (=O), OH, CN, $NH_2$, $-NH(C_{1-4}$ alkyl) and $-N(C_{1-4}$ alkyl$)_2$;
$L^B$ is a group selected from the following (1) to (21):

(1) -CyL1-,
(2) -CyL1-La-,
(3) -CyL1-Lb-,
(4) -CyL1-La-CyL2-La-,
(5) -CyL1-$NR^{L1}$-,
(6) -CyL1-C(O)-,
(7) -CyL1-C(O)-$NR^{L1}$-
(8) -CyL1-$NR^{L1}$-C(O)-,
(9) -CyL1-CyL2-,
(10) -$NR^{L1}$-CyL1-La-,
(11) -$NR^{L1}$-CyL1-Lb-,
(12) -$NR^{L1}$-CyL3-$NR^{L2}$-,
(13) -$NR^{L1}$-CyL3-La-$NR^{L2}$-
(14) -$NR^{L1}$-CyL1-C(O)-,
(15) -$NR^{L1}$-La-CyL1-La-,
(16) -La-CyL1-,
(17) -O-La-,
(18) -S-La-,
(19) -$NR^{L1}$-La-,
(20) -CyL1-La-CyL3-, and
(21) -CyL1-Lc-CyL4-;

wherein:

in the (1) to (21), the leftmost extending bond of each group is connected to the $L^A$ and the rightmost extending bond of each group is connected to the

LBM

moiety, or the leftmost extending bond of each group is connected to the

LBM

moiety and the rightmost extending bond of each group is connected to the $L^A$,
CyL1 and CyL2, at each occurrence, are each independently selected from 3-to 12-membered heterocycloalkylene, wherein the heterocycloalkylene preferably has 1, 2 or more nitrogen heteroatoms and 0, 1 or 2 heteroatoms selected from O and S,
CyL3, at each occurrence, is independently selected from $C_{3-12}$ cycloalkylene or 3- to 12-membered heterocycloalkylene,
CyL4, at each occurrence, is independently selected from 5- to 12-membered heteroarylene,
La, at each occurrence, is independently selected from $C_{1-4}$ hydrocarbylene,
Lb, at each occurrence, is independently selected from straight $C_{2-4}$ hydrocarbylene, wherein 1 or 2 but not all $CH_2$ in the straight $C_{2-4}$ hydrocarbylene are replaced with 1 or 2 groups selected from O, S, $NR^{L1}$ and C(O),
Lc, at each occurrence, is independently selected from a bond or $C_{1-4}$ hydrocarbylene,
CyL1, CyL2, CyL3, CyL4, La, Lb and Lc are each optionally substituted with 1 or more groups independently selected from: $C_{1-4}$ alkyl, $C_{1-4}$ haloalkyl, halogen, OH, CN, $NH_2$, $-NH(C_{1-4}$ alkyl) and $-N(C_{1-4}$ alkyl$)_2$, preferably methyl, ethyl, F, Cl, Br, OH, CN and $NH_2$, more preferably methyl, F, Cl and OH; and
$R^{L1}$ and $R^{L2}$, at each occurrence, are each independently selected from H and $C_{1-4}$ alkyl;

the

IRAK4 ligand

moiety has:

(I) a structure of formula (1):

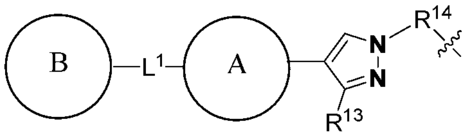

(1)

wherein:

ring A

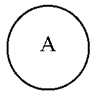

is selected from 5- to 6-membered heteroaryl, and the 5- to 6-membered heteroaryl is optionally substituted with a substituent $R^k$;

$R^k$ is selected from $C_{1-6}$ alkyl, $C_{3-7}$ cycloalkyl, $R^pR^qN$-, $C_{1-6}$ haloalkyl, $C_{1-6}$ heteroalkyl (e.g., $C_{1-6}$ alkoxy), 4- to 9-membered heterocyclyl (e.g., 5- to 6-membered saturated heterocycloalkyl), $C_{6-10}$ aryl, and 5- to 10-membered heteroaryl;

$R^p$ and $R^q$ are each independently selected from hydrogen, $C_{1-6}$ alkyl, $C_{1-6}$ alkoxy, and $C_{1-6}$ haloalkyl;

ring B

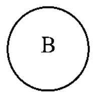

is selected from the following (1) to (3):

(1) 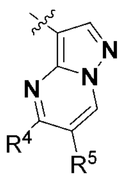 ,

wherein:

$R^4$ is selected from hydrogen, $C_{1-6}$ alkyl, $C_{4-9}$ cycloalkyl, $C_{4-9}$ cycloalkyl-$C_{1-6}$ alkyl-, 4- to 9-membered saturated heterocycloalkyl, 4- to 9-membered saturated heterocycloalkyl-$C_{1-6}$ alkyl-, and $-NR_{Na}R_{Nb}$ (wherein the $R_{Na}$ and $R_{Nb}$ are each independently selected from H, $C_{1-6}$ alkyl, and $C_{3-6}$ cycloalkyl, and the $C_{1-6}$ alkyl and the $C_{3-6}$ cycloalkyl may be optionally substituted with 1-3 substituents selected from halogen, hydroxy, $C_{3-6}$ cycloalkyl, $C_{3-6}$ halocycloalkyl, and 4- to 7-membered heterocyclyl), and the $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl- are optionally substituted with 1-3 substituents selected from $C_{1-6}$ alkyl, $C_{1-6}$ alkoxy, $C_{1-6}$ haloalkyl, $C_{1-6}$ haloalkoxy, halogen, hydroxy, hydroxy-$C_{1-6}$ alkylene-, cyano, oxo, $-NH_2$, $-NH(C_{1-6}$ alkyl), and $-N(C_{1-6}$ alkyl)($C_{1-6}$ alkyl);

$R^5$ is selected from hydrogen, cyano, $C_{1-6}$ alkyl, $-C(O)NH_2$, and $-NR^lR^m$;

$R^1$ and $R^m$ are each independently selected from hydrogen, $C_{1-6}$ alkyl, and $C_{3-6}$ cycloalkyl;

(2) 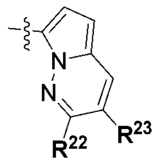 ,

wherein:

$R^{22}$ is selected from hydrogen, $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl-;
$R^{23}$ is selected from hydrogen, $C_{1-6}$ alkyl, cyano, carboxyl, -C(O)$NH_2$, -$NR^lR^m$,

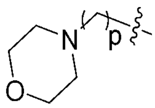 , 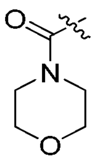 , 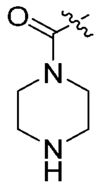 , 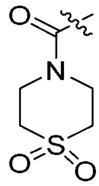 , 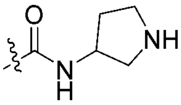 , and 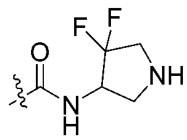 ;

and $R^l$ and $R^m$ are each independently selected from hydrogen, $C_{1-6}$ alkyl, and $C_{3-6}$ cycloalkyl;

(3) 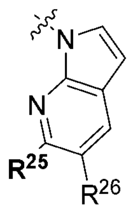 ,

wherein:

$R^{25}$ is selected from hydrogen, $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl-; $R^{26}$ is selected from hydrogen, $C_{1-6}$ alkyl, cyano, carboxyl, and -C(O)$NH_2$;
$L^1$ is selected from a direct bond, $C_{1-6}$ alkyl, -NH-, -O-, and -S-;
$R^{13}$ is selected from $C_{1-6}$ alkyl and $C_{1-6}$ haloalkyl;
$R^{14}$ is selected from $C_{3-6}$ cycloalkylene, 5- to 12-membered spiro heterocycloalkylene and piperidylidene, and the $C_{3-6}$ cycloalkylene, 5- to 12-membered spiro heterocycloalkylene and piperidylidene are optionally substituted with 1-2 substituents selected from hydroxy$C_{1-6}$ alkyl (e.g., hydroxymethyl), formyl, and $C_{1-6}$ alkyl;

or

(II) a structure of formula (2)

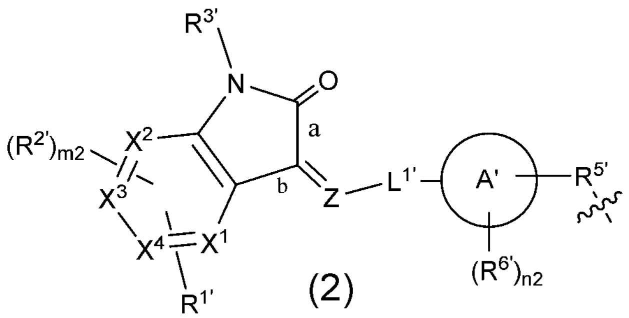

(2)

wherein:

ring A'

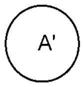

is selected from 5- to 10-membered heteroaryl;
$X^1$, $X^2$, $X^3$ and $X^4$ are each independently N or CH; and at least one of $X^1$, $X^2$, $X^3$ and $X^4$ is not N;
Z is $CR^{4'}$;
each of the letters "a" and "b" indicates the bond between the ring carbon atom connected to Z and adjacent two ring carbon atoms;

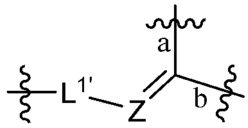

moiety is represented by a structure of the following formula (i) or formula (ii):

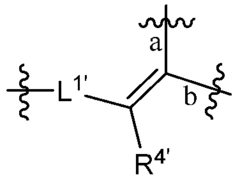

(i) or

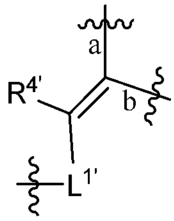

(ii) ;

$L^{1'}$ is selected from a direct bond and $NR^{7'}$; $R^{1'}$ is $-L^{2'}-R^{1a}$;
$L^{2'}$ is $-S(O)_2NR^{1b}-*$, $-C(O)-NR^{1b}-*$, $-NR^{1b}-C(O)-*$ or $-NR^{1b}-S(O)_2-*$, wherein the bond indicated with * is connected to $R^{1a}$;
$R^{1a}$ is selected from $C_{6-10}$ aryl and 5- to 10-membered heteroaryl, wherein the $C_{6-10}$ aryl and the 5- to 10-membered heteroaryl are each optionally substituted with 1 or more substituents independently selected from the following groups: halogen, OH, SH, $-NR^{1e}R^{1f}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{1e}R^{1f}$;
each $R^{2'}$ is independently selected from: H, halogen, OH, SH, $-NR^{2a}R^{2b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, - $C_{1-6}$ alkylene-OH, $-C_{1-6}$ alkylene-SH, $-C_{1-6}$ alkylene-CN, $-C_{1-6}$ alkylene-$NR^{2a}R^{2b}$;
m2 is 0, 1, 2 or 3;
$R^{3'}$ and $R^{7'}$ are each independently selected from: H, $C_{1-6}$ alkyl, $C_{1-6}$ haloalkyl, $C_{2-6}$ alkenyl, $C_{2-6}$ haloalkenyl, $-C_{1-6}$ alkylene-$O-C_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{3a}R^{3b}$;
$R^{4'}$ is selected from: H, D, halogen, OH, SH, $-NR^{4a}R^{4b}$, CN, $C_{1-6}$ alkyl, $C_{1-6}$ haloalkyl, $C_{2-6}$ alkenyl, $C_{2-6}$ haloalkenyl, $-C_{1-6}$ alkylene-$O-C_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{4a}R^{4b}$;
$R^{5'}$ is selected from $C_{3-10}$ cyclohydrocarbylene and 3- to 10-membered heterocyclylene, wherein the $C_{3-10}$ cyclohydrocarbylene and the 3- to 10-membered heterocyclylene are each optionally substituted with 1 or more substituents independently selected from the following groups: halogen, OH, SH, $-NR^{5a}R^{5b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, - $C_{1-6}$ alkylene-OH, $-C_{1-6}$ alkylene-SH, $-C_{1-6}$ alkylene-CN and $-C_{1-6}$ alkylene-$NR^{5a}R^{5b}$.
$R^{6'}$ is selected from: H, halogen, OH, SH, $-NR^{6a}R^{6b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-

$OC_{1-6}$ alkyl, $-C_{1-6}$ alkylene-OH, - $C_{1-6}$ alkylene-SH, $-C_{1-6}$ alkylene-CN and $-C_{1-6}$ alkylene-$NR^{6a}R^{6b}$;
n2 is 0, 1, 2, 3 or 4; and
$R^{1b}$, $R^{1e}$, $R^{1f}$, $R^{2a}$, $R^{2b}$, $R^{3a}$, $R^{3b}$, $R^{4a}$, $R^{4b}$, $R^{5a}$, $R^{5b}$, $R^{6a}$, and $R^{6b}$, at each occurrence, are independently selected from H and $C_{1-6}$ alkyl; and
the

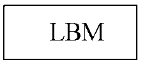

moiety is a ligase binding moiety.

In some embodiments, a compound of formula (A), or a stereoisomer, a tautomer, a diastereomer, a racemate, a cis-trans isomer, an isotopically-labeled compound (preferably deuterated compound), an N-oxide, a metabolite, an ester, a prodrug, a crystal form, a hydrate, a solvate or a pharmaceutically acceptable salt thereof,
wherein:
$L^A$ is selected from a bond and a straight or branched $C_{1-4}$ alkylene, and the $C_{1-4}$ alkylene is optionally substituted with one or more substituents independently selected from $C_{1-4}$ alkyl, $C_{1-4}$ haloalkyl, halogen, oxo (=O), OH, CN, $NH_2$, $-NH(C_{1-4}$ alkyl) and $-N(C_{1-4}$ alkyl$)_2$;
$L^B$ is a group selected from the following (1) to (19):

(1) -CyL1-,
(2) -CyL1-La-,
(3) -CyL1-Lb-,
(4) -CyL1-La-CyL2-La-,
(5) -CyL1-$NR^{L1}$-,
(6) -CyL1-C(O)-,
(7) -CyL1-C(O)-$NR^{L1}$-,
(8) -CyL1-$NR^{L1}$-C(O)-,
(9) -CyL1-CyL2-,
(10) -$NR^{L1}$-CyL1-La-,
(11) -$NR^{L1}$-CyL1-Lb-,
(12) -$NR^{L1}$-CyL3-$NR^{L2}$-,
(13) -$NR^{L1}$-CyL3-La-$NR^{L2}$-,
(14) -$NR^{L1}$-CyL1-C(O)-,
(15) -$NR^{L1}$-La-CyL1-La-,
(16) -La-CyL1-,
(17) -O-La-,
(18) -S-La-, and
(19) -$NR^{L1}$-La-,

wherein:

in the (1) to (21), the leftmost extending bond of each group is connected to the $L^A$ and the rightmost extending bond of each group is connected to the

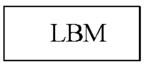

moiety, or the leftmost extending bond of each group is connected to the

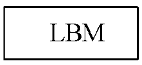

moiety and the rightmost extending bond of each group is connected to the $L^A$,
CyL1 and CyL2, at each occurrence, are each independently selected from 3-to 12-membered heterocycloalkylene, wherein the heterocycloalkylene preferably has 1, 2 or more nitrogen heteroatoms and 0, 1 or 2 heteroatoms selected from O and S,
CyL3, at each occurrence, is independently selected from $C_{3-12}$ cycloalkylene,
CyL4, at each occurrence, is independently selected from 5- to 12-membered heteroarylene,
La, at each occurrence, is independently selected from $C_{1-4}$ hydrocarbylene, Lb, at each occurrence, is independently selected from straight $C_{2-4}$ alkylene, wherein 1 or 2 but not all $CH_2$ in the straight $C_{2-4}$ alkylene are replaced with 1 or 2 groups selected from O, S, $NR^{L1}$ and C(O),
Lc, at each occurrence, is independently selected from a bond or $C_{1-4}$ hydrocarbylene,
CyL1, CyL2, CyL3, CyL4, La, Lb and Lc are each optionally substituted with 1 or more groups independently selected from: $C_{1-4}$ alkyl, $C_{1-4}$ haloalkyl, halogen, OH, CN, $NH_2$, -NH($C_{1-4}$ alkyl) and -N($C_{1-4}$ alkyl)$_2$, preferably methyl, ethyl, F, Cl, Br, OH, CN and $NH_2$, more preferably methyl, F, Cl and OH; and
$R^{L1}$ and $R^{L2}$, at each occurrence, are each independently selected from H and $C_{1-4}$ alkyl;
the

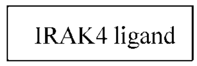

moiety has:

(I) a structure of formula (1):

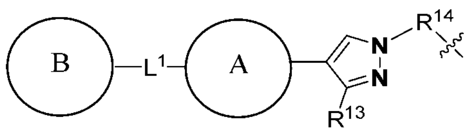

(1)

wherein:

ring A

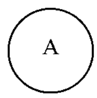

is selected from 5- to 6-membered heteroaryl, and the 5- to 6-membered heteroaryl is optionally substituted with a substituent $R^k$;
$R^k$ is selected from $C_{1-6}$ alkyl, $C_{3-7}$ cycloalkyl, $R^pR^qN$-, $C_{1-6}$ haloalkyl, $C_{1-6}$ heteroalkyl (e.g., $C_{1-6}$ alkoxy), 4- to 9-membered heterocyclyl (e.g., 5- to 6-membered saturated heterocycloalkyl), $C_{6-10}$ aryl, and 5- to 10-membered heteroaryl;
$R^p$ and $R^q$ are each independently selected from hydrogen, $C_{1-6}$ alkyl, $C_{1-6}$ alkoxy, and $C_{1-6}$ haloalkyl;
ring B

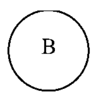

is selected from the following (1) to (3):

(1)

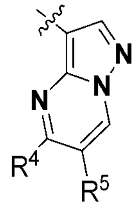

,

wherein:

$R^4$ is selected from hydrogen, $C_{1-6}$ alkyl, $C_{4-9}$ cycloalkyl, $C_{4-9}$ cycloalkyl-$C_{1-6}$ alkyl-, 4- to 9-membered saturated heterocycloalkyl, 4- to 9-membered saturated heterocycloalkyl-$C_{1-6}$ alkyl-, and $-NR_{Na}R_{Nb}$ (wherein the $R_{Na}$ and $R_{Nb}$ are each independently selected from H, $C_{1-6}$ alkyl, and $C_{3-6}$ cycloalkyl, and the $C_{1-6}$ alkyl and the $C_{3-6}$ cycloalkyl may be optionally substituted with 1-3 substituents selected from halogen, hydroxy, $C_{3-6}$ cycloalkyl, $C_{3-6}$ halocycloalkyl, and 4- to 7-membered heterocyclyl), and the $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl- are optionally substituted with 1-3 substituents selected from $C_{1-6}$ alkyl, $C_{1-6}$ alkoxy, $C_{1-6}$ haloalkyl, $C_{1-6}$ haloalkoxy, halogen, hydroxy, hydroxy-$C_{1-6}$ alkylene-, cyano, oxo, $-NH_2$, $-NH(C_{1-6}$ alkyl), and $-N(C_{1-6}$ alkyl)($C_{1-6}$ alkyl);

$R^5$ is selected from hydrogen, cyano, $C_{1-6}$ alkyl, $-C(O)NH_2$, and $-NR^lR^m$;

$R^l$ and $R^m$ are each independently selected from hydrogen, $C_{1-6}$ alkyl, and $C_{3-6}$ cycloalkyl;

(2)

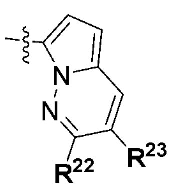

,

wherein:

$R^{22}$ is selected from hydrogen, $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl-;

$R^{23}$ is selected from hydrogen, $C_{1-6}$ alkyl, cyano, carboxyl, $-C(O)NH_2$, $-NR^lR^m$,

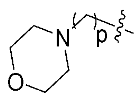 , 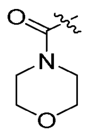 , 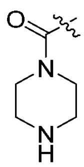 , 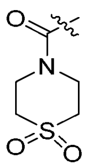 , 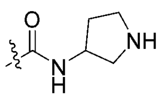 , and 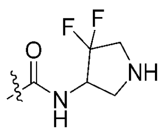 ;

and $R^l$ and $R^m$ are each independently selected from hydrogen, $C_{1-6}$ alkyl, and $C_{3-6}$ cycloalkyl;

(3)

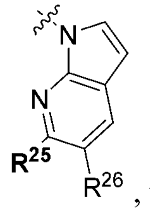

wherein:

$R^{25}$ is selected from hydrogen, $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl-; $R^{26}$ is selected from hydrogen, $C_{1-6}$ alkyl, cyano, carboxyl, and -C(O)$NH_2$;
$L^1$ is selected from a direct bond, $C_{1-6}$ alkyl, -NH-, -O-, and -S-;
$R^{13}$ is selected from $C_{1-6}$ haloalkyl;
$R^{14}$ is selected from $C_{3-6}$ cycloalkylene, 5- to 12-membered spiro heterocycloalkylene and piperidylidene, and the $C_{3-6}$ cycloalkylene, 5- to 12-membered spiro heterocycloalkylene and piperidylidene are optionally substituted with 1-2 substituents selected from hydroxy$C_{1-6}$ alkyl (e.g., hydroxymethyl), formyl, and $C_{1-6}$ alkyl;

or

(II) a structure of formula (2)

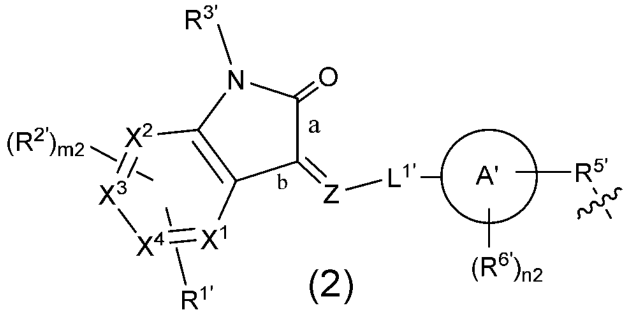

(2)

wherein:

ring A'

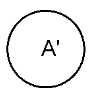

is selected from 5- to 10-membered heteroaryl;
$X^1$, $X^2$, $X^3$ and $X^4$ are each independently N or CH; and at least one of $X^1$, $X^2$, $X^3$ and $X^4$ is not N;
Z is $CR^{4'}$;
each of the letters "a" and "b" indicates the bond between the ring carbon atom connected to Z and adjacent two ring carbon atoms;

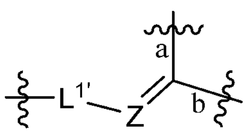

moiety is represented by a structure of the following formula (i) or formula (ii):

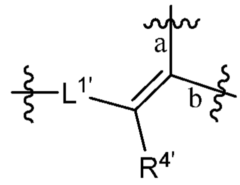 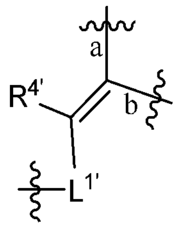

(i) or (ii) ;

$L^{1'}$ is selected from a direct bond and $NR^{7'}$; $R^{1'}$ is $-L^{2'}-R^{1a}$;

$L^{2'}$ is $-S(O)_2NR^{1b}-*$, $-C(O)-NR^{1b}-*$, $-NR^{1b}-C(O)-*$ or $-NR^{1b}-S(O)_2-*$, wherein the bond indicated with * is connected to $R^{1a}$;

$R^{1a}$ is selected from $C_{6-10}$ aryl and 5- to 10-membered heteroaryl, wherein the $C_{6-10}$ aryl and the 5- to 10-membered heteroaryl are each optionally substituted with 1 or more substituents independently selected from the following groups: halogen, OH, SH, $-NR^{1e}R^{1f}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{1e}R^{1f}$;

each $R^{2'}$ is independently selected from: H, halogen, OH, SH, $-NR^{2a}R^{2b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, - $C_{1-6}$ alkylene-OH, $-C_{1-6}$ alkylene-SH, $-C_{1-6}$ alkylene-CN, $-C_{1-6}$ alkylene-$NR^{2a}R^{2b}$;

m2 is 0, 1, 2 or 3;

$R^{3'}$ and $R^{7'}$ are each independently selected from: H, $C_{1-6}$ alkyl, $C_{1-6}$ haloalkyl, $C_{2-6}$ alkenyl, $C_{2-6}$ haloalkenyl, $-C_{1-6}$ alkylene-$O-C_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{3a}R^{3b}$;

$R^{4'}$ is selected from: H, D, halogen, OH, SH, $-NR^{4a}R^{4b}$, CN, $C_{1-6}$ alkyl, $C_{1-6}$ haloalkyl, $C_{2-6}$ alkenyl, $C_{2-6}$ haloalkenyl, $-C_{1-6}$ alkylene-$O-C_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{4a}R^{4b}$;

$R^{5'}$ is selected from $C_{3-10}$ cyclohydrocarbylene and 3- to 10-membered heterocyclylene, wherein the $C_{3-10}$ cyclohydrocarbylene and the 3- to 10-membered heterocyclylene are each optionally substituted with 1 or more substituents independently selected from the following groups: halogen, OH, SH, $-NR^{5a}R^{5b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, - $C_{1-6}$ alkylene-OH, $-C_{1-6}$ alkylene-SH, $-C_{1-6}$ alkylene-CN and $-C_{1-6}$ alkylene-$NR^{5a}R^{5b}$;

$R^{6'}$ is selected from: H, halogen, OH, SH, $-NR^{6a}R^{6b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-O-$C_{1-6}$ alkyl, $-OC_{1-6}$ alkylene-$OC_{1-6}$ alkyl, $-C_{1-6}$ alkylene-OH, - $C_{1-6}$ alkylene-SH, $-C_{1-6}$ alkylene-CN and $-C_{1-6}$ alkylene-$NR^{6a}R^{6b}$;

n2 is 0, 1, 2, 3 or 4; and

$R^{1b}$, $R^{1e}$, $R^{1f}$, $R^{2a}$, $R^{2b}$, $R^{3a}$, $R^{3b}$, $R^{4a}$, $R^{4b}$, $R^{5a}$, $R^{5b}$, $R^{6a}$, and $R^{6b}$, at each occurrence, are independently selected from H and $C_{1-6}$ alkyl; and

the

LBM

moiety is a ligase binding moiety.

Linking group $L^A$

In some embodiments, the present disclosure provides a compound of formula (A) according to the present disclosure, which has an $L^A$ group as defined above.

In some embodiments, $L^A$ is selected from a bond and $C_{1-2}$ alkylene, and the $C_{1-2}$ alkylene is optionally substituted with 1 or more substituents independently selected from $C_{1-2}$ alkyl, $C_{1-2}$ haloalkyl, halogen, oxo, OH, CN, $NH_2$, $-NH(C_{1-2}$ alkyl) and $-N(C_{1-2}$ alkyl$)_2$.

In some embodiments, $L^A$ is selected from a bond and $C_{1-2}$ alkylene, and the C1-2 alkylene is optionally substituted with a substituent selected from C1-2 alkyl, halogen and oxo.

In some embodiments, $L^A$ is selected from a bond, $-CH_2-$, $-CH_2-CH_2-$, $-CH(CH_3)-$ and -C(O)-. In some preferred embodiments, $L^A$ is $-CH_2-$ or $-CH_2-CH_2-$, and more preferably $-CH_2-$.

IRAK4 ligands

I) IRAK ligand having the structure of formula (1)

In some embodiments, the present disclosure provides a compound of formula (A) according to the present disclosure, wherein the

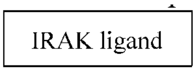

moiety has the structure of formula (1).

In some embodiments, the ring A is selected from 5-membered heteroaryl, the 5-membered heteroaryl contains at least one N atom, and the 5-membered heteroaryl is optionally substituted with a substituent $R^k$.

In some embodiments, the ring A is selected from 5-membered heteroaryl, and the 5-membered heteroaryl contains 2-3 heteroatoms in which at least 2 heteroatoms are N atoms; the 5-membered heteroaryl is optionally substituted with a substituent $R^k$.

In some preferred embodiments, the ring A is selected from 1,2,3-triazolyl, 1,2,4-triazolyl, pyrazolyl, imidazolyl, 1,3,4-thiadiazolyl and 1,3,4-oxadiazolyl.

More preferably, the ring A is selected from

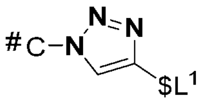 , 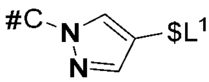

, 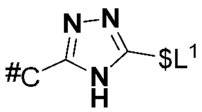 , 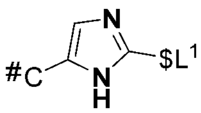 , 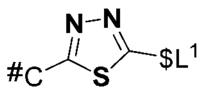 and 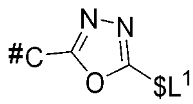 ,

wherein #C represents a connection site to

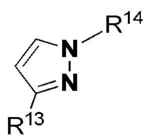

moiety, and $\$L^1$ represents a connection site to $L^1$.

Further preferably, the ring A is selected from

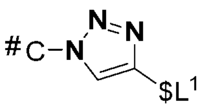

and

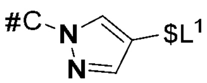

,

wherein #C represents a connection site to

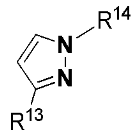

moiety, and $L^1 represents a connection site to $L^1$.

In some embodiments, $R^k$ is selected from $C_{1-6}$ alkyl, $C_{3-6}$ cycloalkyl, $R^pR^qN$-, $C_{1-6}$ haloalkyl and 4- to 9-membered saturated heterocycloalkyl (e.g., 5- to 6-membered saturated heterocycloalkyl).

Preferably, $R^k$ is selected from $C_{1-6}$ alkyl, $C_{3-6}$ cycloalkyl, $R^pR^qN$-, $C_{1-6}$ haloalkyl and morpholinyl.

In some embodiments, $R^p$ and $R^q$ are each independently selected from hydrogen and $C_{1-6}$ alkyl.

In some preferred embodiments, $R^k$ is selected from isopropyl, cyclopropyl, dimethylamino, difluoromethyl, and

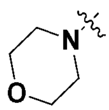

.

In some embodiments, $L^1$ is selected from a direct bond and -NH-, preferably a direct bond.

In some embodiments, $R^{13}$ is selected from $C_{1-4}$ haloalkyl, preferably $-CHF_2$ and trifluoromethyl, and more preferably $-CHF_2$.

In some embodiments, the structure of formula (1) is represented by the following formula (1-1) or (1-2):

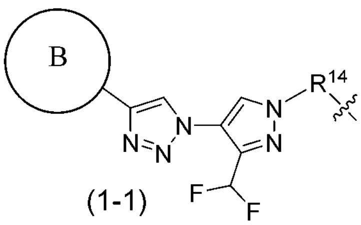

(1-1)

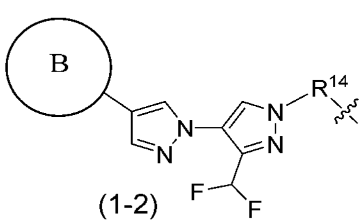

(1-2)

.

In some embodiments, the ring B is a group selected from the following (1) to (3).

In some embodiments, in (1)

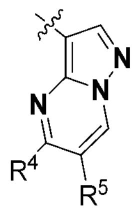

,

$R^4$ is selected from hydrogen, 4- to 7-membered saturated monocyclic heterocycloalkyl, 6- to 9-membered saturated bridged heterocycloalkyl, 6- to 9-membered saturated spiro heterocycloalkyl, 4- to 7-membered saturated monocyclic heterocycloalkyl-$C_{1-4}$ alkyl-, 6- to 9-membered saturated bridged heterocycloalkyl-$C_{1-4}$ alkyl-, $-NR_{Na}R_{Nb}$ (wherein the $R_{Na}$ and $R_{Nb}$ are each independently selected from H, $C_{1-6}$ alkyl, and $C_{3-6}$ cycloalkyl, and the $C_{1-6}$ alkyl and the $C_{3-6}$ cycloalkyl may be optionally substituted with 1-3 substituents selected from halogen, hydroxy, $C_{3-6}$ cycloalkyl, $C_{3-6}$ halocycloalkyl, and 4- to 7-membered heterocyclyl), and the 4- to 7-membered saturated monocyclic heterocycloalkyl, the 6- to 9-membered saturated bridged heterocycloalkyl, the 6- to 9-membered saturated spiro heterocycloalkyl, the 4- to 7-membered saturated monocyclic heterocycloalkyl-$C_{1-4}$ alkyl-, and the 6- to 9-membered saturated bridged heterocycloalkyl-$C_{1-4}$ alkyl- are optionally substituted with 1-3 substituents selected from $C_{1-6}$ alkyl, $C_{1-6}$ alkoxy, $C_{1-6}$ haloalkyl, $C_{1-6}$ haloalkoxy, halogen, hydroxy, hydroxy-$C_{1-6}$ alkylene-, oxo, $-NH_2$, $-NH(C_{1-6}$ alkyl), and $-N(C_{1-6}$ alkyl)($C_{1-6}$ alkyl);

In some embodiments, $R^4$ is selected from hydrogen, 4- to 7-membered saturated monocyclic heterocycloalkyl, 6- to 9-membered saturated bridged heterocycloalkyl, 6- to 9-membered saturated spiro heterocycloalkyl, $-NR_{Na}R_{Nb}$ (wherein the $R_{Na}$ and $R_{Nb}$ are each independently selected from H, $C_{1-6}$ alkyl, and $C_{3-6}$ cycloalkyl, and the $C_{1-6}$ alkyl and the $C_{3-6}$ cycloalkyl may be optionally substituted with 1, 2 or 3 substituents selected from hydroxy and $C_{3-6}$ cycloalkyl), and the 4- to 7-membered saturated monocyclic heterocycloalkyl, the 6- to 9-membered saturated bridged heterocycloalkyl, and the 6- to 9-membered saturated spiro heterocycloalkyl are optionally substituted with 1,

2 or 3 substituents selected from $C_{1-6}$ alkyl, $C_{1-6}$ haloalkyl, hydroxy, hydroxy-$C_{1-6}$ alkylene-, $-NH_2$, $-NH(C_{1-6}$ alkyl), and $-N(C_{1-6}$ alkyl)($C_{1-6}$ alkyl);
$R^4$ is selected from hydrogen,

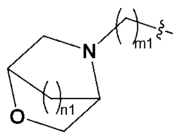, 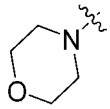, 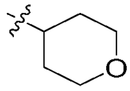, 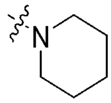,

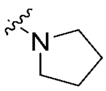, 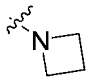, 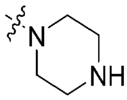, 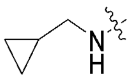, 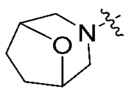, 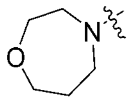, 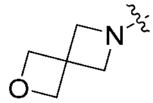,

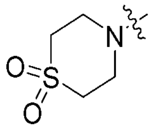, 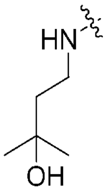 and 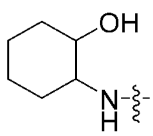, and

the

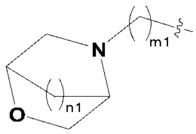, 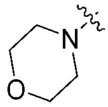, 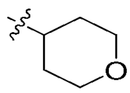

, 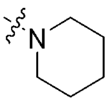, 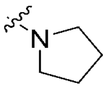, 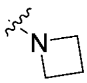, 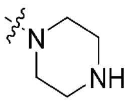, 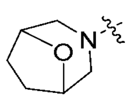, 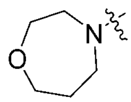 and 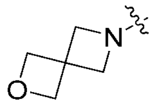

are optionally substituted with 1-3 substituents selected from $C_{1-6}$ alkyl, halogen, hydroxy, hydroxy($C_{1-6}$ alkyl)-, cyano, $-NH_2$, $-N(C_{1-6}$ alkyl) and $-N(C_{1-6}$ alkyl)($C_{1-6}$ alkyl), m1 is selected from 0, 1, 2 and 3, preferably 0, and n1 is selected from 0, 1, 2 and 3, preferably 0 or 1.

In some embodiments, $R^4$ is selected from hydrogen,

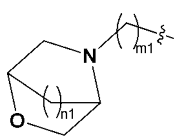, 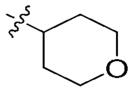

, 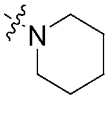, 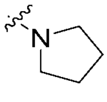, 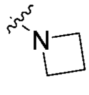, 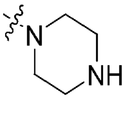, 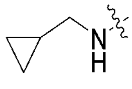, 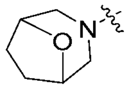, 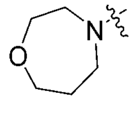,

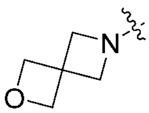, 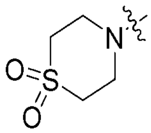, 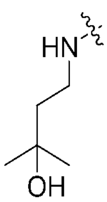 and 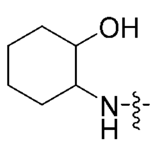,

and the

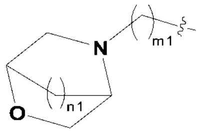,

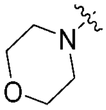, 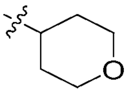, 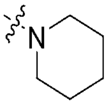, 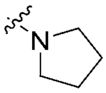, 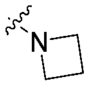, 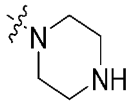, 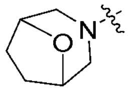, 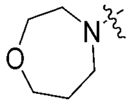

and

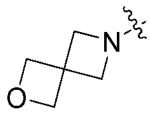

are optionally substituted with 1-3 substituents selected from $C_{1-6}$ alkyl, halogen, hydroxy, hydroxy($C_{1-6}$ alkyl)-, cyano, $-NH_2$, $-N(C_{1-6}$ alkyl) and $-N(C_{1-6}$ alkyl)($C_{1-6}$ alkyl), m1 is selected from 0, 1, 2 and 3, preferably 0, and n1 is selected from 0, 1, 2 and 3, preferably 0 or 1.

In some embodiments, $R^4$ is selected from hydrogen,

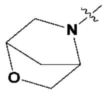, 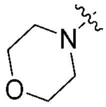,

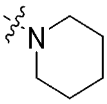, 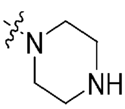, 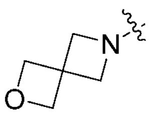, 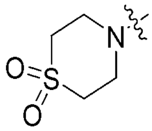, 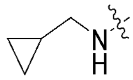, 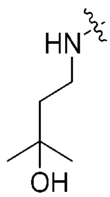,

and

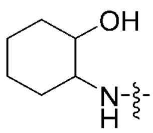,

and the

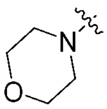, 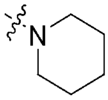, and 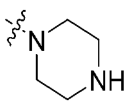

are optionally substituted with 1, 2 or 3 substituents selected from $C_{1-6}$ alkyl, hydroxy, $-NH_2$, and hydroxy($C_{1-6}$ alkyl)-.

In some embodiments, $R^4$ is selected from hydrogen,

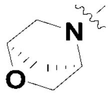, 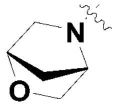,

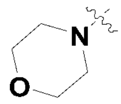,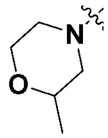

(including

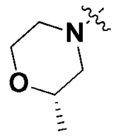 and 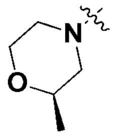

),

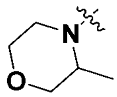

(including

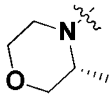 and 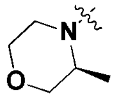

),

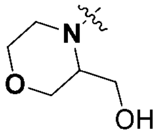

(including

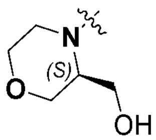 and 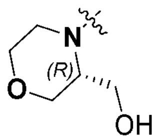

),

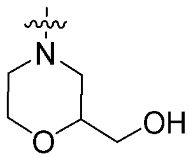

(including

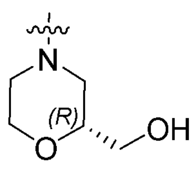 and 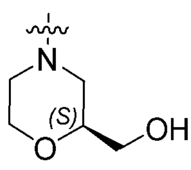

),

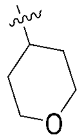 , 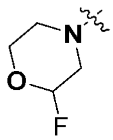 ,

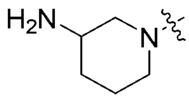

(including

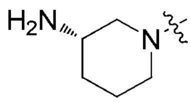 and 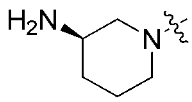

),

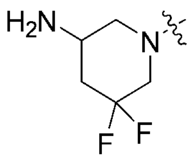 ,

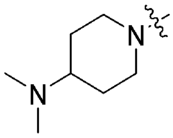 , 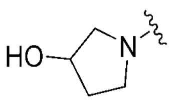 , 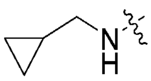 , 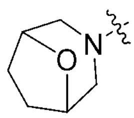 , 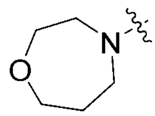 , 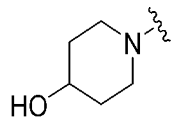 ,

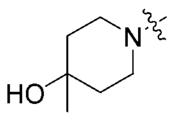 , 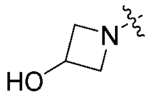 , 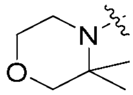 , 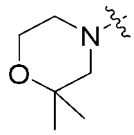 , 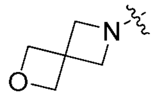 , 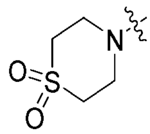 ,

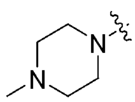 , 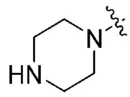 , 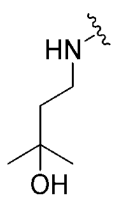 , 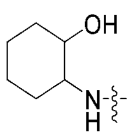

(including

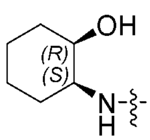 , 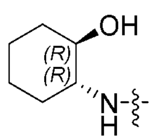 ,

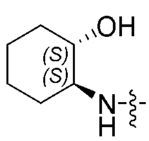 , 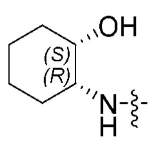 ,

preferably

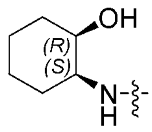 and 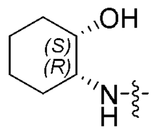

),

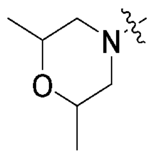

(including

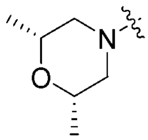, 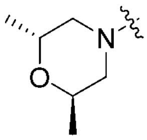, 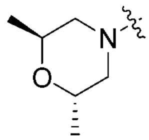, 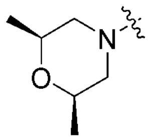,

preferably

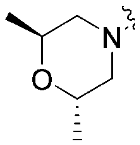

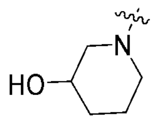

(including

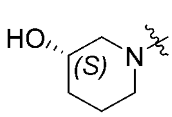 or 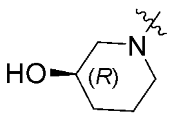

).

In some embodiments, $R^4$ is selected from hydrogen,

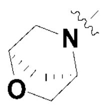, 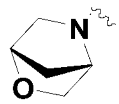,

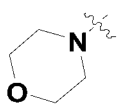, 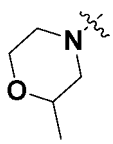

(including

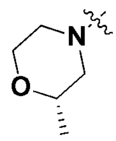 and 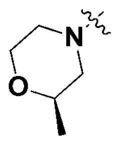 ), 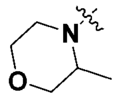

(including

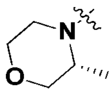 and 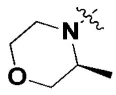 ), 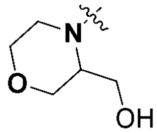

(including

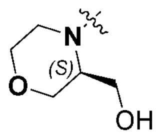 and 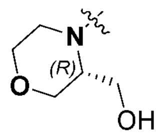

),

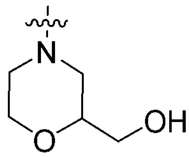

(including

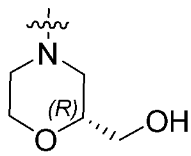 and 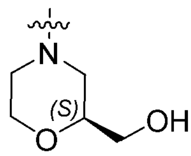

),

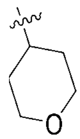, 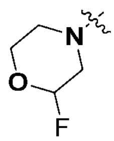,

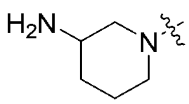

(including

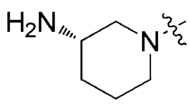 and 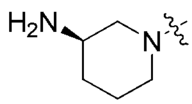

),

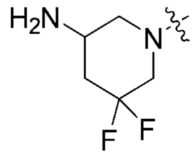,

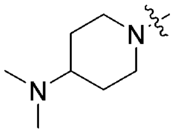, 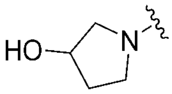, 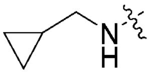, 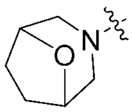, 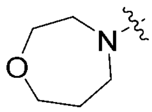, 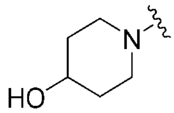,

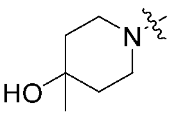, 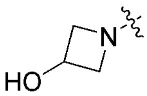, 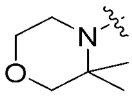, 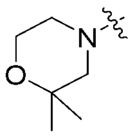, 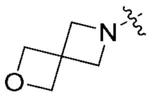, 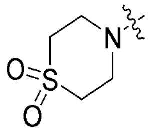,

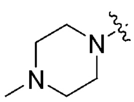, 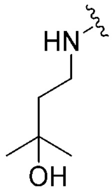, 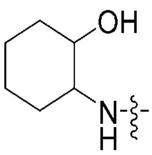

(including

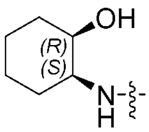, 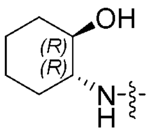, 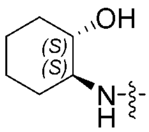

, 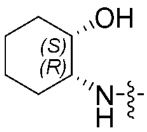,

preferably

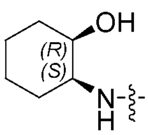 and 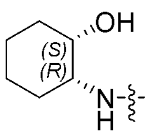

),

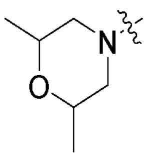

(including

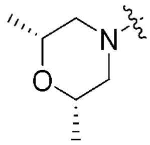, 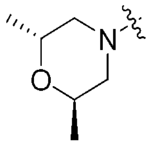, 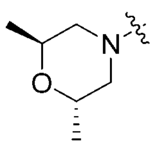, 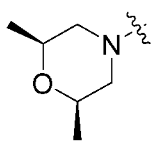,

preferably

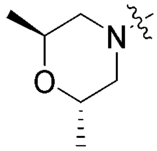

),

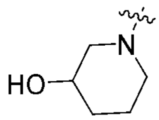

(including

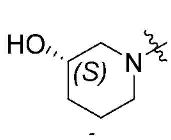 or 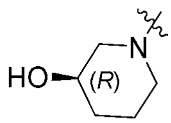

).

In some embodiments, $R^5$ is selected from hydrogen, cyano, $-C(O)NH_2$, and - $NR^lR^m$.

In some embodiments, $R^l$ and $R^m$ are each independently selected from hydrogen and $C_{1-6}$ alkyl.

In some embodiments, $R^5$ is selected from hydrogen and cyano.

In some embodiments,

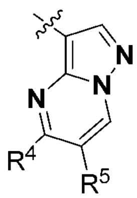

as a whole is selected from

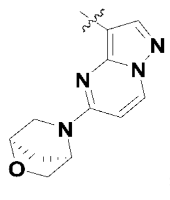,

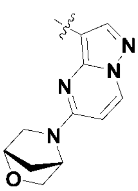, 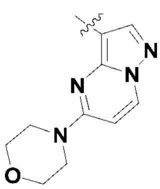, 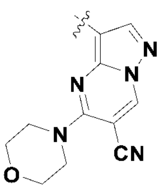, 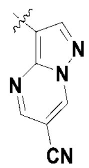, 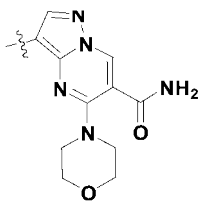, 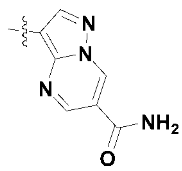,

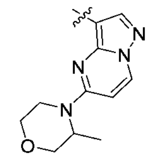

(preferably

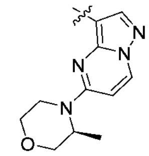

),

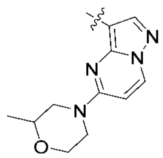

(preferably

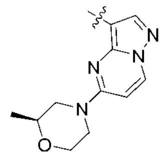

or

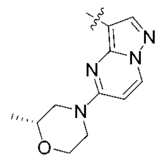

),

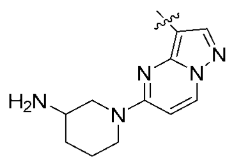

(preferably

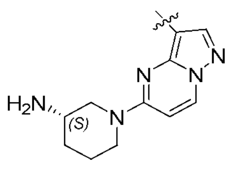

or

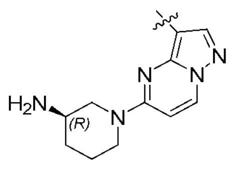

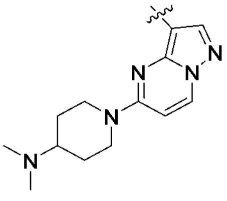, 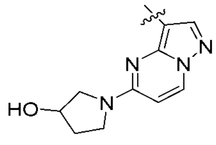, 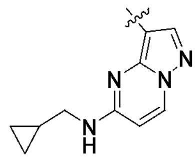, 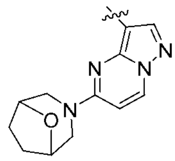, 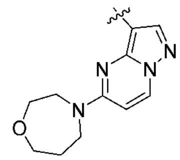, 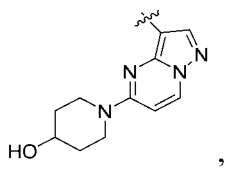, 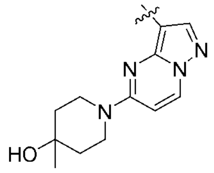, 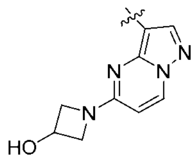, 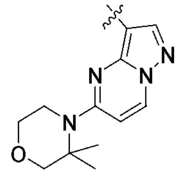, 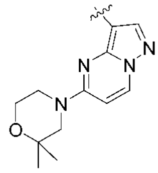, 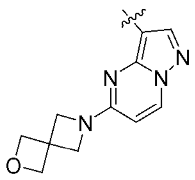, 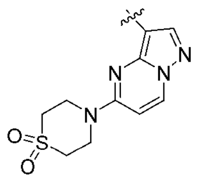, 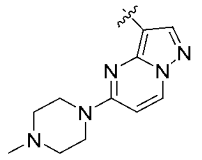, 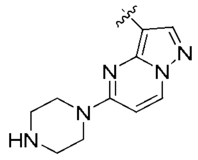, 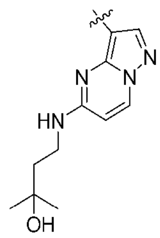,

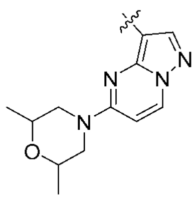

(preferably

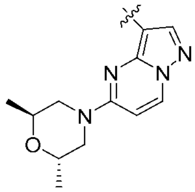

),

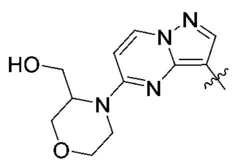

(preferably

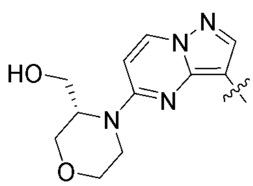

),

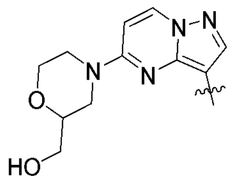

(preferably

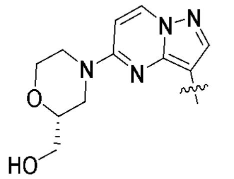

),

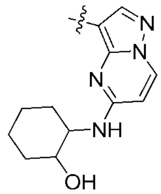

(preferably

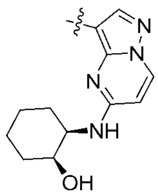

and

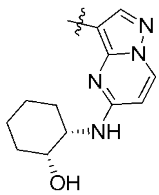

),

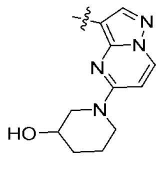

(preferably

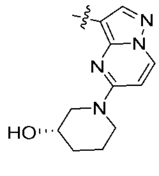

or

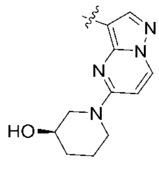

) and

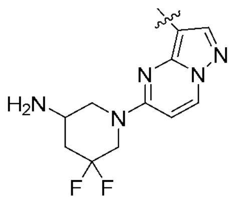

.

In some preferred embodiments,

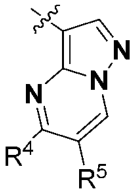

as a whole is selected from

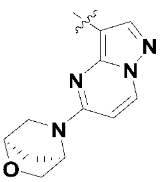, 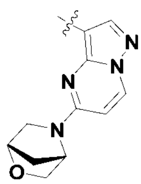, 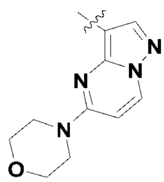, 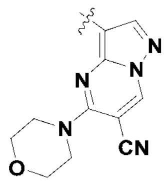, 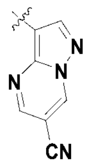, 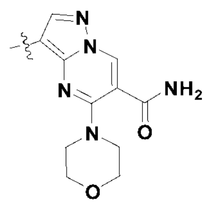,

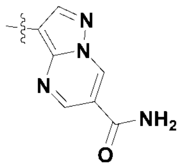, 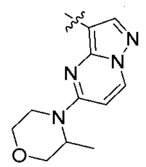

(preferably

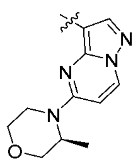

),

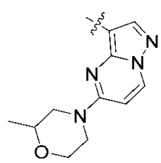

(preferably

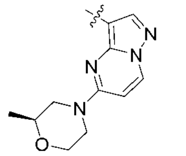

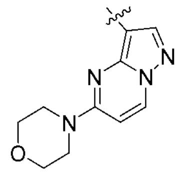

,

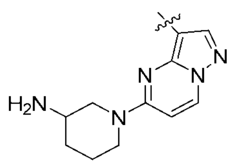

(

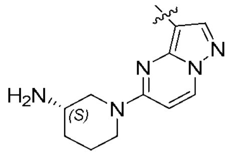

or

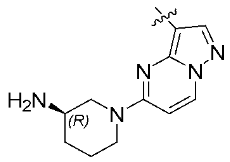

),

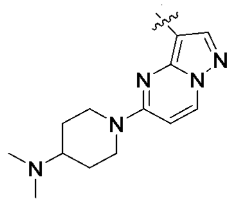

,

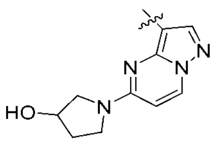

,

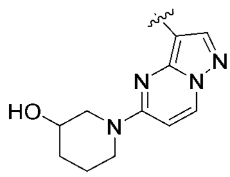

,

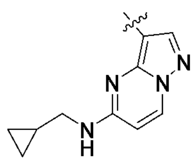

,

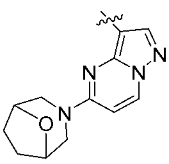

,

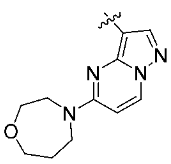

,

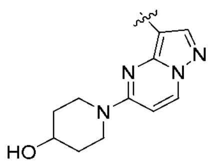

,

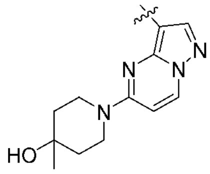

,

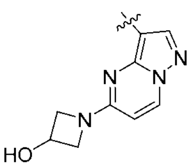

,

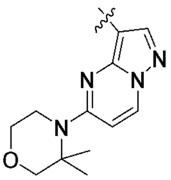

,

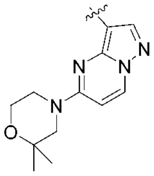

,

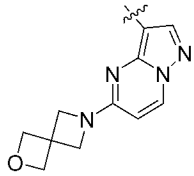

,

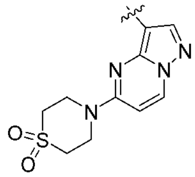

,

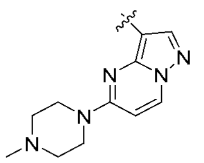 , 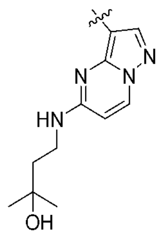 , , 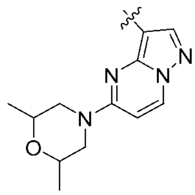

(preferably

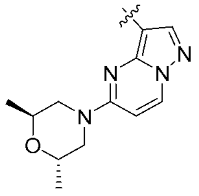

),

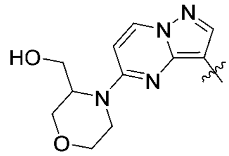

(preferably

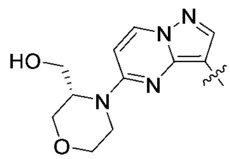

),

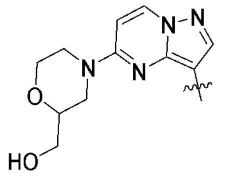

(preferably

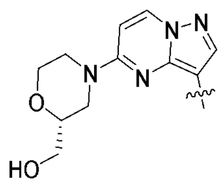

),

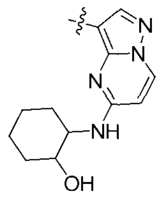

(preferably

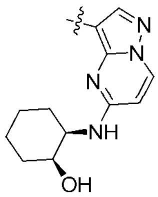

and

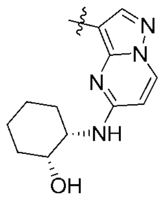

),

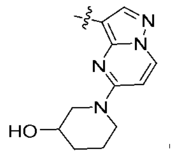

(preferably

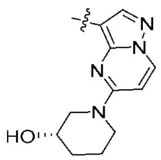

or

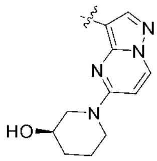

) and

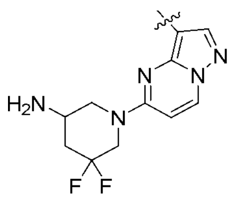

.

In some embodiments,

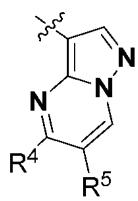

as a whole is selected from

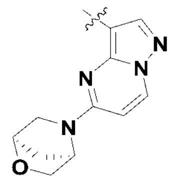,

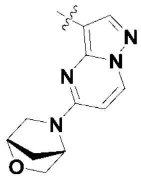, 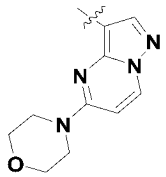, 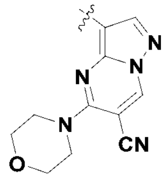, 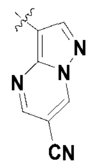, 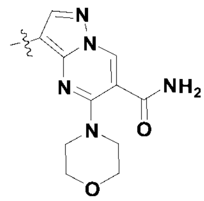, 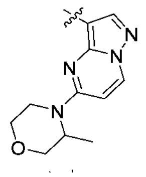

(preferably

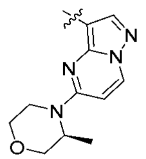

),

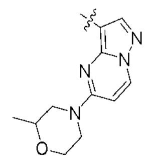

(preferably

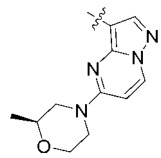

),

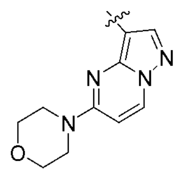,

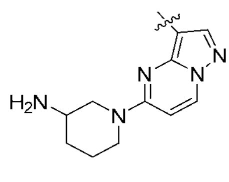

(

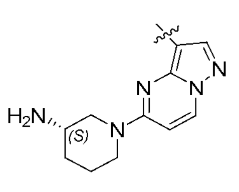
or
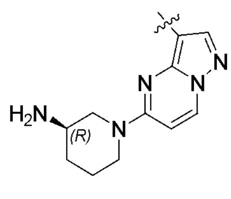

),

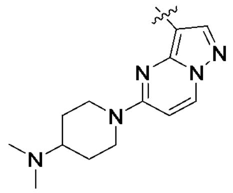
,

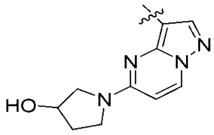
,
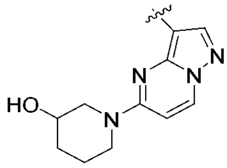
,
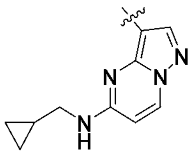
,
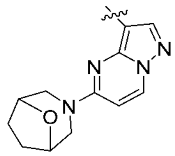
,
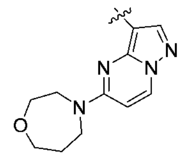
,

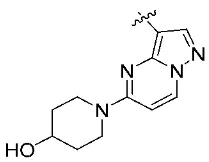
,
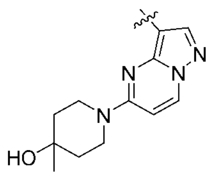
,
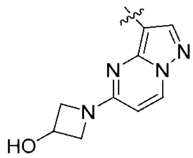
,
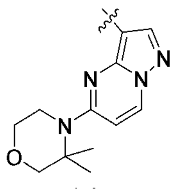
,
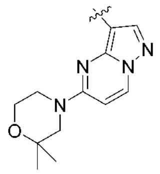
,

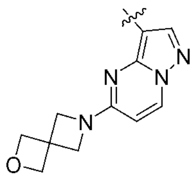
,
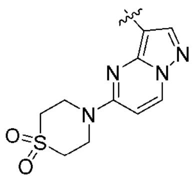
,
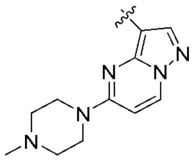
,
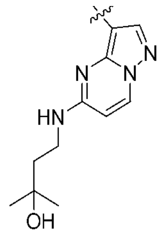
,
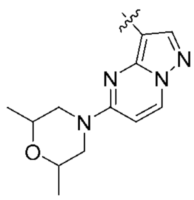

(preferably

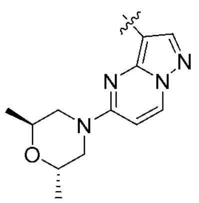

),

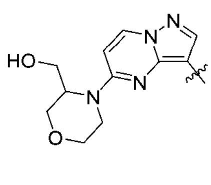

(preferably

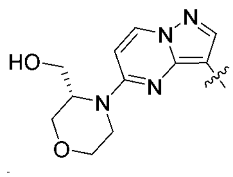

),

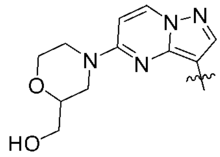

(preferably

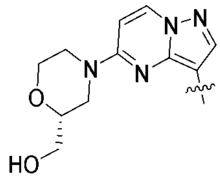

),

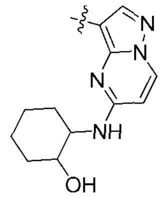

(preferably

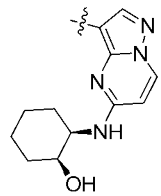

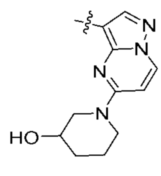

(preferably

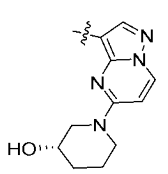

or

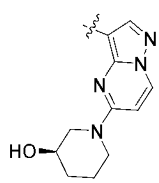

) and

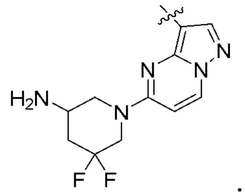

.

**In** some embodiments,

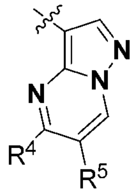

as a whole is selected from

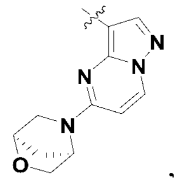

,

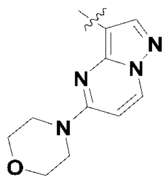

,

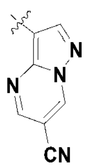

,

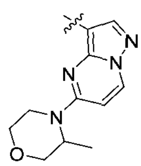

(preferably

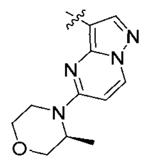

),

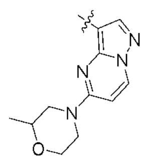

(preferably

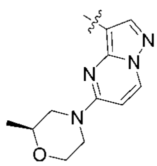

or

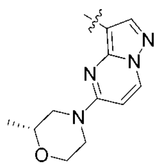

),

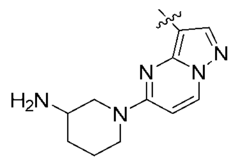

(preferably

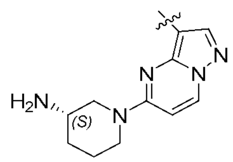

or

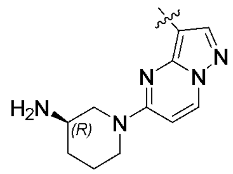

),

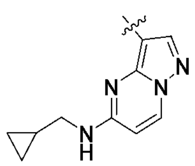 , 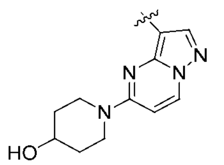 , 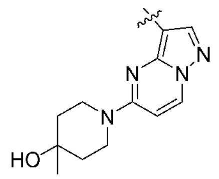 ,

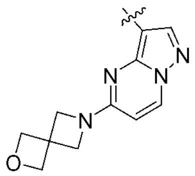 , 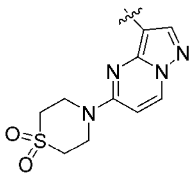 , 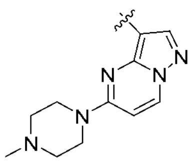 , 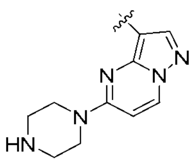 , 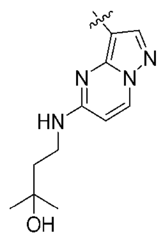 ,

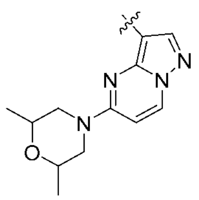

(preferably

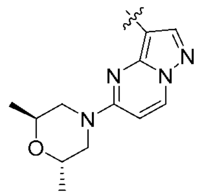

),

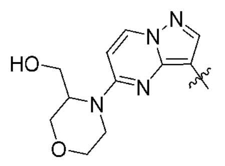

(preferably

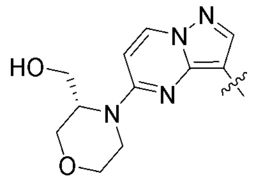

),

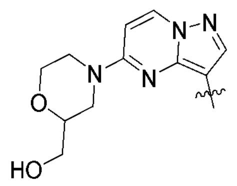

(preferably

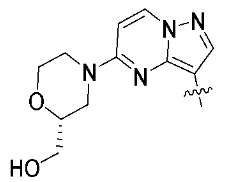

),

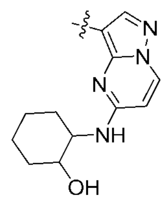

(preferably

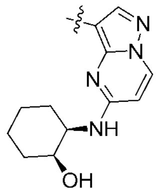 and 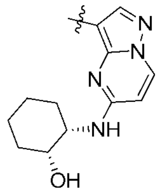

),

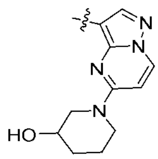

(preferably

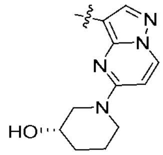

or

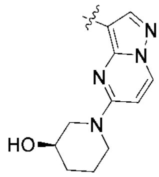

).

In some embodiments, in (2)

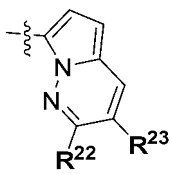

,

$R^{22}$ is selected from hydrogen and

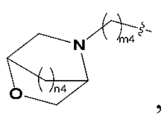

,

m4 is selected from 0, 1, 2, and 3, and n4 is selected from 0, 1, 2, and 3.

Preferably, $R^{22}$ is selected from hydrogen and

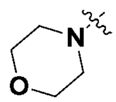

.

More preferably, $R^{22}$ is hydrogen.

In some embodiments, $R^{23}$ is selected from hydrogen, $C_{1-6}$ alkyl, cyano, carboxyl, $-C(O)NH_2$ and $-NR^lR^m$.

Preferably, $R^{23}$ is selected from hydrogen, cyano, carboxyl, $-C(O)NH_2$ and - $NR^lR^m$.

More preferably, $R^{23}$ is selected from hydrogen, cyano, -C(O)$NH_2$ and -$NR^lR^m$.

Further preferably, $R^{23}$ is selected from -C(O)$NH_2$.

In some embodiments, $R^l$ and $R^m$ are each independently selected from hydrogen and $C_{1-6}$ alkyl.

In some preferred embodiments,

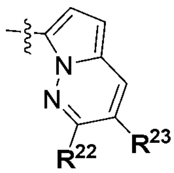

as a whole is selected from

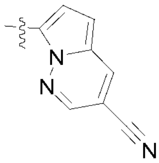, 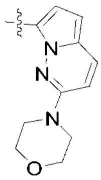, 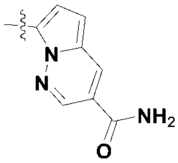, 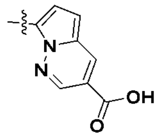, 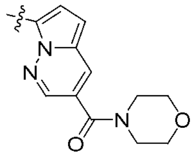, 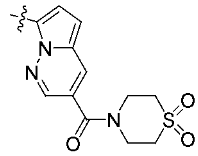,

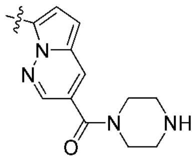, 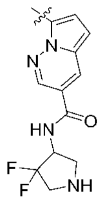 and 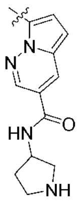.

More preferably,

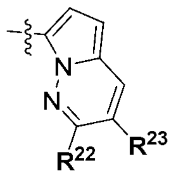

as a whole is selected from

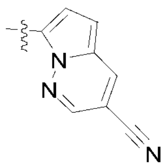,

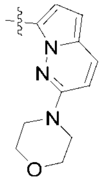 and 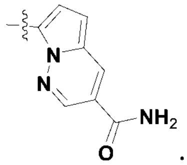.

More preferably,

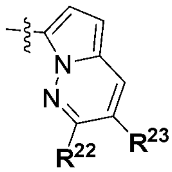

as a whole is

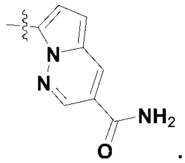

.

In some embodiments, in (3)

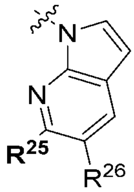

,

$R^{25}$ is selected from hydrogen,

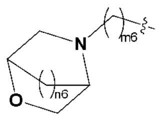

and

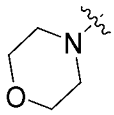

,

m6 is selected from 0, 1, 2 and 3, and n6 is selected from 0, 1, 2 and 3.

Preferably, $R^{25}$ is selected from hydrogen and

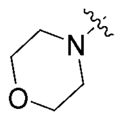

.

More preferably, $R^{25}$ is hydrogen.

In some embodiments, $R^{26}$ is selected from hydrogen, $C_{1-6}$ alkyl, cyano and - $C(O)NH_2$.

Preferably, $R^{26}$ is selected from hydrogen, $C_{1-6}$ alkyl and $-C(O)NH_2$.

More preferably, $R^{26}$ is selected from $-C(O)NH_2$.

In some preferred embodiments,

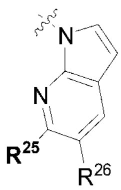

as a whole is selected from

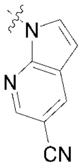,

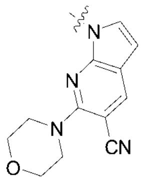, 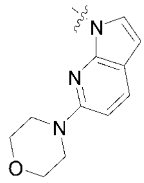, 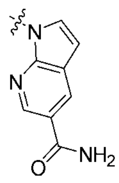 and 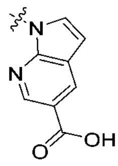.

In some more preferred embodiments,

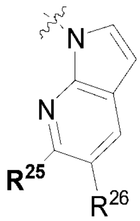

as a whole is

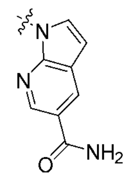.

In some embodiments, $R^{14}$ is selected from $C_{3-6}$ cycloalkylene, and the $C_{3-6}$ cycloalkylene is optionally substituted with 1-2 substituents selected from hydroxy$C_{1-6}$ alkyl (e.g., hydroxymethyl) and formyl. Preferably, $R^{14}$ is selected from

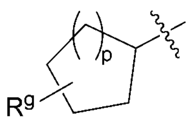;

p is selected from 0, 1, and 2; $R^g$ is selected from hydrogen, hydroxy$C_{1-6}$ alkyl, and formyl. Preferably, $R^g$ is hydrogen.

In some other embodiments, $R^{14}$ is selected from 7- to 11-membered spiro heterocycloalkylene, and the 7- to 11-membered spiro heterocycloalkylene is optionally substituted with 1-2 substituents selected from hydroxy$C_{1-6}$ alkyl (e.g., hydroxymethyl), formyl and $C_{1-3}$ alkyl. Preferably, $R^{14}$ is selected from 9- to 11-membered spiro heterocycloalkylene, and the 9- to 11-membered spiro heterocycloalkylene has 1, 2 or more nitrogen heteroatoms and 0, 1 or 2 heteroatoms selected from O and S.

In some other embodiments, $R^{14}$ is selected from piperidylidene, and the piperidylidene is optionally substituted with 1-2 $C_{1-3}$ alkyl (preferably methyl).

In some preferred embodiments, $R^{14}$ is selected from

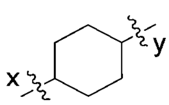,

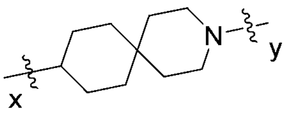, 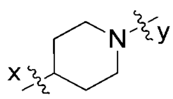, 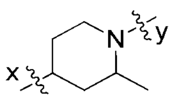 and 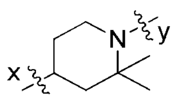,

further more preferably

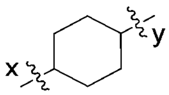, 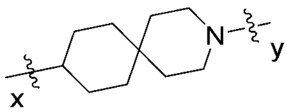, 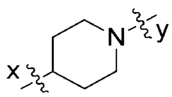

and

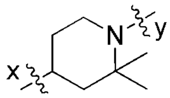,

even more preferably

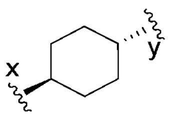, 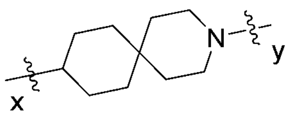,

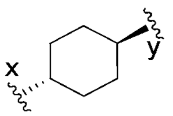, 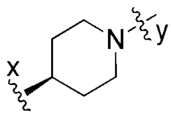, 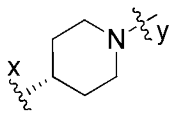, 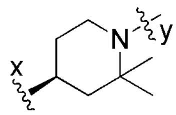 and 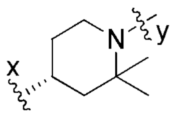,

wherein the bond labeled with x is connected to the pyrazole ring, and the bond labeled with y is connected to the $L^A$.

In some embodiments, the

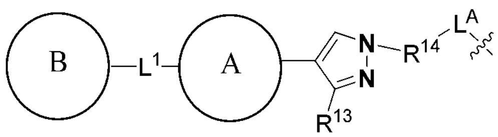

moiety is selected from:

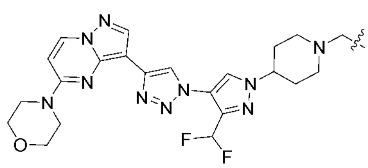, 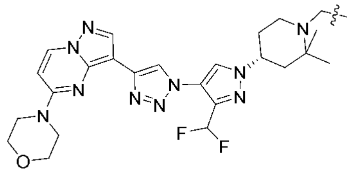,

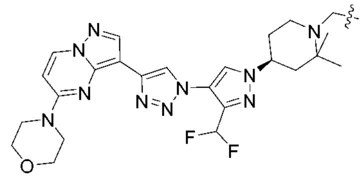, 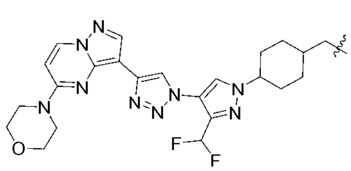,

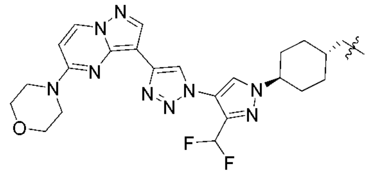, 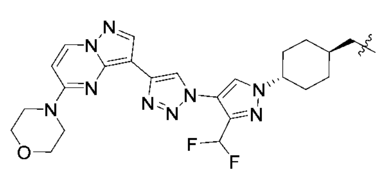,

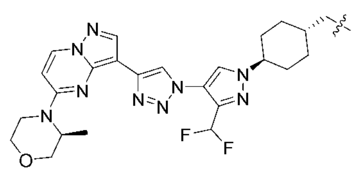, 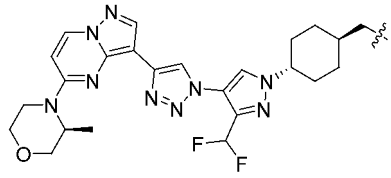,

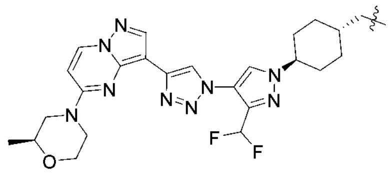, 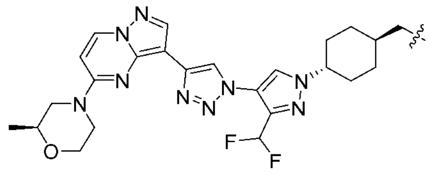,

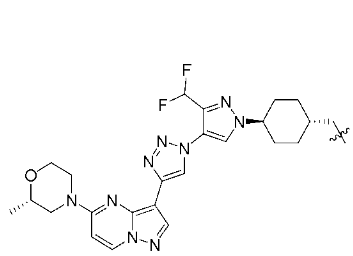, 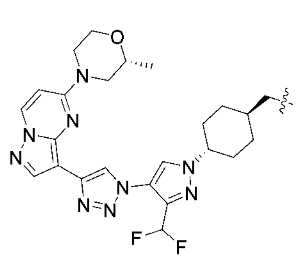, 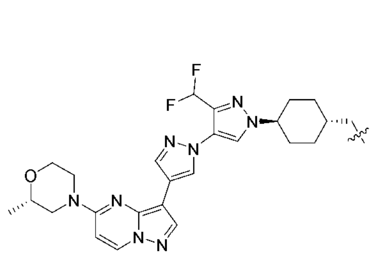,

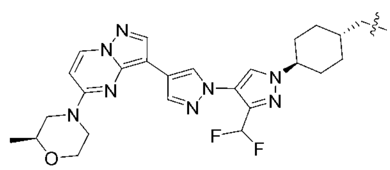, 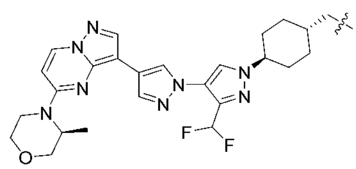,

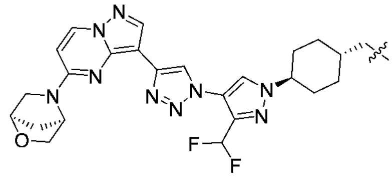, 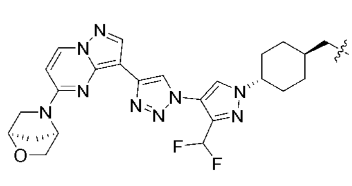,

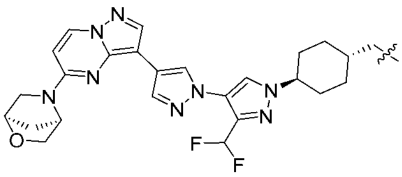, 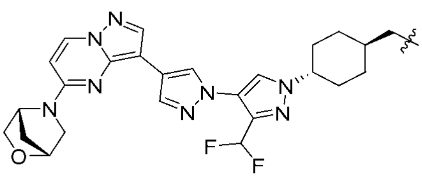,

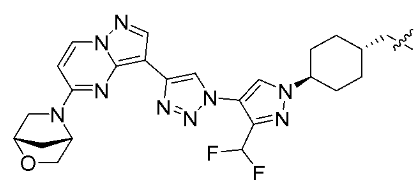 , 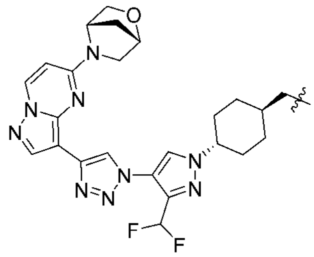 ,

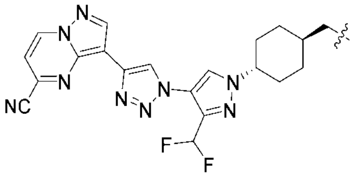 , 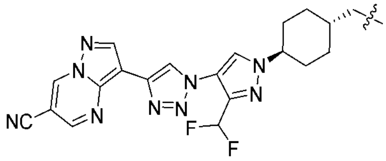 ,

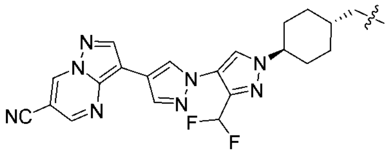 , 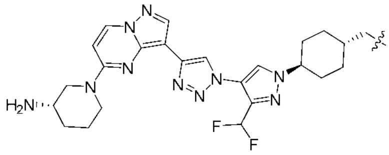 ,

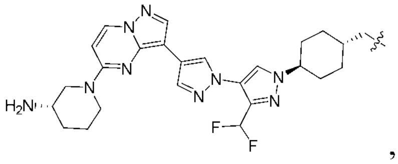 , 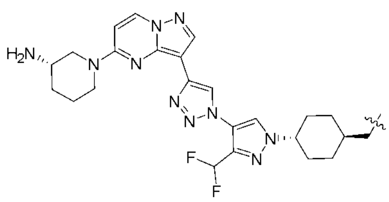 ,

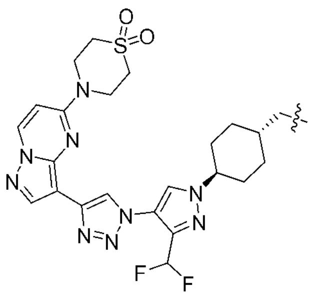 , 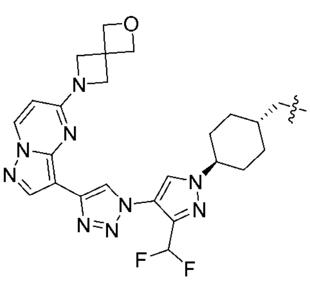 , 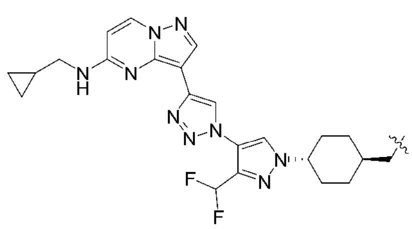 ,

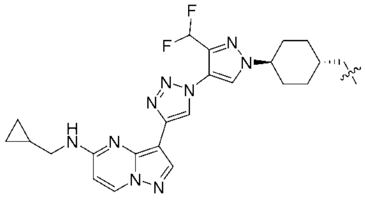 , 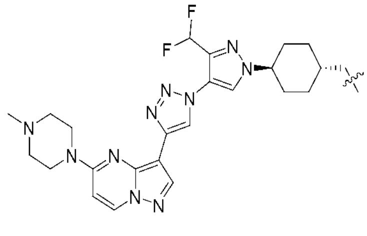 ,

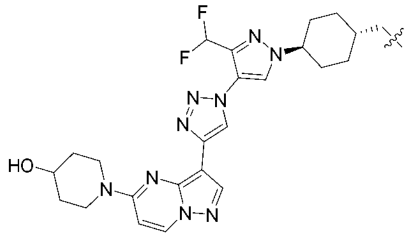,
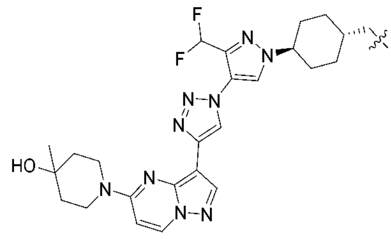,

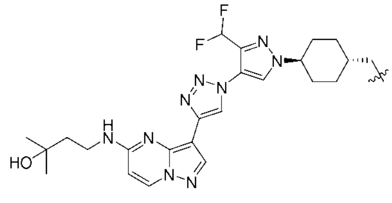,
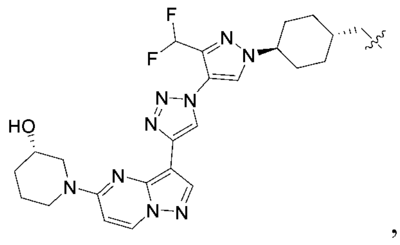,

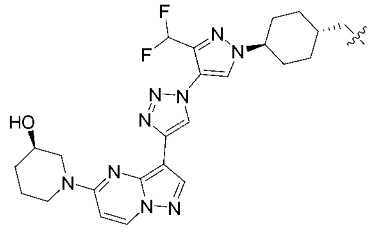,
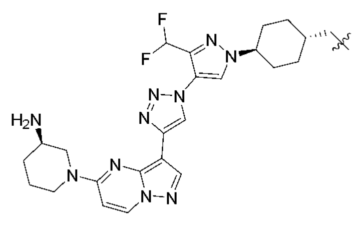,

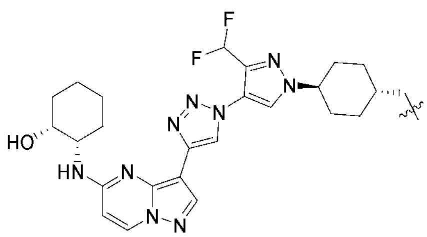,
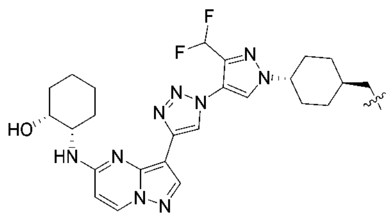,

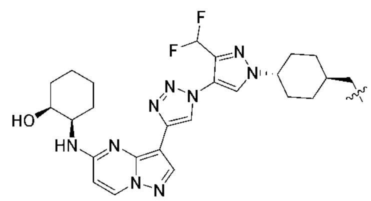,
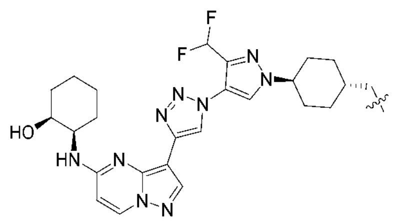,

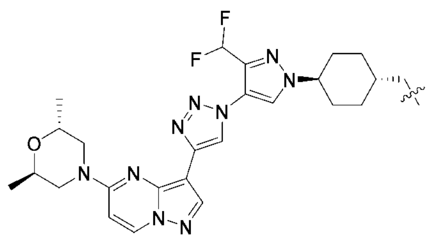,
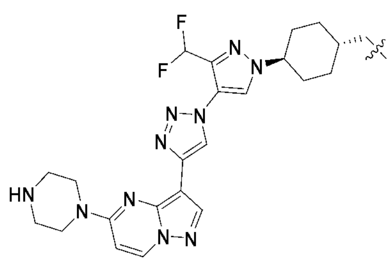,

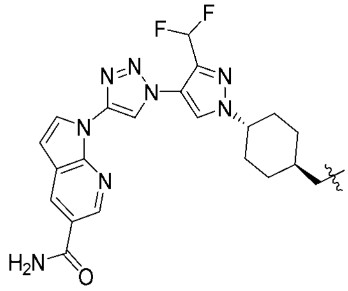
,
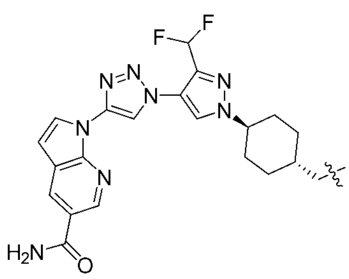
,
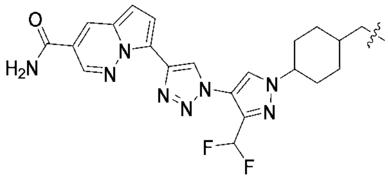
,
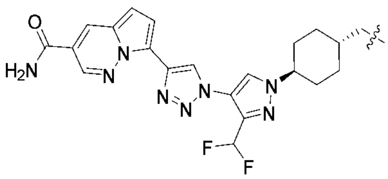
,
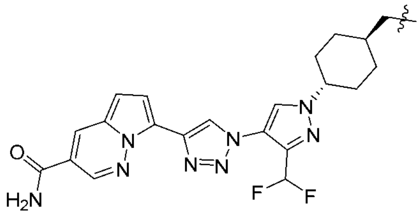
,
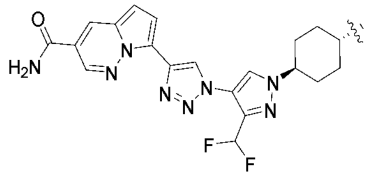
,
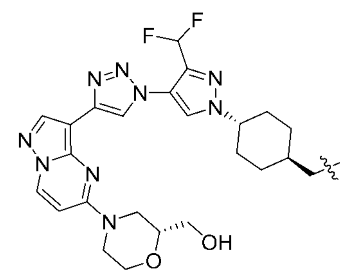
,
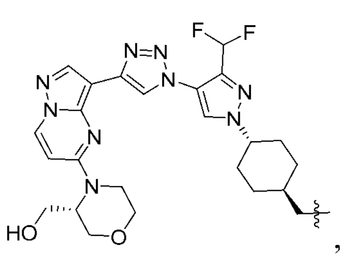
,
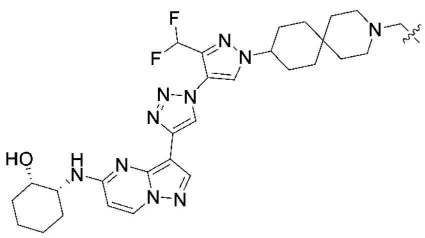
,
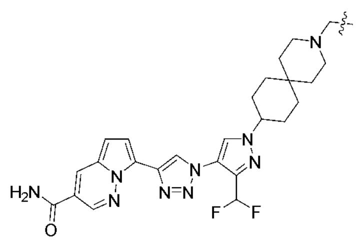
,
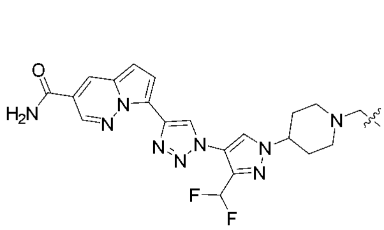
,
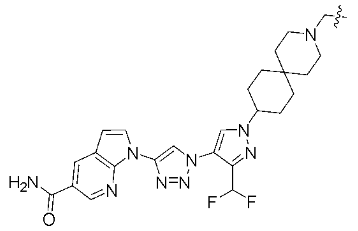

and

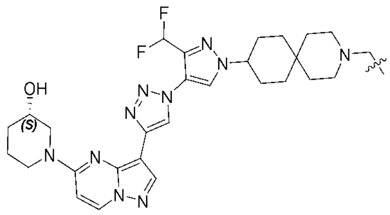.

In some preferred embodiments, the

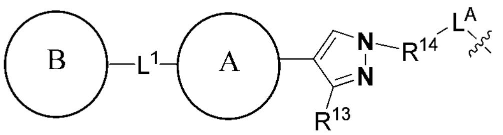

moiety is selected from:

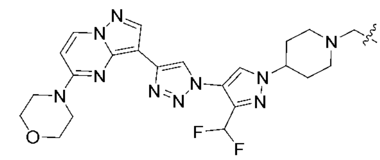, 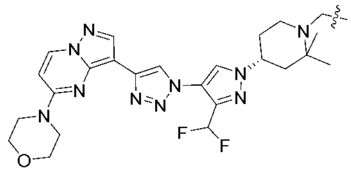,

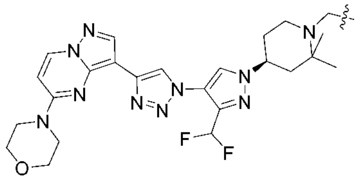, 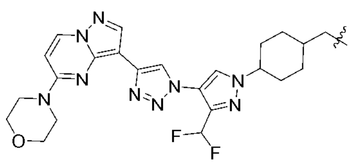,

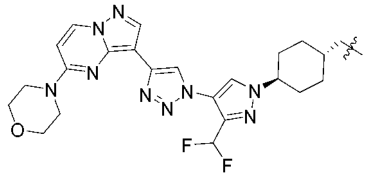, 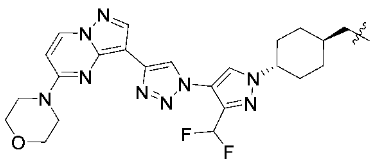,

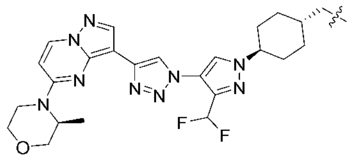, 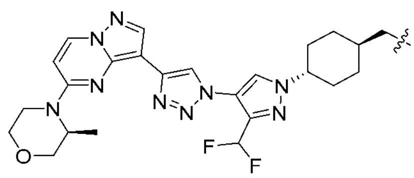,

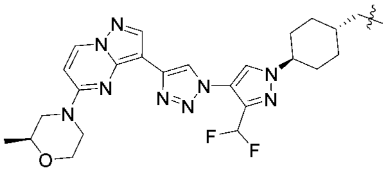, 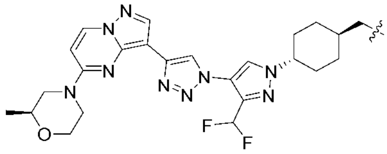,

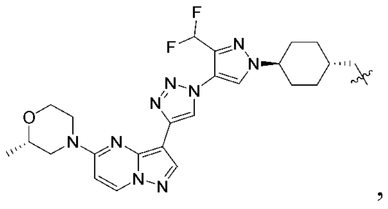,
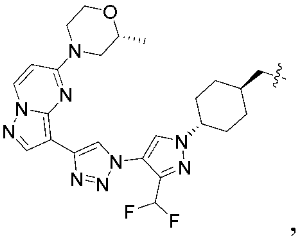,
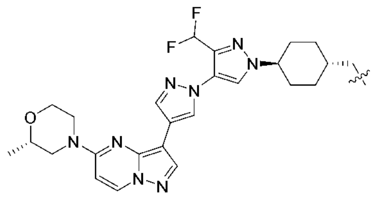,

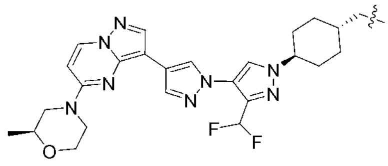,
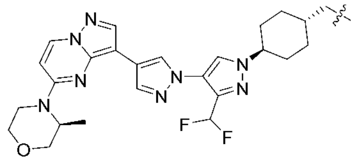,

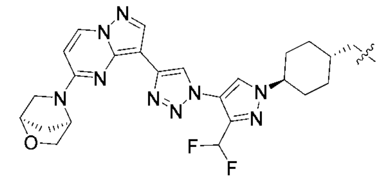,
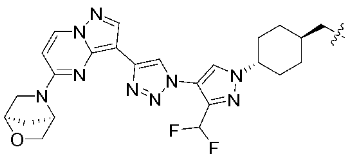,

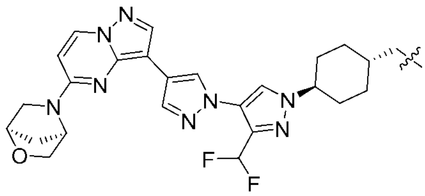,
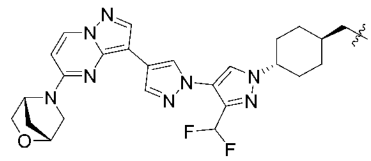,

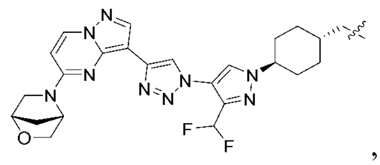,
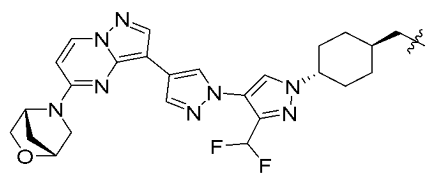,

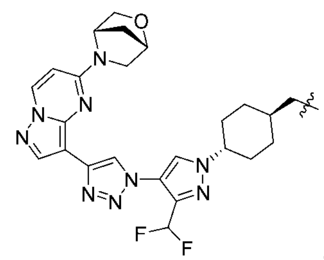,
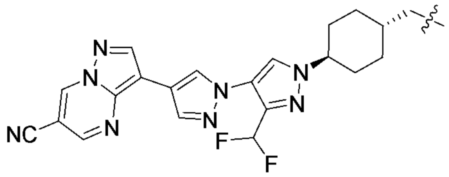,

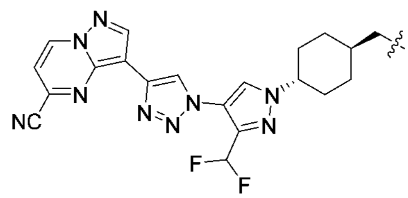,
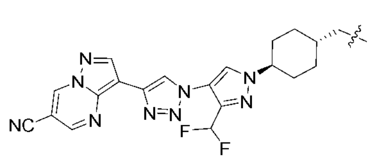,

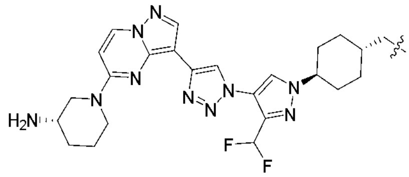
,
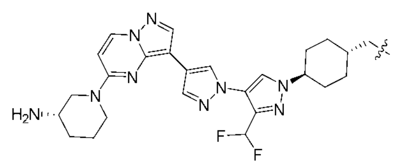
,
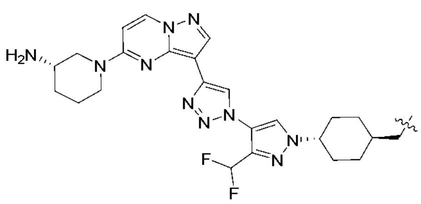
,
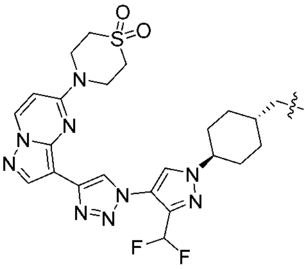
,
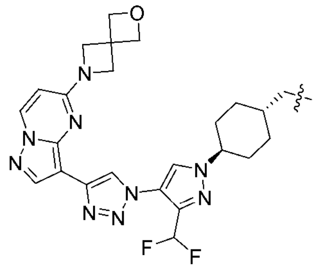
,
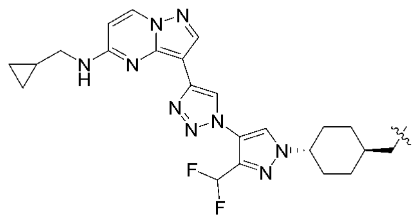
,
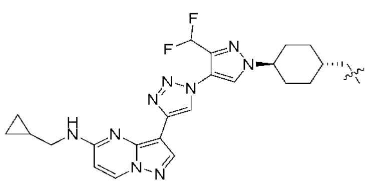
,
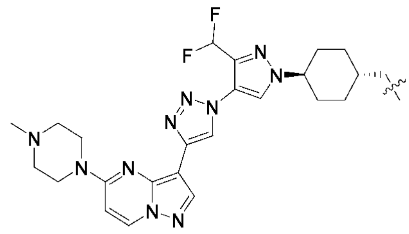
,
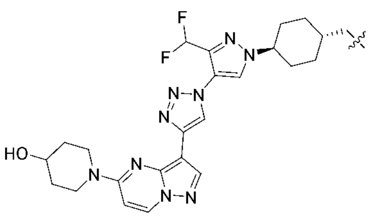
,
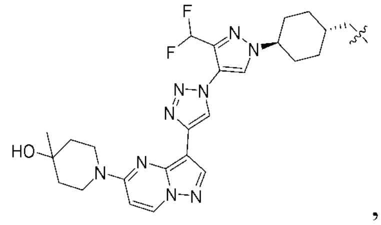
,
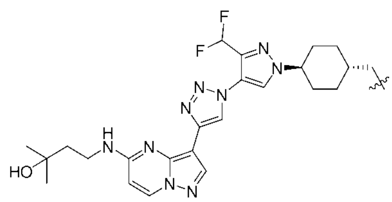
,
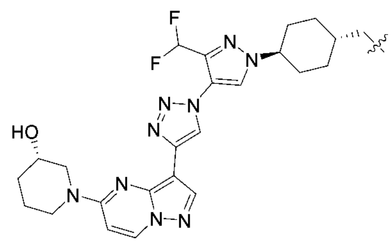
,
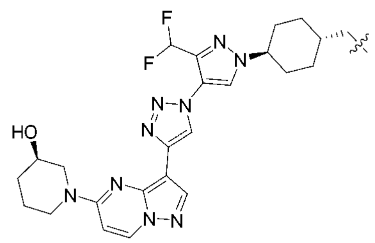
,

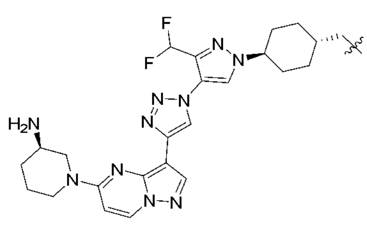,
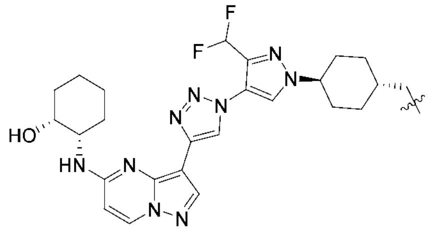,

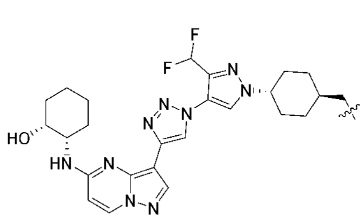,
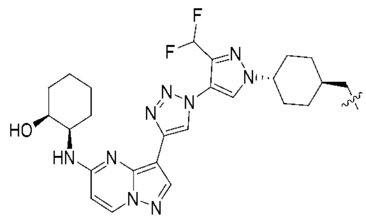,

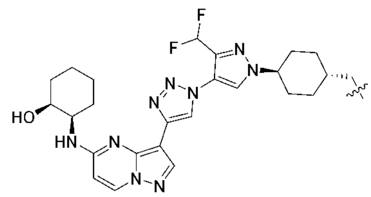,
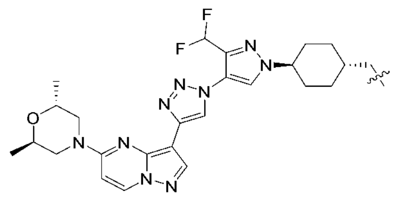,

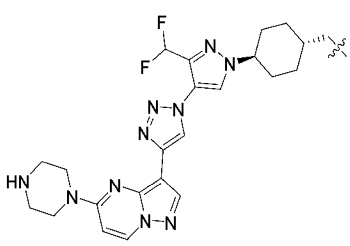,
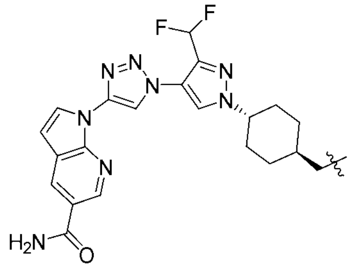,
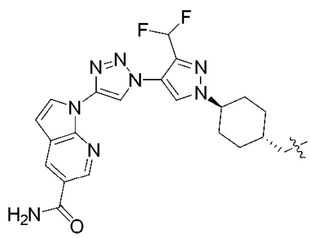,

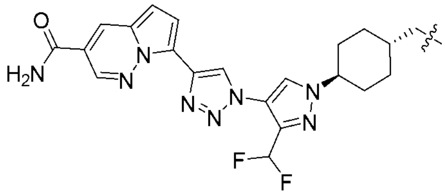,
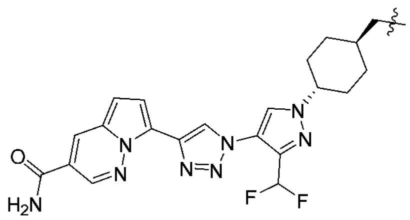,

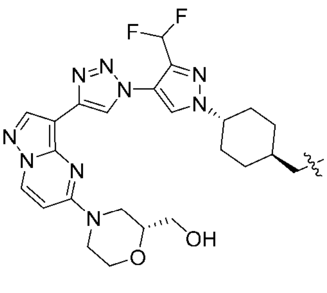,
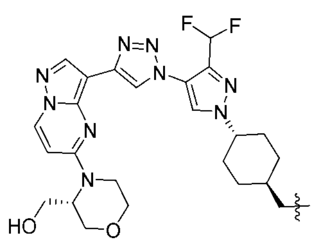,
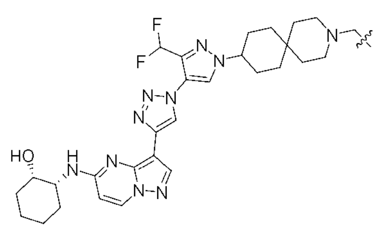,

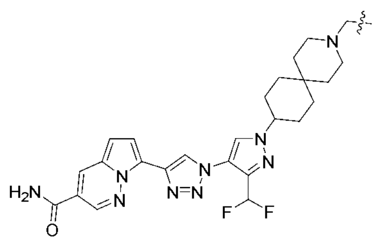

,

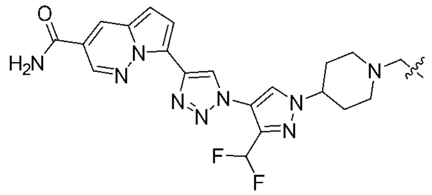

and

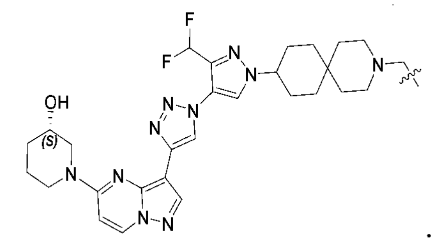

.

In some preferred embodiments, the moiety is selected from:

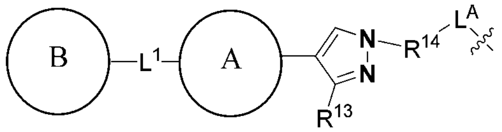

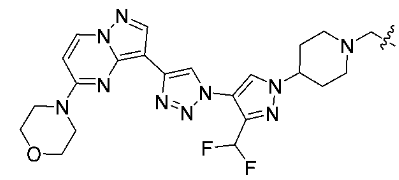

,

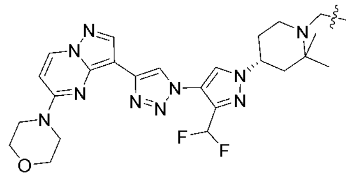

,

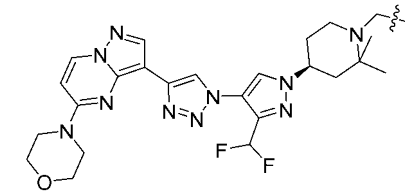

,

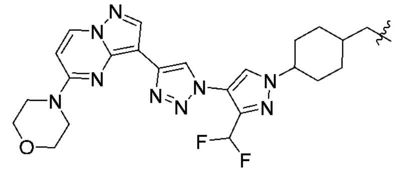

,

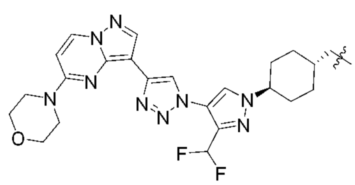

,

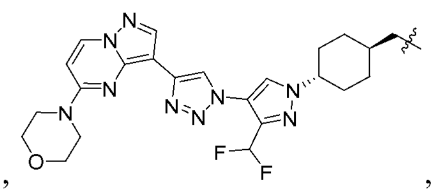

,

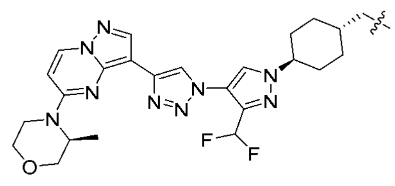

,

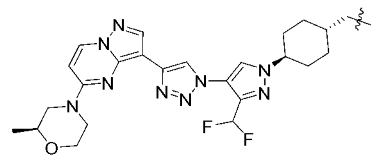

,

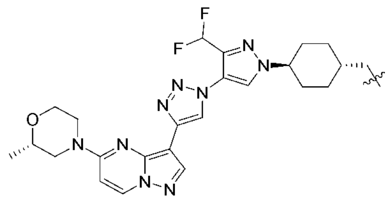, 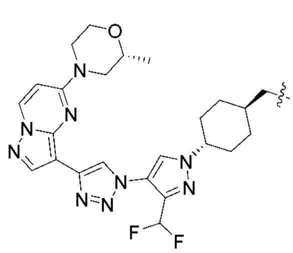, 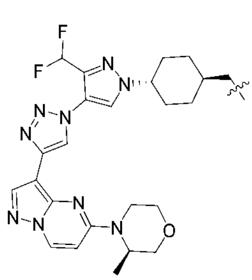,

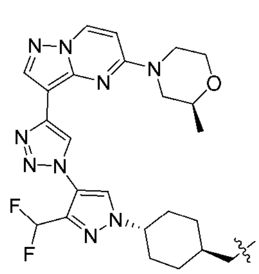, 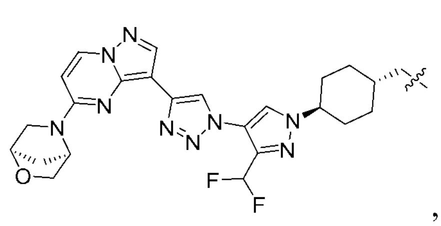, 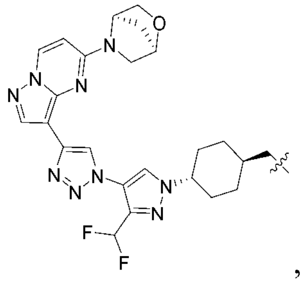,

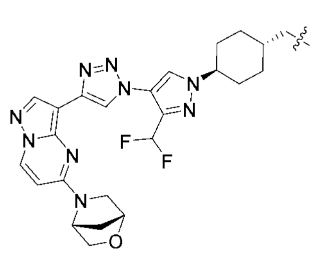, 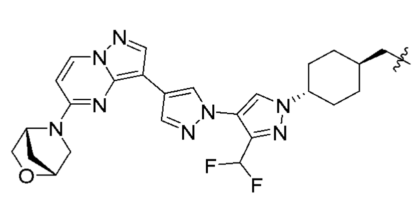, 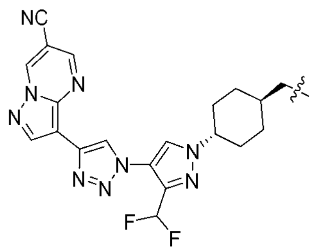,

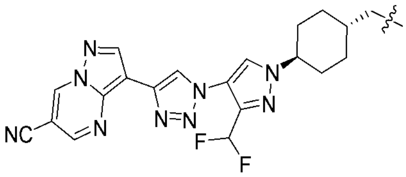, 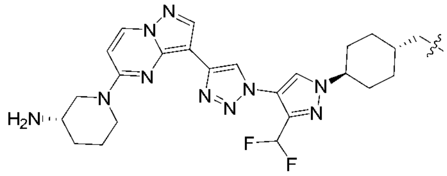,

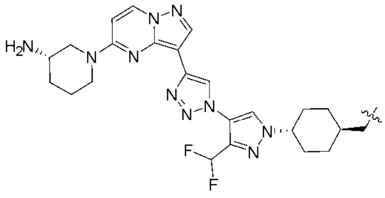, 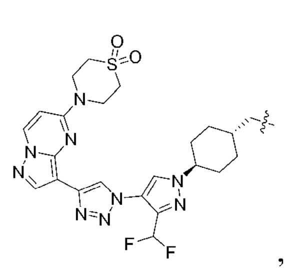, 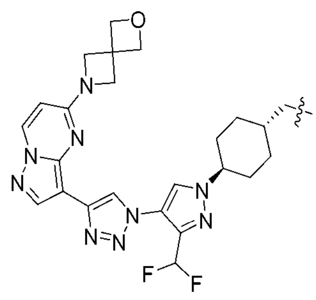,

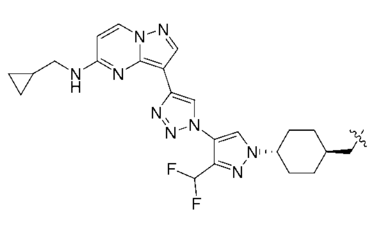, 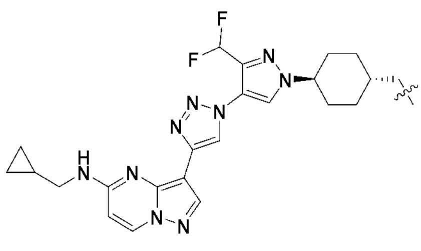,

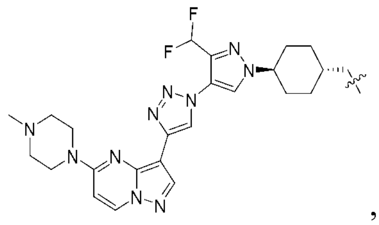 , 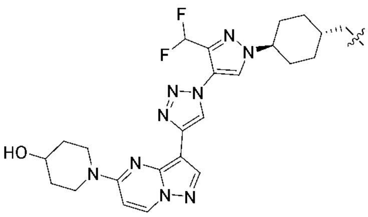 ,

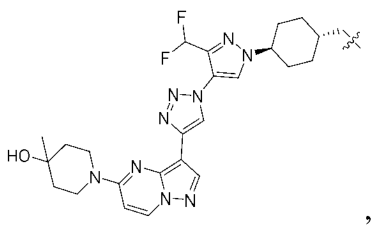 , 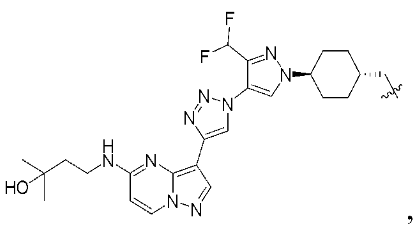 ,

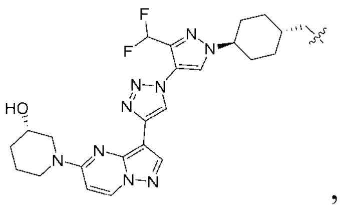 , 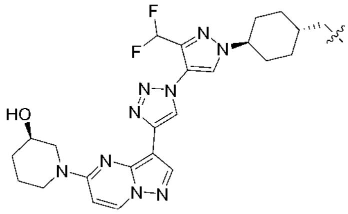 , 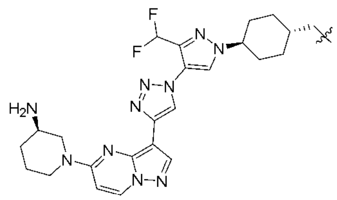 ,

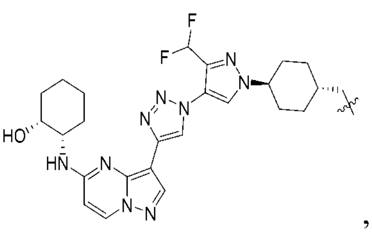 , 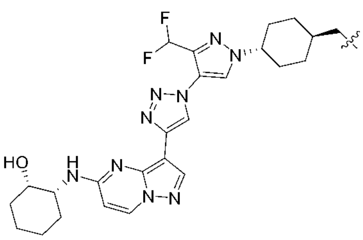 ,

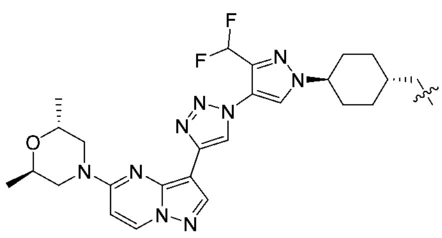 , 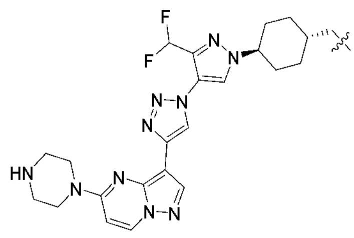 ,

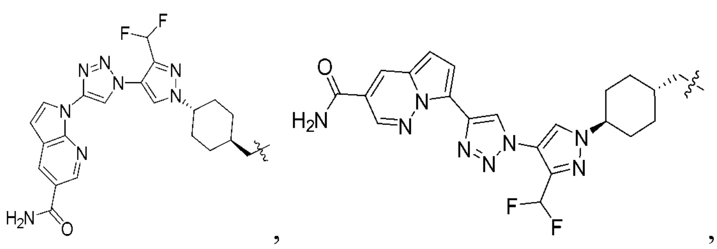 , ,

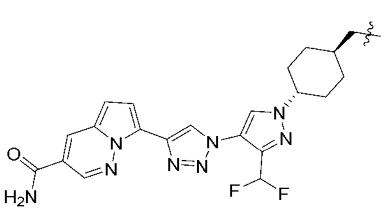,
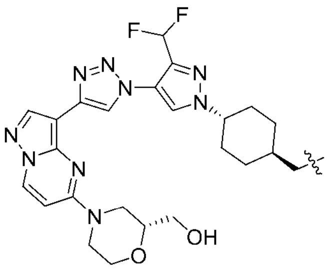,
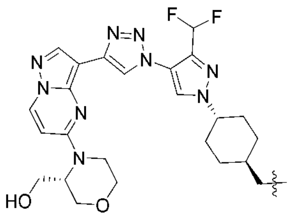,

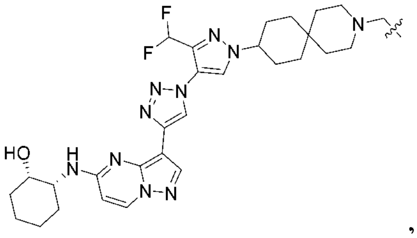,
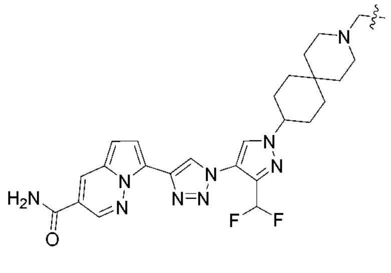

and

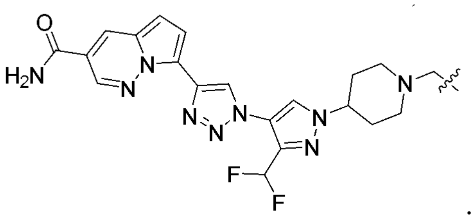.

II) IRAK4 ligand having the structure of formula (2)

In some embodiments, the present disclosure provides a compound of formula (A) according to the present disclosure, wherein the

IRAK4 ligand

moiety has the structure of formula (2).

In some embodiments, $R^{3'}$ is selected from: H, $C_{1-4}$ alkyl, $C_{1-4}$ haloalkyl, $C_{2-4}$ alkenyl, $C_{2-4}$ haloalkenyl, -$C_{1-4}$ alkylene-O-$C_{1-4}$ alkyl, -$C_{1-4}$ alkylene-OH, -$C_{1-4}$ alkylene-SH, -$C_{1-4}$ alkylene-CN and -$C_{1-4}$ alkylene-$NR^{3a}R^{3b}$. Preferably, $R^{3'}$ is selected from H and $C_{1-4}$ alkyl, more preferably H and methyl, and further preferably H.

In some embodiments, the structure of formula (2) is represented by formula (2-1):

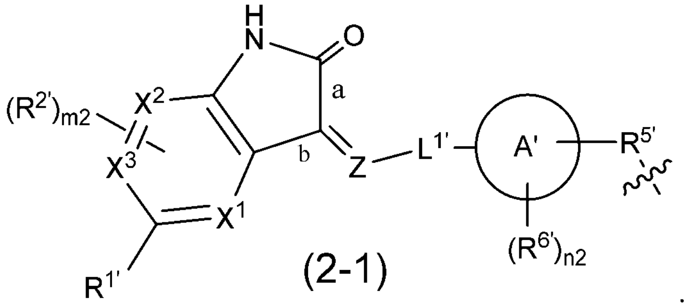
(2-1)
.

In some embodiments, n2 is 0 or 1.

In some embodiments, $R^{5'}$ is selected from: $C_{3-10}$ cyclohydrocarbylene and 3-to 10-membered heterocyclylene, wherein the $C_{3-10}$ cyclohydrocarbylene and the 3-to 10-membered heterocyclylene are each optionally substituted with 1

or more substituents independently selected from the following groups: halogen, OH, SH, - $NR^{5a}R^{5b}$, CN, $C_{1-4}$ alkyl, $C_{1-4}$ haloalkyl, $C_{2-4}$ alkenyl, $C_{2-4}$ haloalkenyl, -O-$C_{1-4}$ alkyl, -O-$C_{1-4}$ haloalkyl, -S-$C_{1-4}$ alkyl, -$C_{1-4}$ alkylene-O-$C_{1-4}$ alkyl, -$OC_{1-4}$ alkylene-$OC_{1-4}$ alkyl, -$C_{1-4}$ alkylene-OH, -$C_{1-4}$ alkylene-SH, -$C_{1-4}$ alkylene-CN and -$C_{1-4}$ alkylene-$NR^{5a}R^{5b}$.

Preferably, $R^{5'}$ is selected from: $C_{3-6}$ cycloalkylene and 5- to 10-membered heterocycloalkylene, wherein the $C_{3-6}$ cycloalkylene and the 5- to 10-membered heterocycloalkylene are each optionally substituted with 1 or more substituents independently selected from the following groups: halogen, OH, SH, -$NR^{5a}R^{5b}$, CN, $C_{1-4}$ alkyl, $C_{1-4}$ haloalkyl, $C_{2-4}$ alkenyl, $C_{2-4}$ haloalkenyl, -O-$C_{1-4}$ alkyl, -O-$C_{1-4}$ haloalkyl, -S-$C_{1-4}$ alkyl, -$C_{1-4}$ alkylene-O-$C_{1-4}$ alkyl, -$OC_{1-4}$ alkylene-$OC_{1-4}$ alkyl, - $C_{1-4}$ alkylene-OH, -$C_{1-4}$ alkylene-SH, -$C_{1-4}$ alkylene-CN and -$C_{1-4}$ alkylene-$NR^{5a}R^{5b}$.

More preferably, $R^{5'}$ is selected from: $C_{3-6}$ cycloalkylene and 5- to 10-membered heterocycloalkylene, wherein the $C_{3-6}$ cycloalkylene and the 5- to 10-membered heterocycloalkylene are each optionally substituted with 1 or more substituents independently selected from the following groups: $C_{1-4}$ alkyl, $C_{1-4}$ haloalkyl, -$C_{1-4}$ alkylene-O-$C_{1-4}$ alkyl, -$OC_{1-4}$ alkylene-$OC_{1-4}$ alkyl, -$C_{1-4}$ alkylene-OH, -$C_{1-4}$ alkylene-SH, -$C_{1-4}$ alkylene-CN and -$C_{1-4}$ alkylene-$NR^{5a}R^{5b}$.

In some further more preferred embodiments, $R^{5'}$ is selected from:

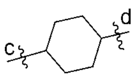, 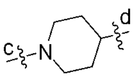, 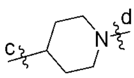, 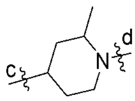 and 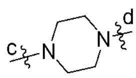,

preferably

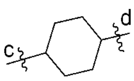, 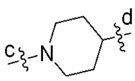 and 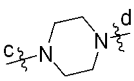,

more preferably

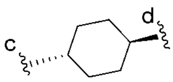, 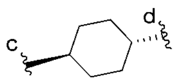, 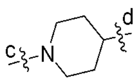 and 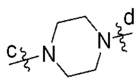,

wherein the bond labeled with "c" is connected to the $L^A$ and the bond labeled with "d" is connected to the ring A'; or preferably, the bond labeled with "c" is connected to the ring A' and the bond labeled with "d" is connected to the $L^A$.

In some embodiments, the ring A' is selected from 5- to 10-membered monocyclic or fused bicyclic heteroaryl.

Preferably, the ring A' is selected from 5- to 6-membered monocyclic heteroaryl and 9- to 10-membered fused bicyclic heteroaryl.

More preferably, the ring A' is selected from: 5- to 6-membered monocyclic heteroaryl and benzo 5- to 6-membered monocyclic heteroaryl, wherein preferably, the ring A' is connected to the $L^{1'}$ via the 5- to 6-membered monocyclic heteroaryl.

More preferably, the ring A' is selected from: 5-membered monocyclic heteroaryl and benzo 5-membered monocyclic heteroaryl, wherein preferably, the ring A' is connected to the $L^1$ via the 5-membered monocyclic heteroaryl.

In some preferred embodiments, any of the above 5-membered monocyclic heteroaryl is selected from furanyl, thienyl, oxazolyl, isoxazolyl, thiazolyl, isothiazolyl, oxadiazolyl, thiadiazolyl, pyrrolyl, pyrazolyl, imidazolyl, triazolyl, and tetrazolyl.

In some more preferred embodiments, the ring A' is selected from pyrazolyl, benzofuranyl, benzothienyl and indolyl. Preferably, the benzofuranyl is connected to the $L^1$ via furan ring, the benzothienyl is connected to the $L^1$ via thiophene ring, and the indolyl is connected to the $L^1$ via pyrrole ring.

In some more preferred embodiments, the ring A' is selected from

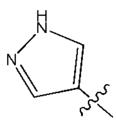,

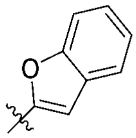, 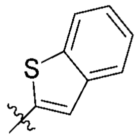 and 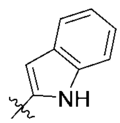.

In some more preferred embodiments, the

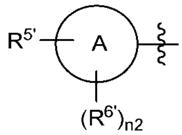

moiety is selected from

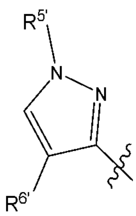, 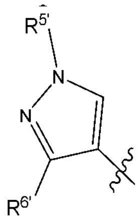, 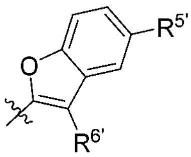, 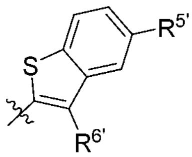 and 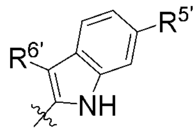,

more preferably

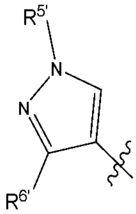, 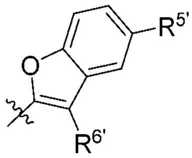 and 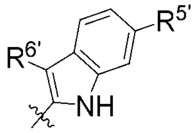.

In some more preferred embodiments, the

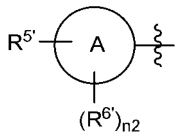

moiety is selected from

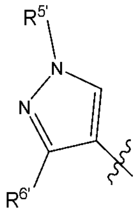 and 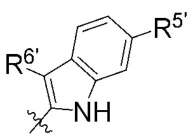.

In some embodiments, $X^1$, $X^2$ and $X^3$ are each CH; or $X^1$ is N, and $X^2$ and $X^3$ are each CH; or $X^2$ is N, and $X^1$ and $X^3$ are each CH; or $X^3$ is N, and $X^1$ and $X^2$ are each CH; or $X^1$ and $X^2$ are each N, and $X^3$ is CH; or $X^1$ and $X^3$ are each N, and $X^2$ is CH; or $X^2$ and $X^3$ are each N, and $X^1$ is CH; or $X^1$, $X^2$ and $X^3$ are each N.

In some preferred embodiments, the structure of formula (2) is represented by formula (2-2) to (2-4):

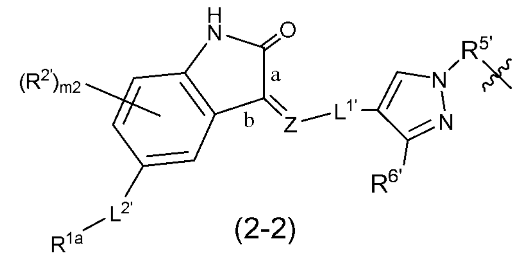

(2-2)

,

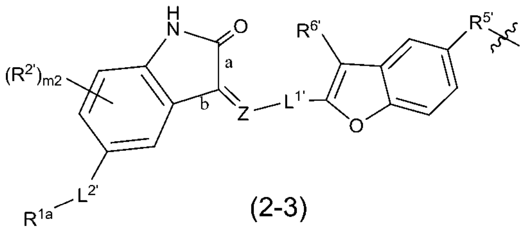

(2-3) ,

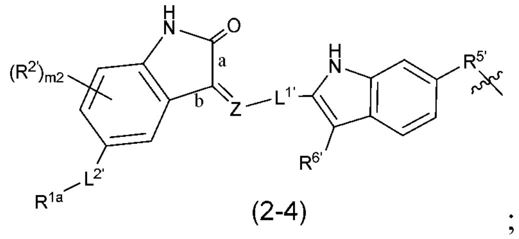

(2-4) ;

more preferably, represented by formula (2-5) to (2-8):

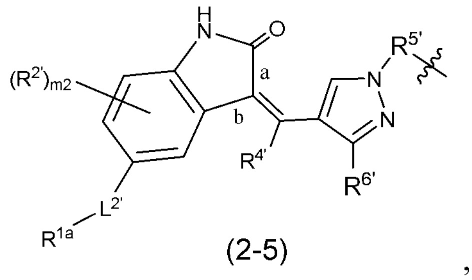

(2-5) ,

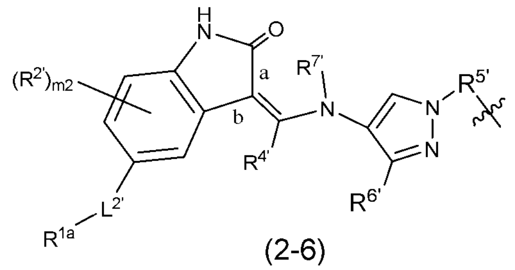

(2-6) ,

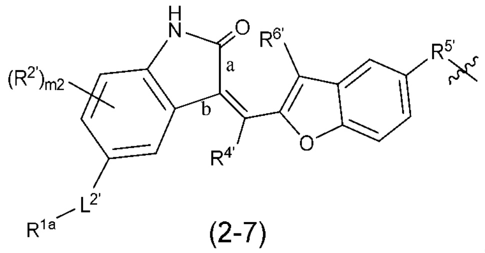

(2-7) ,

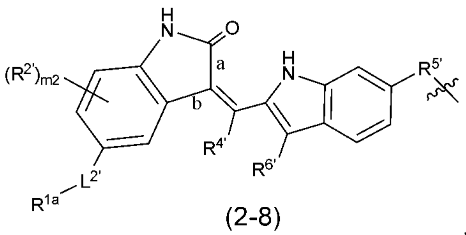

(2-8) .

In some embodiments, $R^{4'}$ is selected from: H, D, halogen, OH, SH, $-NR^{4a}R^{4b}$, CN, $C_{1-4}$ alkyl, $C_{1-4}$ haloalkyl, $C_{2-4}$ alkenyl, $C_{2-4}$ haloalkenyl, $-C_{1-4}$ alkylene-O-$C_{1-4}$ alkyl, $-C_{1-4}$ alkylene-OH, $-C_{1-4}$ alkylene-SH, $-C_{1-4}$ alkylene-CN and $-C_{1-4}$ alkylene-$NR^{4a}R^{4b}$.

Preferably, $R^{4'}$ is selected from H, D, halogen, OH, $-NR^{4a}R^{4b}$, CN, $C_{1-4}$ alkyl and $C_{1-4}$ haloalkyl.

More preferably, $R^{4'}$ is selected from H, D, F, Cl, OH, $-NH_2$, CN, methyl, ethyl, $-CHF_2$ and $-CF_3$.

More preferably, $R^{4'}$ is selected from H, D, F, Cl, methyl and ethyl, further more preferably H.

In some embodiments, $R^{6'}$ is selected from: H, halogen, OH, SH, $-NR^{6a}R^{6b}$, CN, $C_{1-4}$ alkyl, $C_{1-4}$ haloalkyl, $C_{2-4}$ alkenyl, $C_{2-4}$ haloalkenyl, -O-$C_{1-4}$ alkyl, -O-$C_{1-4}$ haloalkyl, -S-$C_{1-4}$ alkyl, $-C_{1-4}$ alkylene-O-$C_{1-4}$ alkyl, $-OC_{1-4}$ alkylene-$OC_{1-4}$ alkyl, - $C_{1-4}$ alkylene-OH, $-C_{1-4}$ alkylene-SH, $-C_{1-4}$ alkylene-CN and $-C_{1-4}$ alkylene-$NR^{6a}R^{6b}$.

Preferably, $R^{6'}$ is selected from: H, halogen, OH, SH, $-NR^{6a}R^{6b}$, CN, $C_{1-4}$ alkyl, $C_{1-4}$ haloalkyl, $-C_{1-4}$ alkylene-OH, $-C_{1-4}$ alkylene-SH, $-C_{1-4}$ alkylene-CN and $-C_{1-4}$ alkylene-$NR^{6a}R^{6b}$,

More preferably, $R^{6'}$ is selected from: H, F, Cl, OH, $-NH_2$, $-NHCH_3$, $-N(CH_3)_2$, CN, methyl, ethyl, $-CHF_2$ and $-CF_3$.

More preferably, $R^{6'}$ is selected from: H, $-NH_2$, $-NHCH_3$, $-N(CH_3)_2$, methyl, ethyl, $-CHF_2$ and $-CF_3$, further more preferably H, methyl, ethyl and $-CHF_2$.

In some embodiments, $R^{7'}$ is selected from: H, $C_{1-4}$ alkyl, $C_{1-4}$ haloalkyl, $C_{2-4}$ alkenyl, $C_{2-4}$ haloalkenyl, $-C_{1-4}$ alkylene-O-$C_{1-4}$ alkyl, $-C_{1-4}$ alkylene-OH, $-C_{1-4}$ alkylene-SH, $-C_{1-4}$ alkylene-CN and $-C_{1-4}$ alkylene-$NR^{3a}R^{3b}$.

Preferably, $R^{7'}$ is selected from H and $C_{1-4}$ alkyl, more preferably H and methyl, further more preferably H.

In some embodiments, the structure of formula (2) is represented by formula (2-9) to (2-12):

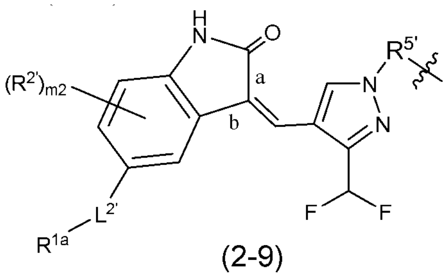

(2-9) ,

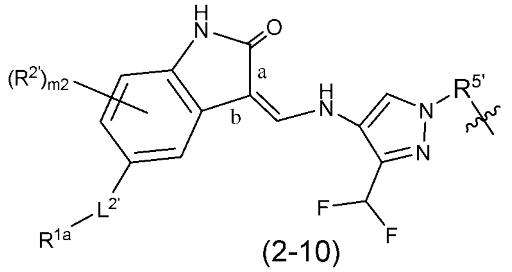

(2-10) ,

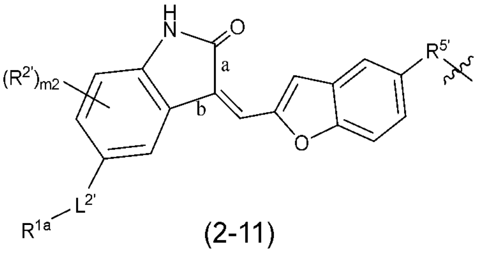

(2-11) ,

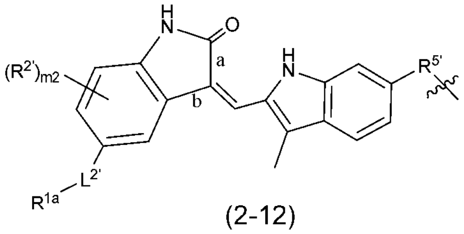

(2-12) .

In some embodiments, $R^{1a}$ is $C_{6-10}$ aryl and 5- to 10-membered heteroaryl, wherein the $C_{6-10}$ aryl and the 5- to 10-membered heteroaryl are each optionally substituted with 1 or more substituents independently selected from the following groups: halogen, OH, SH, $-NR^{1e}R^{1f}$, CN, $C_{1-4}$ alkyl, $C_{1-4}$ haloalkyl, $C_{2-4}$ alkenyl, $C_{2-4}$ haloalkenyl, $-O-C_{1-4}$ alkyl, $-O-C_{1-4}$ haloalkyl, $-S-C_{1-4}$ alkyl, $-C_{1-4}$ alkylene-$O-C_{1-4}$ alkyl, $-OC_{1-4}$ alkylene-$OC_{1-4}$ alkyl, $-C_{1-4}$ alkylene-OH, $-C_{1-4}$ alkylene-SH, $-C_{1-4}$ alkylene-CN and $-C_{1-4}$ alkylene-$NR^{1e}R^{1f}$;

preferably, $R^{1a}$ is phenyl, wherein the phenyl is optionally substituted with 1 or more substituents independently selected from the following groups: halogen, OH, $-NR^{1e}R^{1f}$, CN, $C_{1-4}$ alkyl, $C_{1-4}$ haloalkyl, $-O-C_{1-4}$ alkyl and $-O-C_{1-4}$ haloalkyl, preferably F, Cl, OH, $-NH_2$, CN, methyl and ethyl.

More preferably, $R^{1a}$ is selected from

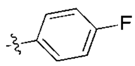 and 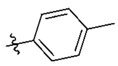 .

In some embodiments, $L^{2'}$ is $-S(O)_2NR^{1b}$-*, $-C(O)-NR^{1b}$-* or $-NR^{1b}-C(O)$-*, preferably $-S(O)_2NR^{1b}$-*, wherein the bond indicated with * is connected to $R^{1a}$.

In some embodiments, each $R^{2'}$ is independently selected from: H, halogen, OH, SH, $-NR^{2a}R^{2b}$, CN, $C_{1-4}$ alkyl, $C_{1-4}$ haloalkyl, $C_{2-4}$ alkenyl, $C_{2-4}$ haloalkenyl, $-O-C_{1-4}$ alkyl, $-O-C_{1-4}$ haloalkyl, $-S-C_{1-4}$ alkyl, $-C_{1-4}$ alkylene-$O-C_{1-4}$ alkyl, $-OC_{1-4}$ alkylene-$OC_{1-4}$ alkyl, $-C_{1-4}$ alkylene-OH, $-C_{1-4}$ alkylene-SH, $-C_{1-4}$ alkylene-CN and $-C_{1-4}$ alkylene-$NR^{2a}R^{2b}$, Preferably, each $R^{2'}$ is independently H.

In some embodiments, $R^{1b}$, $R^{1e}$, $R^{1f}$, $R^{2a}$, $R^{2b}$, $R^{3a}$, $R^{3b}$, $R^{4a}$, $R^{4b}$, $R^{5a}$, $R^{5b}$, $R^{6a}$, and $R^{6b}$, at each occurrence, are independently selected from H and $C_{1-4}$ alkyl, preferably H, methyl and ethyl.

In some preferred embodiments,

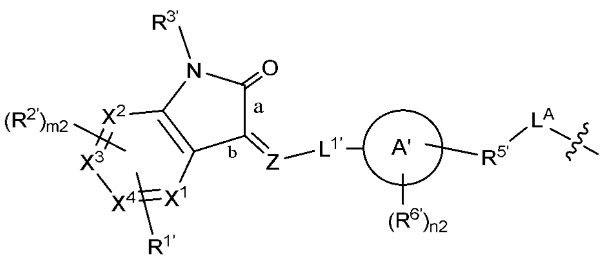

moiety is selected from:

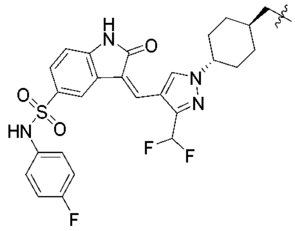, 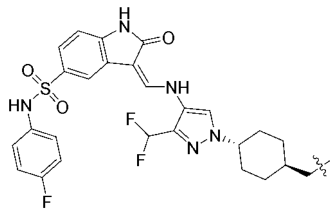, 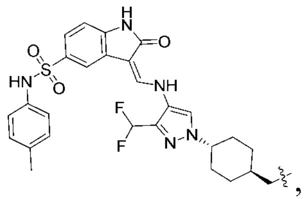,

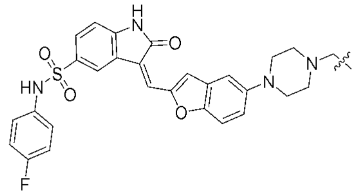, 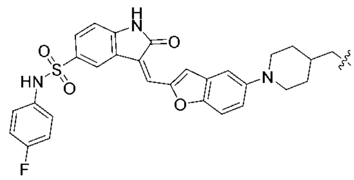

and

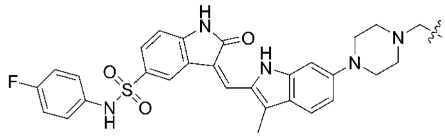.

Linking group $L^B$

In some embodiments, the present disclosure provides a compound of formula (A) according to the present disclosure, which has an $L^B$ group as defined above.

In some embodiments, the CyL1 group and the CyL2 group, at each occurrence, are each independently selected from 4- to 11-membered heterocycloalkylene, preferably 4- to 7-membered monocyclic heterocycloalkylene, 6- to 10-membered fused bicyclic heterocycloalkylene, 6-9-membered bridged heterocycloalkylene and 5- to 12-membered spiro heterocycloalkylene, more preferably 4- to 6-membered monocyclic heterocycloalkylene, 8- to 10-membered fused bicyclic heterocycloalkylene, 6- to 8-membered bridged heterocycloalkylene and 7- to 11-membered spiro heterocycloalkylene. In some preferred embodiments, any of the above heterocycloalkylene has 1, 2 or more nitrogen heteroatoms and 0, 1 or 2 heteroatoms selected from O and S.

In some embodiments, the CyL3 group, at each occurrence, is independently selected from $C_{4-11}$ cycloalkylene, 4- to 11-membered heterocycloalkylene, preferably $C_{4-6}$ monocyclic cycloalkylene, $C_{6-10}$ fused bicyclic cycloalkylene, $C_{6-9}$ bridged cycloalkylene, $C_{5-12}$ spiro cycloalkylene, 4- to 7-membered monocyclic heterocycloalkylene, 6- to 10-membered fused bicyclic heterocycloalkylene, 6- to 9-membered bridged heterocycloalkylene and 5- to 12-membered spiro heterocycloalkylene, more preferably $C_{5-6}$ monocyclic cycloalkylene, $C_{8-10}$ fused bicyclic cycloalkylene, $C_{6-8}$ bridged cycloalkylene, $C_{7-11}$ spiro cycloalkylene, 4- to 6-membered monocyclic heterocycloalkylene, 8- to 10-membered fused bicyclic heterocycloalkylene, 6- to 8-membered bridged heterocycloalkylene and 7- to 11-membered spiro heterocycloalkylene, wherein any of the above heterocycloalkylene preferably has 1, 2 or more nitrogen heteroatoms and 0, 1 or 2 heteroatoms selected from O and S.

In some embodiments, the CyL3 group, at each occurrence, is independently selected from $C_{4-11}$ cycloalkylene, preferably $C_{4-6}$ monocyclic cycloalkylene, $C_{6-10}$ fused bicyclic cycloalkylene, $C_{6-9}$ bridged cycloalkylene and $C_{5-12}$ spiro cycloalkylene, more preferably $C_{5-6}$ monocyclic cycloalkylene, $C_{8-10}$ fused bicyclic cycloalkylene, $C_{6-8}$ bridged cycloalkylene and $C_{7-11}$ spiro cycloalkylene.

In some embodiments, the CyL3 group, at each occurrence, is independently selected from $C_{5-6}$ monocyclic cycloalkylene and $C_{9-11}$ spiro cycloalkylene.

In some embodiments, the CyL4 group, at each occurrence, is independently selected from 5- to 10-membered heteroarylene, preferably 5- to 6-membered heteroarylene, more preferably 5- to 6-membered nitrogen-containing heteroarylene.

In some embodiments, La, at each occurrence, is independently selected from $C_{1-4}$ alkylene, $C_{2-4}$ alkenylene, and $C_{2-4}$ alkynylene.

Preferably, La, at each occurrence, is independently selected from $-CH_2-$, $-(CH_2)_2-$, $-(CH_2)_3-$, $-(CH_2)_4-$, $-CH=CH-$, $-CH_2-CH=CH-$, $-CH=CH-CH_2-$, $-C\equiv C-$, $-CH_2-C\equiv C-$, $-C\equiv C-CH_2-$, $-C\equiv C-CH_2CH_2-$, $-CH_2CH_2-C\equiv C-$ and $-CH_2-C\equiv C-CH_2-$.

More preferably, La, at each occurrence, is independently selected from $-CH_2-$, $-(CH_2)_2-$, $-(CH_2)_3-$, -C≡C-, $-CH_2-C≡C-$, $-C≡C-CH_2-$, $-C≡C-CH_2CH_2-$ and - $CH_2CH_2-C≡C-$.

In some embodiments, Lb, at each occurrence, is independently selected from -O-straight $C_{1-3}$ alkylene, -straight $C_{1-3}$ alkylene-O-, -O-$C_{2-3}$ alkenylene, -$C_{2-3}$ alkenylene-O-, -O-$C_{2-3}$ alkynylene, -$C_{2-3}$ alkynylene-O-, -$NR^{8'}$-straight $C_{1-3}$ alkylene-, -straight $C_{1-3}$ alkylene-$NR^{8'}$-, -straight $C_{1-2}$ alkylene-$NR^{8'}$-straight $C_{1-2}$ alkylene-, -straight $C_{1-2}$ alkylene-C(O)-$NR^{8'}$-, -$NR^{8'}$-C(O)-straight $C_{1-2}$ alkylene-, - C(O)-straight $C_{1-3}$ alkylene-, -straight $C_{1-3}$ alkylene-C(O)-, -straight $C_{1-2}$ alkylene-$NR^{8'}$-C(O)- and -C(O)-$NR^{8'}$-straight $C_{1-2}$ alkylene-.

In some embodiments, Lb, at each occurrence, is independently selected from -O-straight $C_{1-3}$ alkylene, -straight $C_{1-3}$ alkylene-O-, -O-$C_{2-3}$ alkenylene, -$C_{2-3}$ alkenylene-O-, -O-$C_{2-3}$ alkynylene, -$C_{2-3}$ alkynylene-O-, -$NR^{8'}$-straight $C_{1-3}$ alkylene-, -straight $C_{1-3}$ alkylene-$NR^{8'}$-, -straight $C_{1-2}$ alkylene-$NR^{8'}$-straight $C_{1-2}$ alkylene-, -straight $C_{1-2}$ alkylene-C(O)-$NR^{8'}$-, -$NR^{8'}$-C(O)-straight $C_{1-2}$ alkylene-, - C(O)-straight $C_{1-3}$ alkylene-, and -straight $C_{1-3}$ alkylene-C(O)-, wherein $R^{8'}$, at each occurrence, is independently selected from H and $C_{1-4}$ alkyl.

In some embodiments, Lb, at each occurrence, is independently selected from -O-$C_{2-3}$ alkynylene, -$NR^{8'}$-straight $C_{1-3}$ alkylene-, -straight $C_{1-3}$ alkylene-$NR^{8'}$-, - straight $C_{1-2}$ alkylene-$NR^{8'}$-straight $C_{1-2}$ alkylene-, -straight $C_{1-2}$ alkylene-C(O)-$NR^{8'}$-, and -C(O)-straight $C_{1-3}$ alkylene-.

In some embodiments, Lc, at each occurrence, is independently selected from a bond or straight $C_{1-3}$ alkylene, preferably a bond, methylene or ethylene, more preferably a bond or methylene.

In some embodiments, $R^{L1}$ $R^{L2}$, and $R^{8'}$, at each occurrence, are each independently selected from H, methyl and ethyl, more preferably H and methyl.

In some preferred embodiments, $L^B$ is a group selected from the following (1) to (21):

(1)

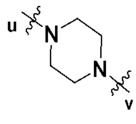,
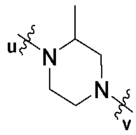

(preferably

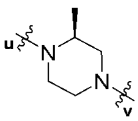

and

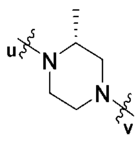),
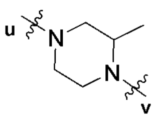

(preferably

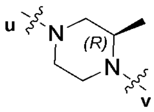 and 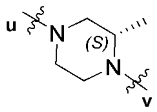

),

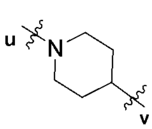, 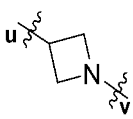, 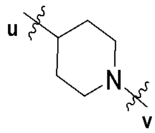,

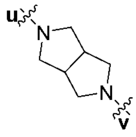, 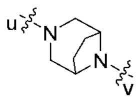, 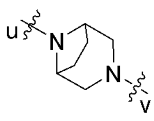, 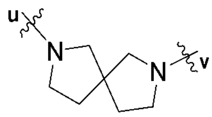, 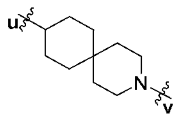, 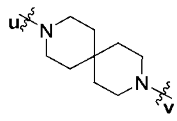,

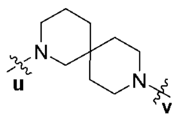, 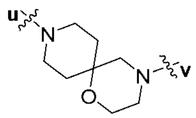, 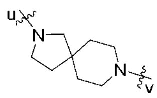, 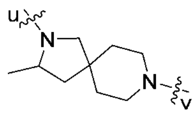

(preferably

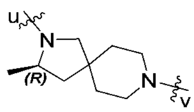 and 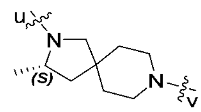), 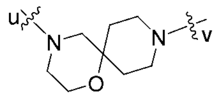, 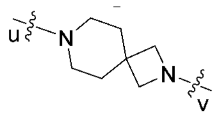,

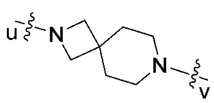, 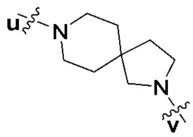 and 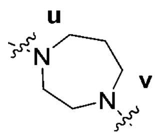,

(2)

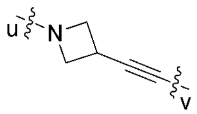, 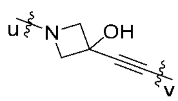, 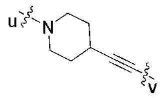, 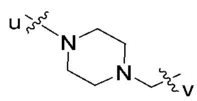,

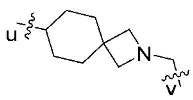, 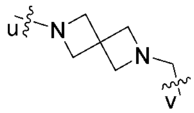, 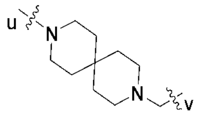, 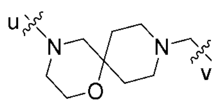, 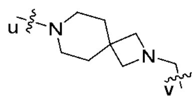

and

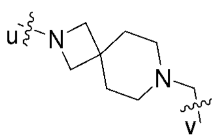,

(3)

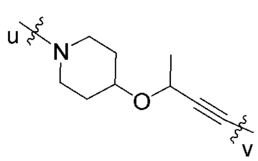

(preferably

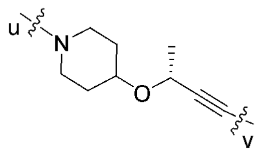

and

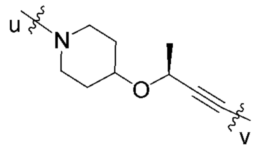 ), 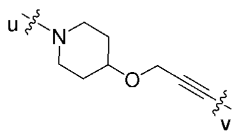 , 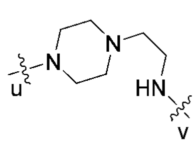 , 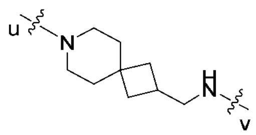 ,

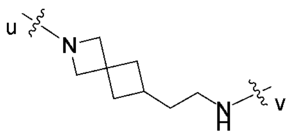 , 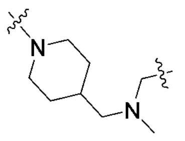 , 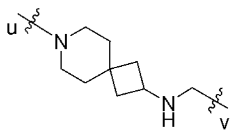 , 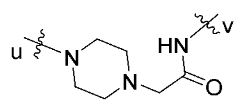 ,

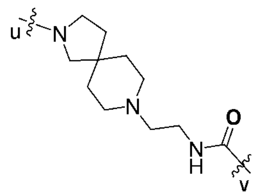 and 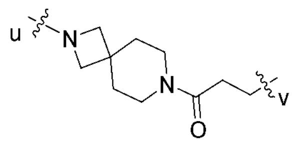 ,

(4)

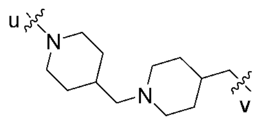 ,

*(5)*

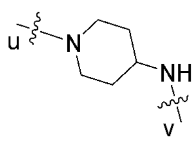 ,

(6)

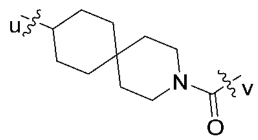 ,

(7)

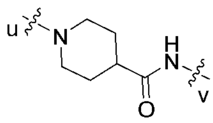 and 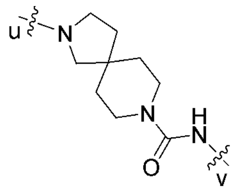,

(8)

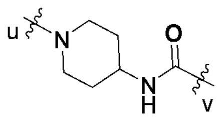,

(9)

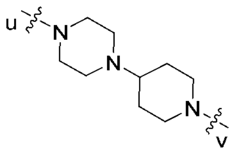, 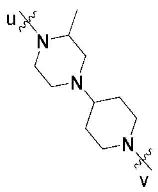

(preferably

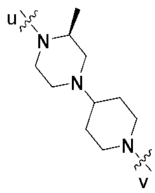 and 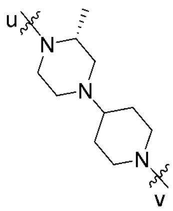

),

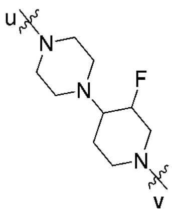

(preferably

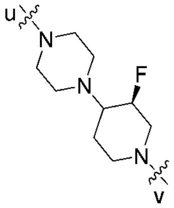, 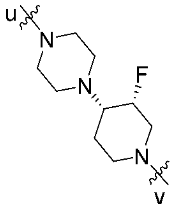, 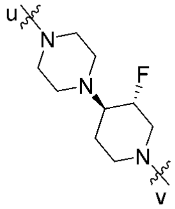,

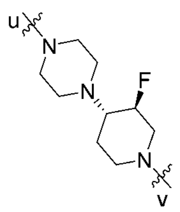 and 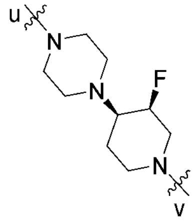

),

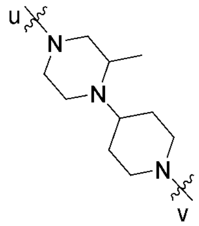

(preferably

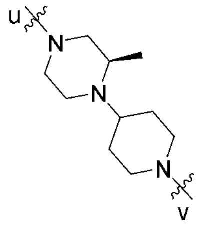

and

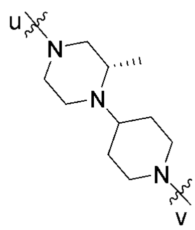 ), 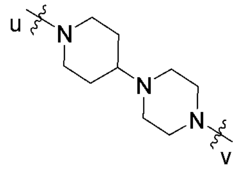 , 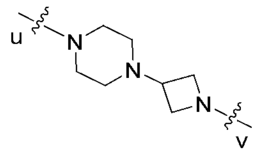 , 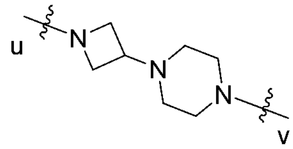 ,

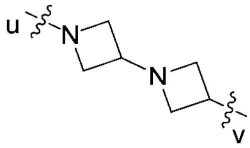 , and 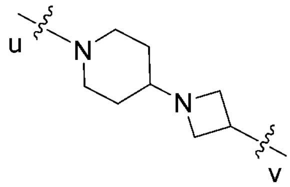 ;

(10)

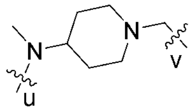 and 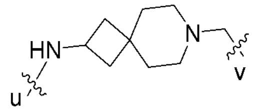 ,

(11)

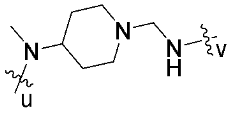 and 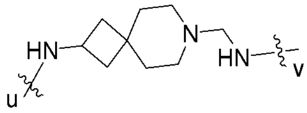,

(12)

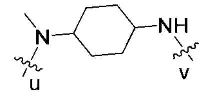

(preferably

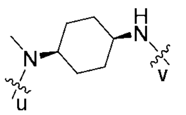, 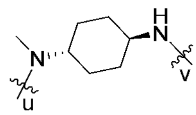,

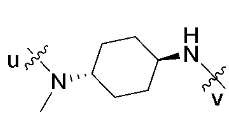 and 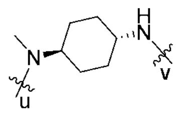

) and

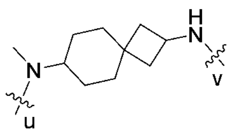,

(13)

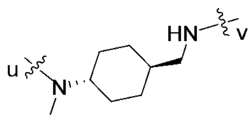 and 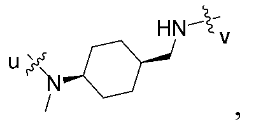,

(14)

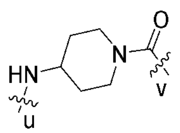,

(15)

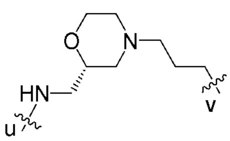, 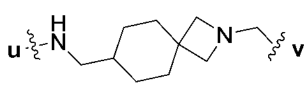 and 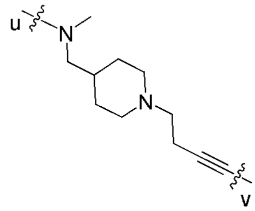,

(16)

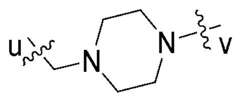

,

(17)

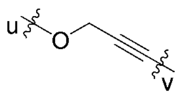

,

(18)

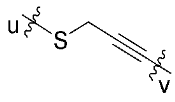

,

(19)

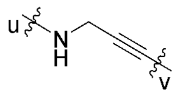

,

(20)

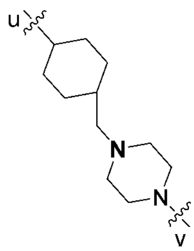

preferably (

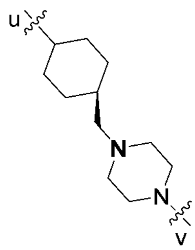

and

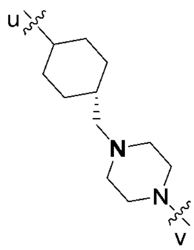

) and

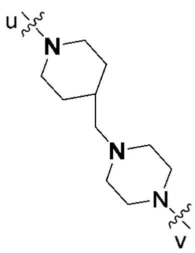

and

(21)

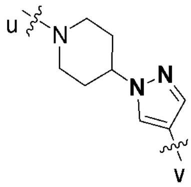 and 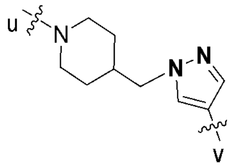;

wherein preferably, in any group of the above (1) to (21), the bond labeled with "u" is connected to the LA, and the bond labeled with "v" is connected to the

LBM

moiety.

In some preferred embodiments, $L^B$ is a group selected from the following (1) to (21):

(1)

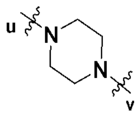, 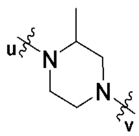

(preferably

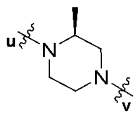 and 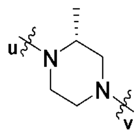

),

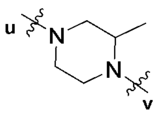

(preferably

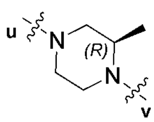 and 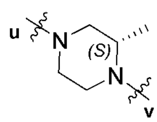

),

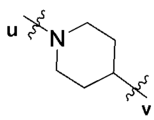, 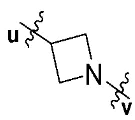, 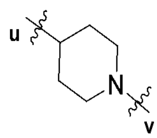,

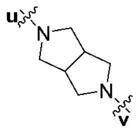, 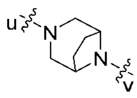, 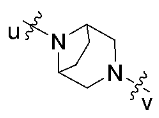, 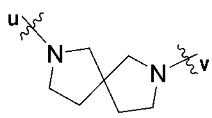, 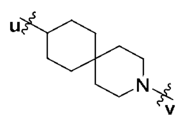, 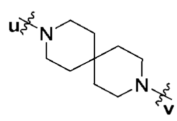,

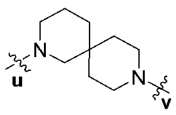, 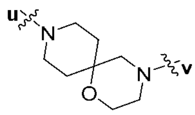, 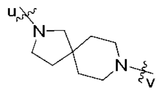, 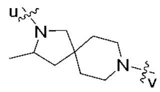

(preferably

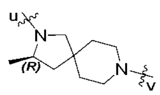

and

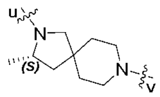

),

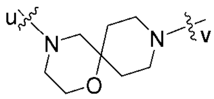, 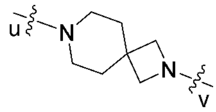, 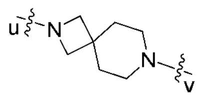, 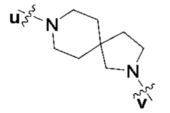

and

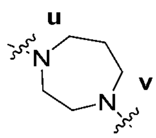,

(2)

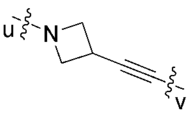, 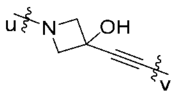, 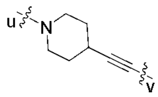, 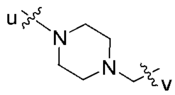, 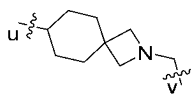,

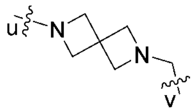, 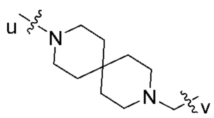, 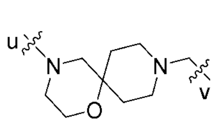, 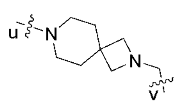

and

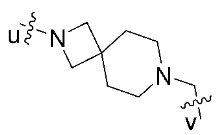,

(3)

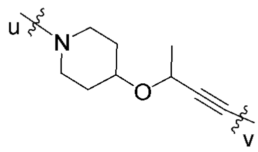

(preferably

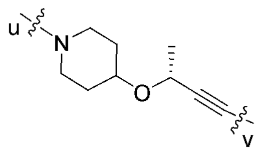

and

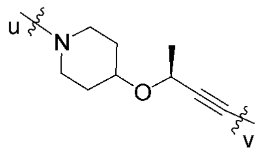

),

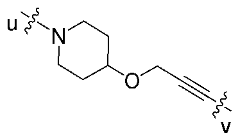 , 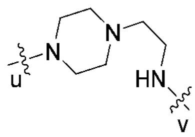 , 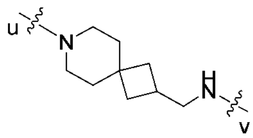 ,

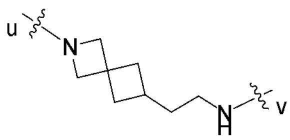 , 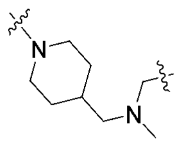 , 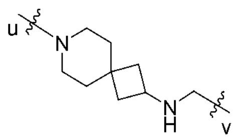 , 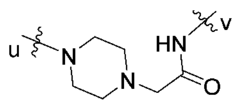

and

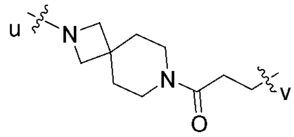 ,

(4)

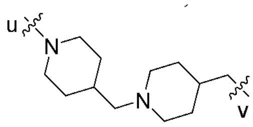 ,

(5)

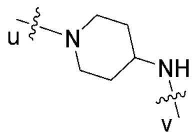,

(6)

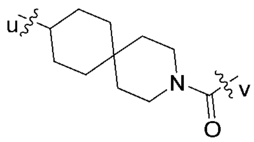,

(7)

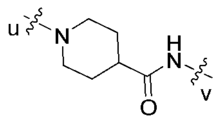 and 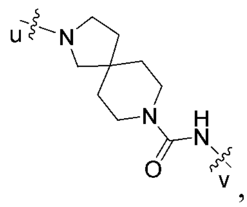,

(8)

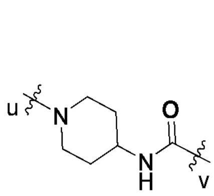 and 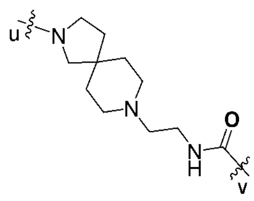,

(9)

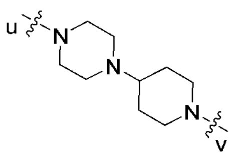, 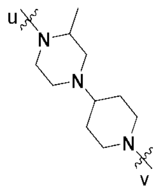

(preferably

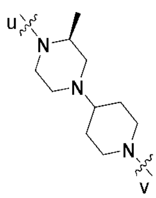 and 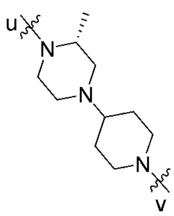

),

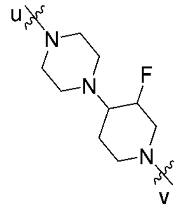

(preferably

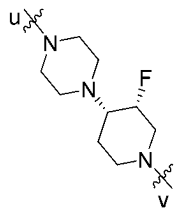 , 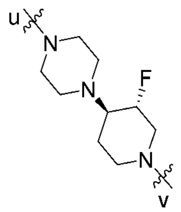 , 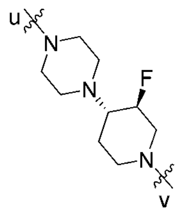

and

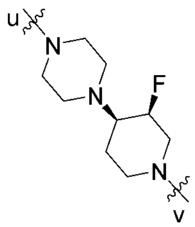

),

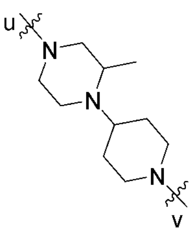

(preferably

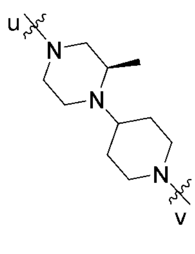 and 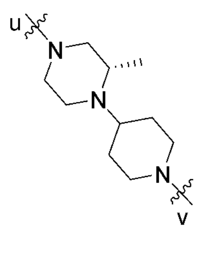

),

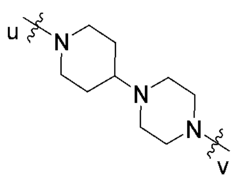 , 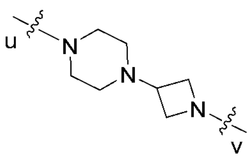 , 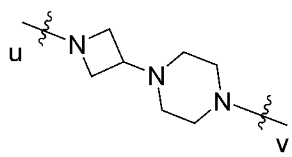

and

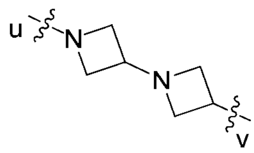, 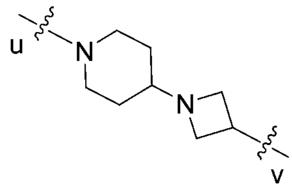,

(10)

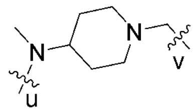 and 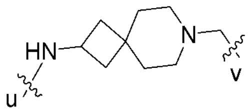,

(11)

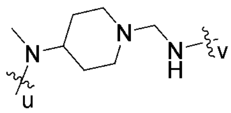 and 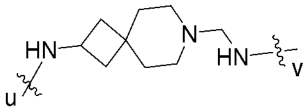,

(12)

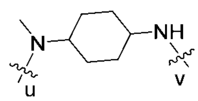

(preferably

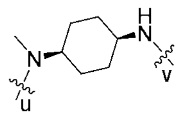, 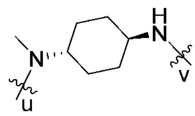,

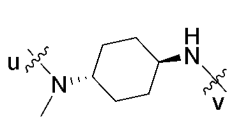 and 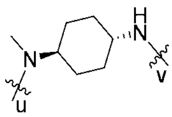

)

and 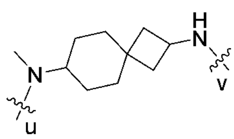,

(13)

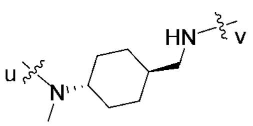 and 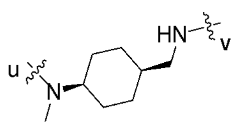,

(14)

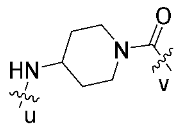

,

(15)

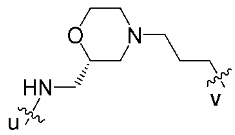

and

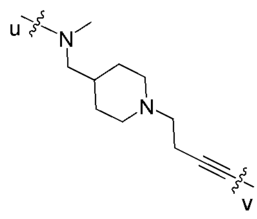

,

(16)

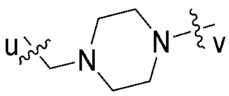

,

(17)

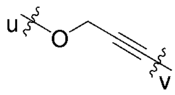

,

(18)

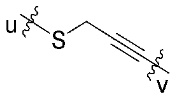

,

(19)

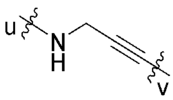

,

(20)

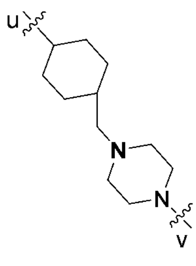

preferably (

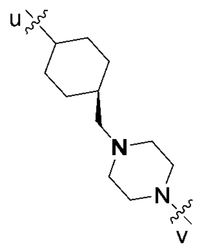 and 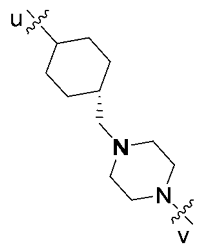

) and

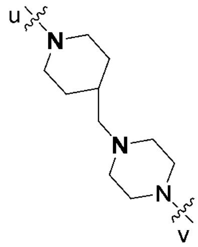

and

(21)

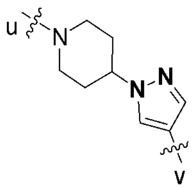 and 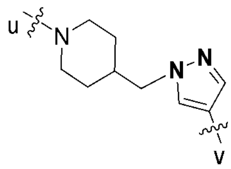;

wherein preferably, in any group of the above (1) to (21), the bond labeled with "u" is connected to the LA, and the bond labeled with "v" is connected to the

LBM

moiety.

In some preferred embodiments, $L^B$ is a group selected from the following (1) to (19):

(1)

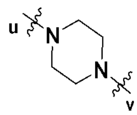, 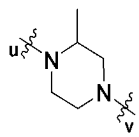

(preferably

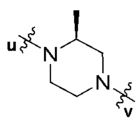 and 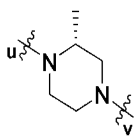), 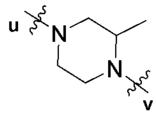

(preferably

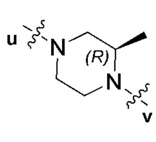 and 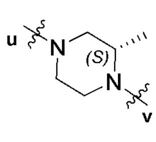 ), 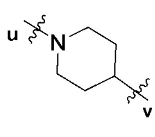, 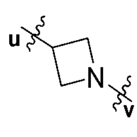, 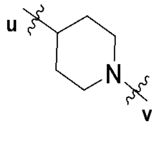,

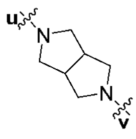, 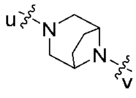, 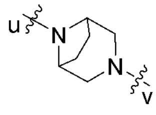, 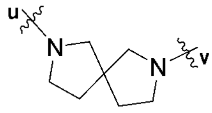, 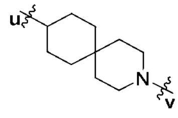, 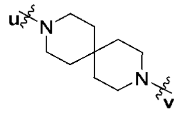,

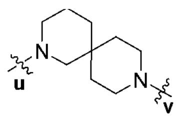, 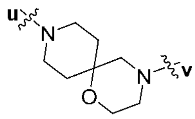, 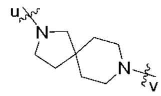, 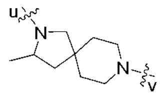

(preferably

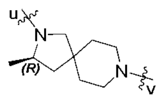

and

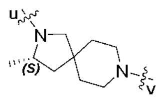

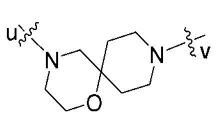, 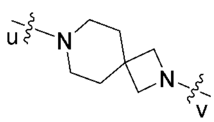, 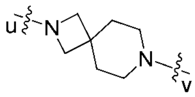

and

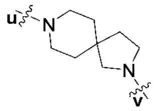,

(2)

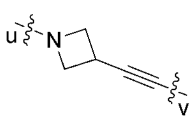, 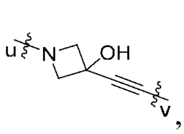, 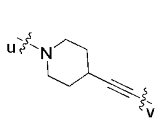, 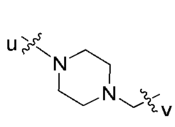, 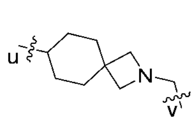,

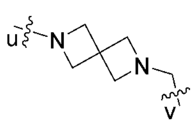, 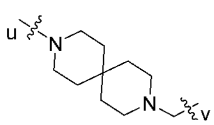, 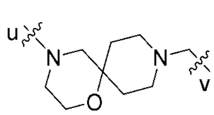, 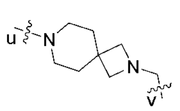

and

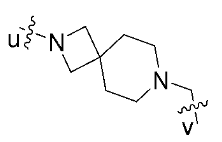

,

(3)

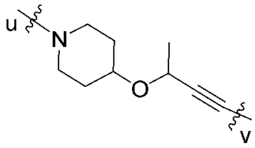

(preferably

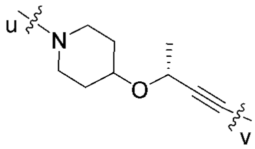

and

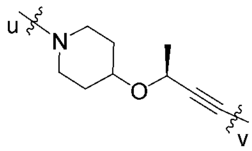 ), 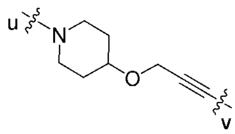 , 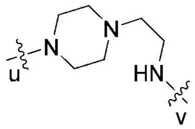 , 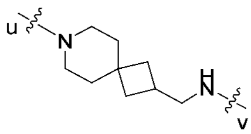 ,

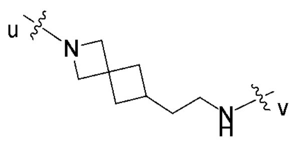 , 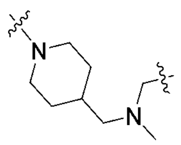 , 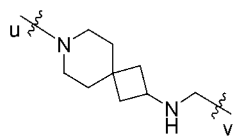 , 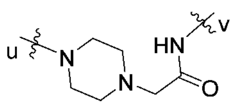

and

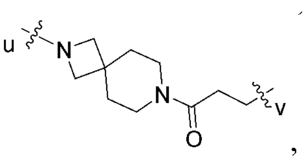

,

(4)

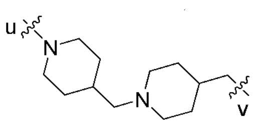 ,

(5)

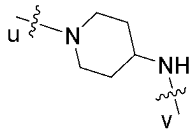,

(6)

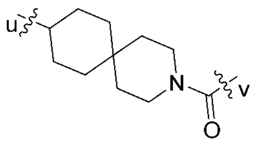,

(7)

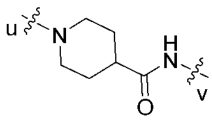 and 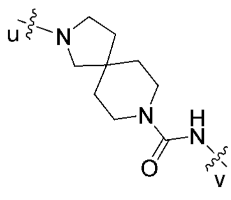,

(8)

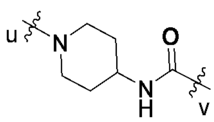 and 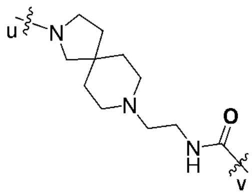,

(9)

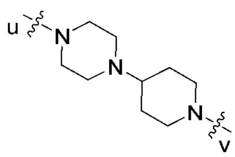, 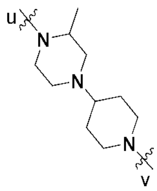

(preferably

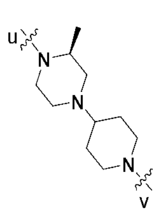 and 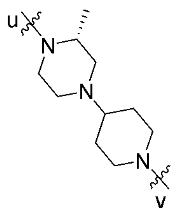

),

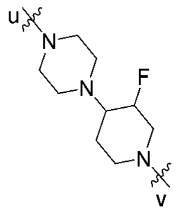

(preferably

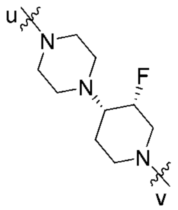,
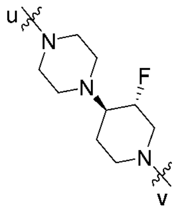,
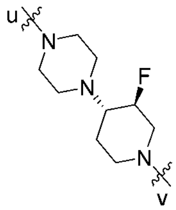

and

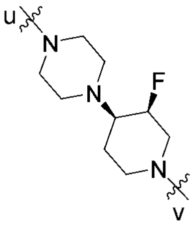

),

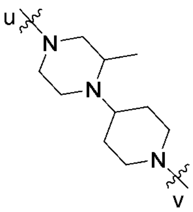

(preferably

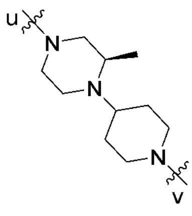 and 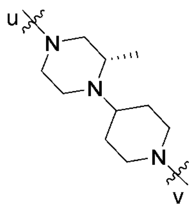),

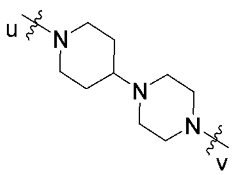,
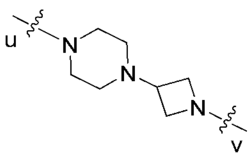,
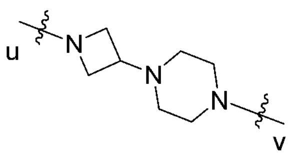

and

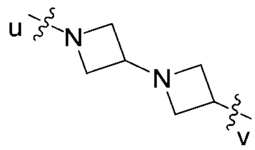,

(10)

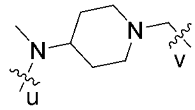 and 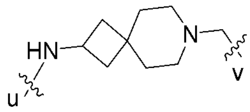,

(11)

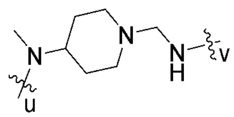 and 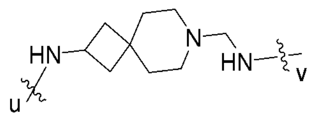,

(12)

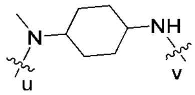

(preferably

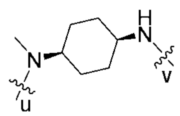, 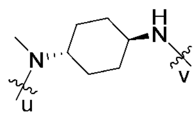,

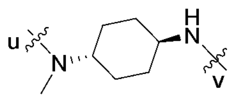 and 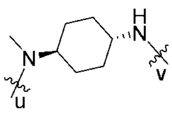

) and

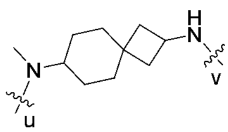,

(13)

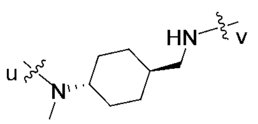 and 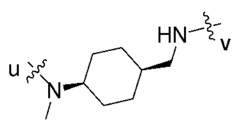,

(14)

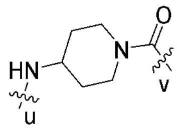,

(15)

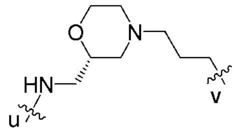 and 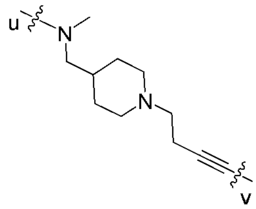,

(16)

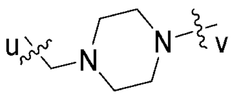,

(17)

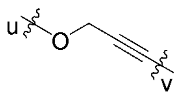,

(18)

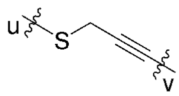,

and

(19)

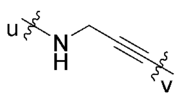;

wherein preferably, in any group of the above (1) to (19), the bond labeled with "u" is connected to the LA, and the bond labeled with "v" is connected to the

LBM

moiety.

Ligase binding moiety

In some embodiments, the present disclosure provides a compound of formula (A) according to the present disclosure, wherein the

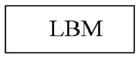

moiety is an E3 ubiquitin ligase ligand.

In some preferred embodiments, the

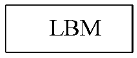

moiety is selected from:

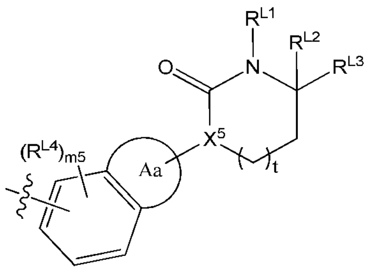

,

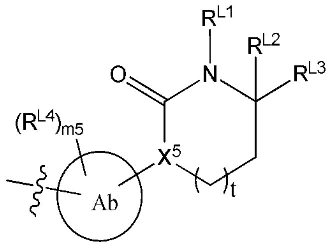

and

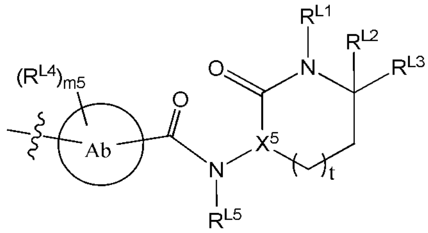

,

wherein:

ring Aa

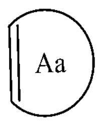

is 5-membered heterocyclyl or 5-membered heteroaryl, preferably 5-membered heterocyclyl or 5-membered heteroaryl having 1, 2 or more N heteroatoms, wherein the 5-membered heterocyclyl and the 5-membered heteroaryl are optionally substituted with one or more substituents independently selected from H, halogen, OH, $NH_2$, CN, oxo and $C_{1-4}$ alkyl,

preferably,

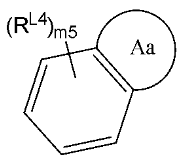

moiety is selected from

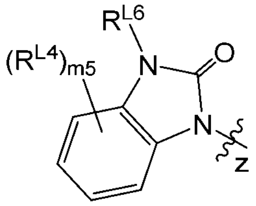

,

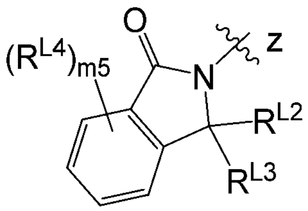 and 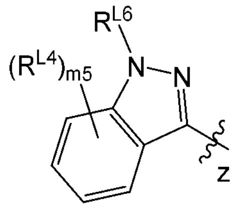,

wherein the bond labeled with "z" is connected to $X^5$;
each ring

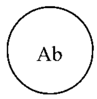

is independently phenyl or 5- to 6-membered heteroaryl, preferably phenyl;
$X^5$ is $CR^{L7}$ or N;
t is 0 or 1, preferably 1;
$R^{L1}$, $R^{L5}$ and $R^{L6}$, at each occurrence, are each independently selected from H and $C_{1-4}$ alkyl, preferably H and methyl;
$R^{L2}$ and $R^{L3}$, at each occurrence, are each independently selected from H and $C_{1-4}$ alkyl, preferably H and methyl; or $R^{L2}$ and $R^{L3}$ together form oxo;
$R^{L4}$ and $R^{L7}$, at each occurrence, are each independently selected from H, halogen, OH, $NH_2$, CN and $C_{1-4}$ alkyl, preferably H, F, Cl, Br and $C_{1-2}$ alkyl, more preferably H, F, Cl and methyl;
m5 is 0, 1, 2, 3 or 4, preferably 1 or 2.

In some more preferred embodiments, the

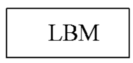

LBM moiety is selected from:

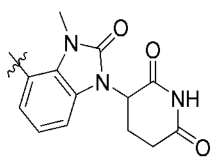, 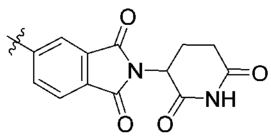, 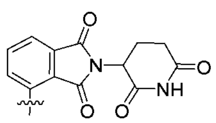, 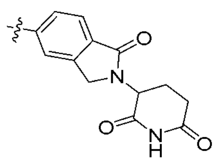,

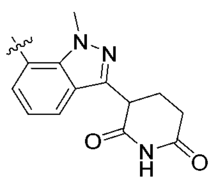, 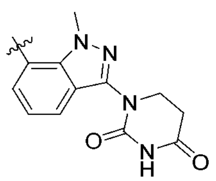, 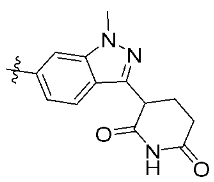, 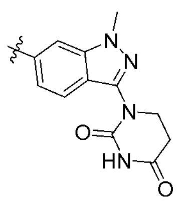,

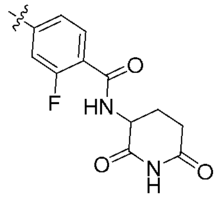 and 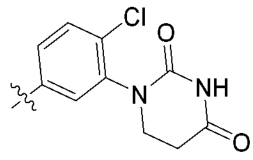.

In some more preferred embodiments,

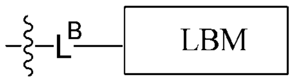

moiety is selected from:

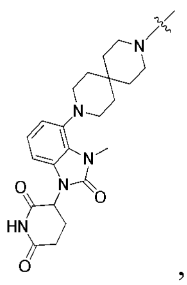, 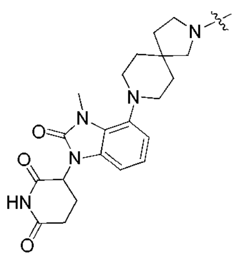, 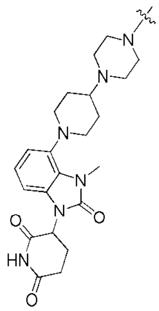, 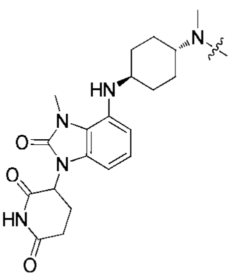, 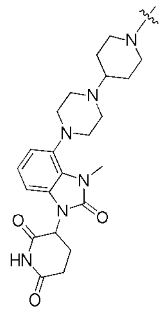

, 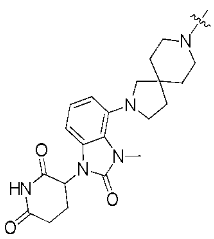, 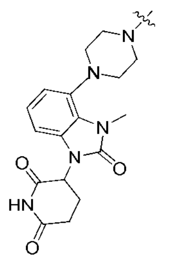, 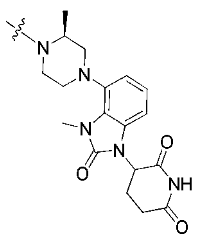, 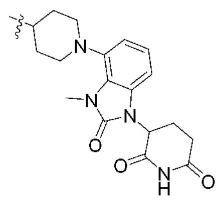,

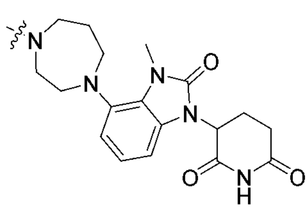, 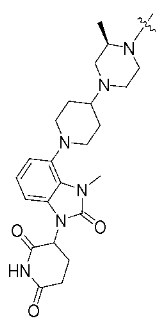, 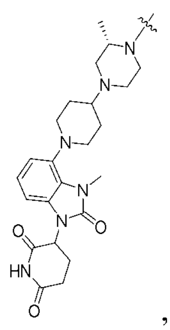, 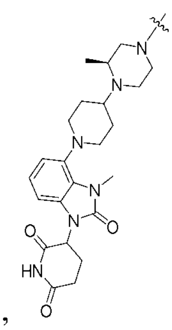, 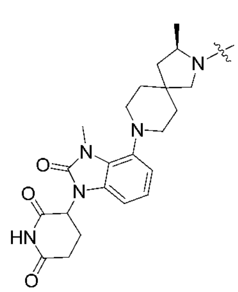

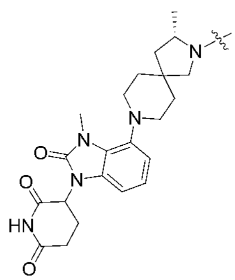 and 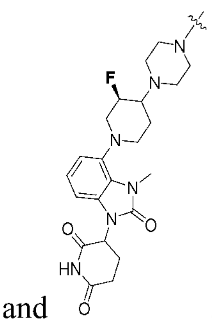.

In some more preferred embodiments, selected from:

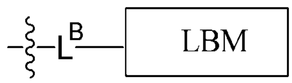

moiety is

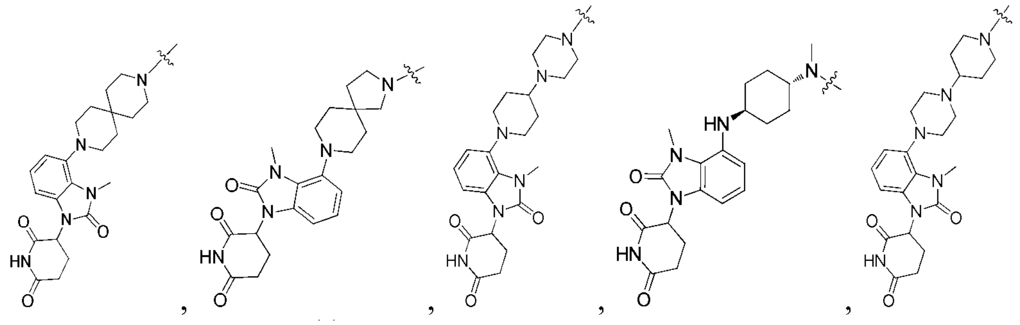

, , , ,

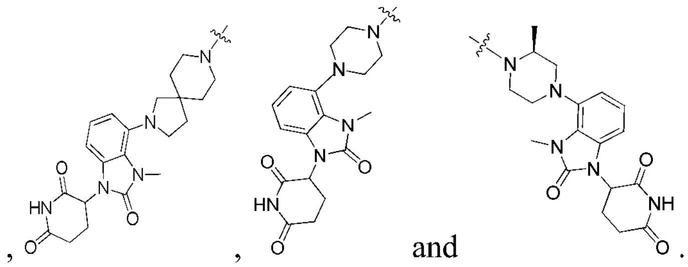

, , and .

In some preferred embodiments, the present disclosure provides a compound of formula (A), wherein the compound has a structure of formula (B):

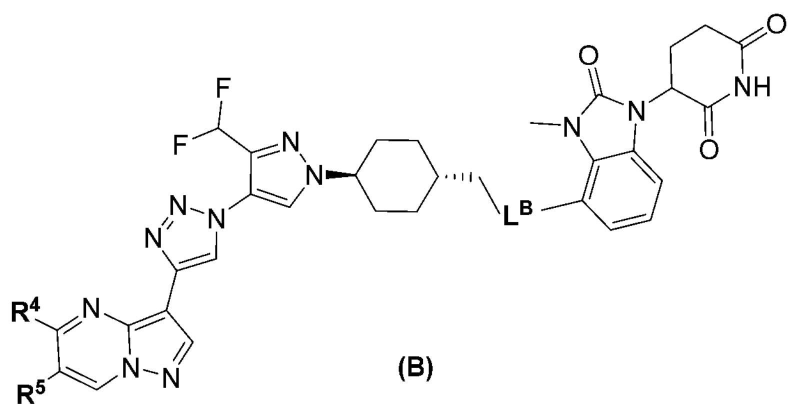

(B)

wherein:

$L^B$ is selected from:

(1) -CyL1-, wherein the CyL1 group here is selected from 7- to 11-membered spiro heterocycloalkylene, more preferably 9- to 11-membered spiro heterocycloalkylene, wherein the 7- to 11-membered spiro heterocycloalkylene and the 9- to 11-membered spiro heterocycloalkylene each have 1 or 2, preferably 2 nitrogen heteroatoms, and are optionally substituted with 1 or more groups independently selected from the following: $C_{1-4}$ alkyl and halogen, preferably methyl, F and Cl, more preferably methyl and F; or

(2) -CyL1-CyL2-, wherein the CyL1 group and the CyL2 group here are each independently selected from 4- to 7-membered monocyclic heterocycloalkylene, more preferably 4- to 6-membered monocyclic heterocycloalkylene, wherein the 4-to 7-membered monocyclic heterocycloalkylene and the 4- to 6-membered monocyclic heterocycloalkylene each have 1 or 2 nitrogen heteroatoms, and are optionally substituted with 1 or more groups independently selected from the following: $C_{1-4}$ alkyl and halogen, preferably methyl, F and Cl, more preferably methyl and F;

$R^4$ is selected from 4- to 6-membered saturated monocyclic heterocycloalkyl and $-NR_{Na}R_{Nb}$, where the $R_{Na}$ is selected from H and $C_{1-6}$ alkyl and the $R_{Nb}$ is selected from $C_{3-6}$ cycloalkyl, and wherein the 4- to 6-membered saturated monocyclic heterocycloalkyl and the $C_{3-6}$ cycloalkyl are substituted with 1-3 substituents selected from halogen and hydroxy; and $R^5$ is hydrogen.

In some embodiments, $L^B$ is selected from:

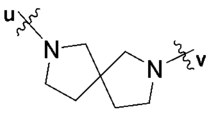, 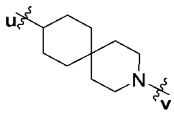, 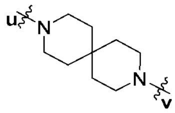, 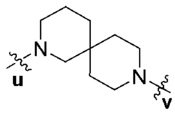, 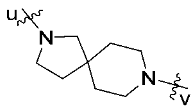,

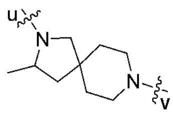

(preferably

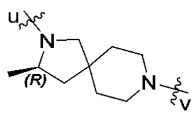 and 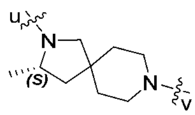

),

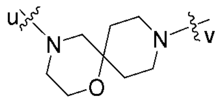,

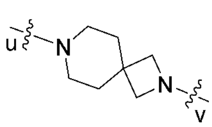, 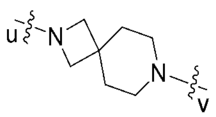 and 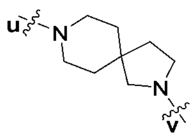, 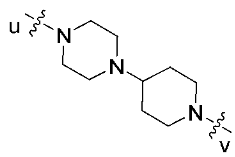,

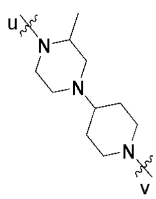

(preferably

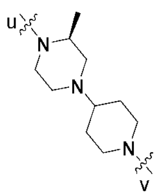 and 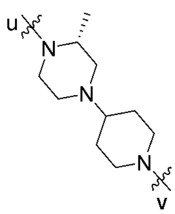

),

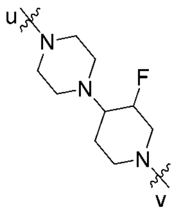

(preferably

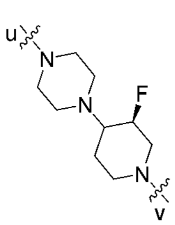 , 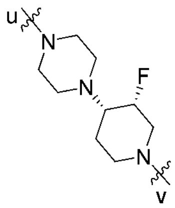 , 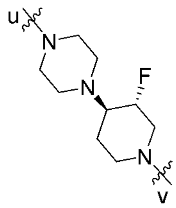 , 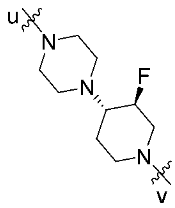 and 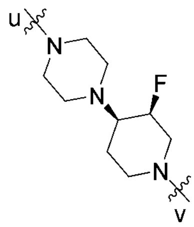 ),

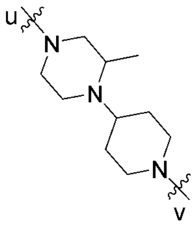

(preferably

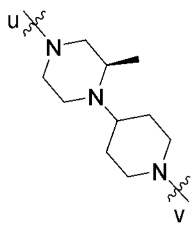 and 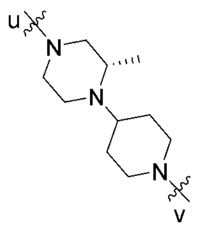

),

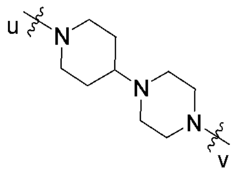,

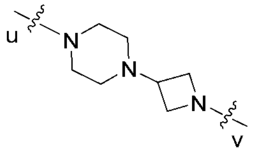 , 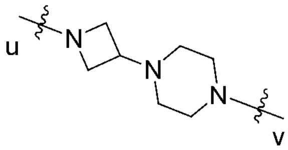 and 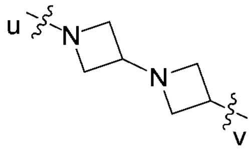 ,

more preferably

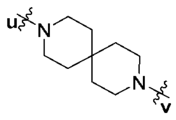 and 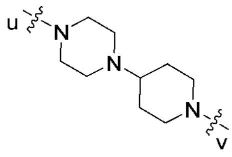,

wherein in any of the above groups, the bond labeled with "u" is connected to

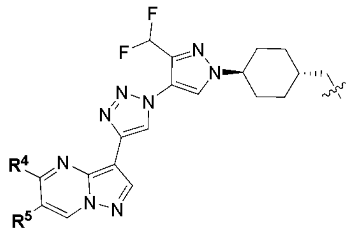

moiety, and the bond labeled with "v" is connected to

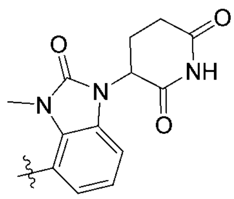

moiety.

In some preferred embodiments, $L^B$ is selected from:

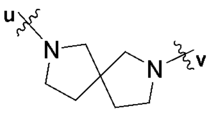, 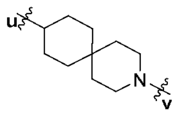, 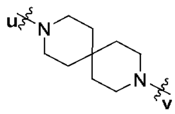, 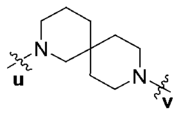, 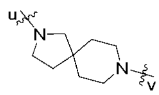,

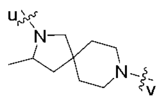

(preferably

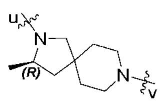 and 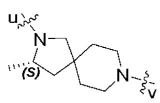

),

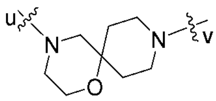,

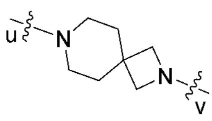, 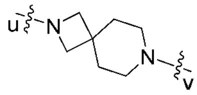 and 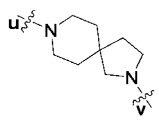, 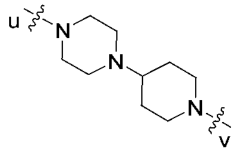, 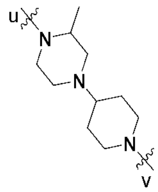

(preferably

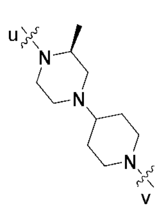 and 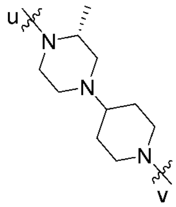

),

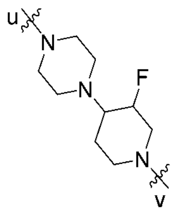

(preferably

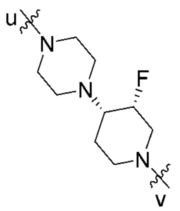, 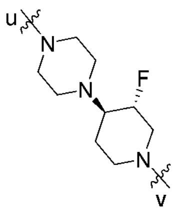, 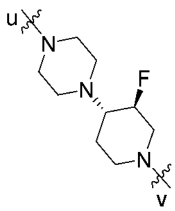 and 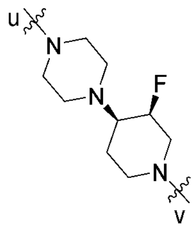), 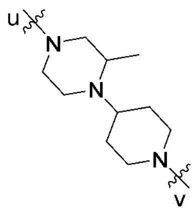

(preferably

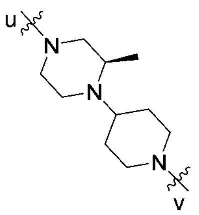

and

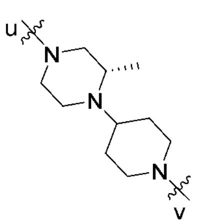

),

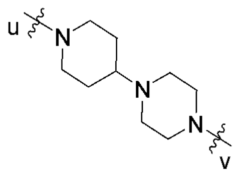

,

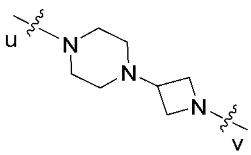

,

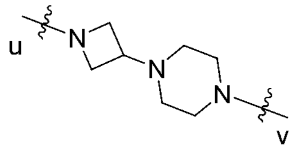

and

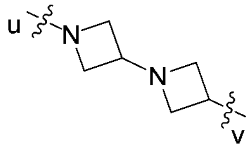

,

more preferably

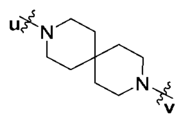

and

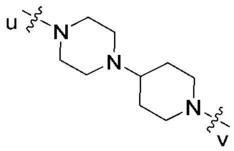

,

wherein in any of the above groups, the bond labeled with "u" is connected to

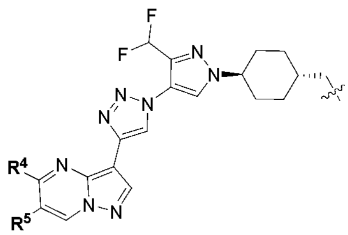

moiety, and the bond labeled with "v" is connected to

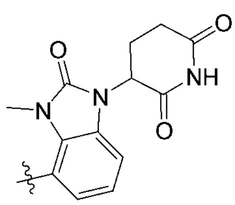

moiety.

In some preferred embodiments, $R^4$ is selected from 4- to 6-membered saturated monocyclic nitrogen-containing heterocycloalkyl (preferably piperidinyl) and $-NR_{Na}R_{Nb}$, wherein the $R_{Na}$ is H and the $R_{Nb}$ is selected from $C_{3-6}$ cycloalkyl (preferably cyclohexyl), and wherein the 4- to 6-membered saturated monocyclic nitrogen-containing heterocycloalkyl and the $C_{3-6}$ cycloalkyl are substituted with 1 hydroxy.

In some more preferred embodiments, $R^4$ is selected from

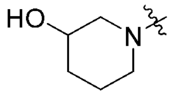

and

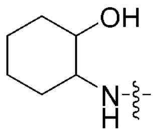,

more preferably

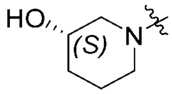 and 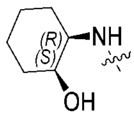 .

The present disclosure encompasses compounds obtained by combining any of the embodiments.

In some embodiments, the present disclosure provides the compound of formula (A), or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, wherein the compound is selected from the following Table 1:

Table 1:

| | |
|---|---|
| 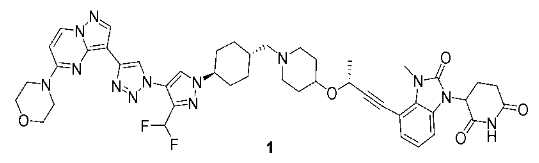 1 | 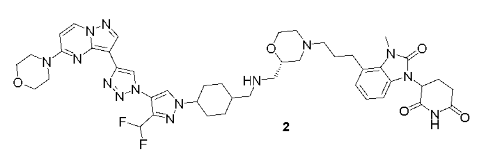 2 |
| 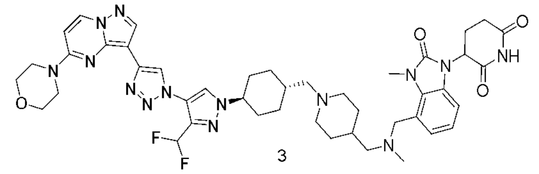 3 | 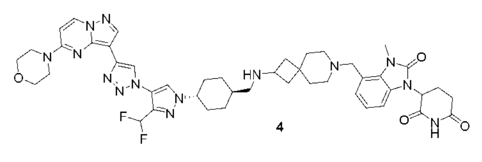 4 |
| 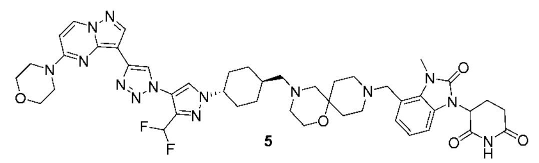 5 | 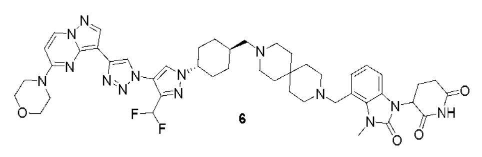 6 |
| 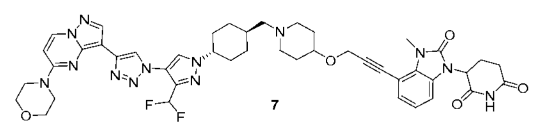 7 | 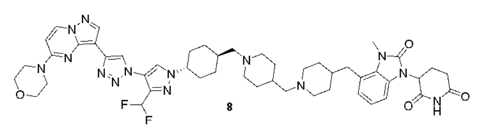 8 |
| 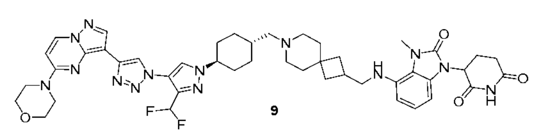 9 | 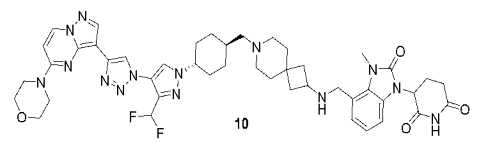 10 |
| 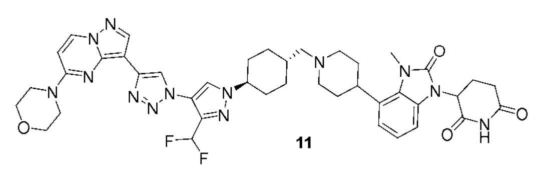 11 | 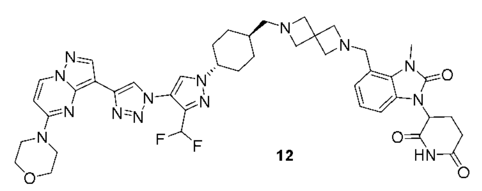 12 |
| 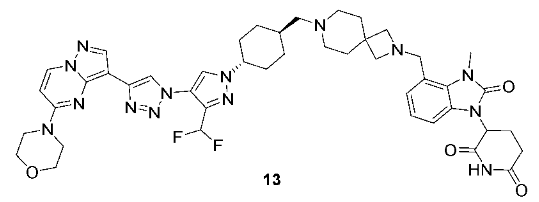 13 | 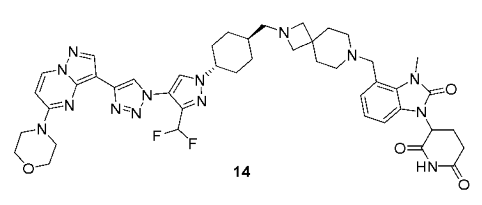 14 |
| 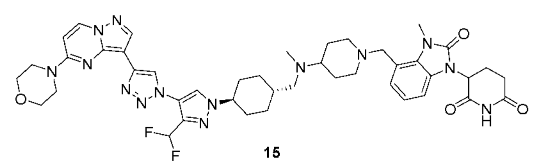 15 | 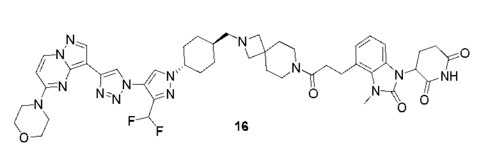 16 |

| | |
|---|---|
| 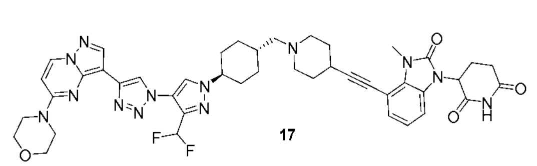 17 | 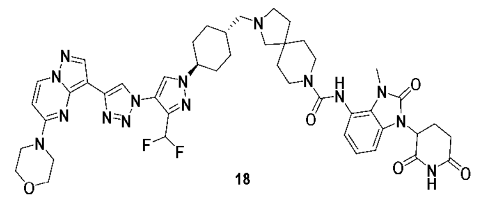 18 |
| 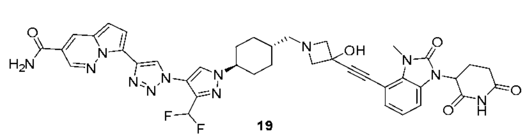 19 | 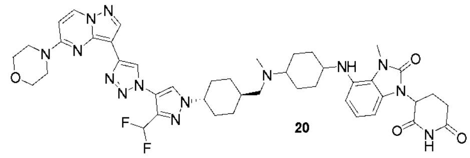 20 |
| 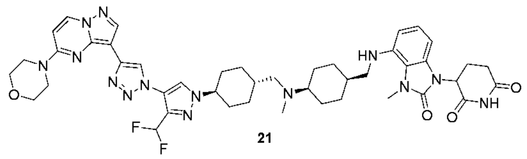 21 | 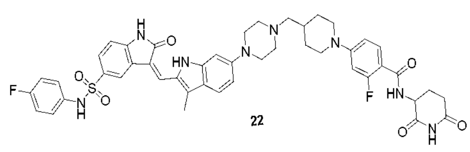 22 |
| 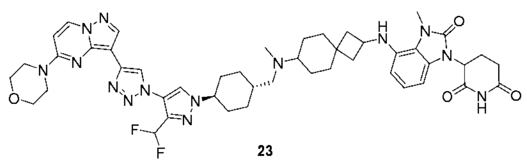 23 | 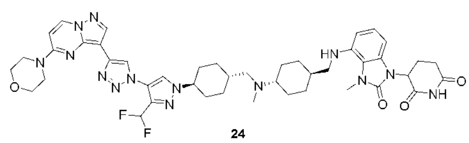 24 |
| 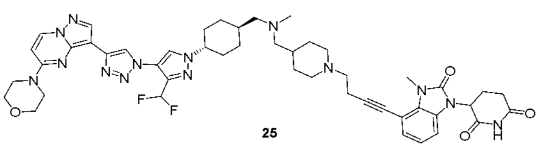 25 | 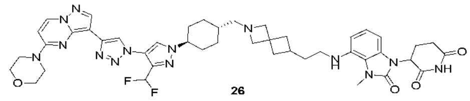 26 |
| 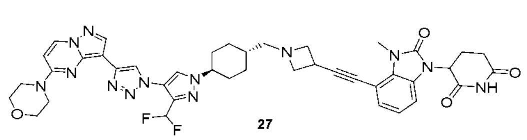 27 | 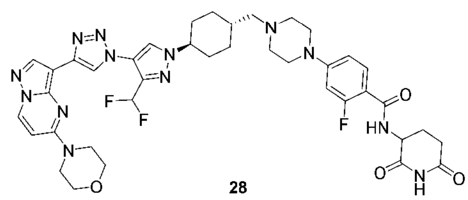 28 |
| 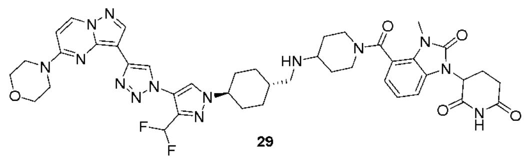 29 | 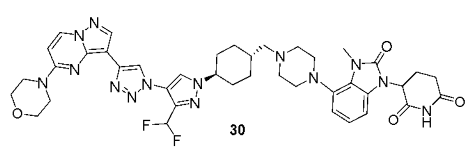 30 |
| 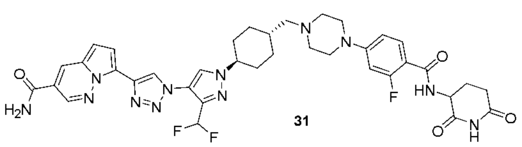 31 | 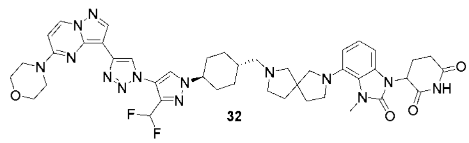 32 |
| 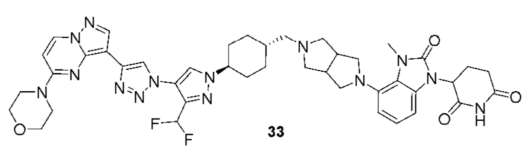 33 | 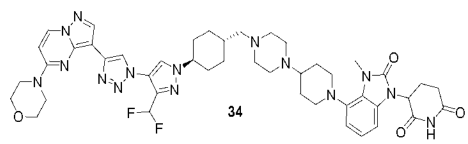 34 |

| | |
|---|---|
| 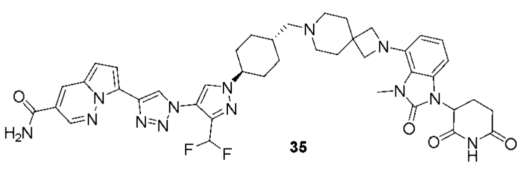 35 | 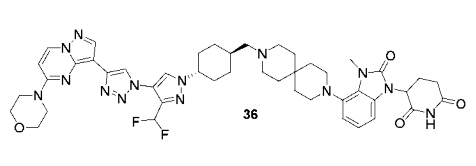 36 |
| 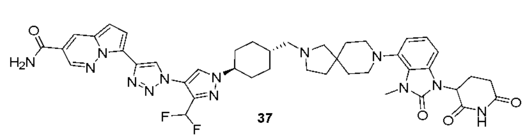 37 | 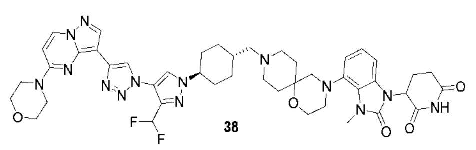 38 |
| 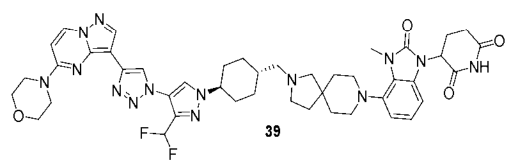 39 | 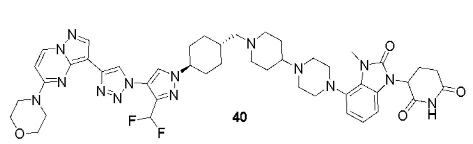 40 |
| 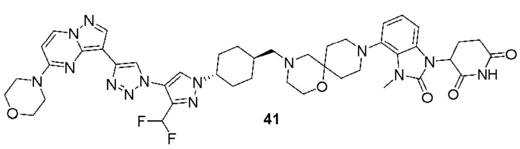 41 | 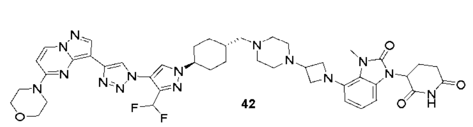 42 |
| 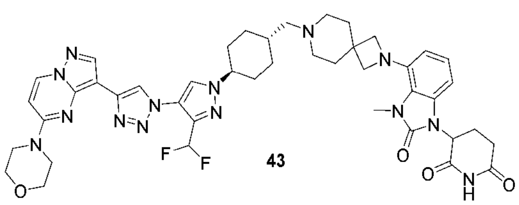 43 | 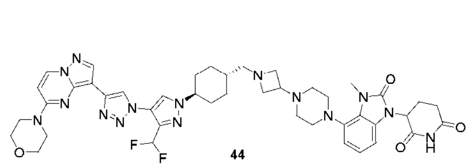 44 |
| 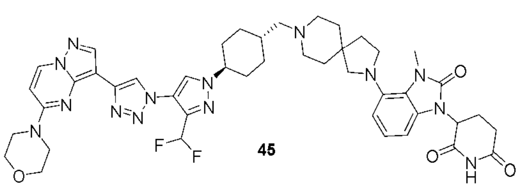 45 | 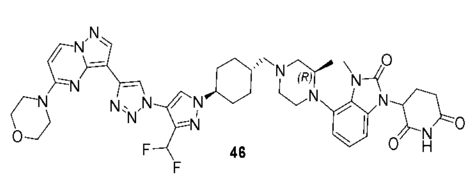 46 |
| 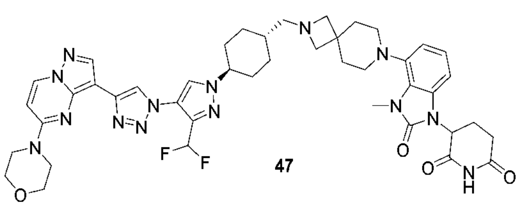 47 | 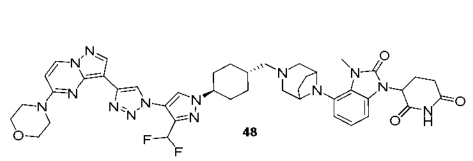 48 |
| 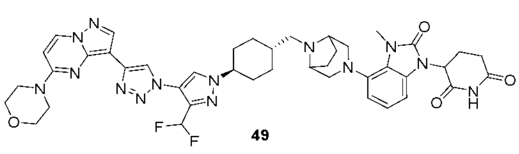 49 | 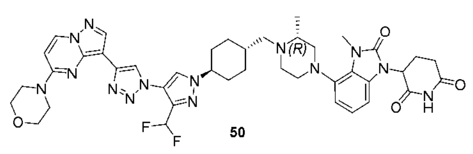 50 |
| 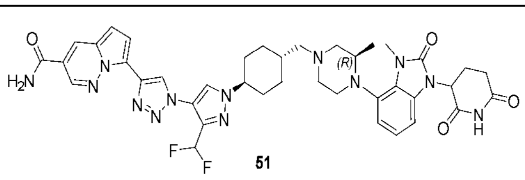 51 | 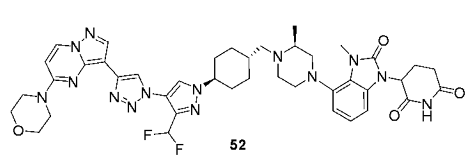 52 |

| | |
|---|---|
| 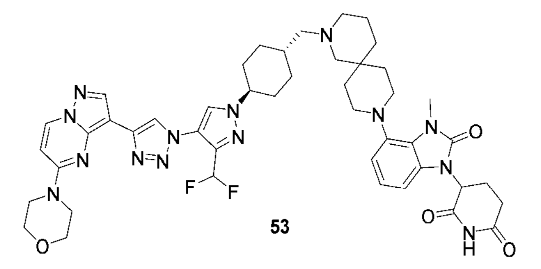 53 | 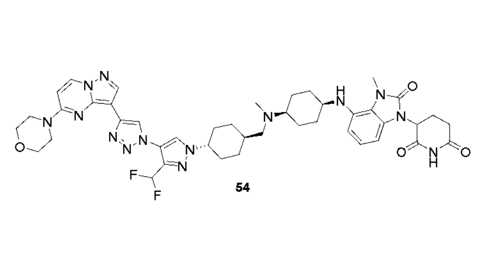 54 |
| 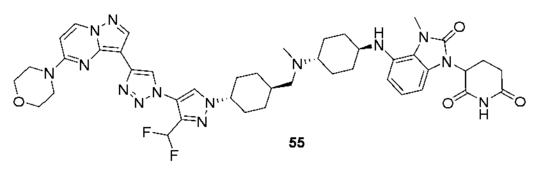 55 | 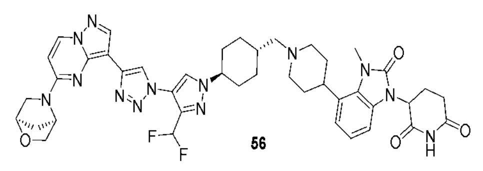 56 |
| 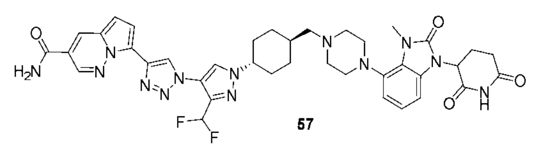 57 | 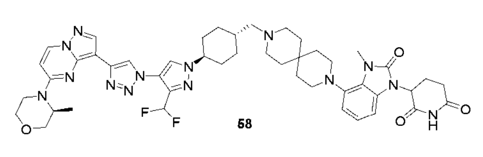 58 |
| 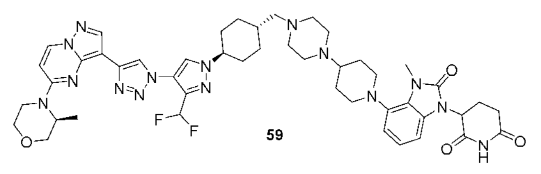 59 | 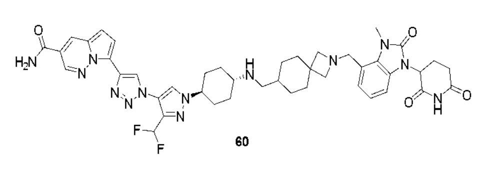 60 |
| 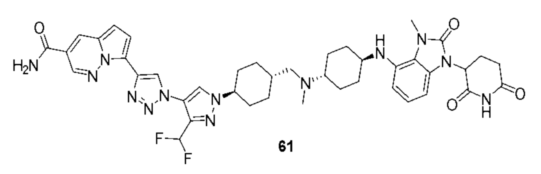 61 | 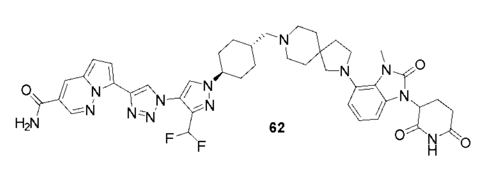 62 |
| 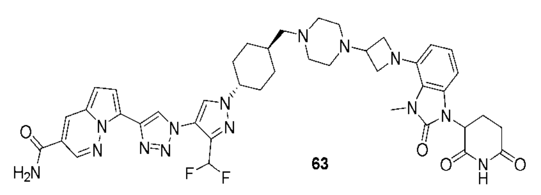 63 | 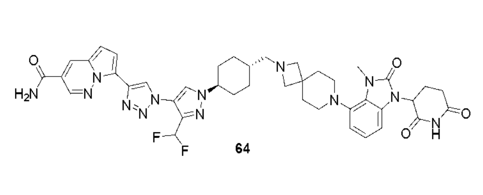 64 |
| 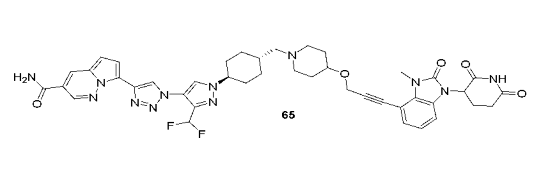 65 | 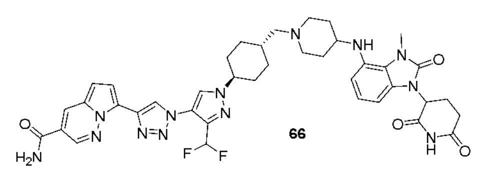 66 |
| 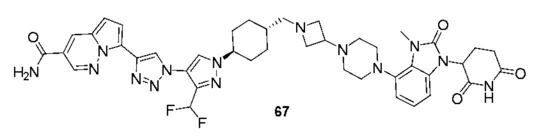 67 | 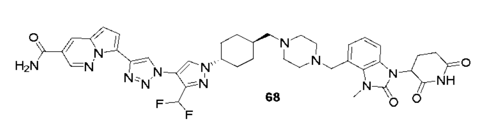 68 |
| 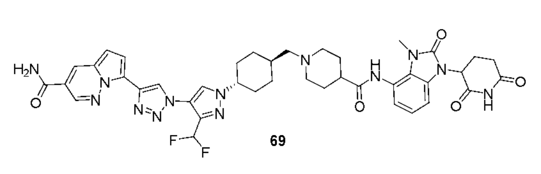 69 | 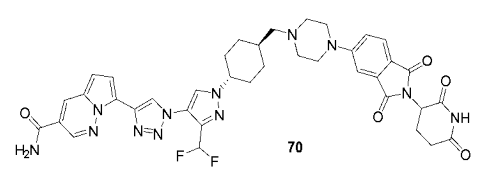 70 |

| | |
|---|---|
| 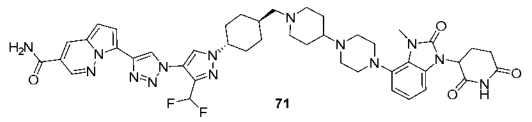 71 | 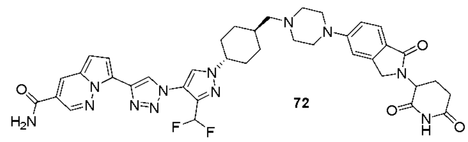 72 |
| 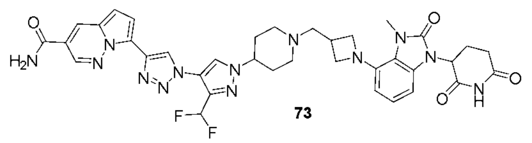 73 | 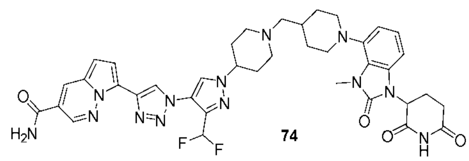 74 |
| 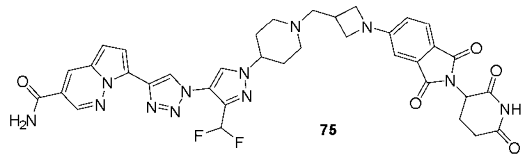 75 | 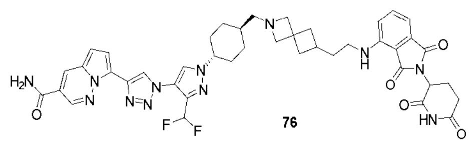 76 |
| 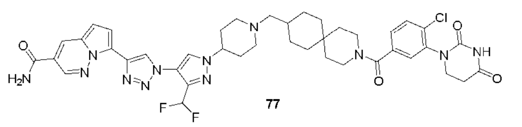 77 | 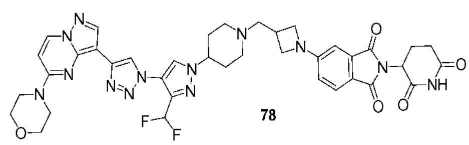 78 |
| 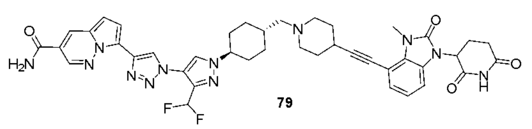 79 | 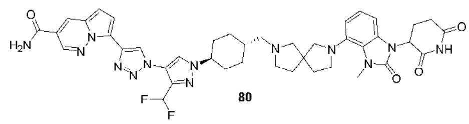 80 |
| 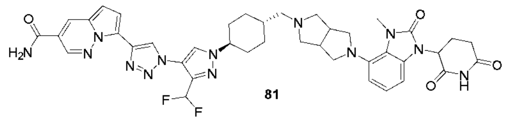 81 | 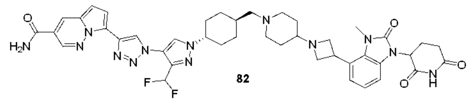 82 |
| 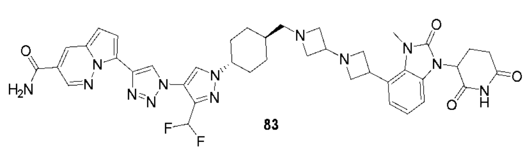 83 | 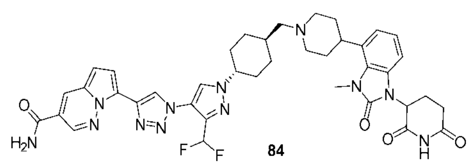 84 |
| 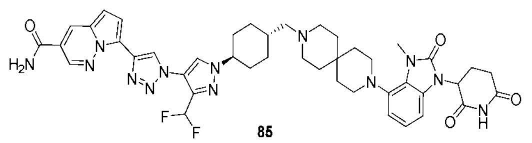 85 | 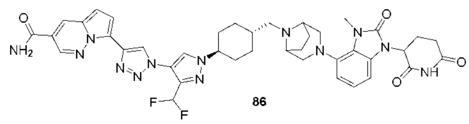 86 |
| 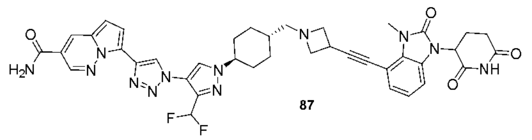 87 | 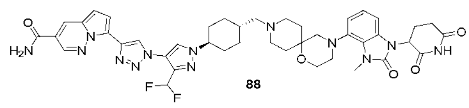 88 |
| 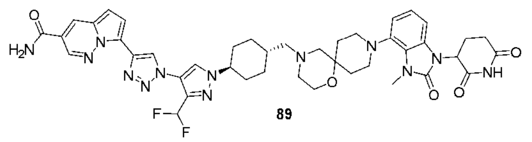 89 | 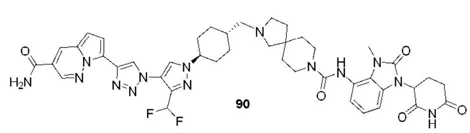 90 |

| | |
|---|---|
| 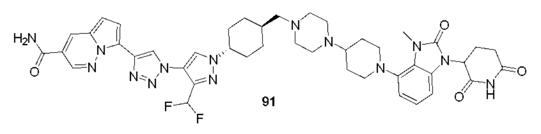 91 | 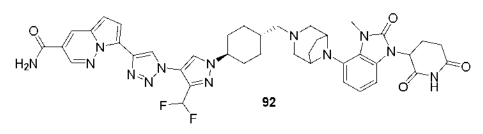 92 |
| 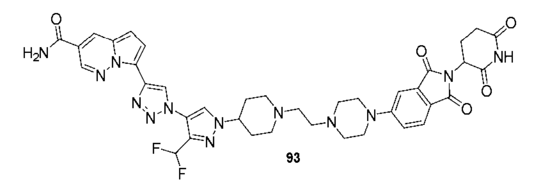 93 | 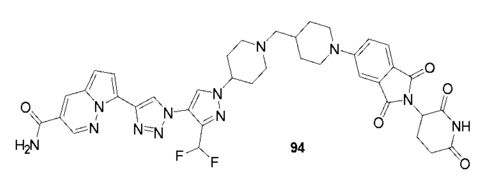 94 |
| 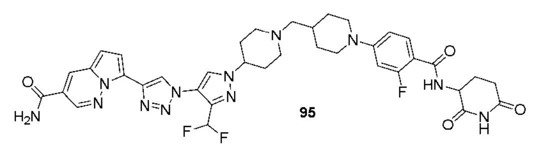 95 | 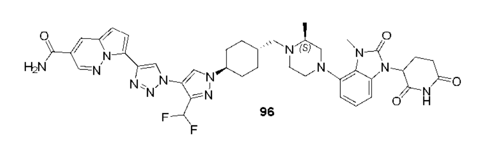 96 |
| 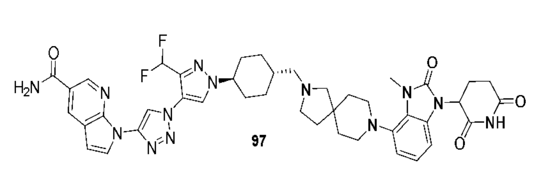 97 | 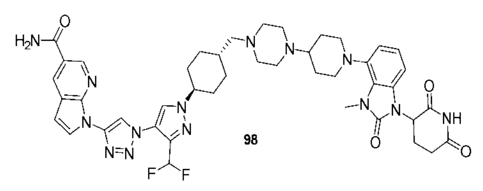 98 |
| 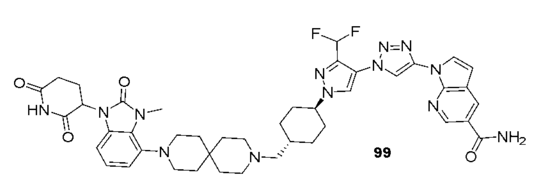 99 | 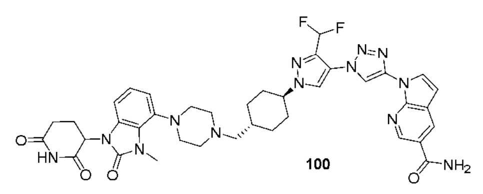 100 |
| 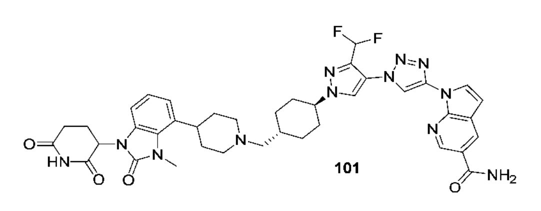 101 | 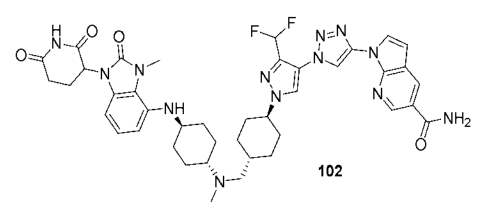 102 |
| 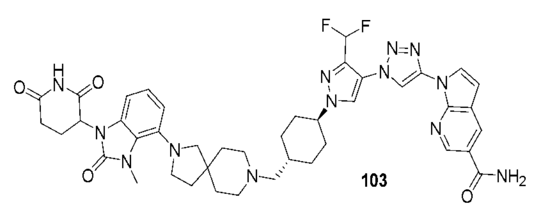 103 | 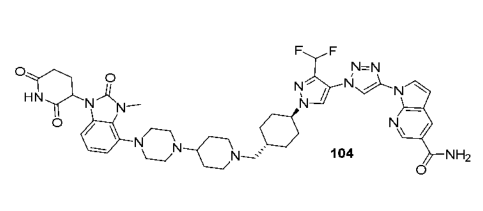 104 |
| 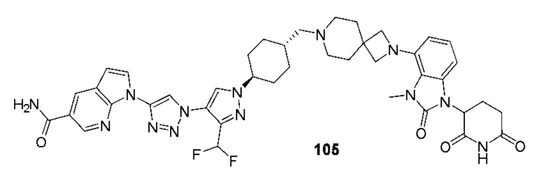 105 | 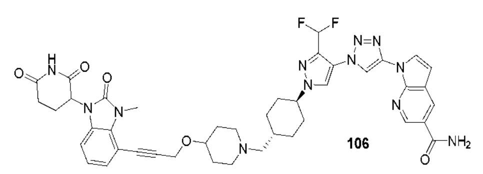 106 |

| | |
|---|---|
| 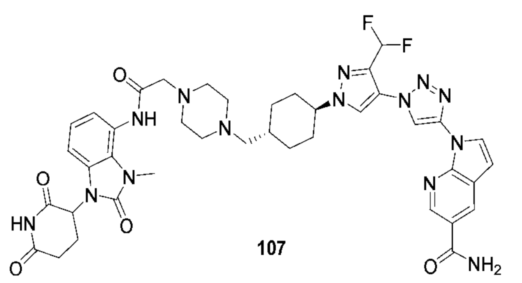 107 | 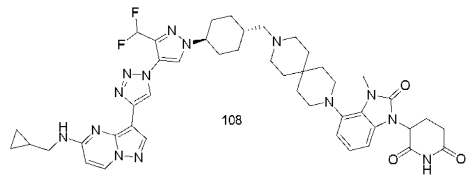 108 |
| 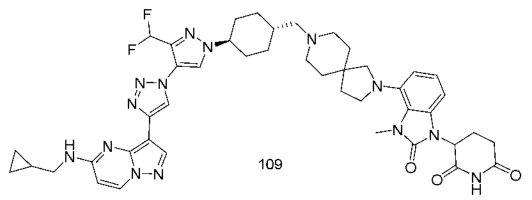 109 | 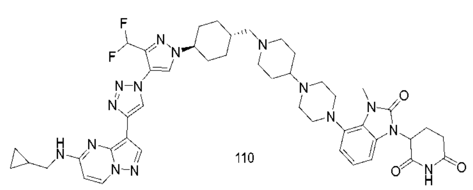 110 |
| 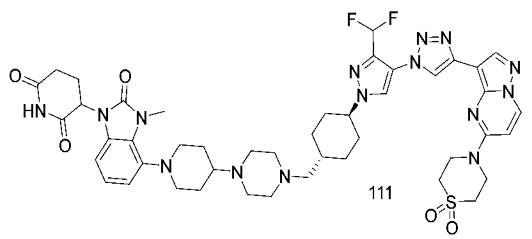 111 | 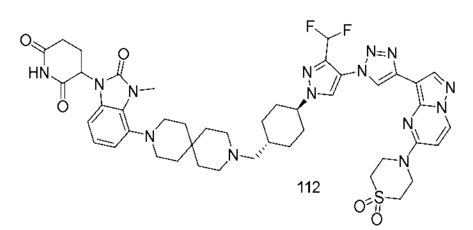 112 |
| 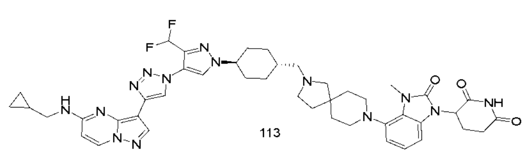 113 | 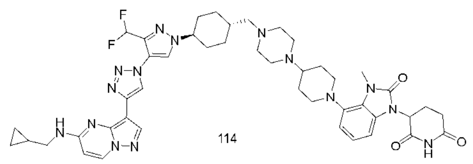 114 |
| 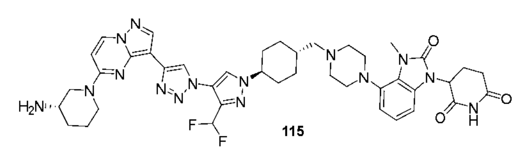 115 | 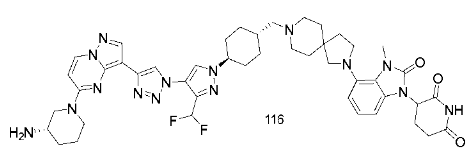 116 |
| 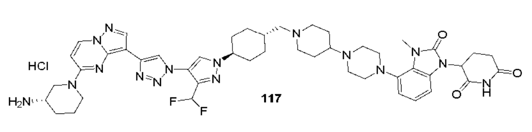 117 | 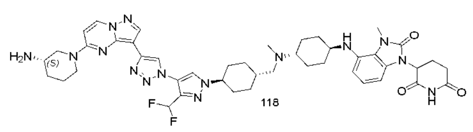 118 |
| 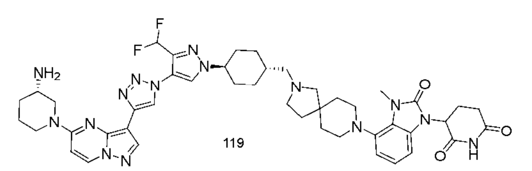 119 | 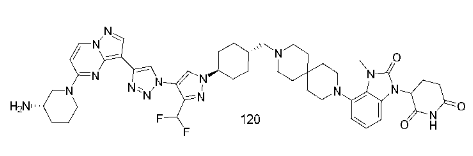 120 |
| 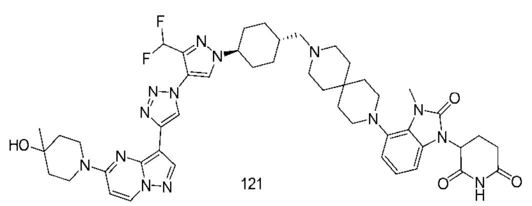 121 | 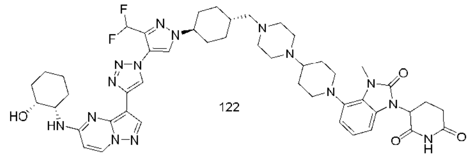 122 |

| | |
|---|---|
| 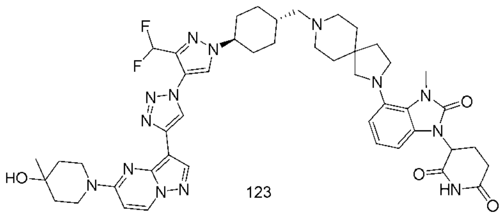 123 | 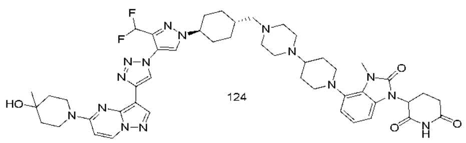 124 |
| 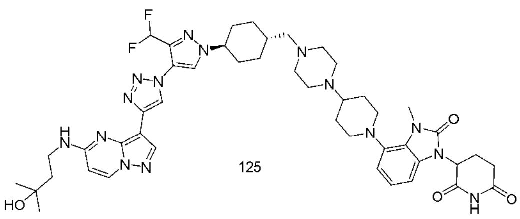 125 | 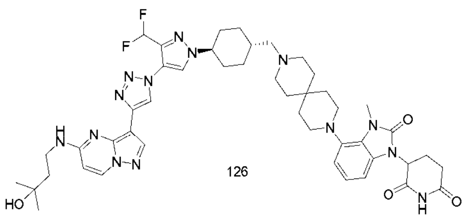 126 |
| 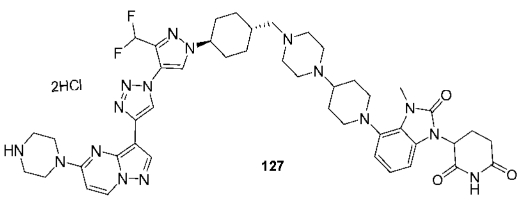 2HCl 127 | 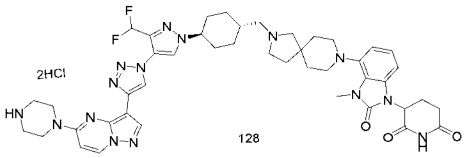 2HCl 128 |
| 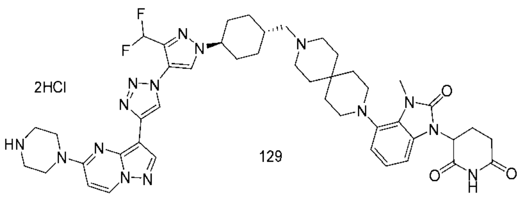 2HCl 129 | 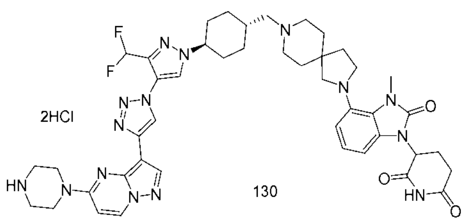 2HCl 130 |
| 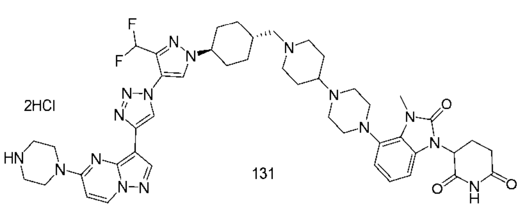 2HCl 131 | 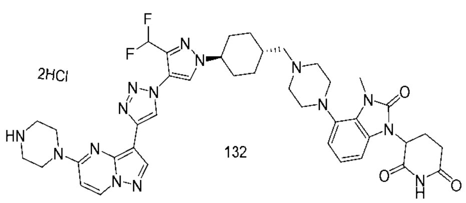 2HCl 132 |
| 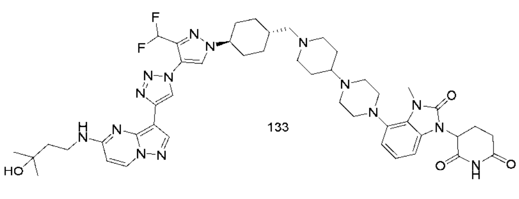 133 | 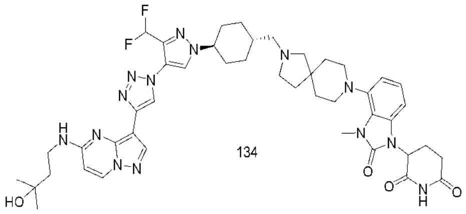 134 |
| 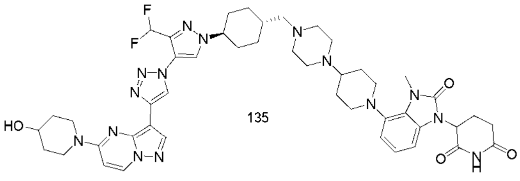 135 | 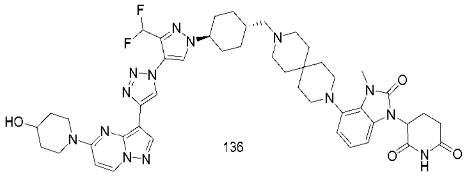 136 |

| | |
|---|---|
| 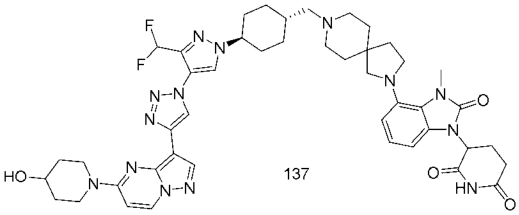 137 | 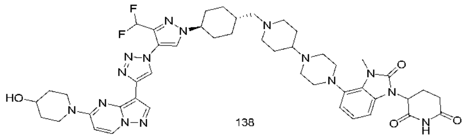 138 |
| 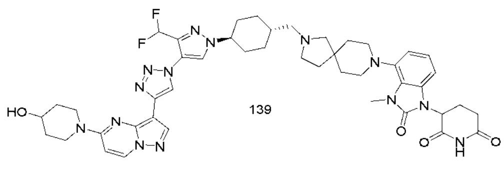 139 | 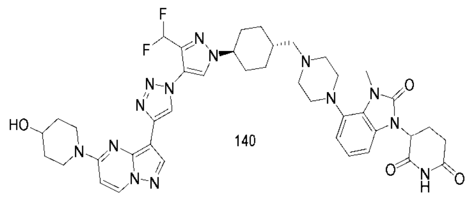 140 |
| 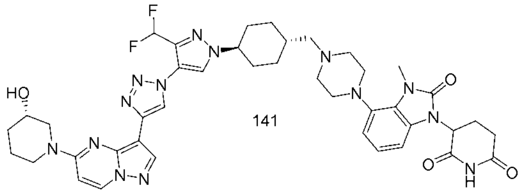 141 | 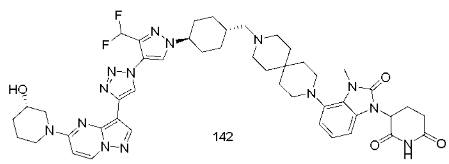 142 |
| 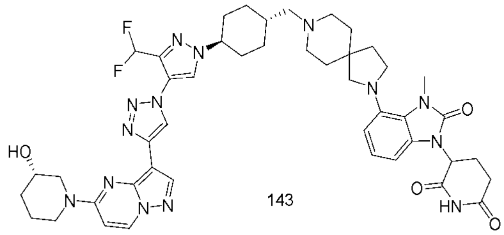 143 | 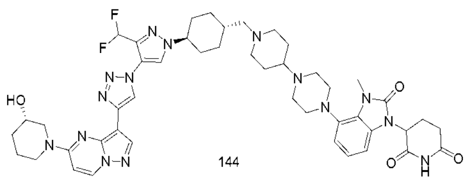 144 |
| 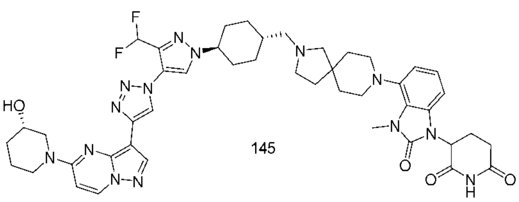 145 | 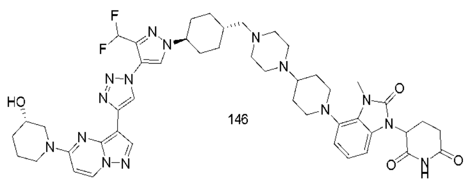 146 |
| 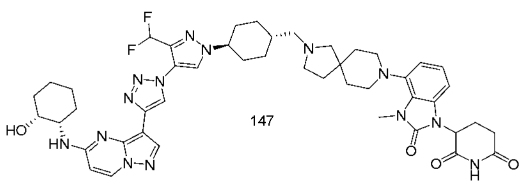 147 | 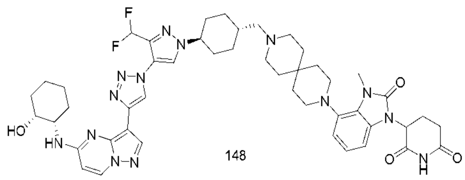 148 |
| 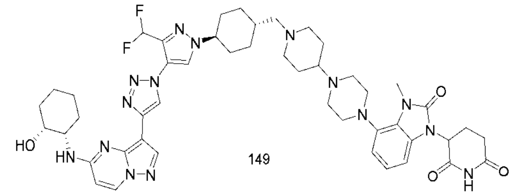 149 | 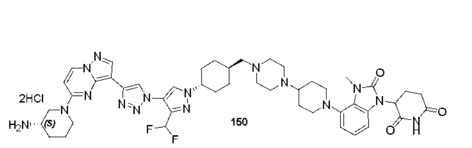 2HCl 150 |

| | |
|---|---|
| 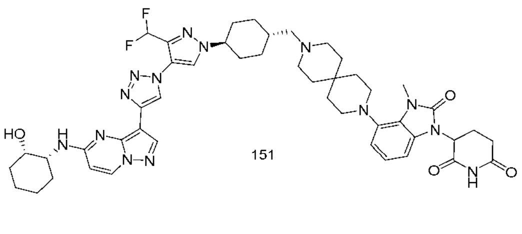 151 | 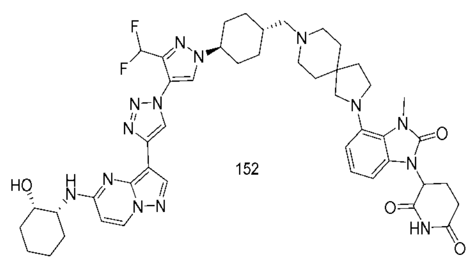 152 |
| 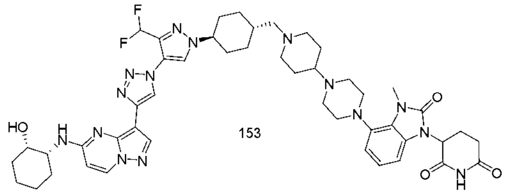 153 | 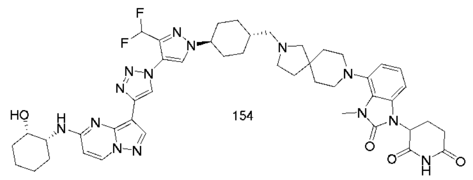 154 |
| 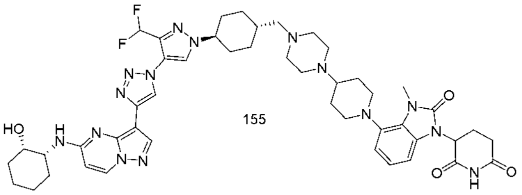 155 | 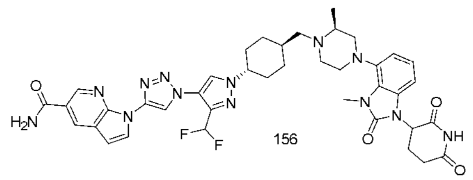 156 |
| 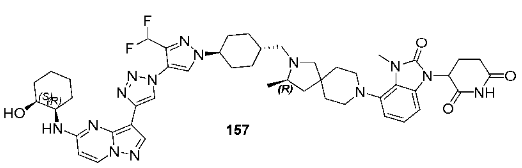 **157** | 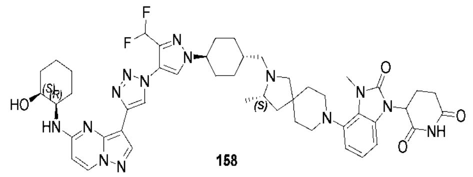 **158** |
| 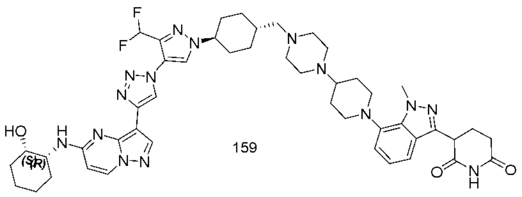 159 | 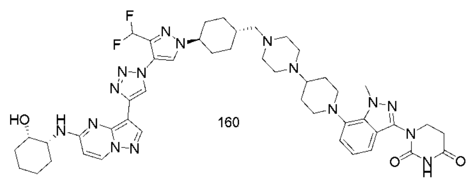 160 |
| 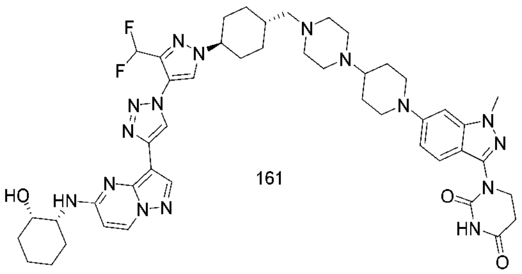 161 | 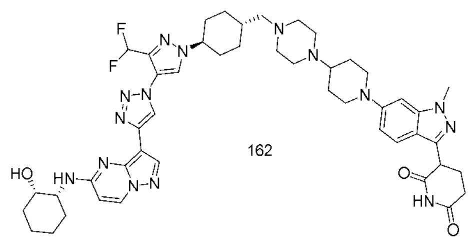 162 |
| 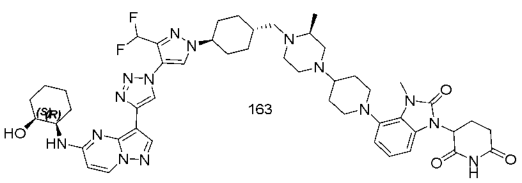 163 | 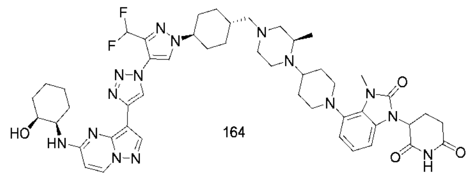 164 |

| | |
|---|---|
| 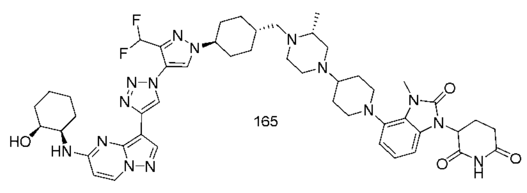 165 | 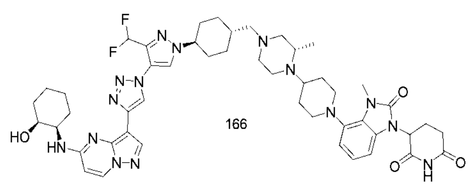 166 |
| 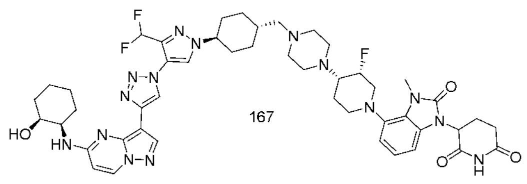 167 | 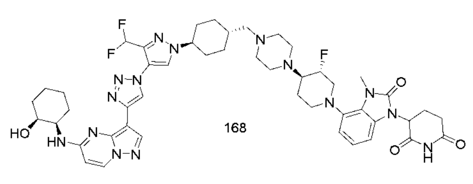 168 |
| 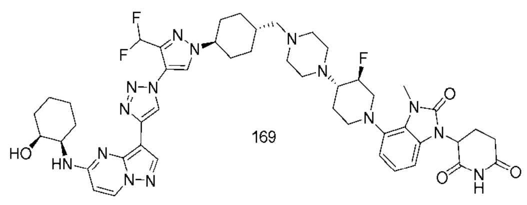 169 | 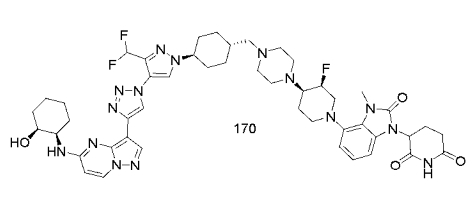 170 |
| 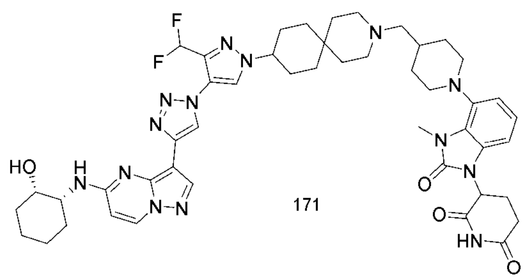 171 | 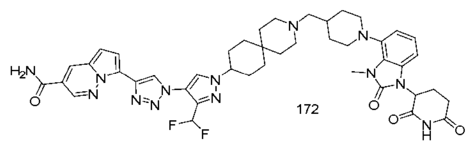 172 |
| 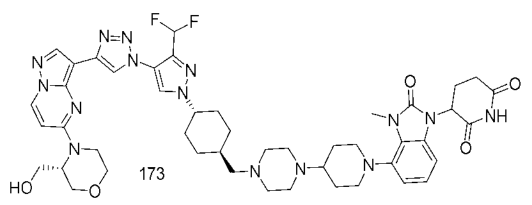 173 | 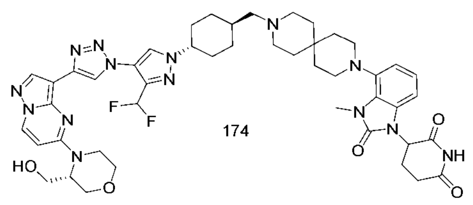 174 |
| 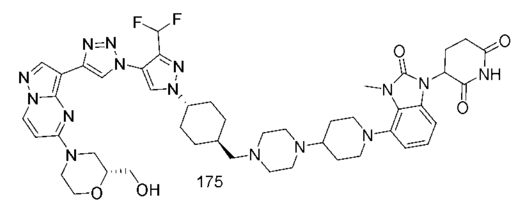 175 | 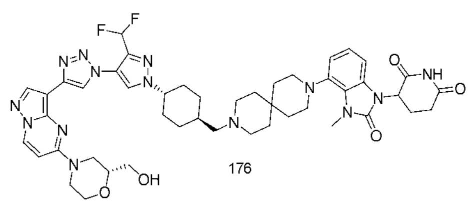 176 |
| 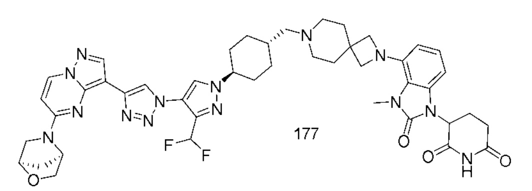 177 | 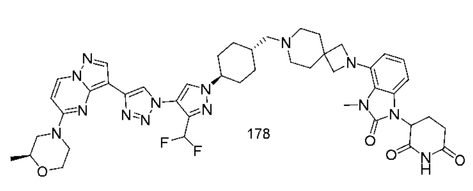 178 |

| | |
|---|---|
| 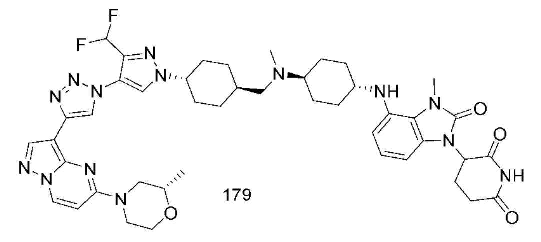 179 | 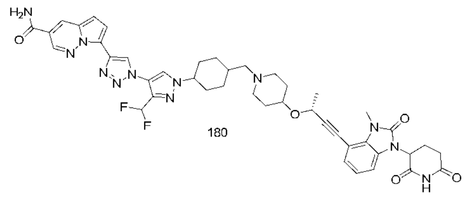 180 |
| 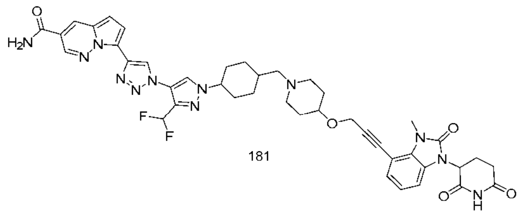 181 | 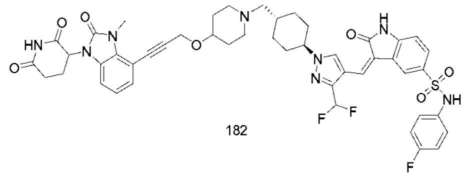 182 |
| 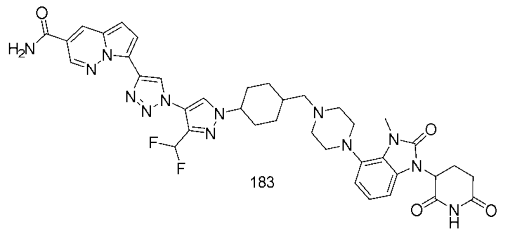 183 | 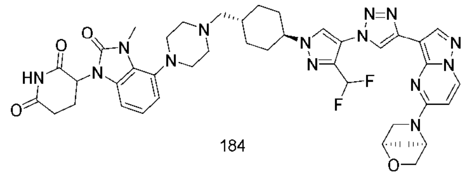 184 |
| 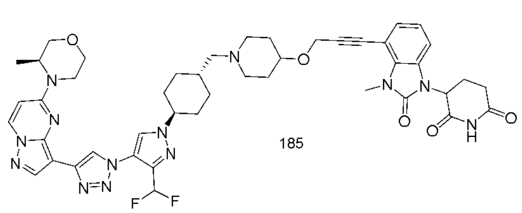 185 | 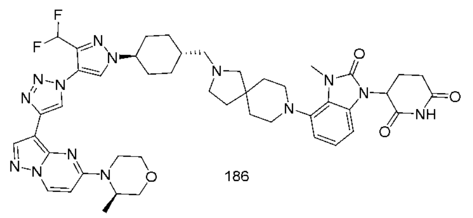 186 |
| 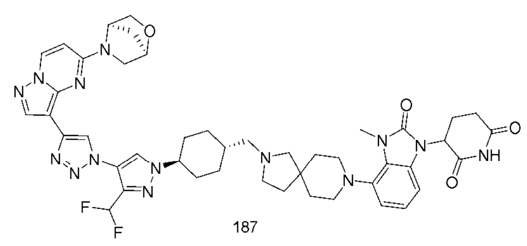 187 | 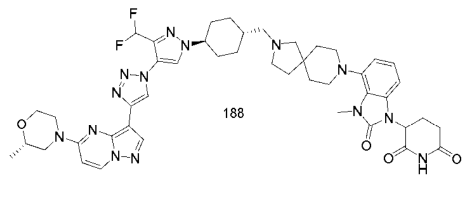 188 |
| 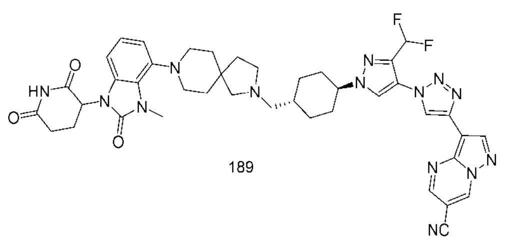 189 | 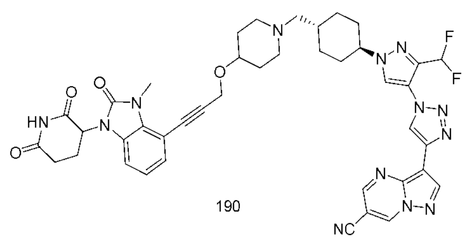 190 |
| 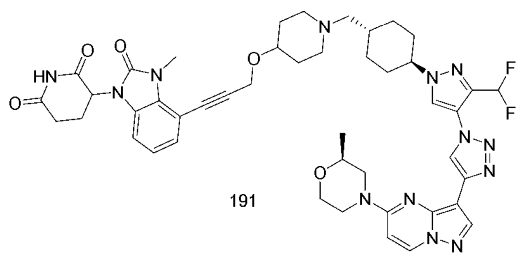 191 | 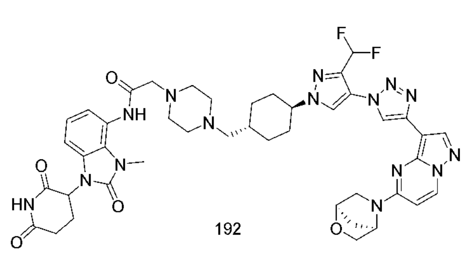 192 |

| | |
|---|---|
| 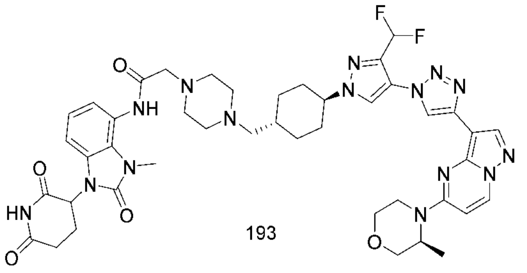 193 | 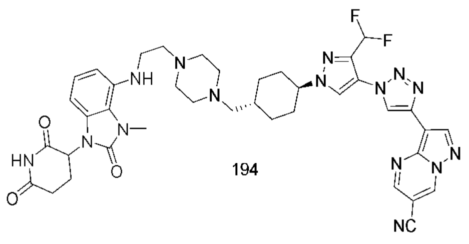 194 |
| 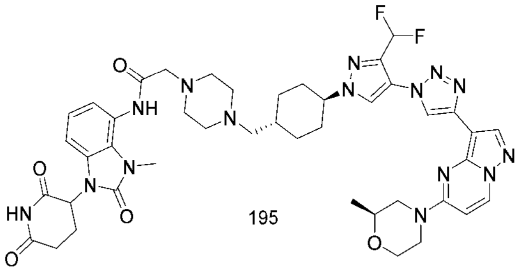 195 | 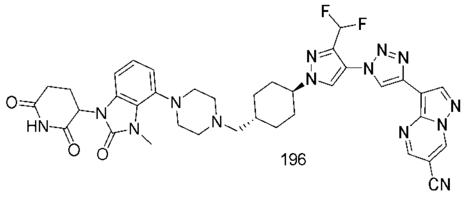 196 |
| 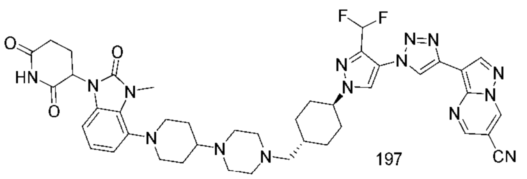 197 | 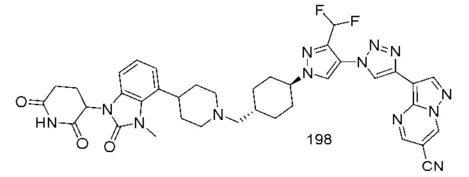 198 |
| 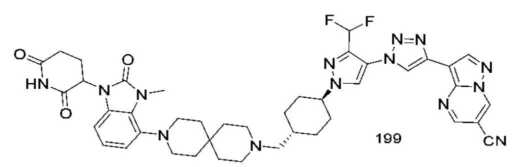 199 | 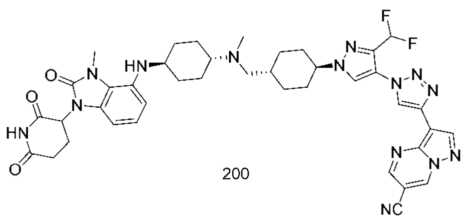 200 |
| 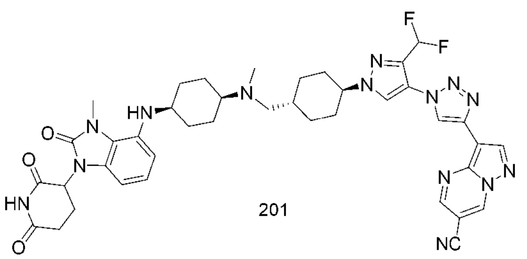 201 | 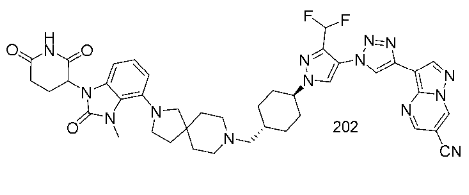 202 |
| 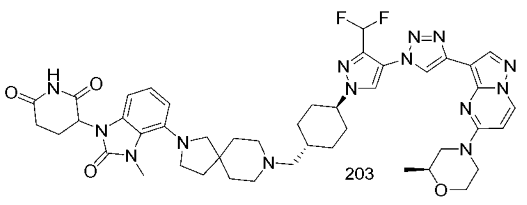 203 | 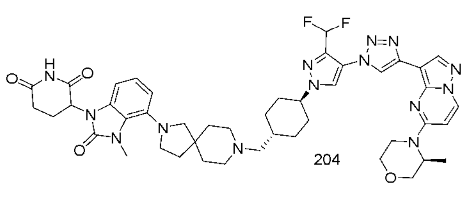 204 |
| 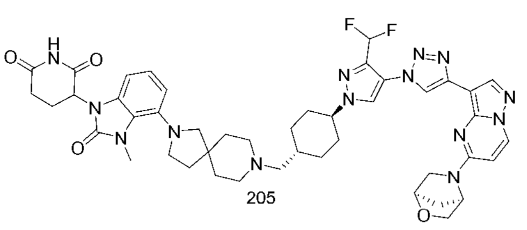 205 | 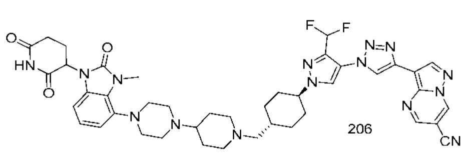 206 |

| | |
|---|---|
| 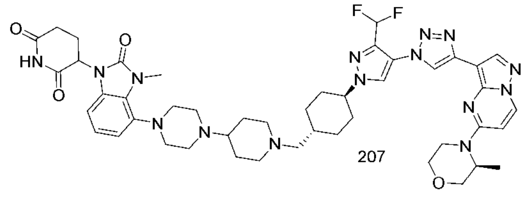 207 | 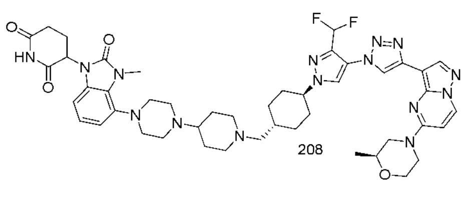 208 |
| 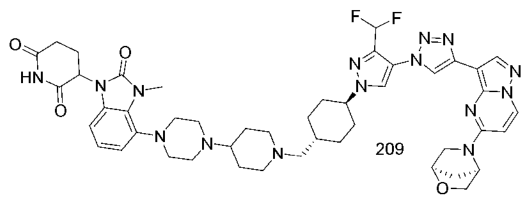 209 | 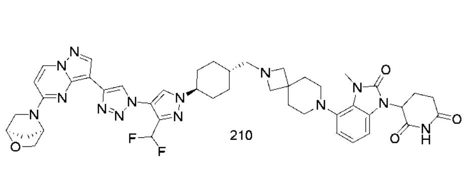 210 |
| 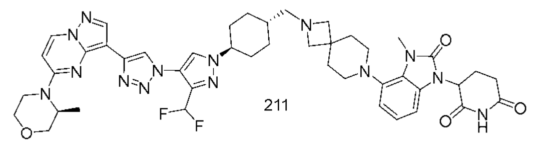 211 | 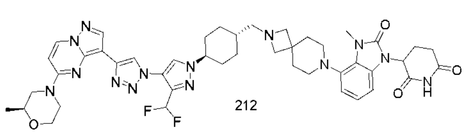 212 |
| 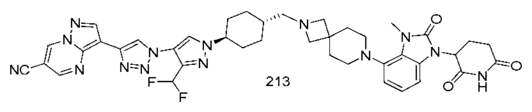 213 | 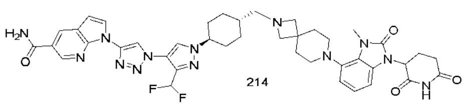 214 |
| 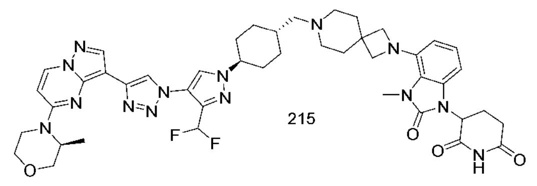 215 | 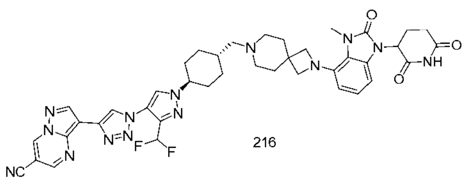 216 |
| 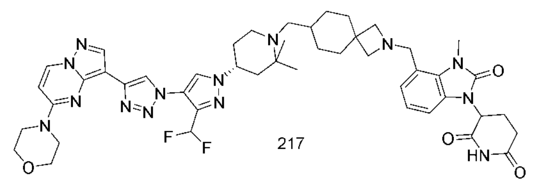 217 | 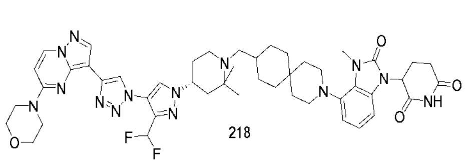 218 |
| 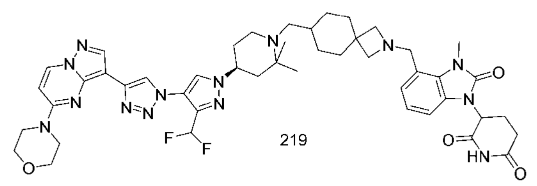 219 | 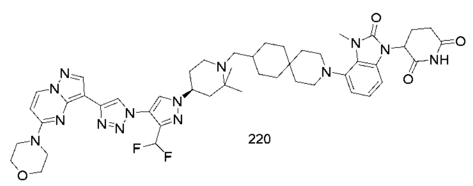 220 |
| 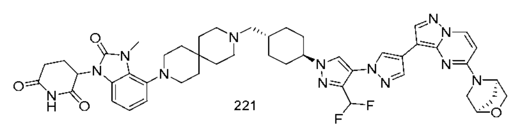 221 | 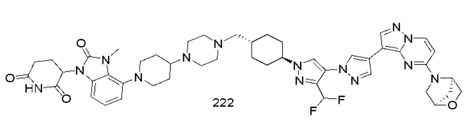 222 |
| 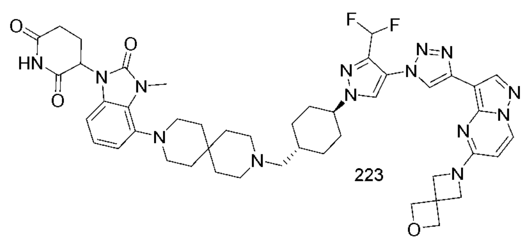 223 | 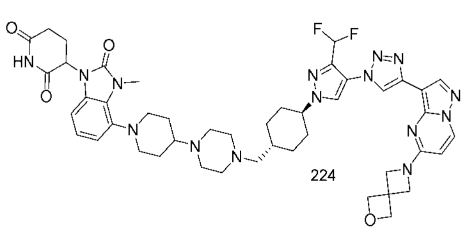 224 |

| | |
|---|---|
| 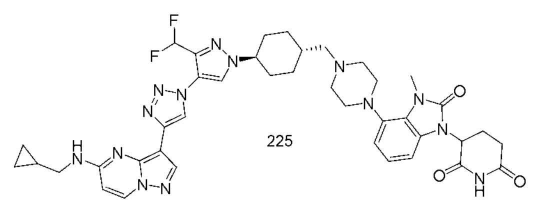 225 | 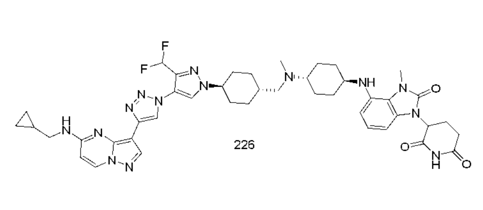 226 |
| 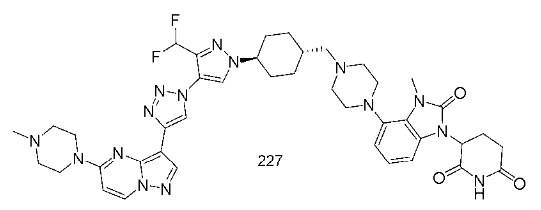 227 | 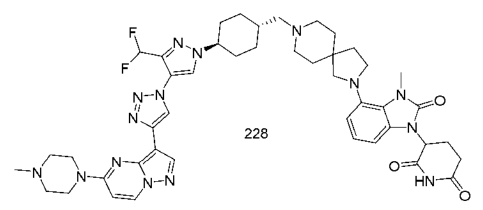 228 |
| 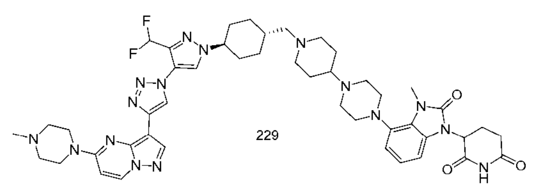 229 | 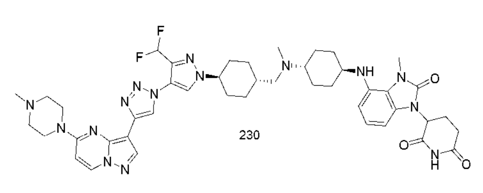 230 |
| 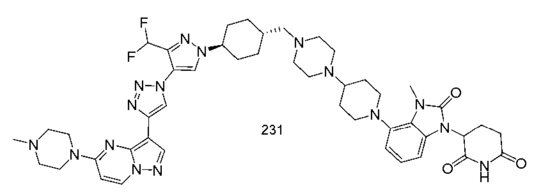 231 | 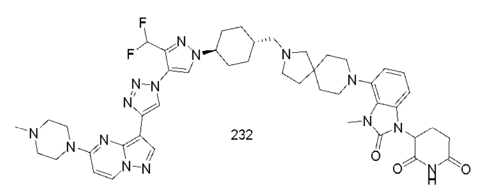 232 |
| 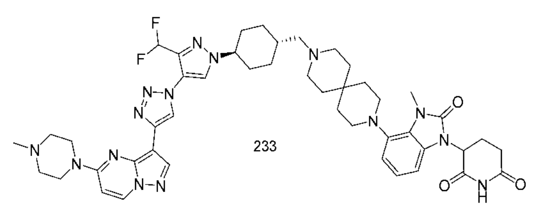 233 | 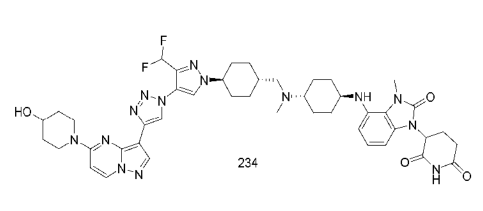 234 |
| 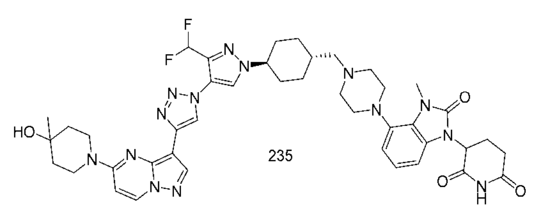 235 | 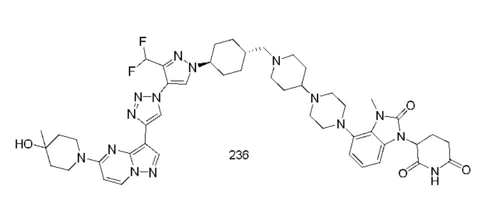 236 |
| 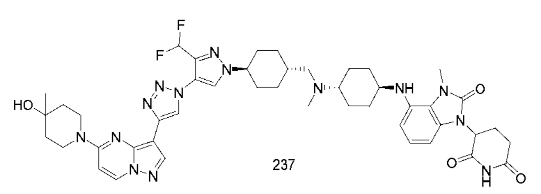 237 | 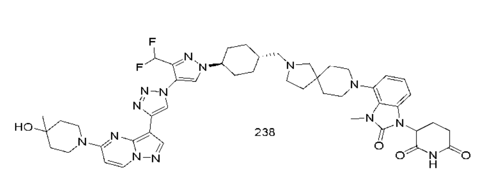 238 |
| 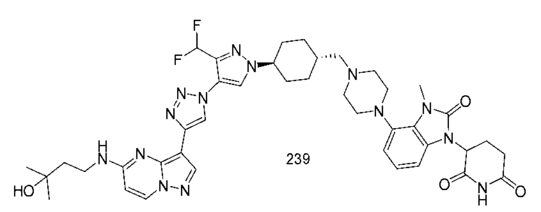 239 | 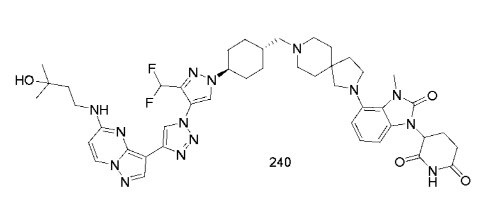 240 |

| | |
|---|---|
| 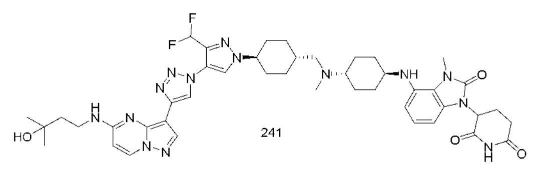 241 | 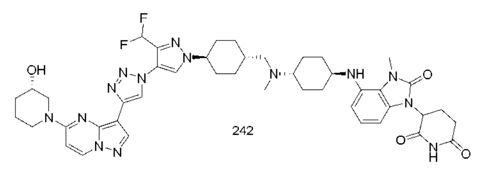 242 |
| 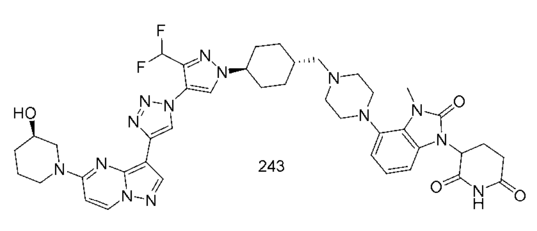 243 | 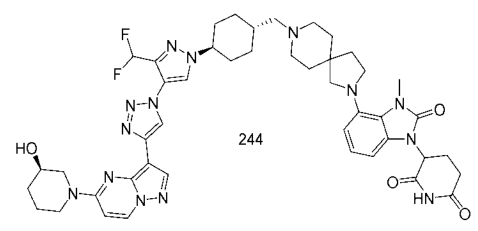 244 |
| 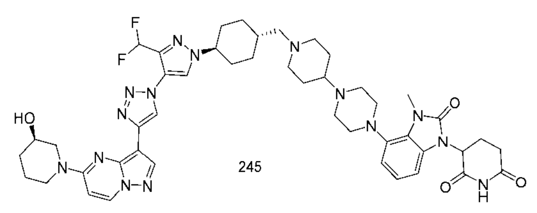 245 | 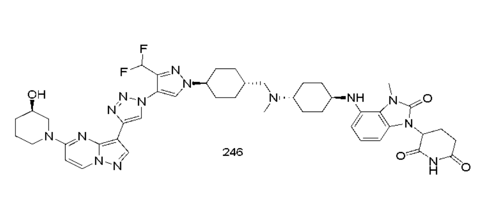 246 |
| 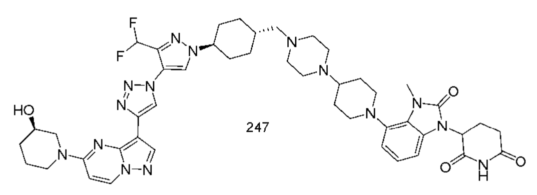 247 | 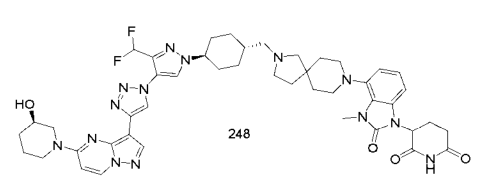 248 |
| 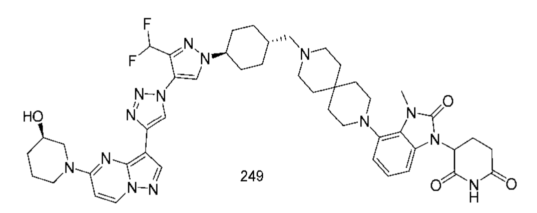 249 | 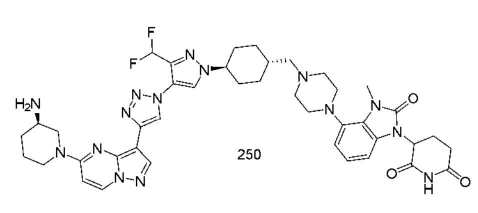 250 |
| 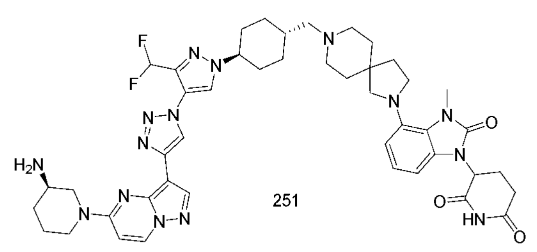 251 | 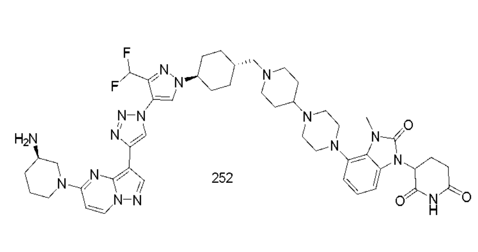 252 |
| 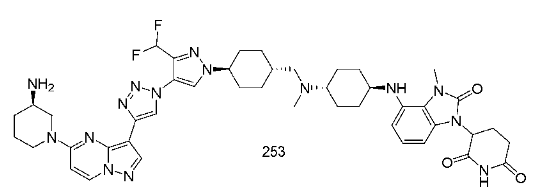 253 | 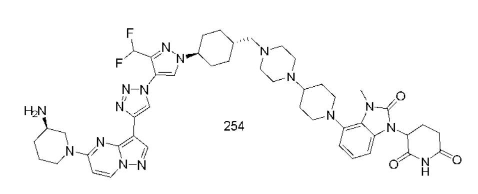 254 |
| 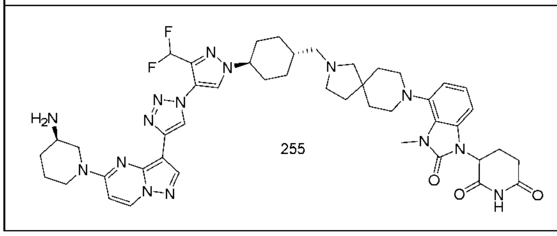 255 | 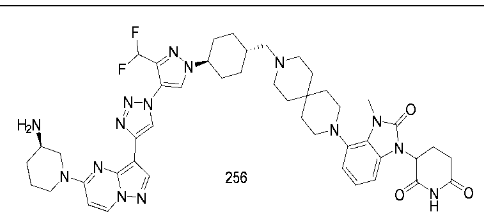 256 |

| | |
|---|---|
| 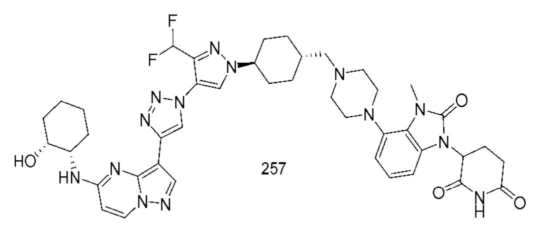 257 | 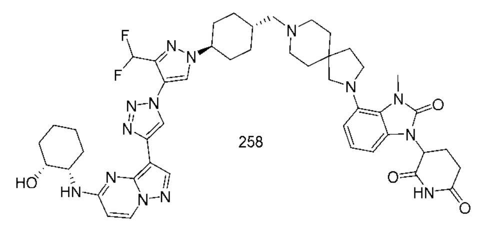 258 |
| 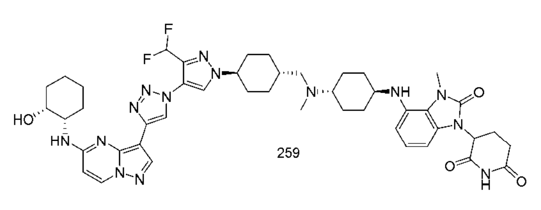 259 | 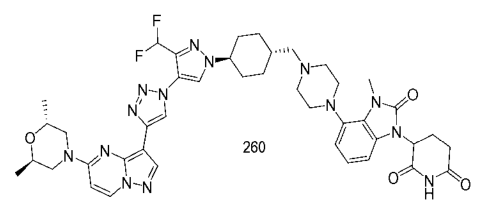 260 |
| 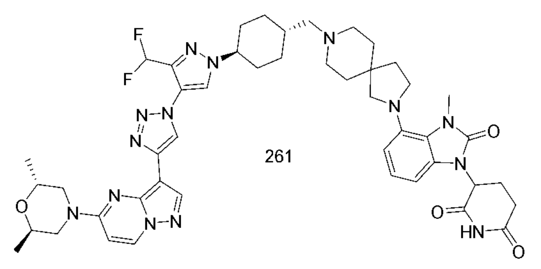 261 | 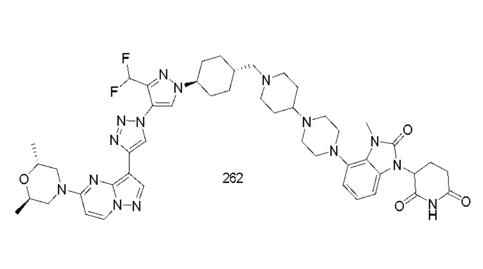 262 |
| 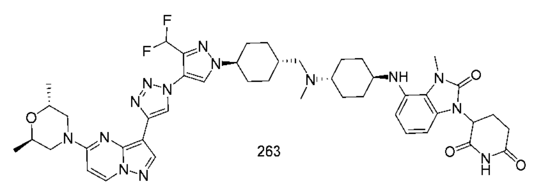 263 | 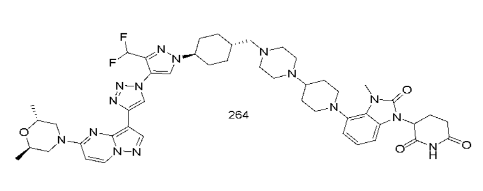 264 |
| 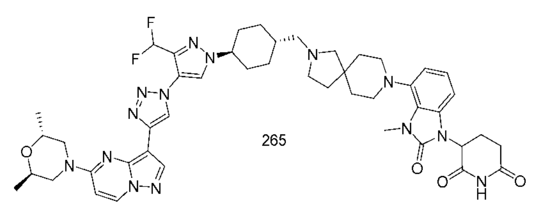 265 | 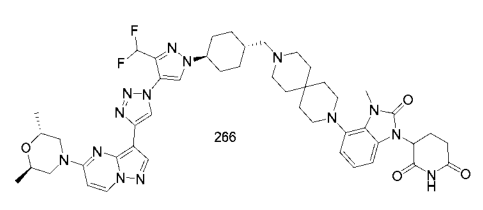 266 |
| 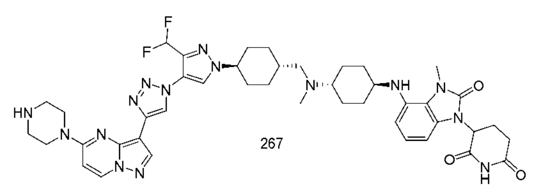 267 | 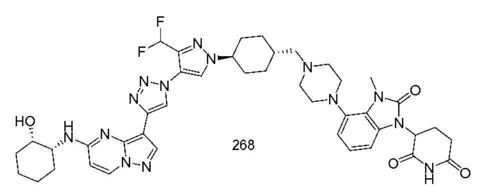 268 |
| 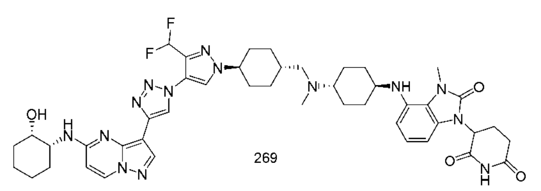 269 | 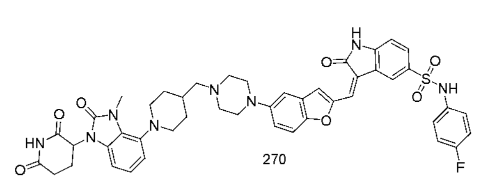 270 |
| 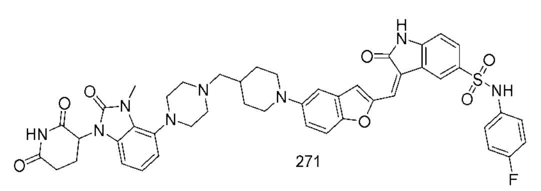 271 | 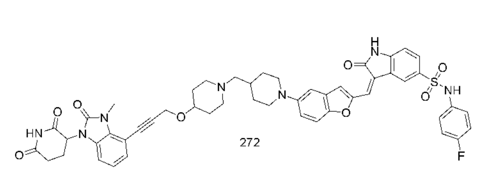 272 |

| | |
|---|---|
| 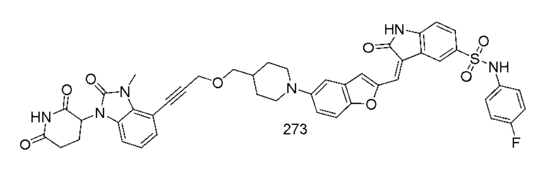 273 | 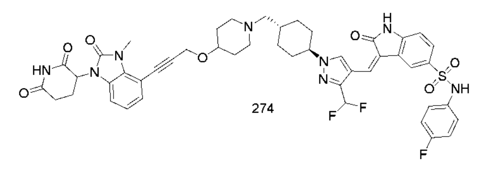 274 |
| 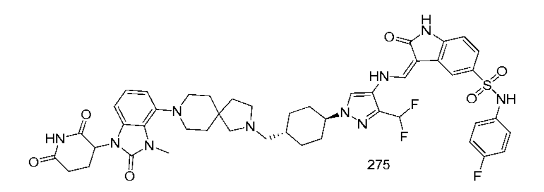 275 | 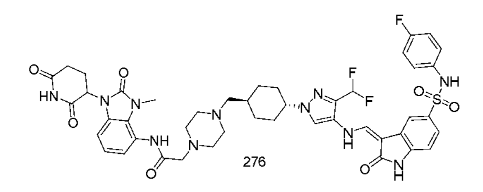 276 |
| 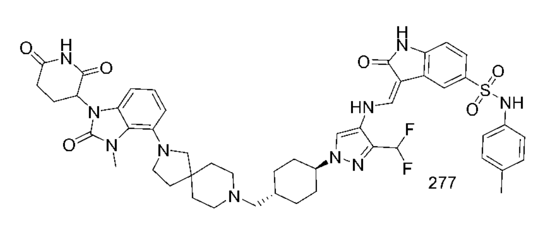 277 | 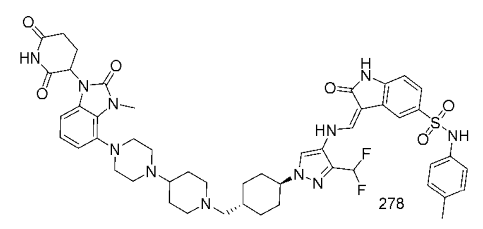 278 |
| 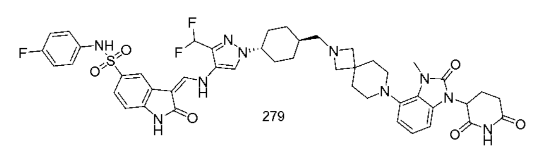 279 | 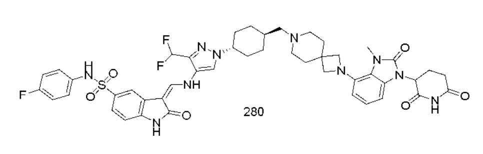 280 |
| 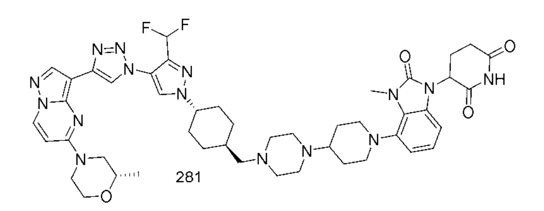 281 | 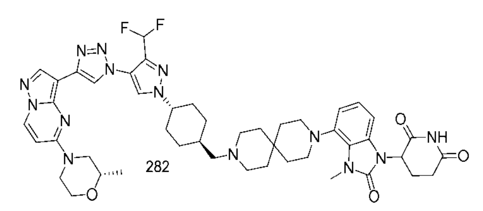 282 |
| 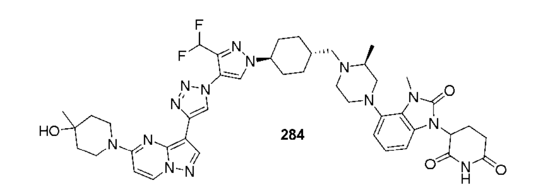 **284** | 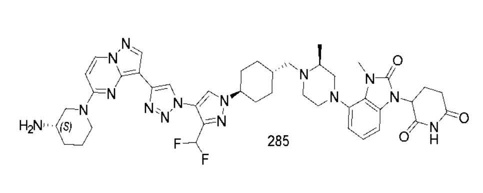 285 |
| 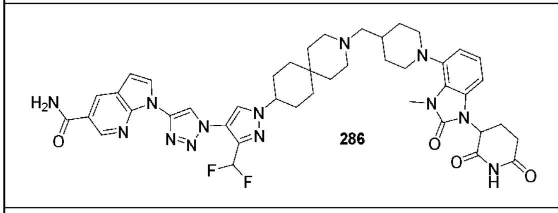 **286** | 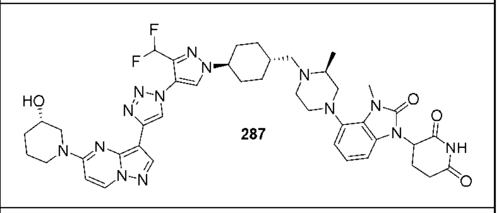 **287** |
| 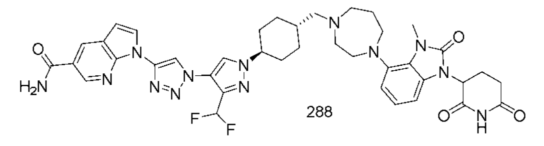 288 | 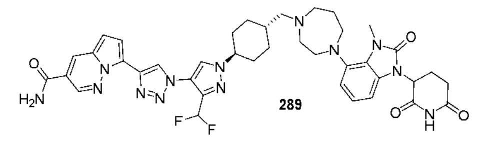 **289** |
| 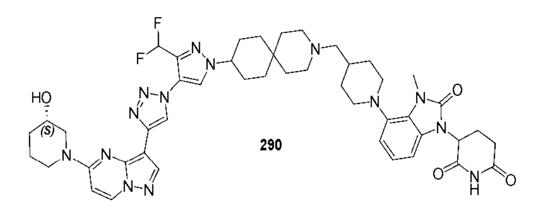 **290** | 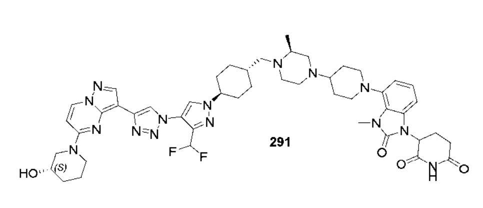 **291** |

| | |
|---|---|
| 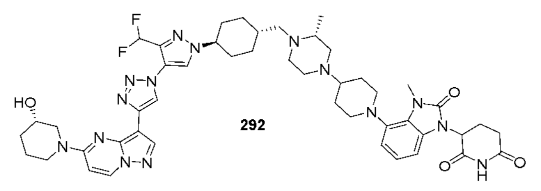 292 | 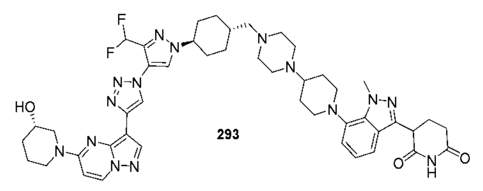 293 |
| 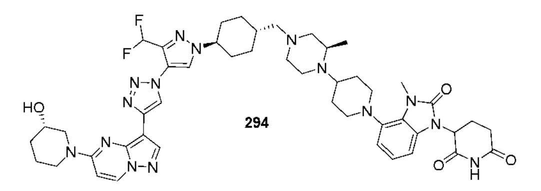 294 | 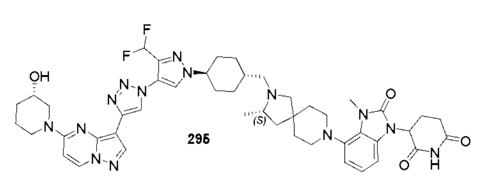 295 |
| 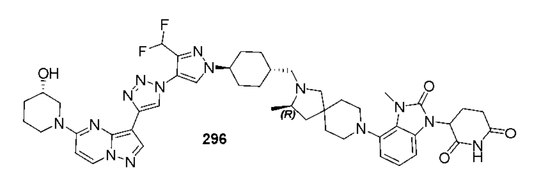 296 | 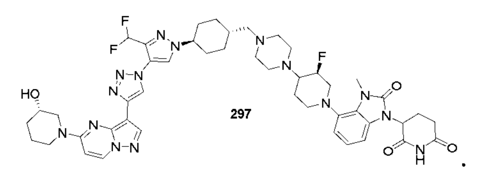 297 |

In some embodiments, the present disclosure provides the compound of formula (A), or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, wherein the compound is selected from compound 1 to compound 282.

In some preferred embodiments, the compound is selected from:

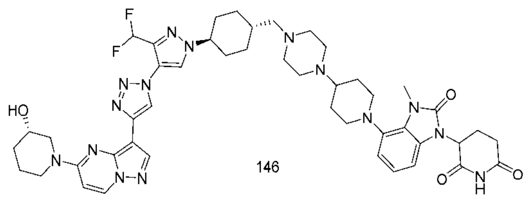

146

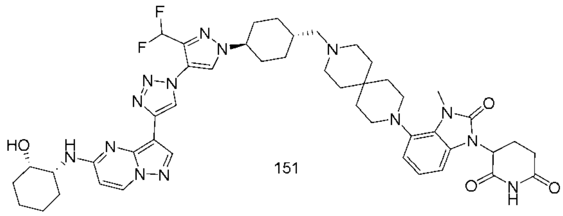

151

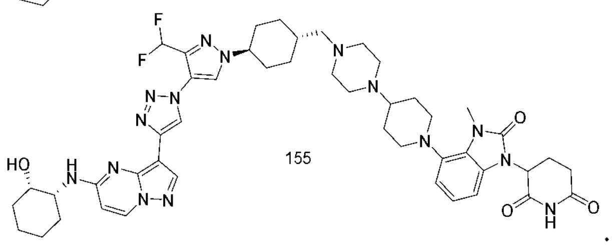

155

## Pharmaceutical composition and use

In another aspect, the present disclosure provides a pharmaceutical composition comprising a therapeutically effective amount of the compound of the present disclosure, or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, and one or more pharmaceutically acceptable carriers. The pharmaceutical composition is preferably a solid preparation, a liquid preparation or a transdermal preparation.

In another aspect, the present disclosure provides a use of the compound of the present disclosure, or the stereoisomer, the tautomer, the diastereomer, the racemate, the cis-trans isomer, the isotopically-labeled compound

(preferably deuterated compound), the N-oxide, the metabolite, the ester, the prodrug, the crystal form, the hydrate, the solvate or the pharmaceutically acceptable salt thereof, or the pharmaceutical composition of the present disclosure in the preparation of a medicament.

In some embodiments, the compound of the present disclosure, the pharmaceutical composition of the present disclosure, or the medicament are used to treat a disease, disorder or condition associated with kinase protein IRAK4.

In another aspect, the present disclosure further provides a method for treating, alleviating a symptoms of, or delaying development or onset of a disease, disorder or condition associated with kinase protein IRAK4, comprising administering to an individual in need thereof an effective amount of the compound of the present disclosure or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, or the pharmaceutical composition of the present disclosure.

In yet another aspect, the present disclosure provides the compound of the present disclosure or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, or the pharmaceutical composition of the present disclosure for treating a disease, disorder or condition associated with kinase protein IRAK4.

In another aspect, the present disclosure further provides a use of the compound of the present disclosure or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, or the pharmaceutical composition of the present disclosure in the preparation of a medicament as an IRAK4 inhibitor.

In yet another aspect, the present disclosure provides a method for inhibiting IRAK4 activity in an individual, comprising administering to an individual in need thereof an effective amount of the compound of the present disclosure or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof, or the pharmaceutical composition of the present disclosure.

In another aspect, the present disclosure provides a method for targeted degradation of kinase protein IRAK4, comprising contacting the kinase protein IRAK4 with the compound of formula (A) of the present disclosure or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof in the presence of an E3 ubiquitin ligase. In some embodiments, the method is performed in vitro or ex vivo. In some other embodiments, the method is performed in vivo.

In some embodiments, the disease, disorder or condition associated with kinase protein IRAK4 is selected from: autoimmune condition, inflammatory condition, cancer, transplant rejection, thromboembolism, atherosclerosis, myocardial infarction and metabolic syndrome.

In some embodiments, the inflammatory condition is selected from: osteoarthritis, gout, gouty arthritis, chronic obstructive pulmonary disease, periodic fever, atopic dermatitis, hidradenitis suppurativa, chronic nephritis, allergic eczema, lymphadenectasis, sepsis, irritable bowel syndrome (IBD), ulcerative colitis, asthma and allergies, preferably osteoarthritis, chronic obstructive pulmonary disease, atopic dermatitis, hidradenitis suppurativa and chronic nephritis.

In some embodiments, the autoimmune condition is selected from: Crohn’s disease, rheumatoid arthritis, systemic lupus erythematosus, lupus nephritis, cutaneous lupus, psoriasis, psoriatic arthritis, multiple sclerosis, neuropathic pain, ankylosing spondylitis, reactive arthritis and systemic juvenile idiopathic arthritis, preferably psoriasis.

In some embodiments, the transplant rejection is selected from graft-versus-host disease and allogeneic transplant rejection.

In some embodiments, the cancer is selected from: brain cancer, kidney cancer, liver cancer, stomach cancer, vaginal cancer, ovarian cancer, stomach tumor, breast cancer, bladder cancer, colon cancer, prostate cancer, pancreatic cancer, lung cancer, cervical cancer, testicular cancer, skin cancer, bone cancer, thyroid cancer, sarcoma, glioblastoma, neuroblastoma, gastrointestinal cancer, neck and head tumor, adenoma, adenocarcinoma, keratoacanthoma, epidermoid carcinoma, large cell carcinoma, non-small cell lung cancer, Hodgkin and non-Hodgkin lymphoma, mastocarcinoma, follicular carcinoma, papillary carcinoma, seminoma, melanoma, acute myeloid leukemia, chronic myeloid leukemia, diffuse large B-cell lymphoma, activated B-cell-like diffuse large B-cell lymphoma, chronic lymphocytic leukemia, chronic lymphocytic lymphoma, primary effusion lymphoma, Burkitt’s lymphoma/leukemia, acute lymphocytic leukemia, B-cell prolymphocytic leukemia, lymphoplasmacytic lymphoma, Waldenstrom’s macroglobulinemia, splenic marginal zone lymphoma, intravascular large B-cell lymphoma, plasmacytoma, and multiple myeloma.

In the present disclosure, "pharmaceutically acceptable carrier" refers to a diluent, adjuvant, excipient or vehicle that is administered with a therapeutic agent and is suitable for contacting the tissues of humans and/or other animals without excessive toxicity, irritation, allergic reaction or other problems or complications corresponding to a reasonable benefit/risk ratio within the scope of reasonable medical judgment.

Unless otherwise specified, as used herein, the term "treating" means reversing, alleviating or inhibiting the disorders or conditions to which such term applies, or the progression of one or more symptoms of such disorders or conditions, or preventing such disorders or conditions, or one or more symptoms of such disorders or conditions.

As used herein, "individual" includes human or non-human animals. Exemplary human individuals include human individuals suffering from diseases (e.g., diseases described herein) (referred to as patients) or normal individuals. In the present disclosure, "non-human animals" include all vertebrates, such as non-mammals (e.g., birds, amphibians, reptiles) and mammals, such as non-human primates, livestock and/or domesticated animals (e.g., sheep, dogs, cats, cows, pigs, etc.).

In another embodiment, the pharmaceutical composition of the present disclosure may further comprise one or more additional therapeutic or prophylactic agents.

## Example

Embodiments of the present disclosure will be described in detail below with reference to examples; however, those skilled in the art will understand that the following examples are only used to illustrate the present disclosure and should not be regarded as limiting the scope of the present disclosure. If no specific conditions are specified in the examples, they are carried out according to conventional conditions or conditions recommended by the manufacturer. If manufacturers of reagents or instruments used are not specified, they are conventional products commercially available.

NMR was measured using a Bruker Avance III 400 nuclear magnetic spectrometer, and the chemical shift ($\delta$) was given in units of $10^{-6}$(ppm). The solvent was deuterated methanol ($CD_3OD$), deuterated chloroform ($CDCl_3$) or hexadeuterated dimethyl sulfoxide (DMSO-d6), etc., and the internal standard was tetramethylsilane (TMS).

MS was measured using an Agilent (ESI) mass spectrometer (Agilent 1260, Agilent 6125B).

High performance liquid chromatography (HPLC) measurement conditions: Gilson high pressure liquid chromatograph (Gilson GX-281), C18 column (10 $\mu$M, 19 mm $\times$ 250 mm), with UV detection band of 220 nm and 254 nm, elution condition of 5%-95% acetonitrile (containing 0.05% v/v formic acid or ammonium bicarbonate) gradient elution for 15 min.

Biotage Isolera rapid purification system was used for reverse phase purification.

Thin layer chromatography silica gel plate (aluminum plate (20 cm $\times$ 20 cm $\times$ 1 mm) produced by Meck, or GF 254 produced in Yantai) was used for thin layer chromatography separation and purification.

Biotage Initiator + (400 W, RT ~ 300°C) microwave reactor was used for reaction under microwave.

TLC or LCMS was commonly used for reaction monitoring, and the commonly used developing solvent systems were: dichloromethane/methanol, n-hexane/ethyl acetate, petroleum ether/ethyl acetate, and the volume ratio of the solvents was adjusted according to the polarity of the compound or adjusted by adding triethylamine, etc.

The silica gel used in column chromatography was generally 100 - 200 mesh silica gel. Commonly used eluent systems were: dichloromethane/methanol, petroleum ether/ethyl acetate, and the volume ratio of the solvents was adjusted according to the polarity of the compound, and or adjusted by adding a small amount of triethylamine.

The reagents and solvents of the present disclosure were purchased from Aldrich Chemical Company, Energy Chemical, J&K Scientific, Bide Pharmatech Ltd., PharmaBlock Sciences (Nanjing), Inc. and Shanghai Titan Scientific Co., Ltd.

## Synthesis example

Example 1 : 3-(4-((R)-3-((1-(((1r,4R)-4-(3-(difluoromethyl)-4-(4-(5-morpholinopyrazolo[1,5-a]pyrimidin-3-yl)-1H-1,2,3-triazol-1-yl)-1H-pyrazol-1-yl)cyclohexyl)methyl)piperidin-4-yl)oxy)but-1-yn-1-yl)-3-methyl-2-oxo-2,3-dihydro-1H-benzo [d]imidazol-1-yl)piperidine-2,6-dione (1)

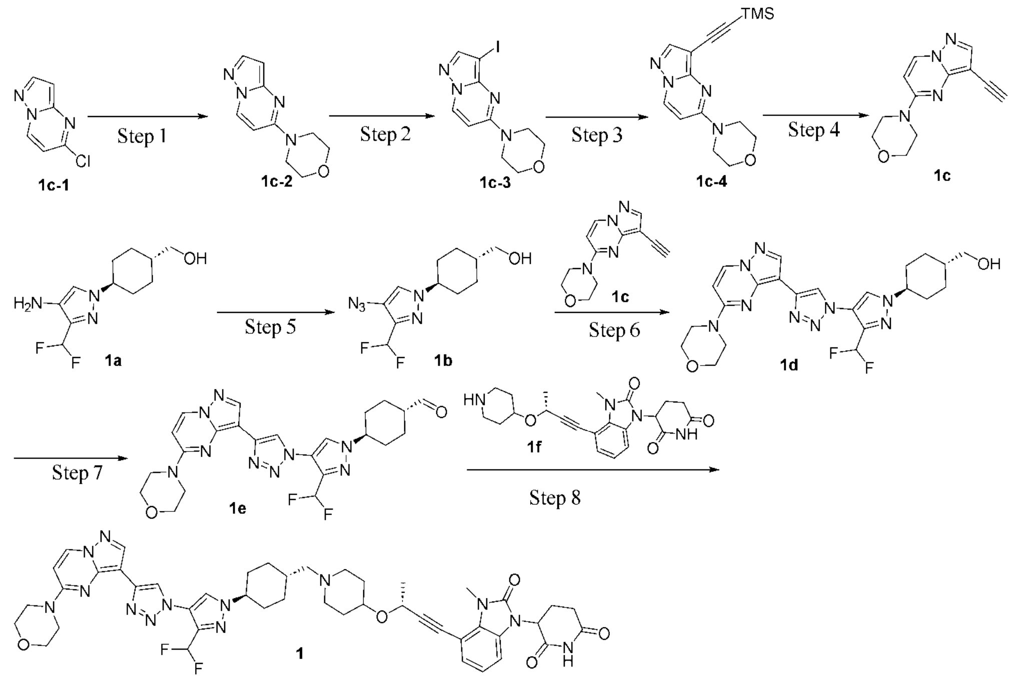


1) Step 1: Compound 1c-1 (5.00 g, 32.56 mmol) and acetonitrile (50 mL) were added to a 250 mL single-neck bottle in sequence at room temperature and stirred until dissolved. Morpholine (4.25 g, 48.8 mmol) and *N, N*-diisopropylethylamine (12.6 g, 97.7 mmol) were added in sequence. Nitrogen replacement was performed 3 times, and the reaction solution was stirred at 80°C for 18 h. The reaction solution was concentrated under reduced pressure, and the residue was purified by column chromatography (dichloromethane/methanol = 100/1) to obtain compound 1c-2. MS m/z(ESI):205.4[M+H]$^+$.

2) Step 2: Compound 1c-2 (2.00 g, 9.79 mmol) and acetonitrile (25 mL) were added to a 100 mL single-neck bottle at room temperature and stirred until dissolved. N-iodosuccinimide (3.30 g, 14.7 mmol) was added, nitrogen replacement was performed 3 times, and the reaction solution was stirred at 25°C for 18 h. The reaction solution was concentrated under reduced pressure, and the residue was purified by column chromatography (dichloromethane/-methanol = 100/1) to obtain compound 1c-3. MS m/z(ESI):330.9[M+H]$^+$.

3) Step 3: Compound 1c-3 (500 mg, 1.51 mmol) and tetrahydrofuran (10 mL) were added to a 100 mL single-neck bottle in sequence at room temperature and stirred until dissolved. Trimethylsilylacetylene (1.07 mL, 7.57 mmol), cuprous iodide (28.8 mg, 0.150 mmol), triethylamine (460 mg, 4.54 mmol) and bistriphenylphosphine palladium dichloride (106.31 mg, 0.15 mmol) were added in sequence. Nitrogen replacement was performed 3 times, and the reaction solution was stirred at 30°C for 18 h. The reaction solution was concentrated under reduced pressure and the residue was purified by column chromatography (dichloromethane/petroleum ether = 50/1) to obtain compound 1c-4. MS m/z(ESI):300.8[M+H]$^+$.

4) Step 4: Compound 1c-4 (300 mg, 1.00 mmol) and anhydrous methanol (10 mL) were added to a 50 mL single-neck bottle in sequence at room temperature and stirred until dissolved. Potassium carbonate (276 mg, 2.00 mmol) was added, and the reaction solution was stirred at 25°C for 1 h. The obtained mixture was concentrated under reduced pressure, water (10 mL) was added to the residue, and the obtained mixture was extracted with ethyl acetate (30 mL × 3), the organic phases were combined, washed with brine (20 mL), dried over sodium sulfate, filtered, and the filtrate was distilled under reduced pressure to obtain a product containing compound 1c, which was directly used for subsequent reaction. MS m/z(ESI):228.9[M+H]$^+$.

5) Step 5: Compound 1a (3.00 g, 12.2 mmol, prepared by the method disclosed for the intermediate step 6 compound on page 137 of the specification of the patent application "WO 2022161414 A1") was dissolved in acetonitrile (35 mL), and isoamyl nitrite (2.10 g, 17.9 mmol, Bide Pharmatech Ltd.) was added at 0°C. After the reaction solution was stirred for 30 min, azidotrimethylsilane (2.00 g, 17.4 mmol) was added. The reaction solution was stirred at 25°C for 18 h. The reaction solution was poured into water (30 mL), and the aqueous phase was extracted with ethyl acetate (30 mL × 3). The combined organic phase was washed with saturated brine (50 mL), dried over anhydrous sodium sulfate and filtered. The filtrate was concentrated under reduced pressure to obtain compound 1b. MS m/z(ESI):272.1[M+H]$^+$.

6) Step 6: Compound 1b (2.20 g, 8.11 mmol) was dissolved in ethanol (30 mL) and water (10 mL). Sodium ascorbate (0.33 g, 1.67 mmol, Energy Chemical), copper sulfate pentahydrate (0.41 g, 1.64 mmol, Energy Chemical) and compound 1c (1.86 g, 8.15 mmol) were added to the reaction solution in sequence. The reaction solution was stirred at

25°C for 18 h. The reaction solution was filtered, the solid was collected and slurried with a mixed solvent (petroleum ether/ethyl acetate = 1/1, 30 mL) to obtain compound 1d. $^1$H NMR (400 MHz, DMSO-$d_6$): δ 8.76 (d, *J* = 7.9 Hz, 1H), 8.66 (s, 1H), 8.53 (s, 1H), 8.38 (s, 1H), 7.20 (t, *J* = 53.2 Hz, 1H), 6.81 (d, *J* = 7.9 Hz, 1H), 4.50 (s, 1H), 4.27 (t, *J* = 11.9 Hz, 1H), 3.73 (s, 8H), 3.28 (d, *J* = 5.7 Hz, 2H), 2.19 - 2.07 (m, 2H), 1.93 - 1.76 (m, 4H), 1.54 - 1.38 (m, 1H), 1.20 - 1.07 (m, 2H).

7) Step 7: Compound 1d (3.20 g, 6.41 mmol) was dissolved in dichloromethane (50 mL), and Dess-Martin oxidant (4.10 g, 9.67 mmol) was added. The reaction solution was stirred at 25°C for two hours. The reaction solution was poured into water (80 mL), and the aqueous phase was extracted with dichloromethane (50 mL × 3). The combined organic phase was washed with saturated brine (100 mL), dried over anhydrous sodium sulfate and filtered. The filtrate was concentrated under reduced pressure, and the residue was slurried with a mixed solvent (petroleum ether/ethyl acetate = 1/1, 30 mL) to obtain compound 1e. $^1$H NMR (400 MHz, DMSO-$d_6$): δ 9.63 (s, 1H), 8.76 (d, *J* = 7.9 Hz, 1H), 8.67 (s, 1H), 8.54 (s, 1H), 8.38 (s, 1H), 7.20 (t, *J* = 53.2 Hz, 1H), 6.81 (d, *J* = 7.9 Hz, 1H), 4.39 - 4.26 (m, 1H), 3.73 (s, 8H), 2.45 - 2.36 (m, 1H), 2.24 - 2.16 (m, 2H), 2.16 - 2.07 (m, 2H), 1.94 - 1.84 (m, 2H), 1.49 - 1.35 (m, 2H).

8) Step 8: Compound 1f (50.0 mg, 0.12 mmol, prepared by the method disclosed for the intermediate AVF on page 1148 of the specification of the patent application "CN 113423427") was dissolved in a mixed solution of tetrahydrofuran (1 mL) and N,N-dimethylformamide (1 mL). Triethylamine (0.03 mL, 0.23 mmol), acetic acid (0.03 mL, 0.45 mmol), sodium triacetoxyborohydride (25.8 mg, 0.12 mmol), and compound 1e (60.6 mg, 0.12 mmol) were added and reacted under stirring for 2 h. Water (5 mL) was added to the reaction solution, and the obtained mixture was extracted with ethyl acetate (5 mL×3). The organic phases were combined, dried over anhydrous sodium sulfate and filtered to remove the desiccant, and then the filtrate was concentrated under reduced pressure. The crude product was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 1. MS m/z(ESI):892.7[M+1]$^+$. $^1$H NMR(400MHz, $CDCl_3$): δ 8.61 (s, 1H), 8.57 (s, 1H), 8.38 (d, J=7.6 Hz, 1H), 8.11 (s, 1H), 7.25 - 7.20 (m, 1H), 7.18 - 6.77 (m, 3H), 6.39 (d, *J* = 7.6 Hz, 1H), 5.27 - 5.20 (m, 1H), 4.60 - 4.50 (m, 1H), 4.25 - 4.15 (m, 1H), 3.87 - 3.76 (m, 13H), 2.95 - 2.81 (m, 5H), 2.28 - 2.21 (m, 10H), 1.81 - 1.61 (m, 4H), 1.56 - 1.54 (m, 6H), 1.27 - 1.00 (m, 1H).

Example 2: 3-(4-(3-((S)-2-((((4-(-3-(difluoromethyl)-4-(4-(5-morpholinopyrazolo [1,5-a]pyrimidin-3-yl)-1H-1,2,3-triazol-1-yl)-1H-pyrazol-1-ylcyclohexyl)methyl) amino)methyl)morpholinyl)propyl)-3-methyl-2-oxo-2,3-dihydro-1H-benzo [d]imidazol-1-yl)piperidine-2,6-dione (2)

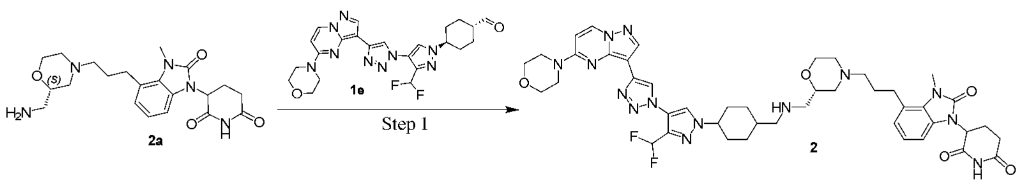


Compound 2a (80.0 mg, 0.190 mmol, prepared by the method disclosed for the intermediate SR on page 934 of the specification of the patent application "US 20190192668 A1") and compound 1e (95.8 mg, 0.190 mmol) were dissolved in ethanol (2 mL). Acetic acid (0.10 mL, 1.75 mmol) was added, and the reaction solution was stirred at 25°C for 10 min, and sodium cyanoborohydride (48.4 mg, 0.770 mmol) was added. The reaction solution was stirred for 2 h. The reaction solution was filtered, and the residue was concentrated under reduced pressure and purified by high performance liquid chromatography (Waters-2545, chromatographic column: SharpSil-TC18, 30*150 mm, 5 μm; mobile phase: water (containing 10 mmol/L formic acid) and acetonitrile, gradient ratio: acetonitrile 38%-45%, flow rate: 30 mL/min) to obtain compound 2. MS m/z(ESI):897.6[M+1]$^+$.

Example 3: 3-(4-((((1-(((1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-yl)cyclohexyl)methyl)piperidin-4-yl)methyl)(methyl)amino)methyl)-3-methyl-2-oxo-2,3-dihydro-1H-benzo[d]imidazol-1-yl)piperidine-2,6-dione (3)

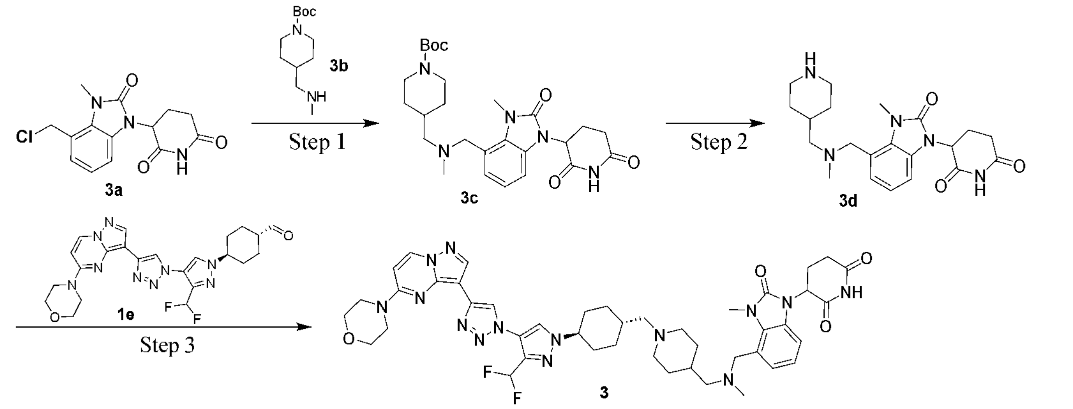


1) Step 1: Compound 3a (100 mg, 0.320 mmol, prepared by the method disclosed for the intermediate B32-2 on page 574 of the specification of the patent application "WO 2020206424 A1") was dissolved in acetonitrile (5 mL), and compound 3b (74.0 mg, 0.320 mmol, Bide Pharmatech Ltd.) and potassium carbonate (135 mg, 0.970 mmol) were added. After the reaction solution was stirred at 80°C for 2 h, water (10 mL) was added to the reaction solution, and the aqueous phase was extracted with ethyl acetate (10 mL × 3). The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 20/1) to obtain compound 3c.

2) Step 2: Compound 3c (50 mg, 0.100 mmol) was dissolved in dichloromethane (3 mL), and trifluoroacetic acid (3 mL) was added. The reaction solution was stirred at 25°C for 1 h, and then concentrated under reduced pressure to obtain compound 3d, the residue was directly used as the raw material for the next step without purification. MS m/z(ESI):400.2[M+1]+.

3) Step 3: Compound 3d (35.0 mg, 90.0 μmol) and compound 1e (45.0 mg, 90.0 μmol) were dissolved in tetrahydrofuran (2 mL) and N,N-dimethylformamide (1 mL), and acetic acid (0.1 mL) and sodium borohydride acetate (60.0 mg, 0.280 mmol) were added in sequence. The reaction solution was stirred at 25°C for 2 h. The reaction solution was poured into water (10 mL) and dichloromethane (10 mL × 3) was used for extraction. The combined organic phase was washed with saturated brine (20 mL), dried over anhydrous sodium sulfate and filtered. The filtrate was concentrated under reduced pressure, and then purified by high performance liquid chromatography (Waters-2545, chromatographic column: SharpSil-TC18, 30*150 mm, 5 μm; mobile phase: water (containing 10 mmol/L formic acid) and acetonitrile, gradient ratio: acetonitrile 38%-45%, flow rate: 30 mL/min) to obtain compound 3. MSm/z(ESI):441.6 [M/2+1]+.

Example 4: 3-(4-(2-(((1R,4R)-4-(3-difluoromethyl)-4-(4-(5-morpholinylpyrazolo [1,5-a]pyrimidin-3-yl)-1H-1,2,3-triazol-1-yl)-1H-pyrazol-1-yl)cyclohexyl)methyl) amino)-7-azaspiro[3.5]nonan-7-yl)methyl)-3-methyl-2-oxo-2,3-dihydro-1H-benzoimidazol-1-yl)piperidine-2,6-dione (4)

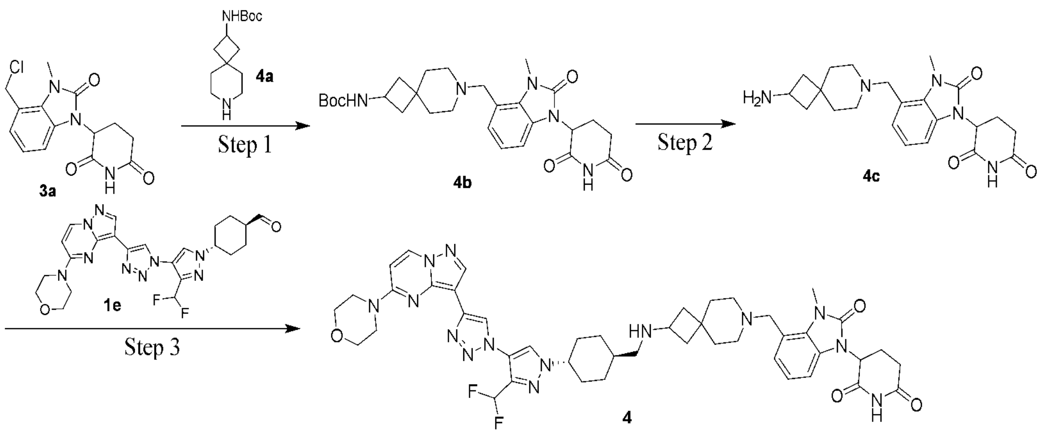


1) Step 1: Compound 3a (160 mg, 0.520 mmol, prepared by the method disclosed for the intermediate B32-2 on page

574 of the specification of the patent application "WO 2020206424 A1") and compound 4a (162 mg, 0.680 mmol, Bide Pharmatech Ltd.) were dissolved in acetonitrile (6 mL), and potassium carbonate (138 mg, 1.04 mmol) was added. The reaction solution was heated to 80°C under nitrogen atmosphere and reacted for 2 h. The reaction solution was poured into water (10 mL), and ethyl acetate (10 mL × 3) was used for extraction. The combined organic phase was washed with saturated brine (20 mL), dried over anhydrous sodium sulfate and filtered. The filtrate was concentrated under reduced pressure, and the residue was purified by silica gel column chromatography with an eluent system (dichloromethane/methanol = 20/1 to 10/1) to obtain compound 4b. MS m/z(ESI):512.2[M+1]$^+$.

2) Step 2: Compound 4b (120 mg, 0.230 mmol) was dissolved in dichloromethane (3 mL), and trifluoroacetic acid (1 mL) was added. The reaction solution was stirred at 25°C for 1 h. The reaction solution was concentrated under reduced pressure to obtain compound 4c. MS m/z(ESI):412.2[M+1]$^+$.

3) Step 3: Compound 4c (60.0 mg, 0.150 mmol) and compound 1e (75.0 mg, 0.150 mmol) were dissolved in tetrahydrofuran (2 mL) and N,N-dimethylformamide (1 mL), and acetic acid (0.1 mL) and sodium borohydride acetate (90.0 mg, 0.430 mmol) were added in sequence. The reaction solution was stirred at 25°C for 2 h. The reaction solution was poured into water (10 mL) and dichloromethane (10 mL × 3) was used for extraction. The combined organic phase was washed with saturated brine (20 mL), dried over anhydrous sodium sulfate and filtered. The filtrate was concentrated under reduced pressure, and the residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 4. MS m/z(ESI):893.6[M+1]$^+$.

Example 5: 3-(4-(4-(1R,4R)-4-(3-difluoromethyl)-4-(4-(5-morpholinylpyrazolo [1,5-a]pyrimidin-3-yl)-1H-1,2,3-triazol-1-yl)-1H-pyrazol-1-yl)cyclohexyl)methyl)-1-oxa-4,9-diazaspiro[5.5]undecan-9-yl)methyl)-3-methyl-2-oxo-2,3-dihydro-1H-benzoimidazol-1-yl)piperidine-2,6-dione (5)

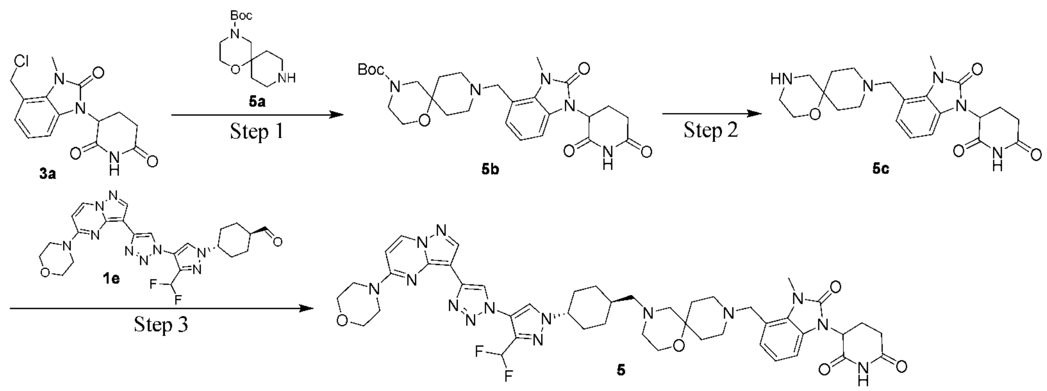


1) Step 1: Compound 3a (100 mg, 0.320 mmol, prepared by the method disclosed for the intermediate B32-2 on page 574 of the specification of the patent application "WO 2020206424 A1") and compound 5a (100 mg, 0.390 mmol, Bide Pharmatech Ltd.) were dissolved in acetonitrile (5 mL), and potassium carbonate (150 mg, 1.09 mmol) was added. The reaction solution was heated to 80°C under nitrogen atmosphere and reacted for 18 h. The reaction solution was poured into water (10 mL), and ethyl acetate (10 mL × 3) was used for extraction. The combined organic phase was washed with saturated brine (20 mL), dried over anhydrous sodium sulfate and filtered. The filtrate was concentrated under reduced pressure, and the residue was purified by silica gel column chromatography with an eluent system (dichloromethane/methanol = 20/1 to 10/1) to obtain compound 5b. MS m/z(ESI):528.7[M+1]$^+$.

2) Step 2: Compound 5b (100 mg, 0.190 mmol) was dissolved in dichloromethane (3 mL), and trifluoroacetic acid (1 mL) was added. The reaction solution was stirred at 25°C for 1 h. The reaction solution was concentrated under reduced pressure to obtain compound 5c. MS m/z(ESI):428.2[M+1]$^+$.

3) Step 3: Compound 5c (50.0 mg, 0.120 mmol) and compound 1e (65.0 mg, 0.130 mmol) were dissolved in tetrahydrofuran (2 mL) and N,N-dimethylformamide (1 mL), and acetic acid (0.1 mL) and sodium borohydride acetate (75.0 mg, 0.360 mmol) were added in sequence. The reaction solution was stirred at 25°C for 2 h. The reaction solution was poured into water (10 mL) and dichloromethane (10 mL × 3) was used for extraction. The combined organic phase was washed with saturated brine (20 mL), dried over anhydrous sodium sulfate and filtered. The filtrate was concentrated under reduced pressure, and the residue was purified by high performance liquid chromatography (A: 0.1% formic acid/water, B: acetonitrile, chromatographic column: Waters-SunFire-C18-10μm-19×250 mm; mobile

phase: water (containing 0.1% formic acid) and acetonitrile, gradient ratio: acetonitrile 32%-48%, flow rate: 25 mL/min) to obtain compound 5. MS m/z(ESI):909.4[M+1]$^+$.

Example 6: 3-(4-(4-((1R,4R)-4-(3-difluoromethyl)-4-(4-(5-morpholinylpyrazolo [1,5-a]pyrimidin-3-yl)-1H-1,2,3-triazol-1-yl)-1H-pyrazol-1-yl)cyclohexyl)methyl) amino)piperidin-1-carbonyl)-3-methyl-2-oxo-2,3-dihydro-1H-benzoimidazol-1-yl)piperidine-2,6-dione (6)

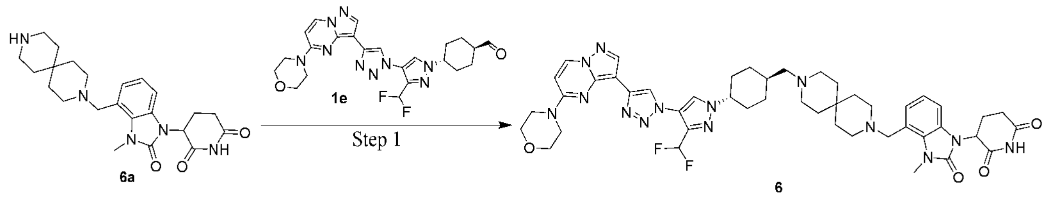


Compound 6a (80.0 mg, 0.190 mmol, prepared by the method disclosed for the intermediate CA on page 411 of the specification of the patent application "WO 2022236058 A1") and compound 1e (100 mg, 0.200 mmol) were dissolved in tetrahydrofuran (3 mL) and N,N-dimethylformamide (1 mL), and acetic acid (0.2 mL) and sodium borohydride acetate (120 mg, 0.570 mmol) were added in sequence. The reaction solution was stirred at 25°C for 2 h. The reaction solution was poured into water (10 mL) and dichloromethane (10 mL × 3) was used for extraction. The combined organic phase was washed with saturated brine (20 mL), dried over anhydrous sodium sulfate and filtered. The filtrate was concentrated under reduced pressure, and the residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 30%-70%, flow rate: 25 mL/min) to obtain compound 6. MS m/z(ESI):907.6[M+1]$^+$.

Example 7: 3-(4-(3-(1-(1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)piperidin-4-yl)oxy)prop-1-ynyl)-3-methyl-2-oxo-2,3-dihydro-1H-benzoimidazol-1-yl)piperidine-2,6-dione (7)

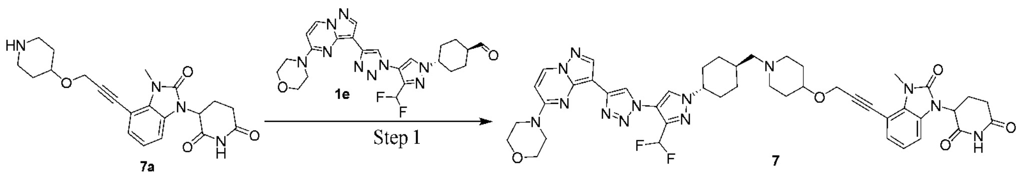


Compound 7a (50.0 mg, 0.130 mmol, prepared by the method disclosed for the intermediate APT on page 91 of the specification of the patent application "WO 2021247899 A1") and compound 1e (65.0 mg, 0.130 mmol) were dissolved in tetrahydrofuran (1 mL) and N,N-dimethylformamide (1 mL), and acetic acid (0.1 mL) and sodium borohydride acetate (75.0 mg, 0.360 mmol) were added in sequence. The reaction solution was stirred at 25°C for 2 h. The reaction solution was poured into water (10 mL) and dichloromethane (10 mL × 3) was used for extraction. The combined organic phase was washed with saturated brine (20 mL), dried over anhydrous sodium sulfate and filtered to remove the desiccant, and then the filtrate was concentrated under reduced pressure, and the residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 40%-75%, flow rate: 25 mL/min) to obtain compound 7. MS m/z(ESI):439.9[M/2+1]$^+$.

Example 8: 3-(4-(1-(((1R,4R)-4-(3-(difluoromethyl)-4-(4-(5-morpholinylpyrazolo[1,5-a]pyrimidin-3-yl)-1H-1,2,3-triazol-1-yl)-1H-pyrazol-1-yl)cyclohexylmethyl)piperidin-4-yl)methyl-3-methyl-2-oxo-2,3-dihydro-1H-benzo[d]imidazol-1-yl) piperidine-2,6-dione (8)

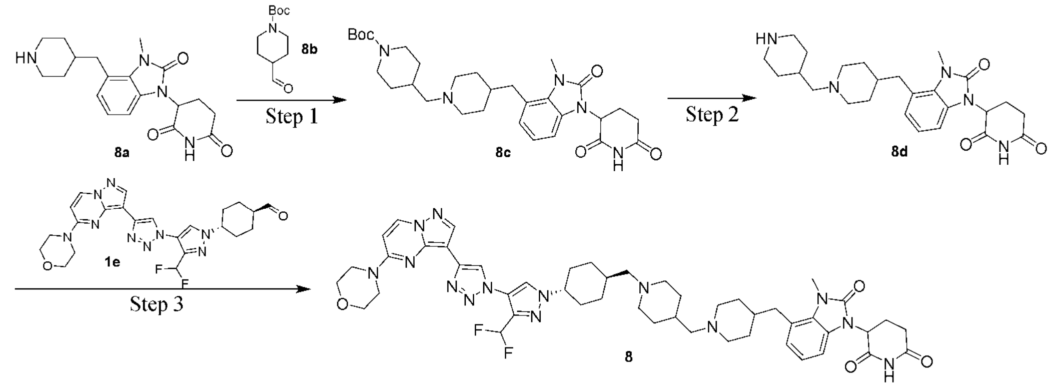


8a 8c 8d 1e 8

1) Step 1: Compound 8a (240 mg, 0.670 mmol, prepared by the method disclosed for the intermediate step AVW compound on page 639 of the specification of the patent application "WO 2020/264499 A1") was dissolved in dichloromethane (10 mL), and triethylamine (0.20 mL, 1.35 mmol), compound 8b (144 mg, 0.670 mmol, Bide Pharmatech Ltd.), acetic acid (0.08 mL, 1.35 mmol), sodium borohydride acetate (428 mg, 2.02 mmol) were added. The reaction solution was reacted at 25°C for 2 h. The reaction solution was concentrated under reduced pressure and then purified by high performance liquid chromatography (GILSON-215, chromatographic column: Boston-Prime C18, 30×150 mm, 5 μm; mobile phase: water (containing 0.05% ammonium hydroxide) and acetonitrile, gradient ratio: acetonitrile 45%-65%, flow rate: 35 mL/min) to obtain compound 8c. MS m/z(ESI):554.3$[M+1]^+$.

2) Step 2: Compound 8c (10.0 mg, 0.200 mmol) was dissolved in dichloromethane (0.5 mL). Trifluoroacetic acid (0.5 mL) was added. The reaction solution was reacted at 25°C for 1 h. The reaction solution was concentrated under reduced pressure to obtain compound 8d. MS m/z(ESI):454.4$[M+1]^+$.

3) Step 3: Compound 8d (8.00 mg, 20.0 μmol) was dissolved in tetrahydrofuran (1 mL) and 1,2-dichloroethane (1 mL). Triethylamine (1.78 mg, 20.0 μmol), compound 1e (8.78 mg, 20.0 μmol) and acetic acid (1.06 mg, 20.0 umol) were added and reacted at room temperature for 1 h. Sodium triacetylborohydride (22.4 mg, 110 umol) was added and the obtained mixture was reacted at room temperature for 1 h. The reaction solution was concentrated under reduced pressure, and the residue was purified by silica gel plate chromatography with an eluent system (dichloromethane/methanol = 20/1) to obtain compound 8. MS m/z(ESI):935.5[M+1].

Example 9: 3-(4-(7-(1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)-7-azaspiro[3.5]nonan-2-ylmethyl)amino)-3-methyl-2-oxo-2,3-dihydro-1H-benzoimidazol-1-yl)piperidine-2,6-dione (9)

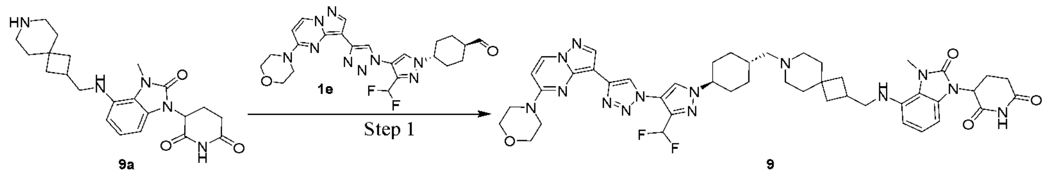


9a 1e 9

Compound 9a (40.0 mg, 0.100 mmol, prepared by the method disclosed for the intermediate BAQ on page 786 of the specification of the patent application "WO 2020264499") was dissolved in 1,2-dichloroethane (2 mL) and N,N-dimethylformamide (1 mL), and triethylamine (0.1 mL) was added and the obtained mixture was stirred for 0.5 h. Compound 1e (59.0 mg, 90.0 μmol) and acetic acid (0.1 mL) were added to the reaction solution and the obtained mixture was stirred for 0.5 h. Sodium cyanoborohydride (19.0 mg, 0.300 mmol) was added to the reaction solution. The reaction solution was stirred at 25°C for 2 h. The reaction solution was concentrated under reduced pressure and then purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 9. MS m/z(ESI):893.5$[M+1]^+$. $^1$H NMR (400 MHz, DMSO-$d_6$) δ 11.06 (s, 1H), 8.77 (d, $J$ = 7.9 Hz, 1H), 8.65 (s, 1H), 8.53 (s, 1H), 8.38 (s, 1H), 7.19 (t, $J$ = 53.0 Hz, 1H), 6.86 - 6.81 (m, 2H), 6.50 (d, $J$ = 8.5 Hz, 1H), 6.42 (d, $J$ = 8.3 Hz, 1H), 5.32 - 5.27 (m, 1H), 4.93 - 4.87 (m, 1H), 4.32 - 4.25 (m, 1H), 3.73 (s, 8H), 3.61 (s, 3H), 3.10 - 3.02 (m, 2H), 2.72 - 2.60 (m, 3H), 2.59 - 2.55 (m, 2H), 2.40 - 2.30 (m, 3H), 2.25 - 1.75 (m, 12H), 1.70 - 1.50 (m, 6H), 1.10 - 1.00 (m, 2H).

Example 10: 3-(4-(((7-(((1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-yl(cyclohexyl)methyl)-7-azaspiro[3.5]nonan-2-yl)amino)methyl)-3-methyl-2-oxo-2,3-dihydro-1H-benzo[d]imidazol-1-yl)piperidine-2,6-dione (10)

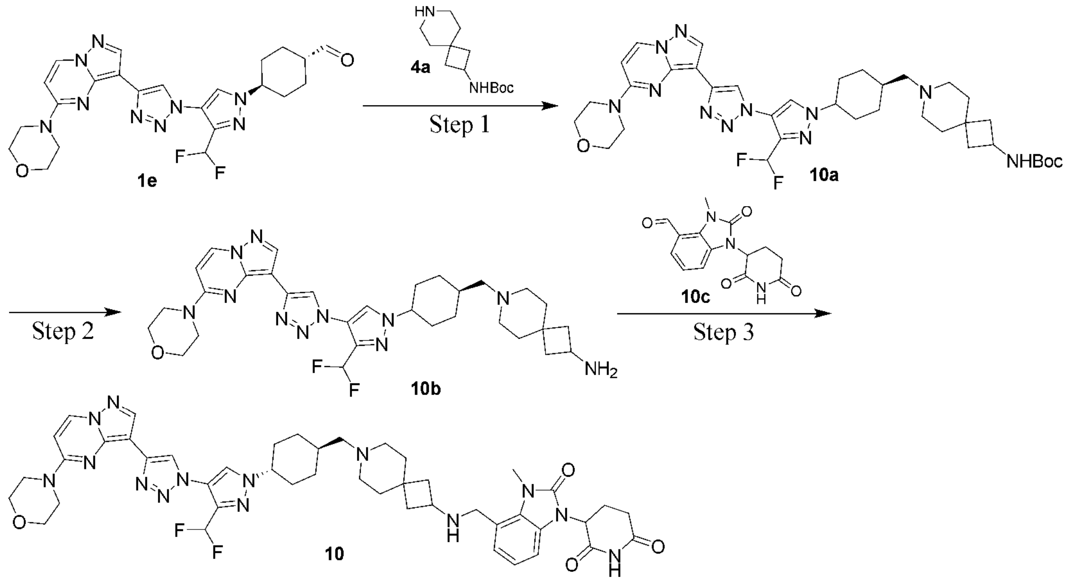


1) Step 1: Compound 1e (120 mg, 0.240 mmol) was dissolved in dichloromethane (6 mL), and compound 4a (74.9 mg, 0.310 mmol, Bide Pharmatech Ltd.) and acetic acid (14.4 mg, 0.240 mmol) were added. The reaction solution was stirred at 25°C for 10 min, and then sodium triacetoxyborohydride (306 mg, 1.45 mmol) was added. The reaction solution was stirred at 25°C for 1 h. The reaction solution was poured into water (5 mL) and the aqueous phase was extracted with dichloromethane (5 mL × 3). The combined organic phase was washed with saturated brine (5 mL), dried over anhydrous sodium sulfate and filtered to remove the desiccant, and then the filtrate was concentrated under reduced pressure. The residue was purified by silica gel column chromatography with an eluent system (dichloromethane/methanol = 20/1 to 10/1) to obtain compound 10a. MS m/z(ESI):722.4$[M+1]^+$.

2) Step 2: Compound 10a (40.0 mg, 60.0 μmol) was dissolved in dichloromethane (1 mL), and trifluoroacetic acid (1 mL) was added. The reaction solution was stirred at 25°C for 1 h. The reaction solution was concentrated under reduced pressure to obtain compound 10b. MS m/z(ESI):622.7$[M+1]^+$.

3) Step 3: Compound 10b (80.0 mg, 0.130 mmol) was dissolved in tetrahydrofuran (1 mL), and compound 10c (36.9 mg, 0.130 mmol, prepared by the method disclosed for the intermediate on page 171 of the specification of the patent application "WO 2022140472 A1") and tetraethyl titanate (0.050 mL, 0.26 mmol) were added. The reaction solution was stirred at 80°C for 18 h, then returned to room temperature, and sodium triacetoxyborohydride (40.9 mg, 0.190 mmol) was added, and the reaction was stirred at room temperature for 2 h. The reaction solution was poured into water (5 mL), and the aqueous phase was extracted with dichloromethane (5 mL × 3). The combined organic phase was washed with saturated brine (10 mL), dried over anhydrous sodium sulfate and filtered. The filtrate was concentrated under reduced pressure, and the residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 10. MS m/z(ESI):893.6$[M+1]^+$.

Example 11: 3-(4-(1-(((1r,4r)-4-(3-(difluoromethyl)-4-(4-(5-morpholinopyrazolo[1,5-a]pyrimidin-3-yl)-1H- 1,2,3-triazol-1-yl)-1H-pyrazol-1-yl)cyclohexyl)methyl)piperidin-4-yl)-3-methyl-2-oxo-2,3-dihydro-1H-benzo[ d]imidazol-1-yl)piperidine-2,6-dione (11)

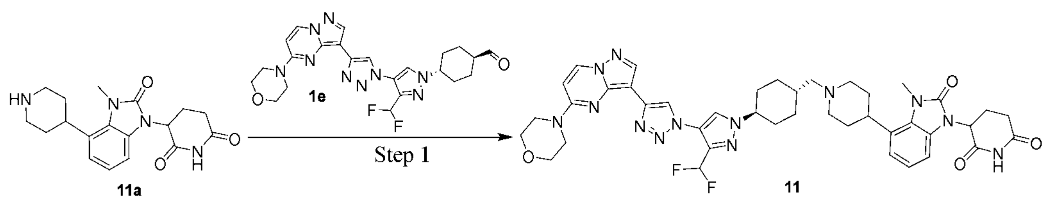


Compound 11a (165 mg, 0.480 mmol, prepared by the method disclosed for the intermediate 199 on page 222 of the specification of the patent application "WO 2021158634 A1") was dissolved in dichloromethane (5 mL) and tetrahydrofuran (5 mL), and triethylamine (40.7 mg, 0.400 mmol) was added. The reaction solution was stirred at 25°C for 10 min, and then acetic acid (24.1 mg, 0.400 mmol) and compound 1e (200 mg, 0.400 mmol) were added. The reaction solution was stirred at 25°C under nitrogen protection for 1 h, and then sodium borohydride acetate (511 mg, 2.41 mmol) was added. The reaction solution was stirred at 25°C under nitrogen protection for 1 h, and then quenched with saturated sodium bicarbonate aqueous solution (8 mL) and dichloromethane (10 mL). The aqueous phase was subjected to liquid separation and then extracted with dichloromethane (5 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-T C18,30*150 mm, 5 μm; mobile phase: water (containing 10 mmol/L formic acid) and acetonitrile, gradient ratio: acetonitrile 38%-45%, flow rate: 30 mL/min) to obtain compound 11. MS m/z(ESI):824.4[M+1]$^+$. $^1$H NMR (400 MHz, DMSO-$d_6$): δ 11.10 (s, 1H), 8.76 (d, $J$ = 4.0 Hz, 1H), 8.65 (s, 1H), 8.54 (s, 1H), 8.38 (s, 1H), 7.20 (t, $J$ = 53.2 Hz, 1H), 7.05 - 6.94 (m, 3H), 6.81 (d, $J$ = 4.0 Hz, 1H), 5.42 - 5.28 (m, 1H), 4.34 - 4.26 (m, 1H), 3.72 (s, 8H), 3.58 (s, 3H), 3.27 - 3.21 (m, 2H), 3.06 - 3.00 (m, 2H), 2.93 - 2.85 (m, 1H), 2.72 - 2.59 (m, 2H), 2.30 - 2.23 (m, 2H), 2.20 - 2.11 (m, 4H), 2.02 - 1.94 (m, 3H), 1.85 - 1.77 (m, 5H), 1.69 - 1.62 (m, 1H), 1.17 - 1.06 (m, 2H).

Example 12: 3-(4-((6-(((1r,4r)-4-(3-(difluoromethyl)-4-(4-(5-morpholinopyrazolo[1,5-a]pyrimidin-3-yl)-1H -1,2,3-triazol-1-yl)-1H-pyrazol-1-yl)cyclohexyl)methyl)-2,6-diazaspiro[3.3]hept-2-yl)methyl)-3-methyl-2-oxo-2,3-dihydro-1H-benzo[d]imidazol-1-yl)piperidine-2,6-dione (12)

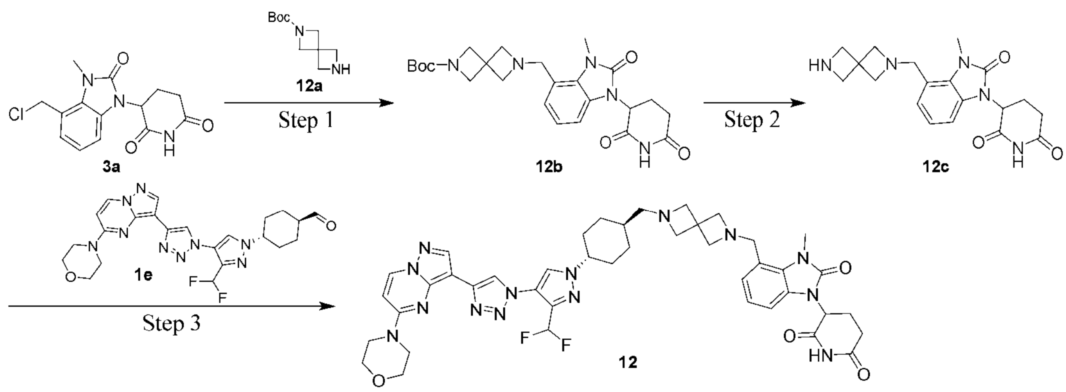


1) Step 1: Compound 3a (120 mg, 0.390 mmol, prepared by the method disclosed for the intermediate B32-2 on page 574 of the specification of the patent application "WO 2020206424 A1") and compound 12a (92.8 mg, 0.470 mmol, Bide Pharmatech Ltd.) were dissolved in acetonitrile (8 mL), and potassium carbonate (55.0 mg, 0.390 mmol) was added, and the obtained mixture was reacted under stirring for 1.5 h at 80°C. Water (50 mL) was added to the reaction solution, and the obtained mixture was extracted with dichloromethane (50 mL × 3). The organic phases were combined and washed with saturated brine. The organic phase was dried over anhydrous sodium sulfate and filtered to remove the desiccant, and then the filtrate was concentrated under reduced pressure. The residue was purified by silica gel column chromatography with an eluent system (dichloromethane/methanol = 40/1 to 15/1) to obtain compound 12b. MS m/z(ESI):470.6[M+1]$^+$.

2) Step 2: Compound 12b (120 mg, 0.260 mmol) was dissolved in dichloromethane (5 mL), trifluoroacetic acid (0.8 mL) was added, and the reaction solution was reacted under stirring at 25°C for 2 h. The reaction solution was concentrated under reduced pressure to obtain compound 12c. MS m/z(ESI):370[M+1]$^+$.

3) Step 3: Compound 12c (66.8 mg, 0.181 mmol) was dissolved in 1,2-dichloroethane (3 mL) and tetrahydrofuran (3 mL), triethylamine (18.3 mg, 0.181 mmol) was added until the pH value was greater than 8, and the mixture was stirred

at 25°C for 10 min. The reaction solution was cooled to -10°C, acetic acid (50.3 mg, 0.271 mmol) was added, and compound 1e (90.0 mg, 0.181 mmol) was added. The reaction solution was warmed to 25°C and stirred at this temperature for 20 min, and then sodium borohydride acetate (76.7 mg, 0.361 mmol) was added. The reaction solution was stirred at 25°C for 1 h. The reaction solution was concentrated under reduced pressure and then purified by high performance liquid chromatography (Waters-2545, chromatographic column: SharpSil-T C18, 30*150 mm, 5 μm; mobile phase: water (containing 10 mmol/L ammonium bicarbonate) and acetonitrile, gradient ratio: acetonitrile 38%-45%, flow rate: 30 mL/min) to obtain compound 12. MS m/z(ESI):851.6[M+1]$^+$. $^1$H NMR (400 MHz, DMSO-$d_6$):δ 11.10 (s, 1H), 8.76 (d, *J* = 7.9 Hz, 1H), 8.64 (s, 1H), 8.53 (d, *J*= 3.9 Hz, 1H), 8.20 (s, 1H), 7.34 - 7.02 (m, 2H), 7.00 - 6.88 (m, 2H), 6.81 (d, *J* = 7.9 Hz, 1H), 5.37 (dd, *J* = 12.6, 5.3 Hz, 1H), 4.25 (t, *J*= 11.7 Hz, 1H), 3.76 - 3.71 (m, 10H), 3.63 (s, 3H), 3.35 - 3.27 (m, 4H), 3.24 (s, 4H), 2.94 - 2.85 (m, 1H), 2.74 - 2.59 (m, 2H), 2.31 (d, J = 6.5 Hz, 2H), 2.17 - 2.05 (m, 2H), 2.03 - 1.97 (m, 1H), 1.91 - 1.81 (m, 2H), 1.79 - 1.72 (m, 1H), 1.68 - 1.48 (m, 1H), 1.46 - 1.24 (m, 1H), 1.20 - 0.94 (m, 2H).

Example 13: 3-(4-((7-(((1r,4r)-4-(3-(difluoromethyl)-4-(4-(5-morpholinopyrazolo[1,5-a]pyrimidin-3-yl)-1H -1,2,3-triazol-1-yl)-1H-pyrazol-1-yl)cyclohexyl)methyl)-2,7-diazaspiro[3.5]nonan-2-yl)methyl)-3-methyl-2-oxo-2,3-dihydro-1H-benzo[d]imidazol-1-yl)piperidine-2,6-dione (13)

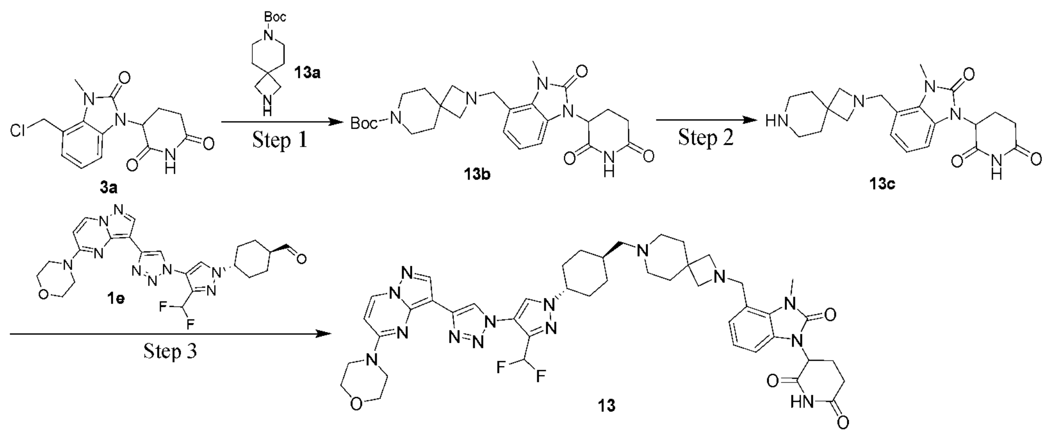


1) Step 1: Compound 3a (100 mg, 0.321 mmol, prepared by the method disclosed for the intermediate B32-2 on page 574 of the specification of the patent application "WO 2020206424 A1") and compound 13a (73.5 mg, 0.321 mmol, Bide Pharmatech Ltd.) were dissolved in acetonitrile (8 mL), and potassium carbonate (112 mg, 0.810 mmol) was added, and the reaction solution was reacted under stirring for 1.5 h at 80°C. Then water (50 mL) was added to the reaction solution, and the obtained mixture was extracted with dichloromethane (50 mL × 3). The organic phases were combined and washed with saturated brine. The organic phase was dried over anhydrous sodium sulfate and filtered to remove the desiccant, and then the filtrate was concentrated under reduced pressure. The residue was purified by silica gel column chromatography with an eluent system (dichloromethane/methanol = 40/1 to 15/1) to obtain compound 13b. MS m/z(ESI):498.6[M+1]$^+$.

2) Step 2: Compound 13b (25.0 mg, 51.0 μmol) was dissolved in dichloromethane (4 mL), trifluoroacetic acid (0.8 mL) was added, and the reaction solution was reacted at 25°C for 2 h. The reaction solution was concentrated under reduced pressure to obtain compound 13c. MS m/z(ESI):398[M+1]$^+$.

3) Step 3: Compound 13c (25.0 mg, 61.0 μmol) was dissolved in 1,2-dichloroethane (3 mL) and tetrahydrofuran (3 mL), triethylamine (8.12 mg, 81.00 μmol) was added until the pH value was greater than 8, and the reaction solution was stirred at 25°C for 10 min. The reaction solution was cooled to -10°C, and acetic acid (186 mg, 91.0 μmol) and compound 1e (30.0 mg, 61.0 μmol) were added. The reaction solution was warmed to 25°C and stirred at this temperature for 20 min, and then sodium borohydride acetate (25.7 mg, 0.121 mmol) was added. The reaction solution was stirred at 25°C for 1 h. The reaction solution was concentrated under reduced pressure and then purified by high performance liquid chromatography (Waters-2545, chromatographic column: SharpSil-T C18, 30×150 mm, 5 μm; mobile phase: water (containing 10 mmol/L ammonium bicarbonate) and acetonitrile, gradient ratio: acetonitrile 38%-45%, flow rate: 30 mL/min) to obtain compound 13. MS m/z(ESI):879.5[M+1]$^+$.

Example 14: 3-(4-(2-(1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)-2,7-diazaspiro[3.5]nonan-7-yl)methyl)-3-methyl-2-oxo-2,3-dihydro-1H-benzoimidazol-1-yl)piperidine-2,6-dione (14)

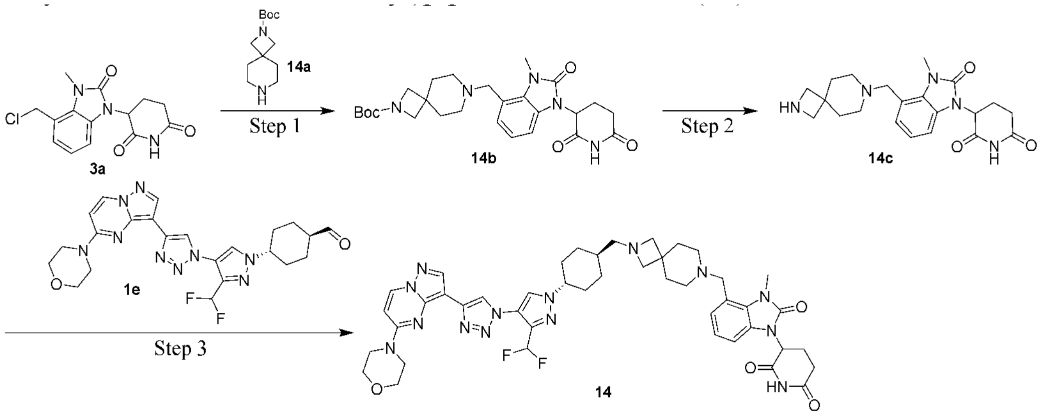


1) Step 1: Compound 3a (100 mg, 0.321 mmol, prepared by the method disclosed for the intermediate B32-2 on page 574 of the specification of the patent application "WO 2020206424 A1") and compound 14a (100 mg, 0.440 mmol, Bide Pharmatech Ltd.) were dissolved in acetonitrile (2 mL), and potassium carbonate (122 mg, 0.880 mmol) was added. The reaction solution was subjected to nitrogen replacement three times and reacted at 80°C for 2 h. The reaction solution was quenched with saturated ammonium chloride aqueous solution (20 mL) and extracted with dichloromethane (20 mL × 2). The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 30/1) to obtain compound 14b. MS m/z(ESI):498[M+1]$^+$.
2) Step 2: Compound 14b (80.0 mg, 0.160 mmol) was dissolved in dichloromethane (2 mL), and trifluoroacetic acid (1 mL) was added. The reaction solution was stirred at 25°C for 1 h. The reaction solution was concentrated under reduced pressure to obtain compound 14c. MS m/z(ESI):398.2[M+1]$^+$.
3) Step 3: Compound 14c (35.0 mg, 90.0 μmol) was dissolved in a solution of 1,2-dichloroethane (2 mL) and N,N-dimethylformamide (1 mL), triethylamine (0.05 mL) was added, and the reaction solution was stirred for 0.5 h. Compound 1e (54.0 mg, 0.110 mmol) and acetic acid (0.1 mL) were added to the reaction solution and the obtained mixture was stirred for 0.5 h. Sodium borohydride acetate (17.0 mg, 0.270 mmol, Energy Chemical) was added to the reaction solution. The reaction solution was stirred at 25°C for 2 h and then concentrated under reduced pressure to obtain a residue which was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 14. MS m/z(ESI):879.6[M+1]$^+$. $^1$HNMR (400 MHz, DMSO-$d_6$): δ11.10 (s, 1H), 8.76 (d, *J* = 7.9 Hz, 1H), 8.64 (s, 1H), 8.53 (s, 1H), 8.38 (s, 1H), 7.32 - 7.05 (m, 3H), 6.95 (t, *J* = 7.7 Hz, 1H), 6.86 (d, *J* = 7.2 Hz, 1H), 6.81 (d, *J*= 7.7 Hz, 1H), 6.65 (s, 1H), 5.40 - 5.30 (m, 2H), 4.31 - 4.20 (m, 1H), 3.72 (s, 8H), 3.67 (s, 3H), 3.58 (s, 2H), 2.90 (s, 3H), 2.70 - 2.62 (m, 2H), 2.30 - 2.26 (m, 2H), 2.13 - 2.07 (m, 2H), 2.01 - 1.98 (m, 3H), 1.80 - 1.72 (m, 2H), 1.65 - 1.61 (m, 3H), 1.47 - 1.42 (m, 2H), 1.13 - 1.01 (m, 2H), 0.90 - 0.80 (m, 3H).

Example 15: 3-(4-(4-((1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)(methyl)amino)piperidin-1-yl)methyl)-3-methyl-2-oxo-2,3-dihydro-1H-benzoimidazol-1-yl)piperidine-2,6-dione (15)

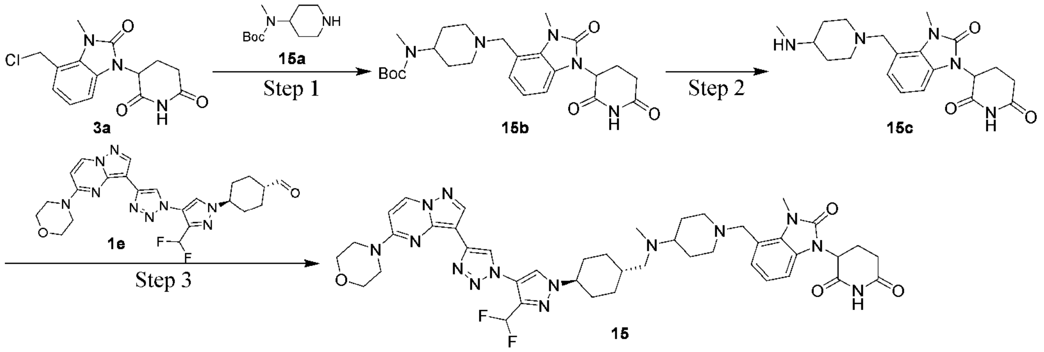


1) Step 1: Compound 3a (80.0 mg, 0.260 mmol, prepared by the method disclosed for the intermediate B32-2 on page 574 of the specification of the patent application "WO 2020206424A1") and compound 15a (67.0 mg, 0.310 mmol, Bide Pharmatech Ltd.) were dissolved in acetonitrile (5 mL), and potassium carbonate (71.0 mg, 0.520 mmol) was added. The reaction solution was subjected to nitrogen replacement three times and reacted at 80°C for 2 h. The reaction solution was diluted with ammonium chloride aqueous solution (20 mL), and extracted with dichloromethane (20 mL × 2). The organic phases were combined and dried over anhydrous sodium sulfate and filtered to remove the desiccant, and then the filtrate was concentrated under reduced pressure. The residue was purified by silica gel column chromatography (dichloromethane/methanol = 100/1 to 30/1) to obtain compound 15b. MS m/z(ESI):486.7 $[M+1]^+$.
2) Step 2: Compound 15b (80.0 mg, 0.160 mmol) was dissolved in dichloromethane (2 mL), and trifluoroacetic acid (1 mL) was added. The reaction was stirred at 25°C for 1 h. The reaction solution was concentrated under reduced pressure to obtain crude compound 15c. MS m/z(ESI):368.2$[M+1]^+$.
3) Step 3: Compound 15c (30.0 mg, 80.0 μmol) was dissolved in 1,2-dichloroethane (2 mL) and N,N-dimethylformamide (1 mL), triethylamine (0.1 mL) was added, and the obtained mixture was stirred for 0.5 h. Compound 1e (47.0 mg, 90.0 μmol) and glacial acetic acid (0.1 mL) were added to the reaction solution and the obtained mixture was stirred for 0.5 h. Finally, sodium borohydride acetate (15.0 mg, 0.230 mmol) was added to the reaction solution. The reaction solution was stirred at 25°C for 2 h. The reaction solution was concentrated under reduced pressure and then purified by high performance liquid chromatography (A: 0.1% formic acid/water, B: acetonitrile, chromatographic column: Waters-SunFire-C18-10μm-19*250 mm; mobile phase: water (containing 0.1% formic acid) and acetonitrile, gradient ratio: acetonitrile 32%-48%, flow rate: 25 mL/min) to obtain compound 15. MS m/z(ESI):867.7$[M+1]^+$. $^1$HNMR (400 MHz, DMSO-$d_6$):δ 11.13 (s, 1H), 8.77 (d, *J* = 7.9 Hz, 1H), 8.65 (s, 1H), 8.53 (s, 1H), 8.38 (s, 1H), 8.16 (s, 1H), 7.20 (t, *J* = 47.8 Hz, 1H), 7.09 - 7.04 (m, 1H), 6.96 (t, *J* = 7.7 Hz, 1H), 6.89 - 6.82 (m, 1H), 6.82 (d, *J* = 7.9 Hz, 1H), 5.39 (dd, *J* = 12.5, 5.4 Hz, 1H), 4.31 - 4.21 (m, 1H), 3.72 (s, 8H), 3.68 (s, 3H), 3.62 (s, 2H), 2.92 - 2.84 (m, 3H), 2.77 - 2.69 (m, 1H), 2.68 - 2.56 (m, 2H), 2.44 - 2.38 (m, 1H), 2.31 - 2.27 (m, 2H), 2.23 (s, 3H), 2.15 - 2.09 (m, 2H), 2.01 - 1.91 (m, 4H), 1.84 - 1.75 (m, 2H), 1.70 - 1.62 (m, 2H), 1.47 - 1.34 (m, 2H), 1.11 - 0.99 (m, 2H).

Example 16: 3-(4-(3-(2-(1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)-2,7-diazaspiro[3.5]nonan-7-yl)-3-oxopropyl)-3-methyl-2-oxo-2,3-dihydro-1H-benzoimidazol-1-yl)piperidine-2,6-dione (16)

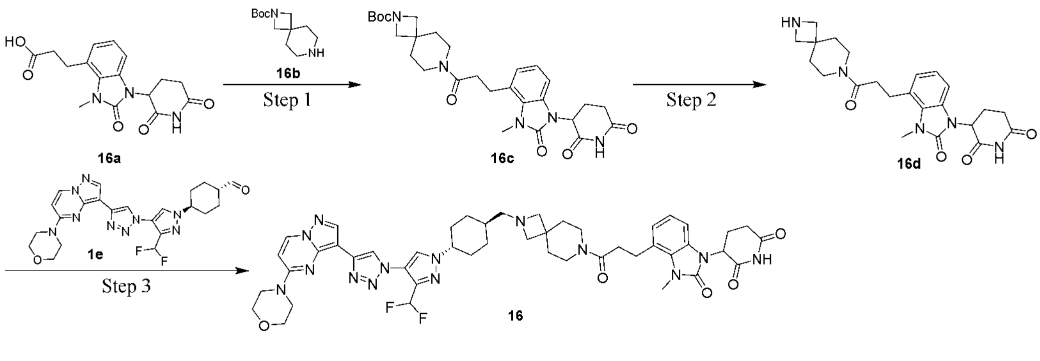


1) Step 1: Compound 16a (100 mg, 0.300 mmol, prepared by the method disclosed for the intermediate B-116 on page

171 of the specification of the patent application "WO 2023019166 A1") and N,N-diisopropylethylamine (0.2 mL, 1.21 mmol) were dissolved in N,N-dimethylformamide (2.5 mL), and benzotriazole-*N*,*N*,*N'*,*N'*-tetramethyluronium hexafluorophosphate (138 mg, 0.360 mmol) was added. After the reaction solution was reacted at 25°C for 10 min, compound 16b (88.8 mg, 0.390 mmol) was added. The reaction solution was stirred for 1 h, and then diluted with ethyl acetate (2 mL) and quenched with water (4 mL). The aqueous phase was separated and then extracted with ethyl acetate (2 mL × 2). The combined organic phase was dried over anhydrous sodium sulfate and filtered to remove the desiccant, and then the filtrate was concentrated under reduced pressure. The residue was purified on silica gel plate for thin layer chromatography (dichloromethane/methanol = 10/1) to obtain compound 16c. MS m/z(ESI):540.7 $[M+1]^+$.

2) Step 2: Compound 16c (45 mg, 80.0 μmol) was dissolved in dichloromethane (2 mL), and trifluoroacetic acid (0.5 mL) was added. The reaction solution was reacted at 25°C for 30 min and then concentrated under reduced pressure to obtain compound 16d. MS m/z(ESI):440.6$[M+1]^+$.

3) Step 3: Compound 16d (36.0 mg, 80.0 μmol) was dissolved in 1,2-dichloroethane (2 mL) and tetrahydrofuran (2 mL), and triethylamine (7.53 mg, 70.0 μmol) was added. The reaction solution was stirred at 25°C for 10 min, and then acetic acid (4.47 mg, 70.0 μmol) and compound 1e (37.0 mg, 70.0 μmol) were added. The reaction solution was stirred at 25°C under nitrogen protection for 1 h, and then sodium borohydride acetate (94.6 mg, 0.780 mmol) was added. The reaction solution was stirred at 25°C under nitrogen protection for 1 h, and then quenched with saturated sodium bicarbonate aqueous solution (5 mL) and dichloromethane (5 mL). The aqueous phase was subjected to liquid separation and then extracted with dichloromethane (3 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-T C18,30×150 mm, 5 μm; mobile phase: water (containing 10 mmol/L formic acid) and acetonitrile, gradient ratio: acetonitrile 38%-45%, flow rate: 30 mL/min) to obtain compound 16 (23.5 mg). MS m/z(ESI):921.8 $[M+1]^+$. $^1$H NMR (400 MHz, DMSO-$d_6$): δ 11.09 (s, 1H), 8.76 (d, *J* = 7.9 Hz, 1H), 8.70 - 8.63 (m, 1H), 8.54 - 8.52 (m, 1H), 8.37 (s, 1H), 7.23 (t, *J*= 26.6 Hz, 1H), 6.98 - 6.90 (m, 3H), 6.81 (d, *J* = 7.9 Hz, 1H), 5.45 - 5.30 (m, 1H), 4.32 - 4.19 (m, 1H), 3.72 (s, 8H), 3.57 (s, 3H), 3.28 - 3.25 (m, 2H), 3.15 - 3.11 (m, 2H), 2.91 (s, 4H), 2.70 - 2.60 (m, 4H), 2.47 - 2.40 (m, 2H), 2.29 - 2.27 (m, 1H), 2.14 - 2.07 (m, 2H), 2.00 - 1.95 (m, 1H), 1.92 - 1.85 (m, 2H), 1.82 - 1.71 (m, 2H), 1.55 (s, 6H), 1.39 - 1.29 (m, 1H), 1.15 - 0.99 (m, 2H).

Example 17: 3-(4-(1-(1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)piperidin-4-yl)ethynyl)-3-methyl-2-oxo-2,3-dihydro-1H-benzoimidazol-1-yl)piperidine-2,6-dione (17)

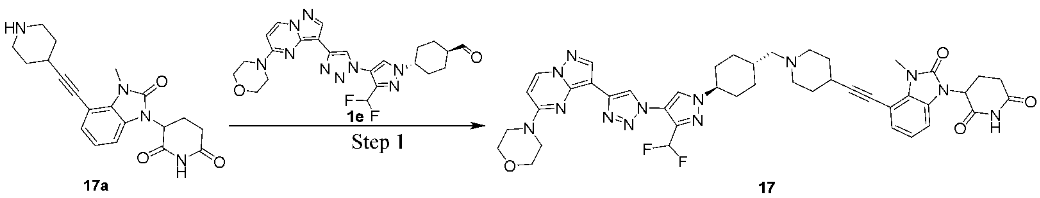

17a
17

Compound 17a (50.0 mg, 0.140 mmol, prepared by the method disclosed for the intermediate YL on page 858 of the specification of the patent application "WO 2020113233") and triethylamine (14.0 mg, 0.140 mmol) were dissolved in N,N-dimethylformamide (2 mL) and tetrahydrofuran (2 mL) and the obtained mixture was stirred for 10 min. Acetic acid (13.0 mg, 0.220 mmol) and compound 1e (67.0 mg, 0.130 mmol) were added, and the reaction solution was stirred at 25°C for 0.5 h. Sodium borohydride acetate (87.0 mg, 0.410 mmol) was slowly added, and the reaction solution was stirred for 1 h. The reaction solution was subjected to removal of the solvent under reduced pressure, and the residue was purified by high performance liquid chromatography (Gilson_306_1741, chromatographic column: Waters-SunFire-C18-10μm-19×250 mm; mobile phase: water (containing 10 mmol/L ammonium bicarbonate) and acetonitrile, gradient ratio: acetonitrile 46%-95%, flow rate: 25 mL/min) to obtain compound 17. MS m/z(ESI):848.6$[M+1]^+$. $^1$H NMR(400 MHz, DMSO-$d_6$) δ11.14 (s, 1H), 8.77 (d, *J*= 7.9 Hz, 1H), 8.66 (s, 1H), 8.54 (s, 1H), 8.38 (s, 1H), 7.20 (t, *J* = 53.2 Hz, 1H), 7.11 - 7.07 (m, 1H), 7.05 - 7.01 (m, 1H), 6.99 - 6.97 (m, 1H), 6.82 (d, *J*= 8.0 Hz, 1H), 5.41 - 5.37 (m, 1H), 4.28 - 4.25 (m, 1H), 3.73 (s, 8H), 3.65 (s, 3H), 2.92 - 2.85(m, 1H), 2.71 - 2.60 (m, 5H), 2.15 - 2.12 (m, 6H), 2.03 - 2.00 (m, 1H), 1.92 - 1.87 (m, 4H), 1.83 - 1.80 (m, 2H), 1.68 - 1.65 (m, 3H), 1.09 - 1.07 (m, 2H).

Example 18: 2-(((1r,4r)-4-(3-(difluoromethyl)-4-(4-(5-morpholinopyrazolo [1,5-a]pyrimidin-3-yl)-1H-1,2,3-triazol-1-yl)-1H-pyrazol-1-yl)cyclohexyl)methyl)-N-(1-(2,6-dioxopiperidin-3-yl)-3-methyl-2-oxo-2,3-dihydro-1H-benzo[d]imidazol-4-yl)-2,8-diazaspiro[4.5]decan-8-carboxamide (18)

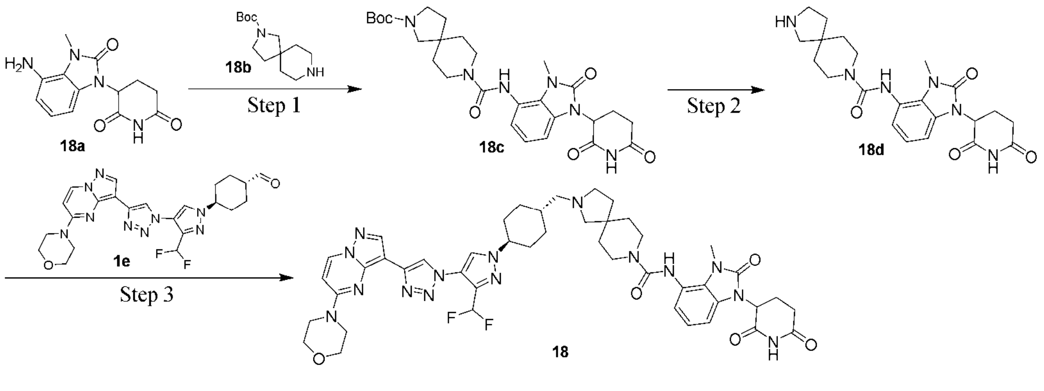


1) Step 1: Compound 18a (171 mg, 0.620 mmol, prepared by the method disclosed for the intermediate 495 on page 172 of the specification of the patent application "WO 2022068933 A1") and N,N-diisopropylethylamine (0.3 mL, 1.87 mmol) were dissolved in tetrahydrofuran (5 mL). A solution of triphosgene (130 mg, 0.440 mmol) in tetrahydrofuran (2 mL) was added dropwise. The reaction solution was stirred at 50°C for 0.5 h. After the reaction solution was cooled to 25°C, compound 18b (150 mg, 0.620 mmol, Bide Pharmatech Ltd.) was added to the reaction solution. After the reaction solution was reacted at 25°C for 0.5 h, the reaction solution was dropped into a mixed solution of saturated sodium bicarbonate aqueous solution (10 mL) and dichloromethane (20 mL) for quenching. The aqueous phase was subjected to liquid separation and then extracted with dichloromethane (10 mL × 3). The combined organic phase was dried over anhydrous sodium sulfate and filtered to remove the desiccant, and then the filtrate was concentrated under reduced pressure. The residue was purified by silica gel column chromatography with an eluent system (dichloromethane/methanol = 100/1 to 15/1) to obtain compound 18c. MS m/z(ESI):541.4$[M+1]^+$. $^1$H NMR (400 MHz, $CDCl_3$): δ 8.69 - 8.41 (m, 1H), 6.97 (t, $J$ = 8.0 Hz, 1H), 6.84 (d, $J$ = 8.0 Hz, 1H), 6.71 - 6.55 (m, 2H), 5.15 (dd, $J$ = 12.3, 5.0 Hz, 1H), 3.54 - 3.49 (m, 6H), 3.44 - 3.38 (m, 2H), 3.29 - 3.18 (m, 3H), 2.81 - 2.64 (m, 2H), 2.24 - 2.15 (m, 1H), 1.76 (t, $J$= 7.1 Hz, 2H), 1.50 - 1.38 (m, 14H).

2) Step 2: Compound 18c (171 mg, 0.620 mmol) was dissolved in hydrogen chloride in dioxane (2 mL, 4.0 M). The reaction solution was stirred at 25°C for 1 h and then concentrated under reduced pressure to obtain crude compound 18d. MS m/z(ESI):441.6$[M+1]^+$. $^1$H NMR (400 MHz, MeOD): δ 7.09 - 7.01 (m, 2H), 6.87 (dd, $J$= 7.4, 1.5 Hz, 1H), 5.34 (dd, $J$= 12.5, 5.4 Hz, 1H), 3.74 - 3.65 (m, 2H), 3.59 - 3.53 (m, 2H), 3.51 (s, 3H), 3.42 (t, $J$ = 7.5 Hz, 2H), 3.18 (s, 2H), 2.18 - 2.10 (m, 1H), 2.02 (t, $J$= 7.5 Hz, 2H), 1.70 (t, $J$= 5.6 Hz, 4H), 1.38 - 1.26 (m, 3H).

3) Step 3: Compound 18d (58.4 mg, 0.130 mmol) was dissolved in tetrahydrofuran (1 mL) and N,N-dimethylformamide (1 mL), and triethylamine (12.2 mg, 0.120 mmol) was added. The reaction solution was stirred at 25°C for 5 min, and then acetic acid (7.24 mg, 0.120 mmol) and compound 1e (60.0 mg, 0.120 mmol) were added. The reaction solution was stirred at 25°C for 0.5 h, and then sodium borohydride acetate (127 mg, 0.600 mmol) was added. The reaction solution was stirred at 25°C for 0.5 h, and then quenched with saturated sodium bicarbonate aqueous solution (5 mL) and dichloromethane (10 mL). The aqueous phase was subjected to liquid separation and then extracted with dichloromethane (5 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-T C18,30×150 mm, 5 μm; mobile phase: water (containing 10 mmol/L formic acid) and acetonitrile, gradient ratio: acetonitrile 38%-45%, flow rate: 30 mL/min) to obtain compound 18 (34.6 mg). MS m/z(ESI):922.7$[M+1]^+$.

Example 19: 3-(4-((R)-3-((1-(((1r,4R)-4-(3-(difluoromethyl)-4-(4-(5-morpholinopyrazolo[1,5-a]pyrimidin-3-yl)-1H-1,2,3-triazol-1-yl)-1H-pyrazol-1-yl)cyclohexyl)methyl)piperidin-4-yl)oxy)but-1-yn-1-yl)-3-methyl-2-oxo-2,3-dihydro-1H-benzo [d]imidazol-1-yl)piperidine-2,6-dione (19)

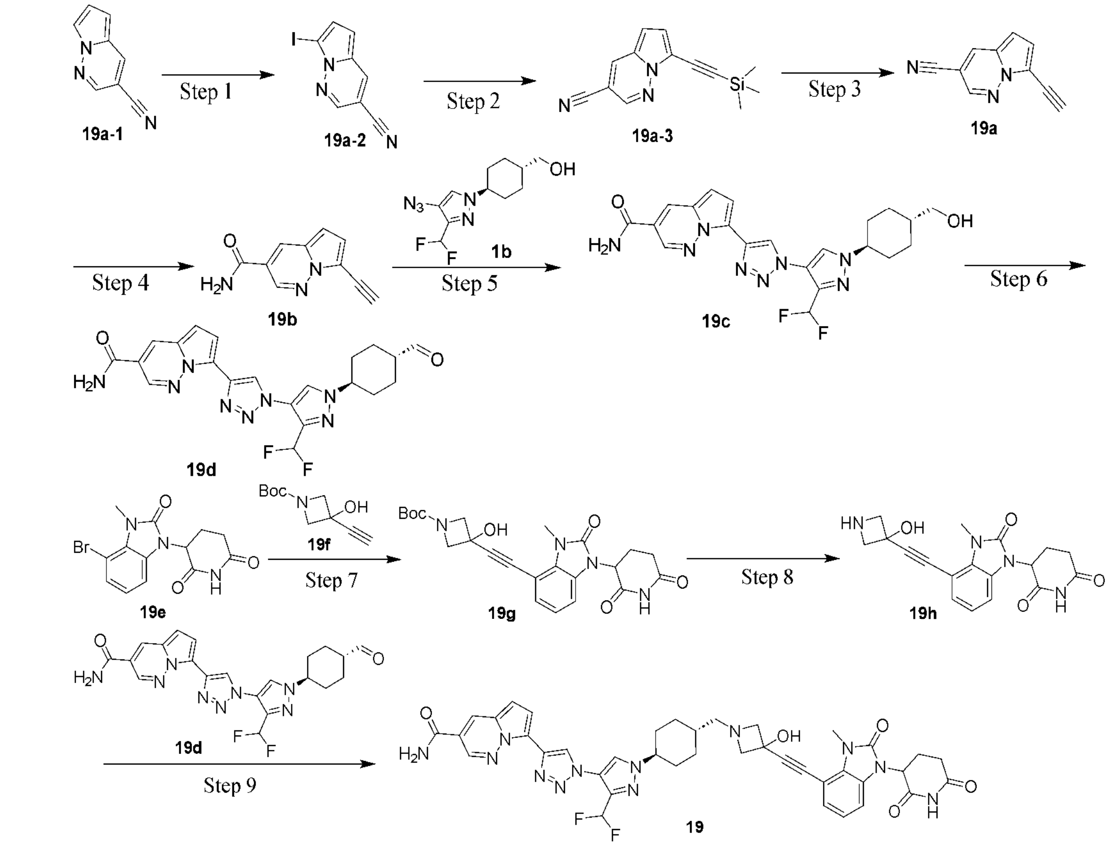


1) Step 1: Compound 19a-1 (300 mg, 2.10 mmol, prepared by the method disclosed for the intermediate pyrrolo[1,2-b] pyndazine-3-carbonitrile on page 75 of the specification of the patent application "WO 2015117563 A1") and N-iodosuccinimide (707 mg, 3.14 mmol) were added to acetonitrile (10 mL) at room temperature. The reaction solution was stirred at 60°C for 1 h. The reaction was completed as monitored by TLC, and saturated sodium sulfite solution (10 mL) was carefully added to the reaction solution. Then the mixture was extracted with ethyl acetate (100 mL), and the organic phase was washed with saturated brine (100 mL × 2), dried over anhydrous sodium sulfate, filtered and concentrated under reduced pressure. The residue was purified by flash chromatographic column (petroleum ether/ethyl acetate = 10/1) to obtain compound 19a-2. $^1$H NMR (400 MHz, $CDCl_3$): δ 8.27 (d, *J* = 2.1 Hz, 1H), 8.05 (d, *J*= 2.1 Hz, 1H), 7.24 (d, *J* = 4.7 Hz, 1H), 6.95 (d, *J* = 4.7 Hz, 1H).

2) Step 2: Compound 19a-2 (1.16 g, 4.31 mmol) was added to tetrahydrofuran (10 mL), and cuprous iodide (80 mg, 0.43 mmol), bistriphenylphosphine palladium dichloride (300 mg, 0.43 mmol) and triethylamine (1.20 mL, 8.62 mmol) were added, and then nitrogen replacement was performed, and trimethylsilylacetylene (6.14 mL, 43.1 mmol) was added, and the obtained mixture was stirred at room temperature overnight. The reaction system was added to water, and then the obtained mixture was extracted with ethyl acetate (15 mL × 3). The organic phases were combined, washed with saturated brine, dried over anhydrous sodium sulfate. The organic phases were combined and concentrated. The residue was purified by silica gel column chromatography (petroleum ether/ethyl acetate = 20/1) to obtain compound 19a-3. $^1$H NMR (400 MHz, DMSO-$d_6$): δ 8.75 (d, *J* = 2.2 Hz, 1H), 8.63 (d, *J* = 2.2 Hz, 1H), 7.38 (d, *J* = 4.7 Hz, 1H), 6.96 (d, *J*= 4.8 Hz, 1H), 0.27 (s, 9H).

3) Step 3: Compound 19a-3 (816 mg, 3.41 mmol) and potassium carbonate (1.41 g, 10.2 mmol) were added to methanol (10 mL) and the obtained mixture was stirred at room temperature for 2 h. Water was added to the reaction system, and then the obtained mixture was extracted with ethyl acetate (15 mL × 3). The organic phases were combined, washed with saturated brine, dried over anhydrous sodium sulfate, filtered, and concentrated. The residue was purified by silica gel column chromatography (EA/PE = 1/20) to obtain compound 19a. $^1$H NMR (400 MHz, $CDCl_3$) δ 8.28 (d, *J* = 2.2 Hz, 1H), 8.12 (d, *J* = 2.2 Hz, 1H), 7.25 (s, 1H), 6.82 (d, *J* = 4.7 Hz, 1H), 3.78 (s, 1H).

4) Step 4: Compound 19a (300 mg, 1.79 mmol) was dissolved in dimethyl sulfoxide (0.6 mL) and ethanol (3 mL). Sodium hydroxide (86.0 mg, 2.15 mmol) and hydrogen peroxide (0.2 mL, 30% aqueous solution) were added at 0°C. The reaction solution was kept at 0°C and reacted under stirring for 30 min. Water (15 mL) was added to the reaction solution, and ethyl acetate (10 mL × 3) was used for extraction. The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 20/1)

to obtain compound 19b. MS m/z(ESI):186.1[M+1]$^+$.

5) Step 5: Compound 19b (200 mg, 1.08 mmol) and compound 1b (351 mg, 1.30 mmol) were dissolved in water (4 mL) and ethanol (4 mL), and copper sulfate pentahydrate (27.0 mg, 0.110 mmol, Energy Chemical) and sodium ascorbate (21.0 mg, 0.11 mmol, Energy Chemical) were added. The reaction solution was reacted under stirring at 25°C for 12 h, and then concentrated under reduced pressure to remove ethanol. Water (15 mL) was added to the reaction solution, and ethyl acetate (10 mL × 3) was used for extraction. The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 20/1) to obtain compound 19c. MS m/z(ESI):457.2[M+1]$^+$.

6) Step 6: Compound 19c (33.0 mg, 70.0 μmol) was dissolved in dichloromethane (2 mL). Dess-Martin oxidant (61.0 mg, 0.140 mmol) was slowly added to the reaction solution at 30°C. The reaction solution was reacted under stirring at 30°C for 30 min, and then the reaction system was added to saturated sodium bicarbonate aqueous solution (5 mL) for quenching, and dichloromethane (5 mL × 3) was used for extraction. The combined organic phase was dried over anhydrous sodium sulfate and filtered to remove the desiccant, and then the filtrate was concentrated under reduced pressure to obtain compound 19d, the residue was directly used as the raw material for the next step without purification. MS m/z(ESI):455.1[M+1]$^+$.

7) Step 7: Compound 19e (150 mg, 0.440 mmol, prepared by the method disclosed for the step 3 product on page 88 of the specification in the patent application "WO 2022012623 A") was dissolved in N,N-dimethylformamide (2 mL), and compound 19f (104 mg, 0.530 mmol, Bide Pharmatech Ltd.), bistriphenylphosphine palladium dichloride (31.1 mg, 40.0 μmol, Bide Pharmatech Ltd.) and triethylamine (0.3 mL, 1.77 mmol) were added and the obtained mixture was reacted for 0.5 h. The reaction solution was heated to 50°C and reacted at this temperature for 3 h. After the reaction solution returned to 25°C, water (5 mL) was added, and the aqueous phase was extracted with ethyl acetate (5 mL × 3). The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 10/1) to obtain compound 19g. MS m/z(ESI):453.3[M-1]$^-$.

8) Step 8: Compound 19g (60.0 mg, 0.130 mmol) was dissolved in dichloromethane (2 mL), and trifluoroacetic acid (1 mL) was added. The reaction solution was reacted under stirring at 25°C for 2 h. The reaction solution was concentrated under reduced pressure to obtain compound 19h. MSm/z(ESI):355.2[M+1]$^+$.

9) Step 9: Compound 19h (60.0 mg, 0.170 mmol) was dissolved in a mixed solution of tetrahydrofuran (1 mL) and N,N-dimethylformamide (1 mL). Triethylamine (0.02 mL, 0.170 mmol) was added, and the obtained mixture was reacted at 25°C for 10 min. Compound 19d (92.3 mg, 0.200 mmol) and acetic acid (0.02 mL, 0.340 mmol) were added, and the obtained mixture was reacted for 2 h. Then, sodium triacetoxyborohydride (214 mg, 1.02 mmol) was added, and the reaction solution was reacted at 25°C for 1 h. The reaction solution was concentrated under reduced pressure, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 19. MS m/z(ESI):793.7[M+1]$^+$. $^1$HNMR(400MHz,DMSO-$d_6$): δ 11.13 (s, 1H), 9.04 - 8.99 (m, 1H), 8.80 - 8.72 (m, 2H), 8.67 - 8.65 (m, 1H), 8.15 (s, 1H), 7.67 - 7.64 (m, 1H), 7.57 (s, 1H), 7.27 - 7.13 (m, 2H), 7.12 - 7.09 (m, 1H), 7.06 - 7.02 (m, 2H), 6.27 (s, 1H), 5.46 - 5.36 (m, 1H), 4.39 - 4.24 (m, 1H), 3.71 - 3.63 (m, 5H), 3.16 - 3.12 (m, 2H), 2.95 - 2.83 (m, 1H), 2.75 - 2.63 (m, 2H), 2.37 - 2.32 (m, 2H), 2.21 - 2.11 (m, 2H), 2.06 - 1.98 (m, 2H), 1.97 - 1.84 (m, 3H), 1.82 - 1.78 (m, 1H), 1.64 - 1.59 (m, 1H), 1.15 - 1.09 (m, 1H).

Example 20: 3-(4-((4-((((1r,4r)-4-(3-(difluoromethyl)-4-(4-(5-morpholinopyrazolo[1,5-a]pyrimidin-3-yl)- 1H-1,2,3-triazol-1-yl)-1H-pyrazol-1-yl)cyclohexyl)methyl)(methyl)amino)cyclohexyl)amino)-3-methyl-2-oxo-2,3-dihydro-1H-benzo [d]imidazol-1-yl)piperidine-2,6-dione (20)

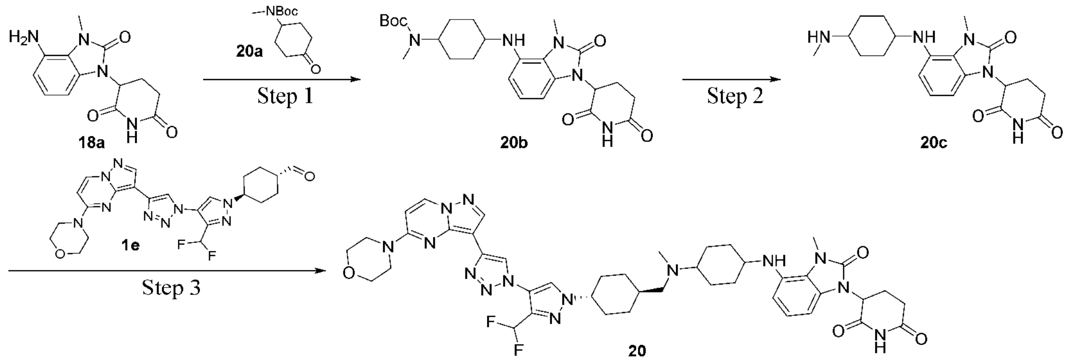


18a 20b 20c 20

1) Step 1: Compound 18a (100 mg, 0.361 mol, prepared by the method disclosed for the intermediate CD on page 394 of the specification of the patent application "WO 2021/188948 A1") and compound 20a (106 mg, 0.471 mmol, Bide Pharmatech Ltd.) were dissolved in dioxane (8 mL), and tetraethyl titanate (0.5 mL, 0.721 mmol) was added. The reaction solution was stirred at 80°C under nitrogen protection for 18 h. The reaction solution was cooled to 25°C and sodium cyanoborohydride(45.3 mg, 0.721 mmol) was added. The reaction solution was stirred at 25°C for 1 h. 50 mL of water was added to the reaction solution, and the obtained mixture was extracted with dichloromethane (50 mL × 3). The organic phases were combined and washed with saturated brine. The organic phase was dried over anhydrous sodium sulfate and filtered to remove the desiccant, and then the filtrate was concentrated under reduced pressure. The residue was purified by silica gel column chromatography with an eluent system (dichloromethane/methanol = 15/1) to obtain compound 20b. MS m/z(ESI):486.3[M+1]$^+$.

2) Step 2: Compound 20b (30.0 mg, 0.0610 mmol) was dissolved in a solution of hydrogen chloride in dioxane (3 mL, 4.0 M), and the reaction solution was reacted at 25°C for 2 h. The reaction solution was concentrated under reduced pressure to obtain compound 20c. MS m/z(ESI):386.5[M+1]$^+$.

3) Step 3: Compound 20c (25.0 mg, 0.0610 mmol) was dissolved in ethanol (3 mL), and triethylamine (8.12 mg, 0.0810 mmol) was added until the pH value was greater than 8. The reaction solution was stirred at 25°C for 10 min. The reaction solution was cooled to -10°C, and acetic acid (186 mg, 0.0910 mmol) and compound 1e (38.7 mg, 0.0810 mmol) were added. The reaction solution was warmed to 25°C, and stirred for 20 min at this temperature, and then sodium cyanoborohydride (10.1 mg, 0.161 mmol, Bide Pharmatech Ltd.) was added. The reaction solution was stirred at 25°C for 1 h. The reaction solution was concentrated under reduced pressure and then purified by high performance liquid chromatography (Waters-2545, chromatographic column: SharpSil-T C18, 30×150 mm, 5 μm; mobile phase: water (containing 10 mmol/L ammonium bicarbonate) and acetonitrile, gradient ratio: acetonitrile 38%-45%, flow rate: 30 mL/min) to obtain compound 20. MS m/z(ESI):867.4[M+1]$^+$,

Example 21: 3-(4-((((1S,4s)-4-((((1r,4R)-4-(3-(difluoromethyl)-4-(4-(5-morpholinopyrazolo[1,5-a] pyrimidin-3-yl)-1H-1,2,3-triazol-1-yl)-1H-pyrazol-1-yl)cyclohexyl)methyl)(methyl)amino)cyclohexyl)methyl)amino)-3-methyl- 2-oxo-2,3-dihydro-1H-benzo[d]imidazol-1-yl)piperidine-2,6-dione (21)

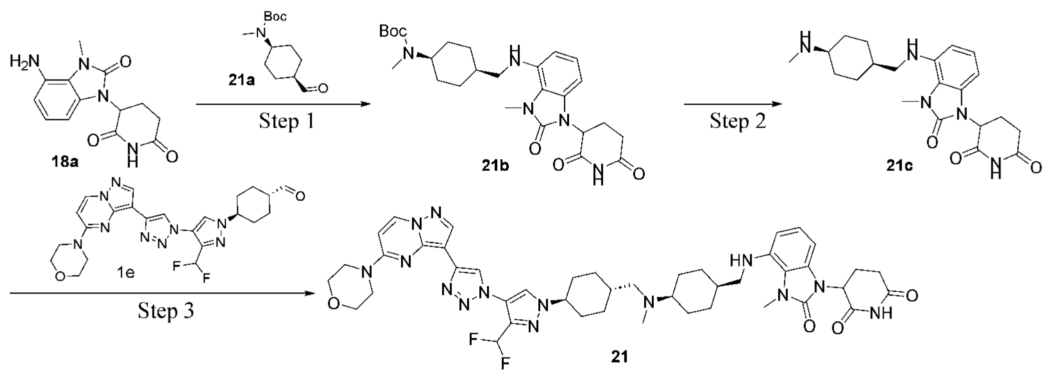


18a 21b 21c 21

1) Step 1: Compound 18a (100 mg, 0.360 mmol, prepared by the method disclosed for step 4 product on page 171 of the specification of the patent application "WO 2022068933 A1") was dissolved in 1,4-dioxane (5 mL), and compound 21a (88.0 mg, 0.360 mmol, Bide Pharmatech Ltd.) and tetraethyl titanate (166 mg, 0.730 mmol) were added. The

reaction solution was reacted under stirring at 80°C for 12 h. The reaction solution was cooled to 25°C, sodium cyanoborohydride (69.0 mg, 1.09 mmol) was added to the reaction solution, and the obtained mixture was stirred for 1 h. Then water (10 mL) was added to the reaction solution, and the aqueous phase was extracted with ethyl acetate (10 mL × 3). The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 20/1) to obtain compound 21b. MS m/z(ESI):522.2[M+23]$^+$.
2) Step 2: Compound 21b (180 mg, 0.360 mmol) was dissolved in dichloromethane (2 mL), and trifluoroacetic acid (2 mL) was added. The reaction solution was reacted under stirring for 1 h at 25°C, and then concentrated under reduced pressure to obtain compound 21c, the residue was directly used as the raw material for the next step without purification.. MS m/z(ESI):400.2[M+1]$^+$.
3) Step 3: Compound 21c (75.0 mg, 0.190 mmol) was dissolved in dichloromethane (3 mL), triethylamine (0.03 mL, 0.190 mmol) was added, and the reaction solution was reacted under stirring at 25°C for 15 min, and then compound 1e (93.0 mg, 0.190 mmol) and glacial acetic acid (0.01 mL, 0.19 mmol) were added. The obtained mixture was reacted under stirring at 25°C for 1 h, and sodium cyanoborohydride (35.0 mg, 0.560 mmol) was added. After reaction under stirring for 1 h, the reaction system was directly concentrated under reduced pressure, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 21. MS m/z(ESI):881.6[M+1]$^+$. $^1$H NMR (400 MHz, DMSO-$d_6$): δ 11.07 (s, 1H), 8.76 (d, $J$ = 7.9 Hz, 1H), 8.74 - 8.55 (m, 2H), 8.54 (s, 1H), 8.39 (s, 1H), 7.33 - 7.07 (m, 1H), 6.90 - 6.78 (m, 2H), 6.56 - 6.32 (m, 2H), 5.38 - 5.23 (m, 1H), 5.11 - 4.90 (m, 1H), 4.37 - 4.26 (m, 1H), 3.73 (s, 8H), 3.63 - 3.61 (m, 2H), 3.57 - 3.55 (m, 1H), 2.93 - 2.88 (m, 2H), 2.69 - 2.62 (m, 2H), 2.44 - 2.37 (m, 3H), 2.17 - 2.11 (m, 2H), 2.01 - 1.92 (m, 5H), 1.90 - 1.81 (m, 4H), 1.69 - 1.53 (m, 4H), 1.37 - 1.21 (m, 3H), 1.16 - 1.00 (m, 4H).

Example 22: N-(2,6-dioxopiperidin-3-yl)-2-fluoro-4-(4-{[4-(2-{[(3Z)-5-[(4-fluorophenyl)sulfamoyl]-2-oxo-2,3-dihydro-1H-indol-3-ylidene]methyl}-3-methyl-1H-indol-6-yl)piperazin-1-yl]methyl}piperidin-1-yl)benzamide (22)

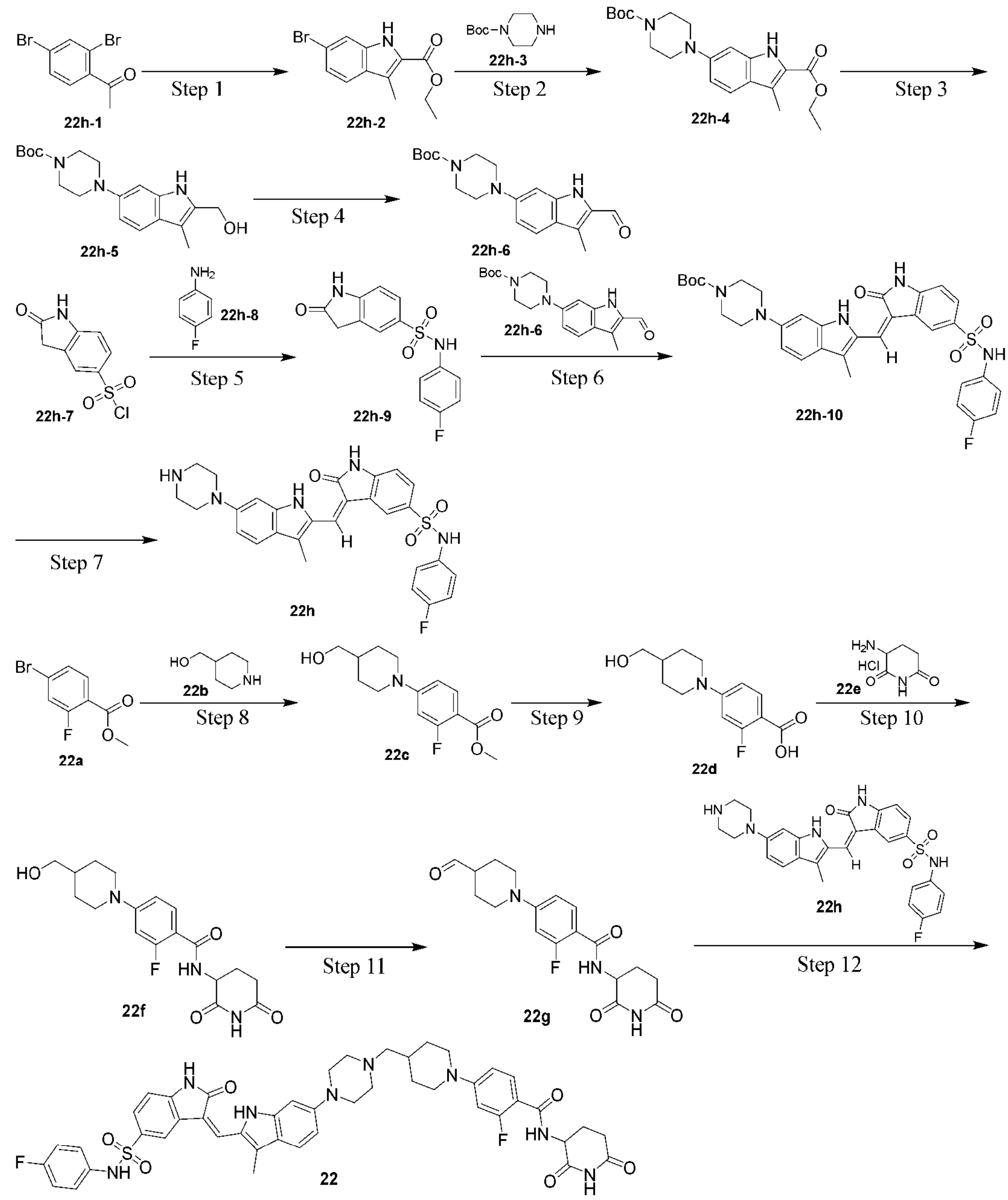


1) Step 1: Compound 22h-1 (2.00 g, 7.20 mmol) and dimethyl sulfoxide (15 mL) were added into a single-neck bottle in sequence and stirred until dissolved. Ethyl isocyanoacetate (895 mg, 7.92 mmol), cuprous iodide (137 mg, 0.72 mmol) and cesium carbonate (4.69 g, 14.4 mmol) were added in sequence. The reaction solution was stirred at 50°C under nitrogen protection for 16 h. 150 mL of water was added to the reaction solution, and ethyl acetate was used for extraction (30 mL × 3). The organic phases were combined, dried over anhydrous sodium sulfate, and filtered, and the filtrate was concentrated under reduced pressure. The residue was purified by column chromatography (petroleum ether/ethyl acetate = 10/1) to obtain compound 22h-2. MS m/z(ESI):284.0[M+1]+.

2) Step 2: Compound 22h-2 (1.20 g, 4.25 mmol) and tetrahydrofuran (18 mL) were added to a single-neck bottle in sequence and stirred until dissolved. Compound 22h-3 (1.19 g, 6.38 mmol), tris(dibenzylideneacetone)dipalladium (194.7 mg, 0.21 mmol), 2-dicyclohexylphosphino-2',4',6'-triisopropylbiphenyl (202.76 mg, 0.43 mmol) and sodium tert-butoxide (834.1 mg, 9.36 mmol) were added in sequence. The reaction solution was stirred at 65°C under nitrogen protection for 16 h. Water (80 mL) was added to the reaction solution, and ethyl acetate was used for extraction (30 mL × 3). The organic phases were combined, dried over anhydrous sodium sulfate, and filtered, and the filtrate was concentrated under reduced pressure. The residue was purified by column chromatography (petroleum ether/ethyl acetate = 3/1) to obtain compound 22h-4. MS m/z(ESI):388.1$[M+1]^+$.

3) Step 3: Lithium aluminum tetrahydride (117.5 mg, 3.10 mmol) and tetrahydrofuran (5 mL) were added to a single-neck bottle, stirred and cooled to 0°C. Compound 22h-4 (600 mg, 1.55 mmol) was dissolved in tetrahydrofuran (5 mL) and stirred until dissolved. The solution of compound 22h-4 in tetrahydrofuran was slowly added dropwise to the solution of lithium aluminum tetrahydride in tetrahydrofuran at 0 °C. The reaction solution was stirred at 0°C for 5 min and stirred at 25°C for 20 min. Saturated ammonium chloride (50 mL) was added to the reaction solution, and ethyl acetate was used for extraction (30 mL × 3). The organic phases were combined, dried over anhydrous sodium sulfate, and filtered, and the filtrate was concentrated under reduced pressure. The residue was purified by column chromatography (petroleum ether/ethyl acetate = 2/1) to obtain compound 22h-5. MS m/z(ESI):346.1$[M+1]^+$.

4) Step 4: Compound 22h-5 (600 mg, 1.74 mmol) and chloroform (15 mL) were added into a single-neck bottle in sequence and stirred until dissolved. Manganese dioxide (1.51 g, 17.37 mmol) was added. The reaction solution was stirred at 25°C for 16 h. The reaction solution was filtered, and the filtrate was concentrated under reduced pressure. The residue was purified by column chromatography (petroleum ether/ethyl acetate = 4/1) to obtain compound 22h-6. MS m/z(ESI):344.1$[M+1]^+$.

5) Step 5: Compound 22h-7 (1.00 g, 4.32 mmol), pyridine (0.68 g, 8.64 mmol), compound 22h-8 (0.96 g, 8.64 mmol) and tetrahydrofuran (10 mL) were added to a single-neck bottle, and the reaction was stirred at 25°C for 2 h. Water (50 mL) was added to the reaction solution, and ethyl acetate was used for extraction (40 mL × 3). The organic phase was dried over anhydrous sodium sulfate and concentrated under reduced pressure. The crude product was slurried with petroleum ether/ethyl acetate (5/1) to obtain compound 22h-9. MS m/z(ESI):307.0$[M+1]^+$.

6) Step 6: Compound 22h-6 (120 mg, 0.35 mmol) and ethanol (2 mL) were added into a single-neck bottle and stirred until dissolved. Then compound 22h-9 (117.1 mg, 0.38 mmol) and piperidine (29.8 mg, 0.35 mmol) were added in sequence. The reaction solution was stirred at 70°C for 2 h. The reaction solution was then cooled to 25°C, and filtered, and the solid was collected and dried to obtain compound 22h-10. MS m/z(ESI):632.1$[M+1]^+$.

7) Step 7: Compound 22h-10 (180 mg, 0.28 mmol) and tetrahydrofuran (2 mL) were added to a single-neck bottle in sequence, and stirred until dissolved, and then hydrogen chloride in dioxane (4 M, 15 mL) was added. The reaction solution was reacted under stirring at 25°C for 0.5 h. The reaction solution was concentrated, the residue was dissolved in tetrahydrofuran (40 mL) and aqueous ammonia (6 mL) was added, and the solution was stirred at ambient temperature for 1 h. After concentration, water (30 mL) was added and the obtained mixture was stirred at ambient temperature for 1 h. Filtration was performed and the solid was collected. The solid was dissolved in tetrahydrofuran (15 mL) and petroleum ether (40 mL) was slowly added under stirring. The solid precipitated and the suspension was stirred for 30 min. Filtration was performed, and the solid was collected and dried to obtain compound 22h. MS m/z(ESI):532.1$[M+1]^+$.

8) Step 8: Compound 22a (1.50 g, 6.44 mmol, Bide Pharmatech Ltd.) and 1,4-dioxane (15 mL) were added to a three-neck bottle in sequence, followed by compound 22b (1.48 g, 12.9 mmol), (2-dicyclohexylphosphino-2',6'-diisopropoxy-1,1'-biphenyl)(2-amino-1,1'-biphenyl-2-yl) palladium (II) methanesulfonate (270 mg, 0.320 mmol, adamas) and cesium carbonate (5.24 g, 16.1 mmol). The reactor was subjected to nitrogen replacement three times and the mixture was stirred at 100°C for 2 h in a nitrogen atmosphere. After the reaction was completed, water (20 mL) was added to the reaction solution and the obtained mixture was extracted with ethyl acetate (50 mL × 2). The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 5/10) to obtain compound 22c. MS m/z(ESI):268.1$[M+1]^+$.

9) Step 9: Compound 22c (1.00 g, 3.74 mmol) and tetrahydrofuran (10 mL) were added to a single-neck bottle in sequence, followed by lithium hydroxide (780 mg, 18.7 mmol) and water (3 mL). The reaction solution was stirred at 80°C for 5 h. After the reaction was completed, ethyl acetate (10 mL) was added and the obtained mixture was extracted with water (20 mL × 2). Hydrochloric acid (1.0 M) was slowly added dropwise to the aqueous phase until the pH value was 3, and solids precipitated. After filtering and washing with water, the filter cake was taken and dried under reduced pressure with an oil pump to obtain compound 22d. $^1H$ NMR (400 MHz, DMSO-$d_6$): δ 12.75 - 12.01 (m, 1H), 7.66 (t, $J$ = 9.0 Hz, 1H), 6.77 - 6.66 (m, 2H), 4.50 (s, 1H), 3.93 - 3.89 (m, 2H), 3.27 - 3.24 (m, 2H), 2.86 - 2.79 (m, 2H), 1.80 - 1.65(m, 2H), 1.65 - 1.55 (m, 1H), 1.20 - 1.05 (m, 2H).

10) Step 10: Compound 22d (200 mg, 0.730 mmol) and *N,N*-dimethylformamide (2 mL) were added in sequence to a single-neck bottle, followed by compound 22e (112 mg, 0.730 mmol), 1-hydroxybenzotriazole (147 mg, 1.09 mmol), 1-(3-dimethylaminopropyl)-3-ethylcarbodiimide hydrochloride (348 mg, 1.82 mmol) and N,N-diisopropylethylamine (0.40 mL, 2.18 mmol), and the reaction solution was stirred at 25°C for 2 h. After the reaction was completed, water (10 mL) was added to the reaction solution and the aqueous phase was extracted with ethyl acetate (20 mL × 2). The combined organic phase was washed with saturated brine (30 mL × 2), dried over anhydrous sodium sulfate and filtered to remove the desiccant, and then the filtrate was concentrated under reduced pressure to obtain an oily residue. 3 to 5 drops of dichloromethane were added to the residue, and solid precipitated after standing for 30 min. The solid was collected to obtain a crude compound 22f.

11) Step 11: Compound 22f (100 mg, 0.280 mmol) was dissolved in dichloromethane (1 mL), and Dess-Martin reagent (175 mg, 0.410 mmol) was slowly added in portions. The reaction solution was stirred at 25°C for 2 h. The reaction

solution was poured into water (10 mL), and the aqueous phase was extracted with ethyl acetate (10 mL × 2). The organic phases were combined and washed with saturated brine (30 mL × 2). The organic phase was dried over anhydrous sodium sulfate and filtered to remove the desiccant, and the filtrate was concentrated under reduced pressure to obtain a residue. The residue was purified by silica gel column chromatography with an eluent system (dichloromethane/methanol = 30/1 to 20/1) to obtain compound 22g. MS m/z(ESI):364.1[M+1]$^+$.

12) Step 12: Compound 22g (50.0 mg, 0.100 mmol) and *N,N*-dimethylformamide (0.5 mL) were added to a single-neck bottle in sequence, followed by compound 22h (51.5 mg, 0.100 mmol) and a drop of glacial acetic acid. The reaction solution was stirred at 25°C for 1 h, and then sodium borohydride acetate (40.9 mg, 0.190 mmol) was added, and the obtained mixture was stirred at 25°C for 1 h. After the reaction solution was filtered, the residue was purified by high performance liquid chromatography (ACSSH-CH, chromatographic column: Phenomenex Gemini NX 150×30 mm, 5 μm; mobile phase: water (containing formic acid) and acetonitrile, gradient ratio: acetonitrile 24%-64%, flow rate: 60 mL/min) to obtain compound 22. MS m/z(ESI):877.5[M+1]$^+$. $^1$H NMR (400 MHz, DMSO-$d_6$): δ 12.74 (s, 1H), 11.31 (s, 1H), 10.84 (s, 1H), 8.22 (s, 1H), 8.00 (t, *J*= 7.3 Hz, 1H), 7.87 (s, 1H), 7.62 (t, *J*= 9.1 Hz, 1H), 7.53 - 7.45 (m, 2H), 7.16 - 7.05 (m, 4H), 6.99 - 6.92 (m, 2H), 6.86 (s, 1H), 6.83 - 6.72 (m, 2H), 4.77 - 4.68 (m, 1H), 3.93 - 3.84 (m, 2H), 3.30 - 3.17 (m, 8H), 2.90 - 2.80 (m, 2H), 2.79 - 2.71 (m, 1H), 2.67 (s, 1H), 2.58 (s, 3H), 2.33 (s, 1H), 2.25 - 2.18 (m, 2H), 2.15 - 2.08 (m, 1H), 2.05 - 1.96 (m, 1H), 1.87 - 1.76 (m, 3H), 1.25 - 1.11 (m, 2H).

Example 23: 3-(4-((7-((((1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-yl)cyclohexyl)methyl)(methyl)amino)spiro[3.5]nonan-2-yl)amino)-3-methyl-2-oxo-2,3-dihydro-1H-benzo[d]imidazol-1-yl)piperidine-2,6-dione (23)

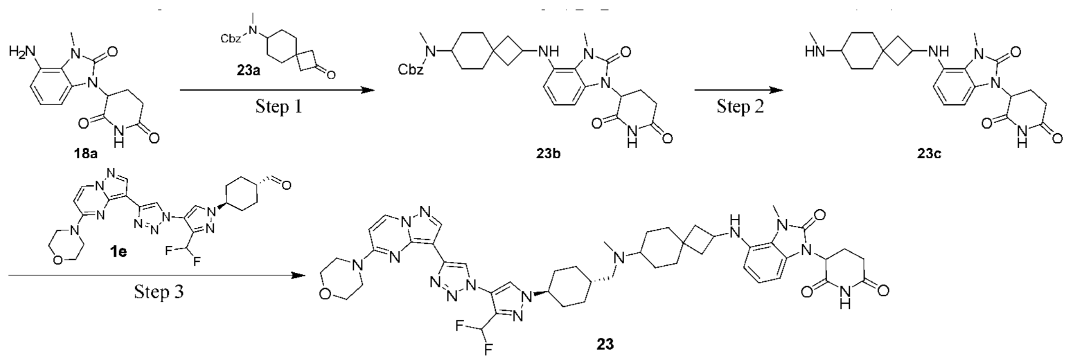

18a 23a 23b 23c 1e 23

1) Step 1: Compound 18a (200 mg, 0.731 mol, prepared by the method disclosed for the intermediate CD on page 394 of the specification of the patent application "WO 2021/188948 A1") and compound 23a (307 mg, 1.02 mmol, prepared by the method disclosed for the intermediate 00967 on page 374 of the specification of the patent application "WO 2021/188948 A1") were dissolved in dioxane (8 mL), and tetraethyl titanate (0.5 mL, 1.46 mmol) was added. The reaction solution was stirred at 80°C under nitrogen protection for 18 h. The reaction solution was cooled to room temperature and sodium cyanoborohydride (91.6 mg, 1.46 mmol) was added. The reaction solution was stirred at 25°C for 1 h. Water (50 mL) was added to the reaction solution, the aqueous phase was extracted with ethyl acetate (50 mL × 3), the combined organic phase was washed with saturated brine (30 mL), dried over anhydrous sodium sulfate, filtered to remove the desiccant and concentrated under reduced pressure. The residue was purified by high performance liquid chromatography (Waters-2545, chromatographic column: SharpSil-T C18, 30×150 mm, 5 μm); mobile phase: water (containing 10 mmol/L formic acid) and acetonitrile, gradient ratio: acetonitrile 38%-45%, flow rate: 30 mL/min) to obtain compound 23b. MS m/z(ESI):560.0[M+1]$^+$.

2) Step 2: Compound 23b (40.0 mg, 0.0710 mmol) was dissolved in methanol (5 mL) and tetrahydrofuran solution (5 mL), wet palladium carbon (10.0 mg, 10%) was added, the reaction solution was subjected to hydrogen replacement three times, stirred at 25°C for 18 h, filtered and concentrated to obtain compound 23c. MS m/z(ESI):426.6[M+1]$^+$.

3) Step 3: Compound 23c (40.0 mg, 0.0910 mmol) and compound 1e (46.8 mg, 0.0910 mmol) were dissolved in 1,2-dichloroethane (2 mL) and tetrahydrofuran (2 mL), and acetic acid (5.41 mg, 0.0910 mmol) was added. The reaction solution was stirred at 25°C for 20 min, and then sodium borohydride acetate (38.2 mg, 0.181 mmol) was added. After the reaction solution was stirred for 1 h, the reaction solution was concentrated under reduced pressure and then purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 23. MS m/z(ESI):907.7[M+1]$^+$.

Example 24: 3-(4-((((1R,4r)-4-((((1r,4R)-4-(3-(difluoromethyl)-4-(4-(5-morpholinopyrazolo[1,5-a] pyrimidin-3-yl)-1H-1,2,3-triazol-1-yl)-1H-pyrazol-1-yl)cyclohexyl)methyl)(methyl)amino)cyclohexyl)methyl)amino)-3-methyl-2-oxo-2,3-dihydro-1H-benzo[d]imidazol-1-yl)piperidine-2,6-dione (24)

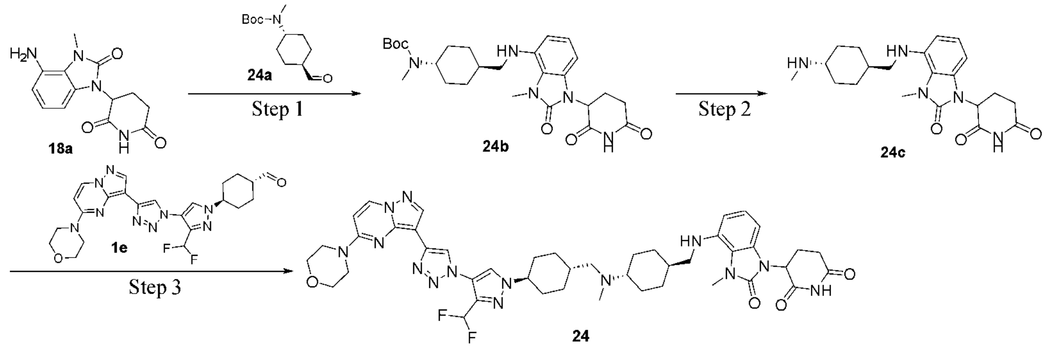


1) Step 1: Compound 18a (54.0 mg, 0.200 mmol, prepared by the method disclosed for step 4 product on page 171 of the specification of the patent application "WO 2022068933 A1") was dissolved in 1,4-dioxane (5 mL), and compound 24a (95.0 mg, 0.390 mmol, Bide Pharmatech Ltd.) and tetraethyl titanate (90.0 mg, 0.390 mmol) were added. The reaction solution was reacted under stirring at 80°C for 12 h. The reaction solution was cooled to 25°C, and sodium cyanoborohydride (37.0 mg, 0.590 mmol) was added to the reaction solution. The obtained mixture was stirred at room temperature for 1 h, then water (10 mL) was added to the reaction solution, and ethyl acetate (10 mL × 3) was used for extraction. The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 20/1) to obtain compound 24b. MS m/z(ESI):444.2 $[M-55]^+$.

2) Step 2: Compound 24b (150 mg, 0.360 mmol) was dissolved in dichloromethane (4 mL), and trifluoroacetic acid (2 mL) was added. The reaction solution was stirred at 25°C for 1 h, and then concentrated under reduced pressure to obtain compound 24c, the residue was directly used as the raw material for the next step without purification. MS m/z(ESI):400.2$[M+1]^+$.

3) Step 3: Compound 24c (25.0 mg, 60.0 μmol) was dissolved in a mixed solution of N,N-dimethylformamide (0.5 mL) and tetrahydrofuran (2 mL), and triethylamine (0.01 mL, 60.0 μmol) was added. The reaction solution was reacted under stirring at 25°C for 15 min, and then compound 1e (31.0 mg, 60.0 μmol) and acetic acid (0.01 mL, 0.190 mmol) were added. The obtained mixture was reacted for 1 h, and then sodium cyanoborohydride (12.0 mg, 0.190 mmol) was added. The obtained mixture was reacted under stirring at room temperature for 1 h, then the reaction system was concentrated, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 24. MS m/z(ESI):881.3 $[M+1]^+$.

Example 25: 3-(4-(4-(4-[(4-)(1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-yl(cyclohexyl)methyl)(methyl)amino)methyl)piperidin-1-yl)but-1-ynyl)-3-methyl-2-oxo-2,3-dihydro-1H-benzo[d]imidazol-1-yl)piperidine-2,6-dione (25)

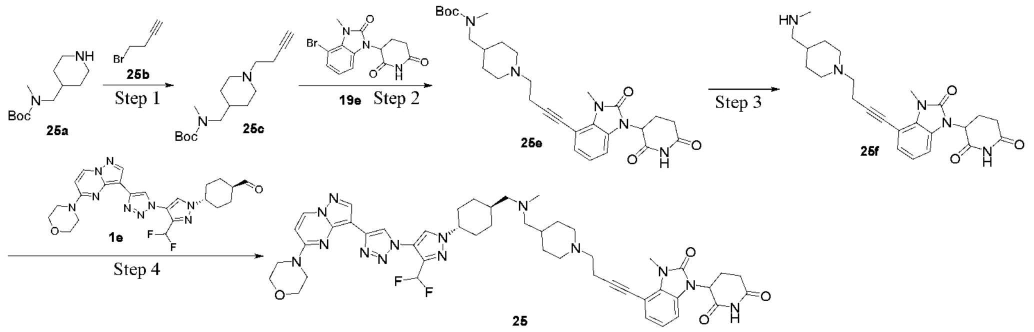


1) Step 1: Compound 25a (1.00 g, 4.38 mmol, Bide Pharmatech Ltd.) and compound 25b (0.6 mL, 6.57 mmol, Bide Pharmatech Ltd.) were dissolved in acetonitrile (20 mL), and potassium carbonate (1.81 g, 13.1 mmol) was added. The reaction solution was reacted under stirring at 80°C for 18 h, and water (20 mL) was added to the reaction solution. The aqueous phase was extracted with ethyl acetate (30 mL × 3). The combined organic phase was washed with saturated brine (20 mL), dried over anhydrous sodium sulfate and filtered to remove the desiccant, and then the filtrate was concentrated under reduced pressure. The residue was purified by silica gel column chromatography with an eluent system (petroleum ether/ethyl acetate = 5/1) to obtain compound 25c. MS m/z(ESI):281.0[M+1]$^+$.

2) Step 2: Compound 25c (200 mg, 0.710 mmol) and compound 19e (241 mg, 0.710 mmol, prepared by the method disclosed for step 3 product on page 88 of the specification of the patent application "WO 2022012623 A") were dissolved in N,N-dimethylformamide (7 mL), and bistriphenylphosphine palladium dichloride (50.0 mg, 0.0710 mmol, adamas), triethylamine (0.3 mL, 2.14 mmol) and cuprous iodide (13.5 mg, 0.0710 mmol) were added. Under nitrogen protection, the reaction was carried out at 80°C for 18 h, the reaction solution was concentrated under reduced pressure, and the residue was purified by silica gel column chromatography with an eluent system (dichloromethane/methanol = 40/1 to 20/1) to obtain compound 25e. MS m/z(ESI):538.0[M+1]$^+$.

3) Step 3: 25e (80.0 mg, 0.145 mmol) was dissolved in dichloromethane (3 mL), trifluoroacetic acid (1 mL) was added, and the reaction solution was reacted at 25°C for 2 h. The reaction solution was concentrated under reduced pressure to obtain 25f. MS m/z(ESI): 438.0[M+1]$^+$.

4) Step 4: Triethylamine (14.2 mg, 0.141 mmol) was added to a solution of compound 25f (61.5 mg, 0.141 mmol) in 1,2-dichloroethane (3 mL) and tetrahydrofuran (5 mL) until the pH was greater than 8, and the mixture was stirred at 25° C for 10 min. Acetic acid (12.6 mg, 0.212 mmol) was added at -10°C, and compound 1e (70.0 mg, 0.141 mmol) was added. Afterwards, the mixture was stirred at 25°C for 20 min and sodium borohydride acetate (59.6 mg, 0.282 mmol, Bide Pharmatech Ltd.) was added. The mixture was stirred for a further 1 h at 25°C. The reaction solution was concentrated under reduced pressure and then purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 25. MS m/z(ESI):919.5[M+1]$^+$. $^1$H NMR (400 MHz, DMSO-$d_6$): δ 11.12 (s, 1H), 8.77 (d, *J* = 7.9 Hz, 1H), 8.66 (s, 1H), 8.54 (s, 1H), 8.38 (s, 1H), 7.34 - 7.09 (m, 2H), 7.06 - 7.03 (m, 1H), 7.02 - 6.96 (m, 1H), 6.82 (d, *J* = 7.8 Hz, 1H), 5.43 - 5.34 (m, 1H), 4.35 - 4.23 (m, 1H), 3.73 (s, 8H), 3.68 (s, 3H), 2.90 (d, *J* = 11.9 Hz, 3H), 2.65 (t, *J* = 9.2 Hz, 3H), 2.57 (d, *J* = 6.6 Hz, 2H), 2.17 - 2.06 (m, 10H), 2.00 - 1.90 (m, 5H), 1.85 - 1.77 (m, 2H), 1.75 - 1.67 (m, 2H), 1.57 - 1.50 (m, 1H), 1.47 - 1.39 (m, 1H), 1.11 - 1.00 (m, 4H).

Example 26: 3-(4-((2-(2-(((1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-yl)cyclohexyl)methyl)-2-azaspiro[3.3]heptan-6-yl)ehtyl)amino)-3-methyl-2-oxo-2,3-dihydro-1H-benzo[d]imidazol-1-yl)piperidine-2,6-dione (26)

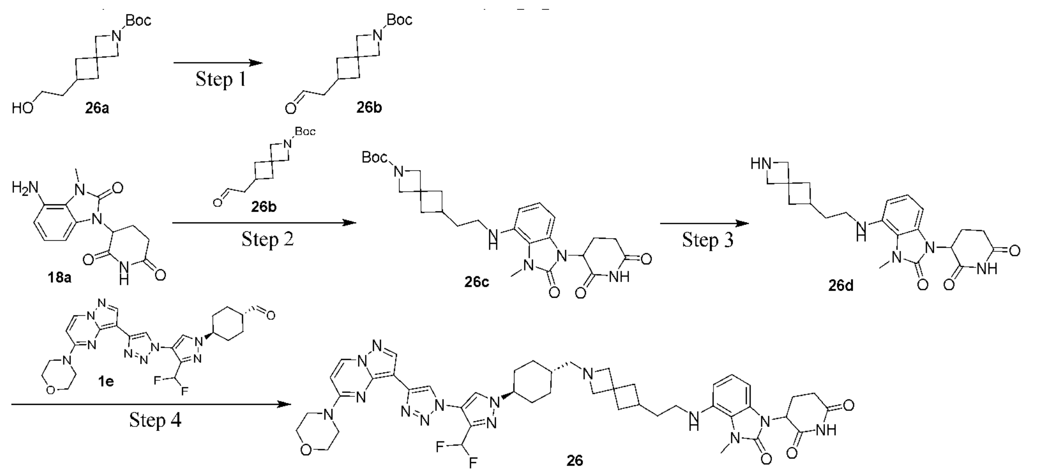


1) Step 1: Compound 26a (310 mg, 1.28 mmol, Bide Pharmatech Ltd.) was dissolved in dichloromethane (10 mL), and Dess-Martin oxidant (1.09 g, 2.57 mmol) was added in portions at 0°C. The reaction solution was stirred for 1 h at 0°C and then filtered through diatomaceous earth. The filtrate was concentrated under reduced pressure, and the residue was purified with neutral alumina and an eluent system (petroleum ether/ethyl acetate = 100/1 to 3/1) to obtain compound 26b. $^1$H NMR (400 MHz, $CDCl_3$): δ 9.83 - 9.60 (m, 1H), 3.95 (s, 2H), 3.81 (s, 2H), 2.64 - 2.51 (m, 3H), 2.44 - 2.35 (m, 2H), 1.91 - 1.83 (m, 2H), 1.43 (s, 9H).

2) Step 2: Compound 18a (220 mg, 0.800 mmol, prepared by the method disclosed for the intermediate 495 on page 172 of the specification of the patent application "WO 2022068933 A1"), compound 26b (230 mg, 0.960 mmol) and ethyl titanate (0.3 mL, 1.60 mmol) were dissolved in dioxane (10 mL). The reaction solution was stirred at 100°C under nitrogen protection for 12 h, and then the reaction solution was cooled to 25°C. Sodium cyanoborohydride (252 mg, 4.01 mmol) was added, and the reaction was stirred at 25°C for 1 h, and then the reaction solution was quenched with water (20 mL) and ethyl acetate (20 mL). The reaction solution was filtered through diatomaceous earth, and the aqueous phase was subjected to liquid separation and then extracted with ethyl acetate (10 mL × 3). The combined organic phase was dried over anhydrous sodium sulfate and filtered to remove the desiccant, and then the filtrate was concentrated under reduced pressure. The residue was purified by silica gel column chromatography with an eluent system (dichloromethane/methanol = 100/1 to 20/1) to obtain compound 26c. MS m/z(ESI):520.2[M+23]$^+$. $^1$H NMR (400 MHz, $CDCl_3$): δ 8.14 (s, 1H), 7.66 - 7.55 (m, 1H), 7.10 - 7.06 (m, 1H), 6.87 - 6.84 (m, 1H), 5.25 - 5.19 (m, 1H), 3.88 (s, 4H), 3.75 (s, 2H), 3.28 - 3.21 (m, 2H), 2.26 - 2.20 (m, 4H), 2.14 - 2.06 (m, 3H), 1.80 - 1.71 (m, 4H), 1.43 - 1.42 (m, 10H).

3) Step 3: Compound 26c (80.0 mg, 0.160 mmol) was dissolved in dichloromethane (4 mL), and trifluoroacetic acid (1 mL) was added at 25°C. The reaction solution was stirred for 1 h and then concentrated under reduced pressure to obtain compound 26d. MS m/z(ESI):398.2[M+1]$^+$.

4) Step 4: Compound 26d (62.3 mg, 0.160 mmol) was dissolved in 1,2-dichloroethane (2 mL) and tetrahydrofuran (2 mL), and triethylamine (13.2 mg, 0.130 mmol) was added. The reaction solution was stirred at 25°C for 10 min, and then acetic acid (7.85 mg, 0.130 mmol) and compound 1e (65.0 mg, 0.130 mmol) were added. The reaction solution was stirred at 25°C under nitrogen protection for 1 h, and then sodium borohydride acetate (166 mg, 0.780 mmol) was added. The reaction solution was stirred at 25°C under nitrogen protection for 12 h, and then quenched with saturated sodium bicarbonate aqueous solution (5 mL) and dichloromethane (10 mL). The aqueous phase was subjected to liquid separation and then extracted with dichloromethane (3 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-T C18,30×150 mm, 5 μm; mobile phase: water (containing 10 mmol/L formic acid) and acetonitrile, gradient ratio: acetonitrile 38%-45%, flow rate: 30 mL/min) to obtain compound 26. MS m/z(ESI):879.4[M+1]$^+$. $^1$H NMR (400 MHz, *DMSO-d*$_6$): δ 11.05 (s, 1H), 8.76 (d, *J* = 7.8 Hz, 1H), 8.63 (s, 1H), 8.53 (s, 1H), 8.37 (s, 1H), 7.18 (t, *J* = 53.2 Hz, 1H), 6.88 - 6.84 (m, 1H), 6.81 (d, *J* = 7.9 Hz, 1H), 6.49 (d, *J* = 8.0 Hz, 1H), 6.39 (d, *J* = 8.2 Hz, 1H), 5.30 - 5.24 (m, 1H), 5.04 - 4.86 (m, 1H), 4.31 - 4.19 (m, 1H), 3.72 (s, 8H), 3.60 (s, 3H), 3.13 - 3.10 (m, 2H), 2.99 - 2.83 (m, 5H), 2.69 - 2.65 (m, 2H), 2.34 - 2.31 (m, 1H), 2.29 - 2.26 (m, 2H), 2.23 - 2.21 (m, 2H), 2.12 - 2.07 (m, 2H), 2.01 - 1.92 (m, 2H), 1.88 - 1.84 (m, 2H), 1.78 - 1.68 (m, 5H), 1.12 - 1.03 (m, 2H).

Example 27: 3-(4-(1-((1r,4r)-4-(3-(difluoromethyl)-4-(4-(5-morpholinylpyrazolo[1,5-a]pyrimidin-3-yl)-1H-1,2,3-triazol-1-yl)-1H-pyrazol-1-yl)cyclohexyl)azabutan-3-ylethyl)-3-methyl-2-oxo-2,3-dihydro-1H-benzo[d]imidazol-1-yl)piperidine-2,6-dione (27)

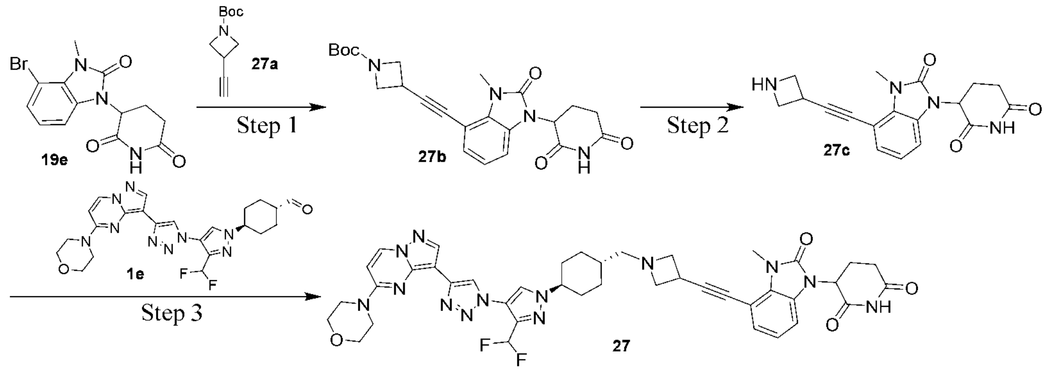


1) Step 1: Compound 19e (120 mg, 0.661 mmol, prepared by the method disclosed for step 3 product on page 88 of the specification of the patent application "WO 2022012623 A") and compound 27a (172 mg, 0.508 mmol, Bide Pharmatech Ltd.) were dissolved in N,N-dimethylformamide (2 mL), and triethylamine (0.2 mL, 1.53 mmol), cuprous iodide (19.0 mg, 0.1 mmol) and dichlorobis(triphenylphosphine)palladium (II) (35.8 mg, 51.0 μmol) were added. The reaction solution was subjected to nitrogen replacement, and then stirred at 80°C for 12 h. The reaction solution was cooled to room temperature, water (5 mL) was added, and the aqueous phase was extracted with ethyl acetate (5 mL × 3). The organic phase was washed with saturated brine (5 mL), dried over anhydrous sodium sulfate, filtered and concentrated under reduced pressure. The residue was subjected to silica gel column chromatography (dichloromethane/methanol = 100/1 to 20/1) to obtain compound 27b. MS m/z(ESI): 461.1[M+23]$^+$.

2) Step 2: Compound 27b (50.0 mg, 0.114 mmol) was dissolved in dichloromethane (1 mL), trifluoroacetic acid (0.5 mL) was added, and the reaction was stirred at 20°C for 1 h. The reaction solution was concentrated under reduced pressure to obtain a crude product, which was purified by reverse phase column (water/acetonitrile = 25/75 to 80/20) to obtain compound 27c. MS m/z(ESI): 339.1[M+1]$^+$.

3) Step 3: Compound 27c (30.0 mg, 88.7 μmol) and compound 1e (44.1 mg, 88.7 μmol) were dissolved in N,N-dimethylformamide (1 mL), triethylamine (8.97 mg, 88.7 μmol) was added, and the obtained mixture was stirred for 5 min. Then glacial acetic acid (7.95 mg, 133 μmol) was added, and the reaction solution was stirred at 40°C for 2 h, and then sodium borohydride acetate (37.4 mg, 177 μmol) was added and the obtained mixture was stirred at 40°C for 2 h. After the reaction was filtered, purification was performed by high performance liquid chromatography (ACSSH-CP, chromatographic column: C18 150×30 mm; mobile phase: water (formic acid)-acetonitrile, gradient ratio: acetonitrile 3%-43%, flow rate: 30 mL/min) to obtain compound 27. MS m/z(ESI):820.4[M+1]$^+$.

Example 28: 4-(4-((1r,4r)-4-(3-(difluoromethyl)-4-(4-(5-morpholinopyrazolo[[1,5-a]pyrimidin-3-yl)-1H-1,2,3-triazol-1-yl)-1H-pyrazol-1-yl)cyclohexylmethyl) piperazin-1-yl)-N-(2,6-dioxopiperidin-3-yl)-2-fluorobenzamide (28)

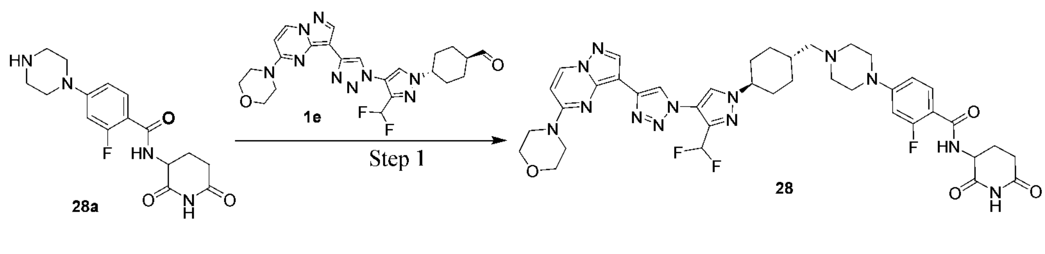


Compound 28a (80.0 mg, 220 μmol, prepared by the method disclosed for the intermediate step 7 compound on page 867 of the specification of the patent application "WO 2023/017446 Al"), compound 1e (107 mg, 220 μmol) and triethylamine (0.05 mL) were dissolved in tetrahydrofuran (3 mL) and N,N-dimethylformamide (1.5 mL). The reaction solution was stirred at 15°C for 10 min, and then acetic acid (0.05 mL) and sodium triacetoxyborohydride (136 mg, 0.65 mmol) were added. The reaction solution was reacted under stirring at 15°C for half an hour. Water (15 mL) was added to

the reaction solution, and the aqueous phase was extracted with dichloromethane (15 mL × 3), dried, and concentrated. The residue was purified by high performance liquid chromatography (Waters-2545, chromatographic column: Boston Green ODS 150×30 mm, 5um; mobile phase: Waters (FA-$CH_3CN$, gradient ratio: acetonitrile 16%-56%, flow rate: 30 mL/min) to obtain compound 28. MS m/z(ESI):816.5[M+1]$^+$. $^1$H NMR (400 MHz, DMSO-$d_6$): δ 10.85 (s, 1H), 8.76 (d, *J* = 7.9 Hz, 1H), 8.66 (s, 1H), 8.54 (s, 1H), 8.38 (s, 1H), 8.05 (t, *J* = 7.2 Hz, 1H), 7.63 (t, *J* = 9.0 Hz, 1H), 7.34 - 7.05 (m, 1H), 6.87 - 6.74 (m, 3H), 4.79 - 4.67 (m, 1H), 4.36 - 4.22 (m, 1H), 3.72 (s, 8H), 3.30 (s, 8H), 2.84 - 2.70 (m, 1H), 2.47 - 2.46 (m, 1H), 2.24 - 2.06 (m, 5H), 2.05 - 1.92 (m, 3H), 1.90 - 1.76 (m, 2H), 1.75 - 1.60 (m, 1H), 1.20 - 1.00 (m, 2H).

Example 29: 3-(4-(4-(((1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)amino)piperidin-1-carbonyl)-3-methyl-2-oxo-2,3-dihydro-1H-benzoimidazol-1-yl)piperidine-2,6-dione (29)

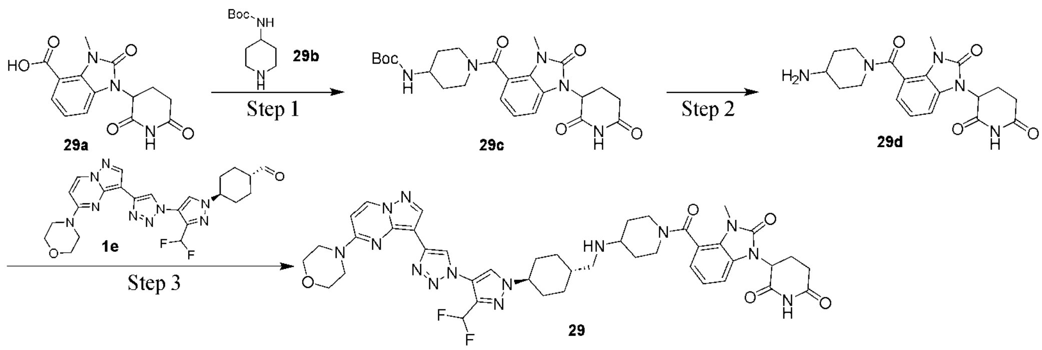


1) Step 1: Compounds 29a (200 mg, 0.660 mmol, prepared by the method disclosed in the specification of the patent application "WO 2020/113233 A1") and compound 29b (150 mg, 0.750 mmol, Bide Pharmatech Ltd.) were dissolved in N,N-dimethylformamide (5 mL), and 2-(7-azobenzotriazole)-N,N,N',N'-tetramethyluronium hexafluorophosphate (250 mg, 0.660 mmol) and N,N-diisopropylethylamine (250 mg, 1.93 mmol) were added in sequence. The reaction solution was stirred at 25°C for 2 h. The reaction solution was poured into water (30 mL), and ethyl acetate (10 mL × 3) was used for extraction. The combined organic phase was washed with saturated brine (50 mL), dried over anhydrous sodium sulfate and filtered. The filtrate was concentrated under reduced pressure, and the residue was purified by silica gel column chromatography with an eluent system (dichloromethane/methanol = 60/1 to 10/1) to obtain compound 29c. MS m/z(ESI):508.2[M+23]$^+$.

2) Step 2: Compound 29c (120 mg, 0.250 mmol) was dissolved in dichloromethane (3 mL), and trifluoroacetic acid (1 mL) was added. The reaction solution was stirred at 25°C for 2 h. The reaction solution was concentrated under reduced pressure to obtain compound 29d. MS m/z(ESI):386.1[M+1]$^+$.

3) Step 3: Compound 29d (50.0 mg, 0.130 mmol) and compound 1e (65.0 mg, 0.130 mmol) were dissolved in tetrahydrofuran (2 mL) and N,N-dimethylformamide (1 mL), and acetic acid (0.1 mL) and sodium borohydride acetate (75.0 mg, 0.360 mmol) were added in sequence. The reaction solution was stirred at 25°C for 2 h. The reaction solution was poured into water (10 mL) and the aqueous phase was extracted with dichloromethane (10 mL × 3). The combined organic phase was washed with saturated brine (20 mL), dried over anhydrous sodium sulfate and filtered. The filtrate was concentrated under reduced pressure, and the residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 29. MS m/z(ESI):867.7[M+1]$^+$.

Example 30: 3-(4-(1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)piperazin-1-yl)-3-methyl-2-oxo-2,3-dihydro-1H-benzoimidazol-1-yl)piperidine-2,6-dione (30)

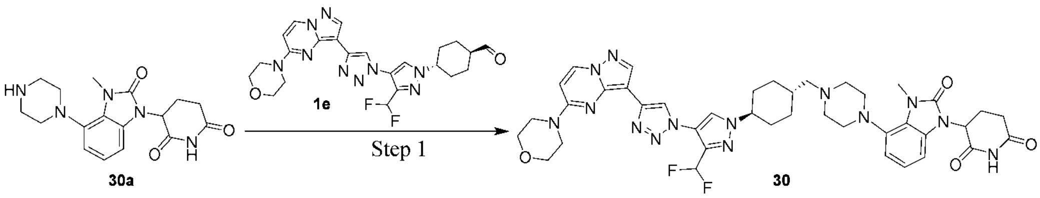


Compound 30a (30.0 mg, 90.0 μmol, prepared by the method disclosed for the intermediate GS on page 550 of the specification of the patent application "WO 2022236058 A1") was dissolved in dichloroethane (2 mL) and N,N-dimethylformamide (1 mL) solution, and triethylamine (0.1 mL) was added. The reaction solution was stirred for 0.5 h. Compound 1e (52.0 mg, 0.100 mmol) and acetic acid (0.2 mL) were added to the reaction solution, and the obtained mixture was stirred for 0.5 h. Sodium borohydride acetate (55.0 mg, 0.260 mmol) was added to the reaction solution. The reaction solution was stirred at 25°C for 2 h. The reaction solution was concentrated under reduced pressure and then purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 30. MS m/z(ESI):825.6[M+1]$^+$. $^1$HNMR (400 MHz, DMSO-$d_6$):δ 11.11 (s, 1H), 8.78 (d, *J* = 7.8 Hz, 1H), 8.68 (s, 1H), 8.55 (s, 1H), 8.40 (s, 1H), 7.22 (t, *J* = 53.2 Hz, 1H), 7.04 - 6.90 (m, 3H), 6.86 - 6.80 (m, 1H), 5.42 - 5.32 (m, 1H), 4.41 - 4.22 (m, 1H), 3.75 (s, 8H), 3.66 (s, 3H), 3.10 - 2.83 (m, 7H), 2.75 - 2.59 (m, 3H), 2.29 - 2.07 (m, 4H), 2.04 - 1.94 (m, 3H), 1.93 - 1.81 (m, 2H), 1.37 - 0.91 (m, 4H).

Example 31: 7-(1-(3-(difluoromethyl)-1-(1r,4r)-4-(4-(4-(2,6-dioxopyridin-3-yl)carbamoyl)-3-fluorophenylpiperazin-1-yl) methyl)cyclohexyl-1H-pyrazol-4-yl)-1H-1,2,3-triazol-4-yl)pyrrolo[1,2-b]pyridazine-3-carboxamide (31)

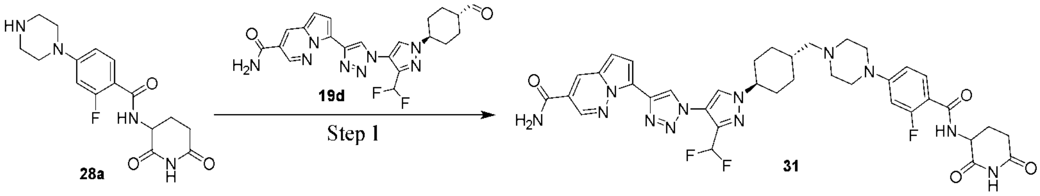


Compound 28a (40.0 mg, 0.110 mmol, prepared by the method disclosed for the intermediate step 7 compound on page 867 of the specification of the patent application "WO 2023/017446 Al"), compound 19d (49.0 mg, 0.110 mmol) and triethylamine (0.1 mL) were dissolved in tetrahydrofuran (2 mL) and N,N-dimethylformamide (1 mL), and the reaction solution was stirred at 15°C for 10 min. Acetic acid (0.1 mL) and sodium triacetoxyborohydride (68.3 mg, 0.320 mmol) were added to the reaction solution, and the obtained mixture was reacted under stirring at 15°C for half an hour. Water (10 mL) was added to the reaction solution, and the organic phase was extracted with dichloromethane (10 mL × 3). The combined organic phase was dried over anhydrous sodium sulfate, filtered, and concentrated. The residue was purified by high performance liquid chromatography (Waters-2545, chromatographic column: C18 150×30 mm; mobile phase: Waters (FA-$CH_3CN$, gradient ratio: acetonitrile 16%-56%, flow rate: 30 mL/min) to obtain compound 31. MS m/z(ESI):773.4 [M+1]$^+$. $^1$H NMR (400 MHz, DMSO-$d_6$): δ 10.85 (s, 1H), 9.01 (s, 1H), 8.78 (d, *J* = 2.2 Hz, 1H), 8.75 (s, 1H), 8.66 (d, *J* = 2.2 Hz, 1H), 8.19 - 8.12 (m, 1H), 8.06 (d, *J* = 6.7 Hz, 1H), 7.68 - 7.54 (m, 3H), 7.38 - 7.09 (m, 1H), 7.05 (d, *J* = 4.6 Hz, 1H), 6.89 - 6.74 (m, 2H), 4.81 - 4.67 (m, 1H), 4.40 - 4.23 (m, 1H), 3.32 - 3.28 (m, 8H), 2.85 - 2.72 (m, 1H), 2.46 - 2.44 (m, 1H), 2.26 - 2.09 (m, 5H), 2.02 - 1.94 (m, 3H), 1.89 - 1.78 (m, 2H), 1.72 - 1.59 (m, 1H), 1.20 - 1.05 (m, 2H).

Example 32: 3-(4-((1r,4r)-4-(3-(difluoromethyl)-4-(4-(5-morpholinopyrazolo[[1,5-a]pyrimidin-3-yl)-1H-1,2,3-triazol-1-yl)-1H-pyrazol-1-yl)cyclohexylmethyl)-2,7-diazaspiro[4.4]non-2-yl)-3-methyl-2-oxo-2,3-dihydro-1H-benzo[d]imidazol-1-yl)piperidine-2,6-dione (32)

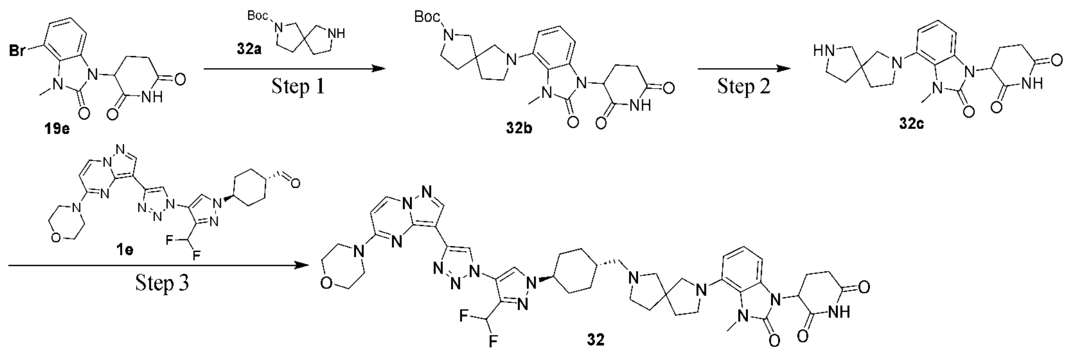


1) Step 1: Compound 19e (300 mg, 0.890 mmol, prepared by the method disclosed for step 3 product on page 88 of the specification of the patent application "WO 2022012623 A") was dissolved in toluene (10 mL). Compound 32a (301 mg, 1.33 mmol, Accela ChemBio Co., Ltd.), palladium acetate (39.8 mg, 0.180 mmol) and 2-dicyclohexylphosphino-2,6-diisopropoxy-1,1-biphenyl (82.8 mg, 0.180 mmol) were added. Lithium bis(trimethylsilyl)amide(4.4 mL, 4.44 mmol, 1.0 M solution in tetrahydrofuran) was added dropwise into the reaction solution under nitrogen protection. The reaction solution was reacted under stirring at 80°C under nitrogen protection for 2 h. Water (30 mL) was added to the reaction solution, the aqueous phase was extracted with dichloromethane (30 mL × 3), and the combined organic phase was dried over anhydrous sodium sulfate and filtered. The filtrate was concentrated under reduced pressure. The residue was purified by silica gel column chromatography with an eluent system (petroleum ether/ethyl acetate = 3/1 to 1/1) to obtain compound 32b. MS m/z(ESI):484.3$[M+1]^+$.

2) Step 2: Compound 32b (200 mg, 0.410 mmol) was dissolved in dichloromethane (4 mL), trifluoroacetic acid (4 mL) was added, and the obtained mixture was reacted under stirring at 15°C for 30 min. The reaction solution was concentrated to obtain compound 32c. MS m/z(ESI):384.0$[M+1]^+$.

3) Step 3: Compound 32c (100 mg, 0.260 mmol), compound 1e (130 mg, 0.260 mmol) and triethylamine (0.5 mL) were dissolved in tetrahydrofuran (3 mL) and N,N-dimethylformamide (1.5 mL), and the obtained mixture was stirred at 15°C for 10 min. Afterwards, acetic acid (0.5 mL) and sodium triacetoxyborohydride (165 mg, 0.780 mmol) were added to the reaction solution, and the obtained mixture was reacted under stirring at 15°C for half an hour. Water (15 mL) was added to the reaction solution, and dichloromethane (15 mL × 3) was used for extraction. The organic phase was dried with anhydrous sodium sulfate and filtered. The filtrate was concentrated under reduced pressure, and the residue was purified by high performance liquid chromatography (Waters-2545, chromatographic column: C18 150×30 mm; mobile phase: Waters (FA)-$CH_3CN$, gradient ratio: acetonitrile 13%-53%, flow rate: 30 mL/min) to obtain compound 32. MS m/z(ESI):865.4$[M+1]^+$. $^1H$ NMR (400 MHz, DMSO-$d_6$): δ 11.10 (s, 1H), 8.76 (d, *J* = 7.9 Hz, 1H), 8.65 (s, 1H), 8.53 (s, 1H), 8.38 (s, 1H), 8.18 (s, 1H), 7.33 - 7.05 (m, 1H), 7.00 - 6.93 (m, 2H), 6.85 (dd, *J* = 1.9, 6.2 Hz, 1H), 6.81 (d, *J* = 7.9 Hz, 1H), 5.34 (dd, *J* = 5.4, 12.6 Hz, 1H), 4.28 (m, *J* = 3.4, 8.3, 11.9 Hz, 1H), 3.72 (s, 8H), 3.61 - 3.60 (m, 2H), 3.11 - 3.03 (m, 3H), 3.00 - 2.88 (m, 2H), 2.73 - 2.57 (m, 5H), 2.33 (d, *J* = 6.8 Hz, 2H), 2.17 - 2.09 (m, 2H), 2.03 - 1.77 (m, 10H), 1.59 - 1.47 (m, 1H), 1.16 - 1.03 (m, 2H).

Example 33: 3-(4-((1R,4R)-4-(3-(difluoromethyl)-4-(4-(5-morpholinopyrazolo[[1,5-a]pyrimidin-3-yl)-1H-1,2,3-triazol-1-yl)-1H-pyrazol-1-yl)cyclohexyl)methylhexahydropyrrolo[3,4-c]pyrrol-2(1H)-yl)-3-methyl-2-oxo-2,3-dihydro-1H-benzo[d]imidazol-1-yl)piperidine-2,6-dione (33)

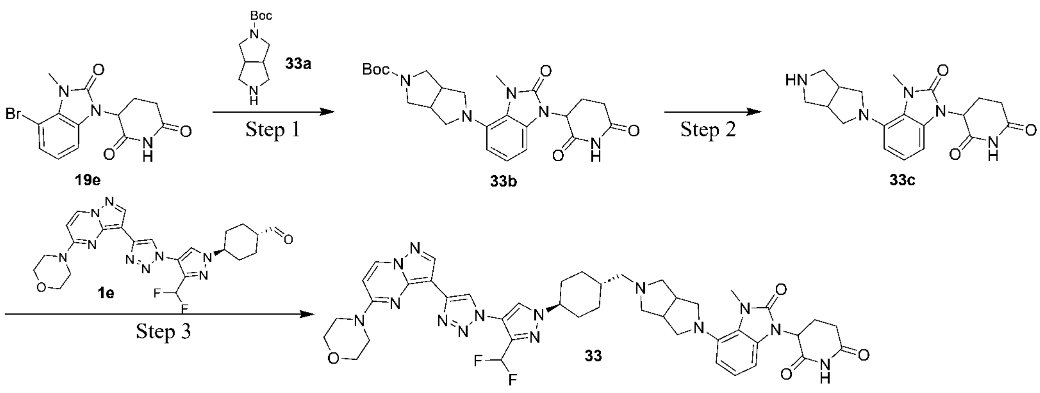

1) Step 1: Compound 33a (314 mg, 1.48 mmol, Bide Pharmatech Ltd.) was dissolved in toluene (5 mL). Compound 19e (500 mg, 1.48 mmol, prepared by the method disclosed for step 3 product on page 88 of the specification of the patent application "WO 2022012623 A"), 2-dicyclohexylphosphino-2,6-diisopropoxy-1,1-biphenyl (138 mg, 0.300 mmol) and (2-amino-[1,1-biphenyl]-2-yl)(dicyclohexyl(2,6-diisopropoxy-[1,1-biphenyl]-2-yl)phosphoryl)palladium chloride (230 mg, 0.300 mmol) were added. Under nitrogen protection, lithium bis(trimethylsilyl)amide (3.7 mL, 3.70 mmol, 1.0 M solution in tetrahydrofuran) was added dropwise to the reaction solution. The reaction solution was reacted at 80°C for 2 h. The reaction solution was concentrated under reduced pressure, and the residue was purified by silica gel column chromatography with an eluent system (petroleum ether/ethyl acetate = 3/1 to 1/1) to obtain compound 33b. MS m/z(ESI):470.3 $[M+1]^+$.

2) Step 2: Compound 33b (50.0 mg, 0.110 mmol) was dissolved in dichloromethane (2 mL), and trifluoroacetic acid (0.4 mL) was added. The reaction solution was reacted at 25°C for 1 h. The reaction solution was concentrated under reduced pressure to obtain compound 33c. MS m/z(ESI):370.2$[M+1]^+$.

3) Step 3: Compound 33c (39.0 mg, 0.110 mmol), compound 1e (57.8 mg, 0.120 mmol) and triethylamine (21.4 mg, 0.210 mmol) were dissolved in methanol (1 mL) and N,N-dimethylformamide (0.5 mL). Sodium cyanoborohydride (5.71 mg, 30.0 μmol) was added. The obtained mixture was reacted at room temperature for 2 h. The reaction solution was concentrated under reduced pressure and then purified by high performance liquid chromatography (Gilson GX-281, chromatographic column: Boston Prime C18, 30×150 mm, 5 μm; mobile phase: water (containing 0.0500% aqueous ammonia and 10.0 mmol/L ammonium bicarbonate) and acetonitrile, gradient ratio: acetonitrile 40%-70%, flow rate: 25 mL/min) to obtain compound 33. MS m/z(ESI):851.4$[M+1]^+$. $^1H$ NMR (400 MHz, DMSO-$d_6$): δ 11.10 (s, 1H), 8.76 (d, $J$ = 4.9 Hz, 1H), 8.66 (s, 1H), 8.54 (s, 1H), 8.38 (s, 1H), 7.37 - 7.04 (m, 1H), 6.98 - 6.79 (m, 4H), 5.45 - 5.22 (m, 1H), 4.37 - 4.20 (m, 1H), 3.76 - 3.64 (m, 11H), 3.16 (s, 2H), 2.94 - 2.60 (m, 10H), 2.25 (s, 2H), 2.13 (s, 2H), 1.99 (s, 3H), 1.82 (d, $J$ = 10.3 Hz, 2H), 1.59 - 1.45 (m, 1H), 1.28 - 1.00 (m, 3H).

Example 34: 3-(4-(4-((1R,4R)-4-(3-(difluoromethyl)-4-(4-(5-morpholinopyrazolo[[1,5-a]pyrimidin-3-yl)-1H-1,2,3-triazol-1-yl)-1H-pyrazol-1-yl)cyclohexyl-methylpiperazin-1-yl)piperazin-1-yl)-3-methyl-2-oxo-2,3-dihydro-1H-benzo[d]imidazol-1-yl)piperazin-2,6-dione (34)

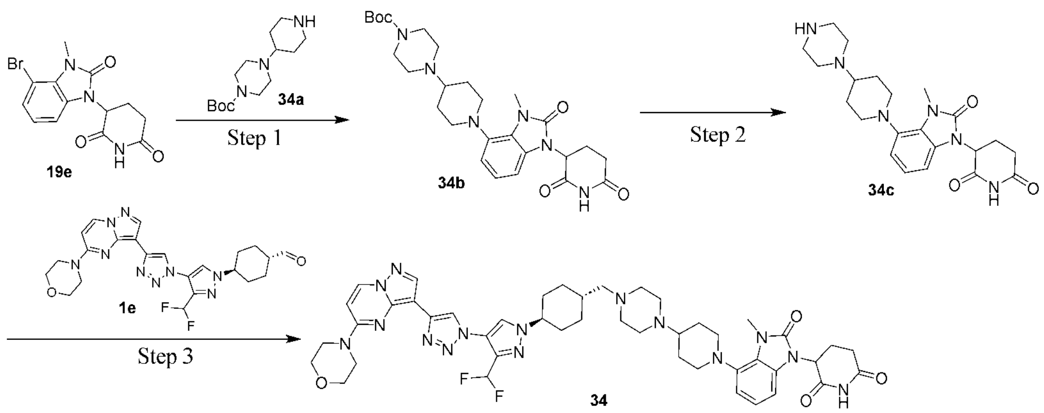


1) Step 1: Compound 34a (398 mg, 1.48 mmol, Bide Pharmatech Ltd.) was dissolved in toluene (5 mL), and compound 19e (500 mg, 1.48 mmol, prepared by the method disclosed for step 3 product on page 88 of the specification of the patent application "WO 2022012623 A"), 2-dicyclohexylphosphino-2,6-diisopropoxy-1,1-biphenyl (138 mg, 0.300 mmol) and (2-amino-[1,1-biphenyl]-2-yl)(dicyclohexyl(2,6-diisopropoxy-[1,1-biphenyl]-2-yl)phosphoryl)palladium chloride (230 mg, 0.300 mmol) were added. Lithium bis(trimethylsilyl)amide (3.7 mL, 3.70 mmol, 1.0 M solution in tetrahydrofuran) was added dropwise to the reaction solution under nitrogen protection. The obtained mixture was reacted at 80°C for 12 h under nitrogen atmosphere. The reaction solution was concentrated under reduced pressure, and purified by silica gel column chromatography with an eluent system (petroleum ether/ethyl acetate = 3/1 to 1/1) to obtain compound 34b. MS m/z(ESI):527.3$[M+1]^+$.

2) Step 2: Compound 34b (60.0 mg, 0.110 mmol) was dissolved in dichloromethane (2 mL), and trifluoroacetic acid (0.4 mL) was added. The obtained mixture was reacted at room temperature for 1 h. The reaction solution was concentrated under reduced pressure to obtain compound 34c.

3) Step 3: Compound 34c (48.0 mg, 0.110 mmol), compound 1e (61.6 mg, 0.120 mmol) and triethylamine (22.8 mg, 0.230 mmol) were dissolved in methanol (1 mL) and N,N-dimethylformamide (0.5 mL). Sodium cyanoborohydride (5.71 mg, 30.0 μmol) was added. The reaction solution was reacted at 25°C for 2 h. The reaction solution was

concentrated under reduced pressure and then purified by high performance liquid chromatography (Gilson GX-281, chromatographic column: Boston Prime C18, 30×150 mm, 5 μm; mobile phase: water (containing 0.225% formic acid) and acetonitrile, gradient ratio: acetonitrile 11%-41%, flow rate: 25 mL/min) to obtain compound 34. MS m/z(ESI):908.4[M+1]$^+$. $^1$H NMR (400 MHz, DMSO-$d_6$): δ 11.23 - 10.98 (m, 1H), 8.74 (d, *J* = 7.9 Hz, 1H), 8.64 (s, 1H), 8.53 (s, 1H), 8.38 (s, 1H), 8.20 (s, 1H), 7.19 (t, *J* = 53.2 Hz, 1H), 7.00 - 6.94 (m, 1H), 6.92 - 6.85 (m, 2H), 6.80 (d, *J* = 7.9 Hz, 1H), 5.33 (m, *J* = 5.3,12.6 Hz, 1H), 4.35 - 4.21 (m, 1H), 3.62 (s, 3H), 3.20 - 3.07 (m, 4H), 2.95 - 2.79 (m, 2H), 2.76 - 2.62 (m, 6H), 2.44 - 2.28 (m, 6H), 2.23 - 2.06 (m, 6H), 2.04 - 1.72 (m, 10H), 1.70 - 1.50 (m, 4H), 1.11 - 1.02 (m, 2H).

Example 35: (1-(3-(difluoromethyl)-1-((1r,4r)-4-((2-(1-(2,6-dioxopiperidin-3-yl)-3-methyl-2-oxo-2,3-dihydro-1H-benzo [d]imidazol-4-yl)-2,7-diazaspiro[3.5] non-7-yl)methyl)cyclohexyl)-1H-pyrazol-4-yl)-1H-1,2,3-triazol-4-yl)pyrrolo[1,2-b] pyridazine-3-carboxamide (35)

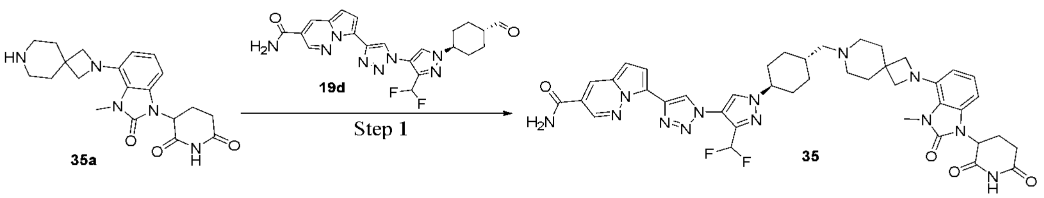


Compound 35a (100 mg, 0.261 mmol, prepared by the method disclosed for the intermediate BIL on page 220 of the specification of the patent application "WO 2021/158634 A1") was dissolved in 1,2-dichloroethane (3 mL) and tetrahydrofuran (3 mL), and triethylamine (52.6 mg, 0.521 mmol) was added until the pH value was greater than 7, and the mixture was stirred at 25°C for 10 min. Acetic acid (146 mg, 0.781 mmol) was added at -10°C, and compound 19d (222 mg, 0.391 mmol) was added. The mixture was stirred at 25°C for 20 min, and sodium borohydride acetate (165 mg, 0.780 mmol) was added. The mixture was stirred for 1 h at 25°C. The reaction solution was concentrated under reduced pressure and then purified by high performance liquid chromatography (Waters-2545, chromatographic column: SharpSil-T C18, 30*150 mm, 5 μm; mobile phase: water (containing 10 mmol/L formic acid) and acetonitrile, gradient ratio: acetonitrile 38%-45%, flow rate: 30 mL/min) to obtain compound 35. MS m/z(ESI):822.8[M+1]$^+$. $^1$H NMR (400 MHz, DMSO-$d_6$): δ 11.10 (s, 1H), 9.01 (s, 1H), 8.79 (d, *J* = 2.1 Hz, 1H), 8.75 (s, 1H), 8.66 (d, *J* = 2.2 Hz, 1H), 8.17 (s, 1H), 7.68 - 7.63 (m, 1H), 7.58 (s, 1H), 7.24 (t, *J* = 53.3 Hz, 1H), 7.05 (d, *J* = 4.6 Hz, 1H), 6.95 (t, *J* = 8.0 Hz, 1H), 6.72 (d, *J* = 8.0 Hz, 1H), 6.68 (d, *J* = 8.0 Hz, 1H), 5.38 - 5.27 (m, 1H), 4.41 - 4.18 (m, 1H), 3.62 - 3.56 (m, 7H), 2.96 - 2.81 (m, 1H), 2.73 - 2.58 (m, 3H), 2.40 - 2.29 (m, 4H), 2.20 - 2.12 (m, 4H), 1.98 - 1.83 (m, 4H), 1.81 - 1.77 (m, 4H), 1.65 - 1.58 (m, 1H), 1.16 - 1.03 (m, 2H).

Example 36: 3-(4-(9-(1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)-3,9-diazaspiro[5.5]undecan-3-yl)-3-methyl-2-oxo-2,3-dihydro-1H-benzoimidazol-1-yl)piperidine-2,6-dione (36)

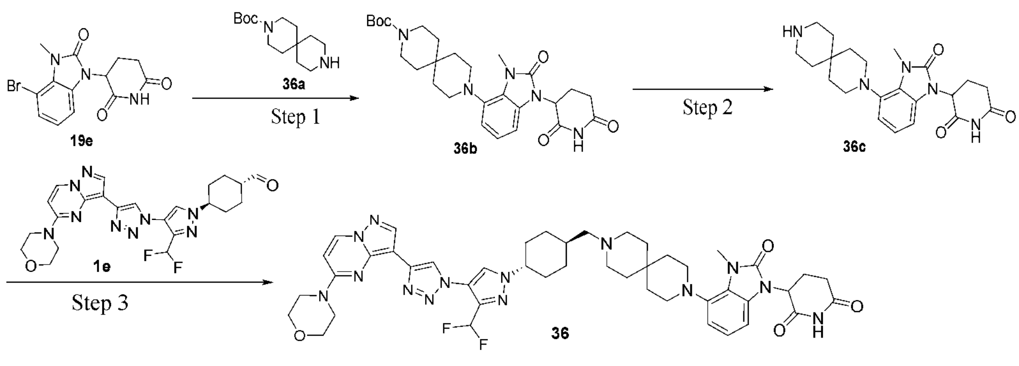


1) Step 1: Compound 19e (200 mg, 0.590 mmol, prepared by the method disclosed for the step 3 product on page 88 of the specification in the patent application "WO 2022012623 A"), compound 36a (301 mg, 1.18 mmol, Bide Pharmatech Ltd.), 2-dicyclohexylphosphino-2',6'-diisopropoxy-1,1'-biphenyl (55.0 mg, 0.120 mmol) and (2-dicyclohexylphosphino-2',6'-diisopropoxy-1,1'-biphenyl)(2-amino-1,1'-biphenyl-2-yl)palladium(II) methanesulfonate (25.0

mg, 30.0 μmol) were dissolved in toluene (3 mL) at room temperature, and lithium bis(trimethylsilyl)amide (3 mL, 3.00 mmol, 1.0 M solution in tetrahydrofuran, Energy Chemical) was added dropwise under nitrogen protection. The reaction solution was stirred at 80°C for 2 h. The reaction solution was cooled to room temperature, water (20 mL) was added, the aqueous phase was extracted with dichloromethane/methanol (15/1, 15 mL × 3). The combined organic phase was washed with saturated brine (15 mL), dried over anhydrous sodium sulfate and filtered to remove the desiccant, and then the filtrate was concentrated under reduced pressure. The residue was purified by silica gel column chromatography (dichloromethane/methanol = 100/1 to 10/1) to obtain compound 36b. MS m/z(ESI):510.4 $[M-1]^-$.

2) Step 2: At room temperature, compound 36b (120 mg, 0.210 mmol) was dissolved in dichloromethane (2 mL), trifluoroacetic acid (2 mL) was added, and the reaction solution was stirred at 25°C for 1 h. The reaction solution was subjected to removal of the solvent under reduced pressure. Compound 36c was obtained without purification. MS m/z(ESI):412.6$[M+1]^+$.

3) Step 3: At room temperature, compound 36c (80.0 mg, 0.140 mmol) and triethylamine (14.0 mg, 0.160 mmol) were dissolved in N,N-dimethylformamide (2 mL) and tetrahydrofuran (2 mL) and stirred for 10 min. Acetic acid (13.0 mg, 0.200 mmol) and compound 1e (61.0 mg, 0.120 mmol) were added, and the reaction solution was stirred at 25°C for 0.5 h. Sodium borohydride acetate (96.0 mg, 0.460 mmol) was slowly added, and the reaction solution was stirred for 1 h. The solvent was removed under reduced pressure. The residue was purified by high performance liquid chromatography (Gilson_306_1741, chromatographic column: Waters-SunFire-C18-10μm-19×250 mm; mobile phase: water (containing 10 mmol/L formic acid) and acetonitrile, gradient ratio: acetonitrile 20%-80%, flow rate: 25 mL/min) to obtain compound 36. MS m/z(ESI):893.8$[M+1]^+$. $^1$H NMR(400 MHz, DMSO-$d_6$) δ11.11 (s, 1H), 8.77 (d, *J* = 7.9 Hz, 1H), 8.66 (s, 1H), 8.54 (s, 1H), 8.38 (s, 1H), 7.20 (t, *J* = 53.2 Hz, 1H), 6.98 - 6.93 (m, 2H), 6.88 - 6.86 (m, 1H), 6.82 (d, *J* = 7.9 Hz, 1H), 5.38 - 5.33 (m, 1H), 4.32 - 4.26 (m, 1H), 3.73 (s, 8H), 3.63 (s, 3H), 2.96 - 2.78 (m, 5H), 2.71 - 2.59 (m, 2H), 2.38 (s, 4H), 2.18 - 2.12 (m, 4H), 2.00 - 1.91 (m, 3H), 1.86 - 1.80 (m, 2H), 1.77 - 1.68 (m, 5H), 1.52 - 1.43 (m, 4H), 1.12 - 1.06 (m, 2H).

Example 37: 7-(1-(3-(difluoromethyl)-1-((1r,4r)-4-((8-(1-(2,6-dioxopiperidin-3-yl)-3-methyl-2-oxo-2,3-dihydro-1H-benzo [d]imidazol-4-yl)-2,8-diazaspiro[4.5] dec-2-yl)methyl)cyclohexyl)-1H-pyrazol-4-yl)-1H-1,2,3-triazol-4-yl)pyrrolo[1,2-b]pyridazine-3-carboxamide (37)

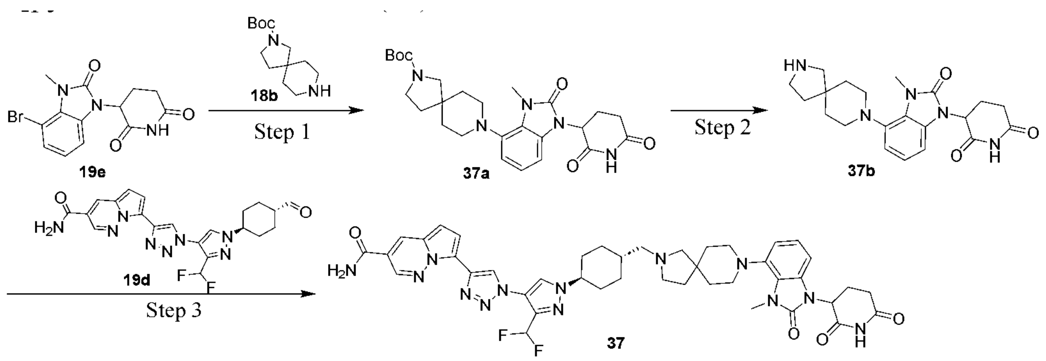


1) Step 1: Compound 19e (300 mg, 0.890 mmol, prepared by the method disclosed for step 3 product on page 88 of the specification of the patent application "WO 2022012623 A"), compound 18b (426 mg, 1.77 mmol, Bide Pharmatech Ltd.), (2-dicyclohexylphosphino-2',6'-diisopropoxy-1,1'-biphenyl) (2-amino-1,1'-biphenyl-2-yl)palladium (II) methanesulfonate (149 mg, 0.180 mmol, adamas) and 2-dicyclohexylphosphino-2',6'-diisopropoxy-1,1'-biphenyl (83.0 mg, 0.180 mmol, Bide Pharmatech Ltd.) were dissolved in toluene (3 mL), and lithium bis(trimethylsilyl)amide (4.4 mL, 4.44 mmol, 1.0 M solution in tetrahydrofuran, Energy Chemical) was added dropwise under nitrogen protection. The reaction solution was reacted at 80°C for 2 h. The reaction solution was quenched with saturated ammonium chloride aqueous solution (20 mL) and the aqueous phase was extracted with dichloromethane (20 mL × 2). The organic phases were combined, dried over anhydrous sodium sulfate and filtered to remove the desiccant, and then the filtrate was concentrated under reduced pressure. The residue was purified by silica gel column chromatography (dichloromethane/methanol = 100/1 to 30/1) to obtain compound 37a. MS m/z(ESI):498.3$[M+1]^+$.

2) Step 2: Compound 37a (100 mg, 0.200 mmol) was dissolved in dichloromethane (2 mL), and trifluoroacetic acid (1 mL) was added. The reaction solution was stirred at 20°C for 1 h. The reaction solution was concentrated under reduced pressure to obtain the crude trifluoroacetate of compound 37b. MS m/z(ESI):398.2$[M+1]^+$.

3) Step 3: The trifluoroacetate of compound 37b (100 mg, 0.250 mmol) was dissolved in tetrahydrofuran (1 mL) and 1,2-dichloroethane (1 mL), and triethylamine (0.04 mL, 0.250 mmol) was added. The reaction solution was stirred for 0.5 h. Compound 19d (114 mg, 0.250 mmol) and acetic acid (0.02 mL, 0.380 mmol) were added to the reaction solution and the obtained mixture was stirred for 0.5 h. Sodium borohydride acetate (105 mg, 0.500 mmol) was added to the reaction solution. The reaction solution was stirred at 25°C for 17 h. The reaction solution was concentrated under reduced pressure and then purified by high performance liquid chromatography (A: 0.1% FA/H2O B: ACN, chromatographic column: Waters-SunFire-C18-10μm-19×250 mm; mobile phase: water (containing 0.1% formic acid) and acetonitrile, gradient ratio: acetonitrile 35%-45%, flow rate: 25 mL/min) to obtain compound 37. MS m/z(ESI):836.8[M+1]$^+$. $^1$H NMR (400 MHz, DMSO-$d_6$): δ11.10 (s, 1H), 9.04 - 8.98 (m, 1H), 8.81 - 8.77 (m, 1H), 8.75 (s, 1H), 8.70 - 8.63 (m, 1H), 8.16 (s, 1H), 7.68 - 7.64 (m, 1H), 7.58 (s, 1H), 7.32 - 7.15 (m, 1H), 7.05 (d, *J* = 4.6 Hz, 1H), 6.99 - 6.90 (m, 2H), 6.89 - 6.84 (m, 1H), 5.40 - 5.30 (m, 1H), 4.35 - 4.24 (m, 1H), 3.64 (s, 3H), 3.04 - 2.88 (m, 3H), 2.80 - 2.58 (m, 6H), 2.37 - 2.26 (m, 3H), 2.19 - 2.10 (m, 2H), 2.03 - 1.92 (m, 3H), 1.85 - 1.51 (m, 10H), 1.18 - 1.04 (m, 2H).

Example 38: 3-(4-(9-(1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)-1-oxa-4,9-diazaspiro[5.5]undecan-4-yl)-3-methyl-2-oxo-2,3-dihydro-1H-benzoimidazol-1-yl)piperidine-2,6-dione (38)

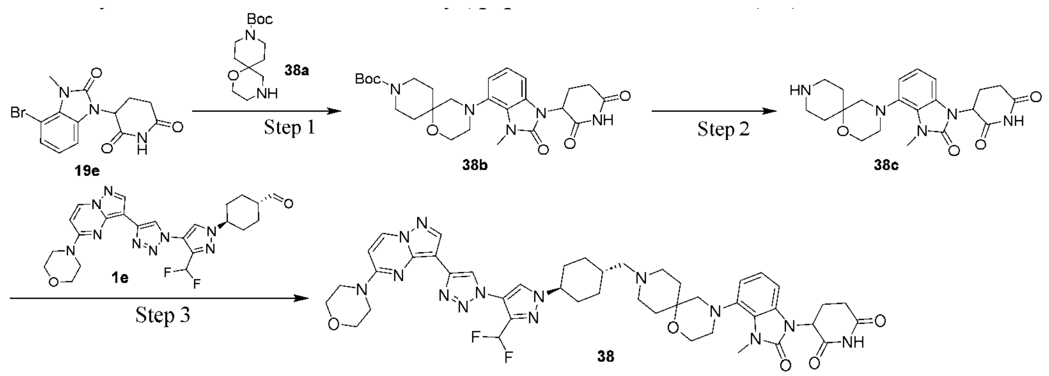


1) Step 1: Compound 19e (400 mg, 1.18 mmol, prepared by the method disclosed for the step 3 product on page 88 of the specification in the patent application "WO 2022012623 A"), compound 38a (606 mg, 2.36 mmol, Bide Pharmatech Ltd.), 2-dicyclohexylphosphino-2',6'-diisopropoxy-1,1'-biphenyl (110 mg, 0.240 mmol) and (2-dicyclohexylphosphino-2',6'-diisopropoxy-1,1'-biphenyl)(2-amino-1,1'-biphenyl-2-yl)palladium(II) methanesulfonate (24.8 mg, 30.0 μmol) were dissolved in toluene (6 mL), and lithium bis(trimethylsilyl)amide (6 mL, 6.00 mmol, 1.0 M solution in tetrahydrofuran, Energy Chemical) was added dropwise under nitrogen protection. The reaction solution was stirred at 80°C for 2 h. The reaction solution was cooled to room temperature, water (40 mL) was added, and the aqueous phase was extracted with dichloromethane/methanol (15/1, 15 mL × 3). The organic phases were combined, washed with saturated brine (15 mL), dried over anhydrous sodium sulfate, filtered to remove the desiccant, and then concentrated under reduced pressure to remove the solvent. The residue was purified by silica gel column chromatography (dichloromethane/methanol = 100/1 to 10/1) to obtain compound 38b. MS m/z(ESI):512.4[M-1]$^-$.

2) Step 2: Compound 38b (230 mg, 0.450 mmol) was dissolved in dichloromethane (2 mL), and trifluoroacetic acid (2 mL) was added. The reaction solution was stirred at 25°C for 1 h. The reaction solution was concentrated under reduced pressure to obtain compound 38c. MS m/z(ESI):414.7[M+1]$^+$.

3) Step 3: Compound 38c (100 mg, 0.240 mmol) and triethylamine (25.0 mg, 0.250 mmol) were dissolved in N,N-dimethylformamide (2 mL) and tetrahydrofuran (2 mL) and stirred for 10 min. Acetic acid (19.0 mg, 0.320 mmol) and compound 1e (99.0 mg, 0.220 mmol) were added, and the reaction solution was stirred at 25°C for 0.5 h. Sodium borohydride acetate (154 mg, 0.730 mmol) was slowly added, and the reaction solution was stirred for 1 h. The solvent was removed under reduced pressure. The residue was purified by high performance liquid chromatography (Gilson_306_1741, chromatographic column: Waters-SunFire-C18-10μm-19×250 mm; mobile phase: water (containing 10 mmol/L formic acid) and acetonitrile, gradient ratio: acetonitrile 2%-98%, flow rate: 25 mL/min) to obtain compound 38. MS m/z(ESI):896.0[M+1]$^+$. $^1$H NMR(400MHz, DMSO-$d_6$): δ11.12 (s, 1H), 8.77 (d, *J* = 7.9 Hz, 1H), 8.65 (s, 1H), 8.54 (s, 1H), 8.38 (s, 1H), 7.20 (t, *J* = 53.2 Hz, 1H), 7.03 - 6.87 (m, 3H), 6.82 (d, *J* = 7.9 Hz, 1H), 5.39 - 5.35 (m, 1H), 4.37 - 4.18 (m, 1H), 4.04 - 3.87 (m, 1H), 3.72 (s, 8H), 3.65 (s, 3H), 3.45 - 3.29 (m, 3H), 3.07 - 2.96 (m, 1H), 2.94 - 2.76 (m, 3H), 2.72 - 2.58 (m, 3H), 2.42 - 2.20 (m, 3H), 2.19 - 2.08 (m, 4H), 2.02 - 1.87 (m, 3H), 1.851.72 (m, 2H), 1.70 -

1.47 (m, 4H), 1.20 - 0.93 (m, 2H).

Example 39: 3-(4-(2-(((1r,4r)-4-(3-(difluoromethyl)-4-(4-(5-morpholinopyrazolo[1,5-a]pyrimidin-3-yl)-1H- 1,2,3-triazol-1-yl)-1H-pyrazol-1-yl)cyclohexyl)methyl)-2,8-diazaspiro[4.5]dec-8-yl)-3-methyl-2-oxo-2,3-dihydro-1H-benzo[d]imidazol-1-yl)piperidine-2,6-dione (39)

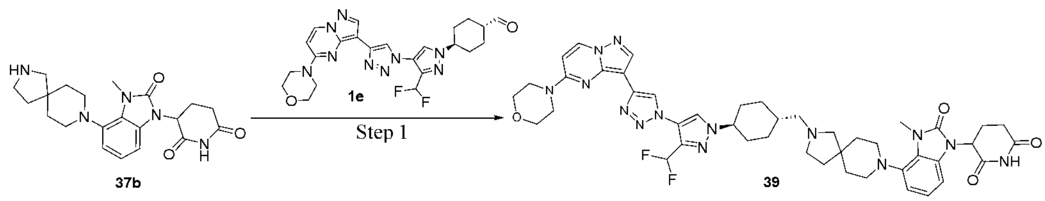


The trifluoroacetate of compound 37b (100 mg, 0.250 mmol) was dissolved in tetrahydrofuran (1 mL) and 1,2-dichloroethane (1 mL), and triethylamine (0.04 mL, 0.250 mmol) was added, and the reaction solution was stirred for 0.5 h. Compound 1e (125 mg, 0.250 mmol) and acetic acid (0.02 mL, 0.380 mmol) were added to the reaction solution and the obtained mixture was stirred for 0.5 h. Sodium borohydride acetate (105 mg, 0.500 mmol) was added to the reaction solution. The reaction solution was stirred at 25°C for 17 h. The reaction solution was concentrated under reduced pressure and then purified by high performance liquid chromatography (A: 0.1% FA/H2O B: ACN, chromatographic column: Waters-CORTECS-C18-2.7μm-4.6×30 mm; mobile phase: water (containing 0.1% formic acid) and acetonitrile, gradient ratio: acetonitrile 75%-85%, flow rate: 25 mL/min) to obtain compound 39. MS m/z(ESI):879.8[M+1]$^+$. $^1$H NMR (400 MHz, DMSO-$d_6$): δ11.11 (s, 1H), 8.77 (d, *J* = 7.6 Hz, 1H), 8.66 (s, 1H), 8.57 - 8.52 (m, 1H), 8.38 (s, 1H), 7.34 - 7.07 (m, 1H), 7.02 - 6.90 (m, 2H), 6.90 - 6.85 (m, 1H), 6.82 (d, *J* = 7.6 Hz, 1H), 5.42 - 5.31 (m, 1H), 4.35 - 4.22 (m, 1H), 3.80 - 3.69 (m, 8H), 3.64 (s, 3H), 3.03 - 2.85 (m, 3H), 2.77 - 2.57 (m, 6H), 2.41 - 2.25 (m, 3H), 2.18 - 2.09 (m, 2H), 2.05 - 1.92 (m, 3H), 1.89 - 1.50 (m, 10H), 1.19 - 1.01 (m, 2H).

Example 40: 3-(4-(4-(-4-(1-((1r,4r)-4-(3-(difluoromethyl)-4-(4-(5-morpholinopyranazolo[1,5-a]pyrimidin-3-yl)-1-1,2,3-triazol-1-yl-1,2,3-triazol-1-pyrazol-1-yl)cyclohexylmethyl)piperidin-4-ylpiperazin-1-yl)-piperazin-1-acyl)-3-methyl-3-methyl-2-oxo-2,3-dihydrodihydro-1H-benzo[imidazol-1-yl-1-piperidin-2,6-piperidinedione (40)

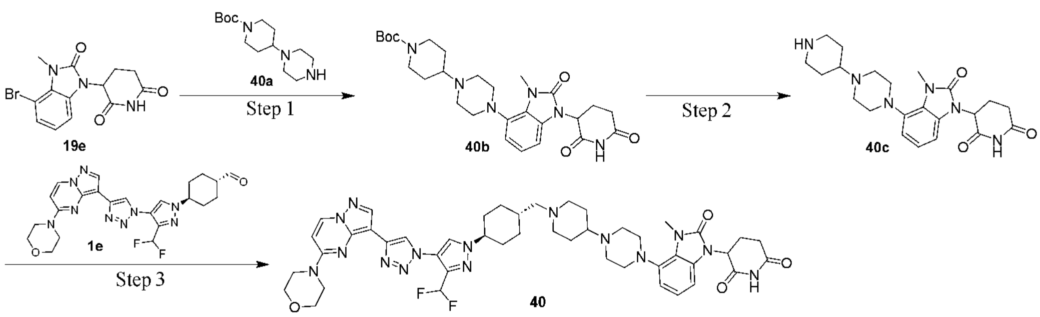


1) Step 1: Compound 19e (150 mg, 0.440 mmol, prepared by the method disclosed for the step 3 product on page 88 of the specification in the patent application "WO 2022012623 A"), compound 40a (179 mg, 0.660 mmol, Bide Pharmatech Ltd.), 2-dicyclohexylphosphino-2',6'-diisopropoxy-1,1'-biphenyl (62.0 mg, 0.130 mmol, Bide Pharmatech Ltd.) and (2-dicyclohexylphosphino-2',6'-diisopropoxy-1,1'-biphenyl)(2-amino-1,1'-biphenyl-2-yl)palladium(II) methanesulfonate (111 mg, 0.130 mmol, adamas) were dissolved in toluene (5 mL), and lithium bis(trimethylsilyl) amide (2.6 mL, 2.60 mmol, 1.0 M solution in tetrahydrofuran, Energy Chemical) was added dropwise under nitrogen protection. The reaction solution was reacted under stirring at 80°C under nitrogen atmosphere for 2 h. The mixture was poured into water (20 mL), and the aqueous phase was extracted with ethyl acetate (30 mL x 3). The combined organic phase was washed with saturated brine (10 mL), dried over anhydrous sodium sulfate, filtered to remove the desiccant, and then concentrated under reduced pressure to remove the solvent. The residue was purified by silica gel chromatography (petroleum ether/ethyl acetate = 10/1 to 0/1) to obtain compound 40b. MS m/z(ESI): 527.3[M+1]$^+$.
2) Step 2: Compound 40b (78.0 mg, 0.150 mmol) was dissolved in dichloromethane (5 mL), trifluoroacetic acid (1 mL)

was added, and the obtained mixture was stirred at 25°C for 2 h. The mixture was concentrated to obtain compound 40c. MS m/z(ESI): 427.3[M+1]$^+$.

3) Step 3: Compound 40c (90.0 mg, 0.120 mmol) was dissolved in N,N-dimethylformamide (2 mL) and tetrahydrofuran (3 mL), triethylamine (24.0 mg, 0.240 mmol) was added, and the obtained mixture was stirred at 25°C for 10 min. Compound 1e (58.0 mg, 0.120 mmol) and acetic acid (35.0 mg, 0.580 mmol) were added to the reaction solution, and the obtained mixture was stirred for 30 min. Sodium borohydride acetate (124 mg, 0.590 mmol) was added to the reaction solution, and the obtained mixture was stirred for 1 h. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 8%-95%, flow rate: 25 mL/min) to obtain compound 40. MS m/z(ESI): 908.6[M+1]$^+$. $^1$H NMR(400MHz, DMSO-$d_6$): δ 11.08 (s, 1H), 8.76 (d, *J* = 8.0 Hz, 1H), 8.65 (s, 1H), 8.53 (s, 1H), 8.38 (s, 1H), 7.19 (t, *J* = 53.2 Hz, 1H), 7.01 - 6.85 (m, 3H), 6.81 (d, *J* = 7.9 Hz, 1H), 5.40 - 5.32 (m, 1H), 4.32 - 4.25 (m, 1H), 3.72 (s, 8H), 3.62 (s, 3H), 3.18 - 2.74 (m, 9H), 2.74 - 2.54 (m, 3H), 2.48 - 2.08 (m, 6H), 2.05 - 1.73 (m, 9H), 1.67 - 1.43 (m, 3H), 1.13 - 1.04 (m, 2H).

Example 41: 3-(4-(4-(((1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-ylcyclohexyl)methyl)-1-oxa-4,9-diazaspiro[5.5]undecan-9-yl)-3-methyl-2,3-dihydro-1H-benzo[d]imidazol-1-yl)piperidine-2,6-dione (41)

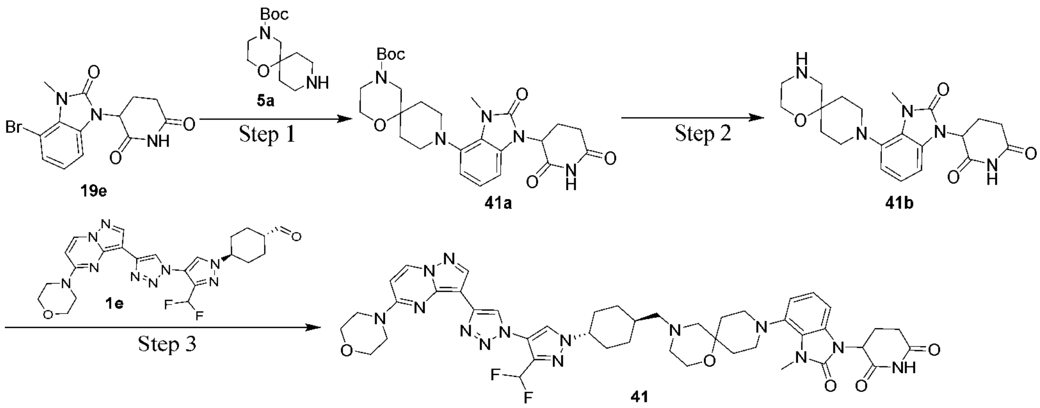


1) Step 1: Compound 19e (300 mg, 0.890 mmol, prepared by the method disclosed for the step 3 product on page 88 of the specification in the patent application "WO 2022012623 A"), compound 5a (340 mg, 1.33 mmol, Bide Pharmatech Ltd.), 2-dicyclohexylphosphino-2',6'-diisopropoxy-1,1'-biphenyl (120 mg, 0.260 mmol, Bide Pharmatech Ltd.) and (2-dicyclohexylphosphino-2',6'-diisopropoxy-1,1'-biphenyl)(2-amino-1,1'-biphenyl-2-yl)palladium(II) methanesulfonate (220 mg, 0.260 mmol, adamas) were dissolved in toluene (12 mL), and lithium bis(trimethylsilyl)amide (5 mL, 5.0 mmol, 1.0 M solution in tetrahydrofuran, Energy Chemical) was added dropwise under nitrogen protection. The reaction solution was reacted under stirring at 80°C under nitrogen atmosphere for 2 h. The mixture was poured into water (20 mL), and the aqueous phase was extracted with ethyl acetate (30 mL) three times. The combined organic phase was washed with saturated brine (10 mL), dried over anhydrous sodium sulfate, filtered to remove the desiccant, and then concentrated under reduced pressure to remove the solvent. The residue was purified by silica gel chromatography (petroleum ether/ethyl acetate = 10/1 to 0/1) to obtain compound 41a. MS m/z(ESI): 514.2[M+1]$^+$.

2) Step 2: Compound 41a (110 mg, 0.210 mmol) was dissolved in dichloromethane (5 mL), trifluoroacetic acid (1 mL) was added, and the obtained mixture was stirred at 25°C for 2 h. The mixture was concentrated to obtain compound 41b. MS m/z(ESI): 414.2[M+1]$^+$.

3) Step 3: Compound 41b (62.0 mg, 0.110 mmol) was dissolved in N,N-dimethylformamide (2 mL) and tetrahydrofuran (3 mL), triethylamine (28.0 mg, 0.280 mmol) was added and the obtained mixture was stirred at 25°C for 10 min. Compound 1e (67.0 mg, 0.130 mmol) and acetic acid (41.0 mg, 0.680 mmol) were added to the reaction solution, and the obtained mixture was stirred at 25°C for 30 min. Sodium borohydride acetate (143 mg, 0.670 mmol) was added to the reaction solution, and the obtained mixture was stirred at 25°C for 1 h. The mixture was concentrated to obtain a residue. The reaction solution was concentrated under reduced pressure and then purified by high performance liquid chromatography (Gilson_306_1741, chromatographic column: Wetch-Ultimate-XB-C18-10μm-21.2×150 mm; mobile phase: water (containing 26.5 mmol/L formic acid) and acetonitrile, gradient ratio: acetonitrile 15%-95%, flow rate: 25 mL/min) to obtain compound 41. MS m/z(ESI): 895.6[M+1]$^+$. $^1$H NMR(400MHz, DMSO-$d_6$): δ 11.08 (s, 1H), 8.76 (d,

*J* = 8.0 Hz, 1H), 8.65 (s, 1H), 8.53 (s, 1H), 8.38 (s, 1H), 7.19 (t, *J* = 53.2 Hz, 1H), 7.01 - 6.89 (m, 2H), 6.86 (d, *J* = 8.0 Hz, 1H), 6.81 (d, *J* = 8.0 Hz, 1H), 5.37 - 5.32 (m, 1H), 4.38 - 4.26 (m, 1H), 3.81 - 3.61 (m, 13H), 3.09 - 2.89 (m, 5H), 2.74 - 2.58 (m, 2H), 2.43 - 2.03 (m, 10H), 2.02 - 1.78 (m, 5H), 1.63 (s, 3H), 1.14 - 1.04 (m, 2H).

Example 42: 3-(4-(3-(4-(((1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-yl)cyclohexyl)methyl)piperazin-1-yl)azetidin-1-yl)-3-methyl-2-oxo-2,3-dihydro-1H-benzo[d]imidazol-1-yl)piperidine-2,6-dione (42)

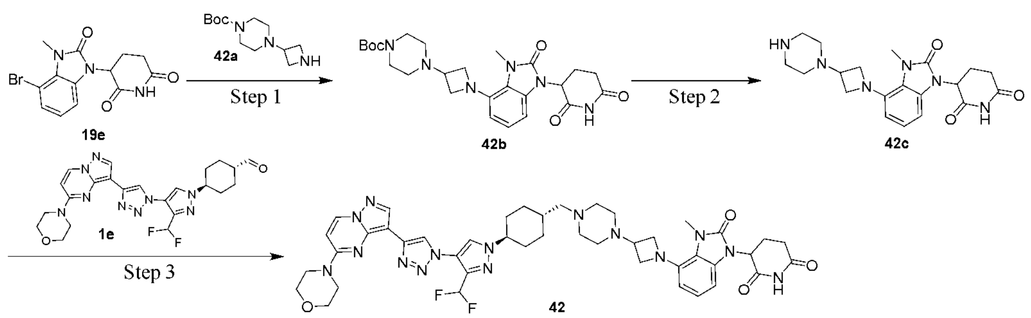


1) Step 1: Compound 42a (100 mg, 0.300 mmol, Leyan), compound 19e (107 mg, 0.440 mmol, prepared by the method disclosed for the step 3 product on page 88 of the specification in the patent application "WO 2022012623 A"), 2-dicyclohexylphosphino-2',6'-diisopropoxy-1,1'-biphenyl (27.6 mg, 60.0 μmol, Bide Pharmatech Ltd.) and (2-dicyclohexylphosphino-2',6'-diisopropoxy-1,1'-biphenyl)(2-amino-1,1'-biphenyl-2-yl)palladium(II) methanesulfonate (251 mg, 0.300 mmol, adamas) were dissolved in toluene (2 mL), and lithium bis(trimethylsilyl)amide (1.5 mL, 1.48 mmol, 1.0 M solution in tetrahydrofuran, Energy Chemical) was added dropwise under nitrogen protection. The reaction solution was reacted at 80°C for 2 h. Water (10 mL) was added to the reaction solution, and the aqueous phase was extracted with ethyl acetate (10 mL × 3). The combined organic phase was washed with saturated brine (10 mL), dried over anhydrous sodium sulfate, filtered to remove the desiccant, and concentrated under reduced pressure to remove the solvent. The residue was purified by silica gel column chromatography with an eluent system (petroleum ether/ethyl acetate = 1/1 to 0/1) to obtain compound 42b. MS m/z(ESI):499.2[M+1]$^+$.

2) Step 2: Compound 42b (80.0 mg, 0.160 mmol) was dissolved in dichloromethane (2 mL), trifluoroacetic acid (1 mL) was added, and the obtained mixture was reacted at 25°C for 3 h. The reaction solution was concentrated to obtain compound 42c. MS m/z(ESI):399.2[M+1]$^+$.

3) Step 3: Compound 42c (30.0 mg, 80.0 μmol) was dissolved in N,N-dimethylformamide (1.5 mL) and tetrahydrofuran (1 mL), triethylamine (0.03 mL, 0.190 mmol) was added, and the obtained mixture was reacted at 25°C for 10 min. Then compound 1e (41.2 mg, 80.0 μmol) and acetic acid (0.03 mL, 0.450 mmol) were added in sequence, and the obtained mixture was reacted at 25°C for 30 min. Then sodium borohydride acetate (95.3 mg, 0.450 mmol) was added, and the obtained mixture was reacted at 25°C for 2 h. The reaction solution was concentrated under reduced pressure to obtain a residue. The residue was purified by high performance liquid chromatography (Gilson _306_1741, chromatographic column: Waters-Xbridge-C18-10μm-19×250 mm; mobile phase: water (containing 26.5 mmol/L formic acid) and acetonitrile, gradient ratio: acetonitrile 15%-95%, flow rate: 25 mL/min) to obtain compound 42. MS m/z(ESI):880.4[M+1]$^+$. $^1$H NMR (400 MHz, DMSO-$d_6$): δ 11.08 (s, 1H), 8.76 (d, *J* = 7.9 Hz, 1H), 8.64 (s, 1H), 8.53 (s, 1H), 8.38 (s, 1H), 7.19 (t, *J* = 53.2 Hz, 1H), 6.95 (t, *J* = 8.0 Hz, 1H), 6.81 (d, *J* = 7.9 Hz, 1H), 6.74 (d, *J* = 7.8 Hz, 1H), 6.67 (d, *J* = 8.2 Hz, 1H), 5.36 - 5.29 (m, 1H), 4.36 - 4.21 (m, 1H), 3.89 (s, 2H), 3.72 (s, 8H), 3.63 (t, *J* = 6.0 Hz, 2H), 3.57 (s, 3H), 3.27 - 3.09 (m, 2H), 2.93 - 2.81 (m, 1H), 2.75 - 2.60 (m, 2H), 2.36 (s, 7H), 2.14 (d, *J* = 7.1 Hz, 4H), 2.01 - 1.76 (m, 5H), 1.59 (s, 1H), 1.11 - 1.05 (m, 2H).

Example 43: 3-(4-(7-(((1r,4r)-4-(3-(difluoromethyl)-4-(4-(5-morpholinopyrazolo[1,5-a]pyrimidin-3-yl)-1H- 1,2,3-triazol-1-yl)-1H-pyrazol-1-yl)cyclohexyl)methyl)-2,7-diazaspiro[3.5]nonan-2-yl)-3-methyl-2-oxo-2,3-dihydro-1H-benzo[d]imidazol-1-yl)piperidine-2,6-dione (43)

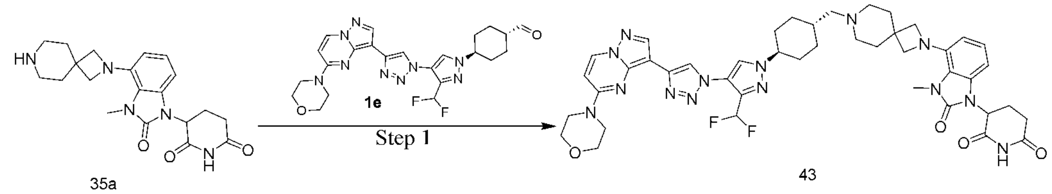


Compound 35a (28.0 mg, 71.0 μmol, prepared by the method disclosed for the intermediate BIL on page 220 of the specification of the patent application "WO 2021/158634 A1") was dissolved in a solution of 1,2-dichloroethane (3 mL) and tetrahydrofuran (3 mL), and triethylamine (8.51 mg, 81.0 μmol) was added until the pH value was greater than 7, and the reaction solution was stirred at 25°C for 10 min. Acetic acid (26.1 mg, 0.141 mmol) and compound 1e (41.8 mg, 81.0 μmol) were added at -10°C. The reaction solution was stirred at 25°C for 20 min, and then sodium borohydride acetate (29.7 mg, 0.141 mmol) was added. The reaction solution was stirred at 25°C for 1 h. The reaction solution was concentrated under reduced pressure and then purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 43. MS m/z(ESI):865.5 $[M+1]^+$. $^1H$ NMR (400 MHz, DMSO-$d_6$): δ 11.09 (s, 1H), 8.77 (d, $J$ = 7.9 Hz, 1H), 8.70 - 8.63 (m, 1H), 8.54 (s, 1H), 8.39 (s, 1H), 8.16 (s, 1H), 7.20 (t, $J$ = 53.2 Hz, 1H), 6.96 (t, $J$ = 8.0 Hz, 1H), 6.82 (d, $J$ = 7.9 Hz, 1H), 6.73 (d, $J$ = 8.1 Hz, 1H), 6.68 (d, $J$ = 8.2 Hz, 1H), 5.38 - 5.27 (m, 1H), 4.36 - 4.23 (m, 1H), 3.73 (s, 8H), 3.62 - 3.52 (m, 8H), 2.93 - 2.84 (m, 1H), 2.70 - 2.61 (m, 2H), 2.41 - 2.27 (m, 4H), 2.20 - 2.09 (m, 4H), 2.03 - 1.86 (m, 4H), 1.82 - 1.78 (m, 4H), 1.68 - 1.56 (m, 1H), 1.15 - 1.01 (m, 2H).

Example 44: 3-(4-(4-(1-(((1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-ylcyclohexyl)methyl)azetidin-3-yl)piperazin-1-yl)-3-methyl-2-oxo-2,3-dihydro-1H-benzo[d]imidazol-1-yl)piperidine-2,6-dione (44)

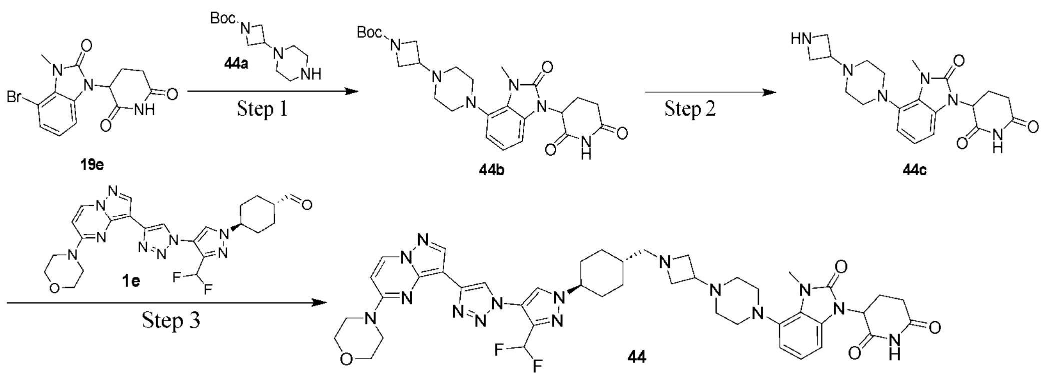


1) Step 1: Compound 19e (150 mg, 0.440 mmol, prepared by the method disclosed for the step 3 product on page 88 of the specification in the patent application "WO 2022012623 A"), compound 44a (160 mg, 0.660 mmol, Bide Pharmatech Ltd.), 2-dicyclohexylphosphino-2',6'-diisopropoxy-1,1'-biphenyl (62.0 mg, 0.130 mmol) and (2-dicyclohexylphosphino-2',6'-diisopropoxy-1,1'-biphenyl)(2-amino-1,1'-biphenyl-2-yl)palladium(II) methanesulfonate (111 mg, 0.130 mmol) were dissolved in toluene (5 mL), and lithium bis(trimethylsilyl)amide (2.3 mL, 2.30 mmol, 1.0 M solution in tetrahydrofuran, Energy Chemical) was added dropwise under nitrogen protection. The reaction solution was reacted under stirring at 80°C under nitrogen atmosphere for 2 h. The mixture was poured into water (20 mL), and the aqueous phase was extracted with ethyl acetate (30 mL × 3). The combined organic phase was washed with saturated brine (10 mL), dried over anhydrous sodium sulfate, filtered to remove the desiccant, and then concentrated under reduced pressure to remove the solvent. The residue was purified by silica gel chromatography (petroleum ether/ethyl acetate = 1/1 to 0/1) to obtain compound 44b. MS m/z(ESI): 499.2$[M+1]^+$.

2) Step 2: Compound 44b (60.0 mg, 0.120 mmol) was dissolved in dichloromethane (5 mL), trifluoroacetic acid (1 mL) was added, and the obtained mixture was stirred at 25°C for 2 h. The mixture was concentrated to obtain compound 44c. MS m/z(ESI): 399.2$[M+1]^+$.

3) Step 3: Compound 44c (68.0 mg, 0.120 mmol) was dissolved in N,N-dimethylformamide (2 mL) and tetrahydrofuran (3 mL), triethylamine (24.0 mg, 0.240 mmol) was added and the obtained mixture was stirred at 25°C for 10 min. Compound 1e (59.0 mg, 0.120 mmol) and acetic acid (40.0 mg, 0.670 mmol) were added to the reaction solution, and the obtained mixture was stirred at 25°C for 30 min. Sodium borohydride acetate (127 mg, 0.600 mmol) was added to

the reaction solution, and the obtained mixture was stirred at 25°C for 1 h. The reaction solution was concentrated to obtain a residue. The reaction solution was concentrated under reduced pressure and then purified by high performance liquid chromatography (Gilson_306_1741, chromatographic column: Waters-SunFire-C18-10μm-19*250 mm; mobile phase: water (containing 26.5 mmol/L formic acid) and acetonitrile, gradient ratio: acetonitrile 15%-95%, flow rate: 25 mL/min) to obtain compound 44. MS m/z(ESI): 440.9[M/2+1]$^+$. $^1$H NMR(400MHz, DMSO-$d_6$): δ 11.11 (s, 1H), 8.77 (d, *J* = 8.0 Hz, 1H), 8.72 - 8.65 (m, 1H), 8.56 - 8.53 (m, 1H), 8.38 (d, *J* = 1.6 Hz, 1H), 7.36 - 7.04 (m, 1H), 7.02 - 6.84 (m, 3H), 6.81 (d, *J* = 8.0 Hz, 1H), 5.40 - 5.33 (m, 1H), 4.43 - 4.19 (m, 1H), 3.72 (s, 8H), 3.61 (s, 3H), 3.48 - 3.43 (m, 2H), 3.34 (s, 1H), 3.08 - 2.56 (m, 12H), 2.33 (d, *J* = 6.0 Hz, 1H), 2.27 - 1.27 (m, 11H), 1.15 - 1.03 (m, 1H).

Example 45: 3-(4-(8-((1R,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl)-2,8-diazaspiro[4.5]dec-2-yl)-3-methyl-2-oxo-2,3-dihydro-1H-benzoimidazol-1-yl)piperidine-2,6-dione (45)

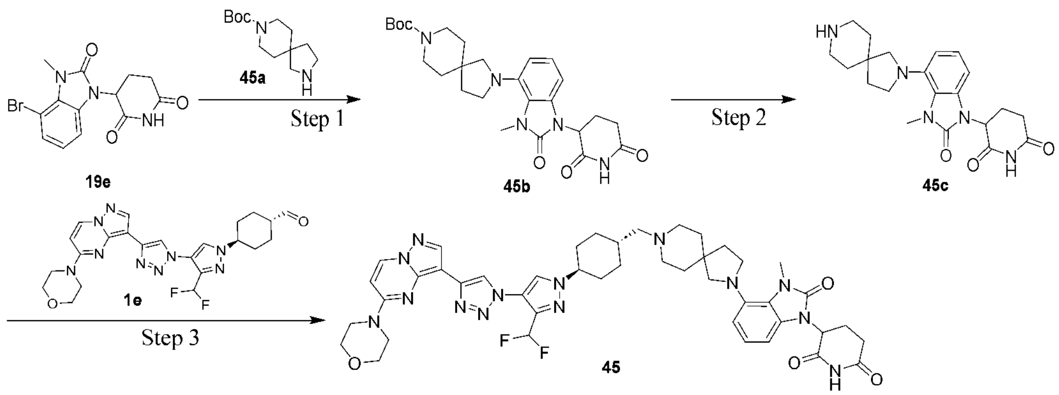


1) Step 1: Compound 19e (500 mg, 1.48 mmol, prepared by the method disclosed for step 3 product on page 88 of the specification of the patent application "WO 2022012623 A"), compound 45a (533 mg, 2.22 mmol, Bide Pharmatech Ltd.), (2-dicyclohexylphosphino-2',6'-diisopropoxy-1,1'-biphenyl) (2-amino-1,1'-biphenyl-2-yl)palladium (II) methanesulfonate (248 mg, 0.300 mmol, Bide Pharmatech Ltd.) and 2-dicyclohexylphosphino-2',6'-diisopropoxy-1,1'-biphenyl (138 mg, 0.300 mmol, Bide Pharmatech Ltd.) were dissolved in toluene (5 mL), and lithium bis(trimethylsilyl) amide (7.4 mL, 7.39 mmol, 1.0 M solution in tetrahydrofuran, Energy Chemical) was added dropwise under nitrogen protection. The reaction solution was reacted at 80°C under nitrogen atmosphere for 2 h. The reaction solution was quenched with saturated ammonium chloride (20 mL), and the aqueous phase was extracted with dichloromethane (20 mL × 2). The organic phases were combined, dried over anhydrous sodium sulfate and filtered to remove the desiccant, and then the filtrate was concentrated under reduced pressure. The residue was purified by silica gel column chromatography (dichloromethane/methanol = 100/1 to 30/1) to obtain compound 45b. MS m/z(ESI):498.4 [M+1]$^+$.

2) Step 2: Compound 45b (80.0 mg, 0.160 mmol) was dissolved in dichloromethane (2 mL), and trifluoroacetic acid (1 mL) was added. The reaction was stirred at 25°C for 1 h. The reaction solution was concentrated under reduced pressure to obtain compound 45c. MS m/z(ESI):398.5[M+1]$^+$.

3) Step 3: Compound 45c (50.0 mg, 0.130 mmol) was dissolved in tetrahydrofuran (2 mL) and N,N-dimethylformamide (1 mL), triethylamine (0.1 mL) was added, and the obtained mixture was stirred for 0.5 h. Compound 1e (75.0 mg, 0.150 mmol) and acetic acid (0.2 mL) were added to the reaction solution, and the obtained mixture was stirred for 0.5 h. Sodium borohydride acetate (80.0 mg, 0.380 mmol) was added to the reaction solution. The reaction solution was stirred at 25°C for 2 h. The reaction solution was concentrated under reduced pressure and then purified by high performance liquid chromatography (A: 0.1% formic acid/water, B: acetonitrile, chromatographic column: Waters-SunFire-C18-10um-19×250 mm; mobile phase: water (containing 0.1% formic acid) and acetonitrile, gradient ratio: acetonitrile 32%-48%, flow rate: 25 mL/min) to obtain compound 45. MS m/z(ESI):877.7[M-1]$^-$. $^1$H NMR (400 MHz, DMSO-$d_6$): δ 11.11 (s, 1H), 8.77 (d, *J* = 7.9 Hz, 1H), 8.66 (s, 1H), 8.54 (s, 1H), 8.38 (s, 1H), 7.20 (t, *J* = 53.2 Hz, 1H), 7.00 - 6.93 (m, 2H), 6.88 - 6.79 (m, 2H), 5.40 - 5.32 (m, 1H), 4.32 - 4.23 (m, 1H), 3.73 (s, 8H), 3.59 (s, 3H), 3.07 (t, *J* = 6.8 Hz, 2H), 2.93 - 2.83 (m, 3H), 2.73 - 2.58 (m, 2H), 2.43 - 2.24 (m, 4H), 2.18 - 2.09 (m, 4H), 2.02 - 1.89 (m, 3H), 1.86 - 1.78 (m, 2H), 1.76 - 1.72 (m, 2H), 1.67 - 1.55 (m, 5H), 1.15 - 0.98 (m, 2H).

Example 46: 3-(4-((R)-4-(((1r,4R)-4-(3-(difluoromethyl)-4-(4-(5-morpholinopyrazolo[1,5-a]pyrimidin-3-yl)-1H-1,2,3-triazol-1-yl)-1H-pyrazol-1-yl)cyclohexyl)methyl)-2-methylpiperazin-1-yl)-3-methyl-2-oxo-2,3-dihydro-1H-benzo[d]imidazol-1-yl)piperidine-2,6-dione (46)

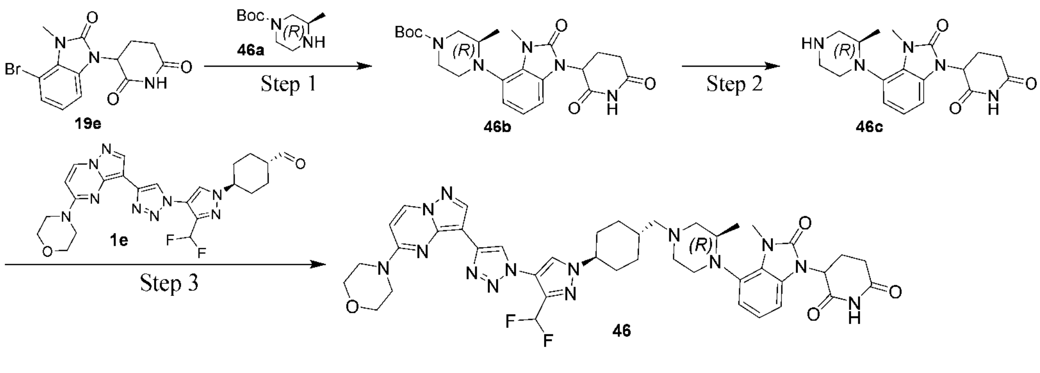


1) Step 1: Compound 46a (400 mg, 1.18 mmol, Bide Pharmatech Ltd.), compound 19e (355 mg, 1.77 mmol, prepared by the method disclosed for the step 3 product on page 88 of the specification in the patent application "WO 2022012623 A"),2-dicyclohexylphosphino-2',6'-diisopropoxy-1,1'-biphenyl (110 mg, 0.240 mmol) and (2-dicyclohexylphosphino-2',6'-diisopropoxy-1,1'-biphenyl)(2-amino-1,1'-biphenyl-2-yl)palladium(II) methanesulfonate (198 mg, 0.240 mmol) were dissolved in toluene (8 mL), and lithium bis(trimethylsilyl)amide (5.9 mL, 5.91 mmol, 1.0 M solution in tetrahydrofuran, Energy Chemical) was added dropwise under nitrogen protection. The reaction solution was reacted at 80°C under nitrogen atmosphere for 3 h. Water (50 mL) was added to the reaction solution, and the aqueous phase was extracted with ethyl acetate (50 mL × 3). The organic phases were combined, washed with saturated brine (30 mL), dried over anhydrous sodium sulfate, filtered and concentrated to obtain a residue. The residue was purified using silica gel column chromatography with an eluent system (petroleum ether/ethyl acetate = 1/1 to 0/1) to obtain compound 46b. MS m/z(ESI):402.1[M-55]$^+$.

2) Step 2: Compound 46b (176 mg, 0.380 mmol) was dissolved in dichloromethane (2 mL), trifluoroacetic acid (1 mL) was added under stirring, and the reaction solution was reacted at 25°C for 3 h. The reaction solution was concentrated to obtain compound 46c. MS m/z(ESI):358.2[M+1]$^+$.

3) Step 3: Compound 46c (70.0 mg, 60.0 μmol) was dissolved in N,N-dimethylformamide (1.5 mL) and tetrahydrofuran (1 mL), triethylamine (0.02 mL, 0.150 mmol) was added, and the reaction solution was reacted at 25°C for 10 min. Then compound 1e (32.5 mg, 70.0 μmol) and acetic acid (0.02 mL, 0.360 mmol) were added in sequence, and the obtained mixture was reacted at 25°C for 30 min. Then sodium borohydride acetate (75.2 mg, 0.360 mmol) was added, and the obtained mixture was reacted at 25°C for 2 h. The reaction solution was concentrated under reduced pressure to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 15%-95%, flow rate: 25 mL/min) to obtain compound 46. MS m/z(ESI):839.4[M+1]$^+$. $^1$H NMR (400 MHz, DMSO-$d_6$) δ 11.09 (s, 1H), 8.76 (d, $J$ = 7.9 Hz, 1H), 8.66 (s, 1H), 8.53 (s, 1H), 8.38 (s, 1H), 7.21 - 7.15 (m, 1H), 7.14 - 7.07 (m, 1H), 6.98 - 6.92 (m, 1H), 6.91 - 6.86 (m, 1H), 6.81 (d, $J$ = 7.9 Hz, 1H), 5.39 - 5.33 (m, 1H), 4.33 - 4.28 (m, 1H), 3.73 (s, 8H), 3.64 (s, 3H), 3.32 - 3.30 (m, 4H), 3.06 - 2.82 (m, 5H), 2.77 - 2.56 (m, 4H), 2.24 - 2.10 (m, 3H), 2.03 - 1.95 (m, 2H), 1.91 - 1.74 (m, 3H), 1.66 - 1.54 (m, 1H), 1.10 - 1.02 (m, 3H).

Example 47: 3-(4-(2-(((1r,4r)-4-(3-(difluoromethyl)-4-(4-(5-morpholinopyrazolo[1,5-a]pyrimidin-3-yl)-1H-1,2,3-triazol-1-yl)-1H-pyrazol-1-ylcyclohexyl)methyl)-2,7-diazaspiro[3.5]non-7-yl)-3-methyl-2-oxo-2,3-dihydro-1H-benzo[d]imidazol-1-yl)piperidine-2,6-dione (47)

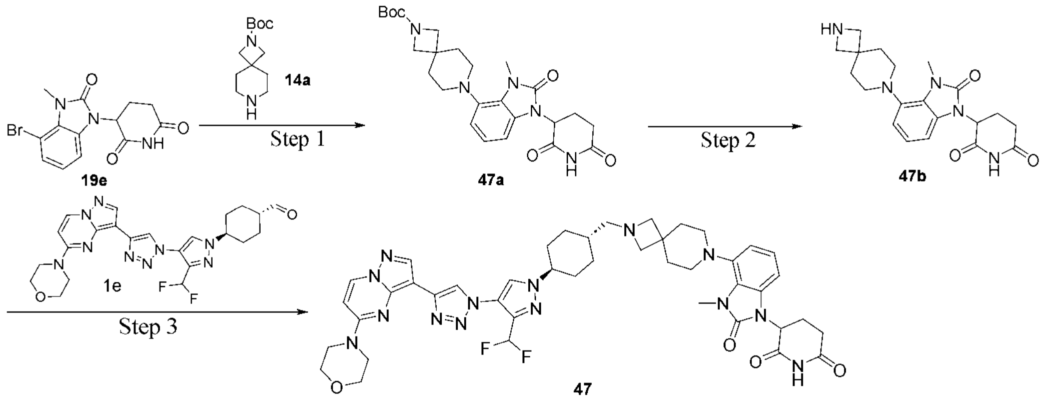


1) Step 1: Compound 19e (150 mg, 0.440 mmol, prepared by the method disclosed for the step 3 product on page 88 of the specification in the patent application "WO 2022012623 A"), compound 14a (151 mg, 0.670 mmol, Bide Pharmatech Ltd.), 2-dicyclohexylphosphino-2',6'-diisopropoxy-1,1'-biphenyl (62.0 mg, 0.130 mmol, Bide Pharmatech Ltd.) and (2-dicyclohexylphosphino-2',6'-diisopropoxy-1,1'-biphenyl)(2-amino-1,1'-biphenyl-2-yl)palladium(II) methanesulfonate (111 mg, 0.130 mmol, adamas) were dissolved in toluene (5 mL), and lithium bis(trimethylsilyl) amide (2.7 mL, 2.70 mmol, 1.0 M solution in tetrahydrofuran, Energy Chemical) was added dropwise under nitrogen protection. The reaction solution was reacted under stirring at 80°C under nitrogen atmosphere for 2 h. The mixture was poured into water (20 mL), and the aqueous phase was extracted with ethyl acetate (30 mL × 3). The combined organic phase was washed with saturated brine (10 mL), dried over anhydrous sodium sulfate, filtered and concentrated to obtain a residue. The residue was purified by silica gel chromatography (petroleum ether/ethyl acetate = 1/1 to 0/1) to obtain compound 47a. MS m/z(ESI):484.3[M+1]$^+$.

2) Step 2: Compound 47a (86.0 mg, 0.180 mmol) was dissolved in 1,4-dioxane (2 mL), and a solution of hydrogen chloride in 1,4-dioxane (2 mL, 4.0 M) was added. The reaction solution was stirred at 25°C for 2 h. The mixture was concentrated to obtain compound 47b. MS m/z(ESI):384.2[M+1]$^+$.

3) Step 3: Compound 47b (60.0 mg, 0.130 mmol) was dissolved in N,N-dimethylformamide (2 mL) and tetrahydrofuran (3 mL), triethylamine (24.0 mg, 0.240 mmol) was added and the obtained mixture was stirred at 25°C for 10 min. Compound 1e (60.0 mg, 0.120 mmol) and acetic acid (40.0 mg, 0.670 mmol) were added to the reaction solution, and the obtained mixture was stirred at 25°C for 30 min. Sodium borohydride acetate (145 mg, 0.680 mmol) was added to the reaction solution, and the obtained mixture was stirred at 25°C for 1 h. The reaction solution was concentrated to obtain a residue. The reaction solution was concentrated under reduced pressure and then purified by high performance liquid chromatography (Gilson_306_1741, chromatographic column: Waters-SunFire-C18-10μm-19*250 mm; mobile phase: water (containing 26.5 mmol/L formic acid) and acetonitrile, gradient ratio: acetonitrile 17%-95%, flow rate: 25 mL/min) to obtain compound 47. MS m/z(ESI):865.6[M+1]$^+$. $^1$H NMR(400MHz, DMSO-$d_6$): δ 11.08 (s, 1H), 8.76 (d, $J$ = 8.0 Hz, 1H), 8.72 - 8.62 (m, 1H), 8.54 (d, $J$ = 4.8 Hz, 1H), 8.38 (s, 1H), 7.36 - 7.04 (m, 1H), 7.01 - 6.93 (m, 1H), 6.92 - 6.84 (m, 2H), 6.81 (d, $J$ = 8.0 Hz, 1H), 5.37 - 5.32 (m, 1H), 4.40 - 4.20 (m, 1H), 3.72 (s, 8H), 3.62 (s, 3H), 3.32 - 2.77 (m, 8H), 2.76 - 2.56 (m, 4H), 2.37 (d, $J$ = 6.4 Hz, 1H), 2.20 - 1.96 (m, 3H), 1.95 - 1.75 (m, 7H), 1.74 - 1.26 (m, 3H), 1.10 - 1.02 (m, 1H).

Example 48: 3-(4-((1r,4r)-4-(3-(difluoromethyl)-4-(4-(5-morpholinopyrazolo[[1,5-a]pyrimidin-3-yl)-1H-1,2,3-triazol-1-yl)-1H-pyrazol-1-yl)cyclohexyl)-3,8-diazabicyclo[3.2.1]oct-8-yl)-3-methyl-2-oxo-2,3-dihydro-1H-benzo[d]imidazol-1-yl) piperidine-2,6-dione (48)

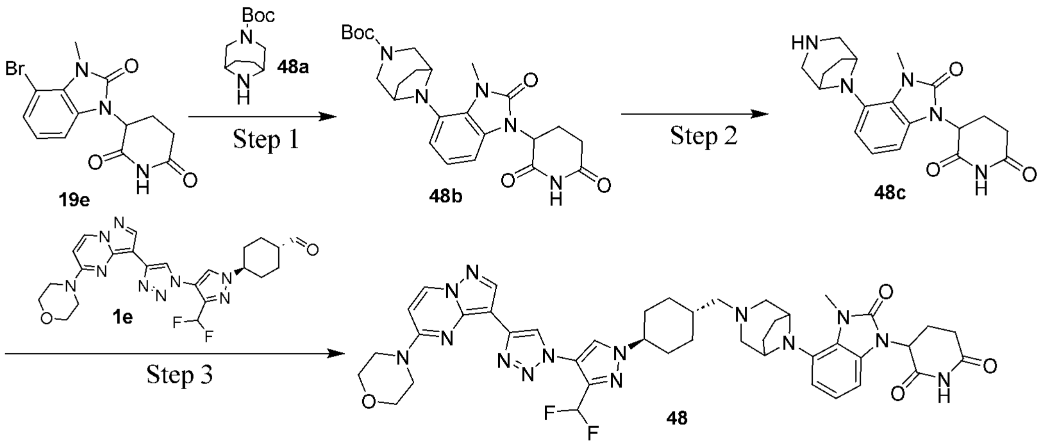


1) Step 1: Compound 19e (500 mg, 1.48 mmol, prepared by the method disclosed for the step 3 product on page 88 of the specification in the patent application "WO 2022012623 A"), compound 48a (471 mg, 2.22 mmol, Bide Pharmatech Ltd.), 2-dicyclohexylphosphino-2,6-diisopropoxy-1,1-biphenyl (138 mg, 30.0 μmol) and (2-dicyclohexylphosphino-2,6-diisopropoxy-1,1-biphenyl)(2-amino-1,1-biphenyl-2-yl)palladium(II) methanesulfonate (248 mg, 30.0 μmol) were dissolved in toluene (5 mL), and lithium bis(trimethylsilyl)amide (8.9 mL, 8.87 mmol, 1 M solution in tetrahydrofuran, Energy Chemical) was added dropwise under nitrogen protection. The obtained mixture was reacted at 80°C for 2 h under nitrogen atmosphere. The reaction solution was concentrated under reduced pressure, and the residue was purified by silica gel column chromatography with an eluent system (dichloromethane/methanol = 40/1 to 15/1) to obtain compound 48b. MS m/z(ESI):470.2[M+1]$^+$.

2) Step 2: Compound 48b (300 mg, 30.0 μmol) was dissolved hydrogen chloride in dioxane (5 mL, 4.0 M), and the obtained mixture was reacted under stirring at 20°C for 1 h. The reaction solution was concentrated under reduced pressure to obtain compound 48c. MS m/z(ESI):370.0[M+1]$^+$.

3) Step 3: Compound 48c (40.9 mg, 0.100 mmol) and compound 1e (50.0 mg, 0.100 mmol) were dissolved in N,N-dimethylformamide (0.5 mL), triethylamine (22.3 mg, 0.200 mmol) and sodium cyanoborohydride (9.43 mg, 0.150 mmol) were added, and the obtained mixture was reacted under stirring at 20°C for 16 h. The reaction solution was filtered, and the filtrate was purified by high performance liquid chromatography (GILSON: GX-281, chromatographic column: Phenomenex Gemini NX 150×30 mm, 5 μm; mobile phase: water (containing 0.225% formic acid) and acetonitrile, gradient ratio: acetonitrile 13%-53%, flow rate: 60 mL/min) to obtain compound 48. MS m/z(ESI):851.5 [M+1]$^+$. $^1$H NMR (400 MHz, DMSO-$d_6$): δ 11.09 (s, 1H), 8.76 (d, *J* = 7.8 Hz, 1H), 8.66 (s, 1H), 8.54 (s, 1H), 8.39 (s, 1H), 7.38 - 7.02 (m, 1H), 6.95 - 6.85 (m, 1H), 6.84 - 6.76 (m, 2H), 6.71 (d, *J* = 8.2 Hz, 1H), 5.37 - 5.31 (m, 1H), 4.34 - 4.27 (m, 1H), 3.80 - 3.68 (m, 13H), 2.96 - 2.82 (m, 1H), 2.79 - 2.58 (m, 4H), 2.50 - 2.40 (m, 2H), 2.25 - 2.12 (m, 4H), 2.05 - 1.92 (m, 3H), 1.90 - 1.75 (m, 6H), 1.70 - 1.50 (m, 1H), 1.16 - 1.03 (m, 2H).

Example 49: 3-(4-((1R,4R)-4-(3-(difluoromethyl)-4-(4-(5-morpholinopyrazolo[[1,5-a]pyrimidin-3-yl)-1H-1,2,3-triazol-1-yl)-1H-pyrazol-1-yl)cyclohexyl)-3,8-diazabicyclo[3.2.1]oct-3-yl)-3-methyl-2-oxo-2,3-dihydro-1H-benzo[d]imidazol-1-yl) piperidine-2,6-dione (49)

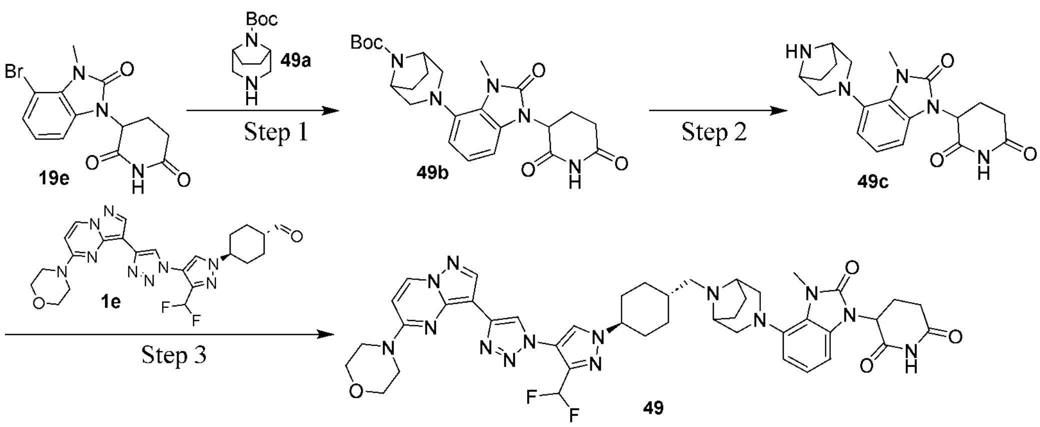

1) Step 1: Compound 19e (500 mg, 1.48 mmol, prepared by the method disclosed for the step 3 product on page 88 of the specification in the patent application "WO 2022012623 A"), compound 49a (471 mg, 2.22 mmol, Bide Pharmatech Ltd.), 2-dicyclohexylphosphino-2,6-diisopropoxy-1,1-biphenyl (248 mg, 0.530 mmol) and (2-dicyclohexylphosphino-2,6-diisopropoxy-1,1-biphenyl)(2-amino-1,1-biphenyl-2-yl)palladium(II) methanesulfonate (17.1 mg, 20.0 μmol) were dissolved in toluene (5.00 mL), and lithium bis(trimethylsilyl)amide (8.4 mL, 8.40 mmol, 1.0 M solution in tetrahydrofuran) was added dropwise under nitrogen protection. The reaction solution was reacted at 80°C under nitrogen atmosphere for 2 h. The reaction solution was concentrated under reduced pressure, and purified by silica gel column chromatography with an eluent system (petroleum ether/ethyl acetate = 3/1 to 1/1) to obtain compound 49b. MS m/z(ESI):470.2[M+1]+.
2) Step 2: Compound 49b (200 mg, 0.430 mmol) was dissolved in hydrogen chloride in 1,4-dioxane (0.5 mL, 4.0 M). The reaction solution was reacted at 25°C for 1 h. The reaction solution was concentrated under reduced pressure to obtain compound 49c.
3) Step 3: Compound 49c (50.0 mg, 140 μmol), compound 1e (67.3 mg, 140 μmol), triethylamine (27.4 mg, 270 μmol) were dissolved in N,N-dimethylformamide (1 mL). Sodium cyanoborohydride (12.8 mg, 0.200 mmol) was added. The obtained mixture was reacted at room temperature for 1 h. The reaction solution was concentrated under reduced pressure and then purified by high performance liquid chromatography (Gilson GX-281, chromatographic column: Boston Prime C18, 30*150 mm, 5 μm; mobile phase: water (containing 0.0500% aqueous ammonia and 10.0 mmol/L ammonium bicarbonate) and acetonitrile, gradient ratio: acetonitrile 47%-77%, flow rate: 25 mL/min) to obtain compound 49. MS m/z(ESI):851.5[M+1]+. $^1$H NMR (400 MHz, DMSO-$d_6$): δ 11.09 (s, 1H), 8.76 (d, *J* = 7.8 Hz, 1H), 8.66 (s, 1H), 8.54 (s, 1H), 8.39 (s, 1H), 7.36 - 7.05 (m, 1H), 6.93 - 6.87 (m, 1H), 6.83 - 6.76 (m, 2H), 6.73 - 6.62 (m, 1H), 5.34 (m, *J* = 5.1, 12.6 Hz, 1H), 4.35 - 4.25 (m, 1H), 3.73 (s, 9H), 3.70 (s, 3H), 2.95 - 2.82 (m, 1H), 2.78 - 2.57 (m, 4H), 2.42 (d, *J* = 9.8 Hz, 2H), 2.26 - 2.10 (m, 4H), 2.06 - 1.93 (m, 3H), 1.91 - 1.75 (m, 6H), 1.65 - 1.55 (m, 1H), 1.19 - 1.04 (m, 3H).

Example 50: 3-(4-((R)-4-(((1r,4R)-4-)3-(difluoromethyl)-4-(4-(5-morpholinopyrazolo[1,5-a]pyrimidin-3-yl)-1H-1,2,3-triazol-1-yl)-1H-pyrazol-1-yl(cyclohexyl)-3-methylpiperazin-1-yl)-3-methyl-2-oxo-2,3-dihydro-1H-benzo[d]imidazol-1-yl)piperidine-2,6-dione (50)

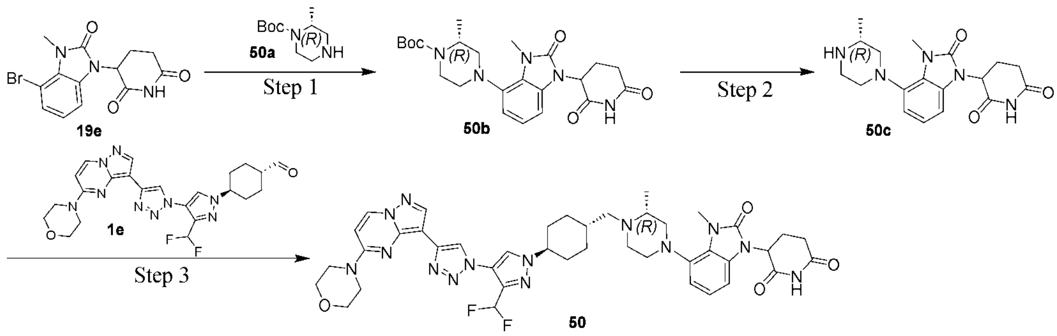


1) Step 1: Compound 19e (400 mg, 1.18 mmol, prepared by the method disclosed for the step 3 product on page 88 of the specification in the patent application "WO 2022012623 A"), compound 50a (355 mg, 1.77 mmol, Bide Pharmatech Ltd.), 2-dicyclohexylphosphino-2',6'-diisopropoxy-1,1'-biphenyl (110 mg, 0.240 mmol, Bide Pharmatech Ltd.) and (2-dicyclohexylphosphino-2',6'-diisopropoxy-1,1'-biphenyl)(2-amino-1,1'-biphenyl-2-yl)palladium(II) methanesulfonate (197 mg, 0.240 mmol, adamas) were dissolved in toluene (12 mL), the reaction solution was subjected to nitrogen replacement three times and then lithium bis(trimethylsilyl)amide (6.5 mL, 6.50 mmol, 1.0 M solution in tetrahydrofuran, Energy Chemical) was added dropwise under nitrogen protection. The reaction solution was reacted under stirring at 80°C under nitrogen atmosphere for 2 h. The mixture was poured into water (20 mL), and the aqueous phase was extracted with ethyl acetate (30 mL × 3). The combined organic phase was washed with saturated brine (10 mL), dried over anhydrous sodium sulfate, filtered and concentrated to obtain a residue. The residue was purified by silica gel chromatography (petroleum ether/ethyl acetate = 1/1 to 0/1) to obtain compound 50b. MS m/z(ESI):458.2[M+1]+.
2) Step 2: Compound 50b (150 mg, 0.110 mmol) was dissolved in dichloromethane (5 mL), trifluoroacetic acid (1 mL) was added, and the reaction solution was stirred at 25°C for 2 h. The reaction solution was concentrated to obtain compound 50c. MS m/z(ESI): 358.2[M+1]+.
3) Step 3: Compound 50c (170 mg, 0.110 mmol) was dissolved in N,N-dimethylformamide (2 mL) and tetrahydrofuran (3 mL), triethylamine (22.0 mg, 0.220 mmol) was added and the obtained mixture was stirred at 25°C for 10 min.

Compound 1e (54.0 mg, 0.110 mmol) and acetic acid (33.0 mg, 0.550 mmol) were added to the reaction solution, the obtained mixture was stirred for 30 min, and then sodium borohydride acetate (115 mg, 0.540 mmol) was added to the reaction solution, and the obtained mixture was stirred for 1 h. The reaction solution was concentrated under reduced pressure, and the obtained residue was purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 6%-95%, flow rate: 25 mL/min) to obtain compound 50. MS m/z(ESI):839.6[M+1]$^+$. $^1$H NMR(400MHz, DMSO-$d_6$): δ 11.09 (s, 1H), 8.76 (d, *J* = 8.0 Hz, 1H), 8.66 (s, 1H), 8.53 (s, 1H), 8.38 (s, 1H), 7.19 (t, *J* = 53.2 Hz, 1H), 7.02 - 6.84 (m, 3H), 6.81 (d, *J* = 8.0 Hz, 1H), 5.43 - 5.28 (m, 1H), 4.30 (t, *J* = 13.2 Hz, 1H), 3.73 (s, 8H), 3.64 (s, 3H), 3.08 - 2.77 (m, 5H), 2.76 - 2.52 (m, 4H), 2.46 - 2.28 (m, 2H), 2.23 - 2.07 (m, 3H), 2.04 - 1.93 (m, 2H), 1.92 - 1.71 (m, 3H), 1.60 (s, 1H), 1.26 - 0.95 (m, 5H).

Example 51: 7-(1-(3-(difluoromethyl)-1-((1R,4r)-4-(((3R)-4-(1-(2,6-dioxopiperidin-3-yl)-3-methyl-2-oxo-2,3-dihydro-1H-benzo[d]imidazol-4-yl)-3-methylpiperazin-1-yl)methyl)cyclohexyl)-1H-pyrazol-4-yl)-1H-1,2,3-triazol-4-yl)pyrrolo[1,2-b]pyridazine-3-carboxamide (51)

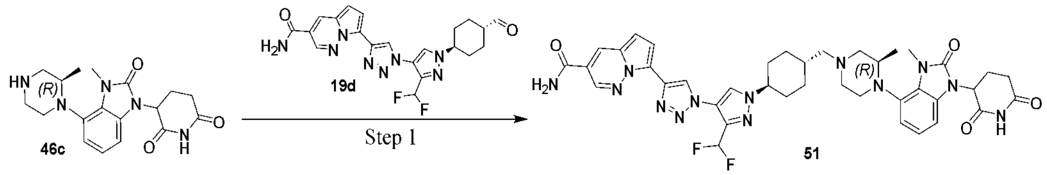


Compound 46c (70.0 mg, 60.0 μmol) was dissolved in N,N-dimethylformamide (1.5 mL) and tetrahydrofuran (1 mL), triethylamine (0.02 mL, 0.150 mmol) was added, and the obtained mixture was reacted at 25°C for 10 min. Then compound 19d (29.7 mg, 70.0 μmol) and acetic acid (0.02 mL, 0.360 mmol) were added in sequence, and the obtained mixture was reacted at 25°C for 30 min. Then sodium borohydride acetate (75.2 mg, 0.360 mmol) was added, and the obtained mixture was reacted at 25°C for 2 h. The reaction solution was concentrated under reduced pressure to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 10%-95%, flow rate: 25 mL/min) to obtain compound 51. MS m/z(ESI):796.4 [M+1]$^+$. $^1$H NMR (400 MHz, DMSO-$d_6$): δ 11.12 (s, 1H), 9.01 (s, 1H), 8.78 (d, *J* = 2.3 Hz, 1H), 8.75 (s, 1H), 8.66 (d, *J* = 2.2 Hz, 1H), 7.65 (d, *J* = 4.6 Hz, 1H), 7.58 (s, 1H), 7.24 (t, *J* = 53.3 Hz, 1H), 7.09 - 6.94 (m, 5H), 5.43 - 5.30 (m, 1H), 4.31 (s, 1H), 3.64 (s, 3H), 3.27 (s, 2H), 2.98 - 2.78 (m, 4H), 2.74 - 2.54 (m, 3H), 2.25 - 2.14 (m, 4H), 2.06 - 1.79 (m, 6H), 1.69 - 1.59 (m, 1H), 1.22 - 1.01 (m, 3H), 0.80 (d, *J* = 5.9 Hz, 2H).

Example 52: 7-(1-(3-difluoromethyl)-1-(1R,4R)-4-(4-(1-(2,6-dioxopiperidin-3-yl)-3-methyl-2-oxo-2,3-dihydro-1H-benzoimidazol-4-yl)ethynyl)piperidin-1-yl)methyl)cyclohexyl)-1H-pyrazol-4-yl)-1H-1,2,3-triazol-4-yl)pyrrolo[1,2-b]pyridazine-3-carboxamide (52)

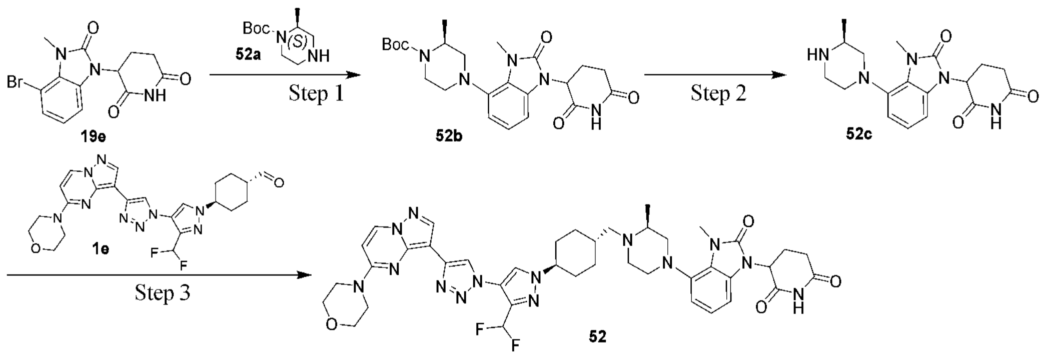


1) Step 1: Compound 19e (200 mg, 0.590 mmol, prepared by the method disclosed for the step 3 product on page 88 of the specification in the patent application "WO 2022012623 A"), compound 52a (355 mg, 1.77 mmol, Bide Pharmatech Ltd.), 2-dicyclohexylphosphino-2',6'-diisopropoxy-1,1'-biphenyl (55.0 mg, 0.120 mmol) and (2-dicyclohexylphosphino-2',6'-diisopropoxy-1,1'-biphenyl)(2-amino-1,1'-biphenyl-2-yl)palladium(II) methanesulfonate (74.0 mg, 90.0 μmol) were dissolved in toluene (3 mL), and lithium bis(trimethylsilyl)amide (4 mL, 4.00 mmol, 1.0 M solution in tetrahydrofuran, Energy Chemical) was added dropwise under nitrogen protection. The reaction solution was stirred at 80°C under nitrogen atmosphere for 2 h. Water (10 mL) was added to the reaction solution, and the aqueous phase was extracted with dichloromethane/methanol (15/1, 15 mL × 3). The combined organic phase was washed with saturated brine (10 mL), dried over anhydrous sodium sulfate, filtered and concentrated to obtain a residue. The residue was purified by silica gel column chromatography (dichloromethane/methanol = 100/1 to 10/1) to obtain compound 52b. MS m/z(ESI):458.3[M+1]+.

2) Step 2: Compound 52b (110 mg, 0.240 mmol) was dissolved in dichloromethane (2 mL), trifluoroacetic acid (2 mL) was added and the reaction solution was stirred at 25°C for 1 h. The reaction solution was concentrated under reduced pressure to remove the solvent to obtain compound 52c. MS m/z(ESI):358.4[M+1]+.

3) Step 3: Compound 52c (50.0 mg, 0.140 mmol) and triethylamine (28.0 mg, 0.280 mmol) were dissolved in N,N-dimethylformamide (2 mL) and tetrahydrofuran (2 mL) and stirred for 10 min. Acetic acid (21.0 mg, 0.350 mmol) and compound 1e (70.0 mg, 0.140 mmol) were added, and the reaction solution was stirred at 25°C for 0.5 h. Sodium borohydride acetate (89.0 mg, 0.4,0 mmol) was slowly added, and the reaction solution was stirred for 1 h. The reaction solution was concentrated under reduced pressure to remove the solvent. The residue was purified by high performance liquid chromatography (Gilson_306_1741, chromatographic column: Waters-SunFire-C18-10μm-19×250 mm; mobile phase: water (containing 10 mmol/L formic acid) and acetonitrile, gradient ratio: acetonitrile 10%-90%, flow rate: 25 mL/min) to obtain compound 52. MS m/z(ESI):839.7[M+1]+. $^1$H NMR(400MHz, DMSO-$d_6$): δ11.10 (s, 1H), 8.77 (d, $J$ = 7.6 Hz, 1H), 8.66 (s, 1H), 8.54 (s, 1H), 8.38 (s, 1H), 7.20 (t, $J$ = 53.2 Hz, 1H), 7.01 - 6.97 (m, 1H), 6.93 - 6.88 (m, 2H), 6.82 (d, $J$ = 8.0 Hz, 1H), 5.38 - 5.33 (m, 1H), 4.30 - 4.27 (m, 1H), 3.73 (s, 8H), 3.64 (s, 3H), 2.99 - 2.84 (m, 6H), 2.71 - 2.59 (m, 4H), 2.18 - 2.14 (m, 3H), 2.01 - 1.98 (m, 2H), 1.86 - 1.83 (m, 4H), 1.18 - 1.05 (m, 6H).

Example 53: 3-(4-(2-(((1r,4r)-4-(3-(difluoromethyl)-4-(4-(5-morpholinopyrazolo[1,5-a]pyrimidin-3-yl)-1H- 1,2,3-triazol-1-yl)-1H-pyrazol-1-yl)cyclohexyl)methyl)-2,9-diazaspiro[5.5]undecan-9-yl)-3-methyl-2-oxo-2,3-dihydro-1H-benzo [d]imidazol-1-yl)piperidine-2,6-dione (53)

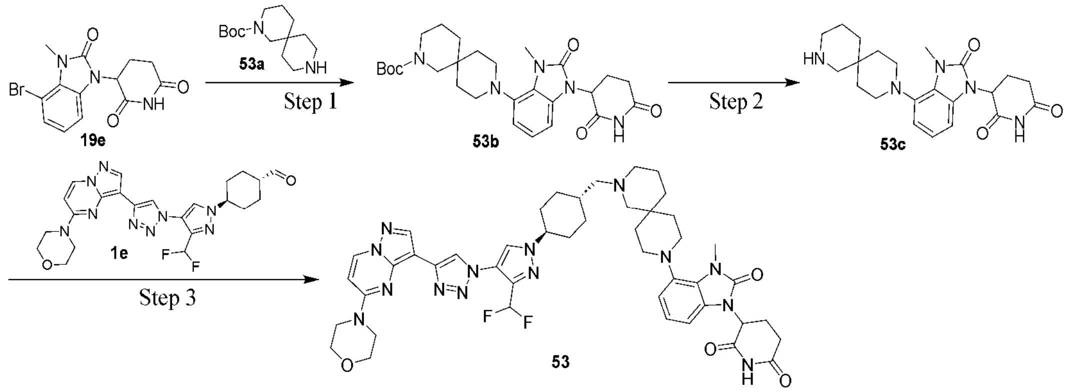


1) Step 1: (2-dicyclohexylphosphino-2',6'-diisopropoxy-1,1'-biphenyl)(2-amino-1,1'-biphenyl-2-yl) palladium (II) methanesulfonate (74.0 mg, 90.0 μmol, adamas) was added to a solution containing compound 19e (150 mg, 0.440 mmol, prepared by the method disclosed for step 3 product on page 88 of the specification of the patent application "WO 2022012623 A"), compound 53a (226 mg, 0.890 mmol, Bide Pharmatech Ltd.), lithium bis(trimethylsilyl)amide (2.2 mL, 2.22 mmol, 1.0 M solution in tetrahydrofuran, Energy Chemical) and 2-dicyclohexylphosphino-2',6'-diisopropoxy-1,1'-biphenyl (41.0 mg, 90.0 μmol, Bide Pharmatech Ltd.) in toluene (3 mL) under nitrogen protection. The reaction solution was subjected to nitrogen replacement three times and reacted at 80°C for 2 h. The reaction solution was quenched with saturated ammonium chloride aqueous solution (20 mL), and the aqueous phase was extracted with dichloromethane (20 mL × 2). The organic phases were combined, dried over anhydrous sodium sulfate and filtered to remove the desiccant, and then the filtrate was concentrated under reduced pressure. The residue was purified by silica gel column chromatography (dichloromethane/methanol = 100/1 to 30/1) to obtain compound 53b. MS m/z(ESI):512.5[M+1]+.

2) Step 2: Compound 53b (100 mg, 0.200 mmol) was dissolved in dichloromethane (2 mL), and trifluoroacetic acid (1 mL) was added. The reaction solution was stirred at 20°C for 2 h. The reaction solution was concentrated under

reduced pressure to obtain the crude trifluoroacetate of compound 53c. MS m/z(ESI):412.8[M+1]$^+$.

3) Step 3: The trifluoroacetate of compound 53c (100 mg, 0.190 mmol) was dissolved in tetrahydrofuran (1 mL) and 1,2-dichloroethane (1 mL), and triethylamine (0.03 mL, 0.190 mmol) was added, and the reaction solution was stirred for 0.5 h. Compound 1e (114 mg, 0.250 mmol) and acetic acid (0.02 mL, 0.290 mmol) were added to the reaction solution and the obtained mixture was stirred for 0.5 h. Sodium borohydride acetate (80.0 mg, 0.380 mmol) was added to the reaction solution. The reaction solution was stirred at 25°C for 17 h. The reaction solution was concentrated under reduced pressure, and the obtained residue was purified by high performance liquid chromatography (A: 0.1% FA/H2O B: ACN, chromatographic column: Waters-CORTECS-C18-2.7um-4.6×30 mm; mobile phase: water (containing 0.1% formic acid) and acetonitrile, gradient ratio: acetonitrile 90%-70%, flow rate: 25 mL/min) to obtain compound 53. MS m/z(ESI):893.9[M+1]$^+$. $^1$H NMR (400 MHz, DMSO-$d_6$): δ11.08 (s, 1H), 8.76 (d, $J$ = 8.0 Hz, 1H), 8.65 (s, 1H), 8.53 (s, 1H), 8.38 (s, 1H), 7.19 (t, $J$ = 53.2 Hz, 1H), 7.02 - 6.91 (m, 2H), 6.90 - 6.77 (m, 2H), 5.41 - 5.29 (m, 1H), 4.34 - 4.21 (m, 1H), 3.81 - 3.67 (m, 8H), 3.63 (s, 3H), 2.96 - 2.80 (m, 5H), 2.74 - 2.56 (m, 3H), 2.37 - 2.27 (m, 2H), 2.19 - 2.03 (m, 5H), 2.01 - 1.73 (m, 7H), 1.68 - 1.42 (m, 6H), 1.31 - 1.21 (m, 1H), 1.12 - 0.97 (m, 2H).

Example 54: 3-(4-(1S,45)-4-((1r,4R)-4-(3-difluoromethyl)-4-(4-(5-morpholinylpyrazolo[1,5-a]pyrimidin-3-yl)-1H-1,2,3-triazol-1-yl)-1H-pyrazol-1-yl)cyclohexyl)methyl(methyl)amino)cyclohexyl)amino)-3-methyl-2-oxo-2,3-dihydro-1H-benzoimidazol-1-yl)piperidine-2,6-dione (54)

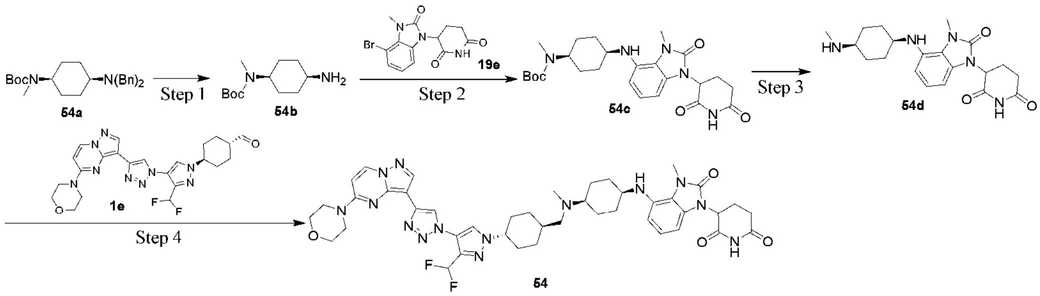


1) Step 1: Compound 54a (1.00 g, 2.45 mmol, prepared by the method disclosed for compound 3A on page 306 of the patent application "WO 2023017442 A") was dissolved in methanol (10 mL) and wet palladium carbon (0.130 g, 10%) was added. The reaction solution was subjected to hydrogen replacement three times. The reaction solution was reacted at 25°C for 12 h. The reaction solution was filtered, and the filtrate was concentrated under reduced pressure to obtain the crude compound 54b. MS m/z(ESI):229.7[M+1]$^+$.

2) Step 2: (2-dicyclohexylphosphino-2',6'-diisopropoxy-1,1'-biphenyl) (2-amino-1,1'-biphenyl-2-yl) palladium (II) methanesulfonate (294 mg, 0.350 mmol, Bide Pharmatech Ltd.), compound 19e (593 mg, 1.75 mmol, prepared by the method disclosed for step 3 product on page 88 of the specification of the patent application "WO 2022012623 A"), compound 54b (600 mg, 2.63 mmol) and 2-dicyclohexylphosphino-2',6'-diisopropoxy-1,1'-biphenyl (164 mg, 0.350 mmol, Bide Pharmatech Ltd.) were dissolved in toluene (5 mL), and lithium bis(trimethylsilyl)amide (8.8 mL, 8.76 mmol, 1.0 M solution in tetrahydrofuran, Energy Chemical) was added dropwise under nitrogen protection. The reaction solution was subjected to nitrogen replacement three times and then reacted at 80°C for 2 h. The reaction solution was quenched with saturated ammonium chloride aqueous solution (20 mL) and the aqueous phase was extracted with dichloromethane (50 mL × 2). The organic phases were combined, dried over anhydrous sodium sulfate and filtered to remove the desiccant, and then the filtrate was concentrated under reduced pressure. The residue was purified by silica gel column chromatography (dichloromethane/methanol = 100/1 to 30/1) to obtain compound 54c. MS m/z(ESI):486.4[M+1]$^+$.

3) Step 3: Compound 54c (300 mg, 0.620 mmol) was dissolved in dichloromethane (2 mL), and trifluoroacetic acid (1 mL) was added. The reaction solution was stirred at 25°C for 1 h. The reaction solution was concentrated under reduced pressure to obtain crude compound 54d. MS m/z(ESI):386.2[M+1]$^+$.

4) Step 4: Compound 54d (200 mg, 0.520 mmol) was dissolved in tetrahydrofuran (2 mL) and N,N-dimethylformamide (1 mL), triethylamine (0.1 mL) was added, and the reaction solution was stirred for 0.5 h. Compound 1e (310 mg, 0.620 mmol) and acetic acid (0.2 mL) were added to the reaction solution, and the obtained mixture was stirred for 0.5 h. Sodium borohydride acetate (80.0 mg, 0.380 mmol) was added to the reaction solution. The reaction solution was stirred at 25°C for 2 h. The reaction solution was concentrated under reduced pressure, and the obtained residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 54. MS m/z(ESI):867.7[M+1]$^+$. $^1$H NMR (400 MHz, DMSO-$d_6$): δ 11.07 (s, 1H), 8.77 (d, *J* = 7.9 Hz, 1H), 8.66 (s, 1H), 8.53 (s, 1H), 8.38 (s, 1H), 7.34 - 7.02 (m, 3H), 6.88 - 6.79 (m, 2H), 6.54 - 6.47 (m, 2H), 5.42 - 5.24 (m, 2H), 4.60 - 4.50 (m, 1H), 4.33 - 4.23 (m, 1H), 3.73 (s, 8H), 3.60 (s, 3H), 2.90 - 2.85 (m, 1H), 2.34 - 2.23 (m, 4H), 2.22 (s, 3H), 2.16 - 2.11 (m, 2H), 2.08 - 2.04 (m, 2H), 1.99 - 1.96 (m, 2H), 1.95 - 1.91 (m, 2H), 1.85 - 1.79 (m, 2H), 1.77 - 1.72 (m, 2H), 1.45 - 1.32 (m, 3H), 1.10 - 1.00 (m, 2H).

Example 55: 3-(4-(((1R,4r)-4-((((1r,4R)-4-(3-(difluoromethyl)-4-(4-(5-morpholinopyrazolo[1,5-a]pyrimidin-3-yl)-1H-1,2,3-triazol-1-yl)-1H-pyrazol-1-yl)cyclohexyl)methyl)(methyl)amino)cyclohexyl)amino)-3-methyl-2-oxo-2,3-dihydro-1H-benzo[d]imidazol-1-yl)piperidine-2,6-dione (55)

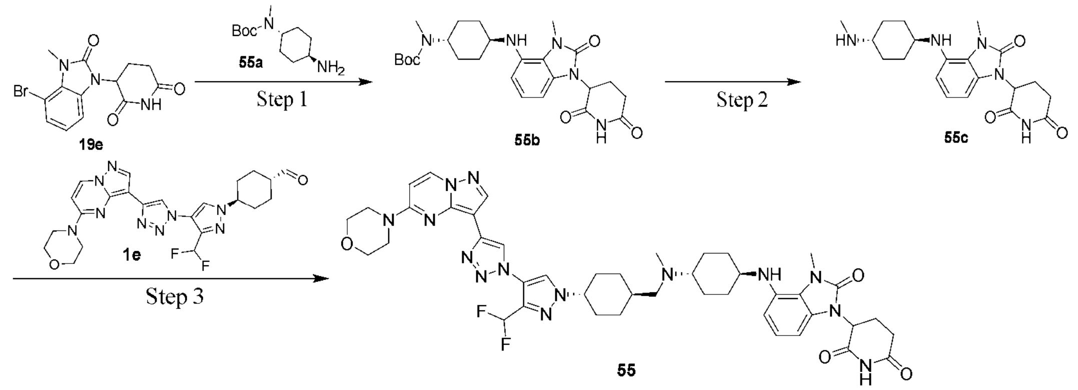


19e 55a 55b 55c 1e 55

1) Step 1: Compound 19e (518 mg, 1.53 mmol, prepared by the method disclosed for step 3 product on page 88 of the specification of the patent application "WO 2022012623 A"), compound 55a (698 mg, 3.06 mmol, prepared by the method disclosed for the intermediate 4 on page 858 of the specification of the patent application "US 2019/0192668 A1"), 2-dicyclohexylphosphino-2',6'-diisopropoxy-1,1'-biphenyl (140 mg, 0.300 mmol, Bide Pharmatech Ltd.), (2-dicyclohexylphosphino-2',6'-diisopropoxy-1,1'-biphenyl)(2-amino-1,1'-biphenyl-2-yl) palladium (II) methanesulfonate (74.2 mg, 90.0 μmol, adamas) were dissolved in toluene (8 mL), and lithium bis(trimethylsilyl)amide (8.9 mL, 8.87 mmol, 1 M solution in tetrahydrofuran, Energy Chemical) was added dropwise under nitrogen protection. The reaction solution was reacted at 80°C under nitrogen atmosphere for 1 h. Water (10 mL) was added to the reaction solution, and the aqueous phase was extracted with dichloromethane (50 mL × 3). The organic phases were combined, dried over anhydrous sodium sulfate and filtered to remove the desiccant, and then the filtrate was concentrated under reduced pressure. The residue was purified by silica gel column chromatography with an eluent system (dichloromethane/methanol = 20/1) to obtain compound 55b. MS m/z(ESI):486.4[M+1]$^+$.
2) Step 2: Compound 55b (350 mg, 0.721 mmol) was dissolved in dichloromethane (6 mL), and trifluoroacetic acid (2 mL) was added. The reaction solution was reacted at 25°C for 2 h, and concentrated under reduced pressure to obtain compound 55c. MS m/z(ESI):386.7[M+1]$^+$.
3) Step 3: Compound 55c (271 mg, 0.701 mmol) was dissolved in 1,2-dichloroethane (5 mL), tetrahydrofuran (5 mL) and N,N-dimethylformamide (5 mL), triethylamine (106 mg, 1.06 mmol) was added until the pH value was greater than 7, and the reaction solution was stirred at 25°C for 10 min. Acetic acid (262 mg, 1.41 mmol) and compound 1e (350 mg, 0.701 mmol) were added at -10°C. The reaction solution was stirred at 25°C for 20 min, and then sodium borohydride acetate (298 mg, 1.41 mmol, Bide Pharmatech Ltd.) was added. The reaction solution was stirred for further 1 h. The reaction solution was concentrated under reduced pressure, and the obtained residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 55. MS m/z(ESI):867.1[M+1]$^+$. $^1$H NMR (400 MHz, DMSO-$d_6$): δ 11.06 (s, 1H), 8.76 (d, *J* = 7.9 Hz, 1H), 8.66 (s, 1H), 8.53 (s, 1H), 8.43 - 8.35 (m, 1H), 7.20 (t, *J* = 53.2 Hz, 1H), 6.90 - 6.78 (m, 2H), 6.55 - 6.47 (m, 2H), 5.38 - 5.29 (m, 1H), 4.65 - 4.44 (m, 1H), 4.36 - 4.22 (m, 1H), 3.73 (s, 8H), 3.61 (s, 3H), 2.93 - 2.83 (m, 1H), 2.72 - 2.62 (m, 2H), 2.31 - 2.25 (m, 3H), 2.23 (s, 3H), 2.18 - 2.06 (m, 5H), 2.02 - 1.87 (m, 4H), 1.84 - 1.73 (m, 4H), 1.44 - 1.34 (m, 2H), 1.32 - 1.21 (m, 2H), 1.10 - 1.00 (m, 2H).

Example 56: 3-(4-(1R,4R)-4-(4-(4-(5-(1R,4R)-2-oxa-5-azabicyclo[2.2.1]hept-5-yl)pyrazolo[1,5-a]pyrimidin-3-yl)-1H-1,2,3-triazol-1-yl)-3-(difluoromethyl)-1H-pyrazol-1-yl)cyclohexyl)methyl)piperidin-4-yl)-3-methyl-2-oxo-2,3-dihydro-1H-benzoimidazol-1-yl)piperidine-2,6-dione (56)

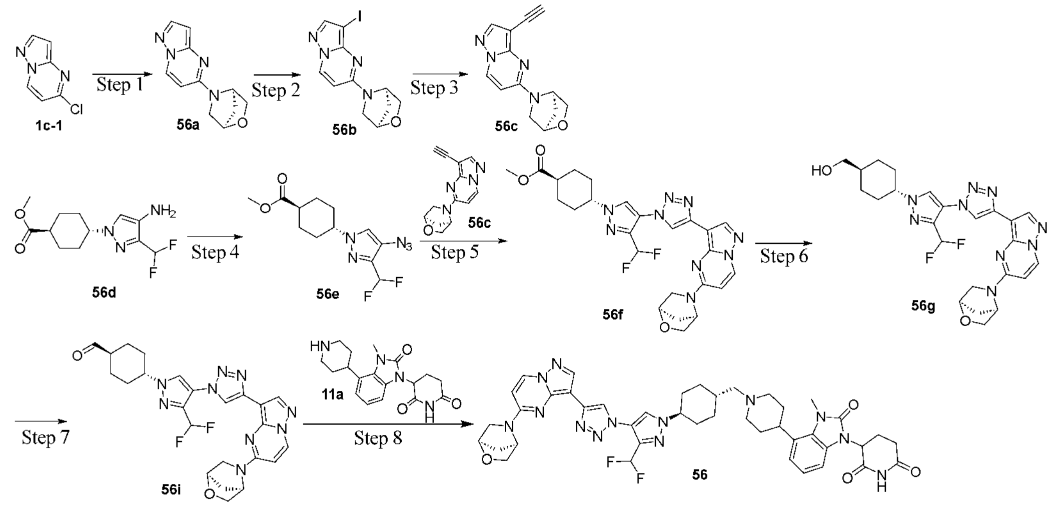


1) Step 1: Compound 1c-1 (3.02 g, 19.5 mmol) was dissolved in acetonitrile (30 mL). N,N-diisopropylethylamine (6.89 g, 53.3 mmol) and (1R,4R)-2-oxa-5-azabicyclo[2.2.1]heptane hydrochloride (2.41 g, 17.8 mmol) were added in sequence. The reaction solution was stirred at 60°C for 4 h. Water (50 mL) was added to the reaction solution, and the obtained mixture was extracted 2 times with ethyl acetate (50 mL), washed with saturated brine (100 mL), dried over anhydrous sodium sulfate, and concentrated under reduced pressure. The residue was purified by column chromatography (petroleum ether/ethyl acetate = 5/1 to 3/1) to obtain compound 56a. MS m/z(ESI):217.1[M+H]$^+$.
2) Step 2: Compound 56a (1.02 g, 4.62 mmol) was dissolved in acetonitrile (20 mL). N-iodosuccinimide (1.56 g, 6.94 mmol) was added. The reaction solution was stirred at 25°C for 18 h. The reaction solution was quenched with saturated sodium thiosulfate aqueous solution (20 ml), extracted with ethyl acetate (30 mL*3), washed with saturated brine (50 mL), dried over anhydrous sodium sulfate, and concentrated under reduced pressure. The residue was purified by column chromatography (petroleum ether/ethyl acetate = 3/1 to 2/1) to obtain compound 56b. MS m/z(ESI):343.0[M+H]$^+$.
3) Step 3: Compound 56b (1.00 g, 2.93 mmol) was dissolved in tetrahydrofuran (10 mL) under stirring. Trimethylsilylacetylene (285 mg, 2.93 mmol), triethylamine (890 mg, 8.77 mmol), cuprous iodide (55.1 mg, 0.29 mmol) and bistriphenylphosphine palladium dichloride (205 mg, 0.29 mmol) were added in sequence. The system was subjected to nitrogen replacement 3 times. The reaction solution was stirred at 25°C for 18 h. Water (50 mL) was added to the reaction solution and the obtained mixture was extracted 3 times with ethyl acetate (30 mL), washed with saturated brine (100 mL), dried over anhydrous sodium sulfate, and concentrated under reduced pressure. The crude product was dissolved in methanol (10 ml), potassium carbonate (810 mg, 5.85 mmol) was added, the reaction solution was stirred at 25°C for 2 h and concentrated under reduced pressure. The residue was purified by column chromatography (petroleum ether/ethyl acetate = 2/1 to 1/1) to obtain compound 56c. MS m/z(ESI):241.1[M+H]$^+$.
4) Step 4: Compound 56d (300 mg, 1.09 mmol, prepared by the method disclosed for the intermediate on page 402 of the specification of the patent application "WO 2022125790 A1") was dissolved in acetonitrile (8 mL). Isoamyl nitrite (153 mg, 1.31 mmol) was added under ice bath. The reaction solution was stirred at 0°C for 1 h. Azidotrimethylsilane (188 mg, 1.64 mmol) was added to the reaction solution. The reaction solution was stirred at 25°C for 16 h. The reaction solution was poured into water (30 mL), the obtained mixture was extracted with ethyl acetate (30 mL × 3), washed with saturated brine (50 mL), and dried over anhydrous sodium sulfate. Compound 56e was obtained by concentration under reduced pressure. MS m/z(ESI):300.1[M+H]$^+$.
5) Step 5: Sodium ascorbate (40 mg, 0.22 mmol), copper sulfate pentahydrate (50 mg, 0.22 mmol) and compound 56c (240 mg, 1.09 mmol) were added to a mixed solution of compound 56e (325 mg, 1.09 mmol) in ethanol (8 mL) and water (5 mL) in sequence. The reaction solution was stirred at 25°C for 2 h. The reaction solution was poured into water (30 mL), the obtained mixture was extracted with ethyl acetate (30 mL × 3), washed with saturated brine (50 mL), dried over anhydrous sodium sulfate and concentrated under reduced pressure. The residue was purified by column chromatography (dichloromethane/methanol = 15/1 to 10/1) to obtain compound 56f. MS m/z(ESI):540.2[M+H]$^+$.
6) Step 6: Compound 56f (320 mg, 0.59 mmol) was added to a 50 mL three-neck bottle, tetrahydrofuran (5 mL) was

added and stirred until dissolved. The system was subjected to nitrogen replacement 3 times and cooled to -50°C, and a solution of lithium aluminum hydride in tetrahydrofuran (0.60 mL, 0.60 mmol, 1.0 M) was added to the reaction solution. The reaction solution was stirred at -50°C for 1 h. Water (20 mL) was added to the reaction solution, and the obtained mixture was extracted with ethyl acetate (20 mL × 3), washed with saturated brine (50 mL × 1), dried over anhydrous sodium sulfate and concentrated under reduced pressure. The residue was purified by column chromatography (methanol/dichloromethane = 15/1 to 10/1) to obtain compound 56g. MS m/z(ESI):512.2[M+H]$^+$.

7) Step 7: Dess-Martin oxidant (250 mg, 0.59 mmol) was added to a solution of compound 56g (200 mg, 0.39 mmol) in dichloromethane (8 mL). The reaction solution was stirred at room temperature for 30 min. Water (15 mL) was added to the reaction solution, and the obtained mixture was extracted with dichloromethane (15 mL × 3). The organic phases were combined, washed with saturated brine (50 mL), and dried over anhydrous sodium sulfate. The filtrate was concentrated under reduced pressure and the residue was slurried with a mixed solvent (petroleum ether/ethyl acetate = 1/1, 5 mL) to obtain compound 56i. MS m/z(ESI):510.1[M+H]$^+$.

8) Step 8: Compound 11a (56.2 mg, 0.140 mmol, prepared by the method disclosed for the intermediate AZK on page 222 of the specification of patent application "WO 2021158634 A1") was dissolved in 1,2-dichloroethane (2 mL) and tetrahydrofuran (2 mL), and triethylamine (13.9 mg, 0.140 mmol) was added. The reaction solution was stirred at 25°C for 10 min, and then acetic acid (8.25 mg, 0.140 mmol) and compound 56i (65.0 mg, 0.130 mmol) were added. The reaction solution was stirred at 25°C under nitrogen protection for 0.5 h, and then sodium borohydride acetate (166 mg, 0.780 mmol) was added. The reaction solution was stirred for 1 h and then quenched with saturated sodium bicarbonate aqueous solution (5 mL) and dichloromethane (5 mL). The aqueous phase was subjected to liquid separation and then extracted with dichloromethane (3 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-T C18,30×150 mm, 5 μm; mobile phase: water (containing 10 mmol/L formic acid) and acetonitrile, gradient ratio: acetonitrile 38%-45%, flow rate: 30 mL/min) to obtain compound 56. MS m/z(ESI):836.4[M+1]$^+$. $^1$H NMR (400 MHz, $CDCl_3$): δ 8.65 (s, 1H), 8.53 (s, 1H), 8.41 - 8.21 (m, 2H), 8.12 (s, 1H), 7.11 - 6.68 (m, 4H), 6.14 (s, 1H), 5.25 - 5.18 (m, 1H), 4.78 (s, 1H), 4.25 - 4.15 (m, 1H), 4.03 - 3.93 (m, 2H), 3.70 (s, 3H), 3.63 - 3.60 (m, 1H), 3.29 - 3.18 (m, 3H), 3.00 - 2.88 (m, 1H), 2.86 - 2.62 (m, 2H), 2.45 - 2.39 (m, 2H), 2.34 - 2.23 (m, 5H), 2.17 - 2.10 (m, 4H), 2.07 - 2.01 (m, 3H), 1.92 - 1.86 (m, 5H), 1.27 - 1.18 (m, 2H).

Example 57: 7-(1-(2,6-dioxopiperidin-3-yl)-3-methyl-2-oxo-2,3-dihydro-1H-imidazol-4-yl)piperazin-1-ylmethyl)cyclohexyl)-1H-pyrazol-4-yl)-1H-pyrazol-4-yl)-1H-pyrazol-4-yl)-1H-1,2,3-triazol-4-yl)pyrrolo[1,2-b]pyridazine-3-carboxamide (57)

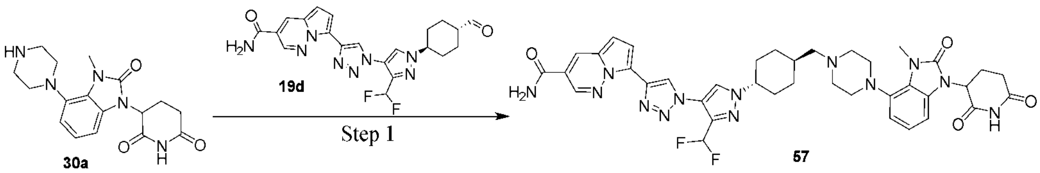


Compound 30a (37.8 mg, 0.110 mmol, prepared by the method disclosed for the intermediate 199 on page 222 of the specification of patent application "WO 2021158634 A1") was dissolved in 1,2-dichloroethane (3 mL) and tetrahydrofuran (3 mL), and triethylamine (5.57 mg, 60.0 μmol) was added. The reaction solution was stirred at 25°C for 10 min, and then acetic acid (3.3 mg, 60.0 μmol) and compound 19d (25 mg, 0.110 mmol) were added. The reaction solution was stirred at 25°C under nitrogen protection for 1 h, and then sodium borohydride acetate (70.0 mg, 0.330 mmol) was added. The reaction solution was stirred at 25°C under nitrogen protection for 1 h, and then quenched with saturated sodium bicarbonate aqueous solution (5 mL) and dichloromethane (5 mL). The aqueous phase was subjected to liquid separation and then extracted with dichloromethane (3 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-T C18,30*150 mm, 5 μm; mobile phase: water (containing 10 mmol/L formic acid) and acetonitrile, gradient ratio: acetonitrile 38%-45%, flow rate: 30 mL/min) to obtain compound 57. MS m/z(ESI):782.6[M+1]$^+$. $^1$H NMR (400 MHz, DMSO-$d_6$): δ 11.09 (s, 1H), 9.01 (s, 1H), 8.78 (d, $J$ = 2.2 Hz, 1H), 8.74 (s, 1H), 8.65 (d, $J$ = 2.2 Hz, 1H), 8.15 (s, 1H), 7.65 (d, $J$ = 4.6 Hz, 1H), 7.55 (s, 1H), 7.17 (t, $J$ = 53.3 Hz, 1H), 7.05 (d, $J$ = 4.6 Hz, 1H), 7.01 - 6.92 (m, 2H), 6.91 - 6.86 (m, 1H), 5.39 - 5.30 (m, 1H), 4.35 - 4.26 (m, 1H), 3.63 (s, 3H), 3.04 - 2.76 (m, 8H), 2.73 - 2.57 (m, 3H), 2.26 - 2.22 (m, 2H), 2.21 - 2.16 (m, 2H), 2.01 - 1.94 (m, 3H), 1.87 - 1.78 (m, 2H), 1.69 - 1.61 (m, 1H), 1.18 - 1.07 (m, 2H).

Example 58: 3-(4-(9-(((1S,4r)-4-(3-(difluoromethyl)-4-(4-(5-((S)-3-methylmorpholinyl)pyrazolo[1,5-a]pyrimidin-3-yl)-1H-1,2,3-triazol-1-yl)-1H-pyrazol-1-yl(cyclohexyl)methyl)-3,9-diazaspiro[5.5]undecan-3-yl)-3-methyl-2-oxo-2,3-dihydro-1H-benzo[d]imidazol-1-yl)piperidine-2,6-dione (58)

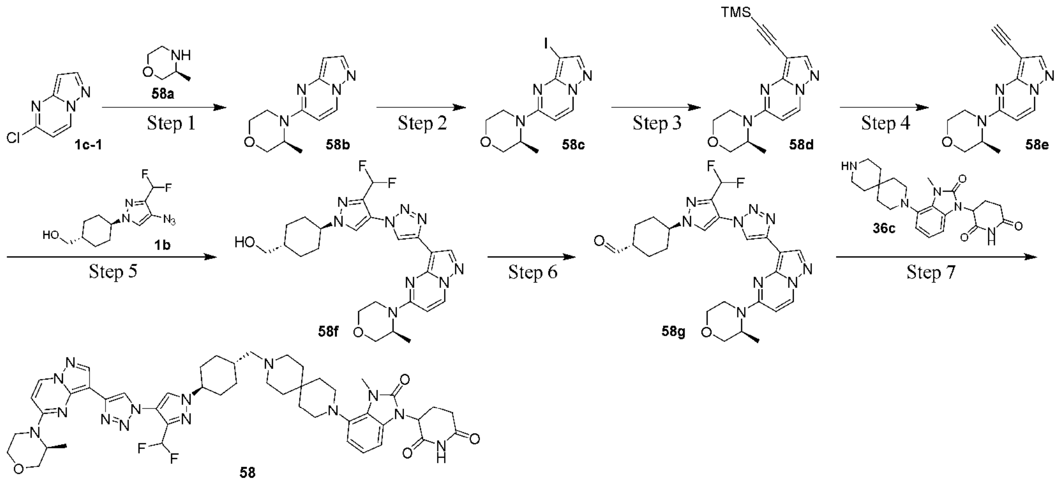


1) Step 1: Compound 1c-1 (15.0 g, 97.7 mmol, Shanghai Haohong) was dissolved in N,N-dimethylformamide (200 mL), cesium carbonate (47.7 g, 147 mmol) and compound 58a (10.4 g, 103 mmol, Bide Pharmatech Ltd.) were added, and the reaction solution was reacted at 110°C for 12 h. Water (1 L) was added to the reaction solution, and the aqueous phase was extracted with ethyl acetate (100 mL × 3). The combined organic phase was dried over anhydrous sodium sulfate and filtered to remove the desiccant, and then the filtrate was concentrated under reduced pressure. The residue was purified by silica gel column chromatography with an eluent system (petroleum ether/ethyl acetate = 3/1 to 1/1) to obtain compound 58b. MS m/z(ESI):219.2[M+1]$^+$.

2) Step 2: Compound 58b (4.00 g, 18.3 mmol) was dissolved in acetonitrile (40 mL), and N-iodosuccinimide (6.19 g, 27.5 mmol) was slowly added in portions. The obtained mixture was reacted at room temperature for 1 h. Water (100 mL) was added to the reaction solution, and the aqueous phase was extracted with ethyl acetate (100 mL × 3). The combined organic phase was dried over anhydrous sodium sulfate and filtered to remove the desiccant, and then the filtrate was concentrated under reduced pressure. The residue was purified by silica gel column chromatography with an eluent system (petroleum ether/ethyl acetate = 3/1 to 1/1) to obtain compound 58c. MS m/z(ESI):345.1[M+1]$^+$.

3) Step 3: Compound 58c (6.00 g, 17.4 mmol) was dissolved in tetrahydrofuran (100 mL), and triethylamine (2.65 g, 26.2 mmol), trimethylsilylacetylene (3.70 mL, 26.2 mmol), dichlorobis(triphenylphosphine)palladium(II) (1.22 g, 1.74 mmol) and cuprous iodide (330 mg, 1.74 mmol) were added. The reaction solution was subjected to nitrogen replacement three times and then reacted at 25°C for 1 h. Water (50 mL) was added to the reaction solution, and the aqueous phase was extracted with ethyl acetate (50 mL × 3). The combined organic phase was dried over anhydrous sodium sulfate and filtered to remove the desiccant, and then the filtrate was concentrated under reduced pressure. The residue was purified by silica gel column chromatography with an eluent system (petroleum ether/ethyl acetate = 3/1 to 1/1) to obtain compound 58d. MS m/z(ESI):315.1[M+1]$^+$.

4) Step 4: Compound 58d (3.00 g, 9.54 mmol) was dissolved in methanol (30 mL), potassium carbonate (2.64 g, 19.1 mmol) was added and the obtained mixture was reacted at 25°C for 1 h. Water (50 mL) was added to the reaction solution, and the aqueous phase was extracted with ethyl acetate (50 mL × 3). The combined organic phase was dried over anhydrous sodium sulfate and filtered to remove the desiccant, and then the filtrate was concentrated under reduced pressure. The residue was purified by silica gel column chromatography with an eluent system (petroleum ether/ethyl acetate = 3/1 to 1/1) to obtain compound 58e. MS m/z(ESI):243.0[M+1]$^+$.

5) Step 5: Compound 58e (1.80 g, 7.43 mmol) was dissolved in tert-butanol (20 mL), and sodium ascorbate (150 mg, 0.740 mmol, Energy Chemical), compound 1b (2.02 g, 7.43 mmol) and cuprous oxide (860 mg, 5.94 mmol, Bide Pharmatech Ltd.) were added. The reaction solution was reacted at 80°C for 1 h. The reaction solution was concentrated under reduced pressure, and the residue was purified by silica gel column chromatography with an eluent system (petroleum ether/ethyl acetate = 2/1 to 1/1) to obtain compound 58f. MS m/z(ESI):514.2[M+1]$^+$. $^1$H NMR (400 MHz, DMSO-$d_6$): δ 8.77 (d, $J$ = 7.9 Hz, 1H), 8.67 (s, 1H), 8.52 (s, 1H), 8.38 (s, 1H), 7.21 (t, $J$ = 53.1 Hz, 1H), 6.79 (d, $J$ = 7.7 Hz, 1H), 4.57 - 4.49 (m, 2H), 4.32 - 4.17 (m, 2H), 4.00 - 3.93 (m, 1H), 3.78 - 3.72 (m, 1H), 3.69 - 3.63 (m, 1H), 3.56 - 3.46 (m, 1H), 3.43 - 3.37 (m, 1H), 3.31 - 3.20 (m, 4H), 2.18 - 2.09 (m, 2H), 1.93 - 1.86 (m, 2H), 1.86 - 1.74 (m,

2H), 1.55 - 1.38 (m, 1H), 1.25 (d, *J* = 6.8 Hz, 3H).

6) Step 6: Compound 58f (780 mg, 1.52 mmol) was dissolved in dichloromethane (25 mL), and Dess-Martin oxidant (980 mg, 2.31 mmol) was added. The reaction solution was stirred at 25°C for two h. The reaction solution was poured into water (30 mL) and the aqueous phase was extracted with dichloromethane (30 mL × 3). The combined organic phase was washed with saturated brine (50 mL), dried over anhydrous sodium sulfate and filtered to remove the desiccant, and then the filtrate was concentrated under reduced pressure. The residue was slurried with a mixed solvent (petroleum ether/ethyl acetate = 1/1, 20 mL) to obtain compound 58g. MS m/z(ESI):512.3[M+1]$^+$.

7) Step 7: Compound 36c (125 mg, 0.170 mmol) was dissolved in N,N-dimethylformamide (0.5 mL) and tetrahydrofuran (2 mL), triethylamine (51.0 mg, 0.500 mmol) was added and the obtained mixture was stirred at 25°C for 10 min. Then compound 58g (87.0 mg, 0.170 mmol) and acetic acid (61.0 mg, 1.02 mmol) were added to the reaction solution, and the obtained mixture was stirred at 25°C for 30 min. Sodium borohydride acetate (217 mg, 1.02 mmol) was added to the reaction solution, and the obtained mixture was stirred at 25°C for 1 h. The reaction solution was concentrated under reduced pressure and the obtained residue was purified by high performance liquid chromatography (Gilson _306_1741, chromatographic column: Waters-SunFire-C18-10μm-19×250 mm; mobile phase: water (containing 26.5 mmol/L formic acid) and acetonitrile, gradient ratio: acetonitrile 19%-95%, flow rate: 25 mL/min) to obtain compound 58. MS m/z(ESI): 907.6[M+1]$^+$. $^1$H NMR (400 MHz, DMSO-$d_6$) δ 11.10 (s, 1H), 8.76 (d, *J* = 8.0 Hz, 1H), 8.66 (s, 1H), 8.53 (s, 1H), 8.38 (s, 1H), 7.20 (t, *J* = 53.2 Hz, 1H), 7.00 - 6.92 (m, 2H), 6.86 (d, *J* = 5.2 Hz, 1H), 6.78 (d, *J* = 8.0 Hz, 1H), 5.35 (dd, *J* = 12.8, 5.2 Hz, 1H), 4.54 (s, 1H), 4.33 - 4.13 (m, 2H), 3.98 - 3.94 (m, 1H), 3.78 - 3.74 (m, 1H), 3.66-3.62 (m, 4H), 3.50 (t, *J* = 10.4 Hz, 1H), 3.27 - 3.19 (m, 1H), 2.87 (s, 5H), 2.73 - 2.57 (m, 2H), 2.36 (s, 4H), 2.23 - 2.07 (m, 4H), 2.04 - 1.76 (m, 5H), 1.73 - 1.37 (m, 9H), 1.24 (d, *J* = 6.8 Hz, 3H), 1.07 (q, *J* =11.2 Hz, 2H).

Example 59: 3-(4-(4-(-4-(((1S,4r)-4-(3-(difluoromethyl)-4-(4-(5-((S)-3-methylmorpholinyl)pyrazolo[1,5-a]pyrimidin-3-yl)-1H-1,2,3-triazol-1-yl)-1H-pyrazol-1-yl(cyclohexyl)methyl)piperazin-1-yl)piperidin-1-yl)-3-methyl-2-oxo-2,3-dihydro-1H-benzo[d]imidazol-1-yl)pyridine-2,6-dione (59)

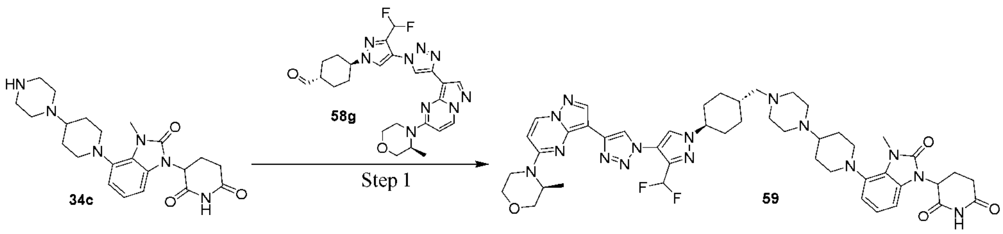


Compound 34c (70.0 mg, 0.130 mmol) was dissolved in N,N-dimethylformamide (1.5 mL) and tetrahydrofuran (1 mL), triethylamine (0.04 mL, 0.320 mmol) was added, and the reaction solution was reacted at 25°C for 10 min. Then 58g (72.9 mg, 0.140 mmol) and acetic acid (0.04 mL, 0.780 mmol) were added in sequence, and the obtained mixture was reacted at 25°C for 30 min. Then sodium borohydride acetate (164 mg, 0.780 mmol) was added, and the obtained mixture was reacted at 25°C for 2 h. The reaction solution was concentrated under reduced pressure and the obtained residue was purified by high performance liquid chromatography (Gilson_306_1741, chromatographic column: Waters-Xbridge-C18-10μm-19×250 mm; mobile phase: water (0.1% formic acid) and acetonitrile, gradient ratio: acetonitrile 15%-95%, flow rate: 25 mL/min) to obtain compound 59 (18.7 mg). MS m/z(ESI):922.6[M+1]$^+$. $^1$HNMR (400 MHz, DMSO-$d_6$): δ 11.11 (s, 1H), 8.77 (d, *J* = 7.9 Hz, 1H), 8.66 (s, 1H), 8.52 (s, 1H), 8.38 (s, 1H), 7.20 (t, *J* = 53.2 Hz, 1H), 6.97 (t, *J* = 7.9 Hz, 1H), 6.92 - 6.83 (m, 2H), 6.78 (d, *J* = 8.0 Hz, 1H), 5.35 (dd, *J* = 12.6, 5.3 Hz, 1H), 4.53 (s, 1H), 4.35 - 4.14 (m, 2H), 3.96 (dd, *J* = 11.7, 4.0 Hz, 1H), 3.75 (d, *J* = 11.4 Hz, 2H), 3.62 (s, 3H), 3.56 - 3.45 (m, 2H), 3.28 - 3.03 (m, 4H), 2.93 - 2.82 (m, 1H), 2.77 - 2.62 (m, 4H), 2.56 (d, *J* = 15.7 Hz, 4H), 2.42 - 2.25 (m, 4H), 2.13 (d, *J* = 7.1 Hz, 4H), 1.98 - 1.78 (m, 6H), 1.60 (d, *J* = 10.2 Hz, 3H), 1.24 (d, *J* = 6.7 Hz, 3H), 1.07 (d, *J* = 13.5 Hz, 2H).

Example 60: 7-(1-(3-(difluoromethyl)-1-((1r,4r)-4-((2-((1-(2,6-dioxopiperidin-3-yl)-3-methyl-2-oxo-3,3-dihydro-1H-benzo[d]imidazol-4-yl)methyl)-2-azaspiro[3.5]non-7-yl)methyl(amino)cyclohexyl)-1H-pyrazol-4-yl)-1H-1,2,3-triazole)pyrrolo[1,2-b]pyridazine-3-carboxamide (60)

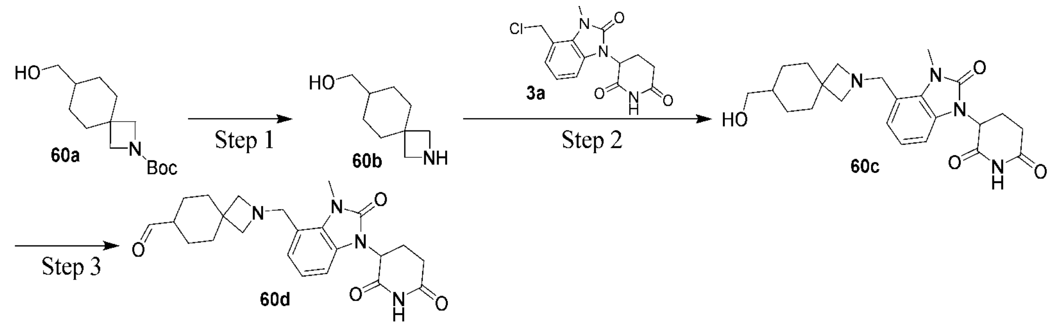

60a 60b 3a 60c 60d

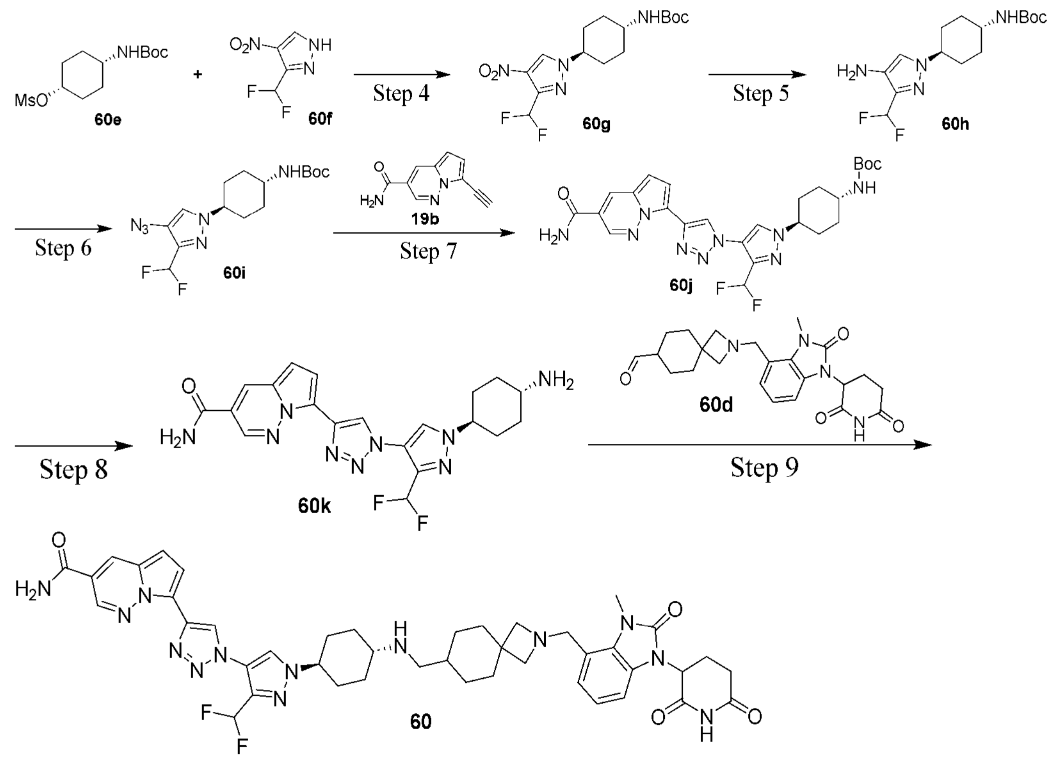

60e 60f 60g 60h 60i 19b 60j 60k 60d 60

1) Step 1: Compound 60a (50.0 mg, 0.100 mmol, Bide Pharmatech Ltd.) was dissolved in dichloromethane (4 mL), and trifluoroacetic acid (1 mL) was added. The reaction solution was reacted under stirring for 2 h at 25°C, and then directly concentrated under reduced pressure to obtain compound 60b, the residue was directly used as the raw material for the next step without purification.MS m/z(ESI):156.1[M+1]$^+$.

2) Step 2: Compound 3a (175 mg, 0.570 mmol, prepared by the method disclosed for the intermediate B32-2 on page 574 of the specification of the patent application "WO 2020206424A1") was dissolved in acetonitrile (8 mL), and compound 60b (106 mg, 0.680 mmol) and potassium carbonate (393 mg, 2.84 mmol) were added. After the reaction solution was reacted under stirring at 80°C for 2 h, water (10 mL) was added to the reaction solution, and the aqueous phase was extracted with ethyl acetate (10 mL × 3). The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 10/1) to obtain compound 60c. MS m/z(ESI):427.3[M+1]$^+$.

3) Step 3: Compound 60c (120 mg, 0.280 mmol) was dissolved in dichloromethane (5 mL). Dess-Martin oxidant (239 mg, 0.560 mmol) was slowly added to the reaction solution at 30°C. The reaction solution was reacted under stirring at 30°C for 2 h, and then the reaction solution was added to saturated sodium bicarbonate aqueous solution (10 mL) for quenching, and dichloromethane (10 mL × 3) was used for extraction. The combined organic phase was dried over anhydrous sodium sulfate and filtered to remove the desiccant, and then the filtrate was concentrated under reduced pressure to obtain compound 60d, the residue was directly used as the raw material for the next step without purification. MS m/z(ESI):425.1[M+1]$^+$.

4) Step 4: Compound 60f (3.00 g, 18.4 mmol) and compound 60e (6.00 g, 20.2 mmol, prepared by the method disclosed for the intermediate k on page 73 of the specification of the patent application "WO 2014195919 A1") were

dissolved in N,N-dimethylformamide (25 mL), and cesium carbonate (18.0 g, 55.2 mmol) was added. The reaction solution was reacted under stirring at 80°C for 12 h, then water (200 mL) was added to the reaction solution and ethyl acetate (150 mL × 3) was used for extraction. The combined organic phase was dried over anhydrous sodium sulfate and filtered to remove the desiccant, and then the filtrate was concentrated under reduced pressure. The residue was purified by silica gel column chromatography (petroleum ether/ethyl acetate = 100/1 to 5/1) to obtain compound 60g. MS m/z(ESI):383.0[M+23]$^+$.

5) Step 5: Compound 60g (2.00 g, 5.55 mmol) was dissolved in methanol (10 mL) and wet palladium carbon (300 mg, 10%) was added. The reaction solution was subjected to hydrogen replacement three times, then reacted under stirring at 25°C for 12 h, and filtered, and the filtrate was concentrated under reduced pressure to obtain compound 60h, the residue was directly used as the raw material for the next step without purification. MS m/z(ESI):331.2[M+1]$^+$.

6) Step 6: Compound 60h (2.00 g, 6.05 mmol) was dissolved in acetonitrile (25 mL), and 2-methyl-2-nitrosopropane (0.9 mL, 7.26 mmol) was added at 0°C. The reaction solution was reacted at 0°C for 30 min, then azidotrimethylsilane (1.2 mL, 9.08 mmol) was added. The reaction solution was heated to 25°C and reacted under stirring at this temperature for 1 h, then water (50 mL) was added to the reaction solution, and ethyl acetate (50 mL × 3) was used extraction. The combined organic phase was dried over anhydrous sodium sulfate and filtered to remove the desiccant, and then the filtrate was concentrated under reduced pressure to obtain compound 60i, the residue was directly used as the raw material for the next step without purification. MS m/z(ESI):301.0[M-55]$^+$.

7) Step 7: Compound 19b (200 mg, 1.08 mmol) and compound 60i (385 mg, 1.08 mmol) were dissolved in water (4 mL) and ethanol (4 mL), and copper sulfate pentahydrate (27.0 mg, 0.110 mmol, Energy Chemical) and sodium ascorbate (21.0 mg, 0.110 mmol, Energy Chemical) were added. The reaction solution was reacted under stirring at 25°C for 12 h, and then concentrated under reduced pressure to remove ethanol. Water (10 mL) was added to the reaction solution, and ethyl acetate (10 mL × 3) was used for extraction. The combined organic phase was dried over anhydrous sodium sulfate and filtered to remove the desiccant, and then the filtrate was concentrated under reduced pressure to obtain compound 60j, the residue was directly used as the raw material for the next step without purification. MS m/z(ESI):542.1[M+1]$^+$.

8) Step 8: Compound 60j (150 mg, 0.280 mmol) was dissolved in hydrogen chloride in 1,4-dioxane (9 mL, 4.0 M). The reaction solution was reacted at 25°C for 1 h and then directly concentrated under reduced pressure to obtain compound 60k, the residue was directly used as the raw material for the next step without purification. MS m/z(ESI):442.3[M+1]$^+$.

9) Step 9: Compound 60k (52.0 mg, 0.120 mmol) was dissolved in 1,2-dichloroethane (2.5 mL) and tetrahydrofuran (2.5 mL), and triethylamine (0.02 mL, 0.120 mmol) was added. The reaction solution was reacted under stirring at 25°C for 15 min, then compound 60d (50.0 mg, 0.150 mmol) and acetic acid (0.01 mL, 0.240 mmol) were added, and the obtained mixture was reacted under stirring for 30 min. Then sodium cyanoborohydride (50.0 mg, 0.240 mmol) was added, and the obtained mixture was reacted under stirring for 30 min, then the reaction system was directly concentrated. The residue was purified by high performance liquid chromatography (Waters-2545, chromatographic column: SharpSil-TC18, 30*150 mm, 5 μm; mobile phase: water (containing 10 mmol/L formic acid) and acetonitrile, gradient ratio: acetonitrile 38%-45%, flow rate: 30 mL/min) to obtain compound 60. MS m/z(ESI):850.4[M+1]$^+$.

Example 61: 7-(1-(3-(difluoromethyl)-1-((1R,4r)-4-((((1r,4R)-4-((1-(2,6-dioxopiperidin-3-yl)- 3-methyl-2-oxo-2,3-dihydro-1H-benzo[d]imidazol-4-yl)amino)cyclohexyl)(methyl)amino)methyl)cyclohexyl)-1H-pyrazol-4-yl)-1H - 1,2,3-triazol-4-yl)pyrrolo[1,2-b]pyridazine-3-carboxamide (61)

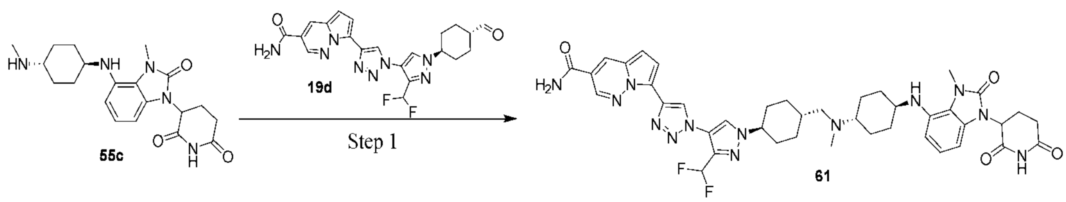


Compound 55c (12.7 mg, 31.0 μmol) was dissolved in 1,2-dichloroethane (1 mL) and tetrahydrofuran (1 mL), triethylamine (3.34 mg, 31.0 μmol) was added until the pH value was greater than 7, and the reaction solution was stirred at 25°C for 10 min. The reaction solution was cooled to -10°C, and acetic acid (8.01 mg, 41.0 μmol) and compound 19d (15.0 mg, 31.0 μmol) were added. The reaction solution was warmed to 25°C and stirred at this temperature for 20 min, and then sodium borohydride acetate (14.1 mg, 71.0 μmol, Bide Pharmatech Ltd.) was added. The reaction solution was stirred at 25°C for 1 h. The reaction solution was concentrated under reduced pressure, and the residue was purified by high performance liquid chromatography (Waters-2545, chromatographic column: SharpSil-T C18, 30×150 mm, 5 μm; mobile

phase: water (containing 10 mmol/L formic acid) and acetonitrile, gradient ratio: acetonitrile 38%-45%, flow rate: 30 mL/min) to obtain compound 61. MS m/z(ESI):824.4[M+1]+,

Example 62: 7-(1-(3-(difluoromethyl)-1-((1r,4r)-4-((2-(1-(2,6-dioxopiperidin-3-yl)-3-methyl-2-oxo-2,3-dihydro-1H-benzo[d]imidazol-4-yl)-2,8-diazaspiro[4.5]dec-8-yl)methyl)cyclohexyl)-1H-pyrazol-4-yl)-1H-pyrazol-1,2,3-triazol-4yl)pyrrolo[1,2-b]pyridazine-3-carboxamide (62)

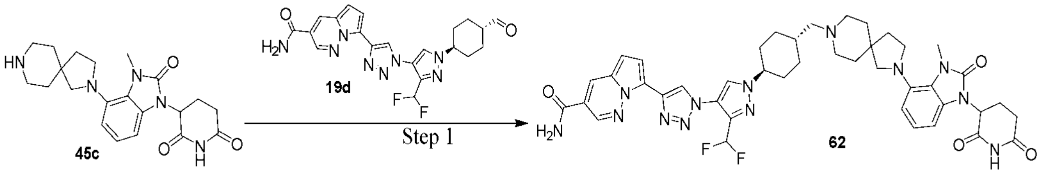


Compound 45c (31.0 mg, 80 μmol) was dissolved in N,N-dimethylformamide (0.7 mL) and tetrahydrofuran (2 mL), and triethylamine (0.01 mL, 80 μmol) was added. The reaction solution was reacted under stirring at 25°C for 15 min, and then compound 19d (35.0 mg, 80 μmol) and acetic acid (0.01 mL, 0.150 mmol) were added, and the obtained mixture was reacted under stirring for 30 min. Then sodium cyanoborohydride (49.0 mg, 0.230 mmol) was added, and the obtained mixture was reacted under stirring for 30 min, then the reaction system was directly concentrated. The residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 62. MS m/z(ESI):836.4[M+1]+. $^1$H NMR (400 MHz, DMSO-$d_6$) δ 11.09 (s, 1H), 9.01 (s, 1H), 8.81 - 8.77 (m, 1H), 8.74 (s, 1H), 8.69 - 8.62 (m, 1H), 8.15 (s, 1H), 7.65 (d, $J$ = 4.7 Hz, 1H), 7.61 - 7.51 (m, 1H), 7.30 (t, $J$ = 53.4 Hz, 1H), 7.05 (d, $J$ = 4.6 Hz, 1H), 6.98 - 6.93 (m, 2H), 6.89 - 6.82 (m, 1H), 5.41 - 5.28 (m, 1H), 4.35 - 4.22 (m, 1H), 3.59 (s, 3H), 3.31 (s, 8H), 3.09 - 3.04 (m, 2H), 2.89 - 2.87 (m, 2H), 2.69 - 2.64 (m, 2H), 2.34 - 2.31 (m, 2H), 2.15 - 2.12 (m, 2H), 1.97 - 1.91 (m, 2H), 1.86 - 1.72 (m, 4H), 1.67 - 1.60 (m, 4H), 1.10 - 1.06 (m, 1H).

Example 63: 7-(1-(3-(difluoromethyl)-1-((1r,4r)-4-((4-(1-(1-(2,6-dioxopiperidin-3-yl)-3-methyl-2-oxo-2,3-dihydro-1H-benzo[d]imidazol-4-yl)azetidin-3-yl)piperazin-1-yl)methyl)cyclohexyl)-1H-pyrazol-4-yl)-1H-1,2,3-triazol-4-yl)pyrrolo[1,2-b]pyridazine-3-carboxamide (63)

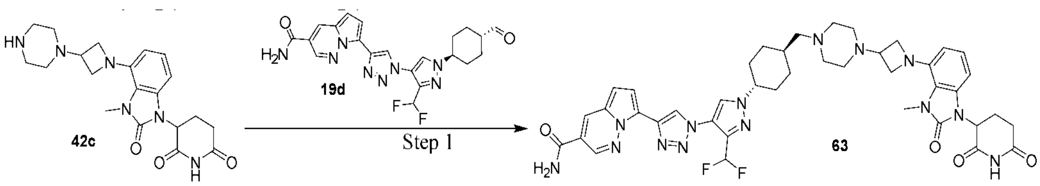


Compound 42c (61.0 mg, 0.150 mmol) was dissolved in N,N-dimethylformamide (1 mL) and tetrahydrofuran (4 mL), and triethylamine (0.02 mL, 0.150 mmol) was added. The reaction solution was reacted under stirring at 25°C for 15 min, and then compound 19d (70.0 mg, 0.150 mmol) and acetic acid (0.02 mL, 0.310 mmol) were added, and the obtained mixture was reacted under stirring for 30 min. Then sodium cyanoborohydride (12.0 mg, 0.190 mmol) was added, and the obtained mixture was reacted under stirring for 30 min, then the reaction system was directly concentrated. The residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 63. MS m/z(ESI):837.3[M+1]+.

Example 64: 7-(1-(2,6-dioxopiperidin-3-yl)-3-methyl-2-oxo-2,3-dihydro-1H-benzoimidazol-4-yl)-2,7-diazaspiro[3.5]nonan-2-yl)methyl)cyclohexyl)-1H-pyrazol-4-yl)-1H-pyrazol-4-yl)-1H-1,2,3-triazol-4-yl)pyrrolo[1,2-b]pyridazine-3-carboxamide (64)

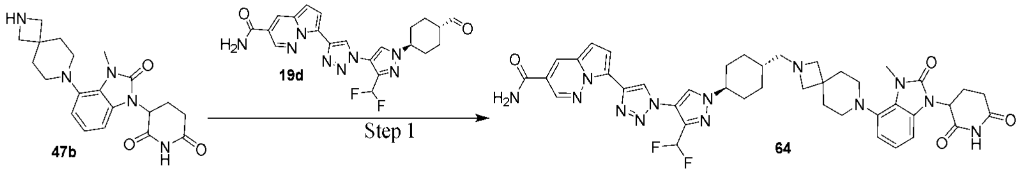


Compound 47b (80.0 mg, 0.210 mmol, prepared by the method disclosed for the intermediate BJR on page 389 of the specification of the patent application "WO 2021/127283 A2") and compound 19d (100 mg, 0.220 mmol) were dissolved in tetrahydrofuran (1 mL) and N,N-dimethylformamide (0.5 mL), and acetic acid (0.1 mL) and sodium borohydride acetate (125 mg, 0.590 mmol) were added in sequence. The reaction solution was stirred at 25°C for 2 h. The reaction solution was poured into water (10 mL), and ethyl acetate (10 mL × 3) was used for extraction. The combined organic phase was washed with saturated brine (20 mL) and filtered to remove the desiccant, and the filtrate was concentrated under reduced pressure. The residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-70%, flow rate: 25 mL/min) to obtain compound 64. MS m/z(ESI):822.3[M+1]$^+$. $^1$H NMR (400 MHz, DMSO-$d_6$): δ 11.11 (s, 1H), 9.01 (s, 1H), 8.83 - 8.74 (m, 2H), 8.66 (d, $J$ = 2.1 Hz, 1H), 8.16 (s, 1H), 7.66 (d, $J$ = 4.6 Hz, 1H), 7.58 (s, 1H), 7.24 (t, $J$ = 53.3 Hz, 1H), 7.05 (d, $J$ = 4.6 Hz, 1H), 6.99 - 6.93 (m, 1H), 6.91 - 6.84 (m, 2H), 5.43 - 5.30 (m, 1H), 4.38 - 4.18 (m, 1H), 3.62 (s, 3H), 3.11 - 2.87 (m, 6H), 2.72 - 2.60 (m, 4H), 2.36 - 2.29 (m, 2H), 2.22 - 2.12 (m, 2H), 1.98 - 1.75 (m, 9H), 1.63 - 1.37 (m, 2H), 1.24 - 1.03 (m, 2H).

Example 65: 7-(1-(3-(difluoromethyl)-1-((1r,4r)-4-(4-(3-(1-(2,6-dioxopiperidin-3-yl)-3-methyl-2-oxo-2,3-dihydro-1H-benzo[d]imidazol-4-yl)prop-2-n-1-yl)oxy)piperidin-1-pyrazol-4-yl)-1H-1,2,3-triazol-4-yl)pyrrolo[1,2-b]pyridazine-3-carboxamide (65)

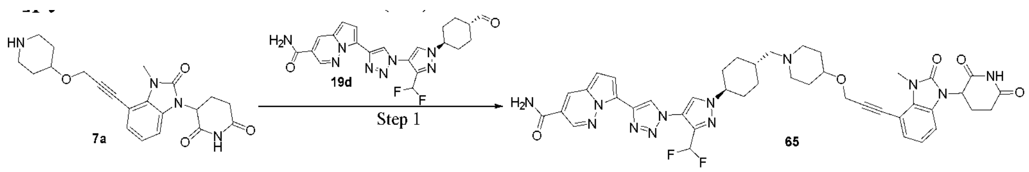


Compound 19d (70.0 mg, 0.150 mmol) and compound 7a (91.6 mg, 0.230 mmol, prepared by the method disclosed for the intermediate 3692 on page 1233 of the specification in the patent application "WO2020/113233A1") were dissolved in N,N-dimethylformamide (1 mL), and triethylamine (46.8 mg, 0.460 mmol) was added. The reaction solution was stirred at 20°C for 5 min, then acetic acid (28.7 mg, 0.150 mmol) was added. The reaction solution was heated to 40°C, and stirred for 2 h at this temperature. The reaction solution was cooled to 20°C, and sodium borohydride acetate (65.0 mg, 0.310 mmol) was added. The reaction solution was reacted under stirring at 20°C for 2 h. Water (2 mL) was added to the reaction solution, and the aqueous phase was extracted with ethyl acetate (5 mL × 3). The combined organic phase was dried over anhydrous sodium sulfate and filtered to remove the desiccant, and then the filtrate was concentrated under reduced pressure. The residue was purified by high performance liquid chromatography (GILSON: GX-281, chromatographic column: Phenomenex C18 75×30 mm×3um; mobile phase: water (containing 0.0500% aqueous ammonia) and acetonitrile, gradient ratio: acetonitrile 37%-77%, flow rate: 60 mL/min) to obtain compound 65. MS m/z(ESI):835.3[M+1]$^+$. $^1$H NMR (400 MHz, DMSO-$d_6$): δ 11.13 (s, 1H), 9.05 - 8.98 (m, 1H), 8.85 - 8.77 (m, 1H), 8.74 (s, 1H), 8.66 (d, $J$ = 2.1 Hz, 1H), 8.16 (s, 1H), 7.66 (d, $J$ = 4.6 Hz, 1H), 7.58 (s, 1H), 7.41 - 7.22 (m, 1H), 7.18 (d, $J$ = 7.6 Hz, 1H), 7.15 - 7.09 (m, 1H), 7.08 - 6.97 (m, 2H), 5.41 (dd, $J$ = 5.1, 12.8 Hz, 1H), 4.48 (s, 2H), 4.35 - 4.23 (m, 1H), 3.65 (s, 3H), 2.96 - 2.80 (m, 1H), 2.76 - 2.61 (m, 4H), 2.28 - 1.98 (m, 8H), 1.96 - 1.75 (m, 5H), 1.65 - 1.43 (m, 4H), 1.14 - 0.98 (m, 2H).

Example 66: 7-(1-(3-(difluoromethyl)-1-((1r,4r)-4-((4-((1-(2,6-dioxopiperidin-3-yl)-3-methyl-2-oxo-2,3-dihydro-1H-benzo[d]imidazol-4-yl)amino)piperidin-1-yl)methyl)cyclohexyl)-1H-pyrazol-4-yl)-1H-1,2,3-triazol-4-yl)pyrrolo[1,2-b]pyridazine-3-carboxamide (66)

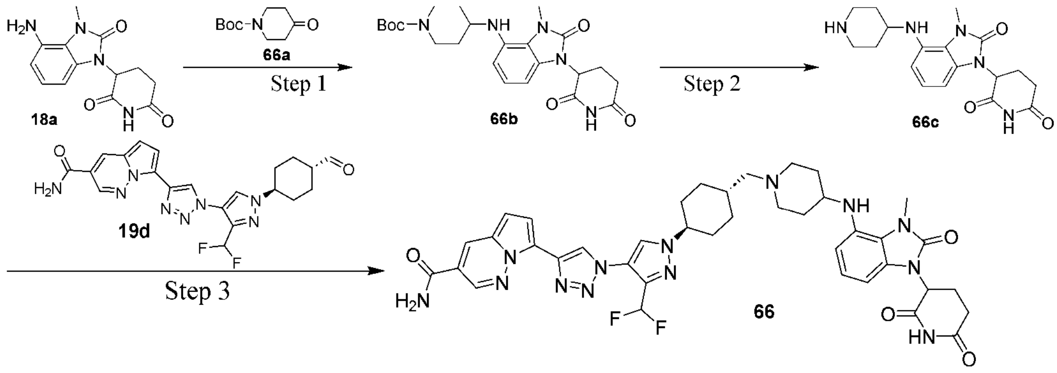


18a 66b 66c 19d 66

1) Step 1: Compound 18a (100 mg, 0.361 mmol, prepared by the method disclosed for the intermediate CD on page 394 of the specification of patent application "WO 2021/188948 A1") and compound 66a (142 mg, 0.721 mmol, Bide Pharmatech Ltd.) were dissolved in 1,4-dioxane (8 mL), and tetraethyl titanate (0.3 mL, 0.731 mmol) was added. The reaction solution was stirred at 80°C under nitrogen protection for 18 h. The reaction solution was cooled to room temperature and sodium cyanoborohydride (45.8 mg, 0.731 mmol) was added. The reaction solution was stirred at 25°C for 1 h. Water (50 mL) was added to the reaction solution, and the aqueous phase was extracted with ethyl acetate (50 mL × 3). The combined organic phase was washed with saturated brine (10 mL), dried over anhydrous sodium sulfate and filtered to remove the desiccant, and then the filtrate was concentrated under reduced pressure. The residue was purified by silica gel column chromatography with an eluent system (dichloromethane/methanol = 40/1 to 5/1) to obtain compound 66b. MS m/z(ESI):456[M-1]$^-$.

2) Step 2: Compound 66b (70.0 mg, 0.151 mmol) was dissolved in dichloromethane (4 mL), and trifluoroacetic acid (1 mL) was added. The reaction solution was reacted at 25°C for 2 h. The reaction solution was concentrated under reduced pressure to obtain 66c. MS m/z(ESI):358.1[M+1]$^+$.

3) Step 3: Compound 66c (32.2 mg, 91.0 μmol) was dissolved in 1,2-dichloroethane (1 mL) and tetrahydrofuran (1 mL), triethylamine (8.91 mg, 91.0 μmol) was added until the pH value was greater than 7. The reaction solution was stirred at 25°C for 10 min. The reaction solution was cooled to -10°C, and acetic acid (21.4 mg, 0.112 mmol) and compound 19d (40.0 mg, 91.0 μmol) were added. The reaction solution was warmed to 25°C and stirred at this temperature for 20 min, and then sodium borohydride acetate (37.3 mg, 0.182 mmol, Bide Pharmatech Ltd.) was added. The reaction solution was stirred at 25°C for 1 h. The reaction solution was concentrated under reduced pressure and then purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 66. MS m/z(ESI):796.3[M+1]$^+$. $^1$H NMR (400 MHz, DMSO-$d_6$) δ 11.06 (s, 1H), 9.00 (s, 1H), 8.78 (d, J = 2.1 Hz, 1H), 8.74 (s, 1H), 8.66 (d, *J* = 2.1 Hz, 1H), 8.18 (s, 1H), 7.65 (d, *J* = 4.6 Hz, 1H), 7.56 (s, 1H), 7.23 (t, *J* = 53.3 Hz, 1H), 7.05 (d, *J* = 4.6 Hz, 1H), 6.86 (t, *J* = 8.0 Hz, 1H), 6.51 (t, *J* = 8.1 Hz, 2H), 5.33 - 5.22 (m, 1H), 4.72 - 4.50 (m, 1H), 4.38 - 4.23 (m, 1H), 3.62 (s, 3H), 3.25 - 3.19 (m, 2H), 2.94 - 2.75 (m, 3H), 2.72 - 2.57 (m, 2H), 2.22 - 2.04 (m, 6H), 2.01 - 1.89 (m, 5H), 1.89 - 1.76 (m, 2H), 1.60 - 1.52 (m, 2H), 1.17 - 1.02 (m, 2H).

Example 67: 7-(1-(3-difluoromethyl)-1-(1R,4R)-4-(3-(4-(1-(2,6-dioxopiperidin-3-yl)-3-methyl-2-oxo-2,3-dihydro-1H-benzoimidazol-4-yl)piperazin-1-yl)azacyclohexyl)-1H-pyrazol-4-yl)-1H-pyrazol-4-yl)-1H-1,2,3-triazol-4-yl)pyrrolo[1,2-b]pyridazine-3-carboxamide (67)

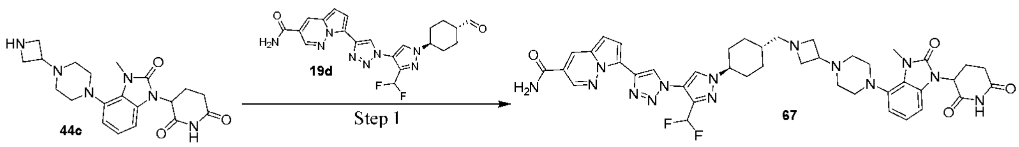


44c 19d 67

Compound 44c (20.0 mg, 50.0 μmol) was dissolved in tetrahydrofuran (2 mL) and N,N-dimethylformamide (1 mL), and triethylamine (0.1 mL) was added. The reaction solution was stirred for 0.5 h. Compound 19d (28.0 mg, 60.0 μmol) and acetic acid (0.2 mL) were added to the reaction solution and the obtained mixture was stirred for 0.5 h. Sodium borohydride acetate (32.0 mg, 0.150 mmol) was added to the reaction solution. The reaction solution was stirred at 25°C for 2 h. The reaction solution was concentrated under reduced pressure and the obtained residue was purified by high

performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 67 (5.00 mg). MS m/z(ESI):837[M+1]$^+$.

Example 68: 7-(1-(2,6-difluoromethyl)-1-(1R,4R)-4-(4-(1-(2,6-dioxopiperidin-3-yl)-3-methyl-2-oxo-2,3-dihydro-1H-benzoimidazol-4-ylmethyl)piperazin-1-yl)methyl)cyclohexyl)-1H-pyrazol-4-yl)-1H-1,2,3-triazol-4-yl)pyrrolo[1,2-b]pyridazine-3-carboxamide (68)

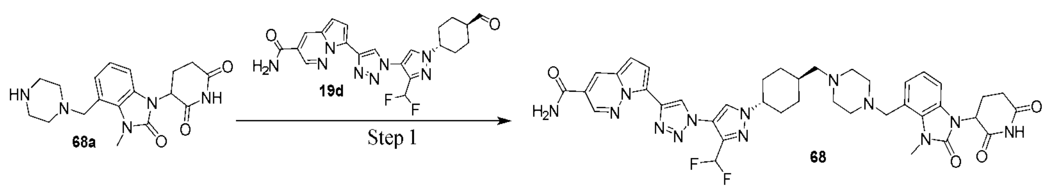


Compound 19d (45.0 mg, 0.100 mmol) and compound 68a (37.2 mg, 0.100 mmol, prepared by the method disclosed for the intermediate ARK on page 697 of the specification of the patent application "WO 2020264499 A1") were dissolved in 1,2-dichloroethane (1 mL), N,N-dimethylformamide (1 mL) and tetrahydrofuran (1 mL), and triethylamine (10.0 mg, 0.100 mmol) was added. The reaction solution was stirred at 25°C for 5 min, and then acetic acid (5.95 mg, 0.100 mmol) was added. The reaction solution was stirred at 25°C for 1 h, and then sodium borohydride acetate (70.0 mg, 0.330 mmol) was added. The reaction solution was stirred at 25°C for 0.5 h, and then quenched with saturated sodium bicarbonate aqueous solution (5 mL) and dichloromethane (10 mL). The aqueous phase was subjected to liquid separation and then extracted with dichloromethane (5 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-T C18,30×150 mm, 5 μm; mobile phase: water (containing 10 mmol/L formic acid) and acetonitrile, gradient ratio: acetonitrile 38%-45%, flow rate: 30 mL/min) to obtain compound 68. MS m/z(ESI):796.2[M+1]$^+$. $^1$H NMR (400 MHz, DMSO-$d_6$): δ 11.11 (s, 1H), 9.00 (s, 1H), 8.78 (d, $J$ = 1.7 Hz, 1H), 8.73 (s, 1H), 8.65 (d, $J$ = 1.8 Hz, 1H), 8.16 (s, 1H), 7.65 (d, $J$ = 4.5 Hz, 1H), 7.58 - 7.35 (m, 1H), 7.28 - 7.04 (m, 3H), 6.96 (t, $J$ = 7.7 Hz, 1H), 6.88 (d, $J$ = 7.5 Hz, 1H), 5.42 - 5.30 (m, 1H), 4.32 - 4.23 (m, 1H), 3.67 (s, 3H), 3.64 (s, 2H), 2.92 - 2.85 (m, 1H), 2.75 - 2.56 (m, 4H), 2.45 - 2.29 (m, 6H), 2.18 - 2.10 (m, 4H), 2.04 - 1.99 (m, 1H), 1.96 - 1.90 (m, 2H), 1.85 - 1.75 (m, 2H), 1.61 - 1.52 (m, 1H), 1.14 - 1.01 (m, 2H).

Example 69: 7-(1-(3-(difluoromethyl)-1-((1r,4r)-4-((4-((1-(2,6-dioxopiperidin-3-yl)-3-methyl-2-oxo-2,3-dihydro-1H-benzo[d]imidazol-4-yl)carbamoyl)piperidin-1-yl)methyl)cyclohexyl)-1H-pyrazol-4-yl)-1H-1,2,3-triazol-4-yl)pyrrolo[1,2-b]pyridazine-3-carboxamide (69)

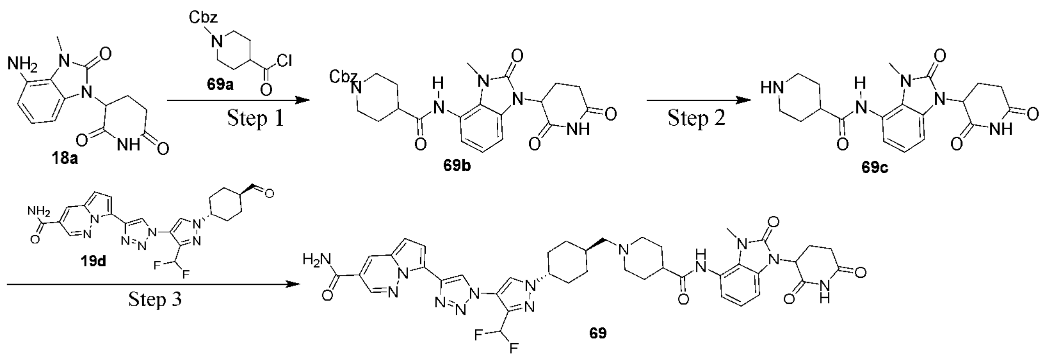


1) Step 1: Compound 18a (100 mg, 0.361 mmol, prepared by the method disclosed for the intermediate CD on page 394 of the specification of the patent application "WO 2021/188948 A1") and compound 69a (174 mg, 0.621 mmol, Bide Pharmatech Ltd.) were dissolved in dichloromethane (2 mL), and triethylamine (0.2 mL, 1.09 mmol) was added. The reaction solution was stirred at 25°C under nitrogen protection for 1 h. Water (10 mL) was added to the reaction solution, and the obtained mixture was extracted with ethyl acetate (15 mL×3). The combined organic phase was washed with saturated brine (10 mL), dried over anhydrous sodium sulfate and filtered to remove the desiccant, and

then the filtrate was concentrated under reduced pressure. The residue was purified by silica gel column chromatography with an eluent system (dichloromethane/methanol = 40/1 to 20/1) to obtain compound 69b. MS m/z(ESI):542 [M+23]+.

2) Step 2: Compound 69b (120 mg, 0.231 mmol) was dissolved in methanol (3 mL) and tetrahydrofuran solution (3 mL), and wet palladium carbon (24 mg, 10%) was added. The reaction solution was subjected to hydrogen replacement three times, and reacted at 25°C under hydrogen atmosphere for 18 h. The reaction solution was filtered and concentrated to obtain compound 69c. MS m/z(ESI):386.1[M+1]+.

3) Step 3: Compound 69c (67.8 mg, 0.181 mmol) and compound 19d (80.1 mg, 0.181 mmol) were dissolved in 1,2-dichloroethane (2 mL) and tetrahydrofuran (2 mL), and acetic acid (39.4 mg, 0.211 mmol) was added. The reaction solution was stirred at 25°C for 20 min, and then sodium borohydride acetate (74.6 mg, 0.351 mmol, Bide Pharmatech Ltd.) was added. The reaction solution was stirred at 25°C for further 1 h. The reaction solution was concentrated under reduced pressure, and the obtained residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 69. MS m/z(ESI):824.2[M+1]+. $^1$H NMR (400 MHz, DMSO-$d_6$): δ 11.11 (s, 1H), 9.63 (s, 1H), 9.01 (s, 1H), 8.79 (d, *J* = 2.1 Hz, 1H), 8.74 (s, 1H), 8.66 (d, *J* = 2.1 Hz, 1H), 8.20 (s, 1H), 7.67 - 7.62 (m, 1H), 7.59 - 7.53 (m, 1H), 7.23 (t, 1H), 7.06 - 6.91 (m, 3H), 6.79 (d, *J* = 6.9 Hz, 1H), 5.48 - 5.28 (m, 1H), 4.34 - 4.24 (m, 1H), 3.47 (s, 1H), 3.39 (s, 3H), 2.96 - 2.90 (m, 2H), 2.78 - 2.61 (m, 2H), 2.23 - 2.11 (m, 5H), 2.00 - 1.89 (m, 5H), 1.87 - 1.75 (m, 5H), 1.73 - 1.64 (m, 2H), 1.14 - 1.02 (m, 2H).

Example 70: 7-(1-(3-(difluoromethyl)-1-((1r,4r)-4-(4-(2-(2,6-dioxopiperidin-3-yl)-1,3-dioxoisoindolin-5-yl)piperazin-1-yl) methyl)cyclohexyl-1H-pyrazol-4-yl)-1H-1,2,3-triazol-4-yl)pyrrolo[1,2-b]pyridazine-3-carboxamide (70)

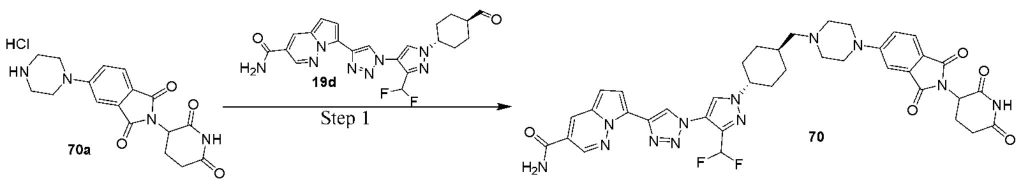


Compound 70a (80.0 mg, 0.210 mmol, prepared by the method of the intermediate on page 568 of the specification of the patent application CN112010858, 2020, A) and triethylamine (21.4 mg, 0.210 mmol) were dissolved in tetrahydrofuran (2 mL) and N,N-dimethylformamide (2 mL). The reaction solution was stirred at 0°C for 10 min. Acetic acid (25.4 mg, 0.420 mmol) and compound 19d (106 mg, 0.230 mmol) were added, and the reaction solution was stirred at 0°C for 30 min. Sodium triacetoxyborohydride (66.8 mg, 0.320 mmol) was added to the reaction solution, and the reaction solution was stirred at 0°C for 1 h. Water (1 mL) was added to the reaction solution for quenching, and the reaction solution was concentrated. The obtained residue was purified by high performance liquid chromatography (Waters-2545, chromatographic column: Boston Green ODS 150×30 mm×5um; mobile phase: Waters (HCl)-$CH_3CN$, gradient ratio: acetonitrile 16%-56%, flow rate: 30 mL/min) to obtain compound 70. MS m/z(ESI):781.4[M+1]+. $^1$H NMR (400 MHz, DMSO-$d_6$): δ 11.09 (s, 1H), 10.64 (s, 1H), 9.01 (s, 1H), 8.79 (d, *J* = 2.2 Hz, 1H), 8.75 (s, 1H), 8.67 (d, *J* = 2.2 Hz, 1H), 8.17 (s, 1H), 7.76 (d, *J* = 8.4 Hz, 1H), 7.65 (d, *J* = 4.5 Hz, 1H), 7.55 (s, 1H), 7.49 (d, *J* = 1.8 Hz, 1H), 7.39 - 7.35 (m, 1H), 7.35 - 7.10 (m, 1H), 7.05 (d, *J* = 4.5 Hz, 1H), 5.13 - 5.07 (m, 1H), 4.42 - 4.30 (m, 1H), 4.20 (d, *J* = 13.0 Hz, 2H), 3.60 - 3.44 (m, 4H), 3.22 - 3.04 (m, 4H), 2.96 - 2.82 (m, 1H), 2.63 - 2.52 (m, 2H), 2.24 - 2.14 (m, 2H), 2.13 - 1.96 (m, 4H), 1.94 - 1.84 (m, 2H), 1.35 - 1.16 (m, 2H).

Example 71: 7-(1-(3-difluoromethyl)-1-(1R,4R)-4-(4-(4-(1-(2,6-dioxopiperidin-3-yl)-3-methyl-2-oxo-2,3-dihydro-1H-benzoimidazol-4-yl)piperazin-1-yl)methyl)cyclohexyl)-1H-pyrazol-4-yl)-1H-1,2,3-triazol-4-yl)pyrrolo[1,2-b]pyridazine-3-carboxamide (71)

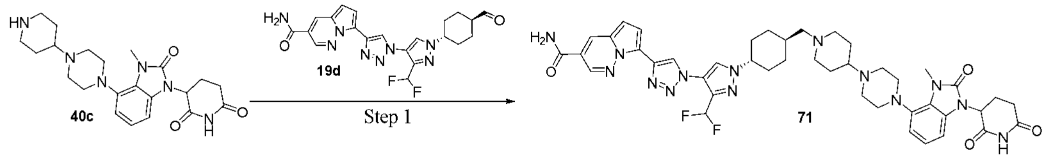

Compound 40c (50.0 mg, 0.120 mmol) and compound 19d (50.0 mg, 0.110 mmol) were dissolved in tetrahydrofuran (1 mL) and N,N-dimethylformamide (0.5 mL), and acetic acid (0.1 mL) and sodium borohydride acetate (75.0 mg, 0.350 mmol) were added in sequence. The reaction solution was stirred at 25°C for 2 h. The reaction solution was poured into water (10 mL), and ethyl acetate (10 mL × 3) was used for extraction. The combined organic phase was washed with saturated brine (10 mL), dried over anhydrous sodium sulfate and filtered to remove the desiccant, and then the filtrate was concentrated under reduced pressure, and the residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-75%, flow rate: 25 mL/min) to obtain compound 71. MS m/z(ESI):865.4[M+1]$^+$. $^1$H NMR (400 MHz, DMSO-$d_6$): δ 11.09 (s, 1H), 9.01 (s, 1H), 8.79 (d, *J* = 2.2 Hz, 1H), 8.74 (s, 1H), 8.66 (d, *J* = 2.1 Hz, 1H), 8.15 (s, 1H), 7.65 (d, *J* = 4.6 Hz, 1H), 7.56 (s, 1H), 7.23 (t, *J* = 53.3 Hz, 1H), 7.05 (d, *J* = 4.6 Hz, 1H), 7.01 - 6.86 (m, 3H), 5.41 - 5.28 (m, 1H), 4.36 - 4.22 (m, 1H), 3.62 (s, 3H), 3.00 - 2.78 (m, 8H), 2.75 - 2.62 (m, 2H), 2.27 - 2.10 (m, 5H), 2.00 - 1.73 (m, 9H), 1.69 - 1.33 (m, 4H), 1.19 - 0.91 (m, 2H).

Example 72: 7-(1-(3-(difluoromethyl)-1-((1r,4r)-4-((4-(2-(2,6-dioxopiperidin-3-yl)-1-oxoisoindolin-5-yl)piperazin-1-yl)methyl)cyclohexyl)-1H-pyrazol-4-yl)-1H-1,2,3-triazol-4yl)pyrrolo[1,2-b]pyridazine-3-carboxamide (72)

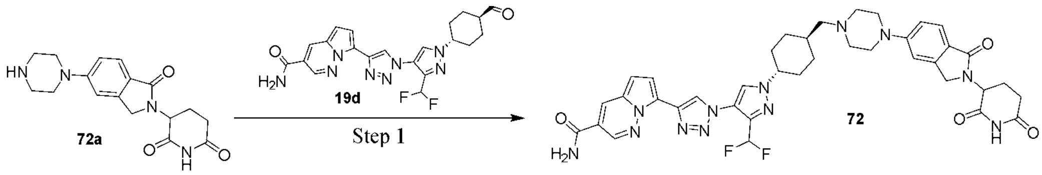


72a 19d 72

Compound 72a (33.0 mg, 0.100 mmol, prepared by the method disclosed for the step 3 product on page 222 of the specification of the patent application "WO 2023023537 A1") was dissolved in *N,N*-dimethylformamide (1 mL) and tetrahydrofuran (4 mL), triethylamine (0.01 mL, 0.100 mmol) was added, and the reaction solution was reacted under stirring at 25°C for 15 min. Then compound 19d (45.0 mg, 0.100 mmol) and acetic acid (0.01 mL, 0.200 mmol) were added, and the obtained mixture was reacted under stirring for 30 min. Then sodium cyanoborohydride (105 mg, 0.500 mmol) was added, and the obtained mixture was reacted under stirring for 30 min. The reaction solution was concentrated under reduced pressure, and the obtained residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 72. MS m/z(ESI):767.2 [M+1]$^+$. $^1$H NMR (400 MHz, DMSO-$d_6$) δ 10.94 (s, 1H), 9.01 (s, 1H), 8.79 (d, *J* = 2.2 Hz, 1H), 8.75 (s, 1H), 8.66 (d, *J* = 2.2 Hz, 1H), 8.15 (s, 1H), 7.65 (d, *J* = 4.6 Hz, 1H), 7.59 - 7.49 (m, 2H), 7.24 (s, 1H), 7.09 - 7.03 (m, 3H), 5.08 - 5.02 (m, 1H), 4.37 - 4.26 (m, 2H), 4.25 - 4.17 (m, 1H), 3.43 - 3.31 (m, 8H), 2.98 - 2.83 (m, 1H), 2.69 - 2.57 (m, 1H), 2.41 - 2.30 (m, 2H), 2.24 - 2.15 (m, 4H), 1.98 - 1.93 (m, 2H), 1.89 - 1.79 (m, 2H), 1.71 - 1.61 (m, 1H), 1.19 - 1.07 (m, 2H).

Example 73: 7-(1-(3-(difluoromethyl)-1-(1-((1-(1-(2,6-dioxopiperidin-3-yl)-3-methyl-2-oxo-2,3-dihydro-1H-benzo[d]imidazol-4-yl)azetidin-3-yl)methyl) piperidin-4-yl)-1H-pyrazol-4-yl)-1H-1,2,3-triazol-4-yl) pyrrolo[1,2-b]pyridazine-3-carboxamide (73)

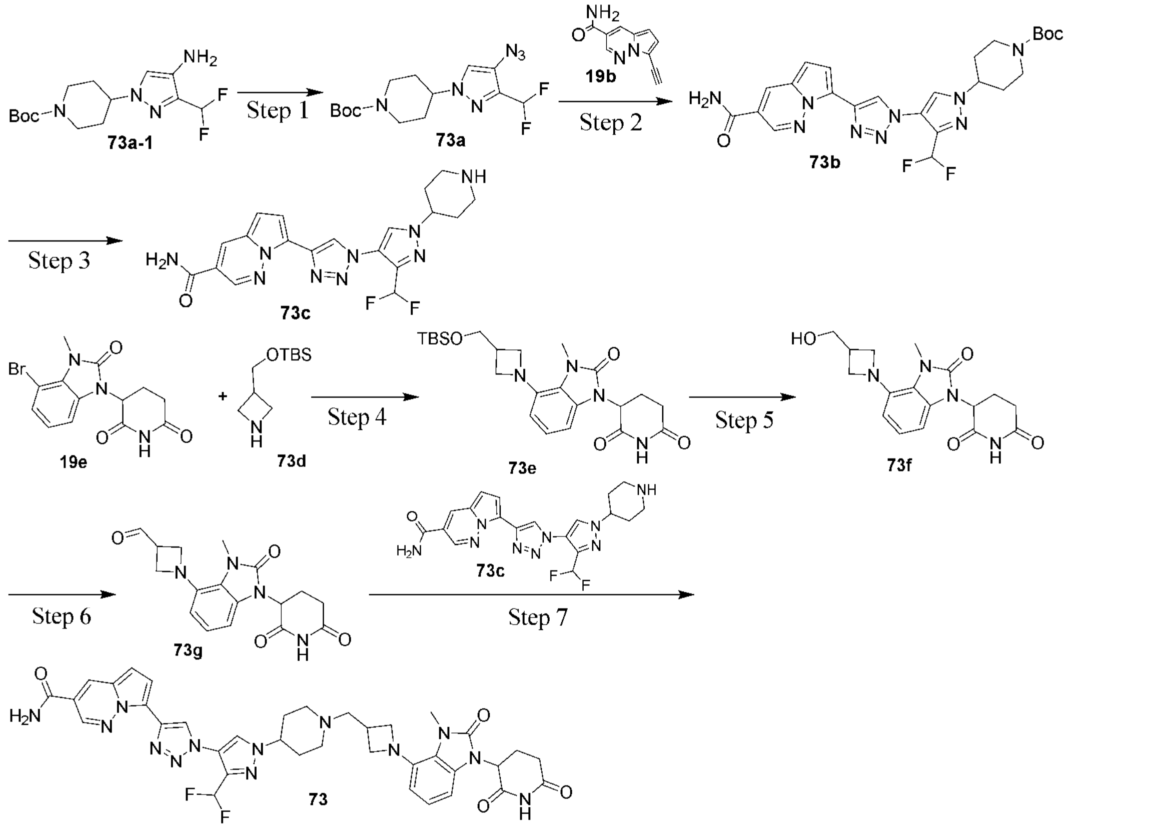


1) Step 1: Compound 73a-1 (1.04 g, 3.29 mmol, prepared by the method disclosed for step 5 product on page 140 of the specification of patent application "WO 2022161414 A1") and acetonitrile (25 mL) were added to a 50 mL three-neck bottle and stirred until dissolved under ice bath. Tert-butyl nitrite (0.47 mL, 3.94 mmol) was slowly added dropwise at 0°C, and the obtained mixture was stirred under ice bath for 30 min. Azidotrimethylsilane (0.65 mL, 4.93 mmol) was slowly added dropwise at 0°C, and the reaction solution was stirred at room temperature for 3 h. Water (40 mL) was added to the reaction solution, and the obtained mixture was extracted with ethyl acetate (25 mL × 3), washed with saturated brine (15 mL), dried over anhydrous sodium sulfate, and concentrated under reduced pressure to obtain compound 73a (1.05 g), which was directly used in the next step without purification. LC-MS: m/z: 287.1 $[M-55]^+$.

2) Step 2: Compound 73a (555 mg, 1.62 mmol) and compound 19b (200 mg, 1.08 mmol) were dissolved in ethanol (4 mL) and water (4 mL), and copper sulfate pentahydrate (27.0 mg, 0.110 mmol, Energy Chemical) and sodium ascorbate (21.4 mg, 0.110 mmol, Energy Chemical) were added. The reaction solution was reacted at 25°C for 12 h, and then concentrated under reduced pressure to remove ethanol. Water (15 mL) was added to the system, and ethyl acetate (15 mL×3) was used for extraction. The combined organic phase was dried over anhydrous sodium sulfate and filtered to remove the desiccant, and then the filtrate was concentrated under reduced pressure. The residue was purified by silica gel column chromatography (dichloromethane/methanol = 100/1 to 20/1) to obtain compound 73b. MS m/z(ESI):528.3$[M+1]^+$.

3) Step 3: Compound 73b (100 mg, 0.190 mmol) was dissolved in dichloromethane (3 mL), and hydrogen chloride in 1,4-dioxane (3 mL, 4 M) was added. The reaction solution was reacted under stirring at 25°C for 1 h, and then concentrated under reduced pressure. The residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 73c. MS m/z(ESI):428.2$[M+1]^+$. $^1$H NMR (400 MHz, DMSO-$d_6$) δ 9.14 - 9.05 (m, 1H), 9.03 (s, 1H), 8.81 - 8.78 (m, 2H), 8.67 (d, $J$ = 2.2 Hz, 1H), 8.17 (s, 1H), 7.66 (d, $J$ = 4.6 Hz, 1H), 7.56 (s, 1H), 7.27 (t, $J$ = 53.2 Hz, 1H), 7.05 (d, $J$ = 4.6 Hz, 1H), 4.77 - 4.61 (m, 1H), 3.47 - 3.40 (m, 2H), 3.17 - 3.07 (m, 2H), 2.37 - 2.30 (m, 2H), 2.27 - 2.17 (m, 2H).

4) Step 4: Compound 19e (420 mg, 1.24 mmol, prepared by the method disclosed for step 3 product on page 88 of the specification of the patent application "WO 2022012623 A"), compound 73d (425 mg, 2.11 mmol, prepared by the method disclosed for the intermediate F on page 413 of the specification of the patent application "WO2023/278759 Al"), 2-dicyclohexylphosphino-2',6'-diisopropoxy-1,1'-biphenyl (116 mg, 0.250 mmol, Bide Pharmatech Ltd.) and (2-dicyclohexylphosphino-2',6'-diisopropoxy-1,1'-biphenyl)(2-amino-1,1'-biphenyl-2-yl) palladium (II) methanesulfonate (104 mg, 0.120 mmol, adamas) were dissolved in toluene (5 mL). Nitrogen was bubbled into the reaction solution for 1 min, and then lithium bis(trimethylsilyl)amide (6.2 mL, 6.21 mmol, 1.0 M solution in tetrahydrofuran, Energy Chemical) was added dropwise. The reaction solution was stirred at 80°C under nitrogen protection for 3 h, and then quenched with saturated ammonium chloride aqueous solution (10 mL) and dichloromethane (15 mL). The mixed solution was filtered through diatomaceous earth, and the aqueous phase was subjected to liquid separation and extracted with dichloromethane (10 mL × 3). The combined organic phase was washed with saturated sodium chloride aqueous solution (15 mL), dried over anhydrous sodium sulfate and filtered to remove the desiccant, and then the filtrate was concentrated under reduced pressure. The residue was purified by silica gel column chromatography with an eluent system (dichloromethane/methanol = 100/1 to 20/1) to obtain compound 73e. MS m/z(ESI):459.2 $[M+1]^+$.

5) Step 5: Compound 73e (460 mg, 1.00 mmol) was dissolved in tetrahydrofuran (10 mL), and tetrabutylammonium fluoride (1.5 mL, 1.50 mmol, 1.0 M solution in tetrahydrofuran ) was added. The reaction solution was stirred at 25°C for 1 h, and then concentrated under reduced pressure. The residue was diluted with dichloromethane (15 mL), the organic phase was washed with saturated sodium bicarbonate aqueous solution (5 mL × 3), dried over anhydrous sodium sulfate and filtered to remove the desiccant, and then the filtrate was concentrated under reduced pressure to obtain compound 73f. MS m/z(ESI):345.1$[M+1]^+$. $^1$H NMR (400 MHz, $CDCl_3$): δ 8.10 (s, 1H), 7.00 (t, *J* = 8.1 Hz, 1H), 6.71 (d, *J* = 8.1 Hz, 1H), 6.47 (d, *J* = 7.8 Hz, 1H), 5.19 (dd, *J* = 12.4, 5.4 Hz, 1H), 3.97 (t, *J* = 7.4 Hz, 2H), 3.92 (d, *J* = 6.3 Hz, 2H), 3.74 (s, 3H), 3.68 (t, *J* = 6.0 Hz, 2H), 2.97 - 2.90 (m, 1H), 2.86 - 2.72 (m, 3H), 2.26 - 2.18 (m, 1H).

6) Step 6: Compound 73f (90.0 mg, 0.260 mmol) was dissolved in dichloromethane (2.5 mL), and Dess-Martin oxidant (144 mg, 0.34 mmol) was added. The reaction solution was stirred at 25°C for 1 h, then diluted with dichloromethane (5 mL) and filtered. The filtrate was concentrated under reduced pressure. The residue was purified by preparative silica gel plate with an eluent system (dichloromethane/methanol = 10/1) to obtain compound 73g. MS m/z(ESI):361.1 $[M+19]^+$.

$^1$H NMR (400 MHz, DMSO-$d_6$): δ 11.08 (s, 1H), 9.90 (d, *J* = 2.1 Hz, 1H), 6.98 (t, *J* = 8.1 Hz, 1H), 6.77 (dd, *J* = 11.8, 8.0 Hz, 2H), 5.34 - 5.30 (m, 1H), 4.01 - 3.95 (m, 4H), 3.57 (s, 3H), 2.86 (d, *J* = 15.9 Hz, 1H), 2.64 (dd, *J* = 27.7, 11.1 Hz, 3H), 2.33 (s, 1H).

7) Step 7: Compound 73g (30.0 mg, 90.0 μmol) and compound 73c (76.9 mg, 0.180 mmol) were dissolved in tetrahydrofuran (2 mL) and N,N-dimethylformamide (2 mL), and triethylamine (8.87 mg, 90.0 μmol) was added. The reaction solution was stirred at 25°C for 5 min, and then acetic acid (5.26 mg, 90.0 μmol) was added. The reaction solution was stirred at 25°C for 1 h, and then sodium borohydride acetate (111 mg, 0.530 mmol) was added. The reaction solution was stirred at 25°C for 0.5 h, and then quenched with saturated sodium bicarbonate aqueous solution (3 mL) and dichloromethane (10 mL). The aqueous phase was subjected to liquid separation and then extracted with dichloromethane (3 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-T C18,30×150 mm, 5 μm; mobile phase: water (containing 10 mmol/L formic acid) and acetonitrile, gradient ratio: acetonitrile 38%-45%, flow rate: 30 mL/min) to obtain compound 73. MS m/z(ESI):754.3$[M+1]^+$. $^1$H NMR (400 MHz, DMSO-$d_6$): δ 11.08 (s, 1H), 9.01 (s, 1H), 8.79 (d, *J* = 2.5 Hz, 2H), 8.65 (d, *J* = 2.2 Hz, 1H), 8.15 (s, 1H), 7.65 (d, *J* = 4.6 Hz, 1H), 7.56 (s, 1H), 7.24 (t, *J* = 53.3 Hz, 1H), 7.05 (d, *J* = 4.6 Hz, 1H), 6.96 (t, *J* = 8.0 Hz, 1H), 6.73 (d, *J* = 8.2 Hz, 1H), 6.66 (d, *J* = 8.2 Hz, 1H), 5.35 - 5.29 (m, 1H), 4.38 - 4.25 (m, 1H), 3.96 (t, *J* = 7.0 Hz, 2H), 3.58 (s, 3H), 3.53 (t, *J* = 6.3 Hz, 2H), 3.01 - 2.95 (m, 2H), 2.92 - 2.83 (m, 2H), 2.70 - 2.61 (m, 4H), 2.20 - 2.10 (m, 3H), 2.08 - 1.95 (m, 4H).

Example 74: 7-(1-(3-(difluoromethyl)-1-(1-((1-(1-(2,6-dioxopiperidin-3-yl)-3-methyl-2-oxo-2,3-dihydro-1H-benzo[d]imidazol-4-yl)piperidin-4-yl)methyl) piperidin-4-yl)-1H-pyrazol-4-yl)-1H-1,2,3-triazol-4-yl)pyrrolo[1,2-b]pyridazine-3-carboxamide (74)

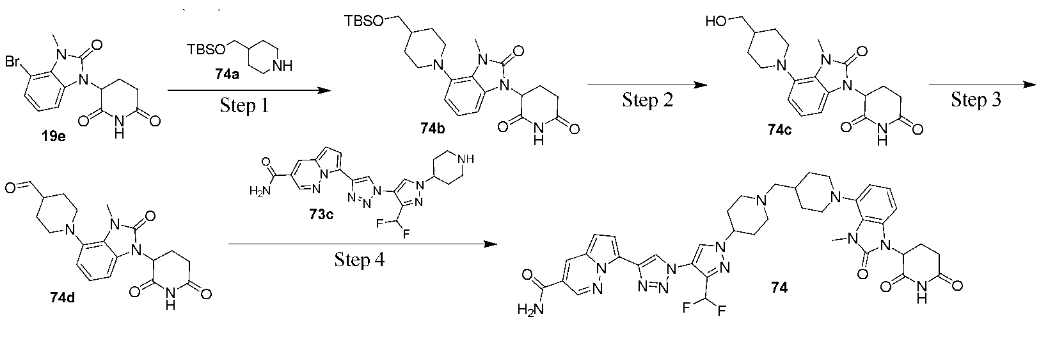

1) Step 1: Compound 19e (500 mg, 1.48 mmol, prepared by the method disclosed for step 3 product on page 88 of the specification of the patent application "WO 2022012623 A"), compound 74a (680 mg, 2.96 mmol, prepared by the method disclosed for the intermediate 1264 on page 904 of the specification of the patent application "WO 2020264499"), 2-dicyclohexylphosphino-2',6'-diisopropoxy-1,1'-biphenyl (140 mg, 0.300 mmol) and (2-dicyclohexylphosphino-2',6'-diisopropoxy-1,1'-biphenyl)(2-amino-1,1'-biphenyl-2-yl)palladium(II) methanesulfonate (74.2 mg, 90.0 μmol) were dissolved in toluene (8 mL), and lithium bis(trimethylsilyl)amide (8.9 mL, 8.87 mmol, 1.0 M solution in tetrahydrofuran, Energy Chemical) was added under nitrogen protection. The reaction solution was reacted at 80°C under nitrogen atmosphere for 1 h. Water (10 mL) was added to the reaction solution, and the aqueous phase was extracted with dichloromethane (10 mL × 3). The combined organic phase was dried over anhydrous sodium sulfate and filtered to remove the desiccant, and then the filtrate was concentrated under reduced pressure. The residue was purified by silica gel column chromatography (petroleum ether/ethyl acetate = 10/1 to 1/1) to obtain compound 74b. MS m/z(ESI):487.2[M+1]$^+$.

2) Step 2: Compound 74b (800 mg, 1.64 mmol) was dissolved in methanol (10 mL), and a solution of hydrogen chloride in 1,4-dioxane (3 mL, 4.0 M) was added. The reaction solution was reacted under stirring for 1 h. The reaction solution was concentrated under reduced pressure, and the residue was directly used in the next step without further purification to obtain compound 74c. MS m/z(ESI):373.1[M+1]$^+$.

3) Step 3: Compound 74c (150 mg, 0.400 mmol) was dissolved in N,N-dimethylformamide (1.5 mL) and dichloromethane (1.5 mL), and Dess-Martin oxidant (341 mg, 0.810 mmol) was added at 0°C. The reaction solution was reacted at 0°C for 1 h and concentrated under reduced pressure. The residue was purified by silica gel column chromatography (dichloromethane/petroleum ether = 10/1) to obtain compound 74d. MS m/z(ESI):371.1[M+1]$^+$.

4) Step 4: Compound 74d (50.0 mg, 0.130 mmol) was dissolved in tetrahydrofuran (1 mL) and N,N-dimethylformamide (1 mL), and triethylamine (0.03 mL, 0.230 mmol) was added. The reaction solution was stirred for 5 min, and then acetic acid (0.03 mL, 0.450 mmol), sodium triacetoxyborohydride (170 mg, 0.810 mmol) and compound 73c (123 mg, 0.270 mmol) were added. The reaction solution was reacted under stirring for 2 h, then water (5 mL) was added to the reaction solution, and the aqueous phase was extracted with ethyl acetate (5 mL × 3). The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 74. MSm/z(ESI):782.4[M+1]$^+$.

Example 75: 7-(1-(3-(difluoromethyl)-1-(1-((1-(2-(2,6-dioxopiperidin-3-yl)-1,3-dioxoisoindolin-5-yl)azetidin-3-yl)methyl) piperidin-4-yl)-1H-pyrazol-4-yl)-1H -1,2,3-triazol-4-yl)pyrrolo[1,2-b]pyridazine-3-carboxamide (75)

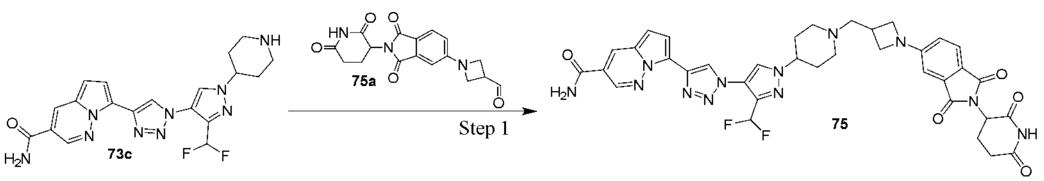


Compound 73c (30.0 mg, 0.702 mmol) and compound 75a (24.0 mg, 0.704 mmol, prepared by the method for the intermediate 36c on page 68 of the specification of the patent application WO 2022/253250 A1) were dissolved in N,N-dimethylformamide (1 mL), triethylamine (0.01 mL, 0.704 mmol) was added, and the obtained mixture was stirred for 5 min. Glacial acetic acid (0.01 mL, 0.704 mmol) was added. The reaction solution was heated to 40°C and stirred at this temperature for 2 h. Sodium borohydride acetate(29.7 mg, 0.140 mmol) was added and the obtained mixture was stirred at 40°C for 2 h. The reaction solution was filtered, and then the residue was purified by high performance liquid chromatography (CAS-CD-SEMI-PREP-L, chromatographic column: Waters xbridge 150×25 mm 10um; mobile phase: water($NH_4HCO_3$)-ACN, gradient ratio: acetonitrile 26%-56%, flow rate: 30 mL/min) to obtain compound 75. MS m/z(ESI):753.2[M+1]$^+$.

Example 76: 7-(1-(3-(difluoromethyl)-1-((1r,4r)-4-((6-(2-((2-(2,6-dioxopiperidin-3-yl)-1,3-dioxoisoindolin-4-yl)amino) ethyl)-2-azaspiro[3.3]hept-2-yl)methyl)cyclohexyl)-1H-pyrazol-4-yl)-1H-1,2,3-triazol-4-yl)pyrrolo[1,2-b]pyridazine-3-carboxamide (76)

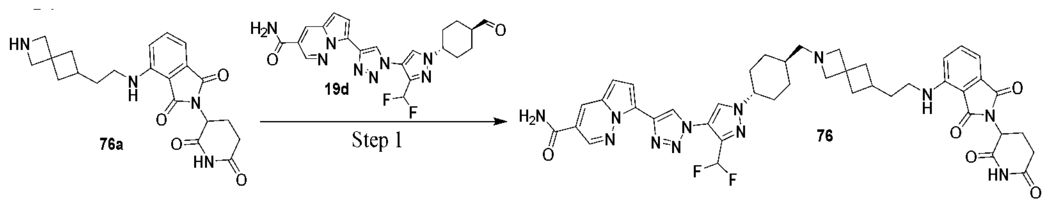


Compound 76a (40.0 mg, 0.100 mmol, prepared by the method disclosed for the intermediate ATH on page 95 of the specification of the patent application "WO 2022236339A1") was dissolved in N,N-dimethylformamide (0.5 mL) and tetrahydrofuran (2 mL), and triethylamine (0.03 mL, 0.222 mmol) was added. The reaction solution was reacted at 25°C for 10 min, then compound 19d (50.4 mg, 0.110 mmol) and acetic acid (0.03 mL, 0.504 mmol) were added in sequence. The reaction solution was stirred for 30 min, then sodium borohydride acetate (106 mg, 0.500 mmol) was added, and the reaction solution was stirred for 2 h. Saturated ammonium chloride aqueous solution (2 mL) was added to the reaction solution. The mixture was concentrated under reduced pressure and the obtained residue was purified by high performance liquid chromatography (Gilson_306_1741, chromatographic column: Waters-Xbridge-C18-10μm-19×250 mm; mobile phase: water (containing 10 mmol/L ammonium bicarbonate) and acetonitrile, gradient ratio: acetonitrile 35%-95%, flow rate: 25 mL/min) to obtain compound 76. MS m/z(ESI):418.4$[M/2+1]^+$. $^1H$ NMR (400 MHz, DMSO-$d_6$): δ 11.09 (s, 1H), 9.00 (d, $J$ = 4.7 Hz, 1H), 8.78 (d, $J$ = 2.0 Hz, 1H), 8.72 (s, 1H), 8.65 (d, $J$ = 2.1 Hz, 1H), 8.14 (s, 1H), 7.65 (d, $J$ = 4.6 Hz, 1H), 7.57 (d, $J$ = 8.2 Hz, 2H), 7.39 - 7.19 (m, 1H), 7.09 - 7.00 (m, 3H), 6.47 (t, $J$ = 5.4 Hz, 1H), 5.05 (dd, $J$ = 12.5, 5.1 Hz, 1H), 4.35 - 4.20 (m, 1H), 3.23 - 3.03 (m, 6H), 2.93 - 2.82 (m, 1H), 2.70 - 2.56 (m, 1H), 2.27 - 2.11 (m, 6H), 2.07 - 1.97 (m, 2H), 1.91 - 1.72 (m, 6H), 1.67 - 1.53 (m, 4H), 1.30 (s, 1H), 1.07 (d, $J$ = 11.8 Hz, 1H).

Example 77: 7-(1-(1-((3-(4-chloro-3-(2,4-dioxotetrahydropyrimidin-1(2H)-yl)benzoyl)-3-azospiro[5.5]undecan-9-yl) methyl)piperidin-4-yl)-3-(difluoromethyl)-1H-pyrazol-4-yl)-1H-1,2,3-triazol-4-yl)pyrrolo[1,2-b]pyridazine-3-carboxamide (77)

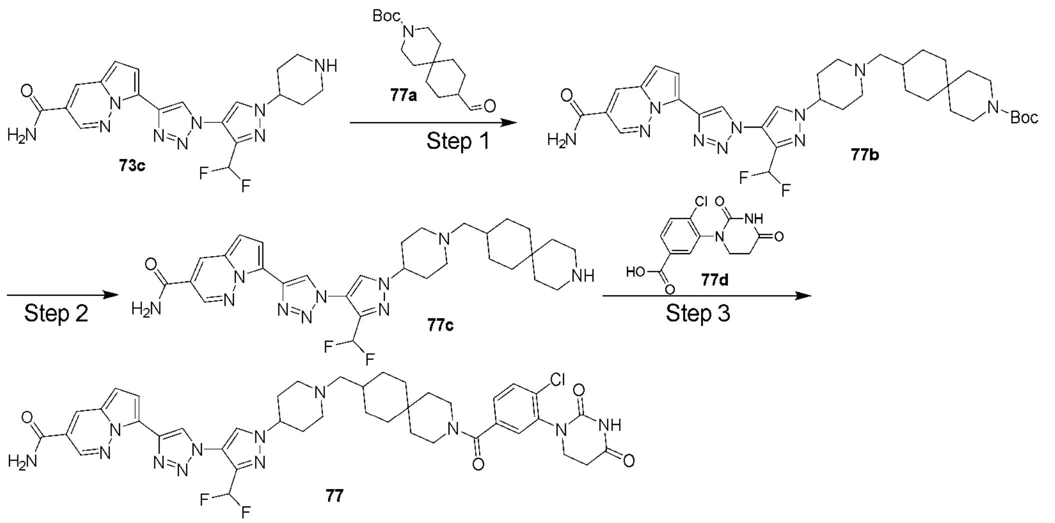


1) Step 1: Compound 73c (70.0 mg, 0.160 mmol) and compound 77a (67.5 mg, 0.240 mmol, Bide Pharmatech Ltd.) were dissolved in N,N-dimethylformamide (0.5 mL), and triethylamine (32.4 mg, 0.320 mmol) and acetic acid (30.1 mg, 0.160 mmol) were added. The reaction solution was heated to 40°C, and reacted under stirring at this temperature for 2 h. The reaction solution was cooled to 20°C, and then sodium borohydride acetate (69.1 mg, 0.330 mmol) was added. The reaction solution was reacted under stirring at 20°C for 2 h. Water (2 mL) was added to the reaction solution, and ethyl acetate (5 mL × 3) was used for extraction. The combined organic phase was dried over anhydrous sodium sulfate and filtered to remove the desiccant, and then the filtrate was concentrated under reduced pressure to obtain compound 77b. MS m/z(ESI):693.4$[M+1]^+$.

2) Step 2: Compound 77b (35.0 mg, 50.0 μmol) was dissolved in dichloromethane (0.5 mL), and trifluoroacetic acid (0.5 mL) was added. The reaction solution was reacted under stirring at 20°C for 1 h. The reaction solution was concentrated under reduced pressure to obtain compound 77c. MS m/z(ESI):593.3$[M+1]^+$.

3) Step 3: Compound 77c (30.0 mg, 50.0 μmol) and compound 77d (15.0 mg, 60.0 μmol, prepared by the method

disclosed for the intermediate 24 on page 217 of the specification of the patent application "WO 2021/055295 A1") were dissolved in N,N-dimethylformamide (0.5 mL), and N,N-diisopropylethylamine (26.2 mg, 200 μmol), 1-(3-dimethylaminopropyl)-3-ethylcarbodiimide hydrochloride (24.3 mg, 0.130 mmol) and 1-hydroxybenzotriazole (10.3 mg, 80.0 μmol) were added. The reaction solution was reacted under stirring at 20°C for 3 h. Water (5 mL) was added to the reaction solution, the aqueous phase was extracted with ethyl acetate (5 mL × 3) and the combined organic phase was concentrated under reduced pressure. The residue was purified by high performance liquid chromatography GILSON: GX-281, chromatographic column: Phenomenex Gemini NX 150×30 mm, 5 μm; mobile phase: water (containing 0.0500% aqueous ammonia) and acetonitrile, gradient ratio: acetonitrile 33%-73%, flow rate: 60 mL/min) to obtain compound 77. MS m/z(ESI):843.4[M+1]$^+$.

Example 78: 5-(3-((4-(3-(difluoromethyl)-4-(4-(5-morpholinopyrazolo[1,5-a]pyrimidin-3-yl)-1H-1,2,3-triazol-1-yl)-1H-pyrazol-1-yl(piperidin-1-yl)methyl)azetidin-1-yl)-2-(2,6-dioxopiperidin-3-yl)isoindoline-1,3-dione (78)

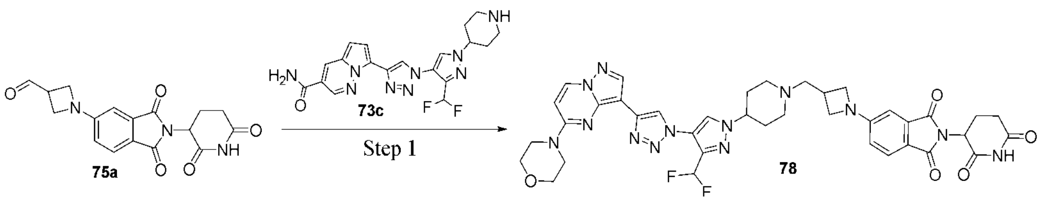


75a

78

Compound 73c (83.0 mg, 0.180 mmol) was dissolved in N,N-dimethylformamide (1 mL) and tetrahydrofuran (4 mL), triethylamine (0.02 mL, 0.18 mmol) was added, and the reaction solution was reacted under stirring at 25°C for 15 min. Then compound 75a (60.0 mg, 0.180 mmol, prepared by the method for the intermediate 36c on page 68 of the specification of the patent application WO 2022/253250 A1) and acetic acid (0.02 mL, 0.350 mmol) were added, and the reaction solution was reacted under stirring for 30 min. Then sodium cyanoborohydride (111 mg, 0.530 mmol) was added, and the reaction system was reacted under stirring for 30 min and then concentrated under reduced pressure. The residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 78. MS m/z(ESI):796.7[M+1]$^+$. $^1$H NMR (400 MHz, DMSO-$d_6$): δ 11.07 (s, 1H), 8.76 (d, $J$ = 7.9 Hz, 1H), 8.70 (s, 1H), 8.53 (s, 1H), 8.38 (s, 1H), 7.64 (d, $J$ = 8.2 Hz, 1H), 7.21 (t, $J$ = 53.2 Hz, 1H), 6.83 - 6.77 (m, 2H), 6.65 (dd, $J$ = 8.4, 2.1 Hz, 1H), 5.06 (dd, $J$ = 12.9, 5.4 Hz, 1H), 4.37 - 4.28 (m, 1H), 4.15 (t, $J$ = 8.1 Hz, 2H), 3.76 - 3.70 (m, 10H), 3.05 - 2.95 (m, 3H), 2.92 - 2.83 (m, 1H), 2.71 - 2.64 (m, 2H), 2.62 - 2.53 (m, 2H), 2.22 - 2.13 (m, 2H), 2.12 - 1.98 (m, 5H).

Example 79: 7-(1-(3-difluoromethyl)-1-(1R,4R)-4-(4-(1-(2,6-dioxopiperidin-3-yl)-3-methyl-2-oxo-2,3-dihydro-1H-benzoimidazol-4-yl)ethynyl)piperidin-1-yl)methyl)cyclohexyl)-1H-pyrazol-4-yl)-1H-1,2,3-triazol-4-yl)pyrrolo[1,2-b]pyridazine-3-carboxamide (79)

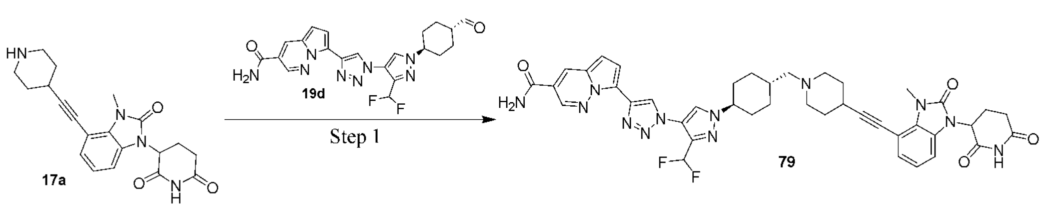


17a

79

Compound 17a (50.0 mg, 0.140 mmol, prepared by the method disclosed for the intermediate YL on page 858 of the specification of the patent application "WO 2020113233") and triethylamine (14.0 mg, 0.140 mmol) were dissolved in N,N-dimethylformamide (1 mL) and tetrahydrofuran (1 mL) and the reaction solution was stirred for 10 min. Acetic acid (10.0 mg, 0.170 mmol) and compound 19d (62.0 mg, 0.140 mmol) were added, and the reaction solution was stirred at 25°C for 0.5 h. Sodium borohydride acetate (86.0 mg, 0.410 mmol) was slowly added, and the reaction solution was stirred for 1 h. The solvent was removed under reduced pressure. High performance liquid chromatography was performed (Gilson _306_1741, chromatographic column: Waters-SunFire-C18-10μm-19×250 mm; mobile phase: water (containing 10 mmol/L ammonium bicarbonate) and acetonitrile, gradient ratio: acetonitrile 36%-64%, flow rate: 25 mL/min) to obtain

compound 79. MS m/z(ESI):805.7[M+1]$^+$. $^1$H NMR(400 MHz, DMSO-$d_6$) δ 11.13 (s, 1H), 9.01 (s, 1H), 8.79 - 8.78 (m, 1H), 8.75 (s, 1H), 8.66 - 8.65 (m, 1H), 7.65 (d, $J$ = 4.6 Hz, 1H), 7.58 (s, 1H), 7.38 - 7.24 (m, 1H), 7.16 - 7.08 (m, 1H), 7.05 (m, 2H), 7.00 (m, 2H), 5.40 - 5.37 (m, 1H), 4.29 - 4.30 (m, 1H), 3.65 (m, 3H), 2.87 (m, 1H), 2.68 (m, 4H), 2.20 - 2.09 (m, 6H), 1.98 - 1.88(m, 5H), 1.86 - 1.76 (m, 3H), 1.72 - 1.62 (m, 3H), 1.14 - 1.04 (m, 2H).

Example 80: 7-(1-(3-(difluoromethyl)-1-((1r,4r)-4-(7-(1-(2,6-dioxy-3-yl)-3-methyl-2-oxo-2,3-dihydro-1H-benzo[d]imidazol-4-yl)-2,7-diazaspiro[4.4]non-2-yl)methyl)-1H-pyrazol-4-yl)-1H-1,2,3-triazol-4-yl)pyrrolo[1,2-b]pyridazine-3-carboxamide (80)

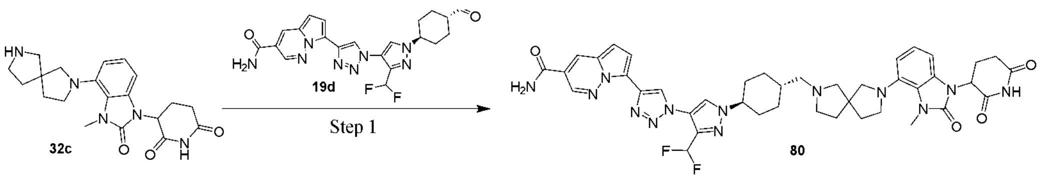


Compound 32c (80.0 mg, 0.220 mmol), compound 19d (107 mg, 0.220 mmol) and triethylamine (0.01 mL) were dissolved in tetrahydrofuran (3 mL) and N,N-dimethylformamide (1.5 mL). The reaction solution was stirred at 15°C for 10 min, and then acetic acid (0.01 mL) and sodium triacetoxyborohydride (136 mg, 0.650 mmol) were added. The reaction solution was reacted under stirring at 15°C for half an hour. Water (15 mL) was added to the reaction solution, and dichloromethane (15 mL × 3) was used for extraction. The combined organic phase was dried over anhydrous sodium sulfate and filtered to remove the desiccant, and then the filtrate was concentrated under reduced pressure. The obtained residue was purified by high performance liquid chromatography (Waters-2545, chromatographic column: C18 150×30 mm; mobile phase: Waters (FA)-$CH_3CN$, gradient ratio: acetonitrile 16%-56%, flow rate: 30 mL/min) to obtain compound 80. MS m/z(ESI):822.3[M+1]$^+$. $^1$H NMR (400 MHz, DMSO-$d_6$): δ 11.10 (s, 1H), 9.00 (s, 1H), 8.78 (d, $J$ = 2.1 Hz, 1H), 8.76 (s, 1H), 8.66 (d, $J$ = 2.1 Hz, 1H), 8.16 (s, 1H), 7.65 (d, $J$ = 4.6 Hz, 1H), 7.57 (s, 1H), 7.24 (t, $J$ = 53.6 Hz, 1H), 7.05 (d, $J$ = 4.6 Hz, 1H), 7.03 - 6.95 (m, 2H), 6.90 (d, $J$ = 6.6 Hz, 1H), 5.42 - 5.30 (m, 1H), 4.39 - 4.27 (m, 1H), 3.78 - 3.68 (m, 2H), 3.61 (s, 3H), 3.23 - 3.00 (m, 8H), 2.95 - 2.83 (m, 1H), 2.76 - 2.57 (m, 3H), 2.26 - 2.10 (m, 5H), 2.09 - 1.96 (m, 5H), 1.92 - 1.84 (m, 2H), 1.30 - 1.21 (m, 2H).

Example 81: 7-(1-(3-(difluoromethyl)-1-((1R,4R)-4-(5-(1-(2,6-dioxypyridin-3-yl)-3-methyl-2-oxo-2,3-dihydro-1H-benzo [d]imidazol-4-yl)hexahydropyrrolo[3,4-c]pyrrol-2(1H)-yl)methyl-1H-pyrazol-4-yl)-1H-1,2,3-triazol-4-yl)pyrrolo[1,2-b]pyridazine-3-carboxamide (81)

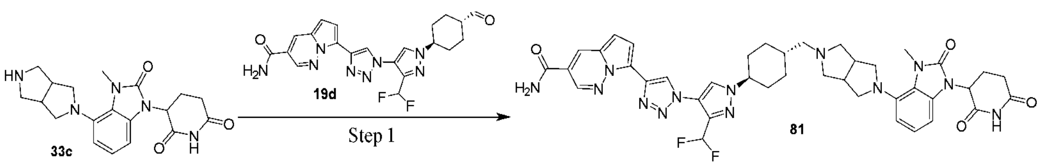


Compound 33c (58.0 mg, 0.160 mmol), compound 19d (71.4 mg, 0.160 mmol) and triethylamine (31.8 mg, 0.310 mmol) were dissolved in N,N-dimethylformamide (1.00 mL), and sodium cyanoborohydride (14.8 mg, 0.240 mmol) was added. The obtained mixture was reacted at room temperature for 2 h. The reaction solution was concentrated under reduced pressure and the obtained residue was purified by high performance liquid chromatography (Gilson GX-281, chromatographic column: Phenomenex Gemini NX C18, 30×150 mm, 5 μm; mobile phase: water (containing 0.0500% aqueous ammonia) and acetonitrile, gradient ratio: acetonitrile 38%-78%, flow rate: 60 mL/min) to obtain compound 81. MS m/z(ESI):808.5[M+1]$^+$. $^1$H NMR (400 MHz, DMSO-$d_6$): δ 11.20 - 11.01 (m, 1H), 9.01 (s, 1H), 8.79 (d, $J$ = 1.9 Hz, 1H), 8.75 (s, 1H), 8.66 (d, $J$ = 1.9 Hz, 1H), 8.20 - 8.13 (m, 1H), 7.66 (d, $J$ = 4.6 Hz, 1H), 7.58 (s, 1H), 7.24 (t, $J$ = 53.3 Hz, 1H), 7.05 (d, $J$ = 4.6 Hz, 1H), 6.98 - 6.91 (m, 2H), 6.87 (d, $J$ = 7.3 Hz, 1H), 5.35 (m, $J$ = 4.9,12.4 Hz, 1H), 4.37 - 4.21 (m, 1H), 3.66 (s, 3H), 3.17 (s, 2H), 2.91 - 2.77 (m, 6H), 2.76 - 2.56 (m, 4H), 2.30 - 2.23 (m, 2H), 2.17 (d, $J$ = 10.3 Hz, 2H), 2.07 - 1.91 (m, 4H), 1.86 - 1.77 (m, 2H), 1.59 - 1.51 (m, 1H), 1.17 - 1.07 (m, 2H).

Example 82: 7-(1-(3-(difluoromethyl)-1-((1r,4r)-4-(4-(3-(1-(2,6-dioxypiperidin-3-yl)-3-methyl-2-oxo-2,3-dihydro-1H-benzo[d]imidazol-4-yl)azabutan-1-yl)piperidin-1-methyl)cyclohexyl-1H-pyrazol-4-yl)-1H-1,2,3-triazol-4-yl)pyrrolo[1,2-b]pyridazine-3-carboxamide (82)

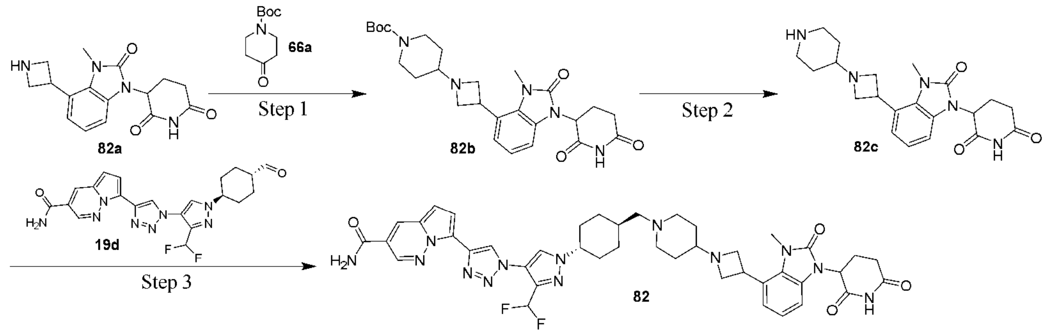


1) Step 1: Compound 82a (50.0 mg, 0.160 mmol, prepared by the method disclosed for the intermediate 001945 on page 748 of the specification of the patent application "WO 2020/264499 A1") and compound 66a (47.5 mg, 0.240 mmol, https://www.labnetwork.com.cn/) were dissolved in N,N-dimethylformamide (0.5 mL), and triethylamine (32.2 mg, 0.320 mmol) and acetic acid (29.7 mg, 0.160 mmol) were added. The reaction solution was heated to 40°C, and reacted under stirring at this temperature for 2 h, and then sodium borohydride acetate (50.3 mg, 0.240 mmol) was added. The reaction solution was reacted under stirring at 20°C for 2 h. The reaction solution was concentrated under reduced pressure and then purified by reversed-phase chromatography with an eluent system (water/acetonitrile = 25/75 to 80/20) to obtain compound 82b. MS m/z(ESI):498.2$[M+1]^+$.

2) Step 2: Compound 82b (15.0 mg, 30.0 μmol) was dissolved in dichloromethane (0.5 mL), and trifluoroacetic acid (0.5 mL) was added. The reaction solution was reacted under stirring at 20°C for 1 h. The reaction solution was concentrated under reduced pressure to obtain compound 82c. MS m/z(ESI):398.3$[M+1]^+$.

3) Step 3: Compound 82c (15.0 mg, 40.0 μmol) and compound 19d (17.2 mg, 40.0 μmol) were dissolved in N,N-dimethylformamide (0.5 mL), and triethylamine (7.64 mg, 80.0 μmol) and acetic acid (7.04 mg, 40.0 μmol) were added. The reaction solution was heated to 40°C, and reacted under stirring at this temperature for 2 h. Sodium borohydride acetate (15.9 mg, 80.0 μmol) was added and the obtained mixture was reacted under stirring for 2 h. The reaction solution was concentrated under reduced pressure and the obtained residue was purified by high performance liquid chromatography (GILSON: GX-281, chromatographic column: C18 150×30 mm; mobile phase: Water (containing 0.0750% trifluoroacetic acid) and acetonitrile, gradient ratio: acetonitrile 13%-53%, flow rate: 30 mL/min) to obtain compound 82. MS m/z(ESI):836.4$[M+1]^+$.

Example 83: 7-(1-(3-(difluoromethyl)-1-(1R,4R)-4-(3-(1-(2,6-dioxopiperidin-3-yl)-3-methyl-2-oxo-2,3-dihydro-1H-benzo [d]imidazol-4-yl)-[1,3'-azetidine]-1'-yl)methyl)-1H-pyrazol-4-yl)-1H-1,2,3-triazol-4-yl)pyrrolo[1,2-b]pyridazine-3-carboxamide (83)

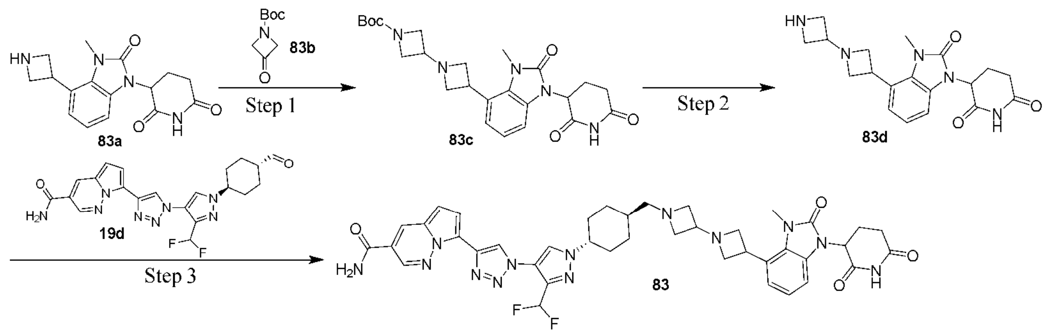


1) Step 1: Compound 83a (50.0 mg, 0.160 mmol, prepared by the method disclosed for the intermediate 001945 on page 748 of the specification of the patent application "WO2020/264499A1") and compound 83b (30.0 mg, 0.170

mmol, Bide Pharmatech Ltd.) were dissolved in N,N-dimethylformamide (0.5 mL), and triethylamine (32.2 mg, 0.320 mmol) and acetic acid (29.7 mg, 0.160 mmol) were added. The reaction solution was reacted under stirring for 2 h, and then sodium borohydride acetate (50.3 mg, 0.240 mmol) was added, and the obtained mixture was reacted under stirring for 2 h. The reaction solution was concentrated under reduced pressure and then the residue was purified by reversed-phase chromatography with an eluent system (water/acetonitrile = 25/75 to 80/20) to obtain compound 83c. MS m/z(ESI):414.2[M-55]$^+$.

2) Step 2: Compound 83c (9.00 mg, 20.0 μmol) was dissolved in dichloromethane (0.5 mL), and trifluoroacetic acid (0.1 mL) was added. The reaction solution was reacted at 20°C for 1 h. The reaction solution was concentrated under reduced pressure to obtain compound 83d.

3) Step 3: Compound 83d (7.00 mg, 20.0 μmol), compound 19d (8.61 mg, 20.0 μmol), triethylamine (3.83 mg, 40.0 μmol) and acetic acid (1.14 mg, 20.0 μmol) were dissolved in N,N-dimethylformamide (0.5 mL), and sodium triacetoxyborohydride (4.00 mg, 20.0 μmol) was added. The reaction solution was reacted at 20°C for 1 h. The reaction solution was concentrated under reduced pressure and then the obtained residue was purified by high performance liquid chromatography (Gilson GX-281, chromatographic column: Boston Prime C18, 30×150 mm, 5 μm; mobile phase: water (containing 0.05% aqueous ammonia and 10.0 mmol/L ammonium bicarbonate) and acetonitrile, gradient ratio: acetonitrile 27%-57%, flow rate: 25 mL/min) to obtain compound 83. MS m/z(ESI):808.5 [M+1]$^+$.

Example 84: 7-(1-(3-(difluoromethyl)-1-((1r,4r)-4-((4-(1-(2,6-dioxopiperidin-3-yl)-3-methyl-2-oxo-2,3-dihydro-1H-benzo [d]imidazol-4-yl)piperidin-1-yl)methyl)cyclohexyl)-1H-pyrazol-4-yl)-1H-1,2,3-triazol-4-yl)pyrrolo[1,2-b]pyridazine-3-carboxamide (84)

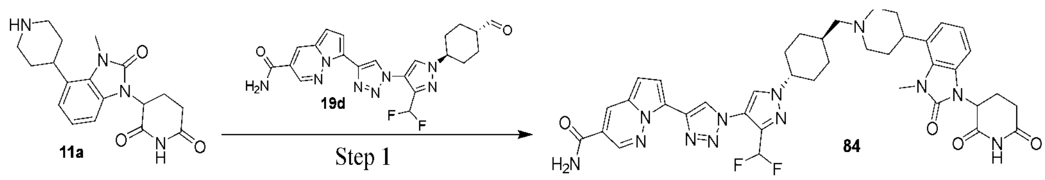


Compound 11a (50.0 mg, 0.150 mmol, prepared by the method disclosed for the intermediate AZK on page 222 of the specification of the patent application "WO 2021158634 A1") and compound 19d (75.0 mg, 0.170 mmol) were dissolved in tetrahydrofuran (3 mL) and N,N-dimethylformamide (1 mL), and acetic acid (0.2 mL) and sodium borohydride acetate (80.0 mg, 0.380 mmol) were added in sequence. The reaction solution was stirred at 25°C for 3 h. The reaction solution was poured into water (10 mL), and ethyl acetate (10 mL × 3) was used for extraction. The combined organic phase was washed with saturated brine (20 mL), dried over anhydrous sodium sulfate and filtered to remove the desiccant, and then the filtrate was concentrated under reduced pressure. The obtained residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 32%-70%, flow rate: 25 mL/min) to obtain compound 84. MS m/z(ESI):781.8[M+1]. $^1$H NMR (400 MHz, DMSO-$d_6$): δ 11.09 (s, 1H), 9.01 (s, 1H), 8.79 (d, $J$ = 2.1 Hz, 1H), 8.75 (s, 1H), 8.66 (d, $J$ = 2.2 Hz, 1H), 8.14 (s, 1H), 7.65 (d, $J$ = 4.6 Hz, 1H), 7.56 (s, 1H), 7.23 (t, $J$ = 53.3 Hz, 1H), 7.07 - 6.95 (m, 4H), 5.45 - 5.30 (m, 1H), 4.38 - 4.23 (m, 1H), 3.59 (s, 3H), 3.28 - 3.21 (m, 1H), 3.07 - 2.83 (m, 3H), 2.72 - 2.62 (m, 2H), 2.27 - 2.15 (m, 4H), 2.11 - 1.95 (m, 5H), 1.89 - 1.75 (m, 6H), 1.70 - 1.61 (m, 1H), 1.20 - 1.08 (m, 2H).

Example 85: 7-(1-(3-(difluoromethyl)-1-((1r,4r)-4-((9-(1-(2,6-dioxopiperidin-3-yl)-3-methyl-2-oxo-2,3-dihydro-1H-benzo [d]imidazol-4-yl)-3,9-diazaspiro[5.5]undecan-3-yl)methyl)cyclohexyl)-1H-pyrazol-4-yl)-1H-1,2,3-triazol-4-yl)pyrrolo[1,2-b]pyridazine-3-carboxamide (85)

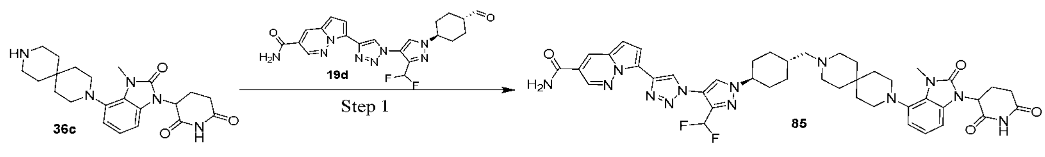


Compound 36c (170 mg, 0.411 mmol) was dissolved in 1,2-dichloroethane (4 mL) and tetrahydrofuran (4 mL),

and triethylamine (50.2 mg, 0.511 mmol) was added until the pH value was greater than 7, and the reaction solution was stirred at 25°C for 10 min. The reaction solution was cooled to -10°C, acetic acid (115 mg, 0.621 mmol) and compound 19d (262 mg, 0.581 mmol) were added. The reaction solution was warmed to 25°C, and stirred at this temperature for 20 min, and then sodium borohydride acetate (175 mg, 0.831 mmol, Bide Pharmatech Ltd.) was added. The reaction solution was stirred for 1 h and then concentrated under reduced pressure. The residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 85. MS m/z(ESI):850.4$[M+1]^+$. $^1H$ NMR (400 MHz, DMSO-$d_6$):δ 11.10 (s, 1H), 9.01 (s, 1H), 8.79 (d, 1H), 8.74 (s, 1H), 8.67 (d, 1H), 8.23 - 8.11 (m, 1H), 7.66 (d, *J* = 4.6 Hz, 1H), 7.57 (s, 1H), 7.17 (t, *J* = 53.4 Hz, 1H), 7.06 (d, *J* = 4.6 Hz, 1H), 7.00 - 6.94 (m, 2H), 6.91 - 6.82 (m, 1H), 5.42 - 5.27 (m, 1H), 4.37 - 4.22 (m, 1H), 3.64 (s, 4H), 2.88 (s, 5H), 2.72 - 2.59 (m, 2H), 2.38 (s, 4H), 2.24 - 2.11 (m, 4H), 2.02 - 1.90 (m, 3H), 1.87 - 1.77 (m, 2H), 1.73 - 1.62 (m, 4H), 1.56 - 1.41 (m, 4H), 1.15 - 1.04 (m, 2H).

Example 86: 7-(1-(3-(difluoromethyl)-1-((1R,4R)-4-(3-(1-(2,6-dioxy-3-yl)-3-methyl-2-oxo-2,3-dihydro-1H-benzo[d]imidazol-4-yl)-3,8-diazabicyclo[3.2.1]oct-8-yl)methyl)cyclohexyl-1H-pyrazol-4-yl)-1H-1,2,3-triazol-4-yl)pyrrolo[1,2-b]pyridazine-3-carboxamide (86)

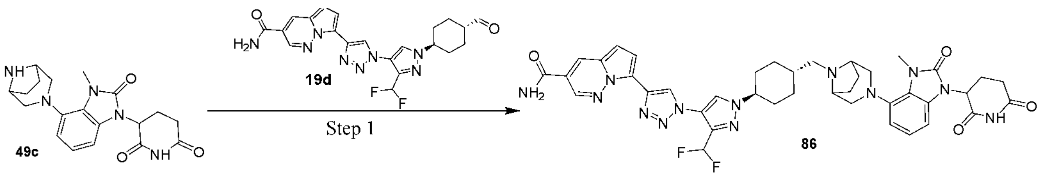


Compound 49c (50.0 mg, 140 μmol), compound 19d (61.5 mg, 140 μmol) and triethylamine (27.4 mg, 270 μmol) were dissolved in N,N-dimethylformamide (1.00 mL), and sodium cyanoborohydride (12.8 mg, 200 μmol) was added. The reaction solution was reacted at 20°C for 1 h. The reaction solution was concentrated under reduced pressure and then the residue was purified by high performance liquid chromatography (Gilson GX-281, chromatographic column: Boston Prime C18, 30×150 mm, 5 μm; mobile phase: water (containing 0.05% aqueous ammonia and 10.0 mmol/L ammonium bicarbonate) and acetonitrile, gradient ratio: acetonitrile 47%-77%, flow rate: 25 mL/min) to obtain compound 86. MS m/z(ESI):808.4$[M+1]^+$.

Example 87: 7-(1-(3-(difluoromethyl)-1-((1r,4r)-4-(3-(1-(2,6-dioxypiperidin-3-yl)-3-methyl-2-oxo-2,3-dihydro-1H-benzo[d]imidazol-4-yl)ethyl)azabutan-1-yl)methyl)cyclohexyl)-1H-pyrazol-4-yl)- 1H -1,2,3-triazol-4-yl)pyrrolo[1,2-b]pyridazine-3-carboxamide (87)

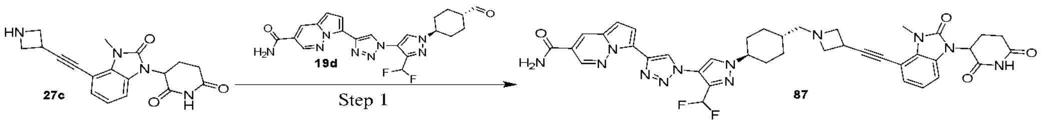


Compound 19d (48.4 mg, 0.107 mmol), compound 27c (36.0 mg, 0.106 mmol) and triethylamine (21.5 mg, 0.213 mmol) were dissolved in N,N-dimethylformamide (1 mL), and the reaction solution was heated to 40°C and stirred at this temperature for 2 h. Sodium cyanoborohydride(13.8 mg, 0.220 mmol) was added to the reaction solution and the obtained mixture was stirred at 40°C for 2 h. The reaction solution was cooled to 25°C and filtered, and then the residue was purified by high performance liquid chromatography (ACSSH-CH, chromatographic column: Phenomenex Gemini NX 150×30 mm, 5 μm; mobile phase: water (aqueous ammonia v/v)-ACN, gradient ratio: acetonitrile 33%-73%, flow rate: 66 mL/min) to obtain compound 87. MS m/z(ESI):777.3$[M+1]^+$.

Example 88: 7-(1-(2,6-dioxopiperidin-3-yl)-3-methyl-2-oxo-2,3-dihydro-1H-oxaimidazol-4-yl)-1-oxa-4,9-diazaspiro[5.5]undecan-9-yl)methyl)cyclohexyl)-1H-pyrazol-4-yl)-1H-pyrazol-4-yl)-1H-1,2,3-triazol-4-yl)pyrrolo[1,2-b]pyridazine-3-carboxamide (88)

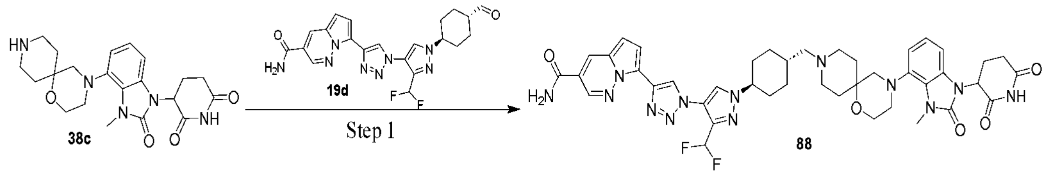


Compound 38c (100 mg, 0.240 mmol) and triethylamine (25.0 mg, 0.250 mmol) were dissolved in N,N-dimethylformamide (2 mL) and tetrahydrofuran (2 mL) and stirred for 10 min. Acetic acid (19.0 mg, 0.320 mmol) and compound 19d (132 mg, 0.290 mmol) were added, and the reaction solution was stirred at 25°C for 0.5 h. Sodium borohydride acetate (153 mg, 0.730 mmol) was slowly added, and the reaction solution was stirred for 1 h. The solvent was removed by concentration under reduced pressure. The residue was purified by high performance liquid chromatography (Gilson_306_1741, chromatographic column: Waters-SunFire-C18-10μm-19×250 mm; mobile phase: water (containing 10 mmol/L formic acid) and acetonitrile, gradient ratio: acetonitrile 8%-92%, flow rate: 25 mL/min) to obtain compound 88. MS m/z(ESI):852.9[M+1]$^+$. $^1$H NMR(400 MHz, DMSO-$d_6$): δ11.09 (s, 1H), 9.00 (s, 1H), 8.78 (d, $J$ = 2.3 Hz, 1H), 8.73(s, 1H), 8.65 (d, $J$ = 2.2 Hz, 1H), 8.18 (s, 1H), 8.15(s, 1H), 7.65 (d, $J$ = 4.6 Hz, 1H), 7.60 - 7.49 (m, 1H), 7.23 (t, $J$ = 53.3 Hz, 1H), 7.05 (d, $J$ = 4.6 Hz, 1H), 7.04 - 6.96 (m, 2H), 6.95 - 6.91 (m, 1H), 5.36 (dd, $J$ = 12.7, 5.4 Hz, 1H), 4.33 - 4.24(m, 1H), 3.96(s, 1H), 3.65(s, 3H), 3.03 - 2.97(m, 1H), 2.95 - 2.74(m, 4H), 2.73 - 2.61(m, 3H), 2.45 - 2.22 (m, 4H), 2.22 - 2.06 (m, 5H), 2.02 - 1.90 (m, 3H), 1.86 - 1.76 (m, 2H), 1.75 - 1.50 (m, 4H), 1.20 - 1.00 (m, 2H).

Example 89: 7-(1-(3-(difluoromethyl)-1-((1r,4r)-4-((9-(1-(2,6-dioxopiperidin-3-yl)-3-methyl-2-oxo-2,3-dihydro-1H-benzo[d]imidazol-4-yl)-1-oxa-4,9-diazaspiro[5.5]undecan-4-yl)methyl)cyclohexyl)-1H-pyrazol-4-yl)-1H-1,2,3-triazol-4-yl)pyrrolo[1,2-b]pyridazine-3-carboxamide (89)

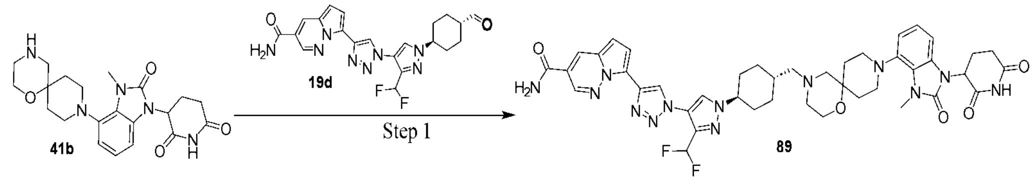


Compound 41b (62.0 mg, 0.110 mmol) was dissolved in N,N-dimethylformamide (3 mL) and tetrahydrofuran (2 mL), triethylamine (21.0 mg, 0.210 mmol) was added and the reaction solution was stirred at 25°C for 10 min. Compound 19d (50.0 mg, 0.110 mmol) and acetic acid (32.0 mg, 0.530 mmol) were added, and the obtained mixture was stirred for 30 min. Then sodium borohydride acetate (115 mg, 0.540 mmol) was added, and the reaction solution was stirred for 1 h. The reaction solution was concentrated, and then the obtained residue was purified by high performance liquid chromatography (Gilson_306_1741, chromatographic column: Waters-SunFire-C18-10μm-19×250 mm; mobile phase: water (containing 10 mmol/L ammonium bicarbonate) and acetonitrile, gradient ratio: acetonitrile 45%-95%, flow rate: 25 mL/min) to obtain compound 89. MS m/z(ESI):426.8[M/2+1]$^+$. $^1$H NMR(400MHz, DMSO-$d_6$): δ 11.07 (s, 1H), 9.03 - 9.00 (m, 1H), 8.83 - 8.71 (m, 2H), 8.65 (d, $J$ = 2.0 Hz, 1H), 8.15 (s, 1H), 7.65 (d, $J$ = 4.6 Hz, 1H), 7.55 (s, 1H), 7.23 (t, $J$ = 53.2 Hz, 1H), 7.05 (d, $J$ = 4.8 Hz, 1H), 7.02 - 6.82 (m, 3H), 5.45 - 5.25 (m, 1H), 4.44 - 4.20 (m, 1H), 3.74 - 3.58 (m, 5H), 3.07 - 2.79 (m, 5H), 2.77 - 2.57 (m, 2H), 2.36 - 2.04 (m, 10H), 2.03 - 1.79 (m, 5H), 1.71 - 1.55 (m, 3H), 1.15 - 1.05 (m, 2H).

Example 90: 2-(((1r,4r)-4-(4-(4-(3-carbamoylpyrrolo[1,2-b]pyridazin-7-yl)-1H-1,2,3-triazol-1-yl) -3-(difluoromethyl)-1H-pyrazol-1-yl)cyclohexyl)methyl)-N-(1-(2,6-dioxopiperidin-3-yl)-3-methyl-2-oxo-2,3-dihydro-1H-benzo[d]imidazol-4-yl)-2,8-diazaspiro[4.5]decan-8-carboxamide (90)

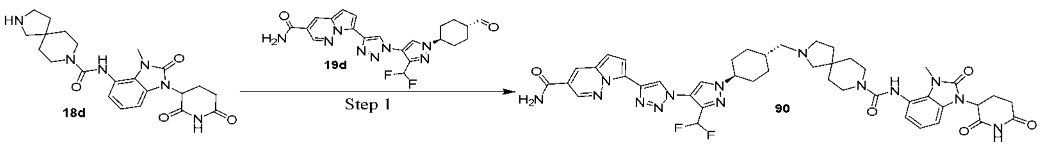


Compound 18d (100 mg, 0.230 mmol) was dissolved in tetrahydrofuran (1 mL) and N, N-dimethylformamide (1 mL), and triethylamine (0.03 mL, 0.230 mmol), acetic acid (0.03 mL, 0.450 mmol), sodium triacetoxyborohydride (123 mg,

0.270 mmol), and compound 19d (123 mg, 0.270 mmol) were added, and the obtained mixture was reacted under stirring for 2 h. Then water (5 mL) was added to the reaction solution, and ethyl acetate (5 mL × 3) was used for extraction. The combined organic phase was dried over anhydrous sodium sulfate and filtered to remove the desiccant, and then the filtrate was concentrated under reduced pressure. The residue was purified by high performance liquid chromatography (Waters-2545, chromatographic column: SharpSil-TC18, 30×150 mm, 5 μm; mobile phase: water (containing 10 mmol/L ammonium bicarbonate) and acetonitrile, gradient ratio: acetonitrile 38%-45%, flow rate: 30 mL/min) to obtain compound 90. MS m/z(ESI):879.8[M+1]$^+$. $^1$H NMR (400 MHz, DMSO-$d_6$): δ 11.11 (s, 1H), 9.04 - 8.99 (m, 1H), 8.81 - 8.77 (m, 1H), 8.75 (s, 1H), 8.66 (d, $J$ = 2.3 Hz, 1H), 8.35 (s, 1H), 8.19 - 8.14 (m, 2H), 7.66 (d, $J$ = 4.6 Hz, 1H), 7.57 (s, 1H), 7.38 - 7.11 (m, 1H), 7.05 (d, $J$ = 4.6 Hz, 1H), 6.99 - 6.94 (m, 2H), 6.80 - 6.75 (m, 1H), 5.42 - 5.34 (m, 1H), 4.37 - 4.23 (m, 1H), 3.52 - 3.46 (m, 3H), 2.94 - 2.85 (m, 1H), 2.77 - 2.69 (m, 1H), 2.67 - 2.55 (m, 4H), 2.43 - 2.40 (m, 2H), 2.35 - 2.26 (m, 2H), 2.22 - 2.13 (m, 2H), 2.04 - 1.95 (m, 3H), 1.89 - 1.79 (m, 2H), 1.70 - 1.59 (m, 3H), 1.57 - 1.46 (m, 5H), 1.22 - 1.02 (m, 2H).

Example 91: 7-(1-(3-(difluoromethyl)-1-((1r,4r)-4-((4-(1-(1-(2,6-dioxopiperidin-3-yl)-3-methyl-2-oxo-2,3-dihydro-1H-benzo[d]imidazol-4-yl)piperidin-4-yl)piperazin-1-yl)methyl)cyclohexyl)-1H-pyrazol-4-yl)-1H-1,2,3-triazol-4-yl)pyrrolo[1,2-b]pyridazine-3-carboxamide (91)

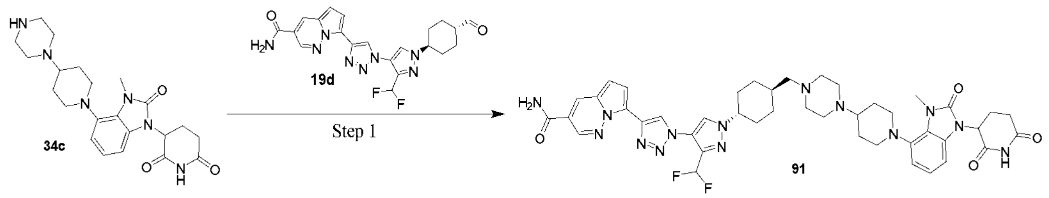


Compound 34c (60.0 mg, 0.140 mmol), compound 19d (64.0 mg, 0.140 mmol) and triethylamine (0.1 mL) were dissolved in tetrahydrofuran (2 mL) and N,N-dimethylformamide (1 mL), and the reaction solution was stirred at 15°C for 10 min. Acetic acid (0.1 mL) and sodium triacetoxyborohydride (89.0 mg, 0.42 mmol) were added, and the reaction solution was reacted under stirring at 15°C for half an hour. Water (8 mL) was added to the reaction solution, and dichloromethane (10 mL × 3) was used for extraction. The combined organic phase was dried over anhydrous sodium sulfate and filtered to remove the desiccant, and then the filtrate was concentrated under reduced pressure. The residue was purified by high performance liquid chromatography (Waters-2545, chromatographic column: C18 150×30 mm; mobile phase: Waters (FA)-$CH_3CN$, gradient ratio: acetonitrile 11%-51%, flow rate: 30 mL/min) to obtain compound 91. MS m/z(ESI):865.4 [M+1]$^+$.

Example 92: 7-(1-(3-(difluoromethyl)-1-((1r,4r)-4-(8-(1-(2,6-dioxopiperidin-3-yl)-3-methyl-2-oxo-2,3-dihydro-1H-benzo[d]imidazol-4-yl)-3,8-diazabicyclo[3.2.1]oct-3-yl)methyl)cyclohexyl-1H-pyrazol-4-yl)-1H-1,2,3-triazol-4-yl)pyrrolo[1,2-b]pyridazine-3-carboxamide (92)

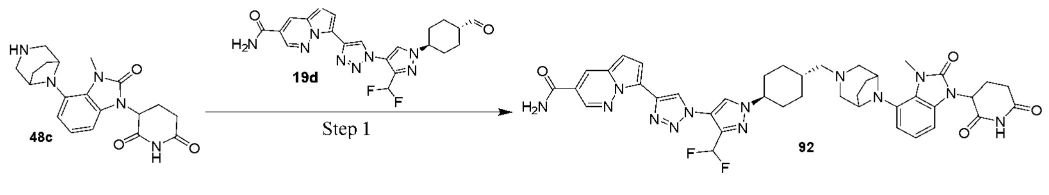


Compound 48c (44.7 mg, 0.120 mmol) and compound 19d (50.0 mg, 0.110 mmol) were dissolved in N,N-dimethylformamide (0.5 mL), triethylamine (22.3 mg, 0.220 mmol) and sodium cyanoborohydride (10.4 mg, 0.170 mmol) were added, and the reaction solution was reacted under stirring at 20°C for 16 h. The reaction solution was filtered, and the filtrate was purified by high performance liquid chromatography (GILSON: GX-281, chromatographic column: Phenomenex Gemini NX 150×30 mm; mobile phase: water (containing 0.0500% aqueous ammonia) and acetonitrile, gradient ratio: acetonitrile 41%-81%, flow rate: 60 mL/min) to obtain compound 92. MS m/z(ESI):808.3[M+1]$^+$.

Example 93: 7-(1-(3-(difluoro)-1-(1-(2-(4-(2-(2,6-dioxopiperidin-3-yl)-1,3-dioxoisoindolin-5-yl)piperazin-4-yl)oxy)piperidin-4-yl)-1H-pyrazol-4-yl)-1H-1,2,3-triazol-4-yl)pyrrolo[1,2-b]pyridazine-3-carboxamide (93)

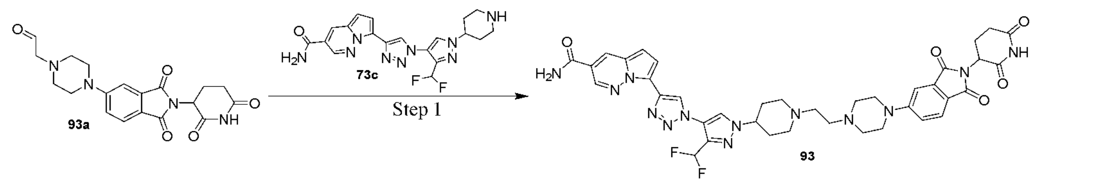


Compound 73c (30.0 mg, 70.0 μmol) and compound 93a (29.6 mg, 80.0 μmol, prepared by the method disclosed for the intermediate 2 on page 300 of the specification of the patent application "WO 2021/207291 A1") were dissolved in dimethyl sulfoxide (0.5 mL) and methanol (0.5 mL), and triethylamine (14.2 mg, 140 μmol), acetic acid (13.1 mg, 70.0 μmol) and sodium cyanoborohydride (8.80 mg, 70.0 μmol) were added, and the reaction solution was stirred at 20°C for 2 h. The reaction solution was concentrated under reduced pressure and then the residue was purified by high performance liquid chromatography (GILSON GX-281, chromatographic column: Phenomenex Gemini NX 150×30 mm, 5 μm; mobile phase: water (containing 0.05% aqueous ammonia) and acetonitrile, gradient ratio: acetonitrile 25%-65%, flow rate: 60 mL/min) to obtain compound 93. MS m/z(ESI):796.4[M+1]$^+$.

Example 94: 7-(1-(3-(difluoromethyl)-1-(1-((1-(2-(2,6-dioxopiperidin-3-yl)-1,3-dioxoisoindolin-5-yl) piperidin-4-yl) methyl)piperidin-4-yl)-1H-pyrazol-4-yl)-1H-1,2,3-triazol-4-yl)pyrrolo[1,2-b]pyridazine-3-carboxamide (94)

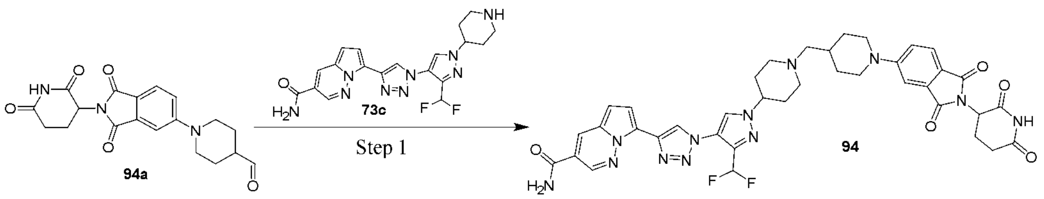


Compound 73c (40.0 mg, 90.0 μmol) and compound 94a (86.4 mg, 0.230 mmol, prepared by the method disclosed for the intermediate 330 on page 137 of the specification of the patent application "WO2021/113557A1") were dissolved in N,N-dimethylformamide (0.5 mL), and triethylamine (28.4 mg, 0.280 mmol) and acetic acid (17.5 mg, 90.0 μmol) were added, and the reaction solution was heated to 40°C and reacted under stirring at this temperature for 2 h. The reaction solution was cooled to 20°C, sodium triacetoxyborohydride (39.5 mg, 0.190 mmol) was added, and the obtained mixture was reacted under stirring for 2 h. Water (10 mL) was added to the reaction solution, and ethyl acetate (10 mL × 3) was used for extraction. The combined organic phase was concentrated under reduced pressure and then purified by high performance liquid chromatography (Gilson GX-281, chromatographic column: Boston Prime C18, 30×150 mm, 5 μm; mobile phase: water (containing 0.0500% aqueous ammonia and 10.0 mmol/L ammonium bicarbonate) and acetonitrile, gradient ratio: acetonitrile 45%-65%, flow rate: 35 mL/min) to obtain compound 94. MS m/z(ESI):781.3[M+1]$^+$. $^1$H NMR (400 MHz, DMSO-$d_6$): δ 11.09 (s, 1H), 9.02 (s, 1H), 8.83 - 8.74 (m, 2H), 8.66 (d, *J* = 2.2 Hz, 1H), 8.17 (s, 1H), 7.66 (m, *J* = 2.0, 6.6 Hz, 2H), 7.58 (s, 1H), 7.40 - 7.31 (m, 1H), 7.27 - 7.23 (m, 1H), 7.13 - 7.04 (m, 1H), 5.09 - 5.03 (m, 1H), 4.40 - 4.27 (m, 1H), 4.07 (d, *J* = 12.2 Hz, 2H), 3.39 - 3.37 (m, 1H), 3.06 - 2.81 (m, 5H), 2.64 - 2.53 (m, 2H), 2.22 (d, *J* = 6.7 Hz, 2H), 2.16 - 1.97 (m, 7H), 1.83 (d, *J* = 11 Hz, 3H), 1.26 - 1.10 (m, 2H).

Example 95: 7-{1-[3-(difluoromethyl)-1-{1-[(1-{4-[(2,6-dioxopiperidin-3-yl)aminocarbonyl]-3-fluorophenyl}piperidin-4-yl) methyl]piperidin-4-yl}-1H-pyrazol-4-yl]-1H-1,2,3-triazol-4-yl}pyrrolo[1,2-b]pyridazine-3-carboxamide (95)

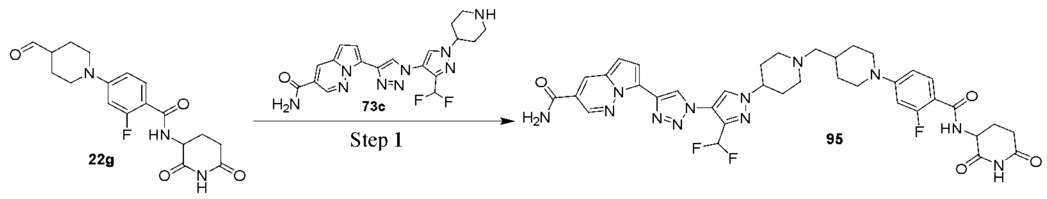


Compound 22g (37.0 mg, 0.100 mmol) was dissolved in N,N-dimethylformamide (0.5 mL), and compound 73c (43.8 mg, 0.100 mmol) and a drop of glacial acetic acid were added. The reaction solution was stirred at 25°C for 1 h, and then sodium triacetylborohydride (43.2 mg, 0.200 mmol) was added. The reaction solution was stirred at 25°C for 1 h. The reaction solution was concentrated under reduced pressure and the residue was purified by high performance liquid chromatography (ACSSH-CH, chromatographic column: Phenomenex Gemini NX 150×30 mm, 5 μm; mobile phase: water (0.1% formic acid) and acetonitrile, gradient ratio: acetonitrile 10%-50%, flow rate: 60 mL/min) to obtain compound 95. MS m/z(ESI):773.5[M+1]$^+$. $^1$H NMR (400 MHz, DMSO-$d_6$) δ 10.86 (s, 1H), 9.02 (s, 1H), 8.79(d, *J* = 2.4 Hz, 2H), 8.66 (d, *J* = 2.2 Hz, 1H), 8.22 - 8.15 (m, 1H), 8.01 (t, *J* = 7.2 Hz, 1H), 7.69 - 7.51 (m, 3H), 7.25 (t, *J* = 53.3 Hz, 1H), 7.05 (d, *J* = 4.6 Hz, 1H), 6.85 - 6.69 (m, 2H), 4.76 - 4.69 (m, 1H), 4.39 - 4.27 (m, 1H), 3.89 (d, *J* = 12.5 Hz, 2H), 3.02 - 2.93 (m, 2H), 2.88 - 2.71 (m, 3H), 2.25 - 2.17 (m, 2H), 2.16 - 1.90 (m, 9H), 1.79 (d, *J* = 11.2 Hz, 3H), 1.19 - 1.11 (m, 2H).

Example 96: 7-{1-[3-(difluoromethyl)-1-[(1S,4r)-4-{[(2S)-4-[1-(2,6-dioxopiperidin-3-yl)-3-methyl-2-oxobenzo[d]imidazol-4-yl]-2-methylpiperazin-1-yl]methyl}cyclohexyl]pyrazol-4-yl]-1,2,3-triazol-4-yl} pyrrolo[2,1-f][1,2]diazepane-3-carboxamide (96)

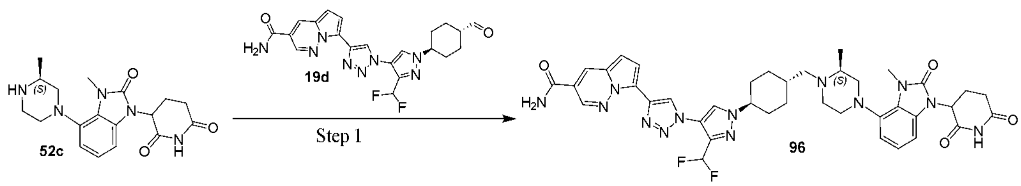


Compound 52c (130 mg, 0.35 mmol) and triethylamine (11.0 mg, 0.11 mmol) were added to N,N-dimethylformamide (2 mL) and tetrahydrofuran (2 mL) at room temperature and the obtained mixture was stirred for 10 min. Acetic acid (9.00 mg, 0.15 mmol) and compound 19d (160 mg, 0.35 mmol) were added, and the reaction solution was stirred at 25°C for 0.5 h. Sodium borohydride acetate (179 mg, 0.15 mmol) was slowly added, and the reaction solution was stirred for 1 h. The solvent was removed under reduced pressure, and the residue was purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 96. MS m/z(ESI):796.6[M+1]$^+$. $^1$H NMR(400MHz, DMSO-$d_6$):δ 11.10 (s, 1H), 9.01 (s, 1H), 8.79 (s, 1H), 8.76 (s, 1H), 8.66 (d, *J* = 2.3 Hz, 1H), 8.16 (s, 1H), 7.65 (d, *J* = 4.6 Hz, 1H), 7.57 (s, 1H), 7.24 (t, *J* = 53.3 Hz, 1H), 7.05 (d, *J* = 4.6 Hz, 1H), 7.01 - 6.88 (m, 3H), 5.38 - 5.33 (m, 1H), 4.34 - 4.28 (m, 1H), 3.64 (s, 3H), 3.00 - 2.85 (m, 5H), 2.71 - 2.59 (m, 5H), 2.34 - 2.33 (m, 1H), 2.20 - 2.17 (m, 3H), 2.00 - 1.98 (m, 2H), 1.86 - 1.83 (m, 3H), 1.64 - 1.56 (m, 1H), 1.15 - 1.05 (m, 5H).

Example 97: 1-(1-(3-(difluoromethyl)-1-((1r,4r)-4-((8-(1-(2,6-dioxopiperidin-3-yl)-3-methyl-2-oxo-3,3-dihydro-1H-benzo [d]imidazol-4-yl)-2,8-diazaspiro[4.5]dec-2-yl)methyl)cyclohexyl)-1H-pyrazol-4-yl)-1H-1-1,2,3-triazolyl)-1H-pyrrolo[2,3-b]pyridine-5-carboxamide (97)

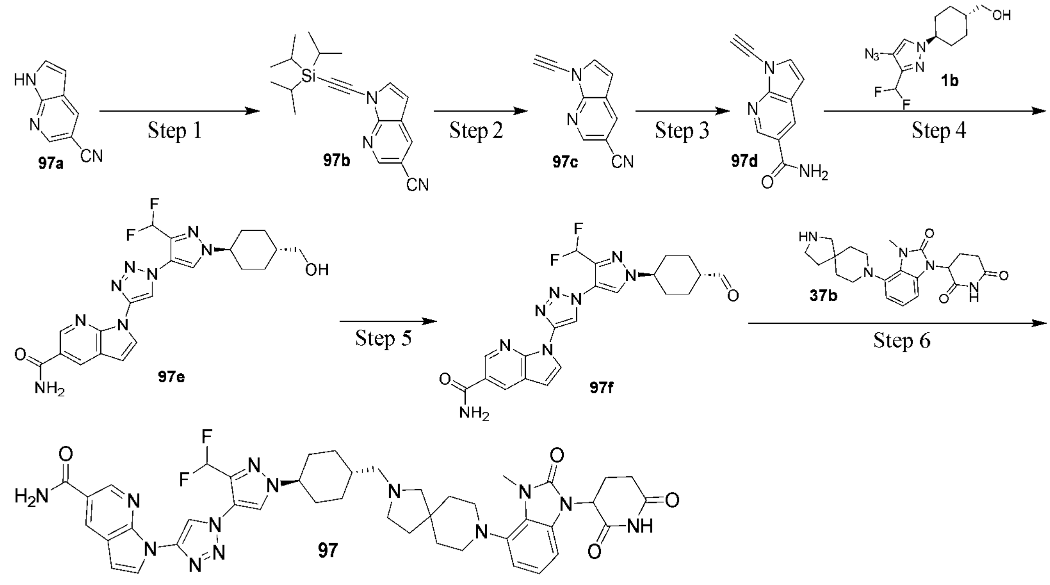


97a 97b 97c 97d 1b 97e 97f 37b 97

1) Step 1: At room temperature, compound 97a (0.500 g, 3.49 mmol, Bide Pharmatech Ltd.) and toluene (10 mL) were added to a 250 mL single-neck bottle and stirred until dissolved. (2-bromoethynyl)triisopropylsilane (1.10 g, 4.19 mmol), 1,10-phenanthroline (0.1 mL, 0.700 mmol), potassium phosphate (1.48 g, 6.98 mmol) and anhydrous copper sulfate (60.0 mg, 0.350 mmol) were added in sequence. The reaction solution was stirred at 80°C under nitrogen protection for 12 h. The reaction solution was cooled to room temperature, water (30 mL) was added to the reaction solution, and the aqueous phase was extracted with ethyl acetate (50 mL $\times$ 3). The combined organic phase was washed with saturated brine (20 mL), dried over anhydrous sodium sulfate and filtered to remove the desiccant, and the filtrate was concentrated under reduced pressure. The residue was purified by silica gel column chromatography (petroleum ether/ethyl acetate = 100/1 to 3/1) to obtain compound 97b. $^1$H NMR (400 MHz, $CDCl_3$) $\delta$ 8.68 (d, *J* = 1.9 Hz, 1H), 8.19 (d, *J* = 1.9 Hz, 1H), 7.46 (d, *J* = 3.8 Hz, 1H), 6.62 (d, *J* = 3.8 Hz, 1H), 1.30 - 1.12 (m, 21H).

2) Step 2: Under ice bath, compound 97b (4.40 g, 13.6 mmol) was added to a 50 mL three-neck bottle, and tetrahydrofuran (50 mL) was added and stirred. Tetrabutylammonium fluoride (15 mL, 15.0 mmol, 1.0 M solution in tetrahydrofuran) was slowly added dropwise to the reaction solution at 0°C, and the reaction solution was stirred at 25°C for 6 h. Saturated brine (50 mL) was added to the reaction solution, liquid separation was performed, and the organic phase was extracted with ethyl acetate (50 mL $\times$ 3). The combined organic phase was washed with saturated sodium bicarbonate (50 mL $\times$ 2), dried over anhydrous sodium sulfate and filtered to remove the desiccant, and the filtrate was concentrated under reduced pressure to obtain compound 97c.

3) Step 3: Compound 97c (1.70 g, 10.2 mmol) was dissolved in dimethyl sulfoxide (10 mL). Potassium carbonate (7.03 g, 50.9 mmol) was added at 0°C and hydrogen peroxide (10.2 mL, 34.0 mmol, 30%) was slowly added dropwise. The reaction solution was reacted at 0°C for 5 min. Water (5 mL) was added to the reaction solution for dilution, and solids precipitated. The reaction solution was filtered and the filter cake was washed 3 times with ethyl acetate (10 mL) and petroleum ether (30 mL) respectively. The solid was dried under reduced pressure to obtain compound 97d. MS m/z(ESI):186.1[M+1]$^+$.

4) Step 4: Compound 97d (0.900 g, 4.86 mmol) and compound 1b were dissolved in ethanol (15 mL) and water (15 mL). Sodium ascorbate (0.100 g, 0.490 mmol, Energy Chemical) and copper sulfate pentahydrate (0.120 g, 0.490 mmol, Energy Chemical) were added to the reaction solution in sequence. The reaction solution was stirred at 25°C for 12 h. The reaction solution was concentrated under reduced pressure to obtain a residue, which was diluted with water (20 mL) and ethyl acetate (20 mL). The mixture was filtered, and the solid was collected and dried under reduced pressure to obtain compound 97e. MS m/z(ESI):457.3[M+1]$^+$.

5) Step 5: Compound 97e (1.26 g, 2.76 mmol) was dissolved in *N,N*-dimethylformamide (10 mL) and dichloromethane (10 mL). Dess-Martin oxidant (1.76 g, 4.14 mmol) was added to the reaction solution in portions. The reaction solution was stirred at 25°C for 1 h. The reaction solution was filtered through diatomaceous earth. The filtrate was concentrated under reduced pressure to obtain a residue. The residue was purified by silica gel column chromatography (dichloromethane/methanol = 100/1 to 20/1) to obtain compound 97f. MS m/z(ESI):455.2[M+1]$^+$. $^1$H NMR (400 MHz, DMSO-$d_6$): $\delta$ 9.64 (s, 1H), 9.04 (s, 1H), 8.90 (d, *J* = 1.8 Hz, 1H), 8.77 (s, 1H), 8.62 (d, *J* = 1.9 Hz, 1H), 8.27 (d, *J* = 3.7 Hz, 1H), 8.19 - 8.09 (m, 1H), 7.53 - 7.45 (m, 1H), 7.25 (t, *J* = 55.1, 51.5 Hz, 1H), 6.94 (d, *J* = 3.7 Hz, 1H), 4.40 - 4.26 (m, 1H), 2.45 - 2.37 (m, 1H), 2.26 - 2.09 (m, 4H), 1.92 - 1.84 (m, 2H), 1.50 - 1.38 (m, 2H).

6) Step 6: Compound 37b (48.1 mg, 0.120 mmol) was dissolved in *N,N*-dimethylformamide (1 mL), triethylamine (11.1

mg, 0.110 mmol) was added, and the reaction solution was stirred at 25°C for 5 min. Acetic acid (6.61 mg, 0.110 mmol) and sodium borohydride acetate (116 mg, 0.550 mmol) were added, the obtained mixture was stirred for 5 min, and then compound 97f (50.0 mg, 0.110 mmol) was added finally. The reaction solution was stirred for 1 h. A drop of water was added to the reaction solution to quench the reaction, and the residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 97. MS m/z(ESI):836.8[M+1]+. $^1$H NMR (400 MHz, DMSO-$d_6$): δ 11.07 (s, 1H), 9.04 (s, 1H), 8.90 (d, *J* = 1.9 Hz, 1H), 8.77 (s, 1H), 8.62 (d, *J* = 1.9 Hz, 1H), 8.27 (d, *J* = 3.7 Hz, 1H), 8.18 - 8.11 (m, 1H), 7.52 - 7.41 (m, 1H), 7.24 (t, *J* = 52 Hz, 1H), 6.99 - 6.91 (m, 3H), 6.89 - 6.84 (m, 1H), 5.39 - 5.28 (m, 1H), 4.37 - 4.25 (m, 1H), 3.64 (s, 3H), 3.02 - 2.96 (m, 2H), 2.76 - 2.69 (m, 2H), 2.76 - 2.69 (m, 2H), 2.68 - 2.66 (m, 1H), 2.65 - 2.62 (m, 1H), 2.38 - 2.25 (m, 4H), 2.19 - 2.14(m, 2H), 2.03 - 1.93 (m, 4H), 1.86 - 1.60 (m, 9H), 1.16 - 1.06 (m, 2H).

Example 98: 1-(1-(3-(difluoromethyl)-1-((1r,4r)-4-((4-(1-(2-(2,6-dioxopiperidin-3-yl)-3-methyl-2-oxo-3,3-dihydro-1H-benzo[d]imidazol-4-yl)piperidin-4-yl)piperazin-1-yl)methyl)cyclohexyl)-1H-pyrazol-4-yl)-1H-1-1,2,3-triazole)-1H-pyrrolo [2,3-b]pyridine-5-carboxamide (98)

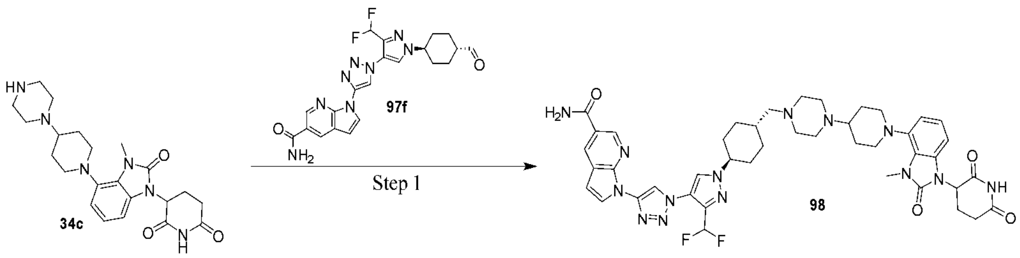


Compound 34c (55.4 mg, 0.130 mmol) was dissolved in *N,N*-dimethylformamide (1 mL), triethylamine (13.3 mg, 0.130 mmol) was added, and the reaction solution was stirred at 25°C for 1 min. Then acetic acid (15.8 mg, 0.260 mmol) and sodium triacetylborohydride (139 mg, 0.660 mmol) were added, and the obtained mixture was stirred for 1 min. Then compound 97f (60.0 mg, 0.130 mmol) was added, and the reaction solution was stirred for 20 min, then diluted with water (0.2 mL) and filtered to obtain a residue which was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 98. MS m/z(ESI):865.7 [M+1]+. $^1$H NMR (400 MHz, DMSO-$d_6$) δ 11.05 (s, 1H), 9.01 (s, 1H), 8.88 (d, *J* = 2.1 Hz, 1H), 8.74 (s, 1H), 8.60 (d, *J* = 2.1 Hz, 1H), 8.24 (d, *J* = 3.8 Hz, 1H), 8.17 - 8.08 (m, 1H), 7.55 - 7.37 (m, 1H), 7.21 (t, *J* = 53.3 Hz, 1H), 6.97 - 6.82 (m, 4H), 5.37 - 5.27 (m, 1H), 4.34 - 4.20 (m, 1H), 3.60 (s, 3H), 3.17 - 3.07 (m, 4H), 2.94 - 2.78 (m, 2H), 2.75 - 2.63 (m, 4H), 2.63 - 2.60 (m, 1H), 2.53 - 2.52 (m, 1H), 2.40 - 2.33 (m, 3H), 2.30 - 2.25 (m, 1H), 2.18 - 2.09 (m, 4H), 2.00 - 1.82 (m, 6H), 1.80 - 1.77 (m, 1H), 1.65 - 1.51 (m, 3H), 1.13 - 1.01 (m, 2H).

Example 99: 1-(1-(3-(difluoromethyl)-1-((1r,4r)-4-((9-(1-(2,6-dioxopiperidin-3-yl)-3-methyl-2-oxo-2,3-dihydro-1H-benzo [d]imidazol-4-yl)-3,9-diazaspiro[5.5]undecan-3-yl)methyl)cyclohexyl)-1H-pyrazol-4-yl)-1H-1,2,3-triazol-4-yl(99)

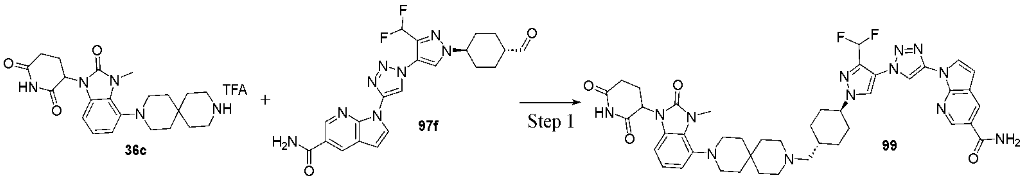


Compound 36c (80.0 mg, 0.120 mmol) was dissolved in N,N-dimethylformamide (0.5 mL) and tetrahydrofuran (2 mL) at 0°C, triethylamine (35.0 mg, 0.350 mmol), sodium borohydride acetate (155 mg, 0.730 mmol), compound 97f (60.0 mg, 0.130 mmol) and acetic acid (44.0 mg, 0.730 mmol) were added, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio:

acetonitrile 18%-95%, flow rate: 25 mL/min) to obtain compound 99. MS m/z(ESI):850.4 [M+1]$^+$. $^1$H NMR (400 MHz, DMSO-$d_6$) δ 11.07 (s, 1H), 9.04 (s, 1H), 8.91 (d, *J* = 2.0 Hz, 1H), 8.76 (s, 1H), 8.62 (d, *J* = 2.0 Hz, 1H), 8.26 (d, *J* = 3.6 Hz, 1H), 8.14 (s, 1H), 7.48 (s, 1H), 7.24 (t, *J* = 53.2 Hz, 1H), 7.03 - 6.90 (m, 3H), 6.89 - 6.81 (m, 1H), 5.43 - 5.27 (m, 1H), 4.30 (t, *J* = 11.6 Hz, 1H), 3.63 (s, 3H), 2.95 - 2.82 (m, 5H), 2.74 - 2.59 (m, 2H), 2.37 - 2.35 (m, 4H), 2.25 - 2.10 (m, 4H), 2.03 - 1.90 (m, 3H), 1.87 - 1.76 (m, 2H), 1.74 - 1.30 (m, 9H), 1.17 - 1.02 (m, 2H).

Example 100: 1-(1-(3-(difluoromethyl)-1-((1r,4r)-4-((4-(1-(2,6-dioxopiperidin-3-yl)-3-methyl-2-oxo-2,3-dihydro-1H-benzo[d]imidazol-4-yl)piperazin-1-yl)methyl)cyclohexyl)-1H-pyrazol-4-yl)-1H-1,2,3-triazol-4-yl)-1H-pyrrolo[2,3-b]pyridine-5-carboxamide (100)

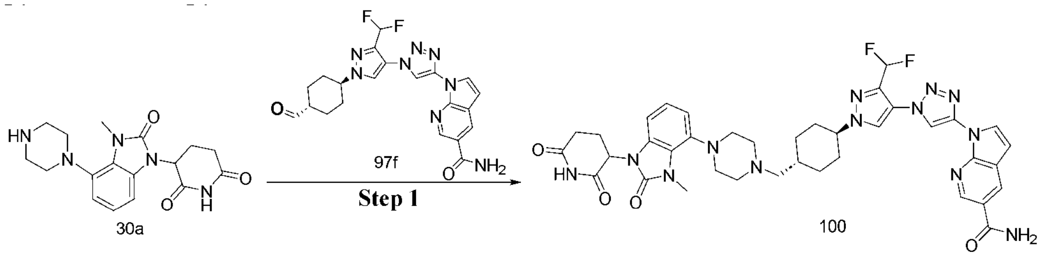

30a
100

Triethylamine (0.20 mL, 1.44 mmol) was added to a solution containing compound 30a (20.0 mg, 0.06 mmol), tetrahydrofuran (2 mL) and *N,N*-dimethylformamide (1 mL), and the obtained mixture was stirred for 0.5 h. Then compound 97f (32.0 mg, 0.07 mmol) and acetic acid (0.5 mL) were added to the above solution and stirred for 0.5 h. Finally, sodium borohydride acetate (37.0 mg, 0.17 mmol, Energy Chemical) was added to the above reaction solution. The reaction was also stirred at 25°C for 2 h. The reaction solution was concentrated under reduced pressure and then purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 100. MS m/z(ESI):782.6 [M+1]$^+$. $^1$H NMR (400 MHz, DMSO-$d_6$) δ 11.08 (s, 1H), 9.04 (s, 1H), 8.91 (d, *J* = 1.9 Hz, 1H), 8.77 (s, 1H), 8.62 (d, *J* = 1.9 Hz, 1H), 8.27 (d, *J* = 3.7 Hz, 1H), 8.19 - 8.11 (m, 2H), 7.49 (s, 1H), 7.38 - 7.11 (m, 1H), 7.01 - 6.93 (m, 3H), 6.91 - 6.85 (m, 1H), 5.40 - 5.31 (m, 1H), 4.36 - 4.26 (m, 1H), 3.64 (s, 3H), 2.99 - 2.84 (m, 6H), 2.69 - 2.61 (m, 2H), 2.38 - 2.32 (m, 1H), 2.28 - 2.23 (m, 2H), 2.22 - 2.12 (m, 3H), 2.04 - 1.95 (m, 3H), 1.89 - 1.76 (m, 2H), 1.68 - 1.61 (m, 1H), 1.31 - 1.22 (m, 1H), 1.20 - 1.02 (m, 2H).

Example 101: 1-(1-(3-(difluoromethyl)-1-((1r,4r)-4-((4-(1-(2,6-dioxopiperidin-3-yl)-3-methyl-2-oxo-2,3-dihydro-1H-benzo[d]imidazol-4-yl)piperidin-1-yl)methyl)cyclohexyl)-1H-pyrazol-4-yl)-1H-1,2,3-triazol-4-yl)-1H-pyrrolo[2,3-b]pyridine-5-carboxamide (101)

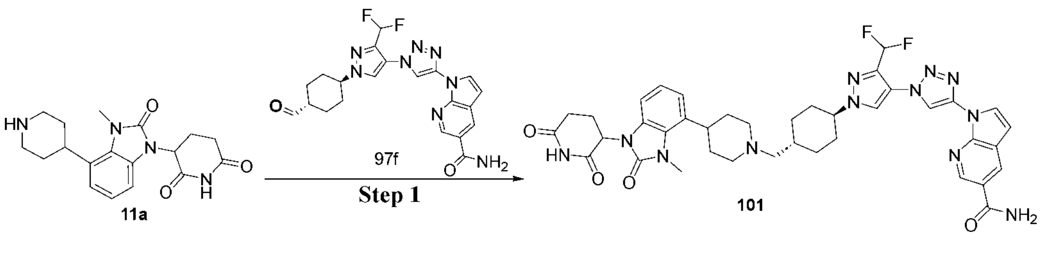

**11a**
**101**

Triethylamine (0.20 mL, 1.44 mmol) was added to a solution containing compound 11a (40.0 mg, 0.12 mmol), tetrahydrofuran (2 mL) and N,N-dimethylformamide (1 mL), and the obtained mixture was stirred for 0.5 h. Then compound 97f (64.0 mg, 0.14 mmol) and acetic acid (0.5 mL) were added to the above solution and stirred for 0.5 h. Finally, sodium borohydride acetate (74.0 mg, 0.35 mmol, Energy Chemical) was added to the above reaction solution. The reaction was also stirred at 25°C for 2 h. The reaction solution was concentrated under reduced pressure and then purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 30%-45%, flow rate: 25 mL/min) to obtain compound 101. MS m/z(ESI):781.6 [M+1]$^+$. $^1$H NMR (400 MHz, DMSO-$d_6$) δ 11.08 (s, 1H), 9.04 (s, 1H), 8.90 (d, *J* = 2.0 Hz, 1H), 8.77 (s, 1H), 8.62 (d, *J* = 1.8 Hz, 1H), 8.27 (d, *J* = 3.6 Hz, 1H), 8.14 (s, 2H),

7.49 (s, 1H), 7.21 (t, *J* = 30.2 Hz, 1H), 7.05 - 7.01 (m, 2H), 7.00 - 6.92 (m, 2H), 5.41 - 5.31 (m, 1H), 4.42 - 4.25 (m, 1H), 3.59 (s, 3H), 3.11 - 3.00 (m, 2H), 2.98 - 2.83 (m, 2H), 2.76 - 2.70 (m, 1H), 2.69 - 2.63 (m, 2H), 2.62 - 2.58 (m, 1H), 2.35 - 2.26 (m, 2H), 2.23 - 2.16 (m, 3H), 2.02 - 1.96 (m, 3H), 1.85 - 1.80 (m, 4H), 1.41 - 1.22 (m, 1H), 1.22 - 1.08 (m, 2H).

Example 102: (1-(3-difluoromethyl)-1-(1R,4r)-4-((1r,4R)-4-(1-(2,6-dioxopiperidin-3-yl)-3-methyl-2-oxo-2,3-dihydro-1H-benzoimidazol-4-yl)amino)cyclohexyl(methyl)amino)methyl)cyclohexyl)-1H-pyrazol-4-yl)-1H-1,2,3-triazol-4-yl)-1H-pyrrolo[2,3-b]pyridine-5-carboxamide (102)

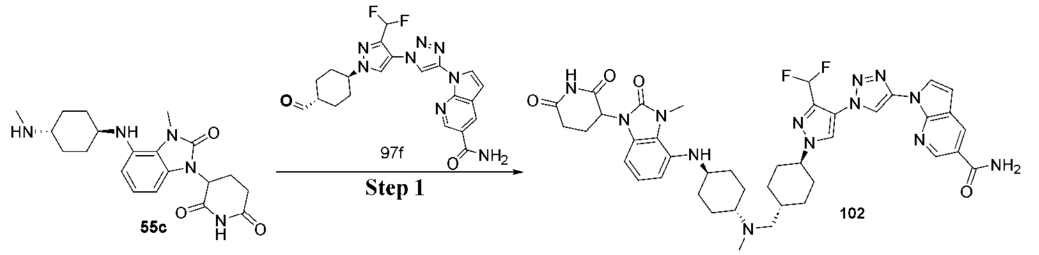


Triethylamine (0.20 mL, 1.44 mmol) was added to a solution containing compound 55c (40.0 mg, 0.10 mmol), tetrahydrofuran (2 mL) and N,N-dimethylformamide (1 mL), and the obtained mixture was stirred for 0.5 h. Then compound 97f (57.0 mg, 0.12 mmol) and acetic acid (0.5 mL) were added to the above solution and stirred for 0.5 h. Finally, sodium borohydride acetate (66.0 mg, 0.31 mmol, Energy Chemical) was added to the above reaction solution. The reaction was also stirred at 25°C for 2 h. The reaction solution was concentrated under reduced pressure and then purified by high performance liquid chromatography (A: 0.1% FA/H2O B: ACN, chromatographic column: Waters-SunFire-C18-10μm-19*250 mm; mobile phase: water (containing 0.1% formic acid) and acetonitrile, gradient ratio: acetonitrile 32%-48%, flow rate: 25 mL/min) to obtain compound 102 (10 mg). MS m/z(ESI):824.9[M+1]$^+$. $^1$H NMR (400 MHz, DMSO-$d_6$) δ 11.05 (s, 1H), 9.05 (d, *J* = 9.5 Hz, 1H), 8.91 (d, *J* = 1.9 Hz, 1H), 8.77 (s, 1H), 8.62 (d, *J* = 2.0 Hz, 1H), 8.27 (d, *J* = 3.7 Hz, 1H), 8.18 (s, 1H), 7.48 (s, 1H), 7.20 (t, *J* = 29.4 Hz, 1H), 6.94 (d, *J* = 3.8 Hz, 1H), 6.87 - 6.81 (m, 1H), 6.53 - 6.46 (m, 2H), 5.32 - 5.23 (m, 1H), 4.64 - 4.46 (m, 1H), 4.38 - 4.23 (m, 1H), 3.60 (s, 3H), 3.17 - 3.05 (m, 2H), 3.03 - 2.78 (m, 2H), 2.75 - 2.63 (m, 2H), 2.63 - 2.56 (m, 2H), 2.43 - 2.34 (m, 2H), 2.29 - 2.25 (m, 2H), 2.22 (s, 3H), 2.20 - 2.14 (m, 2H), 2.10 - 2.04 (m, 2H), 1.98 - 1.94 (m, 1H), 1.80 - 1.75 (m, 2H), 1.43 - 1.34 (m, 2H), 1.29 - 1.24 (m, 2H), 1.13 - 1.00 (m, 2H).

Example 103: 1-(1-(3-(difluoromethyl)-1-((1r,4r)-4-(2-(1-(2,6-dioxopiperidin-3-yl)-3-methyl-2-oxo-2,3-dihydro-1H-benzo[d]imidazol-4-yl)-2,8-diazaspiro[4.5]dec-8-yl)methyl)cyclohexyl)-1H-pyrazol-4-yl)-1H-1,2,3-triazol-4-yl(103)

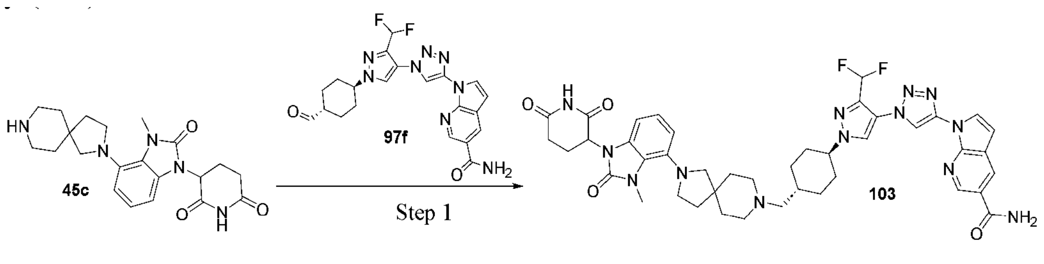


Compound 45c (120 mg, 0.170 mmol) was dissolved in *N,N*-dimethylformamide (0.5 mL) and tetrahydrofuran (2 mL) at 0°C, triethylamine (51.0 mg, 0.50 mmol), sodium borohydride acetate (216 mg, 1.02 mmol), compound 97f (77.0 mg, 0.170 mmol) and acetic acid (61.0 mg, 1.02 mmol) were added, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 21%-95%, flow rate: 25 mL/min) to obtain compound 103. MS m/z(ESI):836.6 [M+1]$^+$. $^1$H NMR (400 MHz, DMSO-$d_6$) δ 11.07 (s, 1H), 9.04 (s, 1H), 8.90 (d, *J* = 2.0 Hz, 1H), 8.76 (s, 1H), 8.62 (d, *J* = 2.0 Hz, 1H), 8.27 (d, *J* = 3.6 Hz, 1H), 8.16 (s, 1H), 7.48 (s, 1H), 7.24 (t, *J* = 53.3 Hz, 1H), 7.03 - 6.92 (m, 3H), 6.89 - 6.81 (m, 1H), 5.40 - 5.28 (m, 1H), 4.35 - 4.22 (m, 1H), 3.59 (s, 3H), 3.07 (t, *J* = 6.8 Hz, 2H), 2.96 - 2.82 (m, 3H), 2.75 - 2.57 (m, 3H), 2.40 (s, 3H), 2.23 - 2.13 (m, 4H), 2.04 - 1.92 (m, 3H), 1.86 - 1.73 (m, 4H), 1.70 - 1.57 (m, 5H), 1.18 - 1.03 (m, 2H).

Example 104: 1-(1-(3-(difluoromethyl)-1-((1r,4r)-4-((4-(4-(1-(2,6-dioxopiperidin-3-yl)-3-methyl-2-oxo-2,3-dihydro-1H-benzo[d]imidazol-4-yl)piperazin-1-yl)piperidin-1-yl)methyl)cyclohexyl)-1H-pyrazol-4-yl)-1H-1,2,3-triazol-4-yl)-1H-pyrrolo[2,3-b]pyridine-5-carboxamide (104)

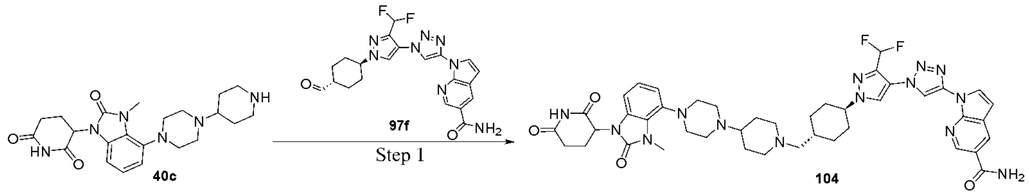


Compound 40c (70.0 mg, 129 μmol), DMF (1 mL) and tetrahydrofuran (1.5 mL) were added to a single-neck bottle at room temperature in sequence, triethylamine (44.9 μL, 324 μmol) was added under stirring, sodium borohydride acetate (164 mg, 777 μmol), compound 97f (64.7 mg, 142 μmol) and acetic acid (44.4 μL, 777 μmol) were added at 0°C, and the obtained mixture was reacted at 25°C for 2 h. Most of the solvent was removed from the reaction solution under vacuum to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 2%-95%, flow rate: 25 mL/min) to obtain compound 104. MS m/z(ESI):865.4[M+1]$^+$. $^1$H NMR (400 MHz, DMSO-$d_6$) δ 11.07 (s, 1H), 9.04 (s, 1H), 8.91 (d, *J* = 2.1 Hz, 1H), 8.76 (s, 1H), 8.62 (d, *J* = 2.1 Hz, 1H), 8.28 - 8.25 (m, 1H), 8.17 - 8.14 (m, 2H), 7.48 (s, 1H), 7.24 (t, *J* = 53.3 Hz, 1H), 7.02 - 6.84 (m, 4H), 5.37 - 5.33 (m, 1H), 4.33 - 4.28 (m, 1H), 3.62 (s, 3H), 3.12 - 2.77 (m, 9H), 2.77 - 2.52 (m, 4H), 2.28 - 2.25 (m, 1H), 2.20 - 2.17 (m, 4H), 1.99 - 1.93 (m, 5H), 1.83 - 1.79 (m, 4H), 1.61 (s, 1H), 1.50 - 1.46 (m, 2H), 1.11 - 1.06 (m, 2H).

Example 105: 1-(1-(3-(difluoromethyl)-1-((1r,4r)-4-((2-(1-(2,6-dioxopiperidin-3-yl)-3-methyl-2-oxo- 2,3-dihydro-1H-benzo[d]imidazol-4-yl)-2,7-diazaspiro[3.5]non-7-yl)methyl)cyclohexyl)-1H-pyrazol-4-yl)-1H-1,2,3-triazol-4-yl)-1H-pyrrolo [2,3-b]pyridine-5-carboxamide (105)

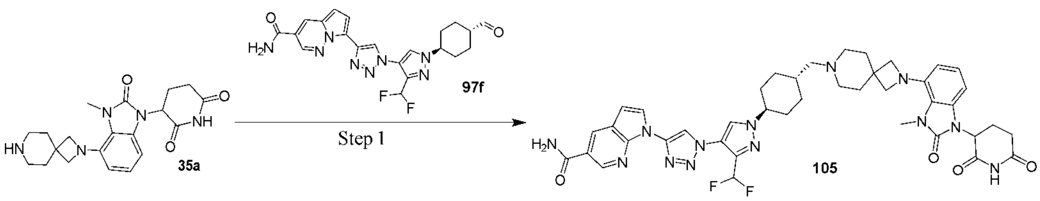


Compound 35a (45.0 mg, 0.12 mmol) and triethylamine (12.0 mg, 0.12 mmol) were added to N,N-dimethylformamide (2 mL) and tetrahydrofuran (2 mL) at room temperature and the obtained mixture was stirred for 10 min. Acetic acid (10.0 mg, 0.17 mmol) and compound 97f (55.0 mg, 0.12 mmol) were added, and the reaction solution was stirred at 25°C for 0.5 h. Sodium borohydride acetate (76.0 mg, 0.36 mmol) was slowly added, and the reaction solution was stirred for 1 h. The solvent was removed under reduced pressure. The residue was purified by high performance liquid chromatography (Gilson_306_1741, chromatographic column: Waters-SunFire-C18-10μm-19×250 mm; mobile phase: water (containing 10 mmol/L ammonium bicarbonate) and acetonitrile, gradient ratio: acetonitrile 10%-90%, flow rate: 25 mL/min) to obtain compound 105. MS m/z(ESI):823.1[M+1]$^+$. $^1$H NMR(400MHz, DMSO-$d_6$):δ 11.06 (s, 1H), 9.04 (s, 1H), 8.91 (d, *J* = 2.0 Hz, 1H), 8.76 (s, 1H), 8.62 (d, *J* = 2.0 Hz, 1H), 8.26 (d, *J* = 3.8 Hz, 1H), 8.17 - 8.09 (m, 1H), 7.48 (s, 1H), 7.24 (t, *J* = 53.3 Hz, 1H), 6.99 - 6.94 (m, 1H), 6.93 - 6.92 (m, 1H), 6.73 - 6.66 (m, 2H), 5.34 - 5.29 (m, 1H), 4.33 - 4.26 (m, 1H), 3.58 - 3.55 (m, 6H), 2.91 - 2.84 (m, 1H), 2.74 - 2.56 (m, 3H), 2.34 - 2.32 (m, 4H), 2.18 - 2.13 (m, 4H), 2.00 - 1.93 (m, 3H), 1.86 - 1.77 (m, 6H), 1.62 - 1.58 (m, 1H), 1.14 - 1.05 (m, 2H).

Example 106: 1-{1-[3-(difluoromethyl)-1-[(1r,4r)-4-{[4-({3-[1-(2,6-dioxohexahydropyridin-3-yl)-3-methyl-2-oxobenzo[d]imidazol-4-yl]prop-2-ynyl}oxy)hexahydropyridin-1-yl]methyl}cyclohexyl]pyrazol-4-yl]-1,2,3-triazol-4-yl}pyrrolo[2,3-b]pyridine-5-carboxamide (106)

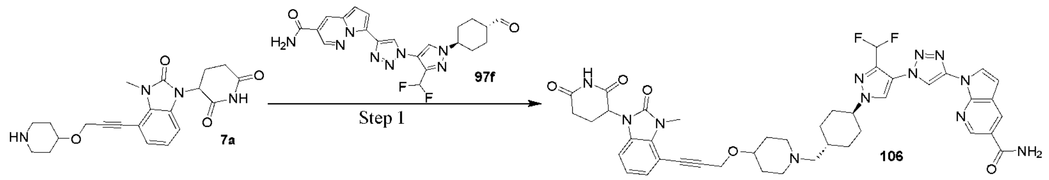


Compound 7a (40.0 mg, 0.10 mmol) (prepared by the method disclosed for the intermediate APT on page 91 of the specification of the patent application "WO 2021247899 A1") and triethylamine (10.0 mg, 0.10 mmol) were added to *N,N*-dimethylformamide (2 mL) and tetrahydrofuran (2 mL) at room temperature and the obtained mixture was stirred for 10 min. Acetic acid (9.00 mg, 0.15 mmol) and compound 97f (46.0 mg, 0.10 mmol) were added, and the reaction solution was stirred at 25°C for 0.5 h. Sodium borohydride acetate (64.0 mg, 0.30 mmol) was slowly added, and the reaction solution was stirred for 0.5 h. The solvent was removed under reduced pressure, and the residue was purified by high performance liquid chromatography (Gilson_306_1741, chromatographic column: Waters-SunFire-C18-10μm-19×250 mm; mobile phase: water (containing 10 mmol/L ammonium bicarbonate) and acetonitrile, gradient ratio: acetonitrile 10%-90%, flow rate: 25 mL/min) to obtain compound 106. MS m/z(ESI):835.7[M+1]$^+$. $^1$H NMR(400MHz, DMSO-$d_6$):δ 11.11 (s, 1H), 9.03 (s, 1H), 8.90 (d, *J* = 2.4 Hz, 1H), 8.76 (s, 1H), 8.62 (d, *J* = 2.0 Hz, 1H), 8.26 (d, *J* = 3.8 Hz, 1H), 8.14 (s, 1H), 7.48 (s, 1H), 7.23 (t, *J* = 53.5 Hz, 1H), 7.18 - 7.16 (m, 1H), 7.13 - 7.10 (m, 1H), 7.05 - 7.01 (m, 1H), 6.93 (d, *J* = 3.8 Hz, 1H), 5.42 - 5.38 (m, 1H), 4.48 (s, 2H), 4.32 - 4.27 (m, 1H), 3.64 (s, 3H), 3.58 - 3.52 (m, 1H), 2.89 - 2.85 (m, 1H), 2.68 - 2.65 (m, 4H), 2.18 - 2.12 (m, 4H), 2.05 - 2.03 (m, 3H), 1.95 - 1.91 (m, 4H), 1.82 - 1.76 (m, 2H), 1.51 - 1.48 (m, 3H), 1.12 - 1.06 (m, 2H).

Example 107: 1-(1-(3-(difluoromethyl)-1-((1r,4r)-4-((4-(2-((1-(2,6-dioxopiperidin-3-yl)-3-methyl-2-oxo-2,3-dihydro-1H-benzo[d]imidazol-4-yl)amino)-2-oxoethyl)piperazin-1-yl)methyl)cyclohexyl)-1H-pyrazol-4-yl)-1H - 1,2,3-triazol-4-yl)-1H-pyrrolo[2,3-b]pyridine-5-carboxamide (107)

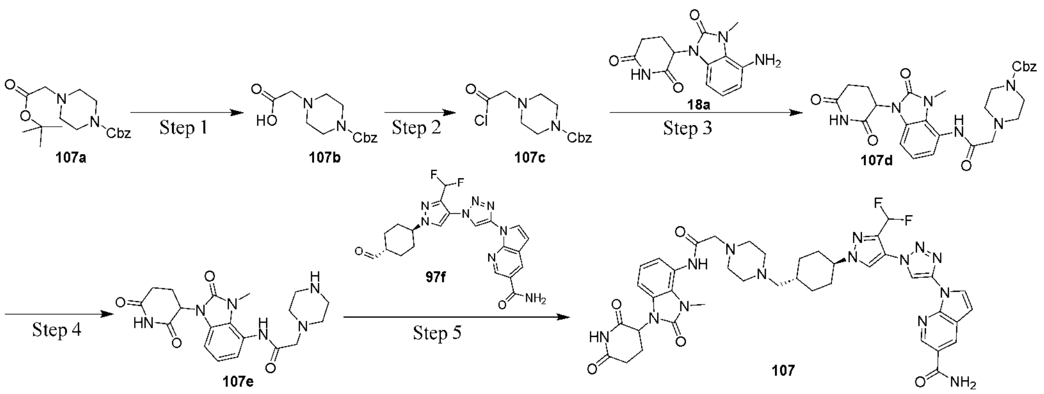


1) Step 1: A solution of compound 107a (4.00 g, 11.9 mol, prepared by the method disclosed for the intermediate 0568 on page 136 of the specification of the patent application "WO 2021/091575") in trifluoroacetic acid (15 mL) was stirred at 25°C for 18 h. The reaction solution was concentrated under reduced pressure, and the residue was purified by reverse phase column (water/acetonitrile = 25/75 to 80/20) to obtain compound 107b. MS m/z(ESI):279.3[M+1]$^+$.
2) Step 2: Compound 107b (1.1 g, 3.95 mmol) was dissolved in dichloromethane (20 mL), oxalyl chloride (1.02 mL, 12.8 mmol) was added at 0°C, and the obtained mixture was reacted for 18 h. The reaction solution was concentrated to obtain compound 107c. MS m/z(ESI):293.1[M+1]$^+$. (MS value for methyl ester of 107c)
3) Step 3: Compound 107c (2.00 g, 6.74 mmol) and compound 18a (934 mg, 3.37 mmol, prepared by the method disclosed for step 4 product on page 171 of the specification of the patent application "WO 2022068933 A1") were dissolved in dichloromethane (30 mL), and triethylamine (1.87 mL, 13.5 mmol) was added. The reaction solution was stirred at 25°C under nitrogen protection for 1 h. Water (100 mL) was added to the reaction solution, and the obtained mixture was extracted with ethyl acetate (150 mL×3). The combined organic phase was washed with saturated brine (100 mL), dried over anhydrous sodium sulfate and filtered to remove the desiccant, and then the filtrate was concentrated under reduced pressure. The residue was purified by silica gel column chromatography with an eluent system (dichloromethane/methanol = 40/1 to 15/1) to obtain compound 107d. MS m/z(ESI):535.2[M+1]$^+$.
4) Step 4: Compound 107d (500 mg, 0.94 mmol) was dissolved in methanol (8 mL) and tetrahydrofuran solution (8 mL), and wet palladium carbon (50 mg, 10%) was added. The reaction solution was subjected to hydrogen

replacement three times, and reacted at 25°C under hydrogen atmosphere for 18 h. The reaction solution was filtered and concentrated to obtain compound 107e. MS m/z(ESI):401.2[M+1]+.

5) Step 5: Compound 107e (65.0 mg, 0.161 mmol) was dissolved in *N,N*-dimethylformamide (5 mL), and sodium borohydride acetate (74.6 mg, 0.351 mmol, Bide Pharmatech Ltd.) was added. The reaction solution was stirred at 25°C for 5 min, and then compound 97f (73.7 mg, 0.161 mmol) was added. The reaction solution was stirred at 25°C for further 30 min. The reaction solution was concentrated under reduced pressure, and the obtained residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 107. MS m/z(ESI):839.6[M+1]+. $^1$H NMR (400 MHz, DMSO-$d_6$): δ 11.09 (s, 1H), 9.66 (s, 1H), 9.04 (s, 1H), 8.90 (d, *J* = 1.8 Hz, 1H), 8.76 (s, 1H), 8.62 (d, *J* = 1.9 Hz, 1H), 8.26 (d, *J* = 3.7 Hz, 1H), 8.16 (s, 1H), 7.48 (s, 1H), 7.24 (t, *J* = 53.3 Hz, 1H), 7.07 - 6.97 (m, 3H), 6.94 (d, *J* = 3.7 Hz, 1H), 5.44 - 5.31 (m, 1H), 4.36 - 4.23 (m, 1H), 3.47 (s, 4H), 3.16 (s, 2H), 2.94 - 2.86 (m, 1H), 2.74 - 2.64 (m, 2H), 2.63 - 2.57 (m, 4H), 2.48 - 2.40 (m, 3H), 2.22 - 2.12 (m, 4H), 2.06 - 1.99 (m, 1H), 1.98 - 1.90 (m, 2H), 1.89 - 1.74 (m, 2H), 1.69 - 1.54 (m, 1H), 1.19 - 1.02 (m, 2H).

Example 108: 3-(4-(9-(((1r,4r)-4-(4-(4-(5-((cyclopropylmethyl)amino) pyrazolo[1,5-a]pyrimidin-3-yl)-1H-1,2,3-triazol-1-yl)-3-(difluoromethyl)-1H-pyrazol-1-yl(cyclohexyl)methyl)-3,9-diazaspiro[5.5]undecan-3-yl)-3-methyl-2-oxo-2,3-dihydro-1H-benzo[d]imidazol-1-yl)piperidine-2,6-dione (108)

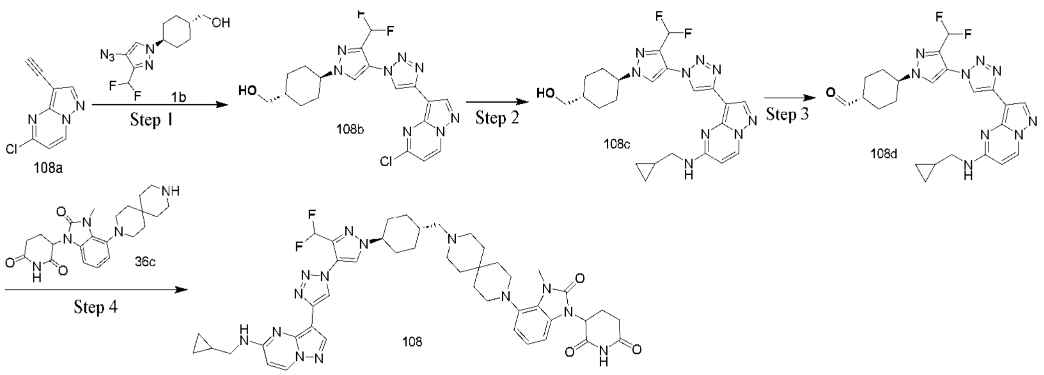


1) Step 1: Compound 108a (2.30 g, 13.0 mmol, prepared by the synthesis method for the intermediate 5-chloro-3-ethynylpyrazolo[1,5-a]pyrimidine on page 102 of the specification of the patent application "WO 2015108490 A2") and compound 1b (3.51 g, 13.0 mmol) were dissolved in a mixed solution of water (40 mL) and ethanol (40 mL), and copper sulfate pentahydrate (320 mg, 1.30 mmol) and sodium ascorbate (260 mg, 1.30 mmol) were added. The obtained mixture was reacted under stirring at room temperature for 12 h, and then concentrated under reduced pressure to remove ethanol. Water (50 mL) was added to the reaction solution, and a large amount of solid precipitated and was filtered. The filter cake was washed twice with (petroleum ether/ethyl acetate = 1/1) and filtered to obtain compound 108b, the obtained filter cake was directly used as the raw material for the next step without purification.. MS m/z(ESI):449.1[M+1]+.

2) Step 2: Cyclopropylmethylamine (0.60 mL, 6.92 mmol) and N,N-diisopropylethylamine (1.63 mL, 9.20 mmol) were added to a solution of compound 108b (1.00 g, 2.23 mmol) in acetonitrile (10 mL), and the mixture was stirred at 80°C under nitrogen atmosphere for 2 h. The mixture was concentrated to obtain a crude product. The crude product was purified by silica gel chromatography (dichloromethane/methanol = 100/1 to 20/1) to obtain compound 108c. MS m/z(ESI): 484.4[M+1]+.

3) Step 3: Dess-Martin oxidant (237 mg, 0.56 mmol) was added to a solution of 108c (180 mg, 0.37 mmol) in dichloromethane (5 mL), and the mixture was reacted under stirring at 25°C for 3 h. The mixture was poured into water (20 mL), and dichloromethane (30 mL × 3) was used for extraction. The combined organic phase was washed with brine (10 mL), dried over anhydrous sodium sulfate, filtered and concentrated to obtain a crude product. The crude product was slurried with petroleum ether/ethyl acetate (1/1, 20 mL) and filtered, and the filter cake was washed with petroleum ether/ethyl acetate (1/1, 20 mL) to obtain compound 108d. MS m/z(ESI): 482.2[M+1]+.

4) Step 4: Compound 36c (65.0 mg, 0.100 mmol, prepared by the method disclosed for the intermediate CA on page 411 of the specification of the patent application "WO 2022236058 A1") was dissolved in N,N-dimethylformamide (1 mL) and tetrahydrofuran (1 mL) at 0°C, triethylamine (30.0 mg, 0.300 mmol) was added, and the obtained mixture was stirred for 10 min. Then sodium borohydride acetate (126 mg, 0.590 mmol), compound 108d (65.0 mg, 0.110 mmol) and acetic acid (30.0 mg, 0.500 mmol) were added to the mixture, and the mixture was stirred at 25°C for 1 h. The

mixture was concentrated to obtain a residue. The reaction solution was concentrated under reduced pressure and then purified by high performance liquid chromatography (Gilson _306_1741, chromatographic column: Waters-SunFire-C18-10μm-19×250 mm; mobile phase: water (containing 26.5 mmol/L formic acid) and acetonitrile, gradient ratio: acetonitrile 16%-95%, flow rate: 25 mL/min) to obtain compound 108. MS m/z(ESI): 439.4[M/2+1]$^+$. $^1$H NMR (400 MHz, DMSO-$d_6$) δ 11.11 (s, 1H), 8.66 (s, 1H), 8.56 - 8.48 (m, 2H), 8.27 (s, 1H), 7.85 (s, 1H), 7.21 (t, *J* = 53.2 Hz, 1H), 7.03 - 6.92 (m, 2H), 6.89 - 6.81 (m, 1H), 6.35 (d, *J* = 7.6 Hz, 1H), 5.40 - 5.31 (m, 1H), 4.31 - 4.25 (m, 1H), 3.63 (s, 3H), 3.32 - 3.27 (m, 2H), 2.96 - 2.82 (m, 5H), 2.73 - 2.57 (m, 2H), 2.35 (s, 4H), 2.18 - 2.08 (m, 4H), 2.01 - 1.90 (m, 3H), 1.87 - 1.76 (m, 2H), 1.73 - 1.37 (m, 9H), 1.16 - 0.99 (m, 3H), 0.52 - 0.45 (m, 2H), 0.30 - 0.22 (m, 2H).

Example 109: 3-(4-(8-(((1r,4r)-4-(4-(4-(5-((cyclopropylmethyl)amino) pyrazolo[1,5-a]pyrimidin-3-yl)-1H-1,2,3-triazol-1-yl)-3-(difluoromethyl)-1H-pyrazol-1-yl(cyclohexyl)methyl)-2,8-diazaspiro[4.5]dec-2-yl)-3-methyl-2-oxo-2,3-dihydro-1H-benzo[d]imidazol-1-yl)piperidine-2,6-dione (109)

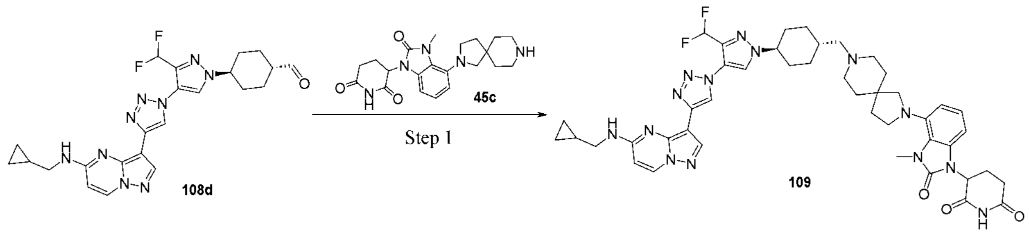


Compound 45c (65.0 mg, 0.100 mmol) was dissolved in N,N-dimethylformamide (1 mL) and tetrahydrofuran (1 mL) at 0°C, triethylamine (30.0 mg, 0.300 mmol) was added, and the obtained mixture was stirred for 10 min. Then sodium borohydride acetate (126 mg, 0.590 mmol), compound 108d (65.0 mg, 0.110 mmol) and acetic acid (30.0 mg, 0.500 mmol) were added to the mixture, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 16%-95%, flow rate: 25 mL/min) to obtain compound 109. MS m/z(ESI): 432.4[M/2+1]$^+$. $^1$H NMR (400 MHz, DMSO-$d_6$) δ 11.11 (s, 1H), 8.66 (s, 1H), 8.58 - 8.48 (m, 2H), 8.27 (s, 1H), 7.84 (s, 1H), 7.21 (t, *J* = 53.2 Hz, 1H), 7.01 - 6.91 (m, 2H), 6.89 - 6.82 (m, 1H), 6.35 (d, *J* = 7.6 Hz, 1H), 5.41 - 5.28 (m, 1H), 4.31 - 4.25 (m, 1H), 3.59 (s, 3H), 3.32 - 3.28 (m, 2H), 3.06 (t, *J* = 6.7 Hz, 2H), 2.96 - 2.82 (m, 3H), 2.73 - 2.57 (m, 2H), 2.45 - 2.24 (m, 4H), 2.14 (d, *J* = 6.9 Hz, 4H), 2.05 - 1.88 (m, 3H), 1.86 - 1.72 (m, 4H), 1.68 - 1.54 (m, 5H), 1.17 - 0.99 (m, 3H), 0.52 - 0.45 (m, 2H), 0.31 - 0.22 (m, 2H).

Example 110: 3-(4-(4-(1-(((1r,4r)-4-(4-(4-(5-((cyclopropylmethyl)amino) pyrazolo[1,5-a]pyrimidin-3-yl)-1H-1,2,3-triazol-1-yl)-3-(difluoromethyl)-1H-pyrazol-1-ylcyclohexyl)methyl)piperidin-4-yl)piperazin-1-yl)3-methyl-2-oxo-2,3-dihydro-1H-benzo[d]imidazol-1-yl)piperidine-2,6-dione (110)

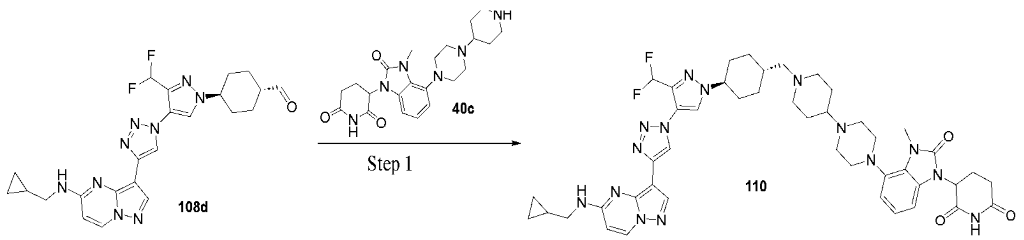


Compound 40c (60.0 mg, 0.110 mmol) was dissolved in *N,N*-dimethylformamide (1 mL) and tetrahydrofuran (1 mL) at 0°C, triethylamine (33.0 mg, 0.330 mmol) was added, and the obtained mixture was stirred for 10 min. Then sodium borohydride acetate (141 mg, 0.670 mmol), compound 108d (64.0 mg, 0.130 mmol) and acetic acid (33.0 mg, 0.550 mmol) were added to the mixture, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 2%-95%, flow rate: 25 mL/min) to obtain

compound 110. MS m/z(ESI): 892.6[M+1]$^+$. $^1$H NMR (400 MHz, DMSO-$d_6$) δ 11.10 (s, 1H), 8.66 (s, 1H), 8.54 - 8.47 (m, 2H), 8.27 (s, 1H), 7.84 (s, 1H), 7.21 (t, $J$ = 53.2 Hz, 1H), 7.03 - 6.84 (m, 3H), 6.35 (d, $J$ = 7.6 Hz, 1H), 5.41 - 5.30 (m, 1H), 4.37 - 4.18 (m, 1H), 3.62 (s, 3H), 3.31 - 3.26 (m, 2H), 3.06 - 2.76 (m, 9H), 2.74 - 2.56 (m, 3H), 2.37 - 2.07 (m, 6H), 2.01 - 1.74 (m, 9H), 1.58 (s, 1H), 1.50 - 1.37 (m, 2H), 1.18 - 0.98 (m, 3H), 0.54 - 0.43 (m, 2H), 0.31 - 0.20 (m, 2H).

Example 111: 3-(4-(4-(((1r,4r)-4-(3-(difluoromethyl)-4-(4-(5-(1,1-dioxodimorpholinyl)pyrazolo[1,5-a]pyrimidin-3-yl)-1H-1,2,3-triazol-1-yl)-1H-pyrazol-1-ylcyclohexyl)methyl)piperazin-1-yl)piperidin-1-yl)-3-methyl-2-oxo-2,3-dihydro-1H-benzo[d]imidazol-1-yl)pyridine-2,6-dione (111)

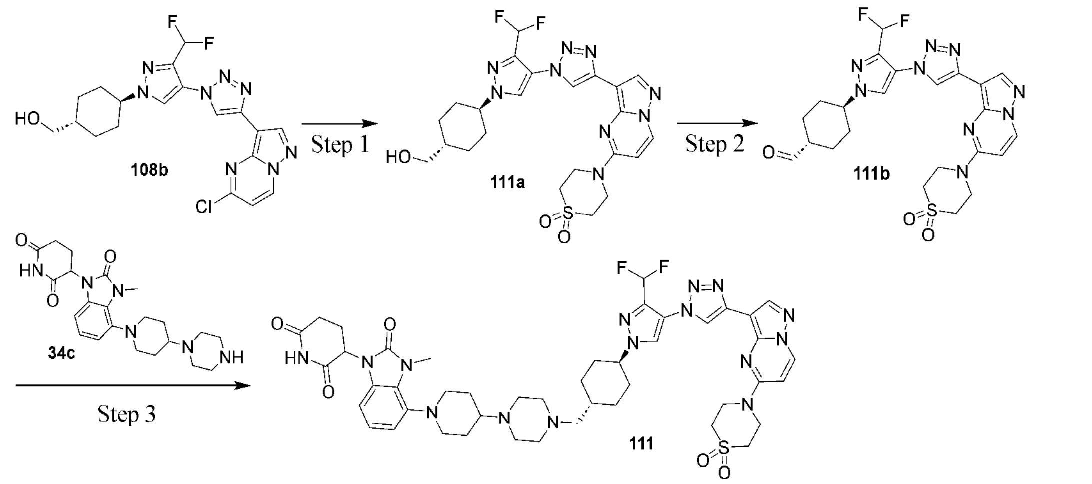


1) Step 1: Compound 108b (500 mg, 1.11 mmol) and thiomorpholine-1,1-dioxide (300 mg, 2.22 mmol) were added to a single-neck bottle at room temperature in sequence, and acetonitrile (10 mL) and *N,N*-diisopropylethylamine (185 μL, 1.11 mmol) were added under stirring. The reaction system was subjected to nitrogen replacement three times and reacted at 80°C for 18 h. The reaction solution was filtered through diatomaceous earth, and the filter cake was washed with ethyl acetate (5 mL × 3). The filtrate was collected and concentrated to obtain a residue. The residue was purified by silica gel column chromatography with an eluent system (dichloromethane/methanol = 100/1-10/1) to obtain compound 111a. MS m/z(ESI):548.2[M+1]$^+$.
2) Step 2: Compound 111a (215 mg, 0.390 mmol) was added to a single-neck bottle at room temperature, followed by N,N-dimethylformamide (8 mL) and Dess-Martin oxidant (250 mg, 0.590 mmol) in sequence under stirring, and the obtained mixture was reacted at 25°C for 2 h. Water (50 mL) was added to the reaction solution, and dichloromethane was used for extraction (30 mL × 3). The dichloromethane layer was collected and washed with saturated brine (30 mL), dried with anhydrous sodium sulfate, filtered and concentrated to obtain a crude product. The crude product was slurried with petroleum ether/ethyl acetate system (1/1, 10 mL) to obtain compound 111b. MS m/z(ESI):546.2[M+1]$^+$.
3) Step 3: Compound 34c (50.0 mg, 74.0 μmol), DMF (1.5 mL) and tetrahydrofuran (1 mL) were added to a single-neck bottle at room temperature in sequence, triethylamine (25.6 μL, 185 μmol) was added under stirring, and the obtained mixture was reacted at 0°C for 10 min. Then compound 111b (40.4 mg, 74.0 μmol), sodium borohydride acetate (93.7 mg, 444 μmol) and acetic acid (25.4 μL, 444 μmol) were added in sequence, and the obtained mixture was reacted at 25°C for 2 h. Most of the solvent was removed from the reaction solution under vacuum to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 17%-95%, flow rate: 25 mL/min) to obtain compound 111 (30.0 mg). MS m/z(ESI):956.4 [M+1]$^+$. $^1$H NMR (400 MHz, DMSO-$d_6$) δ 11.10 (s, 1H), 8.85 (d, $J$ = 7.9 Hz, 1H), 8.66 (s, 1H), 8.56 (s, 1H), 8.42 (s, 1H), 7.20 (t, $J$ = 53.2 Hz, 1H), 7.00 - 6.94 (m, 2H), 6.92 - 6.83 (m, 2H), 5.38 - 5.32 (m, 1H), 4.36 - 4.17 (m, 5H), 3.62 (s, 3H), 3.26 - 3.22 (m, 4H), 3.14 (d, $J$ = 11.0 Hz, 2H), 2.92 - 2.84 (m, 1H), 2.77 - 2.52 (m, 8H), 2.48 - 2.27 (m, 5H), 2.18 - 2.09 (m, 4H), 2.00 - 1.77 (m, 7H), 1.69 - 1.54 (m, 3H), 1.12 - 1.02 (m, 2H).

Example 112: 3-(4-(9-(((1r,4r)-4-(3-(difluoromethyl)-4-(4-(5-(1,1-dioxodimorpholinyl)pyrazolo[1,5-a]pyrimidin-3-yl)-1H-1,2,3-triazol-1-yl)-1H-pyrazol-1-yl(cyclohexyl)methyl)-3,9-diazaspiro[5.5]undecan-3-yl)-3-methyl-2-oxo-2,3-dihydro-1H-benzo[d]imidazol-1-yl)piperidine-2,6-dione (112)

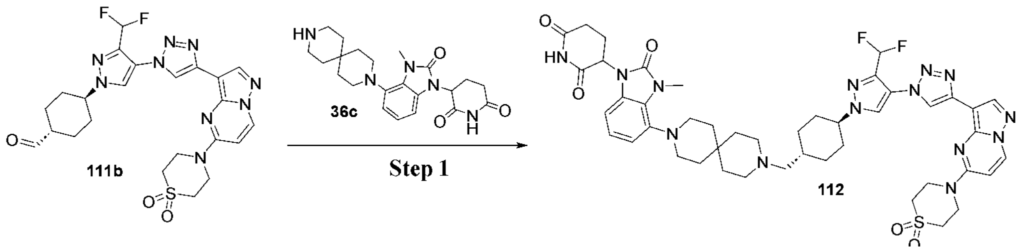


Compound 36c (50.00 mg, 76.1 μmol), DMF (1.5 mL) and tetrahydrofuran (1 mL) were added to a single-neck bottle at room temperature in sequence, triethylamine (26.4 μL, 190 μmol) was added under stirring, and the obtained mixture was reacted at 0°C for 10 min. Then compound 111b (41.5 mg, 76.1 μmol), sodium borohydride acetate (96.3 mg, 457 μmol) and acetic acid (26.1 μL, 457 μmol) were added in sequence, and the obtained mixture was reacted at 25°C for 2 h. Most of the solvent was removed from the reaction solution under vacuum to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 20%-95%, flow rate: 25 mL/min) to obtain compound 112. MS m/z(ESI):941.4[M+1]$^+$. $^1$H NMR (400 MHz, DMSO-$d_6$) δ 11.11 (s, 1H), 8.85 (d, $J$ = 7.9 Hz, 1H), 8.66 (s, 1H), 8.56 (s, 1H), 8.43 (s, 1H), 8.19 (s, 1H), 7.21 (t, $J$ = 53.2 Hz, 1H), 7.01 - 6.93 (m, 3H), 6.90 - 6.83 (m, 1H), 5.38 - 5.33 (m, 1H), 4.37 - 4.12 (m, 5H), 3.63 (s, 3H), 3.27 - 3.22 (m, 4H), 2.96 - 2.81 (m, 5H), 2.75 - 2.58 (m, 2H), 2.46 - 2.31 (m, 4H), 2.25 - 2.08 (m, 4H), 2.04 - 1.90 (m, 3H), 1.89 - 1.77 (m, 2H), 1.77 - 1.34 (m, 9H), 1.17 - 0.99 (m, 2H).

Example 113: 3-(4-(2-(((1r,4r)-4-(4-(4-(5-((cyclopropylmethyl)amino) pyrazolo[1,5-a]pyrimidin-3-yl)-1H-1,2,3-triazol-1-yl)-3-(difluoromethyl)-1H-pyrazol-1-yl(cyclohexyl)methyl)-2,8-diazaspiro[4.5]dec-8-yl)-3-methyl-2-oxo-2,3-dihydro-1H-benzo[d]imidazol-1-yl)piperidine-2,6-dione (113)

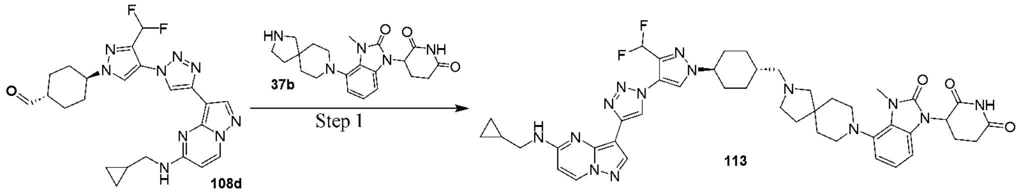


Compound 37b (60.0 mg, 0.140 mmol) was dissolved in *N,N*-dimethylformamide (1 mL) and tetrahydrofuran (1 mL) at 0°C, triethylamine (41.0 mg, 0.410 mmol) was added, and the obtained mixture was stirred for 10 min. Then sodium borohydride acetate (176 mg, 0.830 mmol), compound 108d (70.0 mg, 0.150 mmol) and acetic acid (41.0 mg, 0.680 mmol) were added to the mixture, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 10 mmol/L ammonium bicarbonate) and acetonitrile, gradient ratio: acetonitrile 26%-95%, flow rate: 25 mL/min) to obtain compound 113. MS m/z(ESI): 863.4[M+1]$^+$. $^1$H NMR (400 MHz, DMSO-$d_6$) δ 11.09 (s, 1H), 8.66 (s, 1H), 8.55 - 8.47 (m, 2H), 8.27 (s, 1H), 7.83 (s, 1H), 7.21 (t, $J$ = 53.2 Hz, 1H), 7.00 - 6.82 (m, 3H), 6.35 (d, $J$ = 7.6 Hz, 1H), 5.41 - 5.27 (m, 1H), 4.33 - 4.26 (m, 1H), 3.64 (s, 3H), 3.31 - 3.26 (m, 2H), 3.04 - 2.55 (m, 8H), 2.37 - 2.21 (m, 3H), 2.17 - 2.09 (m, 2H), 2.03 - 1.92 (m, 3H), 1.90 - 1.38 (m, 11H), 1.17 - 1.02 (m, 3H), 0.52 - 0.44 (m, 2H), 0.29 - 0.22 (m, 2H).

Example 114: 3-(4-(4-(4-(((1r,4r)-4-(4-(5-((cyclopropylmethyl)amino) pyrazolo[1,5-a]pyrimidin-3-yl)-1H-1,2,3-triazol-1-yl)-3-(difluoromethyl)-1H-pyrazol-1-ylcyclohexyl)methyl)piperazin-1-yl)piperidin-1-yl)3-methyl-2-oxo-2,3-dihydro-1H-benzo[d]imidazol-1-yl)pyridine-2,6-dione (114)

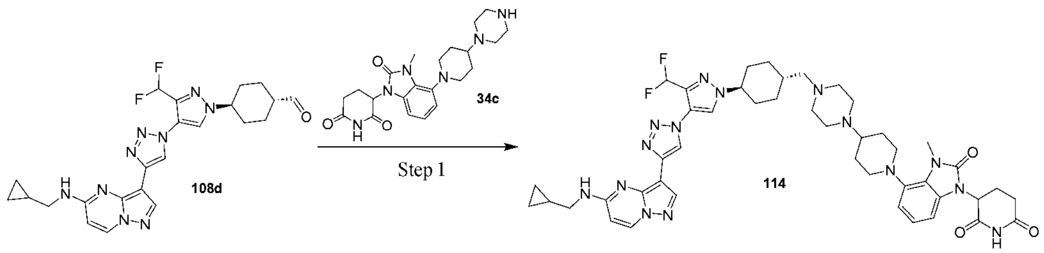


Compound 34c (60.0 mg, 0.110 mmol) was dissolved in *N,N*-dimethylformamide (1 mL) and tetrahydrofuran (1 mL) at 0°C, triethylamine (33.0 mg, 0.330 mmol) was added, and the obtained mixture was stirred for 10 min. Then sodium borohydride acetate (141 mg, 0.670 mmol), compound 108d (64.0 mg, 0.130 mmol) and acetic acid (33.0 mg, 0.550 mmol) were added to the mixture, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 25%-95%, flow rate: 25 mL/min) to obtain compound 114. MS m/z(ESI): 892.6[M+1]$^+$. $^1$H NMR (400 MHz, DMSO-$d_6$) δ 11.10 (s, 1H), 8.66 (s, 1H), 8.55 - 8.48 (m, 2H), 8.27 (s, 1H), 7.84 (s, 1H), 7.21 (t, *J* = 53.2 Hz, 1H), 7.01 - 6.93 (m, 1H), 6.92 - 6.79 (m, 2H), 6.35 (d, *J* = 7.6 Hz, 1H), 5.42 - 5.31 (m, 1H), 4.32 - 4.25 (m, 1H), 3.62 (s, 3H), 3.32 - 3.26 (m, 2H), 3.15 - 3.12 (m, 2H), 2.97 - 2.85 (m, 1H), 2.75 - 2.52 (m, 8H), 2.45 - 2.23 (m, 5H), 2.19 - 2.07 (m, 4H), 2.00 - 1.73 (m, 7H), 1.70 - 1.50 (m, 3H), 1.19 - 0.98 (m, 3H), 0.54 - 0.43 (m, 2H), 0.30 - 0.22 (m, 2H).

Example 115: 3-(4-(4-(1r,4r)-4-(4-(4-(5-(S)-3-aminopiperidin-1-yl)pyrazolo [1,5-a]pyrimidin-3-yl)-1H-1,2,3-triazol-1-yl)-3-(difluoromethyl)-1H-pyrazol-1-yl)cyclohexyl)methyl)piperazin-1-yl)-3-methyl-2-oxo-2,3-dihydro-1H-benzoimidazol-1-yl)piperidine-2,6-dione (115)

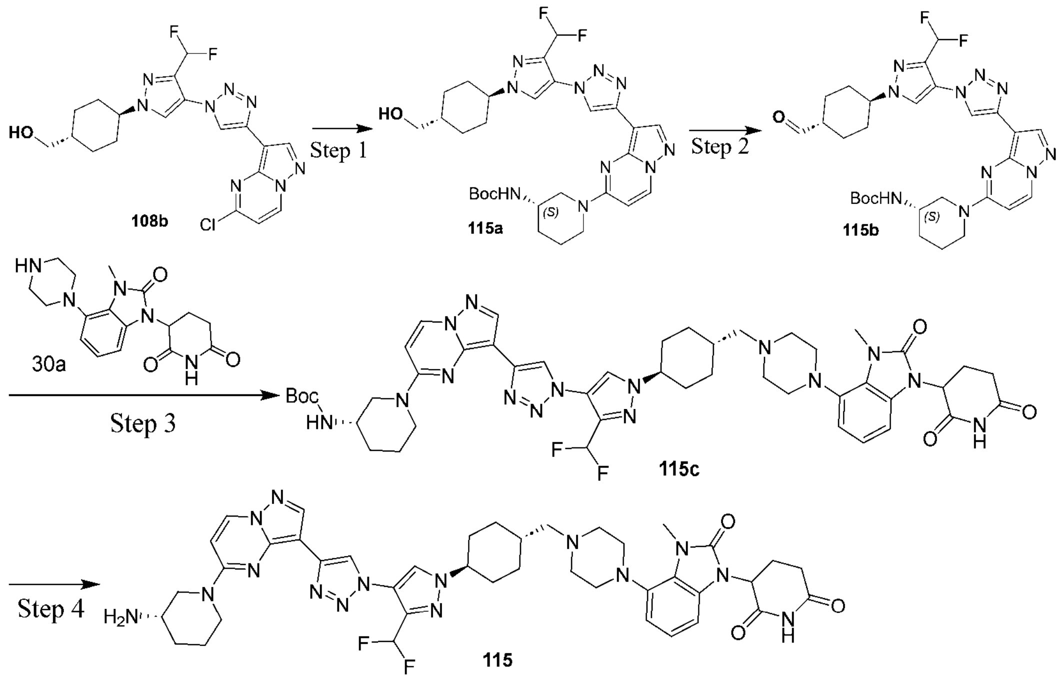


1) Step 1: *N,N*-diisopropylethylamine (1.80 mL, 10.79 mmol) was added to a solution containing compound 108b (1.00 g, 3.60 mmol), (S)-3-Boc-aminopiperidine (1.30 g, 4.31 mmol) and acetonitrile (10 mL) under nitrogen protection. The reaction was subjected to nitrogen replacement three times and then reacted at 60°C for 3 h. Water (10 mL) was added to the reaction solution, and ethyl acetate was used for extraction (20 mL × 2). The organic phases were combined, dried over anhydrous sodium sulfate and filtered to remove the desiccant, and then the filtrate was concentrated under reduced pressure. The residue was purified by silica gel column chromatography (dichloromethane/methanol = 100/1 to 30/1) to obtain compound 115a. MS m/z(ESI):613.4 [M+1]$^+$.

2) Step 2: Dess-Martin oxidant (364.0 mg, 0.86 mmol) was added to a solution containing compound 115a (1 g, 3.60 mmol) and dichloromethane (10 mL) under nitrogen protection. The reaction was subjected to nitrogen replacement three times and then reacted at 25°C for 1 h. Saturated sodium bicarbonate (20 mL) was slowly added to the reaction solution, and ethyl acetate was used for extraction (20 mL × 2). The organic phases were combined, dried over anhydrous sodium sulfate and filtered to remove the desiccant, and then the filtrate was concentrated under reduced pressure to obtain compound 115b (300 mg), which was used directly in the next step without purification. MS m/z(ESI):611.3[M+1]$^+$.

3) Step 3: Triethylamine (0.20 mL, 1.44 mmol) was added to a solution containing compound 30a (35.0 mg, 0.10 mmol), tetrahydrofuran (2 mL) and *N,N*-dimethylformamide (1 mL), and the obtained mixture was stirred for 0.5 h. Then compound 115b (63.0 mg, 0.10 mmol) and acetic acid (0.5 mL) were added to the above solution and stirred for 0.5 h. Finally, sodium borohydride acetate (65.0 mg, 0.31 mmol, Energy Chemical) was added to the above reaction solution. The reaction was also stirred at 25°C for 2 h. The reaction solution was concentrated under reduced pressure and then purified by high performance liquid chromatography (A: 0.1% FA/H2O B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 115c. MS m/z(ESI):469.8[1/2M+1]$^+$.

4) Step 4: Hydrogen chloride in dioxane (1 mL, 4 M) was added to a solution of compound 115c (10 mg, 0.02 mmol) in tetrahydrofuran (1 mL). The reaction was stirred at 25°C for 1 h. The reaction solution was concentrated under reduced pressure, and then pure water (2 mL) was added to the residue. The compound 115 was obtained after lyophilization under vacuum. MS m/z(ESI):838.5[M+1]$^+$.

Example 116: 3-(4-(4-(5-(S)-3-aminopiperidin-1-yl)pyrazolo[1,5-a]pyrimidin-3-yl)-1H-1,2,3-triazol-1-yl)-3-difluoromethyl-1H-pyrazol-1-yl)cyclohexyl)methyl) -2,8-diazaspiro[4.5]dec-2-yl)-3-methyl-2-oxo-2,3-dihydro-1H-benzoimidazol-1-yl)piperidine-2,6-dione (116)

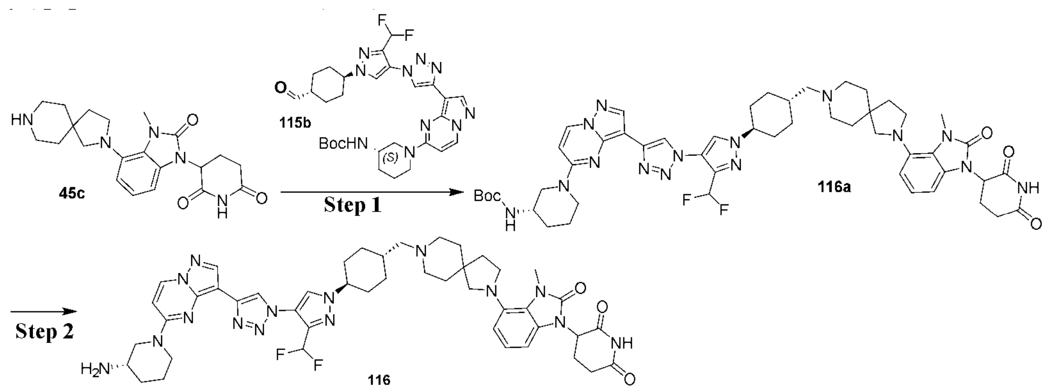


1) Step 1: Triethylamine (0.20 mL, 1.44 mmol) was added to a solution containing compound 45c (40.0 mg, 0.10 mmol), tetrahydrofuran (2 mL) and *N,N*-dimethylformamide (1 mL), and the obtained mixture was stirred for 0.5 h. Then compound 115b (62.0 mg, 0.10 mmol) and acetic acid (0.5 mL) were added to the above solution and stirred for 0.5 h. Finally, sodium borohydride acetate (64.0 mg, 0.30 mmol, Energy Chemical) was added to the above reaction solution. The reaction was also stirred at 25°C for 2 h. The reaction solution was concentrated under reduced pressure and then purified by high performance liquid chromatography (A: 0.1% FA/H2O B: ACN, chromatographic column: Waters-SunFire-C18-10μm-19×250 mm; mobile phase: water (containing 0.1% formic acid) and acetonitrile, gradient ratio: acetonitrile 30%-50%, flow rate: 25 mL/min) to obtain compound 116a. MS m/z(ESI):991.7[M-1]$^-$.

2) Step 2: Hydrogen chloride in dioxane (1 mL, 4 M) was added to a solution containing compound 116a (10 mg, 0.02 mmol) and tetrahydrofuran (1 mL). The reaction was stirred at 25°C for 1 h. The reaction solution was concentrated under reduced pressure, and then pure water (2 mL) was added to the residue. The compound 116 was obtained after lyophilization under vacuum. MS m/z(ESI):892.5[M+1]$^+$.

Example 117: 3-{4-[4-(1-{[(1S,4r)-4-[3-(difluoromethyl)-4-(4-{5-[(3S)-3-aminohexahydropyridin-1-yl]pyrazolo[1,5-a]pyrimidin-3-yl}-1,2,3-triazol-1-yl)pyrazol-1-yl]cyclohexyl]methyl}hexahydropyridin-4-yl)piperazin-1-yl]-3-methyl-2-oxobenzo[d]imidazol-1-yl}hexahydropyridine-2,6-dione hydrochloride (117)

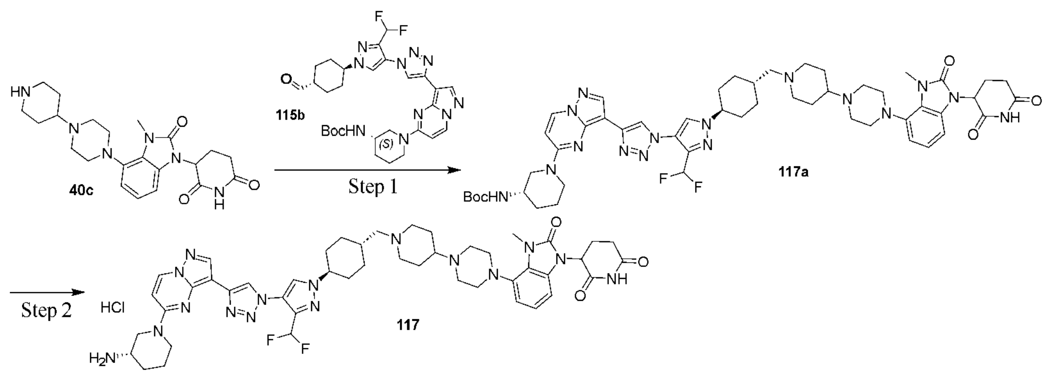


1) Step 1: Compound 40c (60.0 mg, 0.11 mmol) and triethylamine (11.0 mg, 0.11 mmol) were added to N,N-dimethylformamide (1.5 mL) and tetrahydrofuran (1.5 mL) at room temperature and the obtained mixture was stirred for 10 min. Acetic acid (14.0 mg, 0.23 mmol) and compound 115b (51.0 mg, 0.10 mmol) were added, and the reaction solution was stirred at 25°C for 0.5 h. Sodium borohydride acetate (70.0 mg, 0.33 mmol) was slowly added, and the reaction solution was stirred for 0.5 h. The solvent was removed under reduced pressure. The residue was purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 117a. MS m/z(ESI): N/A.

2) Step 2: Compound 117a (12 mg, 0.01 mmol) was added to dioxane (2 mL) at room temperature, and hydrogen chloride in dioxane (2 mL, 4 M) was added. The reaction solution was stirred for 1 h, concentrated under reduced pressure to remove the solvent. Pure water (5 mL) was added, and the obtained mixture was lyophilized under reduced pressure to obtain compound 117. MS m/z(ESI):921.6[M+1]$^+$. $^1$H NMR(400MHz, DMSO-$d_6$):δ 11.50 (s, 1H), 11.12 (s, 1H), 8.92 - 8.88 (m, 1H), 8.78 (d, $J$ = 7.8 Hz, 1H), 8.72 (s, 1H), 8.47 - 8.45 (m, 1H), 8.40 (s, 1H), 7.26 (t, $J$ = 53.5 Hz, 1H), 7.07 - 7.03 (m, 1H), 6.99 - 6.97 (m, 1H), 6.94 - 6.92 (m, 1H), 6.82 (d, $J$ = 8.0 Hz, 1H), 5.41 - 5.37 (m, 1H), 3.73 - 3.69 (m, 4H), 3.64 (s, 3H), 3.59 - 3.57 (m, 3H), 3.51 - 3.46 (m, 4H), 3.39 - 3.73 (m, 4H), 2.99 - 2.91 (m, 4H), 2.72 - 2.60 (m, 3H), 2.39 - 2.33 (m, 4H), 2.20 - 2.17 (m, 2H), 2.05 - 2.01 (m, 5H), 1.91 - 1.85 (m, 3H), 1.73 - 1.70 (m, 1H), 1.24 - 1.19 (m, 5H).

Example 118: 3-(4-(1R,4R)-4-((1r,4R)-4-(4-(4-(5-(S)-3-aminopiperidin-1-yl)pyrazolo[1,5-a]pyrimidin-3-yl)-1H-1,2,3-triazol-1-yl)-3-(difluoromethyl)-1H-pyrazol-1-yl)cyclohexyl)methyl(methyl)amino)cyclohexyl)amino)-3-methyl-2-oxo-2,3-dihydro-1H-benzoimidazol-1-yl)piperidine-2,6-dione (118)

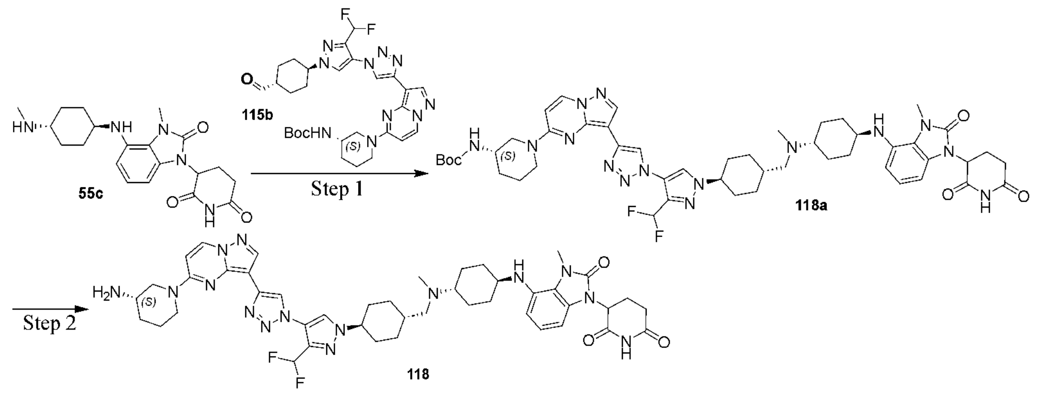


1) Step 1: Triethylamine (0.20 mL, 1.44 mmol) was added to a solution containing compound 55c (48.0 mg, 0.12 mmol), tetrahydrofuran (2 mL) and *N,N*-dimethylformamide (1 mL), and the obtained mixture was stirred for 0.5 h. Then compound 115b (50.0 mg, 0.08 mmol) and acetic acid (0.5 mL) were added to the above solution and stirred for 0.5 h. Finally, sodium borohydride acetate (54.0 mg, 0.24 mmol, Energy Chemical) was added to the above reaction solution. The reaction was also stirred at 25°C for 2 h. The reaction solution was concentrated under reduced pressure and then purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile,

gradient ratio: acetonitrile 30%-50%, flow rate: 25 mL/min) to obtain compound 118a. MS m/z(ESI):980.7[M+1]$^+$.
2) Step 2: Hydrogen chloride in dioxane (1 mL, 4 M) was added to a solution containing compound 118a (10 mg, 0.02 mmol) and tetrahydrofuran (1 mL). The reaction was stirred at 25°C for 1 h. The reaction solution was concentrated under reduced pressure, and then pure water (2 mL) was added to the residue. The compound 118 was obtained after lyophilization under vacuum. MS m/z(ESI):880.4[M+1]$^+$.

Example 119: 3-(4-(4-(5-(S)-3-aminopiperidin-1-yl)pyrazolo[1,5-a]pyrimidin-3-yl)-1H-1,2,3-triazol-1-yl)-3-difluoromethyl-1H-pyrazol-1-yl)cyclohexyl)methyl-2,8-diazaspiro[4.5]dec-8-yl)-3-methyl-2-oxo-2,3-dihydro-1H-benzoimidazol-1-yl)piperidine-2,6-dione (119)

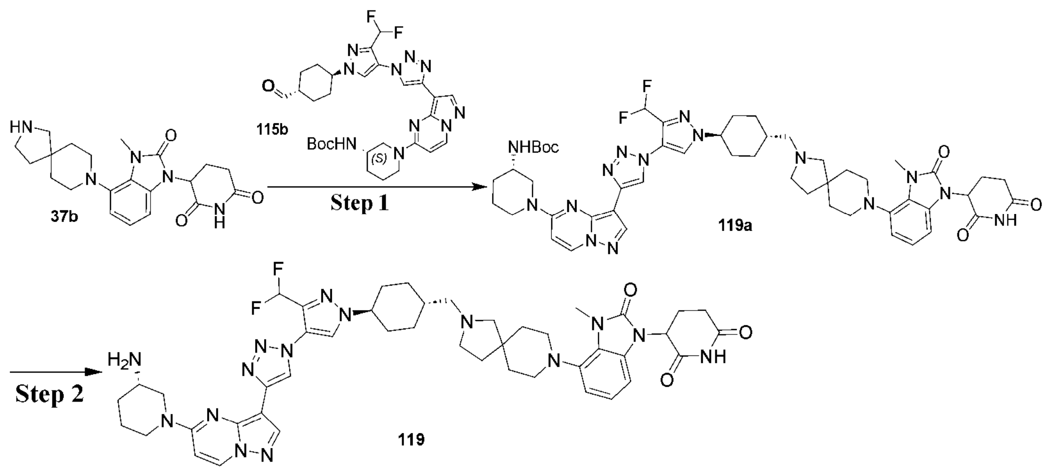


1) Step 1: Triethylamine (0.20 mL, 1.44 mmol) was added to a solution containing compound 37b (49.0 mg, 0.12 mmol), tetrahydrofuran (2 mL) and *N,N*-dimethylformamide (1 mL), and the obtained mixture was stirred for 0.5 h. Then compound 115b (50.0 mg, 0.08 mmol) and acetic acid (0.5 mL) were added to the above solution and stirred for 0.5 h. Finally, sodium borohydride acetate (52.0 mg, 0.25 mmol, Energy Chemical) was added to the above reaction solution. The reaction was also stirred at 25°C for 2 h. The reaction solution was concentrated under reduced pressure and then purified by high performance liquid chromatography (A: 0.1% FA/$H_2O$ B: ACN and acetonitrile, chromatographic column: Waters-SunFire-C18-10μm-19×250 mm; mobile phase: water (containing 0.1% formic acid) and acetonitrile, gradient ratio: acetonitrile 30%-50%, flow rate: 25 mL/min) to obtain compound 119a. MS m/z(ESI):992.7 [M+1]$^+$.
2) Step 2: Hydrogen chloride in dioxane (1 mL, 4 M) was added to a solution of compound 119a (10 mg, 0.02 mmol) in tetrahydrofuran (1 mL). The reaction was stirred at 25°C for 1 h. The reaction solution was concentrated under reduced pressure, and then pure water (2 mL) was added to the residue. The compound 119 was obtained after lyophilization under vacuum. MS m/z(ESI):892.5[M+1]$^+$. $^1$H NMR (400 MHz, DMSO-$d_6$) δ 11.11 (s, 1H), 8.86 (s, 1H), 8.79 (d, *J* = 8.0 Hz, 1H), 8.67 (s, 1H), 8.40 (s, 1H), 8.34 - 8.30 (m, 2H), 7.25 (t, *J* =53.2 Hz,1H), 7.02 - 6.95 (m, 2H), 6.91 - 6.85 (m, 1H), 6.81 (d, *J* = 8.5 Hz, 1H), 5.40 - 5.30 (m, 1H), 4.53 - 4.42 (m, 1H), 4.35 - 4.28 (m, 1H), 4.06 - 3.98 (m, 1H), 3.64 (s, 3H), 3.29 - 3.25 (m, 2H), 3.13 - 3.08 (m, 2H), 3.04 - 3.00 (m, 2H), 2.94 - 2.87 (m, 2H), 2.74 - 2.69 (m, 2H), 2.64 - 2.57 (m, 4H), 2.20 - 2.16 (m, 2H), 2.12 - 2.07 (m, 2H), 2.05 - 1.99 (m, 4H), 1.93 - 1.88 (m, 3H), 1.86 - 1.82 (m, 3H), 1.74 - 1.66 (m, 2H), 1.23 (s, 6H).

Example 120: 3-(4-(4-(5-(S)-3-aminopiperidin-1-yl)pyrazolo[1,5-a]pyrimidin-3-yl)-1H-1,2,3-triazol-1-yl)-3-(difluoromethyl)-1H-pyrazol-1-yl)cyclohexyl)methyl)-3,9-diazaspiro[5.5]undecan-3-yl)-3-methyl-2-oxo-2,3-dihydro-1H-benzoimidazol-1-yl)piperidine-2,6-dione (120)

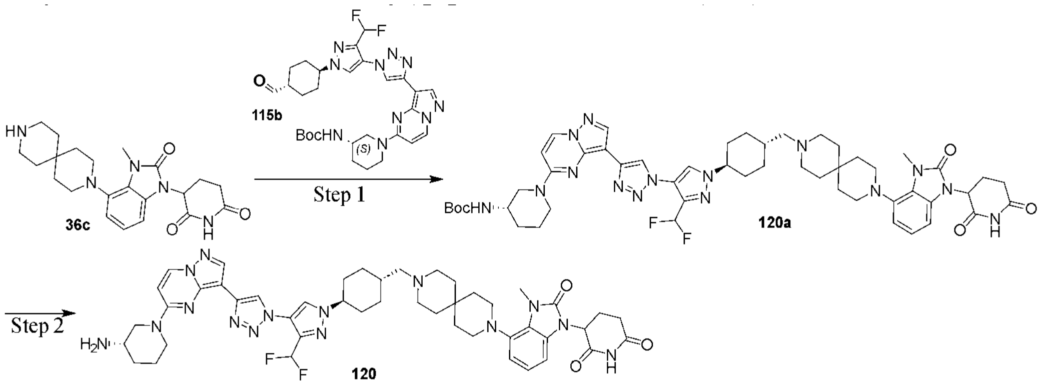


36c
115b
120a
120

1) Step 1: Triethylamine (0.20 mL, 1.44 mmol) was added to a solution of compound 36c (32.92 mg, 0.08 mmol) in tetrahydrofuran (2 mL) and *N,N*-dimethylformamide (1 mL), and the obtained mixture was stirred for 0.5 h. Then compound 115b (50.0 mg, 0.08 mmol) and acetic acid (0.5 mL) were added to the above solution and stirred for 0.5 h. Finally, sodium borohydride acetate (52.0 mg, 0.25 mmol, Energy Chemical) was added to the above reaction solution. The reaction was also stirred at 25°C for 2 h. The reaction solution was concentrated under reduced pressure and then purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 30%-50%, flow rate: 25 mL/min) to obtain compound 120a.

2) Step 2: Hydrogen chloride in dioxane (1 mL, 4 M) was added to a solution of compound 120a (15 mg, 0.02 mmol) in tetrahydrofuran (1 mL). The reaction was stirred at 25°C for 1 h. The reaction solution was concentrated under reduced pressure, and then pure water (2 mL) was added to the residue. The compound 120 was obtained after lyophilization under vacuum. MS m/z(ESI):906.5[M+1]$^+$. $^1$H NMR (400 MHz, DMSO-$d_6$) δ 11.11 (s, 1H), 8.91 (s, 1H), 8.78 (d, *J*= 7.9 Hz, 1H), 8.70 (s, 1H), 8.46 - 8.41 (m, 2H), 8.40 (s, 1H), 7.19 (t, *J*= 53.5 Hz, 1H), 7.02 - 6.95 (m, 2H), 6.91 - 6.86 (m, 1H), 6.84 - 6.79 (m, 1H), 5.40 - 5.30 (m, 1H), 4.62 - 4.46 (m, 1H), 4.36 - 4.25 (m, 1H), 4.08 - 3.98 (m, 1H), 3.64 (s, 3H), 3.41 - 3.36 (m, 4H), 3.07 - 2.99 (m, 4H), 2.94 - 2.89 (m, 4H), 2.71 - 2.63 (m, 3H), 2.20 - 2.15 (m, 3H), 2.06 - 2.00 (m, 4H), 1.99 - 1.91 (m, 4H), 1.89 - 1.83 (m, 2H), 1.77 - 1.72 (m, 2H), 1.71 - 1.65 (m, 2H), 1.62 - 1.54 (m, 2H), 1.52 - 1.36 (m, 4H).

Example 121: 3-(4-(9-(((1r,4r)-4-(3-(difluoromethyl)-4-(4-(5-(4-hydroxy-4-methylpiperidin-1-yl)pyrazolo[1,5-a]pyrimidin-3-yl)-1H-1,2,3-triazol-1-yl)-1H-pyrazol-1-yl(cyclohexyl)methyl)-3,9-diazaspiro[5.5]undecan-3-yl)-3-methyl-2-oxo-2,3-dihydro-1H-benzo[d]imidazol-1-yl)piperidine-2,6-dione (121)

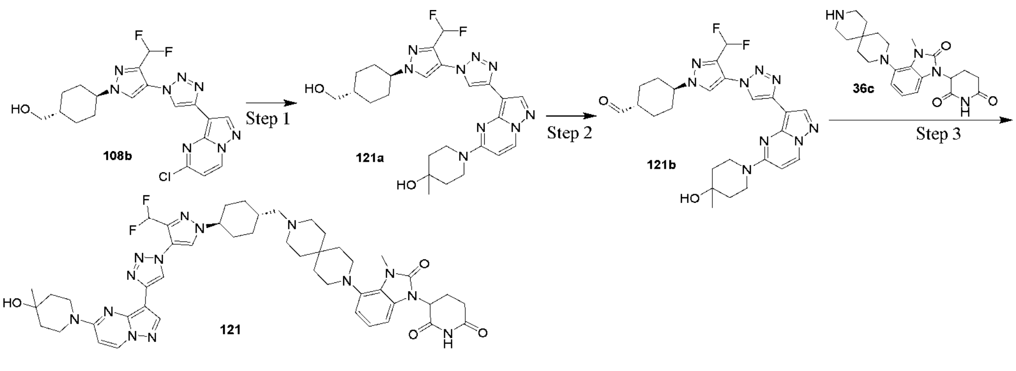


108b
121a
121b
36c
121

1) Step 1: Compound 108b (600 mg, 1.34 mmol), 4-methyl-4-hydroxypiperidine (231 mg, 2.01 mmol) and acetonitrile (10 mL) were added to a single-neck bottle at room temperature in sequence, and *N,N*-diisopropylethylamine (332 μL, 2.01 mmol) was added under stirring, and the reaction system was subjected to nitrogen replacement three times, and reacted at 80°C for 2 h. The reaction solution was concentrated to obtain a residue. The residue was purified by silica gel column chromatography with an eluent system (eluent: methanol/dichloromethane = 0%-10%) to obtain compound 121a. MS m/z(ESI):528.2[M+1]$^+$.

2) Step 2: Compound 121a (310 mg, 0.590 mmol) was added to a single-neck bottle at room temperature in sequence, and dichloromethane (2 mL) and Dess-Martin oxidant (374 mg, 0.880 mmol) were added, and the obtained mixture

was reacted at 25°C for 2 h. Water (50 mL) was added to the reaction solution, and dichloromethane was used for extraction (30 mL × 3). The organic layers were combined, washed with saturated brine (30 mL), dried over anhydrous sodium sulfate, filtered and concentrated to obtain compound 121b. MS m/z(ESI):526.2[M+1]$^+$.

3) Step 3: Compound 36c (50.0 mg, 80.0 μmol), DMF (1 mL) and tetrahydrofuran (1.5 mL) were added to a single-neck bottle at room temperature in sequence, triethylamine (26.4 μL, 190 μmol) was added under stirring, and the obtained mixture was reacted at 0°C for 10 min. Then compound 121b (40.0 mg, 80.0 μmol), sodium borohydride acetate (96.3 mg, 460 μmol) and acetic acid (26.1 μL, 460 μmol) were added in sequence, and the obtained mixture was reacted at 25°C for 2 h. Most of the solvent was removed from the reaction solution under vacuum to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 17%-95%, flow rate: 25 mL/min) to obtain compound 121. MS m/z(ESI):921.4[M+1]$^+$. $^1$H NMR (400 MHz, DMSO-$d_6$) δ 11.11 (s, 1H), 8.68 (t, *J* = 4.0 Hz, 2H), 8.53 (s, 1H), 8.34 (s, 1H), 7.22 (t, *J* = 53.2 Hz, 1H), 6.97 (d, *J* = 5.6 Hz, 2H), 6.87 (d, *J* = 5.6 Hz, 1H), 6.82 (d, *J* = 7.9 Hz, 1H), 5.35 (dd, *J* = 12.6, 5.4 Hz, 1H), 4.47 (s, 1H), 4.34 - 4.24 (m, 1H), 4.14 (s, 2H), 3.63 (s, 3H), 3.50 - 3.43 (m, 3H), 2.96 - 2.81 (m, 5H), 2.74 - 2.58 (m, 2H), 2.45 - 2.30 (m, 4H), 2.23 - 2.08 (m, 4H), 2.04 - 1.89 (m, 3H), 1.89 - 1.78 (m, 2H), 1.71 - 1.40 (m, 12H), 1.16 (s, 3H), 1.13 - 1.01 (m, 2H).

Example 122: 3-(4-(4-(-4-(((1S,4r)-4-(3-(difluoromethyl)-4-(4-(5-((((1S,2R)-2-hydroxycyclohexyl)amino)pyrazolo[1,5-a]pyrimidin-3-yl)-1H-1,2,3-triazol-1-yl)-1H-pyrazol-1-yl(cyclohexyl)methyl)piperazin-1-yl)piperidin-1-yl)-3-methyl-2-oxo-2,3-dihydro-1H-benzo[d]imidazol-1-yl)pyridine-2,6-dione (122)

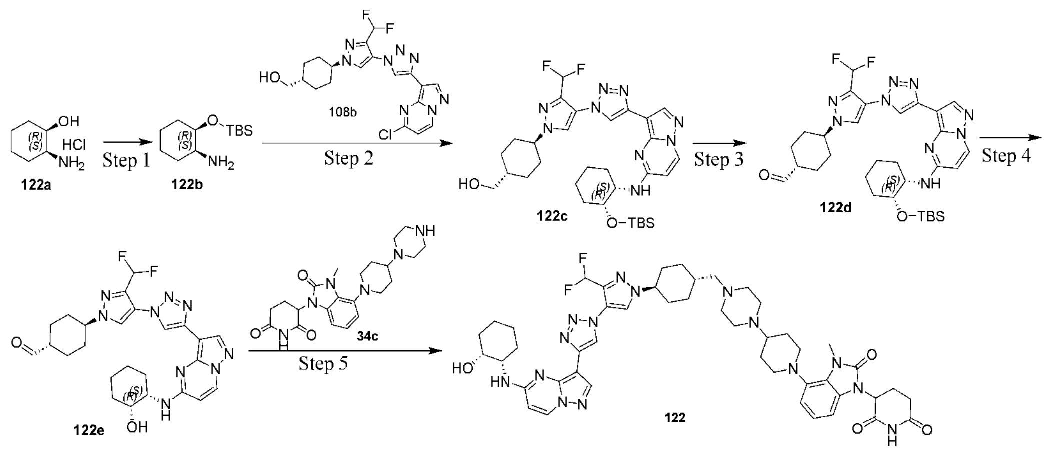


1) Step 1: Compound 122a (5.00 g, 33.0 mmol, Bide Pharmatech Ltd.), 4-dimethylaminopyridine (0.400 g, 3.30 mmol, Bide Pharmatech Ltd.) and imidazole (5.61 g, 82.4 mmol, Bide Pharmatech Ltd.) were dissolved in dichloromethane (50 mL). Tert-butyldimethylsilyl chloride (5.96 g, 39.6 mmol, Bide Pharmatech Ltd.) was added in portions at 25°C. The reaction solution was stirred at 25°C for 12 h. The reaction solution was poured into water (100 mL), and the aqueous phase was extracted with dichloromethane (100 mL × 2). The combined organic phase was washed with saturated brine (100 mL), dried over anhydrous sodium sulfate, and filtered to remove the desiccant, and then the filtrate was concentrated under reduced pressure. The residue was purified by silica gel column chromatography (dichloromethane/methanol = 100/1 to 20/1) to obtain compound 122b. MS m/z(ESI):230.6[M+H]$^+$. $^1$H NMR (400 MHz, DMSO-$d_6$): δ 3.76 - 3.70 (m, 1H), 2.60 - 2.55 (m, 1H), 1.68 - 1.60 (m, 1H), 1.57 - 1.50 (m, 1H), 1.47 - 1.31 (m, 4H), 1.28 - 1.16 (m, 2H), 0.88 (s, 9H), 0.04 (d, *J*= 3.7 Hz, 6H).

2) Step 2: Compounds 108b (800 mg, 1.78 mmol) and compound 122b (532 mg, 2.32 mmol) were dissolved in acetonitrile (15 mL), and *N,N*-diisopropylethylamine (0.9 mL, 5.35 mmol, Bide Pharmatech Ltd.) was added. The reaction solution was stirred at 80°C for 12 h. The reaction solution was concentrated under reduced pressure, and the residue was purified by silica gel column chromatography (dichloromethane/methanol = 100/1 to 40/1) to obtain compound 122c. MS m/z(ESI):642.3[M+H]$^+$. $^1$H NMR (400 MHz, $CDCl_3$) δ 8.59 - 8.54 (m, 1H), 8.32 - 8.16 (m, 1H), 8.11 (s, 1H), 7.54 - 7.27 (m, 1H), 6.10 - 5.79 (m, 1H), 5.20 - 4.87 (m, 1H), 4.26 - 4.04 (m, 3H), 3.56 (d, *J* = 6.2 Hz, 2H), 2.33 (d, *J* = 12.6 Hz, 2H), 2.08 - 2.03 (m, 2H), 1.89 - 1.74 (m, 6H), 1.46 - 1.41 (m, 2H), 1.30 - 1.24 (m, 5H), 0.96 - 0.93 (m, 9H), 0.08 - 0.02 (m, 6H).

3) Step 3: Compound 122c (375 mg, 0.580 mmol) was dissolved in dichloromethane (10 mL), and Dess-Martin oxidant (372 mg, 0.880 mmol, Bide Pharmatech Ltd.) was added. The reaction solution was stirred at 25°C for 1 h. The

reaction solution was quenched with saturated sodium bicarbonate aqueous solution (10 mL) and saturated sodium sulfite aqueous solution (10 mL), and the aqueous phase was extracted with dichloromethane (100 mL × 3). The combined organic phase was dried over anhydrous sodium sulfate and filtered to remove the desiccant, and then the filtrate was concentrated under reduced pressure to obtain compound 122d. MS m/z(ESI):640.4[M+H]$^+$. $^1$H NMR (400 MHz, $CDCl_3$) δ 9.73 (s, 1H), 8.56 (s, 1H), 8.51 (s, 1H), 8.21 (d, *J* = 7.6 Hz, 1H), 8.11 (s, 1H), 6.98 - 6.71 (m, 1H), 5.96 (d, *J* = 7.5 Hz, 1H), 5.19 - 4.94 (m, 1H), 4.25 - 4.13 (m, 2H), 4.12 - 4.09 (m, 1H), 2.42 - 2.35 (m, 3H), 2.31 - 2.25 (m, 2H), 1.95 - 1.86 (m, 2H), 1.83 - 1.68 (m, 5H), 1.55 - 1.40 (m, 5H), 0.95 (s, 9H), 0.07 (s, 3H), 0.03 (s, 3H).

4) Step 4: Tetrabutylammonium fluoride (0.11 mL, 1.0 M solution in tetrahydrofuran) was added to a solution of compound 122d (70.0 mg, 0.110 mmol) in tetrahydrofuran (1.5 mL), and the mixture was stirred at 25°C under nitrogen atmosphere for 8 h. The reaction solution was poured into water and ethyl acetate (20 mL × 3) was used for extraction. The organic phases were combined and dried over anhydrous sodium sulfate, filtered and spin-dried to obtain compound 122e. MS m/z(ESI): 526.2[M+1]$^+$.

5) Step 5: Compound 34c (50.0 mg, 0.100 mmol) was dissolved in *N,N*-dimethylformamide (1 mL) and tetrahydrofuran (1 mL) at 0°C, triethylamine (29.0 mg, 0.290 mmol) was added, and the obtained mixture was stirred for 10 min. Then sodium borohydride acetate (122 mg, 0.580 mmol), compound 122e (50.0 mg, 0.100 mmol) and acetic acid (29.0 mg, 0.480 mmol) were added to the mixture, and the mixture was stirred at 25°C for 1 h. The mixture was concentrated to obtain a residue. The reaction solution was concentrated under reduced pressure and then purified by high performance liquid chromatography (Gilson_306_1741, chromatographic column: XTIMATE-C18; mobile phase: water (containing 26.5 mmol/L formic acid) and acetonitrile, gradient ratio: acetonitrile 19%-95%, flow rate: 25 mL/min) to obtain compound 122. MS m/z(ESI): 936.6[M+1]$^+$. $^1$H NMR (400 MHz, DMSO-$d_6$) δ 11.10 (s, 1H), 8.66 (s, 1H), 8.49 (d, *J* = 7.6 Hz, 1H), 8.43 (s, 1H), 8.26 (s, 1H), 7.49 (d, *J* = 7.3 Hz, 1H), 7.22 (t, *J* = 53.1 Hz, 1H), 7.00 - 6.94 (m, 1H), 6.92 - 6.83 (m, 2H), 6.49 (d, *J* = 7.6 Hz, 1H), 5.42 - 5.30 (m, 1H), 4.73 (s, 1H), 4.29 (t, *J* = 11.8 Hz, 1H), 4.09 (s, 1H), 3.95 (s, 1H), 3.62 (s, 3H), 3.14 (d, *J* = 11.1 Hz, 2H), 2.94 - 2.83 (m, 1H), 2.76 - 2.52 (m, 7H), 2.42 - 2.22 (m, 5H), 2.18 - 2.09 (m, 4H), 2.02 - 1.67 (m, 10H), 1.65 - 1.49 (m, 7H), 1.39 - 1.27 (m, 2H), 1.14 - 1.00 (m, 2H).

Example 123: 3-(4-(8-(((1r,4r)-4-(3-(difluoromethyl)-4-(4-(5-(4-hydroxy-4-methylpiperidin-1-yl)pyrazolo[1,5-a]pyrimidin-3-yl)-1H-1,2,3-triazol-1-yl)-1H-pyrazol-1-yl(cyclohexyl)methyl)-2,8-diazaspiro[4.5]dec-2-yl)-3-methyl-2-oxo-2,3-dihydro-1H-benzo[d]imidazol-1-yl)piperidine-2,6-dione (123)

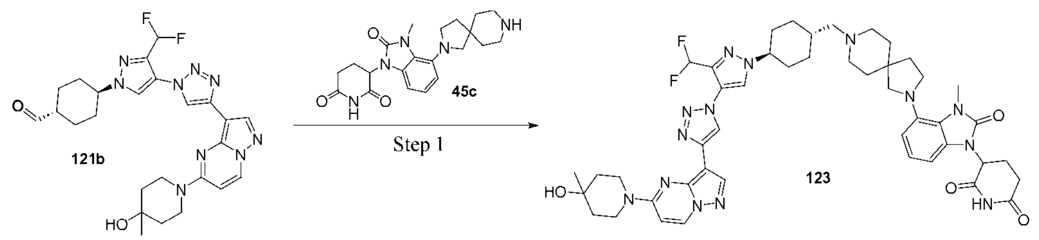


Compound 45c (70.0 mg, 0.110 mmol) was dissolved in *N,N*-dimethylformamide (1 mL) and tetrahydrofuran (1 mL) at 0°C, triethylamine (33.0 mg, 0.330 mmol) was added, and the obtained mixture was stirred for 10 min. Then sodium borohydride acetate (139 mg, 0.660 mmol), compound 121b (60.0 mg, 0.110 mmol) and acetic acid (33.0 mg, 0.550 mmol) were added to the mixture, and the mixture was stirred at 25°C for 1 h. The mixture was concentrated to obtain a residue. The reaction solution was concentrated under reduced pressure and then purified by high performance liquid chromatography (Gilson _306_1741, chromatographic column: Waters-SunFire-C18-10μm-19×250 mm; mobile phase: water (containing 26.5 mmol/L formic acid) and acetonitrile, gradient ratio: acetonitrile 19%-95%, flow rate: 25 mL/min) to obtain compound 123. MS m/z(ESI): 907.6[M+1]$^+$. $^1$H NMR (400 MHz, DMSO-$d_6$) δ 11.10 (s, 1H), 8.72 - 8.61 (m, 2H), 8.52 (s, 1H), 8.33 (s, 1H), 7.22 (t, *J* = 53.2 Hz, 1H), 7.02 - 6.91 (m, 2H), 6.89 - 6.75 (m, 2H), 5.41 - 5.29 (m, 1H), 4.46 (s, 1H), 4.32 - 4.25 (m, 1H), 4.13 (s, 1H), 3.59 (s, 3H), 3.52 - 3.42 (m, 3H), 3.06 (t, *J* = 6.8 Hz, 2H), 2.98 - 2.83 (m, 3H), 2.73 - 2.58 (m, 2H), 2.44 - 2.20 (m, 4H), 2.13 (d, *J* = 7.3 Hz, 4H), 2.04 - 1.89 (m, 3H), 1.86 - 1.71 (m, 4H), 1.69 - 1.46 (m, 9H), 1.15 (s, 3H), 1.12 - 1.01 (m, 2H).

Example 124: 3-(4-(4-(((1r,4r)-4-(3-(difluoromethyl)-4-(4-(5-(4-hydroxy-4-methylpiperidin-1-yl)pyrazolo[1,5-a]pyrimidin-3-yl)-1H-1,2,3-triazol-1-yl)-1H-pyrazol-1-yl(cyclohexyl)methyl)piperazin-1-yl)piperidin-1-yl)-3-methyl-2-oxo-2,3-dihydro-1H-benzo[d]imidazol-1-yl)pyridine-2,6-dione (124)

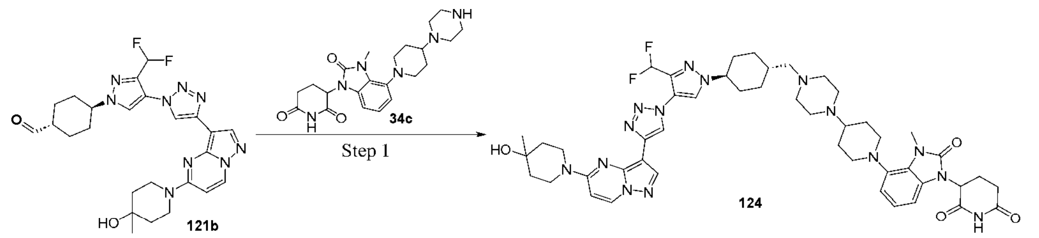

34c
121b
124

Compound 34c (70.0 mg, 100 μmol), DMF (1 mL) and tetrahydrofuran (1.5 mL) were added to a single-neck bottle at room temperature in sequence, triethylamine (35.9 μL, 260 μmol) was added under stirring, and the obtained mixture was reacted at 0°C for 10 min. Then compound 121b (54.5 mg, 100 μmol), sodium borohydride acetate (131 mg, 620 μmol) and acetic acid (35.6 μL, 620 μmol) were added in sequence, and the obtained mixture was reacted at 25°C for 2 h. Most of the solvent was removed from the reaction solution under vacuum to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 2%-95%, flow rate: 25 mL/min) to obtain compound 124. MS m/z(ESI):936.4[M+1]$^+$. $^1$H NMR (400 MHz, DMSO-$d_6$) δ 11.10 (s, 1H), 8.67 (t, *J* = 4.0 Hz, 2H), 8.52 (s, 1H), 8.33 (s, 1H), 7.22 (t, *J* = 53.2 Hz, 1H), 6.97 (t, *J* = 7.9 Hz, 1H), 6.92 - 6.84 (m, 2H), 6.82 (d, *J* = 8.0 Hz, 1H), 5.37 - 5.32 (m, 1H), 4.46 (s, 1H), 4.33 - 4.24 (m, 1H), 4.22 - 4.01 (m, 2H), 3.62 (s, 3H), 3.49 - 3.43 (m, 3H), 3.14 (d, *J* = 11.1 Hz, 2H), 2.92 - 2.83 (m, 1H), 2.75 - 2.62 (m, 4H), 2.60 - 2.54 (m, 3H), 2.46 - 2.25 (m, 5H), 2.13 (d, *J* = 7.8 Hz, 4H), 2.01 - 1.77 (m, 7H), 1.69 - 1.45 (m, 7H), 1.16 (s, 3H), 1.08 - 1.02 (m, 2H).

Example 125: 3-(4-(4-(((1r,4r)-4-(3-(difluoromethyl)-4-(4-(5-((3-hydroxy-3-methylbutyl)amino)pyrazolo[1,5-a]pyrimidin-3-yl)-1H-1,2,3-triazol-1-yl)-1H-pyrazol-1-yl(cyclohexyl)methyl)piperazin-1-yl)piperidin-1-yl)-3-methyl-2-oxo-2,3-dihydro-1H-benzo[d]imidazol-1-yl)pyridine-2,6-dione (125)

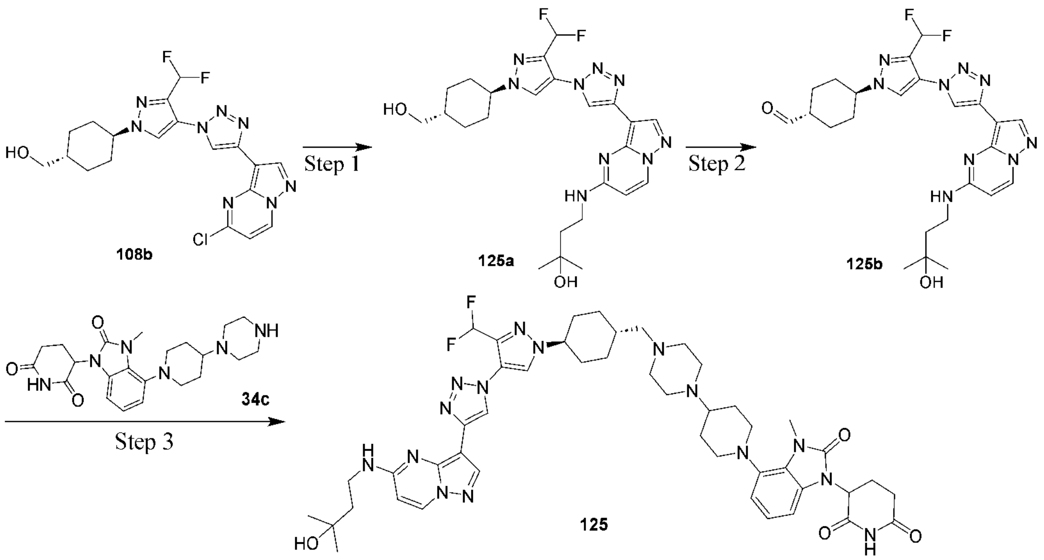

108b
125a
125b
34c
125

1) Step 1: Compound 108b (1.00 g, 2.33 mmol) was dissolved in acetonitrile (50 mL), and 4-amino-2-methylbutan-2-ol (460 mg, 4.46 mmol, Bide Pharmatech Ltd.) and *N,N*- diisopropylethylamine (1.11 mL, 6.68 mmol) were added, and the obtained mixture was reacted under stirring at 80°C for 2 h. Then the reaction system was added to water (200 mL), and extracted with ethyl acetate (100 mL×3). The organic phases were combined, dried over anhydrous sodium sulfate and filtered to remove the desiccant, and then the filtrate was concentrated under reduced pressure. The residue was purified by slurrying with (petroleum ether/ethyl acetate = 1/1) to obtain compound 125a. MS m/z(ESI):516.2[M+1]$^+$.

2) Step 2: Compound 125a (950 mg, 1.84 mmol) was dissolved in a mixed solution of *N*,*N*-dimethylformamide (8 mL) and dichloromethane (30 mL). Dess-Martin oxidant (1.56 g, 3.69 mmol) was added and the obtained mixture was reacted under stirring at room temperature for 1 h. The reaction system was added to a saturated sodium bicarbonate solution (300 mL) and extracted with dichloromethane (100 mL × 3). The organic phases were combined, dried over

anhydrous sodium sulfate and filtered to remove the desiccant, and then the filtrate was concentrated under reduced pressure to obtain compound 125b, the residue was used directly as the raw material for the next step without purification. MS m/z(ESI):514.3[M+1]$^+$.

3) Step 3: Compound 34c (83.1 mg, 0.19 mmoL) was dissolved in a mixed solution of N,N-dimethylformamide (1 mL) and tetrahydrofuran (4 mL), triethylamine (9.85 mg, 0.10 mmoL) was added, and the obtained mixture was reacted under stirring at room temperature for 15 min. Then compound 125b (50.0 mg, 0.10 mmoL) and acetic acid (11.2 mg, 0.19 mmoL) were added and the obtained mixture was reacted under stirring at room temperature for 30 min. Sodium triacetoxyborohydride (61.6 mg, 0.29 mmoL) was then added, and the obtained mixture was reacted under stirring at room temperature for 30 min. The reaction system was then directly concentrated, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 125. MS m/z(ESI):924.7M+1]$^+$. $^1$H NMR (400 MHz, DMSO-$d_6$): δ 11.10 (s, 1H), 8.65 (s, 1H), 8.51 (s, 1H), 8.27 (s, 1H), 7.75 - 7.66 (m, 1H), 7.27 (t, $J$ = 53.3 Hz, 1H), 6.99 - 6.94 (m, 1H), 6.91 - 6.85 (m, 2H), 6.29 (d, $J$ = 7.6 Hz, 1H), 5.41 - 5.30 (m, 1H), 4.52 - 4.22 (m, 2H), 3.62 (s, 3H), 3.52 - 3.45 (m, 4H), 3.18 - 3.09 (m, 3H), 2.91 - 2.84 (m, 1H), 2.73 - 2.63 (m, 4H), 2.60 - 2.53 (m, 4H), 2.39 - 2.28 (m, 4H), 2.17 - 2.10 (m, 4H), 1.95 - 1.86 (m, 4H), 1.83 - 1.76 (m, 2H), 1.74 - 1.69 (m, 2H), 1.65 - 1.55 (m, 3H), 1.13 (s, 6H), 1.11 - 1.03 (m, 2H).

Example 126: 3-(4-(9-(((1r,4r)-4-(3-(difluoromethyl)-4-(4-(5-((3-hydroxy-3-methylbutyl)amino)pyrazolo[1,5-a]pyrimidin-3-yl)-1H-1,2,3-triazol-1-yl)-1H-pyrazol-1-yl(1H-3,9-diazaspiro[5.5]undecan-3-yl)-3-methyl-2-oxo-2,3-dihydro-1H-benzo[d]imidazol-1-yl)piperidine-2,6-dione (126)

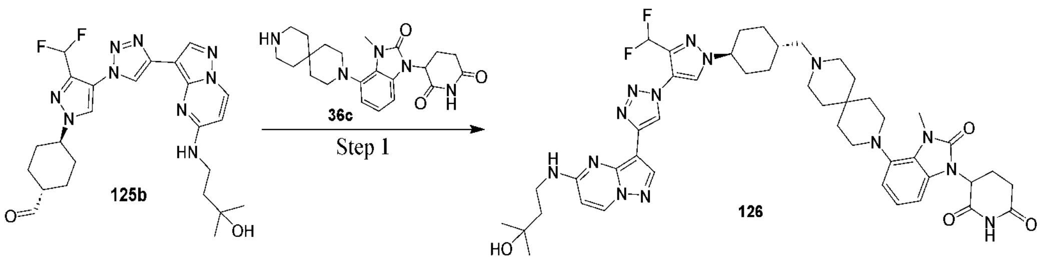


Compound 36c (41.2 mg, 0.10 mmoL) was dissolved in a mixed solution of N,N-dimethylformamide (1 mL) and tetrahydrofuran (4 mL), triethylamine (9.85 mg, 0.10 mmoL) was added, and the obtained mixture was reacted under stirring at room temperature for 15 min. Then compound 125b (50.0 mg, 0.10 mmoL) and acetic acid (11.7 mg, 0.19 mmoL) were added and the obtained mixture was reacted under stirring at room temperature for 30 min. Sodium triacetoxyborohydride (61.6 mg, 0.29 mmoL) was then added, and the obtained mixture was reacted under stirring at room temperature for 30 min. The reaction system was then directly concentrated, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 126. MS m/z(ESI):909.7[M+1]$^+$. $^1$H NMR (400 MHz, DMSO-$d_6$): δ 11.10 (s, 1H), 8.65 (s, 1H), 8.51 (s, 1H), 8.28 (s, 1H), 7.78 - 7.63 (m, 1H), 7.28 (t, $J$ = 53.3 Hz, 1H), 6.99 - 6.95 (m, 2H), 6.89 - 6.84 (m, 1H), 6.29 (d, $J$ = 7.6 Hz, 1H), 5.38 - 5.31 (m, 1H), 4.46 - 4.25 (m, 2H), 3.63 (s, 3H), 3.52 - 3.45 (m, 4H), 2.93 - 2.83 (m, 6H), 2.72 - 2.58 (m, 5H), 2.39 - 2.33 (m, 4H), 2.19 - 2.12 (m, 4H), 2.02 - 1.89 (m, 4H), 1.84 - 1.78 (m, 2H), 1.74 - 1.69 (m, 3H), 1.13 (s, 6H), 1.11 - 1.04 (m, 2H).

Example 127: 3-(4-(4-(4-(((1r,4r)-4-(3-(difluoromethyl)-4-(4-(5-(piperazin-1-yl))pyrazolo[1,5-a]pyrimidin-3-yl)-1H-1,2,3-triazol-1-yl)-1H-pyrazol-1-yl)cyclohexyl)methyl)piperazin-1-yl)piperidin-1-yl)-3-methyl-2-oxo-2,3-dihydro-1H-benzo[d] imidazol-1-yl)piperidine-2,6-dione dihydrochloride (127)

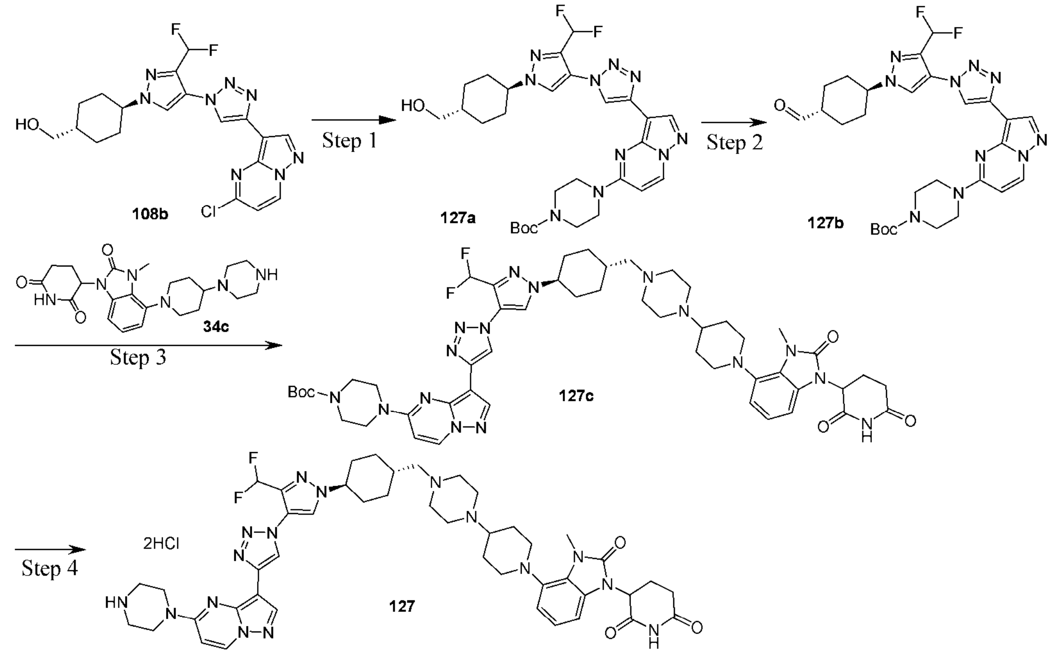


1) Step 1: Compound 108b (900 mg, 2.01 mmol), tert-butylpiperazine-1-carboxylate (560 mg, 3.01 mmol) and N,N-diisopropylethylamine (1.00 mL, 6.01 mmol) were added to acetonitrile (10 mL) at room temperature and the reaction was stirred at room temperature for 5 h. The reaction solution was added to water (30 mL) and dichloromethane (10 mL $\times$ 3) was used for extraction. The organic phase was washed with saturated brine (10 mL) and dried over anhydrous sodium sulfate, and the solvent was removed under reduced pressure to obtain compound 127a, which can be used directly in the next step without purification. MS m/z(ESI):599.0[M+1]$^+$.

2) Step 2: Compound 127a (700 mg, 1.17 mmol) and Dess-Martin oxidant (992 mg, 2.34 mmol) were added to dichloromethane (20 mL) at room temperature, and the reaction solution was stirred for 2 h. The reaction solution was filtered through diatomaceous earth, and washed with dichloromethane (10 mL $\times$ 2), and the filtrate was concentrated under reduced pressure. The residue was added with dichloromethane (8 mL), stirred and filtered, and the filtrate was washed with saturated sodium bicarbonate aqueous solution (20 mL) and saturated brine (20 mL), respectively, and dried over anhydrous sodium sulfate, and the solvent was removed under reduced pressure to obtain compound 127b, the residue can be directly used in the next reaction without purification.. MS m/z(ESI): 597.3[M+1]$^+$.

3) Step 3: Compound 34c (60.0 mg, 0.14 mmol) and triethylamine (14.0 mg, 0.14 mmol) were added to N,N-dimethylformamide (4 mL) at room temperature, and the obtained mixture was stirred for 10 min. Acetic acid (17.0 mg, 0.28 mmol) and compound 127b (84.0 mg, 0.14 mmol) were added, and the reaction solution was stirred at 25°C for 15 min. Sodium borohydride acetate (89.0 mg, 0.42 mmol) was slowly added, and the reaction solution was stirred for 20 min. The solvent was removed under reduced pressure, and the residue was purified by high performance liquid chromatography (Gilson_306_1741, chromatographic column: Waters-SunFire-C18-10um-19*250 mm; mobile phase: water (containing 0.1% formic acid) and acetonitrile, gradient ratio: acetonitrile 10%-90%, flow rate: 25 mL/min) to obtain compound 127c. MS m/z(ESI): 504.7[M/2+1]$^+$.

4) Step 4: Compound 127c (20.0 mg, 0.02 mmol) was added to tetrahydrofuran (2 mL) at room temperature, and hydrogen chloride in dioxane (2 mL, 4.0 M) was added, and the reaction solution was stirred for 1 h. The reaction solution was concentrated under reduced pressure to remove the solvent, and pure water (5 mL) was added and the obtained mixture was freeze-dried to obtain compound 127. MS m/z(ESI): 907.7[M+1]$^+$. $^1$H NMR (400 MHz, DMSO-$d_6$) $\delta$ 11.11 (s, 1H), 9.40(s, 2H), 8.85 (d, $J$ = 7.8 Hz, 1H), 8.70 (s, 1H), 8.59 (s, 1H), 8.42 (s, 1H), 7.23 (t, $J$ = 53.2 Hz, 1H), 7.04 - 6.97 (m, 1H), 6.93 - 6.91 (m, 2H), 6.89 - 6..87 (m, 1H), 5.40 - 5.36 (m, 1H), 4.36 - 4.31 (m, 1H), 4.03 - 4.01 (m, 4H), 3.80 - 3.72 (m, 5H), 3.64 (s, 3H), 3.42 - 3.36 (m, 6H), 3.26 - 3.22 (m, 6H), 2.90 - 2.86 (m, 1H), 2.78 - 2.75 (m, 2H), 2.68 - 2.60 (m, 2H), 2.18 - 2.14 (m, 6H), 2.01 - 1.85 (m, 6H), 1.23 - 1.17 (m, 3H).

Example 128: 3-(4-(2-(((1r,4r)-4-(3-(difluoromethyl)-4-(4-(5-(piperazin-1-yl)pyrazolo[1,5-a]pyrimidin-3-yl)-1H-1,2,3-triazol-1-yl)-1H-pyrazol-1-yl)cyclohexyl)methyl)-2,8-diazaspiro[4.5]dec-8-yl)-3-methyl-2-oxo-2,3-dihydro-1H-benzo[d]imidazol-1-yl)piperidine-2,6-dione dihydrochloride (128)

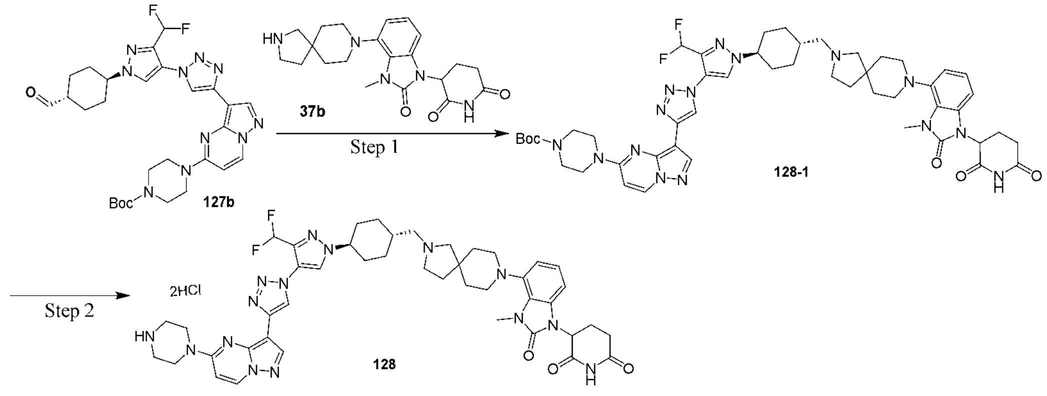


1) Step 1: Compound 37b (60.0 mg, 151 μmol) and triethylamine (15.0 mg, 148 μmol) were added to N,N-dimethylformamide (4 mL) at room temperature, and the obtained mixture was stirred for 10 min. Acetic acid (18.0 mg, 300 μmol) and compound 127b (90.0 mg, 151 μmol) were added, and the reaction solution was stirred at 25°C for 15 min. Sodium borohydride acetate (96.0 mg, 455 μmol) was slowly added, and the reaction solution was stirred for 20 min. The reaction solution was added to water (10 mL) and ethyl acetate (3 mL × 3) was used for extraction. The organic phase was washed with saturated brine (10 mL), dried over anhydrous sodium sulfate and filtered, and the solvent was removed under reduced pressure. The residue was purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 128-1 (15 mg). MS m/z(ESI):979.2[M+1]$^+$.

2) Step 2: Compound 128-1 (15.0 mg, 15.2 μmol) was added to tetrahydrofuran (2 mL) at room temperature, and hydrogen chloride/dioxane (2 mL, 4 M) was added, and the reaction solution was stirred at room temperature for 1 h. The reaction solution was concentrated under reduced pressure to remove the solvent, and pure water (5 mL) was added, and the obtained mixture was lyophilized under reduced pressure. Compound 128 was obtained. MS m/z(ESI):878.6[M+1]$^+$. $^1$H NMR (400 MHz, DMSO-$d_6$) δ 11.11 (s, 1H), 9.32 - 9.17 (m, 2H), 8.86 (d, $J$ = 7.7 Hz, 1H), 8.70 (s, 1H), 8.58 (s, 1H), 8.42 (s, 1H), 7.22 (t, $J$ = 53.2 Hz, 1H), 7.02 - 6.94 (m, 2H), 6.90 - 6.87 (m, 2H), 5.39 - 5.34 (m, 1H), 4.36 - 4.33 (m, 1H), 4.01 (s, 4H), 3.64 (s, 3H), 3.22 (s, 6H), 3.11 - 3.03 (m, 5H), 2.93 - 2.85 (m, 2H), 2.71 - 2.60 (m, 5H), 2.18 - 2.15 (m, 3H), 2.01 - 1.88 (m, 9H), 1.28 - 1.18 (m, 4H).

Example 129: 3-(4-(9-(((1r,4r)-4-(3-(difluoromethyl)-4-(4-(5-(piperazin-1-yl)pyrazolo[1,5-a]pyrimidin-3-yl)-1H-1,2,3-triazol-1-yl)-1H-pyrazol-1-yl)cyclohexyl)methyl)-3,9-diazaspiro[5.5]undecan-3-yl)-3-methyl-2-oxo-2,3-dihydro-1H-benzo[d]imidazol-1-yl)piperidine-2,6-dione dihydrochloride (129)

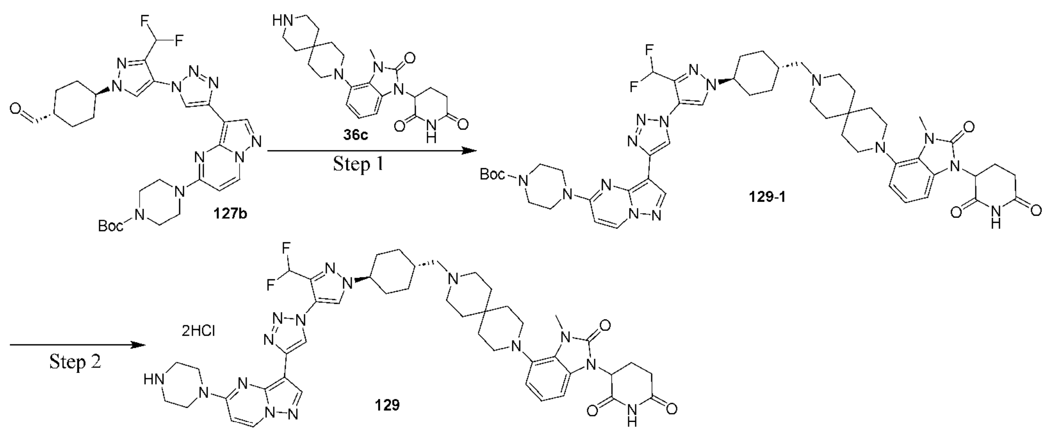


1) Step 1: Compound 36c (60.0 mg, 146 μmol) and triethylamine (15.0 mg, 148 μmol) were added to N,N-dimethylformamide (4 mL) at room temperature, and the obtained mixture was stirred for 10 min. Acetic acid (17.0 mg, 283 μmol) and compound 127b (90.0 mg, 151 μmol) were added, and the reaction solution was stirred at 25°C for 15 min. Sodium borohydride acetate (92.0 mg, 436 μmol) was slowly added, and the reaction solution was

stirred for 20 min. The reaction solution was added to water (10 mL) and ethyl acetate (3 mL × 3) was used for extraction. The organic phase was washed with saturated brine (10 mL), dried over anhydrous sodium sulfate and filtered, and the solvent was removed under reduced pressure. The residue was purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 129-1 (20.0 mg). MS m/z(ESI):922.8[M+1]+.

2) Step 2: Compound 129-1 (35.0 mg, 35.3 μmol) was added to tetrahydrofuran (2 mL) at room temperature, and hydrogen chloride/dioxane (2 mL, 4 M) was added, and the reaction solution was stirred for 1 h. The reaction solution was concentrated under reduced pressure to remove the solvent, pure water (5 mL) was added, and the obtained mixture was freeze-dried under reduced pressure to obtain compound 129. MS m/z(ESI):892.7[M+1]+. $^1$H NMR (400 MHz, DMSO-$d_6$) δ 11.11 (s, 1H), 9.92 - 9.90 (m, 1H), 9.55 - 9.48 (m, 2H), 8.85 (d, *J* = 7.8 Hz, 1H), 8.70 (s, 1H), 8.59 (s, 1H), 8.42 (s, 1H), 7.23 (t, *J* = 53.2 Hz, 1H), 7.02 - 6.96 (m, 2H), 6.90 - 6.87 (m, 2H), 5.40 - 5.35 (m, 1H), 4.37 - 4.31 (m, 1H), 4.04 - 4.02 (m, 4H), 3.72 - 3.68 (m, 5H), 3.40 - 3.36 (m, 2H), 3.22 - 3.20 (m, 4H), 3.05 - 3.01 (m, 4H), 2.92 - 2.89 (m, 4H), 2.74 - 2.61 (m, 2H), 2.28 - 2.15 (m, 4H), 2.07 - 1.84 (m, 7H), 1.78 - 1.72 (m, 2H), 1.55 - 1.43 (m, 2H), 1.29 - 1.19 (m, 2H).

Example 130: 3-(4-(8-(((1r,4r)-4-(3-(difluoromethyl)-4-(4-(5-(piperazin-1-yl)pyrazolo[1,5-a]pyrimidin-3-yl)-1H-1,2,3-triazol-1-yl)-1H-pyrazol-1-yl)cyclohexyl)methyl)-2,8-diazaspiro[4.5]dec-2-yl)-3-methyl-2-oxo-2,3-dihydro-1H-benzo[d]imidazol-1-yl)piperidine-2,6-dione dihydrochloride (130)

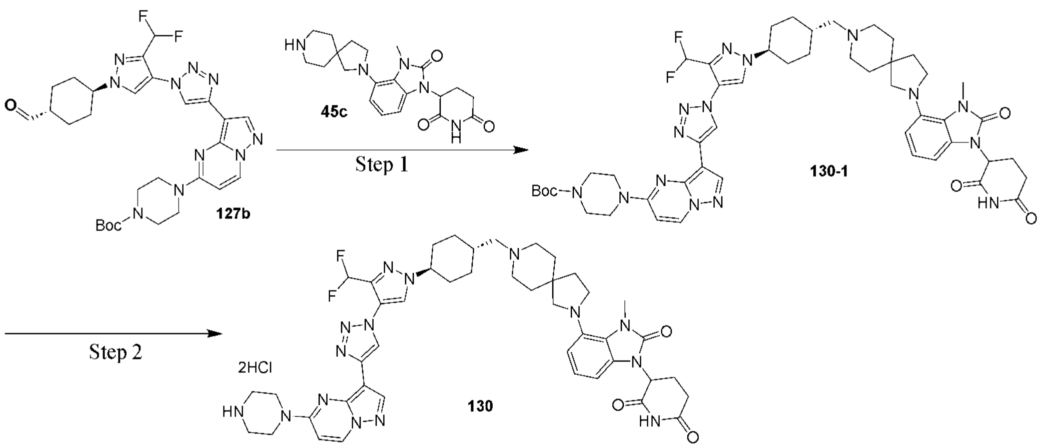


1) Step 1: Compound 45c (60.0 mg, 151 μmol) and triethylamine (15.0 mg, 148 μmol) were added to *N*,*N*-dimethylformamide (4 mL) at room temperature, and the obtained mixture was stirred for 2 min. Acetic acid (18.0 mg, 300 μmol), sodium borohydride acetate (96.0 mg, 455 μmol) and compound 127b (90.0 mg, 151 μmol) were added, and the reaction solution was stirred for 15 min. The residue was purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 130-1. MS m/z(ESI):979.2[M+1]+.

2) Step 2: Compound 130-1 (20.0 mg, 20.2 μmol) was added to tetrahydrofuran (1 mL) at room temperature, and hydrogen chloride/dioxane (1 mL, 4 M) was added, and the reaction solution was stirred for 1 h. The reaction solution was concentrated under reduced pressure to remove the solvent, and pure water (5 mL) was added, and the obtained mixture was lyophilized under reduced pressure. Compound 130 was obtained. MS m/z(ESI):878.6[M+1]+. $^1$H NMR (400 MHz, DMSO-$d_6$) δ 11.10 (s, 1H), 9.30 - 9.19 (m, 2H), 8.86 (d, *J* = 7.8 Hz, 1H), 8.69 (d, *J* = 4.7 Hz, 1H), 8.58 - 8.57 (m, 1H), 8.42 (s, 1H), 7.22 (t, *J* = 53.1, 1H), 7.04 - 6.94 (m, 2H), 6.89 - 6.87 (m, 2H), 5.39 - 5.34 (m, 1H), 4.33 (s, 1H), 4.01 (s, 4H), 3.60 (s, 3H), 3.24 - 3.20 (m, 4H), 3.13 - 3.09 (m, 2H), 3.05 - 3.00 (m, 3H), 2.98 - 2.92 (m, 2H), 2.93 - 2.83 (m, 2H), 2.74 - 2.60 (m, 3H), 2.18 - 2.16 (m, 3H), 2.04 - 1.99 (m, 5H), 1.94 - 1.85 (m, 5H), 1.83 - 1.80 (m, 2H), 1.26 - 1.22 (s, 3H).

Example 131: 3-(4-(4-(1-(1r,4r)-4-(3-difluoromethyl)-4-(4-(5-(piperazin-1-yl)pyrazolo[1,5-a]pyrimidin-3-yl)-1H-1,2,3-triazol-1-yl)-1H-pyrazol-1-yl)cyclohexyl)methyl)piperidin-4-yl)piperazin-1-yl)-3-methyl-2-oxo-2,3-dihydro-1H-benzoimidazol-1-yl)piperidine-2,6-dione hydrochloride (131)

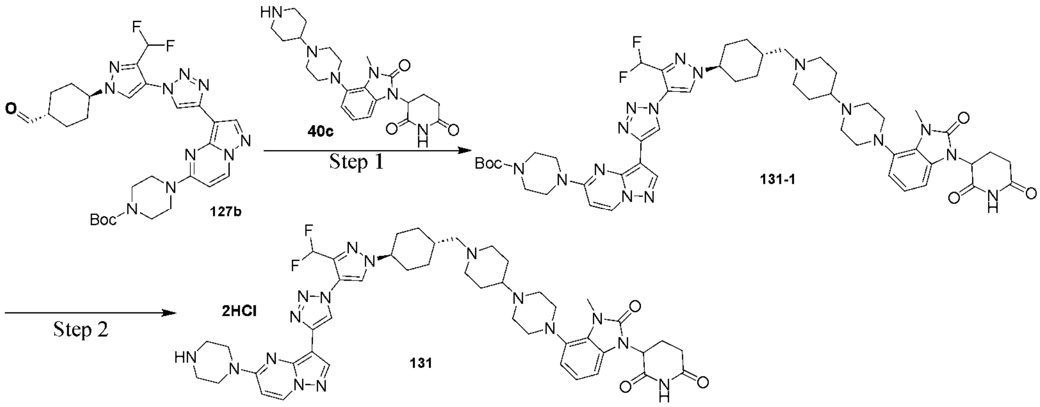

127b, 40c, 131-1, 131

1) Step 1: Triethylamine (0.20 mL, 1.44 mmol) was added to a solution of compound 40c (51.0 mg, 80 μmol), tetrahydrofuran (2 mL) and *N,N*-dimethylformamide (1 mL), and the obtained mixture was stirred for 30 min. Then compound 127b (30 mg, 70 μmol) and acetic acid (0.5 mL) were added to the above solution and the obtained mixture was stirred for 30 min. Finally, sodium borohydride acetate (45.0 mg, 210 μmol) was added to the above reaction solution. The reaction was stirred at 25°C for 2 h. The reaction solution was concentrated under reduced pressure and then purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 131-1. MS m/z(ESI):1007.8[M+1]$^+$.

2) Step 2: Compound 131-1 (30 mg, 0.030 mmol) was added to tetrahydrofuran (2 mL) at room temperature, and hydrogen chloride/dioxane (2 mL, 4 M) was added, and the reaction solution was stirred at room temperature for 1 h. The reaction solution was concentrated under reduced pressure to remove the solvent, and water (5 mL) was added and the obtained mixture was freeze-dried to obtain compound 131. MS m/z(ESI):878.6[M+1]$^+$. $^1$H NMR (400 MHz, DMSO-$d_6$) δ 11.68 - 11.15 (m, 1H), 11.12 (s, 1H), 10.35 (s, 1H), 9.45 (s, 1H), 8.85 (d, *J* = 7.9 Hz, 1H), 8.70 (s, 1H), 8.59 (s, 1H), 8.42 (s, 1H), 7.23 (t, *J* = 53.2 Hz, 1H), 7.08 - 7.03 (m, 1H), 7.01 - 6.96 (m, 1H), 6.96 - 6.91 (m, 1H), 6.88 (d, *J* = 7.9 Hz, 1H), 5.43 - 5.36 (m, 1H), 4.41 - 4.31 (m, 1H), 4.07 - 3.97 (m, 4H), 3.76 - 3.70 (m, 2H), 3.65 (s, 3H), 3.61 - 3.55 (m, 2H), 3.37 - 3.26 (m, 6H), 3.25 - 3.19 (m, 4H), 3.06 - 2.85 (m, 6H), 2.76 - 2.58 (m, 3H), 2.44 - 2.29 (m, 4H), 2.19 - 2.14 (m, 2H), 2.11 - 2.04 (m, 2H), 2.02 - 1.96 (m, 2H), 1.96 - 1.83 (m, 3H).

Example 132: 3-(4-(1R,4R)-4-(3-difluoromethyl)-4-(4-(5-(piperazin-1-yl)pyrazolo[1,5-a]pyrimidin-3-yl)-1H-1,2,3-triazol-1-yl)-1H-pyrazol-1-yl)cyclohexyl)methyl)piperazin-1-yl)-3-methyl-2-oxo-2,3-dihydro-1H-benzoimidazol-1-yl)piperidine-2,6-dione dihydrochloride ((132)

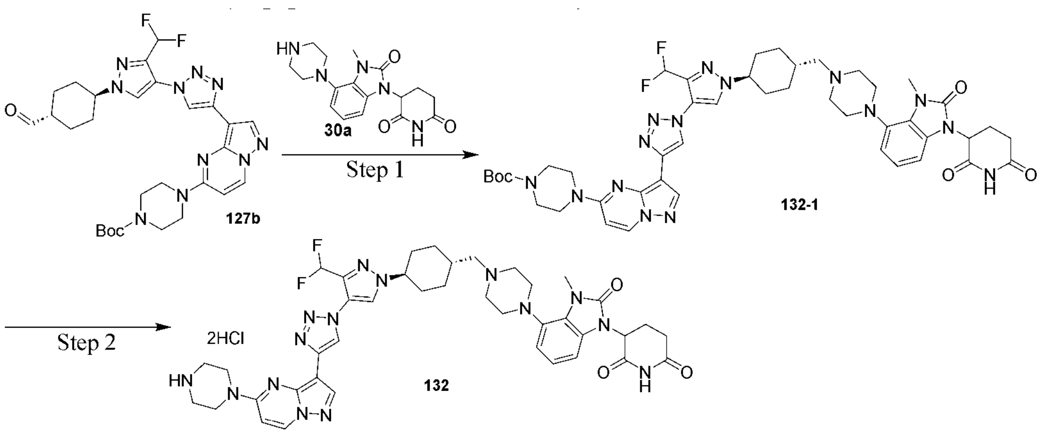

127b, 30a, 132-1, 132

1) Step 1: Triethylamine (0.20 mL, 1.44 mmol) was added to a solution containing compound 30a (31.0 mg, 90.4 μmol), tetrahydrofuran (2 mL) and *N,N*-dimethylformamide (1 mL), and the obtained mixture was stirred for 30 min. Then compound 127b (35.0 mg, 58.7 μmol) and acetic acid (0.5 mL) were added to the above solution and the obtained mixture was stirred for 30 min. Finally, sodium borohydride acetate (41.0 mg, 193 μmol) was added to the above reaction solution. The reaction was stirred at 25°C for 2 h. The reaction solution was concentrated under reduced pressure and then purified by high performance liquid chromatography (Gilson_306_1741, chromatographic column: Waters-SunFire-C18-10μm-19*250 mm; mobile phase: water (containing 0.1% formic acid) and acetonitrile, gradient ratio: acetonitrile 10%-90%, flow rate: 25 mL/min) to obtain compound 132-1. MS m/z(ESI):923.5[M-1]$^-$.

2) Step 2: Compound 132-1 (20.0 mg, 21.7 μmol) was added to tetrahydrofuran (2 mL) at room temperature, and hydrogen chloride/dioxane (2 mL, 4 M) was added, and the reaction solution was stirred for 1 h. The reaction solution was concentrated under reduced pressure to remove the solvent, pure water (5 mL) was added, and the obtained mixture was freeze-dried under reduced pressure to obtain compound 132. MS m/z(ESI):824.6[M+1]$^+$. $^1$H NMR (400 MHz, DMSO-$d_6$) δ 11.18 (s, 1H), 10.47 (s, 1H), 9.45 (s, 1H), 8.91 (d, *J* = 7.9 Hz, 1H), 8.76 (s, 1H), 8.65 (s, 1H), 8.48 (s, 1H), 7.29 (t, *J* = 53.3 Hz, 1H), 7.14 - 7.08 (m, 1H), 7.06 - 6.97 (m, 2H), 6.94 (d, *J* = 7.9 Hz, 1H), 5.49 - 5.41 (m, 1H), 4.46 - 4.37 (m, 1H), 4.12 - 4.04 (m, 4H), 3.71 (s, 3H), 3.69 - 3.62 (m, 2H), 3.39 - 3.33 (m, 3H), 3.32 - 3.25 (m, 6H), 3.22 - 3.14 (m, 2H), 3.02 - 2.90 (m, 1H), 2.83 - 2.67 (m, 2H), 2.27 - 1.91 (m, 9H), 1.39 - 1.25 (m, 3H).

Example 133: 3-(4-(4-(1-(((1r,4r)-4-(3-(difluoromethyl)-4-(4-(5-((3-hydroxy-3-methylbutyl)amino)pyrazolo[1,5-a]pyrimidin-3-yl)-1H-1,2,3-triazol-1-yl)-1H-pyrazol-1-yl(1H-piperidin-4-yl)piperazin-1-yl)-3-methyl-2-oxo-2,3-dihydro-1H-benzo[d]imidazol-1-yl)piperidine-2,6-dione (133)

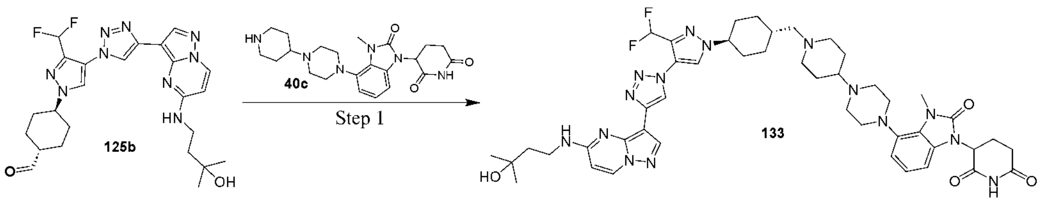


Compound 40c (24.9 mg, 58.4 μmol) was dissolved in a mixed solution of *N,N*-dimethylformamide (1 mL) and tetrahydrofuran (4 mL), triethylamine (5.91 mg, 58.4 μmol) was added, and the obtained mixture was reacted under stirring at room temperature for 15 min. Then compound 125b (30.0 mg, 58.4 μmol) and acetic acid (7.02 mg, 117 μmol) were added and the obtained mixture was reacted under stirring at room temperature for 30 min. Sodium triacetoxyborohydride (37.0 mg, 175 μmol) was then added, and the obtained mixture was reacted under stirring at room temperature for 30 min. The reaction system was then directly concentrated, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 133. MS m/z(ESI):924.7[M+1]$^+$. $^1$H NMR (400 MHz, DMSO-$d_6$): δ 11.10 (s, 1H), 8.64 (s, 1H), 8.51 (s, 1H), 8.27 (s, 1H), 7.74 - 7.65 (m, 1H), 7.14 (t, *J* = 53.2 Hz, 1H), 7.01 - 6.91 (m, 2H), 6.91 - 6.87 (m, 1H), 6.29 (d, *J* = 7.6 Hz, 1H), 5.39 - 5.31 (m, 1H), 4.46 - 4.34 (m, 1H), 4.31 - 4.23 (m, 1H), 3.62 (s, 3H), 3.54 - 3.44 (m, 4H), 2.99 - 2.83 (m, 8H), 2.73 - 2.57 (m, 4H), 2.26 - 2.21 (m, 1H), 2.17 - 2.10 (m, 4H), 1.99 - 1.87 (m, 5H), 1.82 - 1.70 (m, 6H), 1.61 - 1.54 (m, 1H), 1.51 - 1.42 (m, 2H), 1.13 (s, 6H), 1.11 - 1.03 (m, 2H).

Example 134: 3-(4-(2-(((1r,4r)-4-(3-(difluoromethyl))-4-(4-(5-((3-hydroxy-3-methylbutyl)amino)pyrazolo[1,5-a]pyrimidin-3-yl)-1H-1,2,3-triazol-1-yl)-1H-pyrazol-1-yl)cyclohexyl)methyl)-2,8-diazaspiro[4.5]dec-8-yl)-3-methyl-2-oxo-2,3-dihydro-1H-benzo[d]imidazol-1-yl)piperidine-2,6-dione (134)

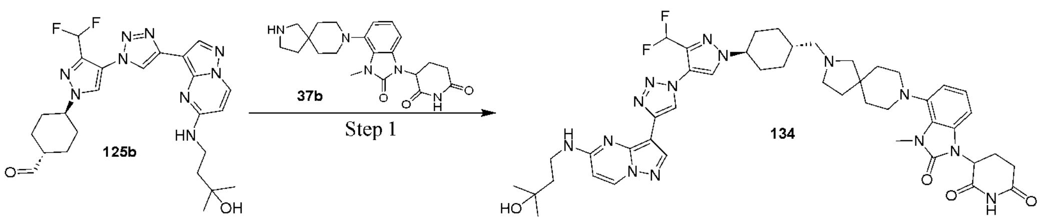

Compound 37b (38.7 mg, 97.4 μmol) was dissolved in a mixed solution *of N,N*-dimethylformamide (1 mL) and tetrahydrofuran (4 mL), triethylamine (9.85 mg, 97.4 μmol) was added, and the obtained mixture was reacted under stirring at room temperature for 15 min. Then compound 125b (50.0 mg, 97.4 μmol) and acetic acid (11.7 mg, 195 μmol) were added and the obtained mixture was reacted under stirring at room temperature for 30 min. Sodium triacetoxyborohydride (61.1 mg, 292 μmol) was then added, and the obtained mixture was reacted under stirring at room temperature for 30 min. The reaction system was then directly concentrated, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 134. MS m/z(ESI):895.7[M+1]$^+$. $^1$H NMR (400 MHz, DMSO-$d_6$): δ 11.10 (s, 1H), 8.66 - 8.64 (m, 1H), 8.52 - 8.51 (m, 1H), 8.27 (s, 1H), 7.74 - 7.65 (m, 1H), 7.14 (t, *J* = 53.2 Hz, 1H), 6.99 - 6.92 (m, 2H), 6.88 - 6.84 (m, 1H), 6.29 (d, *J* = 7.5 Hz, 1H), 5.38 - 5.32 (m, 1H), 4.45 - 4.27 (m, 2H), 3.64 (s, 3H), 3.53 - 3.45 (m, 4H), 3.05 - 2.83 (m, 5H), 2.74 - 2.59 (m, 6H), 2.39 - 2.25 (m, 4H), 2.17 - 2.12 (m, 2H), 2.06 - 1.93 (m, 4H), 1.77 - 1.68 (m, 7H), 1.15 - 1.09 (m, 8H).

Example 135: 3-(4-(4-(4-[((1r,4r)-4-(3-(difluoromethyl)-4-(4-(-4-hydroxypyridopiperidin-1-yl)pyrazolo[1,5-a]pyrimidin-3-yl)-1-pyrimidinedimethyl-1-pyrazol-1,2,3-triazol-4-(4-piperidine-2,6-dione (135)

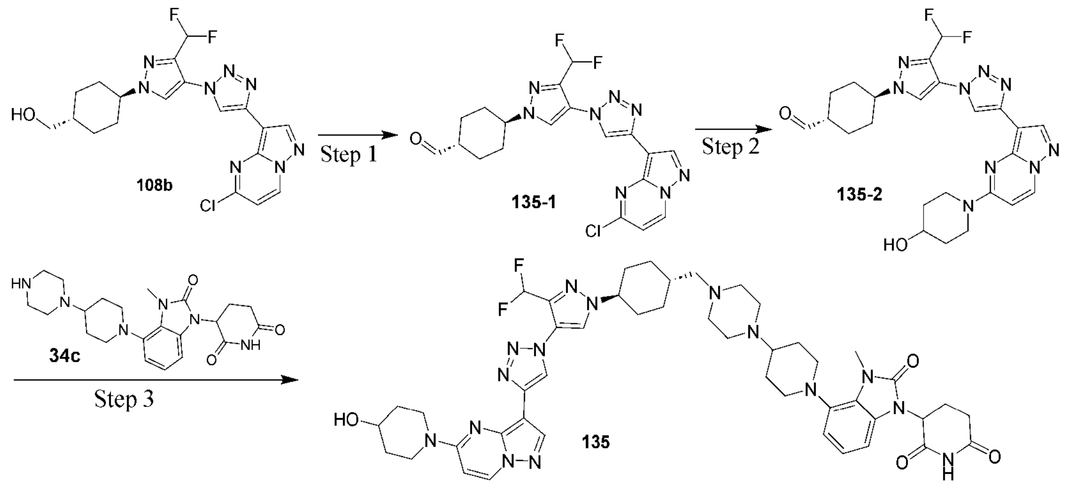


1) Step 1: Dess-Martin (945 mg, 2.23 mmol) was added to a solution containing compound 108b (500 mg, 1.11 mmol) and N,N-dimethylformamide (5 mL) at 0°C, and then the obtained mixture was stirred at 25°C for 2 h. Water (20 mL) was added to the reaction solution for dilution, and the obtained mixture was extracted with ethyl acetate (30 mL×2). The combined organic phase was dried over anhydrous sodium sulfate and filtered to remove the desiccant, and then the filtrate was concentrated under reduced pressure. The residue was purified by silica gel column chromatography with an eluent system (dichloromethane/methanol = 100/1 to 20/1) to obtain compound 135-1. MS m/z(ESI):447.0 [M+1]$^+$.

2) Step 2: N,N-Diisopropylethylamine (174 mg, 1.35 mmol) was added to a solution containing compound 135-1 (200 mg, 0.45 mmol), 4-hydroxypiperidine (91 mg, 0.90 mmol) and acetonitrile (5 mL), and the obtained mixture was stirred at 60°C for 1 h. Water (20 mL) was added to the reaction solution, and the obtained mixture was extracted with ethyl acetate (30 mL×2). The combined organic phase was dried over anhydrous sodium sulfate and filtered to remove the desiccant, and then the filtrate was concentrated under reduced pressure. The residue was purified by silica gel column chromatography with an eluent system (dichloromethane/methanol = 100/1 to 20/1) to obtain compound 135-2. MS m/z(ESI):512.4[M+1]$^+$.

3) Step 3: Sodium borohydride acetate (371 mg, 1.76 mmol) was added to a solution containing compound 34c (188 mg, 0.44 mmol), acetic acid (35 mg, 0.59 mmol) and *N,N*-dimethylformamide (5 mL), and the obtained mixture was stirred at 25°C for 10 min, then compound 135-2 (150 mg, 0.29 mmol) was added. The reaction solution was stirred at 25°C for further 50 min. The reaction solution was concentrated under reduced pressure, and the obtained residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 135. MS m/z(ESI):922.7[M+1]$^+$. $^1$H NMR (400 MHz, DMSO-$d_6$): δ 11.09 (s, 1H), 8.72 - 8.64 (m, 2H), 8.53 (s, 1H), 8.35 (s, 1H), 7.22 (t, *J* = 53.2 Hz, 1H), 7.02 - 6.95 (m, 1H), 6.93 - 6.80 (m, 3H), 5.40 - 5.31 (m, 1H), 4.35 - 4.25 (m, 1H), 4.22 - 4.10 (m, 2H), 3.83 - 3.76 (m, 1H), 3.63 (s, 3H),

3.57 - 3.49 (m, 2H), 3.18 - 3.10 (m, 3H), 2.92 - 2.85 (m, 1H), 2.76 - 2.63 (m, 4H), 2.61 - 2.54 (m, 3H), 2.42 - 2.24 (m, 5H), 2.19 - 2.09 (m, 4H), 2.04 - 1.75 (m, 10H), 1.66 - 1.55 (m, 3H), 1.46 - 1.36 (m, 2H), 1.15 - 1.00 (m, 2H).

Example 136: 3-(4-(9-(((1r,4r)-4-(3-(difluoromethyl)-4-(4-(5-(4-hydroxypiperidin-1-yl))pyrazolo[1,5-a]pyrimidin-3-yl)-1H-1,2,3-triazol-1-yl)-1H-pyrazol-1-yl)cyclohexyl)methyl)-3,9-diazaspiro[5.5]undecan-3-yl)- 3-methyl-2-oxo-2,3-dihydro-1H-benzo[d]imidazol-1-yl)piperidine-2,6-dione (136)

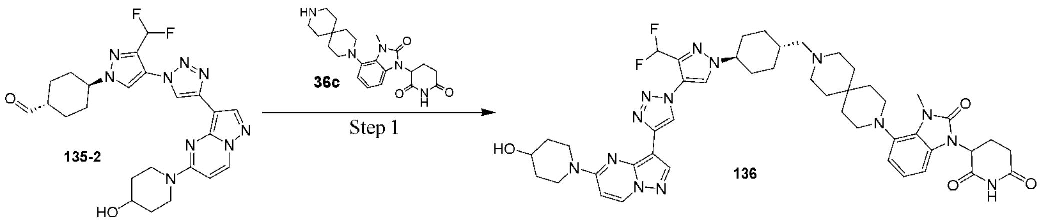


Triethylamine (4.95 mg, 48.8 μmol) was added to a solution of compound 36c (20.1 mg, 48.8 μmol) in N,N-dimethylformamide (1 mL) at room temperature, and the obtained mixture was stirred for 1 min. Sodium triacetylborohydride (41.2 mg, 195 μmol) and acetic acid (2.93 mg, 48.8 μmol) were added, and the obtained mixture was stirred for 1 min. Compound 135-2 (25.0 mg, 48.8 μmol) was added, and the obtained mixture was stirred for 1 h. Water (0.2 mL) was added to the reaction solution for dilution, and the obtained mixture was filtered. The filtrate was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 136. MS m/z(ESI):907.6[M+1]$^+$. $^1$H NMR (400 MHz, DMSO-$d_6$) δ 11.10 (s, 1H), 8.72 - 8.65 (m, 2H), 8.53 (s, 1H), 8.35 (s, 1H), 7.22 (t, *J* = 52.6 Hz, 1H), 7.01 - 6.94 (m, 2H), 6.89 - 6.80 (m, 2H), 5.38 - 5.29 (m, 2H), 4.81 - 4.75 (m, 1H), 4.32 - 4.26 (m, 1H), 4.19 - 4.13 (m, 2H), 3.83 - 3.76 (m, 2H), 3.64 (s, 3H), 2.91 - 2.84 (m, 6H), 2.70 - 2.64 (m, 3H), 2.37 - 2.33 (m, 5H), 2.19 - 2.10 (m, 4H), 2.04 - 1.95 (m, 5H), 1.88 - 1.79 (m, 5H), 1.46 - 1.40 (m, 4H), 1.12 - 1.03 (m, 2H), 0.88 - 0.84 (m, 1H).

Example 137: 3-(4-(8-(((1r,4r)-4-(3-(difluoromethyl)-4-(4-(5-(4-hydroxypiperidin-1-yl))pyrazolo[1,5-a] pyrimidin-3-yl)-1H-1,2,3-triazol-1-yl)-1H-pyrazol-1-yl)cyclohexyl)methyl)-2,8-diazaspiro[4.5]dec-2-yl)- 3-methyl-2-oxo-2,3-dihydro-1H-benzo[d]imidazol-1-yl)piperidine-2,6-dione (137)

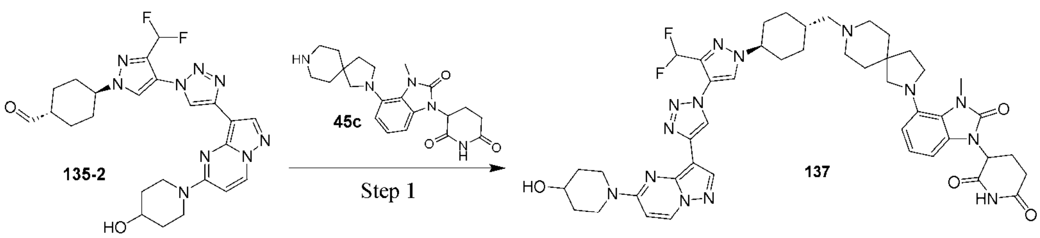


Compound 45c (19.4 mg, 48.9 μmol) was dissolved in a mixed solution of *N,N*-dimethylformamide (1 mL) and tetrahydrofuran (4 mL), triethylamine (4.95 mg, 48.9 μmol) was added, and the obtained mixture was reacted under stirring at room temperature for 15 min. Then compound 135-2 (25.0 mg, 48.9 μmol) and acetic acid (5.87 mg, 97.8 μmol) were added and the obtained mixture was reacted under stirring at room temperature for 30 min. Sodium triacetoxyborohydride (30.9 mg, 147 μmol) was then added, and the obtained mixture was reacted under stirring at room temperature for 30 min. The reaction system was then directly concentrated, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 137. MS m/z(ESI):895.7[M+1]$^+$. $^1$H NMR (400 MHz, DMSO-$d_6$): δ 11.09 (s, 1H), 8.70 - 8.65 (m, 2H), 8.52 (s, 1H), 8.39 (s, 2H), 7.28 (t, *J* = 53.2 Hz, 1H), 6.98 - 6.94 (m, 2H), 6.87 - 6.81 (m, 2H), 5.38 - 5.32 (m, 1H), 4.31 - 4.25 (m, 1H), 4.19 - 4.12 (m, 2H), 3.83 - 3.74 (m, 2H), 3.59 (s, 3H), 3.08 - 3.05 (m, 2H), 2.91 - 2.85 (m, 3H), 2.74 - 2.58 (m, 3H), 2.39 - 2.28 (m, 4H), 2.16 - 2.09 (m, 4H), 1.98 - 1.91 (m, 2H), 1.86 - 1.78 (m, 4H), 1.76 - 1.72 (m, 2H), 1.68 - 1.55 (m, 6H), 1.46 - 1.39 (m, 2H), 1.12 - 1.00 (m, 2H).

Example 138: 3-(4-(4-(1-(((1r,4r)-4-(3-(difluoromethyl)-4-(4-(5-(4-hydroxypiperidin-1-yl))pyrazolo[1,5 -a]pyrimidin-3-yl)-1H-1,2,3-triazol-1-yl)-1H-pyrazol-1-yl)cyclohexyl)methyl)piperidin-4-yl)piperazin-1-yl)- 3-methyl-2-oxo-2,3-dihydro-1H-benzo[d]imidazol-1-yl)piperidine-2,6-dione (138)

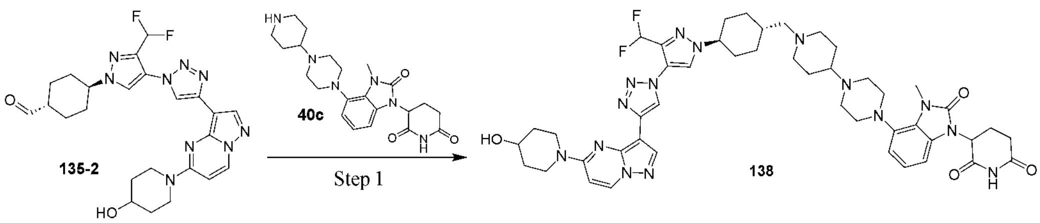


Compound 40c (21.3 mg, 50.0 μmol) was dissolved in a mixed solution of *N,N*-dimethylformamide (1 mL) and tetrahydrofuran (4 mL), triethylamine (4.95 mg, 48.9 μmol) was added, and the obtained mixture was reacted under stirring at room temperature for 15 min. Then compound 135-2 (25.0 mg, 48.9 μmol) and acetic acid (5.87 mg, 97.8 μmol) were added and the obtained mixture was reacted under stirring at room temperature for 30 min. Sodium triacetoxyborohydride (30.9 mg, 147 μmol) was then added, and the obtained mixture was reacted under stirring at room temperature for 30 min. The reaction system was then directly concentrated, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 138. MS m/z(ESI):922.6[M+1]$^+$. $^1$H NMR (400 MHz, DMSO-$d_6$): δ 11.09 (s, 1H), 8.71 - 8.65 (m, 2H), 8.54 - 8.51 (m, 1H), 8.34 (s, 1H), 8.28 (s, 1H), 7.15 (t, *J* = 53.2 Hz, 1H), 7.00 - 6.91 (m, 2H), 6.89 - 6.80 (m, 2H), 5.38 - 5.31 (m, 1H), 4.32 - 4.25 (m, 1H), 4.19 - 4.12 (m, 2H), 3.82 - 3.75 (m, 2H), 3.62 (s, 3H), 3.01 - 2.81 (m, 9H), 2.73 - 2.59 (m, 3H), 2.25 - 2.20 (m, 1H), 2.17 - 2.09 (m, 4H), 2.04 - 1.73 (m, 13H), 1.61 - 1.55 (m, 1H), 1.48 - 1.38 (m, 4H), 1.13 - 1.01 (m, 2H).

Example 139: 3-(4-(2-(((1r,4r)-4-(3-(difluoromethyl)-4-(4-(5-(4-hydroxypiperidin-1-yl))pyrazolo[1,5-a] pyrimidin-3-yl)-1H-1,2,3-triazol-1-yl)-1H-pyrazol-1-yl)cyclohexyl)methyl)-2,8-diazaspiro[4.5]dec-8-yl)- 3-methyl-2-oxo-2,3-dihydro-1H-benzo[d]imidazol-1-yl)piperidine-2,6-dione (139)

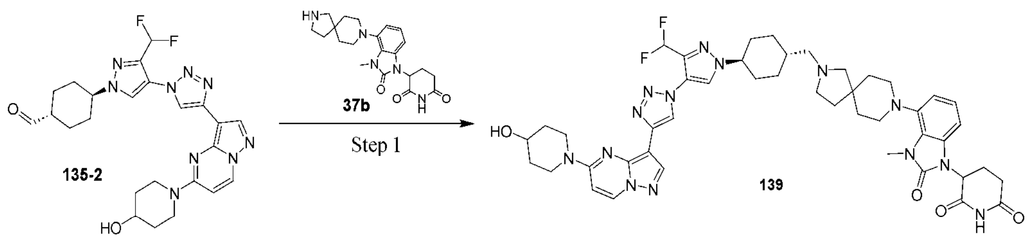


Triethylamine (8.10 mg, 78.1 μmol) was added to a solution of compound 37b (40.1 mg, 101 μmol) in N,N-dimethylformamide (1 mL) at room temperature, and the obtained mixture was stirred for 1 min. Sodium triacetylborohydride (82.4 mg, 390 μmol) and acetic acid (9.39 mg, 156 μmol) were added, and the obtained mixture was stirred for 1 min. Compound 135-2 (40.0 mg, 78.1 μmol) was added, and the obtained mixture was stirred for 1 h. Water (0.2 mL) was added to the reaction solution for dilution, and the obtained mixture was filtered. The residue obtained by filtration was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 139. MS m/z(ESI):893.8[M+1]$^+$. $^1$H NMR (400 MHz, DMSO-$d_6$) δ 8.71 - 8.68 (m, 1H), 8.67 (s, 1H), 8.53 (s, 1H), 8.35 (s, 1H), 7.22 (t, *J* = 53.2 Hz, 1H), 7.03 - 6.91 (m, 2H), 6.92 - 6.85 (m, 1H), 6.83 (d, *J* = 7.9 Hz, 1H), 5.41 - 5.30 (m, 1H), 4.77 (d, *J* = 4.2 Hz, 1H), 4.34 - 4.25 (m, 1H), 4.20 - 4.10 (m, 2H), 3.83 - 3.76 (m, 1H), 3.64 (s, 3H), 3.40 - 3.35 (m, 3H), 3.09 - 2.82 (m, 4H), 2.73 - 2.70 (m, 1H), 2.69 - 2.67 (m, 1H), 2.66 - 2.63 (m, 1H), 2.63 - 2.60(m, 1H), 2.60 - 2.56 (m, 4H), 2.34 - 2.32 (m, 1H), 2.28-2.22 (m, 2H), 2.18 - 2.11 (m, 2H), 2.00 - 1.95 (m, 2H), 1.87 - 1.80 (m, 4H), 1.73 - 1.59 (m, 5H), 1.46 - 1.38 (m, 2H), 1.17 - 1.02 (m, 2H).

Example 140: 3-(4-(4-(((1r,4r)-4-(3-(difluoromethyl)-4-(4-(5-(4-hydroxypiperidin-1-yl))pyrazolo[1,5-a] pyrimidin-3-yl)-1H-1,2,3-triazol-1-yl)-1H-pyrazol-1-yl)cyclohexyl)methyl)piperazin-1-yl)-3-methyl-2-oxo-2,3-dihydro-1H-benzo[d] imidazol-1-yl)piperidine-2,6-dione (140)

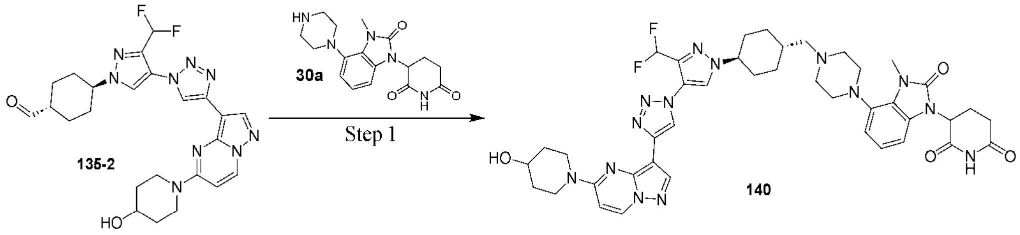


Triethylamine (5.93 mg, 58.6 μmol) was added to a solution of compound 30a (40.2 mg, 117 μmol) in N,N-dimethylformamide (1 mL) at room temperature, and the obtained mixture was stirred for 1 min. Sodium triacetylborohydride (49.4 mg, 234 μmol) and acetic acid (7.04 mg, 117 μmol) were added, and the obtained mixture was stirred for 1 min. Compound 135-2 (30.0 mg, 58.6 μmol) was added, and the obtained mixture was stirred for 1 h. Water (0.2 mL) was added to the reaction solution for dilution, and the obtained mixture was filtered. The residue obtained by filtration was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 140. MS m/z(ESI):839.6[M+1]$^+$.

Example 141: 3-(4-(4-(((1r,4r)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-ylcyclohexyl)methyl)piperazin-1-yl)-3-methyl-2-oxo-2,3-dihydro-1H-benzo[d]imidazol-1-yl)piperidine-2,6-dione (141)

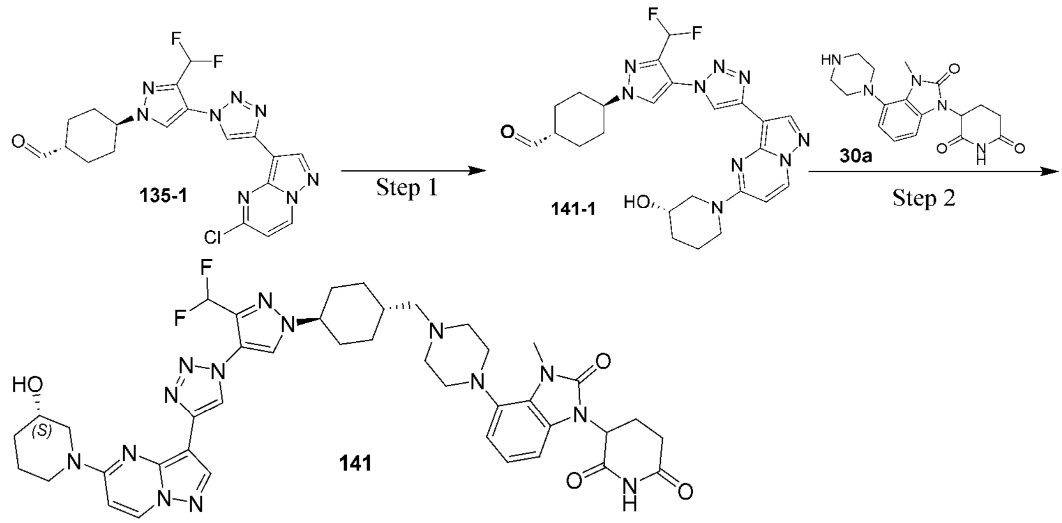


1) Step 1: Compound 135-1 (370 mg, 0.830 mmol) and (S)-3-hydroxypiperidine hydrochloride (228 mg, 1.66 mmol, Bide Pharmatech Ltd.) were added to a single-neck bottle at room temperature in sequence, and acetonitrile (7.5 mL) and *N,N*-diisopropylethylamine (549 μL, 3.31 mmol) were added under stirring. The reaction system was subjected to nitrogen replacement three times and reacted at 80°C for 2 h. Dichloromethane (100 mL) was added to the reaction solution, and the dichloromethane layer was washed with water (30 mL × 3). The dichloromethane layer was collected and washed with saturated brine (30 mL), dried with anhydrous sodium sulfate, filtered and concentrated to obtain a residue. The residue was purified by silica gel column chromatography with an eluent system (methanol/dichloromethane = 0%-10%) to obtain compound 141-1. MS m/z(ESI):512.0[M+1]$^+$.

2) Step 2: Compound 30a (45.0 mg, 98.4 μmol, prepared by the method disclosed for the intermediate GS on page 550 of the specification of the patent application "WO 2022236058 A1"),*N,N*-dimethylformamide (1.5 mL) and tetrahydrofuran (1 mL) were added to a single-neck bottle at room temperature in sequence, triethylamine (34.1 μL, 246

μmol) was added under stirring, and the obtained mixture was reacted at 0°C for 10 min. Then compound 141-1 (50.3 mg, 98.4 μmol), sodium borohydride acetate (125 mg, 590 μmol) and acetic acid (33.8 μL, 590 μmol) were added in sequence, and the obtained mixture was reacted at 25°C for 2 h. Most of the solvent was removed from the reaction solution under vacuum to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 2%-95%, flow rate: 25 mL/min) to obtain compound 141. MS m/z(ESI):839.4[M+1]$^+$. $^1$H NMR (400 MHz, DMSO-$d_6$) δ 11.10 (s, 1H), 8.68 - 8.65 (m, 2H), 8.50 (s, 1H), 8.34 (s, 1H), 7.20 (t, *J* = 53.2 Hz, 1H), 6.99 - 6.92 (m, 2H), 6.91 - 6.86 (m, 1H), 6.77 (d, *J* = 8.0 Hz, 1H), 5.38 - 5.33 (m, 1H), 5.15 - 4.50 (m, 1H), 4.35 - 4.26 (m, 1H), 4.12 - 3.97 (m, 2H), 3.63 (s, 3H), 3.62 - 3.55 (m, 2H), 3.23 - 3.16 (m, 2H), 3.03 - 2.83 (m, 7H), 2.76 - 2.58 (m, 3H), 2.23 (d, *J*= 7.1 Hz, 2H), 2.19 - 2.13 (m, 2H), 2.02 - 1.94 (m, 3H), 1.90 - 1.76 (m, 4H), 1.64 (s, 1H), 1.53 - 1.44 (m, 2H), 1.19 - 1.04 (m, 2H).

Example 142: 3-(4-(9-(((1r,4r)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-ylcyclohexyl)methyl)-3,9-diazaspiro[5.5]undecan-3-yl)-3-methyl-2-oxo-2,3-dihydro-1H-benzo[d]imidazol-1-yl)piperidine-2,6-dione (142)

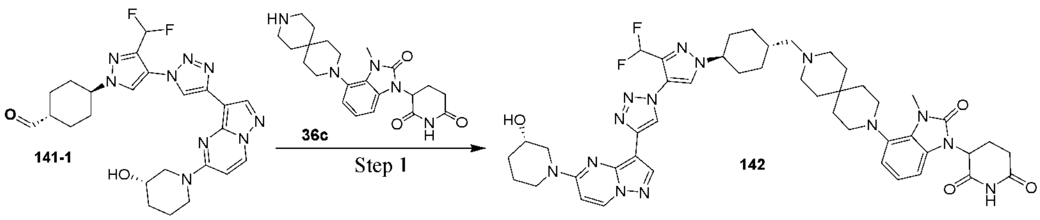


Compound 36c (40.0 mg, 0.097 mmol) was dissolved in *N,N*-dimethylformamide (1 mL) and tetrahydrofuran (1 mL) at 0°C, triethylamine (23.0 mg, 0.228 mmol) was added, and the obtained mixture was stirred for 10 min. Then sodium borohydride acetate (97.0 mg, 0.460 mmol), compound 141-1 (68.0 mg, 0.133 mmol) and acetic acid (23.0 mg, 0.380 mmol) were added to the mixture, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-Xbridge-C18-10μm-19*250 mm; mobile phase: water (containing 10 mmol/L ammonium bicarbonate) and acetonitrile, gradient ratio: acetonitrile 38%-95%, flow rate: 25 mL/min) to obtain compound 142. MS m/z(ESI): 907.4[M+1]$^+$. $^1$H NMR (400 MHz, DMSO-$d_6$) δ 11.09 (s, 1H), 8.68 - 8.63 (m, 2H), 8.49 (s, 1H), 8.33 (s, 1H), 7.20 (t, *J* = 53.3 Hz, 1H), 6.99 - 6.92 (m, 2H), 6.90 - 6.83 (m, 1H), 6.77 (d, *J* = 8.0 Hz, 1H), 5.41 - 5.27 (m, 1H), 4.89 (d, *J* = 4.2 Hz, 1H), 4.32 - 4.26 (m, 1H), 4.14 - 3.94 (m, 2H), 3.63 (s, 3H), 2.87 (s, 4H), 2.70 - 2.61 (m, 2H), 2.37 - 2.32 (m, 4H), 2.18 - 2.11 (m, 4H), 2.01 - 1.76 (m, 9H), 1.73 - 1.39 (m, 13H), 1.12 - 1.02 (m, 2H).

Example 143: 3-(4-(8-(((1r,4r)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-ylcyclohexyl)methyl)-2,8-diazaspiro[4.5]dec-2-yl)-3-methyl-2-oxo-2,3-dihydro-1H-benzo[d]imidazol-1-yl)piperidine-2,6-dione (143)

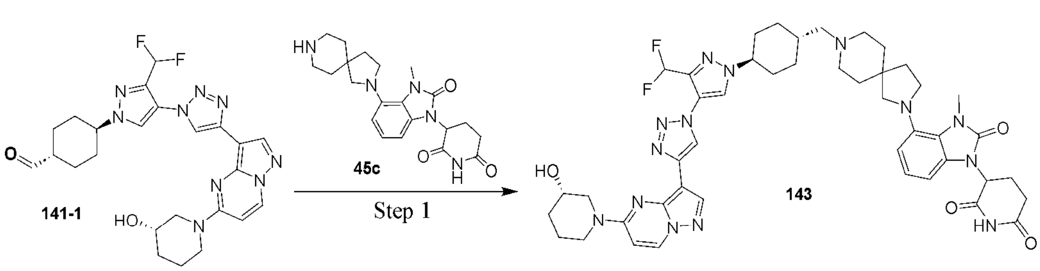


Compound 45c (50.0 mg, 126 μmol), N,N-dimethylformamide (1 mL) and tetrahydrofuran (1.5 mL) were added to a single-neck bottle at room temperature in sequence, triethylamine (34.0 μL, 245 μmol) was added under stirring, and the obtained mixture was reacted at 0°C for 10 min. Then compound 141-1 (50.0 mg, 97.8 μmol), sodium borohydride acetate (124 mg, 587 μmol) and acetic acid (34.0 μL, 595 μmol) were added in sequence, and the obtained mixture was reacted at 25°C for 2 h. Most of the solvent was removed from the reaction solution under vacuum to obtain a residue. The residue

was purified by high performance liquid chromatography (Gilson _306_1741, chromatographic column: Waters-Xbridge-C18-10μm-19×250 mm; mobile phase: water (containing 26.5 mmol/L formic acid) and acetonitrile, gradient ratio: acetonitrile 10%-95%, flow rate: 25 mL/min) to obtain compound 143. MS m/z(ESI):893.4[M+1]$^+$. $^1$H NMR (400 MHz, DMSO-$d_6$) δ 11.10 (s, 1H), 8.77 - 8.63 (m, 2H), 8.50 (s, 1H), 8.34 (s, 1H), 7.20 (t, *J* = 53.2 Hz, 1H), 7.02 - 6.94 (m, 2H), 6.91 - 6.84 (m, 1H), 6.78 (d, *J* = 8.0 Hz, 1H), 5.38 - 5.33 (m, 1H), 4.90 (s, 1H), 4.37 - 4.25 (m, 1H), 4.19 - 3.95 (m, 2H), 3.60 (s, 4H), 3.49 - 3.36 (m, 3H), 3.23 - 3.16 (m, 1H), 3.09 (t, *J* = 6.9 Hz, 2H), 3.00 - 2.79 (m, 4H), 2.76 - 2.58 (m, 5H), 2.20 - 2.10 (m, 2H), 2.02 - 1.85 (m, 5H), 1.85 - 1.66 (m, 9H), 1.54 - 1.42 (m, 2H), 1.28 - 0.99 (m, 2H).

Example 144: 3-(4-(4-(1-(((1r,4r)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl(cyclohexyl)methyl)piperidin-4-yl)piperazin-1-yl)-3-methyl-2-oxo-2,3-dihydro-1H-benzo[d]imidazol-1-yl)piperidine-2,6-dione (144)

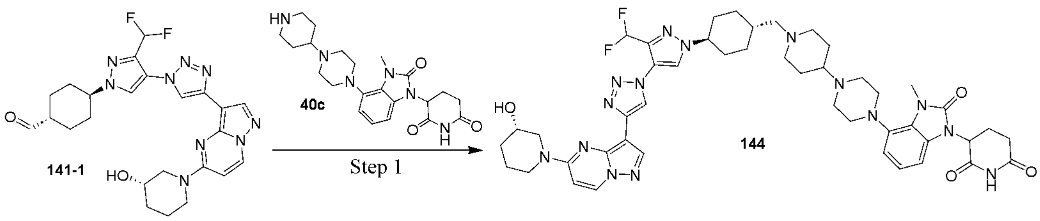

141-1

144

Compound 40c (53.0 mg, 124 μmol), *N,N*-dimethylformamide (1 mL) and tetrahydrofuran (1.5 mL) were added to a single-neck bottle at room temperature in sequence, triethylamine (33.0 μL, 245 μmol) was added under stirring, and the obtained mixture was reacted at 0°C for 10 min. Then compound 141-1 (50.2 mg, 98.1 μmol), sodium borohydride acetate (124 mg, 588 μmol) and acetic acid (33.7 μL, 588 μmol) were added in sequence, and the obtained mixture was reacted at 25°C for 2 h. Most of the solvent was removed from the reaction solution under vacuum to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 7%-95%, flow rate: 25 mL/min) to obtain compound 144. MS m/z(ESI):922.4[M+1]$^+$. $^1$H NMR (400 MHz, DMSO-$d_6$) δ 11.10 (s, 1H), 8.70 - 8.62 (m, 2H), 8.50 (s, 1H), 8.34 (s, 1H), 7.20 (t, *J* = 53.2 Hz, 1H), 6.99 - 6.86 (m, 3H), 6.77 (d, *J* = 8.0 Hz, 1H), 5.38 - 5.33 (m, 1H), 4.33 - 4.24 (m, 1H), 4.18 - 3.92 (m, 3H), 3.62 (s, 3H), 3.61 - 3.56 (m, 2H), 3.49 - 3.36 (m, 2H), 3.23 - 3.17 (m, 1H), 2.99 - 2.81 (m, 8H), 2.72 - 2.58 (m, 3H), 2.33 - 2.20 (m, 2H), 2.16 - 2.11 (m, 3H), 1.95 - 1.76 (m, 10H), 1.64 - 1.41 (m, 6H), 1.23 - 0.96 (m, 2H).

Example 145: 3-(4-(2-(((1r,4r)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-ylcyclohexyl)methyl)-2,8-diazaspiro[4.5]dec-8-yl)-3-methyl-2-oxo-2,3-dihydro-1H-benzo[d]imidazol-1-yl)piperidine-2,6-dione (145)

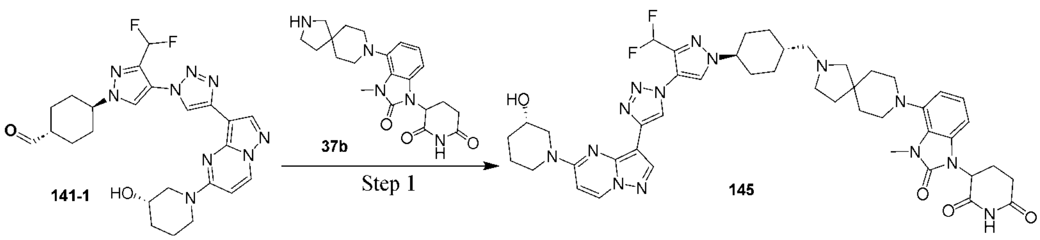

141-1

145

Compound 37b (50.0 mg, 126 μmol), *N,N*-dimethylformamide (1 mL) and tetrahydrofuran (1.5 mL) were added to a single-neck bottle at room temperature in sequence, triethylamine (33.9 μL, 244 μmol) was added under stirring, and the obtained mixture was reacted at 0°C for 10 min. Then compound 141-1 (50.0 mg, 97.8 μmol), sodium borohydride acetate (124 mg, 587 μmol) and acetic acid (33.5 μL, 587 μmol) were added in sequence, and the obtained mixture was reacted at 25°C for 2 h. Most of the solvent was removed from the reaction solution under vacuum to obtain a residue. The residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 2%-95%, flow rate: 25 mL/min) to obtain compound 145. MS m/z(ESI):893.4[M+1]$^+$. $^1$H NMR (400 MHz,

DMSO-$d_6$) δ 11.10 (s, 1H), 8.70 - 8.62 (m, 2H), 8.50 (s, 1H), 8.34 (s, 1H), 7.20 (t, $J$ = 53.2 Hz, 1H), 7.01 - 6.90 (m, 2H), 6.89 - 6.83 (m, 1H), 6.77 (d, $J$ = 8.0 Hz, 1H), 5.38 - 5.32 (m, 1H), 4.35 - 4.25 (m, 1H), 4.11 - 3.97 (m, 2H), 3.64 (s, 3H), 3.62 - 3.56 (m, 2H), 3.48 - 3.40 (m, 2H), 3.27 - 3.12 (m, 2H), 3.01 - 2.85 (m, 3H), 2.80 - 2.54 (m, 6H), 2.38 - 2.22 (m, 3H), 2.18 - 2.10 (m, 2H), 2.04 - 1.93 (m, 3H), 1.89 - 1.60 (m, 10H), 1.53 - 1.45 (m, 2H), 1.18 - 1.00 (m, 2H).

Example 146: 3-(4-(4-(((1r,4r)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl(cyclohexyl)methyl)piperazin-1-yl)piperidin-1-acyl)-3-methyl-2-oxo-2,3-dihydro-1H-benzo[d]imidazol-1-yl)pyridine-2,6-dione (146)

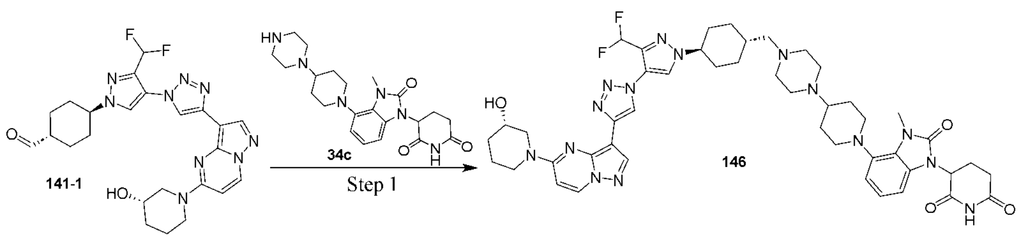


Compound 34c (50.0 mg, 0.117 mmol) was dissolved in *N,N*-dimethylformamide (1 mL) and tetrahydrofuran (1 mL) at 0°C, triethylamine (29.0 mg, 0.290 mmol) was added, and the obtained mixture was stirred for 10 min. Then sodium borohydride acetate (124 mg, 0.590 mmol), compound 141-1 (50.0 mg, 0.098 mmol) and acetic acid (29.0 mg, 0.480 mmol) were added to the mixture, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 15%-95%, flow rate: 25 mL/min) to obtain compound 146. MS m/z(ESI): 922.6[M+1]$^+$. $^1$H NMR (400 MHz, DMSO-$d_6$) δ 11.09 (s, 1H), 8.69 - 8.62 (m, 2H), 8.52 - 8.47 (m, 1H), 8.33 (s, 1H), 7.35 - 7.04 (m, 1H), 7.01 - 6.93 (m, 1H), 6.91 - 6.82 (m, 2H), 6.77 (d, $J$= 8.0 Hz, 1H), 5.40 - 5.29 (m, 1H), 4.33 - 4.22 (m, 1H), 4.15 - 3.95 (m, 2H), 3.67 - 3.56 (m, 5H), 3.24 - 3.06 (m, 4H), 2.94 - 2.83 (m, 1H), 2.77 - 2.55 (m, 7H), 2.44 - 2.24 (m, 5H), 2.19 - 2.06 (m, 4H), 2.05 - 1.69 (m, 10H), 1.65 - 1.45 (m, 5H), 1.17 - 0.97 (m, 2H).

Example 147: 3-(4-(2-(((1S,4r)-4-(3-(difluoromethyl))-4-(4-(5-(((1S,2R)-2-hydroxycyclohexyl)amino)pyrazolo[1,5-a]pyrimidin-3-yl)-1H-1,2,3-triazol-1-yl)-1H-pyrazol-1-yl)cyclohexyl)methyl)-2,8-diazaspiro[4.5]dec-8-yl)-3-methyl-2-oxo-2,3-dihydro-1H-benzo[d]imidazol-1-yl)piperidine-2,6-dione (147)

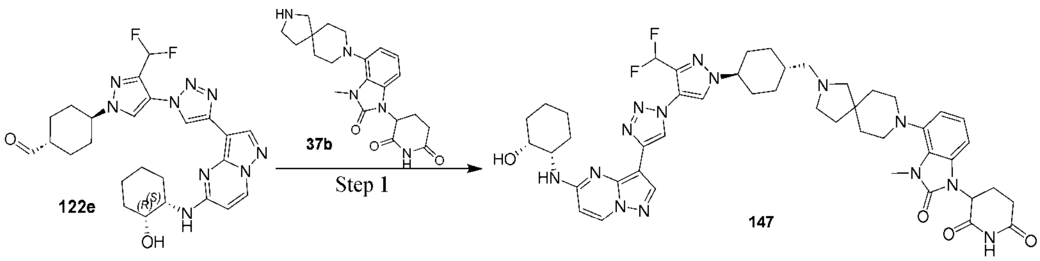


Compound 37b (19.9 mg, 47.6 μmol) was dissolved in a mixed solution of *N,N*-dimethylformamide (1 mL) and tetrahydrofuran (4 mL), triethylamine (4.81 mg, 47.6 μmol) was added, and the obtained mixture was reacted under stirring at room temperature for 15 min. Then compound 122e (25.0 mg, 47.6 μmol) and acetic acid (5.71 mg, 95.2 μmol) were added and the obtained mixture was reacted under stirring at room temperature for 30 min. Sodium triacetoxyborohydride (30.1 mg, 143 μmol) was then added, and the obtained mixture was reacted under stirring at room temperature for 30 min. The reaction system was then directly concentrated, and the residue was purified by high performance liquid chromatography (Waters-2545, chromatographic column: SharpSil-TC18, 30*150 mm, 5 μm; mobile phase: water (containing 10 mmol/L formic acid) and acetonitrile, gradient ratio: acetonitrile 38%-45%, flow rate: 30 mL/min) to obtain compound 147. MS m/z(ESI):907.7[M+1]$^+$. $^1$H NMR (400 MHz, DMSO-$d_6$): δ 11.08 (s, 1H), 8.66 (s, 1H), 8.48 (d, $J$ = 7.6 Hz, 1H), 8.43 (s, 1H), 8.29 (s, 1H), 7.54 - 7.43 (m, 1H), 7.29 (t, $J$ = 53.2 Hz, 1H), 7.00 - 6.91 (m, 2H), 6.89 - 6.84 (m, 1H), 6.49 (d, $J$ = 7.6 Hz, 1H), 5.37 - 5.32 (m, 1H), 4.33 - 4.25 (m, 1H), 4.12 - 4.06 (m, 1H), 3.97 - 3.93 (m, 1H), 3.64 (s, 3H), 3.03 - 2.83 (m, 4H), 2.74 -

2.60 (m, 3H), 2.36 - 2.23 (m, 3H), 2.17 - 2.10 (m, 2H), 2.06 - 1.87 (m, 4H), 1.86 - 1.49 (m, 17H), 1.40 - 1.31 (m, 2H), 1.27 - 1.22 (m, 1H), 1.15 - 1.04 (m, 2H).

Example 148: 3-(4-(9-(((1S,4r)-4-(3-(difluoromethyl))-4-(4-(5-(((1S,2R)-2-hydroxycyclohexyl)amino)pyrazolo[1,5-a]pyrimidin-3-yl)-1H-1,2,3-triazol-1-yl)-1H-pyrazol-1-yl)cyclohexyl)methyl)-3,9-diazaspiro[5.5]undecan- 3-yl)-3-methyl-2-oxo-2,3-dihydro-1H-benzo[d]imidazol-1-yl)piperidine-2,6-dione (148)

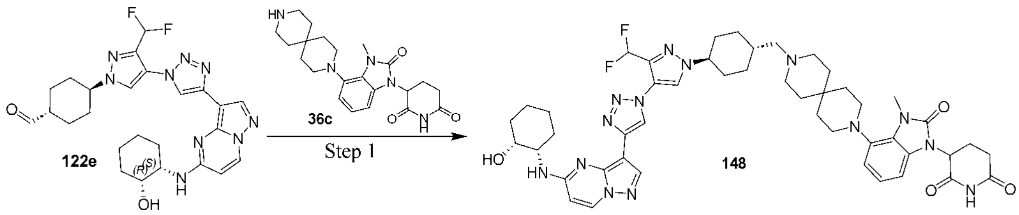


Compound 36c (19.6 mg, 47.6 μmol) was dissolved in a mixed solution of *N,N*-dimethylformamide (1 mL) and tetrahydrofuran (4 mL), triethylamine (4.81 mg, 47.6 μmol) was added, and the obtained mixture was reacted under stirring at room temperature for 15 min. Then compound 122e (25.0 mg, 47.6 μmol) and acetic acid (5.71 mg, 95.2 μmol) were added and the obtained mixture was reacted under stirring at room temperature for 30 min. Sodium triacetoxyborohydride (30.1 mg, 143 μmol) was then added, and the obtained mixture was reacted under stirring at room temperature for 30 min. The reaction system was then directly concentrated, and the residue was purified by high performance liquid chromatography (Waters-2545, chromatographic column: SharpSil-TC18, 30×150 mm, 5 μm; mobile phase: water (containing 10 mmol/L formic acid) and acetonitrile, gradient ratio: acetonitrile 38%-45%, flow rate: 30 mL/min) to obtain compound 148. MS m/z(ESI):921.7[M+1]$^+$. $^1$H NMR (400 MHz, DMSO-$d_6$): δ 11.08 (s, 1H), 8.65 (s, 1H), 8.48 (d, *J* = 7.6 Hz, 1H), 8.43 (s, 1H), 8.26 (s, 1H), 7.52 - 7.41 (m, 1H), 7.22 (t, *J* = 53.1 Hz, 1H), 6.99 - 6.94 (m, 2H), 6.88 - 6.83 (m, 1H), 6.49 (d, *J* = 7.6 Hz, 1H), 5.37 - 5.31 (m, 1H), 4.33 - 4.25 (m, 1H), 4.12 - 4.06 (m, 1H), 3.97 - 3.94 (m, 1H), 3.63 (s, 3H), 2.91 - 2.84 (m, 5H), 2.72 - 2.59 (m, 2H), 2.39 - 2.32 (m, 4H), 2.20 - 2.11 (m, 4H), 2.01 - 1.91 (m, 3H), 1.79 - 1.52 (m, 14H), 1.45 - 1.22 (m, 6H), 1.13 - 1.02 (m, 2H).

Example 149: 3-(4-(4-(1-(((1S,4r)-4-(3-(difluoromethyl)-4-(4-(5-((((1S,2R)-2-hydroxycyclohexyl)amino)pyrazolo[1,5-a]pyrimidin-3-yl)-1H-1,2,3-triazol-1-yl)-1H-pyrazol-1-yl(cyclohexyl)methyl)piperidin-4-yl)piperazin-1-yl)-3-methyl-2-oxo-2,3-dihydro-1H-benzo[d]imidazol-1-yl)piperidine-2,6-dione (149)

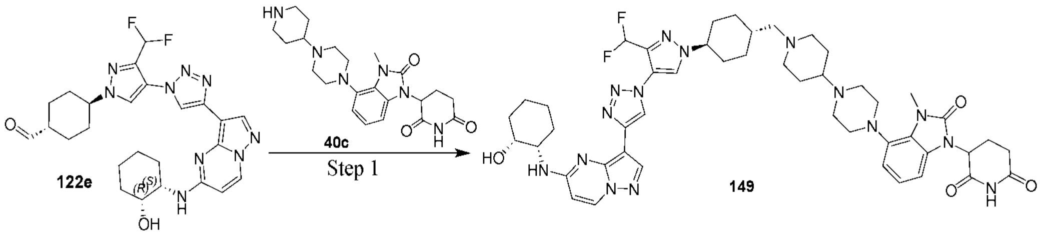


Compound 40c (50.0 mg, 0.117 mmol) was dissolved in *N,N*-dimethylformamide (1 mL) and tetrahydrofuran (1 mL) at 0°C, triethylamine (28.0 mg, 0.277 mmol) was added, and the obtained mixture was stirred for 10 min. Then sodium borohydride acetate (117 mg, 0.552 mmol), compound 122e (70.0 mg, 0.133 mmol) and acetic acid (28.0 mg, 0.467 mmol) were added to the mixture, and the mixture was stirred at 25°C for 1 h. The mixture was concentrated to obtain a residue. The reaction solution was concentrated under reduced pressure and then purified by high performance liquid chromatography (Gilson_306_1741, chromatographic column: Waters-Xbridge-C18-10μm-19×250 mm; mobile phase: water (containing 10 mmol/L ammonium bicarbonate) and acetonitrile, gradient ratio: acetonitrile 38%-95%, flow rate: 25 mL/min) to obtain compound 149. MS m/z(ESI): 468.8[M/2+1]$^+$. $^1$H NMR (400 MHz, DMSO-$d_6$) δ 11.09 (s, 1H), 8.66 (s, 1H), 8.49 (d, *J* = 7.6 Hz, 1H), 8.43 (s, 1H), 8.26 (s, 1H), 7.51 - 7.41 (m, 1H), 7.22 (t, *J* = 53.2 Hz, 1H), 7.02 - 6.84 (m, 3H), 6.49 (d, *J* = 7.7 Hz, 1H), 5.39 - 5.30 (m, 1H), 4.70 (d, *J* = 3.9 Hz, 1H), 4.33 - 4.23 (m, 1H), 4.15 - 4.04 (m, 1H), 3.95 (s, 1H), 3.62 (s, 3H), 3.10 - 2.83 (m, 8H), 2.70 - 2.58 (m, 2H), 2.29 - 2.04 (m, 6H), 1.96 - 1.68 (m, 11H), 1.64 - 1.27 (m, 11H), 1.15 - 0.98 (m, 2H).

Example 150: 3-(4-(4-(1R,4R)-4-(4-(4-(5-(S)-3-aminopiperidin-1-yl)pyrazolo[1,5-a]pyrimidin-3-yl)-1H-1,2,3-triazol-1-yl)-3-(difluoromethyl)-1H-pyrazol-1-yl)cyclohexyl)methyl)piperazin-1-yl)piperidin-1-yl)-3-methyl-2-oxo-2,3-dihydro-1H-benzoimidazol-1-yl)piperidine-2,6-dione hydrochloride (150)

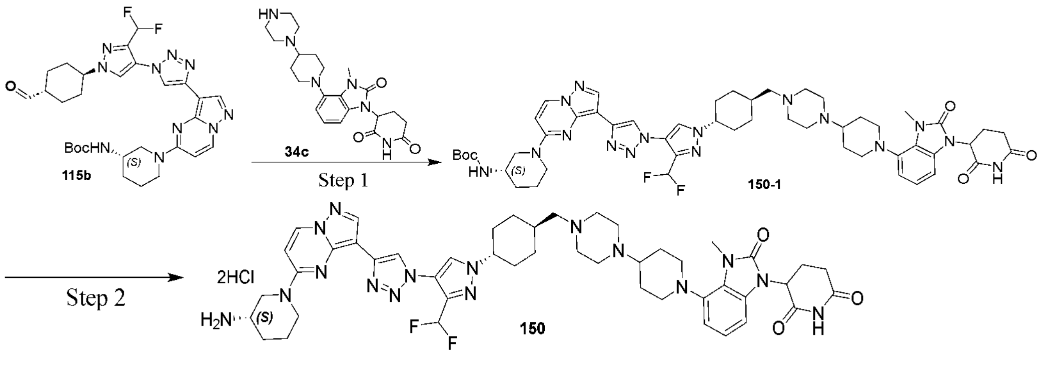


1) Step 1: Triethylamine (0.20 mL, 1.44 mmol) was added to a solution containing compound 34c (50.0 mg, 117 μmol), tetrahydrofuran (2 mL) and *N,N*-dimethylformamide (1 mL), and the obtained mixture was stirred for 30 min. Then compound 115b (72.0 mg, 120 μmol) and acetic acid (0.5 mL) were added to the above solution and the obtained mixture was stirred for 30 min. Finally, sodium borohydride acetate (75.0 mg, 350 μmol) was added to the above reaction solution. The reaction was stirred at 25°C for 2 h. The reaction solution was concentrated under reduced pressure and then purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 150-1. MS m/z(ESI):1021.7[M+1]$^+$.
2) Step 2: Compound 150-1 (10.0 mg, 9.8 μmol) was added to tetrahydrofuran (1 mL) at room temperature, and hydrogen chloride/dioxane (1 mL, 4 M) was added, and the reaction solution was stirred for 1 h. The reaction solution was concentrated under reduced pressure to remove the solvent, and pure water (5 mL) was added, and the obtained mixture was lyophilized under reduced pressure. Compound 150 was obtained. MS m/z(ESI):921.7[M+1]$^+$,

Example 151: 3-(4-(9-((1R,4R)-4-(3-difluoromethyl)-4-(4-(5-((1R,2S)-2-hydroxycyclohexyl)amino)pyrazolo[1,5-a]pyrimidin-3-yl)-1H-1,2,3-triazol-1-yl)-1H-pyrazol-1-yl)cyclohexyl)methyl)-3,9-diazaspiro[5.5]undecan-3-yl)-3-methyl-2-oxo-2,3-dihydro-1H-benzoimidazol-1-yl)piperidine-2,6-dione (151)

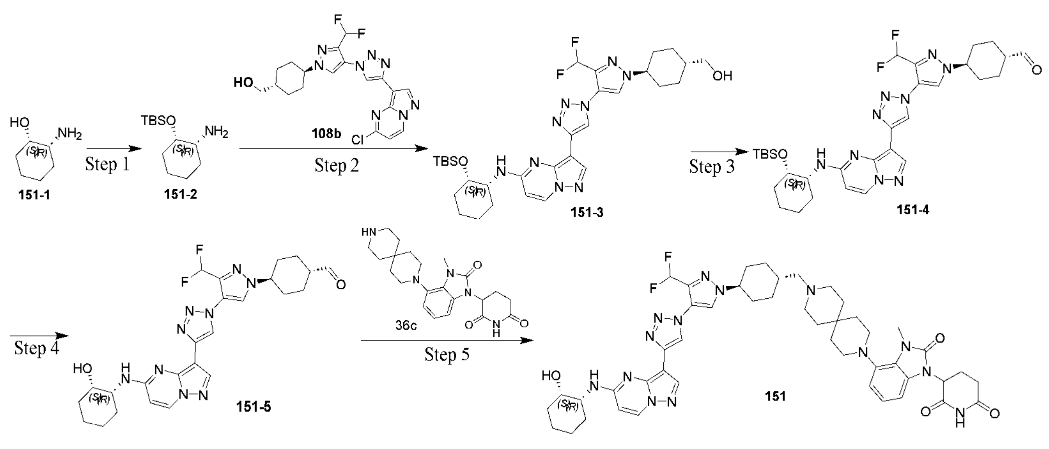


1) Step 1: Tert-butyldimethylsilyl chloride (502 mg, 3.33 mmol) and triethylamine (0.77 mL, 5.56 mmol) were added to a solution of compound 151-1 (320 mg, 2.78 mmol) in dichloromethane (5 mL) at room temperature and the obtained mixture was stirred for 2 h. Saturated ammonium chloride solution (5 mL) was added to the reaction solution, the organic layer was separated, and the aqueous phase was extracted with dichloromethane (5 mL × 3). The organic phases were combined, dried over anhydrous sodium sulfate, filtered, and spin-dried to obtain compound 151-2, the

crude product was directly used in the next step without further purification. MS m/z(ESI):230.6[M+1]$^+$.

2) Step 2: *N*,*N*-diisopropylethylamine (1.30 mL, 7.35 mmol) and compound 108b (1.41 g, 3.14 mmol) were added to a solution of compound 151-2 (600 mg, 2.62 mmol) in acetonitrile (15 mL) at room temperature, and the reaction was stirred at 60°C for 18 h. The reaction was quenched by adding saturated ammonium chloride solution (30 mL) to the reaction solution. The liquid separation was performed. The aqueous phase was extracted 3 times with ethyl acetate (30 mL). The organic phases were combined, dried over anhydrous sodium sulfate, filtered, and spin-dried to obtain compound 151-3, the crude product was directly used in the next step without further purification. MS m/z(ESI):642.4 [M+1]$^+$.

3) Step 3: Dess-Martin oxidant (178 mg, 0.420 mmol) was added to a solution of compound 151-3 (180 mg, 0.289 mmol) in dichloromethane (2 mL) at room temperature, and the reaction was stirred at room temperature for 2 h. Saturated sodium bicarbonate solution (2 mL) was added to the reaction solution to quench the reaction. The liquid separation was performed. The aqueous phase was extracted with dichloromethane (2 mL) 3 times. The organic phases were combined, dried over anhydrous sodium sulfate, filtered, and spin-dried to obtain compound 151-4, the crude product was directly used in the next step without further purification. MS m/z(ESI):640.4[M+1]$^+$.

4) Step 4: Tetrabutylammonium fluoride (0.940 mL, 940 μmmol, 1.0 M solution in tetrahydrofuran) was added to a solution of compound 151-4 (200.0 mg, 310 μmmol) in tetrahydrofuran (2 mL) under nitrogen protection. The reaction was subjected to nitrogen replacement three times and then reacted at 25°C for 3 h. Saturated sodium bicarbonate (20 mL) was added to the reaction solution, and ethyl acetate was used for extraction (20 mL × 3). The organic phases were combined, dried over anhydrous sodium sulfate and filtered to remove the desiccant, and then the filtrate was concentrated under reduced pressure. The residue was purified by silica gel column chromatography (dichloromethane/methanol = 100/1 to 30/1) to obtain compound 151-5. MS m/z(ESI):526.8[M+1]$^+$.

5) Step 5: Triethylamine (0.20 mL, 1.44 mmol) was added to a solution of compound 36c (65.0 mg, 150 μmmol), tetrahydrofuran (2 mL) and *N,N*-dimethylformamide (1 mL), and the obtained mixture was stirred for 30 min. Then compound 151-5 (40.0 mg, 80 μmmol) and acetic acid (0.5 mL) were added to the above solution and the obtained mixture was stirred for 30 min. Finally, sodium borohydride acetate (65.0 mg, 300 μmol) was added to the above reaction solution. The reaction was stirred at 25°C for 2 h. The reaction solution was concentrated under reduced pressure and then purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 2%-95%, flow rate: 25 mL/min) to obtain compound 151. MS m/z(ESI):921.7[M+1]$^+$. $^1$H NMR (400 MHz, DMSO-$d_6$) δ 11.09 (s, 1H), 8.66 (s, 1H), 8.49 (d, *J* = 7.6 Hz, 1H), 8.43 (s, 1H), 8.37 (s, 1H), 8.27 (s, 1H), 7.51 - 7.44 (m, 1H), 7.23 (t, *J* = 53.1 Hz, 1H), 6.99 - 6.95 (m, 2H), 6.89 - 6.85 (m, 1H), 6.50 (d, *J* = 7.6 Hz, 1H), 5.37 - 5.30 (m, 1H), 4.79 - 4.63 (m, 1H), 4.34 - 4.24 (m, 1H), 4.16 - 4.07 (m, 1H), 3.96 (s, 1H), 3.64 (s, 3H), 2.90 - 2.86 (m, 4H), 2.71 - 2.61 (m, 2H), 2.38 - 2.34 (m, 4H), 2.19 - 2.14 (m, 4H), 2.00 - 1.91 (m, 2H), 1.86 - 1.80 (m, 2H), 1.76 - 1.65 (m, 6H), 1.63 - 1.52 (m, 8H), 1.45 - 1.40 (m, 2H), 1.37 - 1.32 (m, 2H), 1.13 - 1.04 (m, 2H).

Example 152: 3-(4-(8-(((1R,4r)-4-(3-(difluoromethyl))-4-(4-(5-(((1R,2S)-2-hydroxycyclohexyl)amino)pyrazolo[1,5-a] pyrimidin-3-yl)-1H-1,2,3-triazol-1-yl)-1H-pyrazol-1-yl)cyclohexyl)methyl)-2,8-diazaspiro[4.5]dec-2-yl)-3-methyl-2-oxo-2,3-dihydro-1H-benzo[d]imidazol-1-yl)piperidine-2,6-dione (152)

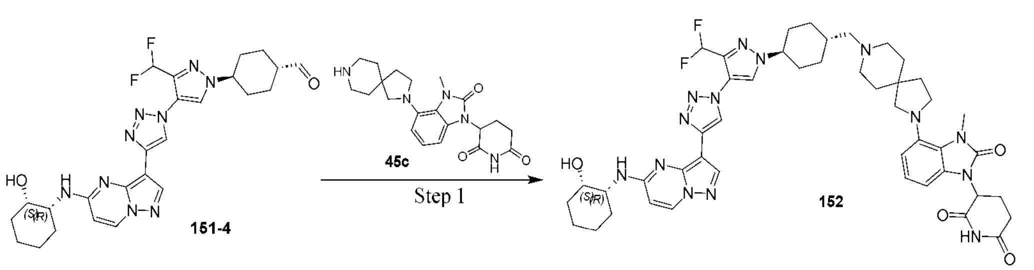


Triethylamine (5.78 mg, 57.1 umol) was added to a solution of compound 45c (27.2 mg, 68.4 umol) in *N*,*N*-dimethylformamide (1 mL) at room temperature and the obtained mixture was stirred for 1 min. Acetic acid (3.43 mg, 57.1 umol) and sodium triacetylborohydride (60.2 mg, 0.29 mmol) were added, and the obtained mixture was stirred for 1 min. Compound 151-4 (30.0 mg, 57.1 umol) was added, and the obtained mixture was stirred for 30 min. Water (0.2 mL) was added to the reaction solution to quench the reaction, and liquid separation was performed. The aqueous phase was extracted with dichloromethane (1 mL) 3 times. The organic phases were combined, dried over anhydrous sodium sulfate, filtered, and spin-dried. The residue was purified by high performance liquid chromatography (Waters-2545, chromatographic column: SharpSil-T C18, 30×150 mm, 5 μm; mobile phase: water (containing 10 mmol/L formic acid) and

acetonitrile, gradient ratio: acetonitrile 38%-45%, flow rate: 30 mL/min) to obtain compound 152. MS m/z(ESI):907.6 $[M+1]^+$.

Example 153: 3-(4-(4-(1-(1R,4R)-4-(3-difluoromethyl)-4-(4-(5-((1R,2S)-2-hydroxycyclohexylamino)pyrazolo[1,5-a]pyrimidin-3-yl)-1H-1,2,3-triazol-1-yl)-1H-pyrazol-1-yl)cyclohexyl)methyl)piperidin-4-yl)piperazin-1-yl)-3-methyl-2-oxo-2,3-dihydro-1H-benzoimidazol-1-yl)piperidine-2,6-dione (153)

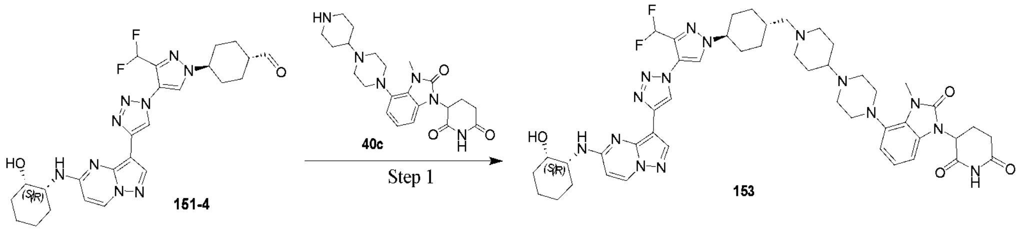


Triethylamine (0.20 mL, 1.44 mmol) was added to a solution of compound 40c (30.0 mg, 70 μmol), tetrahydrofuran (2 mL) and *N,N*-dimethylformamide (1 mL), and the obtained mixture was stirred for 30 min. Then compound 151-4 (45.0 mg, 86 μmol) and acetic acid (0.5 mL) were added to the above solution and the obtained mixture was stirred for 30 min. Finally, sodium borohydride acetate (45.0 mg, 210 μmol) was added to the above reaction solution. The reaction was stirred at 25°C for 2 h. The reaction solution was concentrated under reduced pressure and then purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 153. MS m/z(ESI):936.6$[M+1]^+$. $^1$H NMR (400 MHz, DMSO-$d_6$) δ 11.09 (s, 1H), 8.66 (s, 1H), 8.49 (d, *J* = 7.7 Hz, 1H), 8.43 (s, 1H), 8.33 (s, 2H), 8.27 (s, 1H), 7.64 - 7.45 (m, 1H), 7.23 (t, *J* = 53.1 Hz, 1H), 7.02 - 6.96 (m, 1H), 6.94 - 6.85 (m, 2H), 6.50 (d, *J* = 7.6 Hz, 1H), 5.36 - 5.33 (m, 1H), 4.34 - 4.25 (m, 1H), 4.14 - 4.06 (m, 1H), 3.99 - 3.93 (m, 1H), 3.63 (s, 3H), 3.10 - 2.84 (m, 8H), 2.76 - 2.58 (m, 4H), 2.36 - 2.31 (m, 1H), 2.27 - 2.18 (m, 2H), 2.15 - 2.11 (m, 3H), 2.03 - 1.98 (m, 3H), 1.91 - 1.87 (m, 2H), 1.81 - 1.74 (m, 4H), 1.63 - 1.56 (m, 4H), 1.50 - 1.42 (m, 3H), 1.37 - 1.32 (m, 2H), 1.20 - 1.05 (m, 2H), 0.87 - 0.83 (m, 1H).

Example 154: 3-(4-(2-(1R,4R)-4-(3-difluoromethyl)-4-(4-(5-((1R,2S)-2-hydroxycyclohexyl)amino)pyrazolo[1,5-a]pyrimidin-3-yl)-1H-1,2,3-triazol-1-yl)-1H-pyrazol-1-yl)cyclohexyl)methyl)-2,8-diazaspiro[4.5]dec-8-yl)-3-methyl-2-oxo-2,3-dihydro-1H-benzoimidazol-1-yl)piperidine-2,6-dione (154)

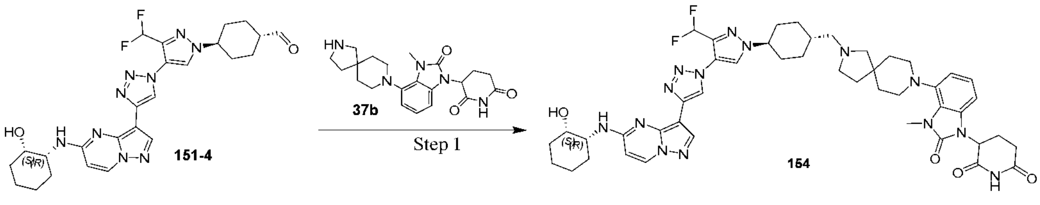


Triethylamine (0.20 mL, 1.44 mmol) was added to a solution containing compound 37b (20.0 mg, 50 μmol), tetrahydrofuran (2 mL) and *N,N*-dimethylformamide (1 mL), and the obtained mixture was stirred for 30 min. Then compound 151-4 (40.0 mg, 76 μmol) and acetic acid (0.5 mL) were added to the above solution and the obtained mixture was stirred for 30 min. Finally, sodium borohydride acetate (32.0 mg, 150 μmol) was added to the above reaction solution. The reaction was stirred at 25°C for 2 h. The reaction solution was concentrated under reduced pressure and then purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-90%, flow rate: 25 mL/min) to obtain compound 154. $^1$H NMR (400 MHz, DMSO-$d_6$) δ 11.09 (s, 1H), 8.66 (s, 1H), 8.49 (d, *J* = 7.6 Hz, 1H), 8.43 (s, 1H), 8.30 - 8.28 (m, 1H), 8.27 (s, 1H), 7.52 - 7.44 (m, 1H), 7.23 (t, *J* = 53.2 Hz, 1H), 7.00 - 6.91 (m, 2H), 6.90 - 6.85 (m, 1H), 6.50 (d, *J* = 7.6 Hz, 1H), 5.37 - 5.32 (m, 1H), 4.80 - 4.60 (m, 1H), 4.35 - 4.26 (m, 1H), 4.15 - 4.04 (m, 1H), 3.99 - 3.94 (m, 1H), 3.64 (s, 3H), 3.05 - 2.92 (m, 3H), 2.90 - 2.84 (m, 1H), 2.80 - 2.71 (m, 2H), 2.67 - 2.59 (m, 2H), 2.39 - 2.30 (m, 2H), 2.29 - 2.21 (m, 2H), 2.18 - 2.11 (m, 2H), 2.02 - 1.96 (m, 4H), 1.78 - 1.67 (m, 7H), 1.60 - 1.50 (m, 4H), 1.48 - 1.41 (m, 1H), 1.38 - 1.30 (m, 3H), 1.20 - 1.05 (m, 2H), 0.87 - 0.83 (m, 1H).

Example 155: 3-(4-(4-((1R,4R)-4-(3-difluoromethyl)-4-(4-(5-((1R,2S)-2-hydroxycyclohexyl)amino)pyrazolo[1,5-a]pyrimidin-3-yl)-1H-1,2,3-triazol-1-yl)-1H-pyrazol-1-yl)cyclohexyl)methyl)piperazin-1-yl)piperidin-1-yl)-3-methyl-2-oxo-2,3-dihydro-1H-benzoimidazol-1-yl)piperidine-2,6-dione (155)

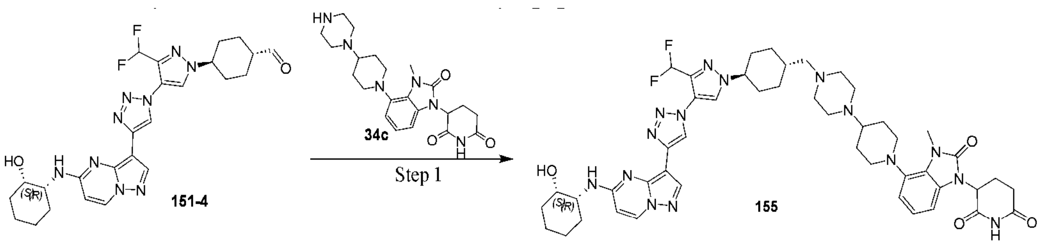


Triethylamine (0.20 mL, 1.44 mmol) was added to a solution containing compound 34c (100.0 mg, 230 μmmol), tetrahydrofuran (2 mL) and *N,N*-dimethylformamide (1 mL), and the obtained mixture was stirred for 30 min. Then compound 151-4 (150.0 mg, 286 μmmol) and acetic acid (0.5 mL) were added to the above solution and the obtained mixture was stirred for 30 min. Finally, sodium borohydride acetate (150 mg, 707 μmmol) was added to the above reaction solution. The reaction was stirred at 25°C for 2 h. The reaction solution was concentrated under reduced pressure and then purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 2%-95%, flow rate: 25 mL/min) to obtain compound 155. MS m/z(ESI):936.9[M+1]$^+$. $^1$H NMR (400 MHz, DMSO-$d_6$): δ 11.09 (s, 1H), 8.66 (s, 1H), 8.49 (d, *J* = 7.6 Hz, 1H), 8.43 (s, 1H), 8.27 (s, 1H), 7.49 - 7.44 (m, 1H), 7.23 (t, *J* = 53.2 Hz, 1H), 7.01 - 6.94 (m, 1H), 6.92 - 6.85 (m, 2H), 6.50 (d, *J* = 7.6 Hz, 1H), 5.38 - 5.33 (m, 1H), 4.73 - 4.67 (m, 1H), 4.34 - 4.24 (m, 1H), 4.13 - 4.06 (m, 1H), 3.96 (s, 1H), 3.63 (s, 3H), 3.17 - 3.12 (m, 2H), 2.94 - 2.83 (m, 1H), 2.75 - 2.63 (m, 4H), 2.59 - 2.54 (m, 3H), 2.39 - 2.27 (m, 4H), 2.17 - 2.11 (m, 4H), 2.03 - 1.96 (m, 2H), 1.95 - 1.86 (m, 4H), 1.83 - 1.70 (m, 4H), 1.63 - 1.53 (m, 6H), 1.43 - 1.22 (m, 4H), 1.12 - 1.02 (m, 2H).

Example 156: 1-(1-(3-(difluoromethyl)-1-((18S,4r)-4-(((2S)-4-(1-(2,6-dioxopiperidin-3-yl)-3-methyl-2-oxo-3,3-dihydro-1H-benzo[d]imidazol-4-yl)-2-methylpiperazin-1-yl)methyl)cyclohexyl)-1H-pyrazol-4-yl)-1H-pyrrolo[2,3-b]pyridine-5-carboxamide (156)

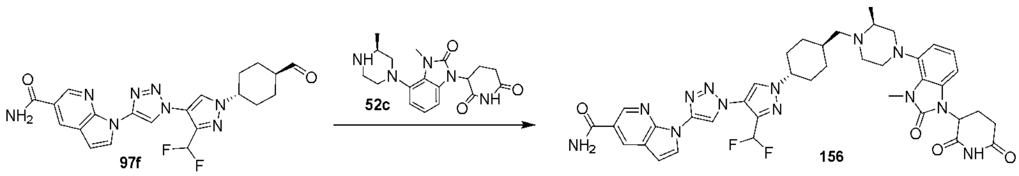


Compound 52c (30 mg, 0.084 mmol) and compound 97f (60 mg, 0.13 mmol) were dissolved in *N*,*N*-dimethylformamide (3 mL), and acetic acid (0.5 mL) and sodium borohydride acetate (90 mg, 0.43 mmol) were added in sequence. The reaction solution was stirred at 25°C for 2 h. The reaction solution was poured into water (10 mL), and dichloromethane (10 mL) was used for extraction three times. The combined organic phase was washed with saturated brine (30 mL), dried over anhydrous sodium sulfate and filtered. The filtrate was concentrated under reduced pressure, and the residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 40%-55%, flow rate: 25 mL/min) to obtain compound 156. MS m/z(ESI):796.6 [M+H]$^+$. $^1$H NMR (400 MHz, DMSO-$d_6$): δ 11.10 (s, 1H), 9.05 (s, 1H), 8.91 (d, *J* = 2.0 Hz, 1H), 8.79 (s, 1H), 8.63 (d, *J* = 2.0 Hz, 1H), 8.35 (s, 1H), 8.28 (d, *J* = 3.7 Hz, 1H), 7.51 (s, 1H), 7.25 (t, *J* = 53.4 Hz, 1H), 7.02 - 6.83 (m, 4H), 5.41 - 5.31 (m, 1H), 4.38 - 4.27 (m, 1H), 3.65 (s, 3H), 3.10 - 2.81 (m, 6H), 2.77 - 2.56 (m, 4H), 2.29 - 1.95 (m, 6H), 1.90 - 1.57 (m, 4H), 1.24 - 1.01 (m, 5H).

Example 161: 1-(6-(4-(4-(((1R,4r)-4-(3-(difluoromethyl)-4-(4-(5-((((3R,2S)-2-hydroxycyclohexyl)amino)pyrazolo[1,5-a] pyrimidin-3-yl)-1H-1,2,3-triazol-1-yl)-1H-pyrazol-1-yl(cyclohexyl)methyl)piperazin-1-yl)piperidin-1-yl)-1-methyl-1H-indazol-3-yl)dihydropyrimidin-2,4(1H,3H)-dione (161)

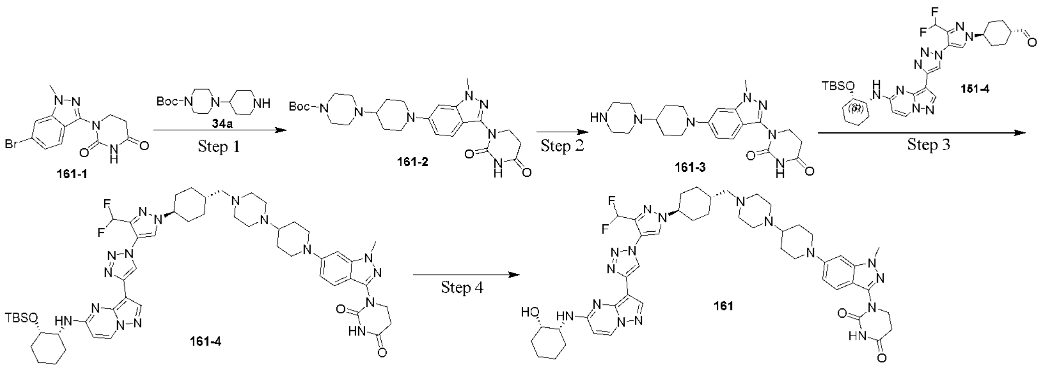


1) Step 1: Compound 161-1 (500 mg, 1.55 mmol, prepared by the method disclosed for the step-3 product on page 109 of the specification of "WO 2023283610 A1"), compound 34a (585 mg, 2.17 mmol), sodium tert-butoxide (446 mg, 4.64 mmol, Bide Pharmatech Ltd.) and di(tri-tert-butylphosphine)palladium (79.1 mg, 0.15 mmol, Bide Pharmatech Ltd.) were dissolved in dimethyl sulfoxide (15 mL), the reaction solution was subjected to nitrogen replacement three times and stirred at 100°C for 1 h. The reaction solution was diluted with (30 mL) and extracted with ethyl acetate (10 mL × 3). The combined organic phase was washed with saturated brine (10 mL), dried over anhydrous sodium sulfate and filtered, and the filtrate was concentrated under reduced pressure. The residue was purified by silica gel column chromatography (dichloromethane/methanol = 100/1 to 30/1) to obtain compound 161-2. MS m/z(ESI):512.9[M+1]$^+$. $^1$H NMR (400 MHz, $CDCl_3$): δ 7.58 - 7.51 (m, 2H), 6.92 (dd, *J* = 9.1, 1.8 Hz, 1H), 6.58 (d, *J* = 1.6 Hz, 1H), 4.09 (t, *J* = 6.7 Hz, 2H), 3.92 (s, 3H), 3.86 - 3.80 (m, 2H), 3.48 (s, 4H), 2.88 (t, *J* = 6.7 Hz, 2H), 2.80 (t, *J* = 11.3 Hz, 2H), 2.62 - 2.48 (m, 5H), 2.00 - 1.91 (m, 2H), 1.78 - 1.70 (m, 2H), 1.48 (s, 9H).
2) Step 2: Compound 161-2 (250 mg, 0.490 mmol) was dissolved in a solution of hydrogen chloride in dioxane (5 mL, 4.0 M), and the reaction solution was stirred at 25°C for 0.5 h. The reaction solution was concentrated under reduced pressure to obtain compound 161-3 (201 mg). MS m/z(ESI):412.3[M+1]$^+$.
3) Step 3: Compound 161-3 (41.8 mg, 0.100 mmol) was dissolved in *N,N*-dimethylformamide (2 mL), and triethylamine (7.91 mg, 80.0 μmol) was added at 25°C. The reaction solution was stirred at 25°C for 5 min, and then acetic acid (4.69 mg, 80.0 μmol) and sodium borohydride acetate (65.9 mg, 0.310 mmol, Bide Pharmatech Ltd.) were added. The reaction solution was stirred for 5 min, and then compound 151-4 (50.0 mg, 80.0 μmol) was added and the reaction solution was stirred for 1 h. The reaction solution was poured into water (2 mL) and dichloromethane (5 mL), and the aqueous phase was extracted with dichloromethane (5 mL × 3). The combined organic phase was dried over anhydrous sodium sulfate and filtered to remove the desiccant, and then the filtrate was concentrated under reduced pressure. The residue was purified on silica gel plate for thin layer chromatography (dichloromethane/methanol (7.0 M ammonia solution) = 20/1) to obtain compound 161-4. MS m/z(ESI):1034.0[M-H]$^-$.
4) Step 4: Compound 161-4 (31.0 mg, 30.0 μmol) was dissolved in hydrogen chloride/dioxane solution (1 mL, 4.0 M), and the reaction solution was stirred at 25°C for 1 h. The reaction solution was concentrated under reduced pressure, and the obtained residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 161 (1.15 mg). MS m/z(ESI):922.0 [M+1]$^+$.

Example 166: 3-(4-(4-((S)-4-(((1R,4S)-4-(3-(difluoromethyl)-4-(4-(5-((((3R,2S)-2-hydroxycyclohexyl)amino)pyrazolo [1,5-a]pyrimidin-3-yl)-1H-1,2,3-triazol-1-yl)-1H-pyrazol-1-yl(cyclohexyl)methyl)-2-methylpiperazin-1-yl)piperidin-1-yl)-3-methyl-2-oxo-2,3-dihydro-1H-benzo[d]imidazol-1-yl)pyridine-2,6-dione (166)

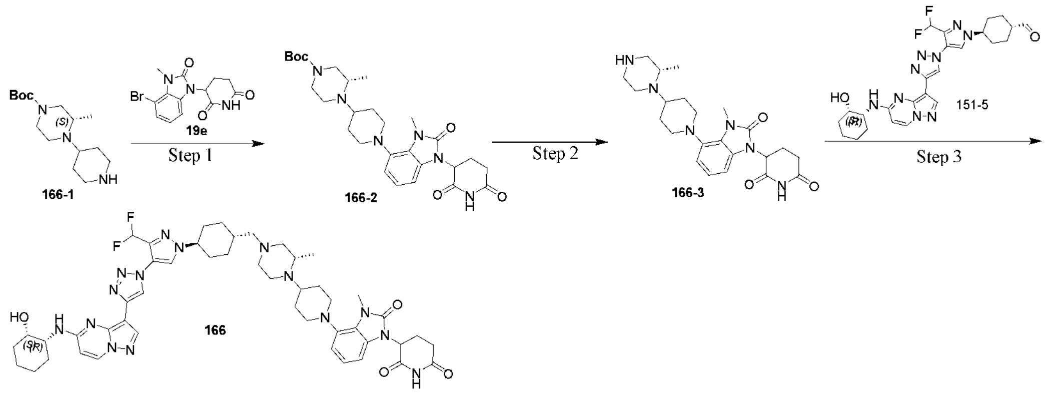


1) Step 1: Compound 166-1 (414 mg, 1.46 mmol, prepared by the method disclosed in Preparation 22 on page 44 of the specification of the patent application "WO 2013163262 A1"), compound 19e (380 mg, 1.12 mmol, prepared by the method disclosed for step 3 product on page 88 of the specification of the patent application "WO 2022012623 A"), 2-dicyclohexylphosphino-2',6'-diisopropoxy-1,1'-biphenyl (105 mg, 0.220 mmol) and (2-dicyclohexylphosphino-2',6'-diisopropoxy-1,1'-biphenyl)(2-amino-1,1'-biphenyl-2-yl) palladium (II) methanesulfonate (188 mg, 0.220 mmol) were added to a single-neck bottle at room temperature in sequence, and then toluene (7 mL) was added. The reaction was subjected to nitrogen replacement three times, and lithium bis(trimethylsilyl)amide (5.62 mL, 5.62 mmol, 1.0 M solution in tetrahydrofuran) was added under stirring, and the obtained mixture was stirred at 80°C for 2 h. Water (50 mL) was added to the reaction solution, and the obtained mixture was extracted with ethyl acetate (50 mL × 3). The organic layers were combined, washed with brine (30 mL), dried over anhydrous sodium sulfate and filtered, and the filtrate was concentrated. The residue was purified by silica gel column chromatography (methanol/dichloromethane = 0%-10%) to obtain compound 166-2. MS m/z(ESI):541.3[M+1]$^+$.

2) Step 2: Compound 166-2 (70.0 mg, 0.130 mmol) and dichloromethane (2 mL) were added to a single-neck bottle at room temperature, trifluoroacetic acid (0.250 mL) was added under stirring, and the obtained mixture was reacted at 25°C for 2 h. The reaction solution was concentrated to obtain compound 166-3. MS m/z(ESI):441.0[M+1]$^+$.

3) Step 3: Compound 166-3 (97.0 mg, 0.180 mmol), *N,N*-dimethylformamide (2 mL) and tetrahydrofuran (1.2 mL) were added to a single-neck bottle at room temperature in sequence, triethylamine (62.4 μL, 0.450 mmol) was added under stirring, and the obtained mixture was stirred at 0°C for 10 min. Then compound 151-5 (87.0 mg, 0.170 mmol), sodium borohydride acetate (228 mg, 1.08 mmol) and acetic acid (61.8 μL, 1.08 mmol) were added in sequence, and the obtained mixture was stirred at 25°C for 2 h. Most of the solvent was removed from the reaction solution under vacuum, and the residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 2%-95%, flow rate: 25 mL/min) to obtain compound 166. MS m/z(ESI):950.4[M+1]$^+$. $^1$H NMR (400 MHz, DMSO-$d_6$) δ 11.09 (s, 1H), 8.66 (s, 1H), 8.49 (d, *J* = 7.6 Hz, 1H), 8.43 (s, 1H), 8.26 (s, 1H), 7.46 (d, *J* = 7.8 Hz, 1H), 7.22 (t, *J* = 53.2 Hz, 1H), 7.02 - 6.82 (m, 3H), 6.49 (d, *J* = 7.6 Hz, 1H), 5.35 (dd, *J* = 12.6, 5.4 Hz, 1H), 4.69 (s, 1H), 4.36 - 4.23 (m, 1H), 4.18 - 4.02 (m, 1H), 3.95 (s, 1H), 3.63 (s, 3H), 3.19 - 3.13 (m, 2H), 3.07 - 2.84 (m, 4H), 2.83 - 2.56 (m, 7H), 2.36 - 2.02 (m, 6H), 2.02 - 1.69 (m, 10H), 1.68 - 1.49 (m, 6H), 1.39 - 1.28 (m, 2H), 1.19 - 0.99 (m, 5H).

Example 171: 3-(4-(4-((9-(3-(difluoromethyl)-4-(4-(5-(((1R,2S)-2-hydroxycyclohexyl)amino)pyrazolo[1,5-a]pyrimidin-3-yl)-1H-1,2,3-triazol-1-yl)-1H-pyrazol-1-yl]-3-azaspiro[5.5]undecan-3-yl)methyl)piperidin-1-yl)-3-methyl-2-oxo-2,3-dihydro-1H-benzo[d]imidazol-1-yl)piperidine-2,6-dione (171)

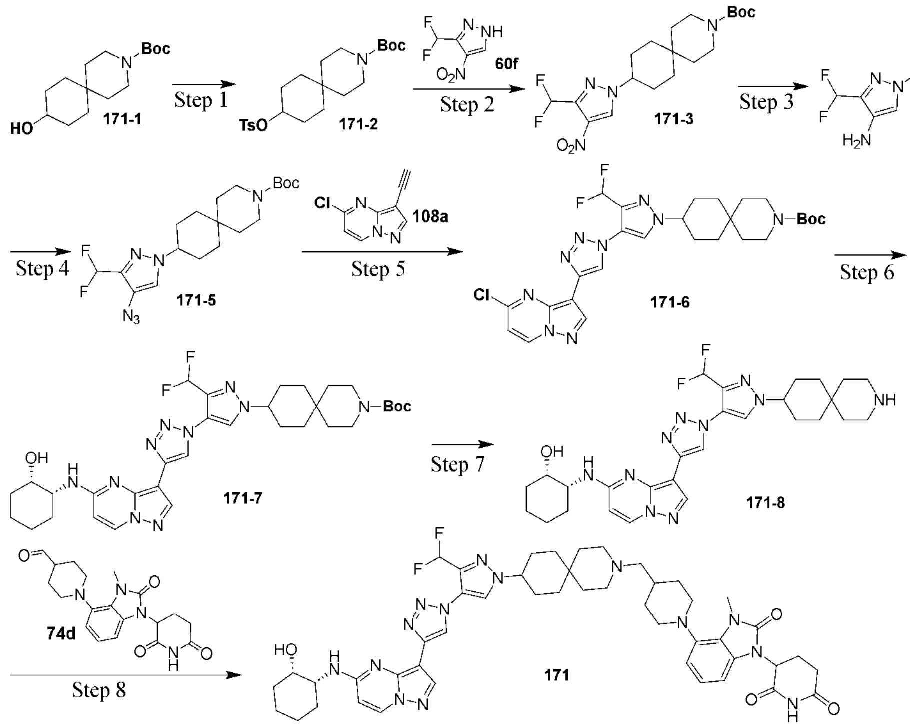


1) Step 1: Compound 171-1 (3.41 g, 12.26 mmol) was added to dichloromethane (100 mL), triethylamine (5.25 mL, 37.9 mmol) and 4-dimethylaminopyridine (310 mg, 2.52 mmol) were added. The mixture was cooled to 0°C, p-toluenesulfonyl chloride (3.13 g, 16.4 mmol) was added. After the addition was completed, the obtained mixture was warmed to 25°C and reacted under stirring for 2 h. The reaction solution was poured into water (100 mL) and dichloromethane was used for extraction (100 mL × 3). The organic phases were combined, dried over anhydrous sodium sulfate and filtered, and the filtrate was concentrated under reduced pressure to obtain compound 171-2, which was directly used in the next step without purification.

2) Step 2: Compound 171-2 (4.29 g, 10.1 mmol) and compound 60f (1.50 g, 9.20 mmol) were added to *N*,*N*-dimethylformamide (30 mL) and the obtained mixture was stirred until dissolved. Cesium carbonate (8.99 g, 27.6 mmol) was added to the reaction solution, and the reaction solution was reacted under stirring at 80°C for 18 h. The reaction solution was filtered and the filtrate was concentrated. The crude product was purified by column chromatography (petroleum ether/ethyl acetate = 6/1) to obtain compound 171-3, and the structure was verified by two-dimensional nuclear magnetic resonance. $^1$H NMR (400 MHz, $CDCl_3$) δ 8.23 (s, 1H), 7.15 (t, *J* =53.2 Hz, 1H), 4.24 - 4.08 (m, 1H), 3.47 - 3.29 (m, 4H), 2.12 - 2.02 (m, 2H), 2.00 - 1.85 (m, 4H), 1.61 - 1.57 (m, 2H), 1.46 (s, 9H), 1.39 - 1.26 (m, 4H).

3) Step 3: Compound 171-3 (1.51 g, 3.62 mmol) was added to methanol (40 mL) and the obtained mixture was stirred until dissolved. Wet palladium carbon (150 mg, 10%) was added in a nitrogen atmosphere. The reaction system was subjected to hydrogen replacement 3 times, and the reaction solution was reacted under stirring in a hydrogen atmosphere at 25°C for 18 h. The reaction solution was filtered, and the filtrate was concentrated under reduced pressure to obtain compound 171-4. MS m/z(ESI): 385.2[M+1]$^+$.

4) Step 4: Tert-butyl nitrite (0.20 mL, 1.56 mmol) was added to compound 171-4 (500 mg, 1.30 mmol), azidotrimethylsilane (0.26 mL, 1.95 mmol) and acetonitrile (3 mL) at 0°C, and the reaction solution was stirred at 25°C for 18 h. The reaction solution was added to water (20 mL) and ethyl acetate (20 mL × 2) was used for extraction. The organic phase was washed with saturated brine (20 mL), dried over anhydrous sodium sulfate and filtered, and the solvent was removed under reduced pressure to obtain compound 171-5. MS m/z(ESI):433.3[M+23]$^+$.

5) Step 5: Sodium ascorbate (23.4 mg, 0.12 mmol) was added to compound 171-5 (485 mg, 1.18 mmol), compound 108a (210 mg, 1.18 mmol), copper sulfate pentahydrate (29.5 mg, 0.118 mmol), water (2 mL) and ethanol (4 mL) at room temperature, and the reaction solution was stirred at 25°C for 18 h. The reaction solution was added to saturated ammonium chloride (20 mL) and ethyl acetate (20 mL × 2) was used for extraction. The organic phases were combined, washed with brine (20 mL), dried over anhydrous sodium sulfate, filtered and subjected to reduced pressure to obtain compound 171-6. MS m/z(ESI):610.3[M+23]$^+$.

6) Step 6: *N*,*N*-diisopropylethylamine (66.0 mg, 0.51 mmol) was added to compound 171-6 (100 mg, 0.17 mmol), (1S,2R)-2-aminocyclohexanol hydrochloride (51.6 mg, 0.34 mmol) and acetonitrile (5 mL) at room temperature, and

the reaction solution was stirred at 50°C for 1 h. The reaction solution was added to water (20 mL) and ethyl acetate (30 mL × 2) was used for extraction. The organic phases were combined, washed with saturated brine (20 mL), dried over anhydrous sodium sulfate and filtered, and the solvent was removed under reduced pressure. The residue was purified by silica gel column chromatography with an eluent system (petroleum ether/ethyl acetate = 100/1 to 10/90) to obtain compound 171-7. MS m/z(ESI):667.5[M+1]$^+$.

7) Step 7: Trifluoroacetic acid (0.5 mL) was added to a solution of compound 171-7 (100 mg, 0.15 mmol) in dichloromethane (2 mL) at room temperature, and the obtained mixture was stirred for 1 h. The reaction solution was concentrated under reduced pressure to obtain compound 171-8. MS m/z(ESI):567.4[M+1]$^+$.

8) Step 8: Compound 171-8 (75.0 mg, 0.133 mmol) and triethylamine (13.4 mg, 0.133 mmol) were added to *N*,*N*-dimethylformamide (3 mL) at room temperature and the obtained mixture was stirred for 5 min. Acetic acid (13.4 mg, 0.223 mmol) was added and the obtained mixture was stirred for 5 min. Compound 74d (34.3 mg, 0.093 mmol) was added to the above solution and the obtained mixture was stirred for 20 min. Finally, sodium borohydride acetate (55.8 mg, 0.263 mmol) was added and the reaction solution was stirred at 0°C for 30 min. The reaction solution was added to water (10 mL) and ethyl acetate (10 mL × 3) was used for extraction. The organic phase was washed with saturated brine (10 mL), dried over anhydrous sodium sulfate and filtered, and the solvent was removed under reduced pressure. The residue was purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 15%-35%, flow rate: 25 mL/min) to obtain compound 171. MS m/z(ESI):921.8[M+1]$^+$. $^1$H NMR (400 MHz, DMSO-$d_6$): δ 11.09 (s, 1H), 8.75 (s, 1H), 8.53 - 8.46 (m, 1H), 8.45 (s, 1H), 8.27 (s, 1H), 7.53 - 7.42 (m, 1H), 7.24 (t, *J* = 53.2 Hz, 1H), 7.01 - 6.94 (m, 1H), 6.93 - 6.84 (m, 2H), 6.50 (d, *J* = 7.6 Hz, 1H), 5.39 - 5.31 (m, 1H), 4.74 - 4.64 (m, 1H), 4.36 - 4.25 (m, 1H), 4.15 - 4.07 (m, 1H), 4.00 - 3.93 (m, 1H), 3.63 (s, 3H), 3.16 - 3.08 (m, 2H), 2.92 - 2.84 (m, 1H), 2.74 - 2.58 (m, 4H), 2.39 - 2.34 (m, 3H), 2.28 - 2.19 (m, 2H), 2.03 - 1.90 (m, 5H), 1.84 - 1.55 (m, 13H), 1.44 - 1.21 (m, 9H).

Example 172: 7-(1-(3-(difluoromethyl)-1-(3-((1-(2,6-dioxopiperidin-3-yl)-3-methyl-2-oxo-2,3-dihydro-1H-benzo[d]imidazol-4-yl)piperidin-4-yl)methyl)-3-azaspiro[5.5]undecan-9-yl)-1H-pyrazol-4-yl)-1H-1,2,3-triazole)pyrrolo[1,2-b]pyridazine-3-carboxamide (172)

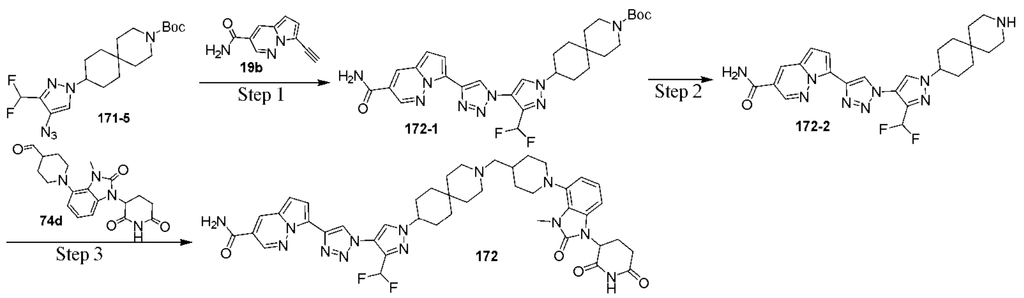


1) Step 1: Compound 19b (50 mg, 270 μmol) and compound 171-5 (111 mg, 270 μmol) were dissolved in a mixed solution of water (3 mL) and ethanol (3 mL), and copper sulfate pentahydrate (6.74 mg, 27.0 μmol) and sodium ascorbate (5.35 mg, 27.0 μmol) were added, and the obtained mixture was reacted under stirring at room temperature for 12 h. Then the ethanol was removed by concentration under reduced pressure. Water (20 mL) was added to the reaction solution, and a large amount of solid precipitated and was filtered. The filter cake was washed with petroleum ether/ethyl acetate (1/1, 5 mL) to obtain compound 172-1.

2) Step 2: Compound 172-1 (265 mg, 445 μmol) was dissolved in dichloromethane (10 mL) solution, and hydrogen chloride in dioxane (10 mL, 4 M) was added, and the obtained mixture was reacted under stirring at room temperature for 1 h. The reaction system was directly concentrated to obtain a crude product. The crude product was directly used as the raw material for the next step without purification. Compound 172-2 was obtained. MS m/z(ESI): 494.4[M-1]$^-$.

3) Step 3: Compound 172-2 (80.3 mg, 162 μmol) was dissolved in *N,N*-dimethylformamide (1 mL) and tetrahydrofuran (4 mL) at room temperature, triethylamine (16.4 mg, 162 μmol) was added, and the obtained mixture was stirred for 15 min. Sodium borohydride acetate (171 mg, 810 μmol), compound 74d (60.0 mg, 162 μmol) and acetic acid (19.5 mg, 324 μmol) were then added to the mixture, and the mixture was stirred at 25°C for 30 min. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio:

acetonitrile 16%-95%, flow rate: 25 mL/min) to obtain compound 172. MS m/z(ESI): 850.8[M+1]$^+$. $^1$H NMR (400 MHz, DMSO-$d_6$) δ 11.09 (s, 1H), 9.01 (s, 1H), 8.81 (s, 1H), 8.79 (d, *J* = 2.3 Hz, 1H), 8.66 (d, *J* = 2.3 Hz, 1H), 8.30 (s, 1H), 7.65 (d, *J* = 4.6 Hz, 1H), 7.56 (s, 1H), 7.24 (t, *J* = 53.3 Hz, 1H), 7.05 (d, *J* = 4.6 Hz, 1H), 7.00 - 6.93 (m, 1H), 6.92 - 6.83 (m, 2H), 5.48 - 5.17 (m, 1H), 4.40 - 4.19 (m, 1H), 3.62 (s, 3H), 3.16 - 3.06 (m, 3H), 2.92 - 2.78 (m, 1H), 2.75 - 2.58 (m, 4H), 2.41 - 2.32 (m, 3H), 2.25 - 2.17 (m, 2H), 2.01 - 1.90 (m, 5H), 1.86 - 1.74 (m, 4H), 1.69 - 1.57 (m, 3H), 1.42 - 1.23 (m, 6H).

Example 173: 3-(4-(4-(-4-(((1S,4r)-4-(3-(difluoromethyl)-4-(4-(5-((S)-3-(hydroxymethyl)morpholinyl)pyrazolo[1,5-a]pyrimidin-3-yl)-1H-1,2,3-triazol-1-yl)-1H-pyrazol-1-yl(cyclohexyl)methyl)piperazin-1-yl)piperidin-1-yl)-3-methyl-2-oxo-2,3-dihydro-1H-benzo[d]imidazol-1-yl)pyridine-2,6-dione (173)

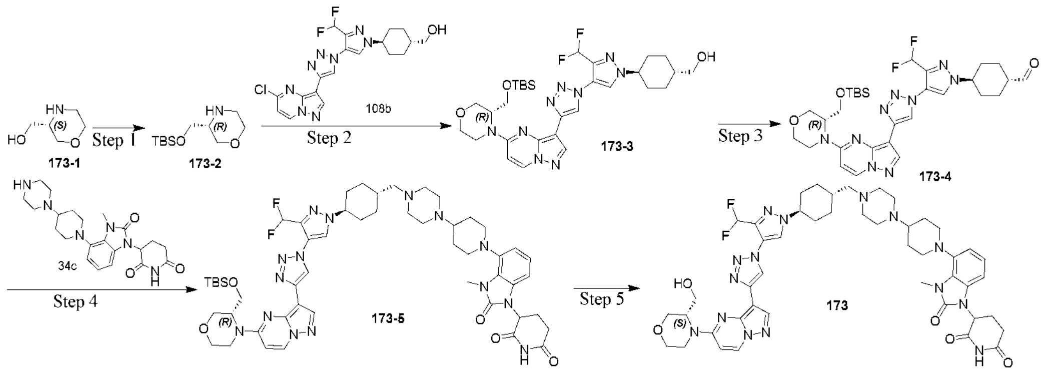


1) Step 1: Compound 173-1 (1.00 g, 6.51 mmol, Bide Pharmatech Ltd.), triethylamine (2.71 mL, 19.5 mmol) and dichloromethane (10 mL) were added to a single-neck bottle at room temperature in sequence, and tert-butyldimethylsilyl chloride (1.18 g, 7.81 mmol) was added under stirring. The reaction system was subjected to nitrogen replacement three times and reacted at 25°C for 2 h. An ammonium chloride aqueous solution (10 mL) was added, the obtained mixture was stirred and extracted with dichloromethane (20 mL × 3). The organic phases were combined, dried over sodium sulfate and filtered, and the filtrate was concentrated to obtain compound 173-2, which was directly used for the next step without purification.

2) Step 2: Compound 173-2 (696 mg, 3.01 mmol), compound 108b (900 mg, 2.01 mmol) and acetonitrile (10 mL) were added to a single-neck bottle at room temperature in sequence, and *N*,*N*-diisopropylethylamine (1.00 mL, 6.02 mmol) was added under stirring. The reaction system was subjected to nitrogen replacement three times and stirred at 80°C for 2 h. Then the reaction solution was concentrated and the residue was purified by silica gel column chromatography (methanol/dichloromethane = 0%-10%) to give compound 173-3. MS m/z(ESI):644.4[M+1]$^+$.

3) Step 3: Compound 173-3 (300 mg, 0.470 mmol) and dichloromethane (3 mL) were added to a single-neck bottle at room temperature in sequence, and Dess-Martin oxidant (296 mg, 0.700 mmol) was added under stirring. The obtained mixture was reacted at 25°C for 2 h. Water (20 mL) was added to the reaction solution, and the obtained mixture was stirred and then extracted with dichloromethane (30 mL × 3). The organic phases were combined, washed with brine (30 mL), dried over anhydrous sodium sulfate and filtered, and the filtrate was concentrated to obtain compound 173-4. MS m/z(ESI):642.4[M+1]$^+$.

4) Step 4: Compound 173-4 (160 mg, 250 μmol), *N*,*N*-dimethylformamide (2 mL) and tetrahydrofuran (3 mL) were added to a single-neck bottle at room temperature in sequence, triethylamine (86.4 μL, 620 μmol) was added under stirring, and the obtained mixture was stirred at 0°C for 10 min. Then compound 34c (106 mg, 250 μmol), sodium borohydride acetate (316 mg, 1.50 mmol) and acetic acid (85.6 μL, 1.50 mmol) were added in sequence, and the obtained mixture was reacted at 25°C for 2 h. Most of the solvent was removed from the reaction solution under vacuum, and the residue was purified by silica gel column chromatography (methanol/dichloromethane = 0%-12%) to obtain compound 173-5. MS m/z(ESI):526.8[M/2+1]$^+$.

5) Step 5: Compound 173-5 (130 mg, 120 μmol), dichloromethane (5 mL) and hydrogen chloride/methanol (1 mL, 4 M) were added to a single-neck bottle at room temperature in sequence, and the obtained mixture was stirred at 25°C for 1 h. Most of the solvent was removed from the reaction solution under vacuum, and the residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 51%-95%, flow rate: 25 mL/min) to obtain compound 173. MS m/z(ESI):938.4[M+1]$^+$. $^1$H NMR (400 MHz, DMSO-$d_6$) δ 11.12 (s, 1H), 8.76 (d, *J* = 8 Hz, 1H), 8.65 (s, 1H), 8.54 (s, 1H), 8.39 (s, 1H), 7.15 (t, *J* = 53.2 Hz, 1H), 6.99 - 6.96 (m, 1H), 6.95 - 6.85 (m, 2H), 6.80 (d, *J* = 8.0 Hz, 1H), 5.43 - 5.31 (m, 1H), 4.54 - 4.13 (m, 4H), 4.06 - 4.04 (m, 1H),

3.98 - 3.96 (m, 1H), 3.78 - 3.73 (m, 1H), 3.65 (s, 3H), 3.60 - 3.54 (m, 4H), 3.24 -3.16 (m, 3H), 2.96 - 2.86 (m, 1H), 2.76 - 2.67 (m, 3H), 2.65 -2.59 (m, 4H), 2.41 - 2.31 (m, 5H), 2.17 - 2.16 (m, 4H), 2.07 - 1.77 (m, 7H), 1.69 - 1.60 (m, 3H), 1.12 - 1.09 (m, 2H).

Example 174: 3-(4-(9-(((1S,4r)-4-(3-(difluoromethyl)-4-(4-(5-((S)-3-(hydroxymethyl)morpholinyl)pyrazolo[1,5-a]pyrimidin-3-yl)-1H-1,2,3-triazol-1-yl)-1H-pyrazol-1-yl(cyclohexyl)-3,9-diazaspiro[5.5]undecan-3-yl)-3-methyl-2-oxo-2,3-dihydro-1H-benzo[d]imidazol-1-yl)piperidine-2,6-dione (174)

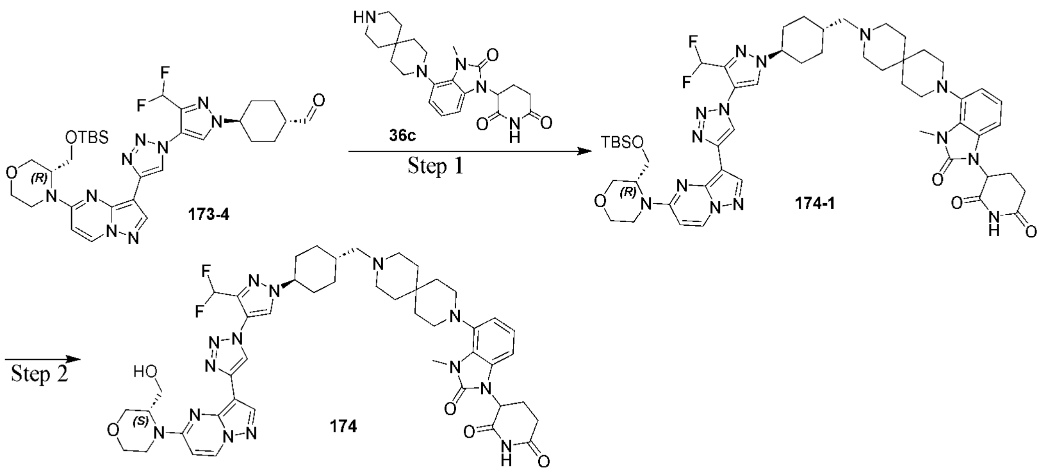


1) Step 1: Compound 173-4 (160 mg, 250 μmol), DMF (2 mL) and tetrahydrofuran (3 mL) were added to a single-neck bottle at room temperature in sequence, triethylamine (86.4 μL, 620 μmol) was added under stirring, and the obtained mixture was cooled to 0°C and stirred for 10 min. Then compound 36c (103 mg, 250 μmol), sodium borohydride acetate (316 mg, 1.50 mmol) and acetic acid (85.6 μL, 1.50 mmol) were added in sequence, and then the obtained mixture was stirred at 25°C for 2 h. Most of the solvent was removed from the reaction solution under vacuum, and the residue was purified by silica gel column chromatography (methanol/dichloromethane = 0%-12%) to obtain compound 174-1. MS m/z(ESI):519.6[1/2M+1]$^+$.

2) Step 2: Compound 174-1 (170 mg, 120 μmol), dichloromethane (5 mL) and hydrogen chloride/methanol (1 mL, 4 M) were added to a single-neck bottle at room temperature in sequence, and the obtained mixture was stirred at 25°C for 1 h. Most of the solvent was removed from the reaction solution under vacuum, and the residue was purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 51%-95%, flow rate: 25 mL/min) to obtain compound 174. MS m/z(ESI):923.4[M+1]$^+$. $^1$H NMR (400 MHz, DMSO-$d_6$) δ 11.09 (s, 1H), 8.73 (d, *J* = 8.0 Hz, 1H), 8.62 (s, 1H), 8.52 (s, 1H), 8.36 (s, 1H), 7.19 (t, *J* = 53.2 Hz, 1H), 6.97 - 6.94 (m, 2H), 6.87 - 6.85 (m, 1H), 6.77 (d, *J* = 8.0 Hz, 1H), 5.37 - 5.31 (m, 1H), 4.37 - 4.21 (m, 3H), 4.02 (d, *J* = 12.0 Hz, 1H), 3.94 (d, *J* = 8.0 Hz, 1H), 3.75 - 3.70 (m, 1H), 3.62 (s, 3H), 3.59 - 3.56 (m, 3H), 3.56 - 3.51 (m, 2H), 3.21 - 3.14 (m, 2H), 2.89 - 2.86 (m, 4H), 2.67 - 2.64 (m, 2H), 2.43 - 2.34 (m, 4H), 2.19 (d, *J* = 6.8 Hz, 2H), 2.15 - 2.12 (m, 2H), 2.02 - 1.90 (m, 3H), 1.82 - 1.80 (m, 2H), 1.72 - 1.60 (m, 4H), 1.52 - 1.44 (m, 4H), 1.10 - 1.06 (m, 2H).

Example 175: 3-(4-(4-(-4-(((1r,4r)-4-(3-(difluoromethyl)-4-(4-(5-(((R)-2-(hydroxymethyl)morpholinyl)pyrazolo[1,5-a]pyrimidin-3-yl)-1H-1,2,3-triazol-1-yl)-1H-pyrazol-1-yl(cyclohexyl)methyl)piperazin-1-yl)piperidin-1-yl)-3-methyl-2-oxo-2,3-dihydro-1H-benzo[d]imidazol-1-yl)pyridine-2,6-dione (175)

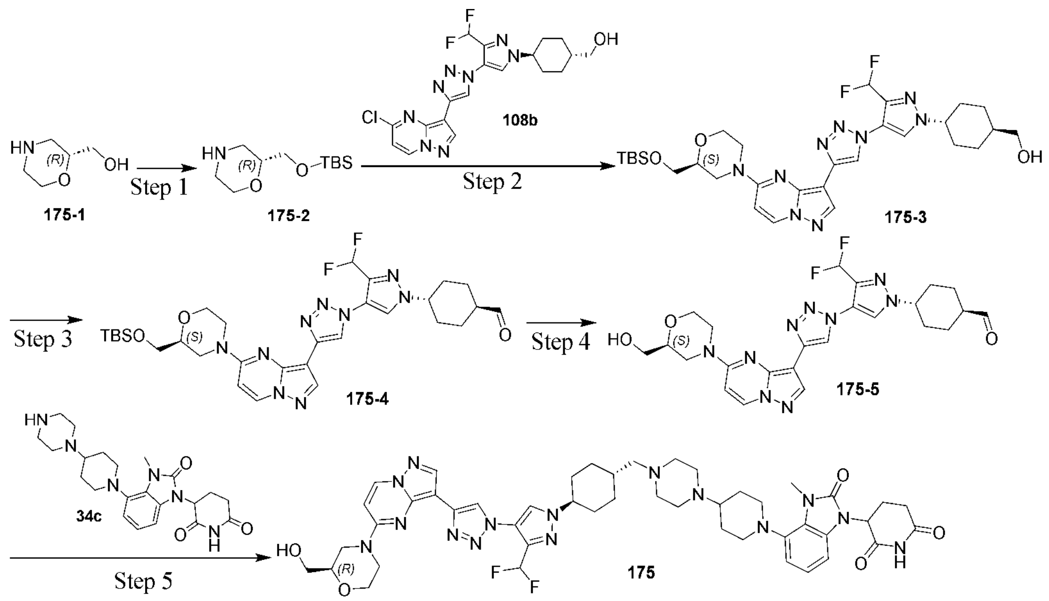


175-1 175-2 108b 175-3 175-4 175-5 34c 175

1) Step 1: Triethylamine (14.2 mL, 102 mmol) was added to a solution of compound 175-1 (4.00 g, 34.2 mmol) and tert-butyldimethylsilyl chloride (10.3 g, 68.3 mmol) in dichloromethane (40 mL). The obtained mixture was stirred at 25°C for 12 h, and then water (30 mL) was added. The organic phase was separated, and the aqueous phase was extracted with dichloromethane (30 mL × 2). The combined organic phase was dried over anhydrous sodium sulfate and filtered, and the filtrate was concentrated under reduced pressure to obtain compound 175-2. MS m/z(ESI):232.6 $[M+1]^+$.

2) Step 2: *N*,*N*-diisopropylethylamine (87.0 mg, 670 μmol) was added to a solution of compound 108b (100 mg, 230 μmol) and compound 175-2 (32.0 mg, 270 μmol) in acetonitrile (5 mL) under nitrogen protection. The mixture was stirred at 60°C for 3 h, cooled to room temperature, diluted with water (10 mL), and extracted with ethyl acetate (20 mL × 2). The organic phases were combined, dried over anhydrous sodium sulfate and filtered, and the filtrate was concentrated under reduced pressure. The residue was purified by silica gel column chromatography (dichloromethane/methanol = 100/1 to 30/1) to obtain compound 175-3. MS m/z(ESI):642.5$[M-1]^-$.

3) Step 3: Compound 175-3 (100 mg, 155 μmol) was added to a solution of Dess-Martin oxidant (396 mg, 233 μmol) in dichloromethane (5 mL) under nitrogen protection, the obtained mixture was subjected to nitrogen replacement three times, and then reacted at 25°C for 1 h. Water (10 mL) was added to the reaction solution, and ethyl acetate (10 mL × 3) was used for extraction. The organic phases were combined, washed with saturated brine (10 mL), dried over anhydrous sodium sulfate, and concentrated under reduced pressure. The residue was purified by column chromatography (dichloromethane/methanol = 100/1 to 30/1) to obtain compound 175-4. MS m/z(ESI):642.5$[M+1]^+$.

4) Step 4: Tetrabutylammonium fluoride (0.19 mL, 190 μmol, 1 M solution in tetrahydrofuran) was added to a tetrahydrofuran (5 mL) solution containing compound 175-4 (80 mg, 120 μmol) under nitrogen protection, and the mixture was subjected to nitrogen replacement three times, and then reacted at 25°C for 1 h. Sodium bicarbonate aqueous solution (10 mL) was added to the reaction solution, and ethyl acetate was used for extraction (20 mL × 2). The organic phases were combined, dried over anhydrous sodium sulfate and filtered, and the filtrate was concentrated under reduced pressure. The residue was purified by silica gel column chromatography (dichloromethane/methanol = 100/1 to 80/1) to obtain compound 175-5. MS m/z(ESI):528.4$[M+1]^+$.

5) Step 5: Triethylamine (0.30 mL, 2.16 mmol) was added to a solution of compound 34c (50.0 mg, 120 μmol), tetrahydrofuran (2 mL) and *N,N*-dimethylformamide (1 mL), and the obtained mixture was stirred for 30 min. Then compound 175-5 (75.0 mg, 140 μmol) and acetic acid (0.5 mL) were added to the above solution and the obtained mixture was stirred for 30 min. Finally, sodium triacetoxyborohydride (80 mg, 350 μmol) was added to the above reaction solution, and then the obtained mixture was stirred at 25°C for 2 h. The reaction solution was concentrated under reduced pressure and then purified by high performance liquid chromatography (A: 0.1% FA/H2O B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 175 (10 mg). MS m/z(ESI):938.8$[M+1]^+$. $^1$H NMR (400 MHz, DMSO-$d_6$): δ 11.09 (s, 1H), 8.76 (d, *J* = 7.9 Hz, 1H), 8.64 (s, 1H), 8.52 (s, 1H), 8.38 (s, 1H), 8.29 (s, 1H), 7.21 (t, *J* = 53.3 Hz, 1H), 7.00 - 6.95 (m, 1H), 6.92 - 6.85 (m, 2H), 6.81 (d, *J* = 7.9 Hz, 1H), 5.38 - 5.30 (m, 1H), 5.00 - 4.61 (m, 1H), 4.43 - 4.34 (m, 2H), 4.32 - 4.24 (m, 1H), 4.01 - 3.93 (m, 1H), 3.63 (s, 3H), 3.55 - 3.49 (m, 5H), 3.18 - 3.11 (m, 4H), 2.94 - 2.82 (m, 3H), 2.74 - 2.63 (m, 4H), 2.40 - 2.29 (m, 4H), 2.18 - 2.12 (m, 4H), 2.00 - 1.78 (m, 8H), 1.65 - 1.55 (m, 3H), 1.27 - 1.23 (m, 1H), 1.14 - 1.01 (m, 1H).

Example 176: 3-(4-(9-(((1r,4r)-4-(3-(difluoromethyl)-4-(4-(5-((R)-2-(hydroxymethyl)morpholinyl)pyrazolo[1,5-a]pyrimidin-3-yl)-1H-1,2,3-triazol-1-yl)-1H-pyrazol-1-yl(cyclohexyl)methyl)-3,9-diazaspiro[5.5]undecan-3-yl)-3-methyl-2-oxo-2,3-dihydro-1H-benzo[d]imidazol-1-yl)piperidine-2,6-dione (176)

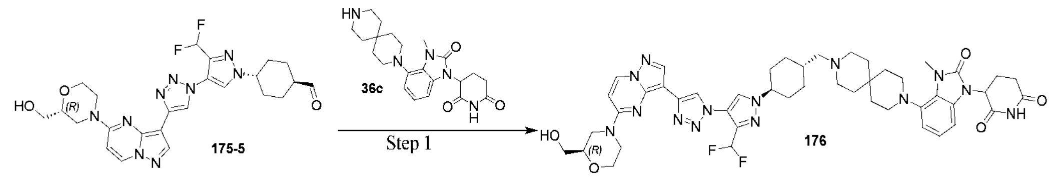


Triethylamine (0.20 mL, 1.44 mmol) was added to a solution of compound 36c (100 mg, 240 μmol) in tetrahydrofuran (2 mL) and *N*,*N*-dimethylformamide (1 mL), and the obtained mixture was stirred for 30 min. Then compound 175-5 (154 mg, 290 μmol) and acetic acid (0.5 mL) were added to the above solution, and the obtained mixture was stirred for 30 min. Finally, sodium triacetoxyborohydride (154 mg, 730 μmol) was added, and the obtained mixture was stirred at 25°C for 2 h. The reaction solution was concentrated under reduced pressure and then purified by high performance liquid chromatography (A: 0.1% FA/H2O B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 176 (10 mg). MS m/z(ESI):426.2[M/2+1]$^+$. $^1$H NMR (400 MHz, DMSO-$d_6$): δ 11.09 (m, 1H), 8.76 (d, *J* = 7.9 Hz, 1H), 8.64 (s, 1H), 8.52 (s, 1H), 8.38 (s, 1H), 8.30 - 8.26 (m, 1H), 7.21 (t, *J* = 53.1 Hz, 1H), 6.99 - 6.95 (m, 2H), 6.89 - 6.85 (m, 1H), 6.81 (d, *J* = 7.8 Hz, 1H), 5.40 - 5.32 (m, 1H), 4.95 - 4.67 (m, 1H), 4.44 - 4.35 (m, 2H), 4.31 - 4.22 (m, 1H), 4.00 - 3.93 (m, 1H), 3.64 (s, 3H), 3.53 - 3.48 (m, 4H), 3.08 - 3.05 (m, 1H), 2.90 - 2.86 (m, 4H), 2.74 - 2.57 (m, 3H), 2.38 - 2.33 (m, 4H), 2.20 - 2.11 (m, 4H), 2.04 - 1.88 (m, 4H), 1.88 - 1.77 (m, 3H), 1.70 - 1.61 (m, 4H), 1.55 - 1.41 (m, 4H), 1.09 - 1.04 (m, 1H).

Example 243: 3-(4-(4-(((1r,4r)-4-(3-(difluoromethyl)-4-(4-(5-(((R)-3-hydroxypiperidin-1-yl)pyrazolo[1,5-a]pyrimidin-3-yl)-1H-1,2,3-triazol-1-yl)-1H-pyrazol-1-ylcyclohexyl)methyl)piperazin-1-yl)-3-methyl-2-oxo-2,3-dihydro-1H-benzo[d]imidazol-1-yl)piperidine-2,6-dione (243)

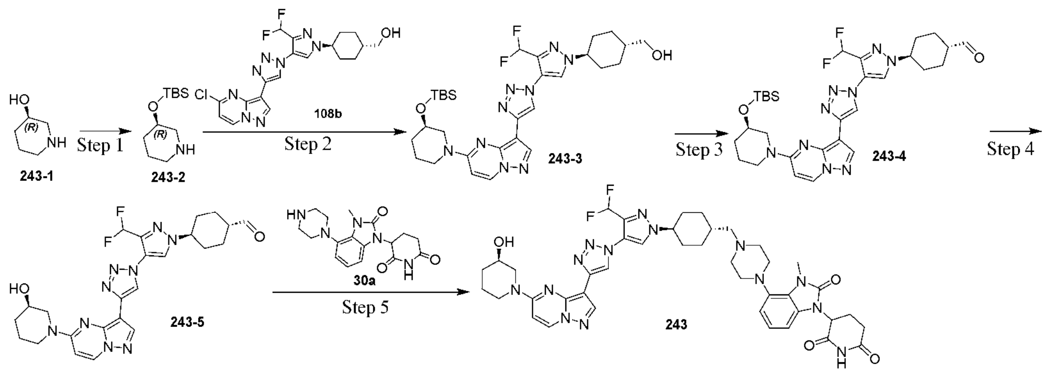


1) Step 1: Tert-butyldimethylsilyl chloride (3.60 g, 24.0 mmol) was added to a solution of compound 243-1 (3.00 g, 21.8 mmol) and N,N-diisopropylethylamine (8.50 g, 65.4 mmol) in tetrahydrofuran (10 mL) under nitrogen protection, and the obtained mixture was subjected to nitrogen replacement three times and stirred at 80°C for 12 h. The reaction solution was cooled to room temperature, diluted with water (10 mL), and extracted with ethyl acetate (20 mL × 2). The organic phases were combined, dried over anhydrous sodium sulfate and filtered, and the filtrate was concentrated under reduced pressure. The residue was purified by silica gel column chromatography (dichloromethane/methanol = 100/1 to 30/1) to obtain compound 243-2. $^1$H NMR (400 MHz, DMSO-$d_6$): δ 387 - 3.65 (m, 1H), 3.60 - 3.51 (m, 1H), 2.90 - 2.80 (m, 1H), 2.75 - 2.65 (m, 1H), 2.40 - 2.30 (m, 1H), 2.28 - 2.20 (m, 1H), 1.80 - 1.75 (m, 1H), 1.64 - 1.58 (m, 1H), 1.38 - 1.20 (m, 2H), 0.86 (s, 8H), 0.00 (s, 6H).

2) Step 2: *N*,*N*-diisopropylethylamine (260 mg, 2.01 mmol) was added to a solution of compound 243-2 (288 mg, 1.34 mmol) and compound 108b (300 mg, 670 μmol ) in acetonitrile (5 mL) under nitrogen protection, and then the obtained mixture was stirred at 60°C for 3 h, cooled to room temperature, diluted with water (10 mL), and extracted with ethyl

acetate (20 mL × 2). The organic phases were combined, dried over anhydrous sodium sulfate and filtered, and the filtrate was concentrated under reduced pressure. The residue was purified by silica gel column chromatography (dichloromethane/methanol = 100/1 to 30/1) to obtain compound 243-3.

3) Step 3: A solution of compound 243-3 (500 mg, 800 μmol) and *N,N*-dimethylformamide (5 mL) was cooled to 0°C, and Dess-Martin oxidant (510 mg, 1.19 mmol) was added, and then the obtained mixture was stirred at 25°C for 2 h. The reaction mixture was diluted with water (20 mL) and extracted with ethyl acetate (30 mL × 2). The combined organic phase was dried over anhydrous sodium sulfate and filtered, and the filtrate was concentrated under reduced pressure. The residue was purified by silica gel column chromatography with an eluent system (dichloromethane/-methanol = 100/1 to 20/1) to obtain compound 243-4. MS m/z(ESI):626.4[M+1]$^+$.

4) Step 4: Tetrabutylammonium fluoride (2.40 mL, 2.40 mmol, 1 M solution in tetrahydrofuran ) was added to a solution of compound 243-4 (300 mg, 480 μmol) in tetrahydrofuran (5 mL) under nitrogen protection, and the obtained mixture was reacted at 25°C for 3 h, then sodium bicarbonate aqueous solution (10 mL) was added, and ethyl acetate was used for extraction (20 mL × 2). The organic phases were combined, dried over anhydrous sodium sulfate and filtered, and the filtrate was concentrated under reduced pressure. The residue was purified by silica gel column chromatography (dichloromethane/methanol = 100/1 to 30/1) to obtain compound 243-5. MS m/z(ESI):512.3[M+1]$^+$.

5) Step 5: Triethylamine (0.20 mL, 1.44 mmol) was added to a solution of compound 30a (50 mg, 150 μmol) in tetrahydrofuran (2 mL) and *N,N*-dimethylformamide (1 mL), and the obtained mixture was stirred for 30 min. Then compound 243-5 (82.0 mg, 160 μmol) and acetic acid (0.3 mL) were added, and the obtained mixture was stirred for 30 min. Then sodium borohydride acetate (93.0 mg, 440 μmol) was added, and the obtained mixture was stirred at 25°C for 2 h, then concentrated under reduced pressure. The residue was purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 243. MS m/z(ESI):839.6[M+1]$^+$. $^1$H NMR (400 MHz, DMSO-$d_6$): δ 11.09 (s, 1H), 8.67 (d, *J* = 7.7 Hz, 2H), 8.50 (s, 1H), 8.36 (s, 1H), 8.34 (s, 1H), 7.27 (t, *J* = 53.0 Hz, 1H), 7.01 - 6.96 (m, 1H), 6.94 - 6.87 (m, 2H), 6.78 (d, *J* = 8.0 Hz, 1H), 5.38 - 5.30 (m, 2H), 4.92 - 4.84 (m, 1H), 4.37 - 4.22 (m, 1H), 4.13 - 3.98 (m, 2H), 3.63 (s, 3H), 3.61 - 3.56 (m, 2H), 2.91 - 2.88 (m, 2H), 2.72 - 2.61 (m, 3H), 2.35 - 2.32 (m, 1H), 2.27 - 2.20 (m, 2H), 2.15 - 2.10 (m, 3H), 2.04 - 1.98 (m, 3H), 1.91 - 1.87 (m, 2H), 1.82 - 1.77 (m, 4H), 1.48 - 1.44 (m, 3H), 1.34 - 1.31 (m, 1H), 1.11 - 1.02 (m, 2H).

Example 245: 3-(4-(4-(1-(((1r,4r)-4-(3-(difluoromethyl)-4-(4-(5-(((R)-3-hydroxypiperidin-1-yl)pyrazolo[1,5-a]pyrimidin-3-yl)-1H-1,2,3-triazol-1-yl)-1H-pyrazol-1-yl(cyclohexyl)methyl)piperidin-4-yl)piperazin-1-yl)-3-methyl-2-oxo-2,3-dihydro-1H-benzo[d]imidazol-1-yl)piperidine-2,6-dione (245)

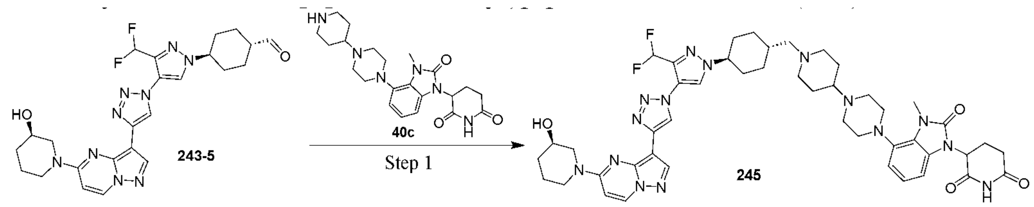


Triethylamine (0.20 mL, 1.44 mmol) was added to a solution of compound 40c (20 mg, 50.0 μmol) in tetrahydrofuran (2 mL) and *N*,*N*-dimethylformamide (1 mL), and the obtained mixture was stirred for 30 min. Then compound 243-5 (29.0 mg, 60.0 μmol) and acetic acid (0.3 mL) were added, and the obtained mixture was stirred for 30 min. Then sodium borohydride acetate (30.0 mg, 140 μmol) was added, and the obtained reaction mixture was stirred at 25°C for 2 h, then concentrated under reduced pressure. The residue was purified by high performance liquid chromatography (A: 0.1% FA/H2O B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 245. MS m/z(ESI):922.7[M+1]$^+$. $^1$H NMR (400 MHz, DMSO-$d_6$): δ 11.09 (m, 1H), 8.75 - 8.62 (m, 2H), 8.50 (s, 1H), 8.37 (s, 1H), 8.34 (s, 1H), 7.20 (t, *J* = 53.2 Hz, 1H), 7.01 - 6.95 (m, 1H), 6.94 - 6.90 (m, 1H), 6.89 - 6.86 (m, 1H), 6.78 (d, *J* = 7.9 Hz, 1H), 5.37 - 5.32 (m, 1H), 4.99 - 4.78 (m, 1H), 4.40 - 4.24 (m, 1H), 4.12 - 3.97 (m, 2H), 3.63 (s, 3H), 3.61 - 3.56 (m, 2H), 2.94 - 2.85 (m, 6H), 2.72 - 2.58 (m, 3H), 2.36 - 2.18 (m, 3H), 2.18 - 2.07 (m, 4H), 2.04 - 1.98 (m, 2H), 1.96 - 1.84 (m, 6H), 1.82 - 1.75 (m, 4H), 1.50 - 1.42 (m, 4H), 1.35 - 1.26 (m, 2H), 1.13 - 1.01 (m, 2H).

Example 247: 3-(4-(4-(((1r,4r)-4-(3-(difluoromethyl)-4-(4-(5-(((R)-3-hydroxypiperidin-1-yl)pyrazolo[1,5-a]pyrimidin-3-yl)-1H-1,2,3-triazol-1-yl)-1H-pyrazol-1-yl(cyclohexyl)methyl)piperazin-1-yl)piperidin-1-acyl)-3-methyl-2-oxo-2,3-dihydro-1H-benzo[d]imidazol-1-yl)pyridine-2,6-dione (247)

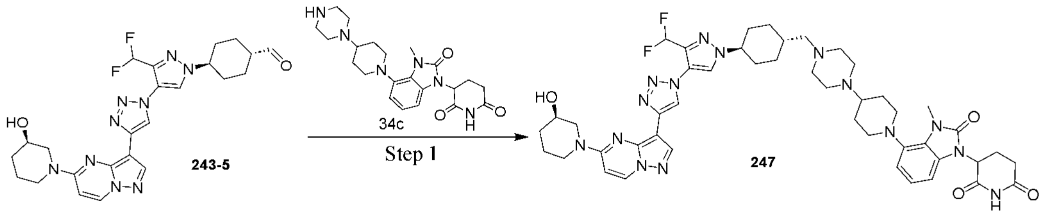


Triethylamine (0.20 mL, 1.44 mmol) was added to a solution of compound 34c (120 mg, 280 μmol) in tetrahydrofuran (2 mL) and *N,N*-dimethylformamide (1 mL), and the obtained mixture was stirred for 30 min. Then compound 243-5 (173 mg, 340 μmol) and acetic acid (0.5 mL) were added, and the obtained mixture was stirred for 30 min. Then sodium borohydride acetate (180 mg, 840 μmol) was added, and the obtained mixture was stirred at 25°C for 2 h, then concentrated under reduced pressure. The residue was purified by high performance liquid chromatography (A: 0.1% FA/H2O B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 247. MS m/z(ESI):922.4[M+1]$^+$. $^1$H NMR (400 MHz, DMSO-$d_6$): δ 11.09 (m, 1H), 8.69 - 8.64 (m, 2H), 8.50 (s, 1H), 8.34 (s, 1H), 8.20 (s, 1H), 7.20 (t, *J* = 53.2 Hz, 1H), 7.01 - 6.85 (m, 3H), 6.78 (d, *J* = 7.9 Hz, 1H), 5.39 - 5.28 (m, 1H), 4.92 - 4.83 (m, 1H), 4.34 - 4.23 (m, 1H), 4.12 - 3.99 (m, 2H), 3.63 (s, 3H), 3.61 - 3.53 (m, 2H), 3.20 - 3.12 (m, 4H), 2.91 - 2.85 (m, 2H), 2.75 - 2.63 (m, 4H), 2.40 - 2.30 (m, 4H), 2.17 - 2.10 (m, 4H), 1.97 - 1.75 (m, 8H), 1.67 - 1.58 (m, 4H), 1.56 - 1.38 (m, 4H), 1.13 - 1.02 (m, 2H).

Example 248: 3-(4-(2-(((1r,4r)-4-(3-(difluoromethyl)-4-(4-(5-(((R)-3-hydroxypiperidin-1-yl)pyrazolo[1,5-a]pyrimidin-3-yl)-1H-1,2,3-triazol-1-yl)-1H-pyrazol-1-ylcyclohexyl)methyl)-2,8-diazaspiro[4.5]dec-8-yl)-3-methyl-2-oxo-2,3-dihydro-1H-benzo[d]imidazol-1-yl)piperidine-2,6-dione (248)

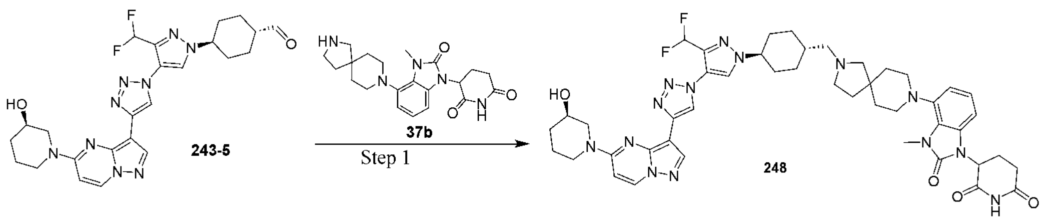


Triethylamine (0.20 mL, 1.44 mmol) was added to a solution of compound 37b (80 mg, 200 μmol) in tetrahydrofuran (2 mL) and N,N-dimethylformamide (1 mL), and the obtained mixture was stirred for 30 min. Then compound 243-5 (115 mg, 220 μmol) and acetic acid (0.5 mL) were added, and the obtained mixture was stirred for 30 min. Then sodium borohydride acetate (128 mg, 600 μmol) was added, and the obtained mixture was stirred at 25°C for 2 h and concentrated under reduced pressure. The residue was purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 248. MS m/z(ESI):893.8[M+1]$^+$. $^1$H NMR (400 MHz, DMSO-$d_6$): δ 11.09 (s, 1H), 8.68 - 8.64 (m, 2H), 8.50 (s, 1H), 8.37 (s, 1H), 8.34 (s, 1H), 7.20 (t, *J* = 53.2 Hz, 1H), 7.01 - 6.87 (m, 3H), 6.78 (d, *J* = 8.0 Hz, 1H), 5.37 - 5.31 (m, 2H), 4.92 - 4.84 (m, 1H), 4.35 - 4.23 (m, 1H), 4.03 - 3.94 (m, 1H), 3.63 (s, 3H), 3.62 - 3.53 (m, 2H), 2.92 - 2.85 (m, 4H), 2.72 - 2.58 (m, 3H), 2.35 - 2.31 (m, 1H), 2.26 - 2.19 (m, 2H), 2.15 - 2.09 (m, 3H), 2.03 - 1.97 (m, 4H), 1.91 - 1.87 (m, 3H), 1.86 - 1.81 (m, 2H), 1.81 - 1.77 (m, 3H), 1.50 - 1.44 (m, 4H), 1.36 - 1.29 (m, 2H), 1.13 - 0.99 (m, 2H).

Example 268: 3-(4-(4-((1R,4r)-4-(3-(difluoromethyl)-4-(4-(5-((1R,2S)-2-hydroxycyclohexyl)amino)pyrazolo[1,5-a]pyrimidin-3-yl)-1H-1,2,3-triazol-1-yl)-1H-pyrazol-1-yl)cyclohexyl)methyl)-piperazin-1-yl)-3-methyl-2-oxo-2,3-dihydro-1H-benzo[d]imidazol-1-yl)piperidine-2,6-dione (268)

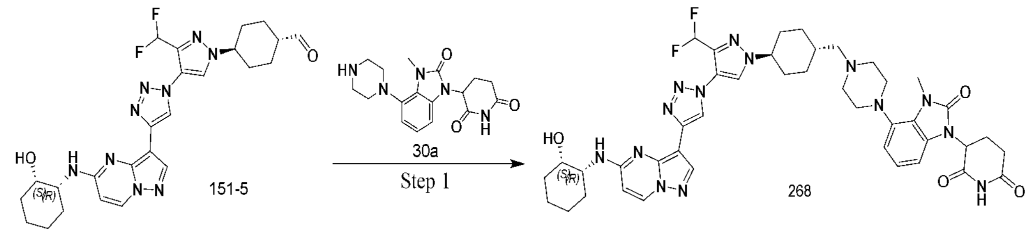


Triethylamine (0.020 mL, 0.15 mmol) was added to a solution of compound 30a (50.0 mg, 150 μmmol) in *N*,*N*-dimethylformamide (1 mL) and the obtained mixture was stirred for 1 min. Then acetic acid (0.02 mL) and sodium borohydride acetate (123 mg, 580 μmmol) were added to the above solution, and the obtained mixture was stirred for 1 min. Finally, compound 151-5 (76.5 mg, 150 μmmol) was added to the above reaction solution. The reaction was stirred at 25°C for 1 h. The reaction solution was concentrated under reduced pressure and then purified by high performance liquid chromatography (Gilson_306_1741, chromatographic column: Waters-Xbridge-C18-10μm-19×250 mm; mobile phase: water (containing 26.5 mmol/L formic acid) and acetonitrile, gradient ratio: acetonitrile 2%-95%, flow rate: 25 mL/min) to obtain compound 268. MS m/z(ESI):853.6[M+1]$^+$. $^1$H NMR (400 MHz, DMSO-$d_6$) δ 11.10 (s, 1H), 8.67 (s, 1H), 8.50 (d, *J* = 7.6 Hz, 1H), 8.43 (s, 1H), 8.27 (s, 1H), 7.53 - 7.47 (m, 1H), 7.23 (t, *J* = 53.1 Hz, 1H), 7.09 - 6.91 (m, 3H), 6.50 (d, *J* = 7.6 Hz, 1H), 5.37 - 5.30 (m, 1H), 4.79 - 4.63 (m, 1H), 4.36 - 4.24 (m, 1H), 4.16 - 4.07 (m, 1H), 3.95 (s, 1H), 3.64 (s, 3H), 3.22 - 3.12 (m, 6H), 3.08 - 2.81 (m, 5H), 2.76 - 2.62 (m, 1H), 2.33 - 2.15 (m, 5H), 2.02 - 1.99 (m, 4H), 1.86 - 1.65 (m, 4H), 1.63 - 1.52 (m, 4H), 1.14 - 1.04 (m, 2H).

Example 284: 3-(4-((S)-4-(((1r,4S)-4-(3-(difluoromethyl)-4-(4-(5-(4-hydroxy-4-methylpiperidin-1-yl)pyrazolo[1,5-a]pyrimidin-3-yl)-1H-1,2,3-triazol-1-yl)-1H-pyrazol-1-yl)cyclohexyl)-3-methylpiperazin-1-yl)-3-methyl-2-oxo-2,3-dihydro-1H-benzo[d]imidazole (284)

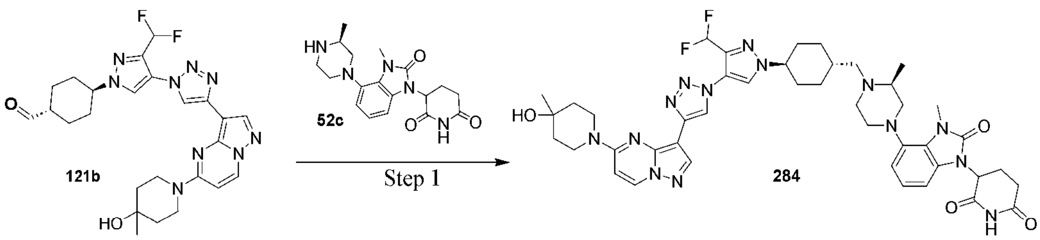


Triethylamine (0.020 mL, 0.15 mmol) was added to a solution of compound 52c (65.3 mg, 183 μmmol) in *N*,*N*-dimethylformamide (1 mL) and the obtained mixture was stirred for 1 min. Then acetic acid (0.010 mL, 167 μmmol) and sodium borohydride acetate (128 mg, 610 μmmol) were added to the above solution, and the obtained mixture was stirred for 1 min. Finally, compound 121b (80.0 mg, 152 μmmol) was added to the above reaction solution. The reaction was stirred at 25°C for 30 min. The reaction solution was concentrated under reduced pressure and then purified by high performance liquid chromatography (Gilson_306_1741, chromatographic column: Waters-Xbridge-C18-10μm-19*250 mm; mobile phase: water (containing 26.5 mmol/L formic acid) and acetonitrile, gradient ratio: acetonitrile 2%-95%, flow rate: 25 mL/min) to obtain compound 284. MS m/z(ESI):867.5[M+1]$^+$. $^1$H NMR (400 MHz, DMSO-$d_6$) δ 11.09 (s, 1H), 8.70 - 8.65 (m, 2H), 8.53 (s, 1H), 8.34 (s, 1H), 7.22 (t, *J* = 53.2 Hz, 1H), 7.04 - 6.85 (m, 3H), 6.82 (d, *J* = 8.0 Hz, 1H), 5.37 - 5.32 (m, 1H), 4.46 (s, 1H), 4.33 - 4.24 (m, 1H), 4.20 - 4.01 (m, 1H), 3.64 (s, 3H), 3.49 - 3.43 (m, 2H), 3.05 - 2.80 (m, 4H), 2.78 - 2.60 (m, 2H), 2.34 - 2.31 (m, 1H), 2.25 - 2.05 (m, 4H), 2.03 - 1.96 (m, 4H), 1.90 - 1.75 (m, 4H), 1.69 - 1.45 (m, 7H), 1.16 (s, 3H), 1.06 (s, 3H), 0.90 - 0.82 (m, 1H).

Example 285: 3-(4-((S)-4-(((1r,4S)-4-(4-(4-(5-(S)-3-aminopiperidin-1-yl)pyrazolo[1,5-a]pyrimidin-3-yl)-1H-1,2,3-triazol-1-yl)-3-(difluoromethyl)-1H-pyrazol-1-yl(cyclohexyl)methyl)-3-methylpiperazin-1-yl)3-methyl-2-oxo-2,3-dihydro-1H-benzo[d]imidazol-1-yl)piperidine-2,6-dione (285)

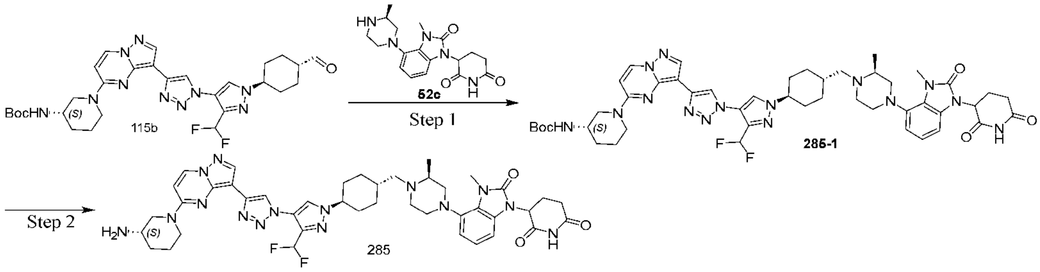


1) Step 1: Triethylamine (0.30 mL, 2.16 mmol) was added to a solution of compound 52c (40 mg, 110 μmol) in tetrahydrofuran (2 mL) and *N,N*-dimethylformamide (1 mL), and the obtained mixture was stirred for 30 min. Then compound 115b (76.0 mg, 120 μmol) and acetic acid (0.5 mL) were added, and the obtained mixture was stirred for 30 min. Then sodium borohydride acetate (71.0 mg, 340 μmol) was added, and the obtained mixture was stirred at 25°C for 2 h, then concentrated under reduced pressure. The residue was purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 285-1. MS m/z(ESI):952.6$[M+1]^+$.

2) Step 2: Hydrogen chloride/dioxane (1 mL, 4 M) was added to a solution of compound 285-1 (20 mg, 21 μmol) in tetrahydrofuran (1 mL). The obtained mixture was stirred at 25°C for 1 h and then concentrated under reduced pressure. The residue was freeze-dried to obtain compound 285. MS m/z(ESI):852.7$[M+1]^+$, $^1H$ NMR (400 MHz, DMSO-$d_6$) δ 11.17 (s, 1H), 8.92 - 8.88 (m, 1H), 8.85 (d, *J* = 7.9 Hz, 1H), 8.71 (s, 1H), 8.45 (s, 1H), 7.44 - 7.19 (m, 2H), 7.17 - 7.05 (m, 2H), 7.05 - 6.92 (m, 2H), 6.86 (d, *J* = 8.2 Hz, 1H), 6.73 - 6.54 (m, 1H), 5.48 - 5.35 (m, 2H), 4.56 - 4.35 (m, 2H), 4.13 - 4.01 (m, 1H), 3.71 (s, 3H), 3.58 - 3.51 (m, 2H), 3.02 - 2.91 (m, 2H), 2.79 - 2.64 (m, 4H), 2.30 - 2.25 (m, 2H), 2.08 - 2.01 (m, 6H), 1.99 - 1.89 (m, 4H), 1.80 - 1.62 (m, 4H), 1.53 - 1.43 (m, 4H), 1.41 - 1.35 (m, 2H).

Example 286: 7-1H-benzo[d]imidazol-4-yl)piperidin-4-yl)methyl)-3-azaspiro[5.5]undecan-9-yl)-1H-pyrazol-4-yl)-1H-1,2,3-triazole)-1H-pyrrolo[2,3-b]pyridine-5-carboxamide (286)

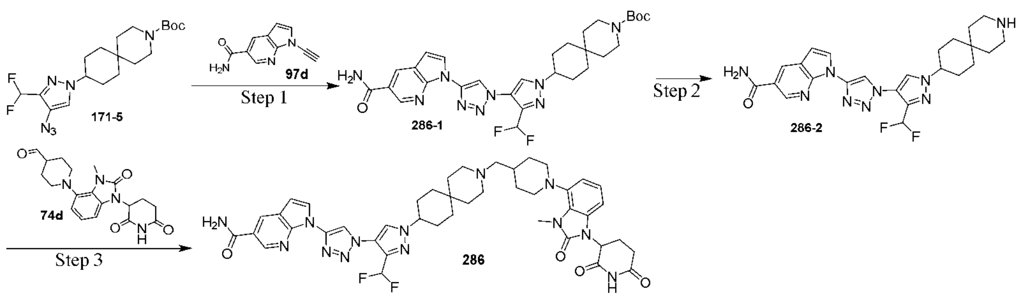


1) Step 1: Compound 97d (50 mg, 270 μmol) and compound 171-5 (111 mg, 270 μmol) were dissolved in a mixed solution of water (3 mL) and ethanol (3 mL), and copper sulfate pentahydrate (6.74 mg, 27.0 μmol) and sodium ascorbate (5.35 mg, 27.0 μmol) were added, and the obtained mixture was reacted under stirring at room temperature for 12 h. Then the ethanol was removed by concentration under reduced pressure. Water (20 mL) was added to the reaction solution, and a large amount of solid precipitated and was filtered. The filter cake was washed with petroleum ether/ethyl acetate (1/1, 5 mL) to obtain compound 286-1. MS m/z(ESI):596.5$[M+1]^+$.

2) Step 2: Compound 286-1 (150 mg, 252 μmol) was dissolved in dichloromethane (10 mL) solution, and hydrogen chloride/dioxane (10 mL, 4 M) was added, and the obtained mixture was reacted under stirring at room temperature for 1 h. The reaction system was directly concentrated to obtain a crude product. The crude product was directly used as the raw material for the next step without purification. Compound 286-2 was obtained. MS m/z(ESI): 496.3$[M+1]^+$.

3) Step 3: Compound 286-2 (66.9 mg, 135 μmol) was dissolved in *N,N*-dimethylformamide (1 mL) and tetrahydrofuran (4 mL) at room temperature, triethylamine (13.7 mg, 135 μmol) was added, and the obtained mixture was stirred for 15 min. Sodium borohydride acetate (142 mg, 675 μmol), compound 74d (50.0 mg, 135 μmol) and acetic acid (16.2 mg, 270 μmol) were then added to the mixture, and the mixture was stirred at 25°C for 30 min. The mixture was concentrated and the residue was purified by high performance liquid chromatography (Gilson_306_1741, chromatographic column: Waters-SunFire-C18-10μm-19×250 mm; mobile phase: water (containing 26.5 mmol/L formic

acid) and acetonitrile, gradient ratio: acetonitrile 16%-95%, flow rate: 25 mL/min) to obtain compound 286. MS m/z(ESI): 850.8[M+1]$^+$. $^1$H NMR (400 MHz, DMSO-$d_6$): δ 11.09 (s, 1H), 9.04 (s, 1H), 8.95 - 8.89 (m, 1H), 8.85 (s, 1H), 8.63 (d, *J* = 2.1 Hz, 1H), 8.27 (d, *J* = 3.8 Hz, 1H), 8.18 (s, 1H), 7.50 (s, 1H), 7.26 (t, *J* = 53.6 Hz, 1H), 7.03 - 6.87 (m, 4H), 5.41 - 5.30 (m, 1H), 4.33 (s, 1H), 3.63 (s, 3H), 3.18 - 2.84 (m, 7H), 2.78 - 2.57 (m, 4H), 2.06 - 1.86 (m, 9H), 1.77 - 1.73 (m, 1H), 1.55 - 1.38 (m, 4H), 1.36 - 1.33 (m, 1H), 1.27 - 1.21 (m, 5H).

Example 287: 3-(4-((S)-4-(((1R,4S)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl)cyclohexyl)methyl)-3-methylpiperazin-1-yl)-3-methyl-2-oxo-2,3-dihydro-1H-benzo[d]imidazol-1-yl)piperidine-2,6-dione (287)

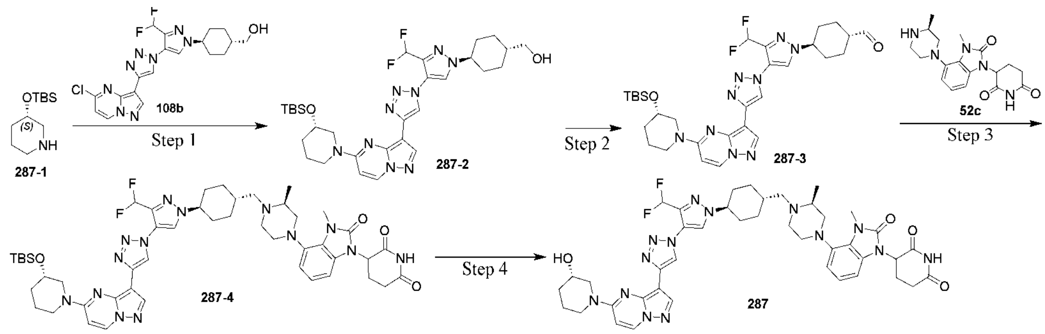


1) Step 1: Compound 108b (500 mg, 1.11 mmol) and compound 287-1 (420 mg, 1 mmol, prepared by the method disclosed for the compound 2 in step 0189 on page 53 of the specification of the patent application "WO 2019089422 A1") were dissolved in acetonitrile (15 mL) and *N*,*N*-diisopropylethylamine (450 mg, 3.48 mmol) was added. The reaction solution was stirred at 70°C for 2 h. The reaction solution was poured into water (50 mL), and ethyl acetate was used for extraction (50 mL × 3). The combined organic phase was washed with saturated brine (100 mL), dried over anhydrous sodium sulfate and filtered, and the filtrate was concentrated under reduced pressure. The residue was purified by silica gel column chromatography (dichloromethane/methanol = 20/1 to 10/1) to obtain compound 287-2. MS m/z(ESI):628.5 [M+1]$^+$.

2) Step 2: Compound 287-2 (600 mg, 0.955 mmol) was dissolved in dichloromethane (15 mL), and Dess-Martin oxidant (600 mg, 1.41 mmol) was added. The reaction solution was stirred at 25°C for 1 h. The reaction solution was poured into water (30 mL) and dichloromethane was used for extraction (30 mL × 3). The combined organic phase was washed with saturated brine (30 mL), dried over anhydrous sodium sulfate and filtered, and the filtrate was concentrated under reduced pressure. The residue was purified by silica gel column chromatography (dichloromethane/methanol = 20/1 to 10/1) to obtain compound 287-3 (480 mg). MS m/z(ESI):626.5 [M+1]$^+$.

3) Step 3: Compound 52c (80 mg, 0.22 mmol) and compound 287-3 (150 mg, 0.240 mmol) were dissolved in N,N-dimethylformamide (5 mL), and acetic acid (0.5 mL) and sodium borohydride acetate (450 mg, 2.13 mmol) were added in sequence. The reaction solution was stirred at 25°C for 3 h. The reaction solution was poured into water (10 mL) and dichloromethane was used for extraction (10 mL × 3). The combined organic phase was washed with saturated brine (30 mL), dried over anhydrous sodium sulfate and filtered, and the filtrate was concentrated under reduced pressure. The residue was purified on silica gel plate for thin layer chromatography (dichloromethane/methanol = 10/1) to obtain compound 287-4. MS m/z(ESI):967.9 [M+1]$^+$.

4) Step 4: Compound 287-4 (60 mg, 0.062 mmol) was dissolved in dioxane (2 mL), and hydrogen chloride/dioxane (1 mL, 4 M) was added. The reaction solution was stirred at 25°C for 1 h. The reaction solution was concentrated under reduced pressure, and the residue was purified by high performance liquid chromatography (A: 0.1% formic acid/water, B: acetonitrile, chromatographic column: Waters-SunFire-C18-10μm-19×250 mm; mobile phase: water (containing 0.1% formic acid) and acetonitrile, gradient ratio: acetonitrile 37%-45%, flow rate: 25 mL/min) to obtain compound 287. MS m/z(ESI):853.8 [M+1]$^+$. $^1$H NMR (400 MHz, DMSO-$d_6$): δ 11.09 (s, 1H), 8.68 - 8.65 (m, 2H), 8.50 (s, 1H), 8.34 (s, 1H), 7.14 (t, *J* = 53.2 Hz, 1H), 7.01 - 6.88 (m, 3H), 6.78 (d, *J* = 8.0 Hz, 1H), 5.39 - 5.32 (m, 1H), 4.92 - 4.86 (m, 1H), 4.35 - 4.27 (m, 1H), 4.12 - 3.97 (m, 2H), 3.66 - 3.59 (m, 4H), 3.47 - 3.42 (m, 1H), 3.24 - 3.17 (m, 2H), 3.04 - 2.84 (m, 5H), 2.71 - 2.59 (m, 3H), 2.23 - 2.13 (m, 3H), 2.02 - 1.78 (m, 8H), 1.68 - 1.41 (m, 4H), 1.19 - 1.02 (m, 5H).

Example 288: 1-(1-(3-(difluoromethyl)-1-((1r,4r)-4-((4-(1-(2,6-dioxopiperidin-3-yl)-3-methyl-2,3-dihydro-1H-benzo[d]imidazol-4-yl)-1,4-diaza-1-yl)methyl)cyclohexyl)-1H-pyrazol-4-yl)-1H-pyrrolo[2,3-b]pyridine-5-carboxamide (288)

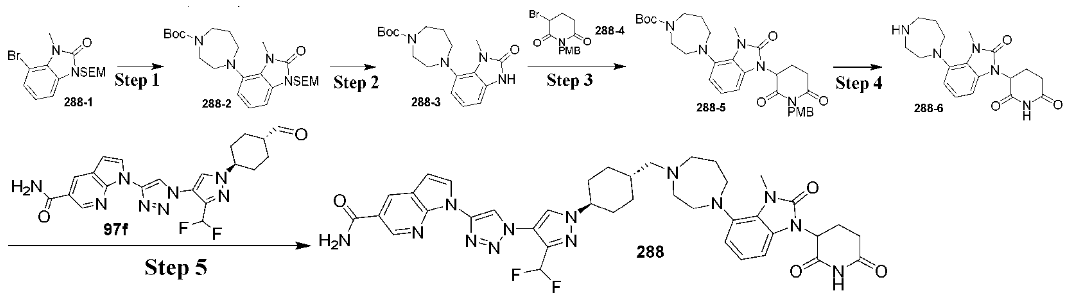


1) Step 1: Compound 288-1 (2.50 g, 7.00 mmol, prepared by the method disclosed for step 001032 product on page 388 of the specification of "WO 2021188948 A1 "), 2-methylpropan-2-yl 1,4-diazepane-1-carboxylate (2.10 g, 10.5 mmol), 2-dicyclohexylphosphino-2',6'-diisopropoxy-1,1'-biphenyl (0.650 g, 1.40 mmol, Bide Pharmatech Ltd.), tris(dibenzylideneacetone)dipalladium (0.650 g, 14.0 mmol, Bide Pharmatech Ltd.), tris(dibenzylideneacetone) dipalladium (0.640 g, 0.700 mmol, Bide Pharmatech Ltd.) and sodium tert-butoxide (1.34 g, 14.0 mmol, Bide Pharmatech Ltd.) were dissolved in dioxane (50 mL), and the reaction solution was subjected to nitrogen replacement three times and stirred at 80°C for 12 h. The reaction solution was diluted with dichloromethane (200 mL) and water (100 mL), and the aqueous phase was extracted with dichloromethane (50 mL × 3). The combined organic phase was washed with saturated brine (50 mL), dried over anhydrous sodium sulfate and filtered to remove the desiccant, and then the filtrate was concentrated under reduced pressure. The residue was purified by silica gel column chromatography (petroleum ether/ethyl acetate = 100/1 to 4/1) to obtain compound 288-2. MS m/z(ESI):477.4[M+1]$^+$.

2) Step 2: Compound 288-2 (2.90 g, 6.08 mmol) was dissolved in tetrahydrofuran (30 mL). Tetrabutylammonium fluoride (42.6 mL, 42.6 mmol, 1.0 M solution in tetrahydrofuran) was added to the reaction solution at 25°C. The reaction solution was heated to 80°C and stirred at this temperature for 12 h. The reaction solution was concentrated under reduced pressure, the residue was diluted with dichloromethane (200 mL), and the organic phase was washed with saturated sodium bicarbonate (50 mL × 3) dried over anhydrous sodium sulfate and filtered to remove the desiccant, and then the filtrate was concentrated under reduced pressure. The residue was purified by silica gel column chromatography (dichloromethane/methanol = 100/1 to 50/1) to obtain compound 288-3. MS m/z(ESI):347.3 [M+1]$^+$.

3) Step 3: Compound 288-3 (1.60 g, 4.62 mmol) was dissolved in tetrahydrofuran (10 mL). Potassium tert-butoxide (6.9 mL, 6.93 mmol, 1.0 M solution in tetrahydrofuran) was slowly added dropwise to the reaction solution at 0°C. The reaction solution was stirred at 0°C under nitrogen atmosphere for 0.5 h, then a solution of compound 288-4 (2.16 g, 6.93 mmol, Bide Pharmatech Ltd.) in tetrahydrofuran (10 mL) was slowly added dropwise. The reaction solution was heated to 25°C and reacted at this temperature for 12 h. The reaction solution was poured into ice water (100 mL), the aqueous phase was subjected to liquid separation and extracted with dichloromethane (50 mL × 3). The combined organic phase was dried over anhydrous sodium sulfate and filtered to remove the desiccant, and then the filtrate was concentrated under reduced pressure. The residue was purified by silica gel column chromatography (petroleum ether/ethyl acetate = 100/1 to 1.5/1) to obtain compound 288-5. MS m/z(ESI):578.4[M+1]$^+$.

4) Step 4: Compound 288-5 (1.80 g, 3.12 mmol) was dissolved in trifluoroacetic acid (15 mL), and trifluoro(1.5 mL) was added to the reaction solution at 25°C. The reaction solution was heated to 80°C and stirred at this temperature for 3 h. The reaction solution was concentrated under reduced pressure, and the residue was purified with a reverse-phase column (0.1% formic acid aqueous solution/acetonitrile = 2.1%) to obtain compound 288-6. MS m/z(ESI):358.4 [M+1]$^+$;

5) Step 5: Compound 288-6 (51.1 mg, 0.140 mmol) was dissolved in *N,N*-dimethylformamide (1.5 mL), and triethylamine (11.1 mg, 0.110 mmol) was added at 25°C. The reaction solution was stirred at 25°C for 5 min, and then acetic acid (6.61 mg, 0.110 mmol) and sodium borohydride acetate (92.8 mg, 0.440 mmol, Bide Pharmatech Ltd.) were added. The reaction solution was stirred for 5 min, and then compound 97f (50.0 mg, 0.110 mmol) was added and the reaction solution was stirred for 1 h. Filtration was performed. The residue was purified by high performance liquid chromatography (Waters-2545, chromatographic column: SharpSil-T C18, 30*150 mm, 5 μm; mobile phase: water (containing 10 mmol/L formic acid) and acetonitrile, gradient ratio: acetonitrile 38%-45%, flow rate: 30 mL/min) to obtain compound 288. MS m/z(ESI):796.7[M+1]$^+$. $^1$H NMR (400 MHz, DMSO-$d_6$): δ 11.08 (s, 1H), 9.06 (d, *J* = 10.4 Hz, 1H), 8.90 (d, *J* = 1.9 Hz, 1H), 8.82 (d, *J* = 31.4 Hz, 1H), 8.62 (d, *J* = 2.0 Hz, 1H), 8.27 (d, *J* = 3.7 Hz, 1H), 8.23 (s, 1H), 7.50

(s, 1H), 7.39 - 7.10 (m, 1H), 7.01 - 6.95 (m, 2H), 6.94 (d, *J* = 3.7 Hz, 1H), 6.89 - 6.84 (m, 1H), 5.41 - 5.28 (m, 1H), 4.45 - 4.20 (m, 1H), 3.66 (s, 3H), 3.15 (s, 5H), 2.90 - 2.61 (m, 7H), 2.40 - 2.32 (m, 2H), 2.22 - 2.14 (m, 2H), 2.04 - 1.95 (m, 3H), 1.93 - 1.80 (m, 4H), 1.62 - 1.54 (m, 1H), 1.14 - 1.10 (m, 1H).

Example 289: 7-(1-(3-(difluoromethyl)-1-((1R,4R)-4-((4-(1-(2,6-dioxopiperidin-3-yl)-3-methyl-2-oxo-2,3-dihydro-1H-benzoimidazol-4-yl)-1,4-diazepan-1-yl)methyl)cyclohexyl)-1H-pyrazol-4-yl)-1H-1,2,3-triazol-4-yl)pyrrolo[1,2-b]pyridazine-3-carboxamide (289)

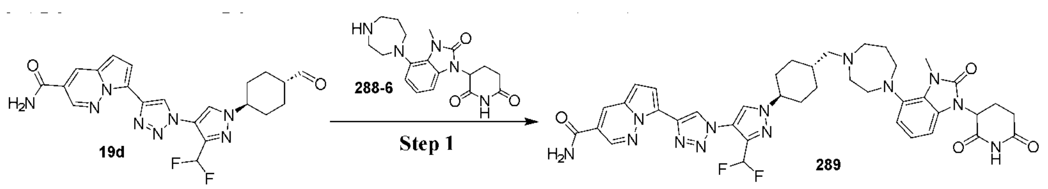


Compound 288-6 (51.1 mg, 143 μmol) was dissolved in *N,N*-dimethylformamide (1 mL) and tetrahydrofuran (4 mL) at room temperature, triethylamine (14.5 mg, 143 μmol) was added, and the obtained mixture was stirred for 15 min. Sodium borohydride acetate (151 mg, 715 μmol), compound 19d (65.0 mg, 143 μmol) and acetic acid (17.2 mg, 286 μmol) were then added to the mixture, and the mixture was stirred at 25°C for 30 min. The mixture was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 16%-95%, flow rate: 25 mL/min) to obtain compound 289. MS m/z(ESI): 796.7[M+1]$^+$. $^1$H NMR (400 MHz, DMSO-$d_6$) δ 11.09 (s, 1H), 9.01 (s, 1H), 8.78 (d, *J* = 2.3 Hz, 1H), 8.75 (s, 1H), 8.65 (d, *J* = 2.2 Hz, 1H), 8.16 (s, 1H), 7.65 (d, *J* = 4.6 Hz, 1H), 7.56 (s, 1H), 7.23 (t, *J* = 53.3 Hz, 1H), 7.05 (d, *J* = 4.6 Hz, 1H), 7.01 - 6.94 (m, 2H), 6.90 - 6.84 (m, 1H), 5.40 - 5.30 (m, 1H), 4.36 - 4.26 (m, 1H), 3.66 (s, 3H), 3.20 - 3.10 (m, 5H), 2.93 - 2.84 (m, 1H), 2.81 - 2.74 (m, 4H), 2.71 - 2.59 (m, 2H), 2.38 (d, *J* = 7.0 Hz, 2H), 2.22 - 2.14 (m, 2H), 2.03 - 1.95 (m, 3H), 1.91 - 1.86 (m, 2H), 1.84 - 1.79 (m, 1H), 1.64 - 1.53 (m, 1H), 1.18 - 1.06 (m, 2H).

Example 290: 3-(4-(4-((9-(3-(difluoromethyl)-4-(4-(5-(((S)-3-hydroxypiperidin-1-yl)pyrazolo[1,5-a]pyrimidin-3-yl)-1H-1,2,3-triazol-1-yl)-1H-pyrazol-3-yl)-3-azaspiro[5.5]undecan-3-yl)methyl)piperidin-1-acyl)-3-methyl-2-oxo-2,3-dihydro-1H-benzo[d]imidazol-1-yl)piperidine-2,6-dione (290)

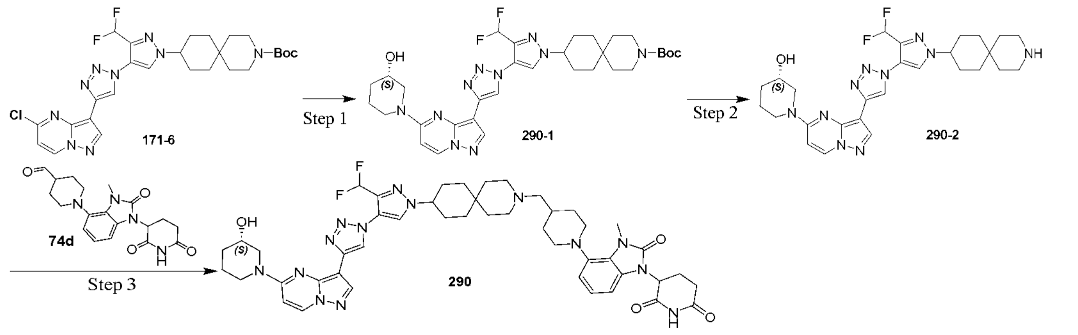


1) Step 1: N,N-diisopropylethylamine (0.36 mL, 2.04 mmol) was added to an solution of compound 171-6 (400 mg, 0.68 mmol) and (S)-3-hydroxypiperidine hydrochloride (187 mg, 1.36 mmol) in acetonitrile (8 mL) at room temperature, and the reaction solution was stirred at 50°C for 1 h. The reaction solution was diluted with water (20 mL) and extracted with ethyl acetate (30 mL × 2). The organic phases were combined, washed with brine (20 mL), dried over anhydrous sodium sulfate and filtered, and the filtrate was concentrated under reduced pressure. The residue was purified by silica gel column chromatography (petroleum ether/ethyl acetate = 100/1 to 10/90) to obtain compound 290-1. MS m/z(ESI):653.5 [M+1]$^+$.

2) Step 2: Trifluoroacetic acid (5 mL) was added to a solution of compound 290-1 (390 mg, 0.60 mmol) in dichloromethane (5 mL) at room temperature, and the obtained mixture was stirred for 1 h. The reaction solution was concentrated under reduced pressure to obtain compound 290-2. MS m/z(ESI):553.8[M+1]$^+$.

3) Step 3: Triethylamine (37.0 mg, 0.36 mmol) was added to compound 290-2 (200 mg, 0.36 mmol) and *N*,*N*-dimethylformamide (5 mL) at room temperature and the obtained mixture was stirred for 5 min. Then acetic acid (37.0 mg, 0.62 mmol) was added and the obtained mixture was stirred for 5 min. Compound 74d (94.0 mg, 0.25 mmol) was added to the above solution and the obtained mixture was stirred for 20 min. Finally, sodium borohydride acetate (153 mg, 0.72 mmol) was added in portions, and the reaction solution was stirred at 0°C for 30 min. The reaction solution was added to water (10 mL) and ethyl acetate (10 mL × 3) was used for extraction. The organic phases were combined, washed with brine (10 mL), dried over anhydrous sodium sulfate and filtered, and the filtrate was concentrated under reduced pressure. The residue was purified by high performance liquid chromatography (Gilson_306_1741, chromatographic column: Waters-SunFire-C18-10μm-19×250 mm; mobile phase: water (containing 0.1% formic acid) and acetonitrile, gradient ratio: acetonitrile 10%-30%, flow rate: 25 mL/min) to obtain compound 290. MS m/z(ESI):907.8[M+1]$^+$. $^1$H NMR (400 MHz, DMSO-$d_6$) δ11.09 (s, 1H), 8.72 (s, 1H), 8.67 (d, *J* = 8.0 Hz, 1H), 8.50 (s, 1H), 8.34 (s, 1H), 7.21 (t,*J* = 53.2 Hz, 1H), 7.01 - 6.94 (m, 1H), 6.93 - 6.83 (m, 2H), 6.78 (d, *J* = 8.0 Hz, 1H), 5.43 - 5.30 (m, 1H), 5.04 - 4.62 (m, 1H), 4.35 - 4.24 (m, 1H), 4.16 - 3.95 (m, 2H), 3.65 - 3.58 (m, 4H), 3.48 - 3.40 (m, 2H), 3.23 - 3.18 (m, 1H), 3.15 - 3.08 (m, 2H), 2.94 - 2.84 (m, 1H), 2.75 - 2.59 (m, 4H), 2.46 - 2.41 (m, 3H), 2.35 - 2.23 (m, 2H), 2.03 - 1.76 (m, 11H), 1.73 - 1.58 (m, 3H), 1.51 - 1.23 (m, 8H).

Example 291: 3-(4-(4-((S)-4-(((1R,4S)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl)cyclohexyl)methyl)-3-methylpiperazin-1-yl)piperidin-1-yl)-3-methyl-2-oxo-2,3-dihydro-1H-benzo[d]imidazol-1-yl)piperidine-2,6-dione (291)

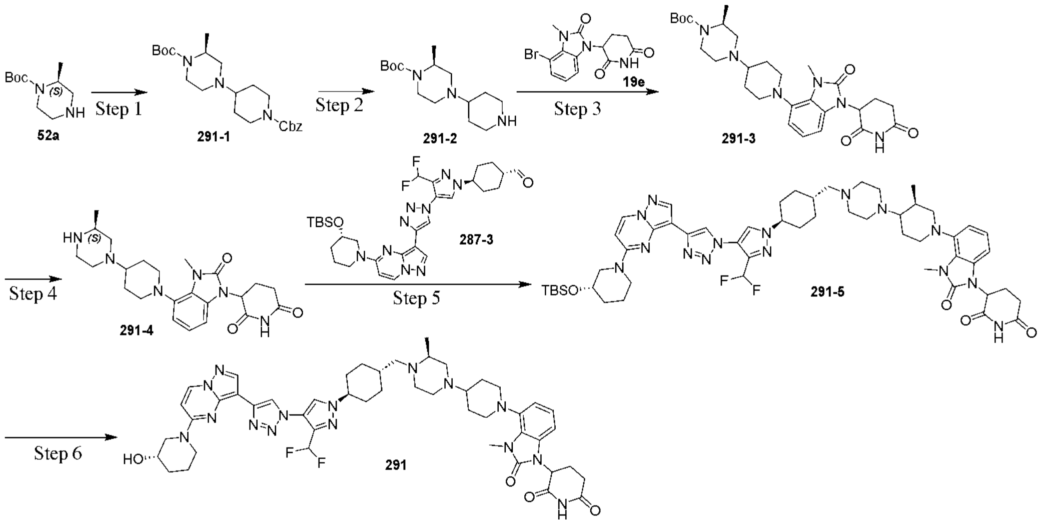


1) Step 1: Compound 52a (4.50 g, 22.5 mmol) and 1-benzyloxycarbonyl-4-piperidone (5.00 g, 21.4 mmol) were dissolved in methanol (80 mL), and acetic acid (2 mL) was added to the reaction solution. The reaction solution was stirred at 25°C for 1 h, and then sodium cyanoborohydride (3.00 g, 62.8 mmol) was added. The reaction solution was further stirred for 2 h. The reaction solution was poured into water (100 mL), and ethyl acetate (80 mL) was used for extraction for three times. The organic phase was washed with saturated brine (100 mL), dried over anhydrous sodium sulfate and filtered. The filtrate was concentrated under reduced pressure, and the residue was subjected to silica gel column chromatography (dichloromethane/methanol = 20/1 to 15/1) to obtain compound 291-1. MS m/z(ESI):418.7 [M+1]$^+$.

2) Step 2: Compound 291-1 (2.80 g, 2.71 mmol) was dissolved in methanol (30 mL), and wet palladium carbon (200 mg, 10%) was added to the reaction solution. The reaction solution was stirred at 25°C under hydrogen atmosphere (1 atm) for 18 h. The reaction solution was filtered, and the filtrate was concentrated under reduced pressure to obtain compound 291-2, which was directly used in the next step. MS m/z(ESI):284.2[M+1]$^+$.

3) Step 3: Compound 19e (450 mg, 1.33 mmol), compound 291-2 (565.7 mg, 2.01 mmol), 2-dicyclohexylphosphino-2',6'-diisopropoxy-1,1'-biphenyl (124 mg, 0.27 mmol), (2-dicyclohexylphosphino-2',6'-diisopropoxy-1,1'-biphenyl)(2-amino-1,1'-biphenyl-2-yl) palladium (II) methanesulfonate (223 mg, 0.27 mmol) were dissolved in toluene (8 mL), lithium bis(trimethylsilyl)amide (6.65 mL, 6.65 mmol, 1 M solution in tetrahydrofuran) was added, and the obtained mixture was reacted at 80°C for 1 h under nitrogen atmosphere. Water (10 mL) was added to the reaction solution, and dichloromethane was used for extraction (50 mL × 3). The organic phases were combined, dried over

anhydrous sodium sulfate and filtered, and the filtrate was concentrated under reduced pressure. The residue was purified by silica gel column chromatography with an eluent system (dichloromethane/methanol = 20/1) to obtain compound 291-3. MS m/z(ESI):541.6[M+1]$^+$.

4) Step 4: Compound 291-3 (90 mg, 0.171 mmol) was dissolved in dioxane (0.5 mL), and hydrogen chloride/dioxane (2 mL, 4 M) was added. The obtained mixture was reacted at room temperature for 2 h, and the reaction solution was concentrated under reduced pressure to obtain compound 291-4. MS m/z(ESI):441.4[M+1]$^+$.

5) Step 5: Compound 291-4 (100 mg, 0.227 mmol) and compound 287-3 (140 mg, 0.224 mmol) were dissolved in *N*,*N*-dimethylformamide (5 mL), and acetic acid (0.5 mL) and sodium borohydride acetate (500 mg, 2.37 mmol) were added in sequence. The reaction solution was stirred at 25°C for 2 h. The reaction solution was poured into water (30 mL) and ethyl acetate (30 mL) was used for extraction three times. The organic phases were combined, washed with brine (50 mL), dried over anhydrous sodium sulfate and filtered, and the filtrate was concentrated under reduced pressure. The residue was purified by silica gel column chromatography (dichloromethane/methanol = 15/1 to 10/1) to obtain compound 291-5. MS m/z(ESI):1049.0 [M-1]$^-$.

6) Step 6: Compound 291-5 (100 mg, 0.0952 mmol) was dissolved in dioxane (2 mL), and hydrogen chloride/dioxane (1 mL, 4 M) was added. The reaction solution was stirred at 25°C for 1 h. The reaction solution was concentrated under reduced pressure, and the residue was purified by high performance liquid chromatography (A: 0.1% formic acid/water, B: acetonitrile, chromatographic column: Waters-SunFire-C18-10$\mu$m-19$\times$250 mm; mobile phase: water (containing 0.1% formic acid) and acetonitrile, gradient ratio: acetonitrile 40%-48%, flow rate: 25 mL/min) to obtain compound 291 (20 mg). MS m/z(ESI):468.8 [M/2+1]$^+$. $^1$H NMR (400 MHz, DMSO-$d_6$): $\delta$ 11.09 (s, 1H), 8.68 - 8.66 (m, 2H), 8.50 (s, 1H), 8.34 (s, 1H), 7.20 (t, *J* = 53.6 Hz, 1H), 6.99 - 6.95 (m, 1H), 6.92 - 6.87 (m, 2H), 6.78 (d, *J* = 8.1 Hz, 1H), 5.38 - 5.32 (m, 1H), 4.90 - 4.87 (m, 1H), 4.32 - 4.26 (m, 1H), 4.10 - 3.98 (m, 2H), 3.65 - 3.58 (m, 5H), 3.20 - 3.13 (m, 4H), 2.83 - 2.66 (m, 7H), 2.39 - 2.26 (m, 5H), 2.18 - 2.12 (m, 4H), 1.95 - 1.78 (m, 10H), 1.60 - 1.48 (m, 4H), 1.07 - 0.99 (m, 5H).

Example 292: 3-(4-(4-(((R)-4-(((1r,4R)-4-)3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl(cyclohexyl)methyl)-3-methylpiperazin-1-yl)piperidin-1-yl)-3-methyl-2-oxo-2,3-dihydro-1H-benzo[d]imidazol-1-yl)pyridine-2,6-dione (292)

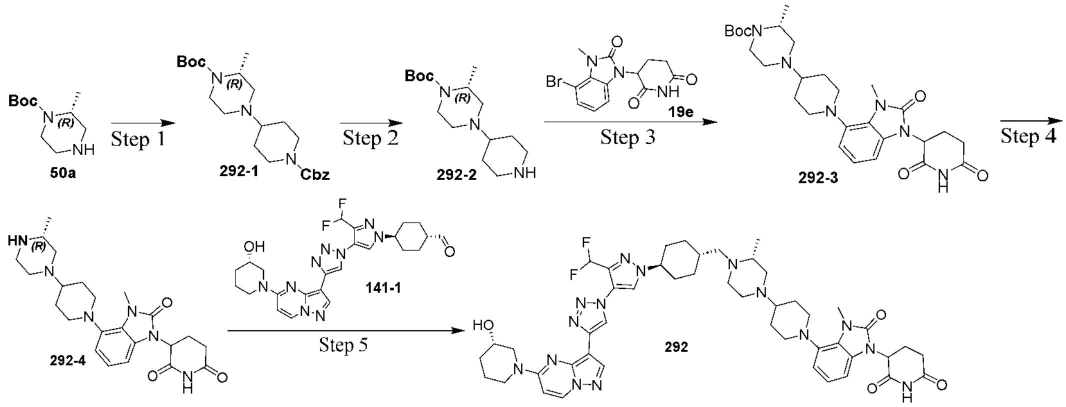


1) Step 1: Compound 1-benzyloxycarbonyl-4-piperidone (5.00 g, 21.4 mmol) and acetic acid (4.90 mL, 85.7 mmol) were added to a solution of compound 50a (5.15 g, 25.7 mmol) in methanol (50 mL) at room temperature, and the obtained mixture was stirred at room temperature for 1 h. Sodium cyanoborohydride (6.73 g, 107 mmol) was added, and the obtained mixture was stirred at room temperature for 18 h. Water (1 mL) was added to quench the reaction. The reaction solution was evaporated to dryness under reduced pressure, and the residue was purified by column chromatography (dichloromethane/methanol = 10/1) to obtain compound 292-1. MS m/z(ESI):418.7[M+1]$^+$.

2) Step 2: Wet palladium carbon (50.0 mg, 10%) was added to a solution of compound 292-1 (2.00 g, 4.79 mmol) in methanol (30 mL) at room temperature, and the system was subjected to hydrogen replacement 3 times. The reaction was stirred for 18 h under hydrogen (1 atm). The reaction solution was evaporated to dryness under reduced pressure to obtain compound 292-2, which was directly used in the next step. MS m/z(ESI):284.3 [M+1]$^+$.

3) Step 3: Compound 19e (600 mg, 1.78 mmol), 2-dicyclohexylphosphino-2',6'-diisopropoxy-1,1'-biphenyl (82.8 mg, 0.18 mmol), (2-dicyclohexylphosphino-2',6'-diisopropoxy-1,1'-biphenyl)(2-amino-1,1'-biphenyl-2-yl)palladium (II) methanesulfonate (148 mg, 0.18 mmol), lithium bis(trimethylsilyl)amide (8.87 mL, 8.87 mmol, 1 M solution in tetrahydrofuran) were added to a solution of compound 292-2 (754 mg, 2.66 mmol) in toluene (10 mL) at room temperature. The system was subjected to nitrogen replacement 3 times and the reaction was stirred at 80°C for 2 h.

The reaction solution was poured into saturated ammonium chloride solution (20 mL) and dichloromethane (20 mL × 3) was used for extraction. The organic phases were combined, dried over anhydrous sodium sulfate, filtered and evaporated to dryness under reduced pressure. The residue was purified by column chromatography (dichloromethane/methanol = 20/1) to obtain compound 292-3 (130 mg). MS m/z(ESI):541.5[M+1]+.

4) Step 4: Trifluoroacetic acid (1 mL) was added to a solution of compound 292-3 (100.0 mg, 185 μmol) in dichloromethane (2 mL). The reaction was stirred at 25°C for 1 h, and the reaction solution was concentrated under reduced pressure to obtain compound 292-4. The product was directly used in the next step without purification.

5) Step 5: Triethylamine (0.30 mL, 0.216 mmol) was added to a solution of compound 292-4 (50 mg, 110 μmol) in tetrahydrofuran (2 mL) and N,N-dimethylformamide (1 mL), and the obtained mixture was stirred for 30 min. Then compound 141-1 (70.0 mg, 120 μmol) and acetic acid (0.5 mL) were added, and the obtained mixture was further stirred for 30 min. Then sodium borohydride acetate (72.0 mg, 330 μmol) was added, and the obtained mixture was stirred at 25°C for 2 h, then concentrated under reduced pressure. The residue was purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 292 (20 mg). MS m/z(ESI):936.6[M+1]+. $^1$H NMR (400 MHz, DMSO-$d_6$) δ 11.09 (m, 1H), 8.67 (s, 1H), 8.65 (s, 1H), 8.50 (s, 1H), 8.34 (s, 1H), 7.20 (t, *J* = 53.2 Hz, 1H), 6.99 - 6.95 (m, 1H), 6.92 - 6.85 (m, 2H), 6.78 (d, *J* = 8.0 Hz, 1H), 5.40 - 5.31 (m, 1H), 4.93 - 4.77 (m, 1H), 4.34 - 4.24 (m, 1H), 4.10 - 3.98 (m, 2H), 3.63 (s, 3H), 3.61 - 3.55 (m, 2H), 3.18 - 3.12 (m, 3H), 2.98 - 2.85 (m, 2H), 2.74 - 2.64 (m, 4H), 2.62 - 2.54 (m, 4H), 2.46 - 2.26 (m, 6H), 2.16 - 2.10 (m, 4H), 2.05 - 1.91 (m, 4H), 1.87 - 1.77 (m, 5H), 1.68 - 1.57 (m, 3H), 1.52 - 1.42 (m, 2H), 1.13 - 1.01 (m, 2H).

Example 293: 3-(7-(4-(4-(((1r,4r)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl(cyclohexyl)methyl)piperazin-1-yl)piperidin-1-acyl)-1-methyl-1H-indazol-3-yl) piperidine-2,6-dione (293)

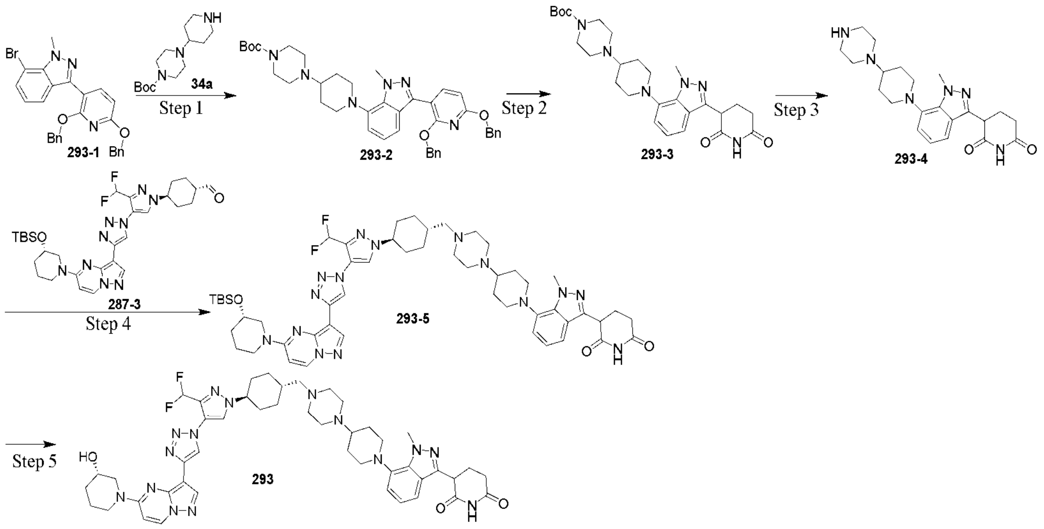


1) Step 1: Compound 293-1 (800 mg, 1.60 mmol) and toluene (5 mL) were added to a single-neck bottle at room temperature and the obtained mixture was stirred. Compound 34a (862 mg, 3.20 mmol), tris(dibenzylideneacetone) dipalladium (147 mg, 0.16 mmol), sodium tert-butoxide (460.0 mg, 4.80 mmol) and 1,1'-binaphthyl-2,2'-bis(diphenylphosphine) (100.0 mg, 0.16 mmol) were added. The reaction solution was stirred at 80°C for 6 h under nitrogen atmosphere. Water (10 mL) was added to the reaction solution, and ethyl acetate was used for extraction (20 mL × 3). The organic phases were combined, washed with saturated brine (20 mL), dried over anhydrous sodium sulfate and filtered, and the filtrate was concentrated under reduced pressure. The residue was purified by column chromatography (dichloromethane/methanol = 0-5%) to obtain compound 293-2. MS m/z(ESI):689.7[M+1]+;

2) Step 2: Wet palladium carbon (20 mg, 10%) was added to a solution of compound 293-2 (120.0 mg, 170 μmol) in ethyl acetate (2 mL) and ethanol (2 mL) at room temperature, the reaction was subjected to hydrogen replacement three times, and then reacted at 25°C for 16 h. Filtration was performed, and then the filtrate was concentrated under reduced pressure. The residue was purified by silica gel column chromatography (dichloromethane/methanol = 100/1 to 30/1) to obtain compound 293-3.

3) Step 3: Trifluoroacetic acid (1 mL) was added to a solution of compound 293-3 (80.0 mg, 160 μmol) in

dichloromethane (2 mL). The reaction was stirred at 25°C for 1 h. The reaction solution was concentrated under reduced pressure to obtain compound 293-4, which was used directly in the next step. MS m/z(ESI):411.7[M+1]+.

4) Step 4: Triethylamine (0.30 mL, 2.16 mmol) was added to a solution of compound 293-4 (50 mg, 120 μmol) in tetrahydrofuran (2 mL) and *N,N*-dimethylformamide (1 mL), and the obtained mixture was stirred for 30 min. Then compound 287-3 (70.0 mg, 150 μmol) and acetic acid (0.5 mL) were added, and the obtained mixture was further stirred for 30 min. Finally, sodium borohydride acetate (78.0 mg, 370 μmol) was added, and the obtained mixture was stirred at 25°C for 2 h, then concentrated under reduced pressure. The residue was purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min) to obtain compound 293-5. MS m/z(ESI):1020.9[M+1]+.

5) Step 5: Compound 293-5 (20 mg, 20 μmol) was added to a 50 mL single-neck bottle at room temperature, and hydrogen chloride/dioxane solution (2 mL, 4 M) was added and the obtained mixture was stirred. The reaction solution was stirred at room temperature for 1.5 h, concentrated under reduced pressure and then purified by high performance liquid chromatography (A: 0.1% FA/$H_2O$ B: ACN, chromatographic column: Waters-SunFire-C18-10μm-19×250 mm; mobile phase: water (containing 0.1% formic acid) and acetonitrile, gradient ratio: acetonitrile 32%-48%, flow rate: 25 mL/min), to obtain compound 293. MS m/z(ESI):906.4[M+1]+. $^1$H NMR (400 MHz, DMSO-$d_6$): δ 10.89 (s, 1H), 8.69 - 8.63 (m, 2H), 8.50 (s, 1H), 8.34 (s, 1H), 7.40 - 7.36 (m, 1H), 7.20 (t, *J* = 53.2 Hz, 1H), 7.03 - 7.00 (m, 2H), 6.78 (d, *J* = 8.0 Hz, 1H), 4.92 - 4.84 (m, 1H), 4.36 - 4.28 (m, 2H), 4.25 (s, 3H), 4.13 - 3.96 (m, 2H), 3.64 - 3.54 (m, 1H), 3.28 - 3.15 (m, 4H), 2.75 - 2.57 (m, 8H), 2.44 (s, 4H), 2.36 - 2.26 (m, 2H), 2.21 - 2.15 (m, 5H), 1.99 - 1.91 (m, 5H), 1.86 - 1.60 (m, 8H), 1.54 - 1.44 (m, 2H).

Example 294: 3-(4-(4-((R)-4-(((1R,4R)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl)cyclohexyl)methyl)-2-methylpiperazin-1-yl)piperidin-1-yl)-3-methyl-2-oxo-2,3-dihydro-1H-benzo[d]imidazol-1-yl)piperidine-2,6-dione (294)

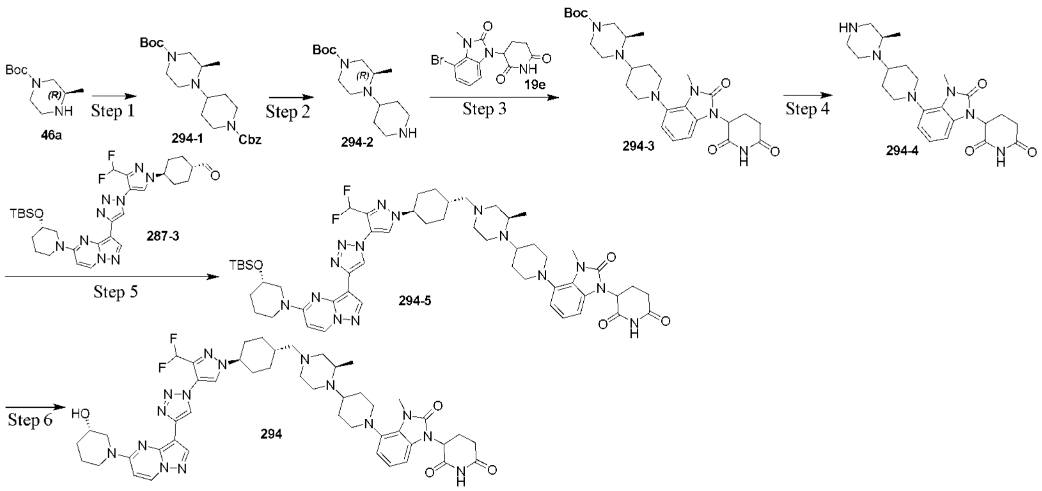


1) Step 1: Compound 1-Cbz-4-piperidone (5.00 g, 21.4 mmol), compound 46a (5.15 g, 25.7 mmol), anhydrous methanol (100 mL), and acetic acid (1.21 mL, 21.4 mmol) were added to a single-neck bottle and stirred until dissolved. Sodium cyanoborohydride (2.69 g, 42.8 mmol) was added. The reaction solution was stirred at 45°C for 18 h, adjusted to about pH 7 by dropwise adding sodium hydroxide aqueous solution (1 M) to the reaction solution, and extracted with ethyl acetate (200 mL×). The organic phases were combined, washed with saturated brine (200 mL), dried over anhydrous sodium sulfate and filtered, and the filtrate was evaporated to dryness under reduced pressure. The residue was purified by reverse phase chromatography (acetonitrile/formic acid aqueous solution (0.1%) = 0-100%) to obtain compound 294-1. MS m/z(ESI):418.4 [M+1]+.

2) Step 2: Compound 294-1 (1.50 g, 3.59 mmol) and anhydrous methanol (30 mL) were added to a single-neck bottle and stirred until dissolved. Wet palladium carbon (150 mg, 10%) was added, and the reaction system was subjected to hydrogen replacement three times and stirred at room temperature for 18 h under hydrogen atmosphere (15 psi). The reaction solution was filtered through diatomaceous earth, and the filter cake was washed with methanol (10 mL × 3). The filtrates were combined and evaporated to dryness under reduced pressure to obtain compound 294-2. MS m/z(ESI):284.2 [M+1]+.

3) Step 3: Compound 294-2 (1.02 g, 3.59 mmol), compound 19e (950 mg, 2.81 mmol, prepared by the method disclosed for step 3 product on page 88 of the specification of the patent application "WO 2022012623 A"), 2-dicyclohexylphosphino-2',6'-diisopropoxy-1,1'-biphenyl (0.26 g, 0.56 mmol), (2-dicyclohexylphosphino-2',6'-diisopropoxy-1,1'-biphenyl)(2-amino-1,1'-biphenyl-2-yl) palladium (II) methanesulfonate (0.47 g, 0.56 mmol) and anhydrous toluene (20 mL) were added to a three-neck bottle. The reaction system was subjected to nitrogen replacement three times, and lithium bis(trimethylsilyl)amide (14.1 mL, 14.1 mmol, 1 M solution in tetrahydrofuran) was added. The reaction solution was reacted at 80°C for 2 h. After the reaction was completed, the reaction solution was poured into water (100 mL) and ethyl acetate (100 mL) was used for extraction three times. The organic phases were combined, washed with saturated brine (100 mL), dried over anhydrous sodium sulfate, filtered, and evaporated to dryness under reduced pressure. The residue was separated by silica gel column chromatography (dichloromethane/methanol = 100/1 to 20/1) to obtain compound 294-3. MS m/z(ESI):541.4 $[M+1]^+$.

4) Step 4: Trifluoroacetic acid (1 mL) was added to a solution containing compound 294-3 (100 mg, 185 μmol) and dichloromethane (2 mL), and the reaction was stirred at 25°C for 1 h. The reaction solution was concentrated under reduced pressure to obtain compound 294-4, which was directly used in the next step. MS m/z(ESI):441.3 $[M+1]^+$.

5) Step 5: Compound 294-4 (63.4 mg, 144 μmol) was dissolved in *N,N*-dimethylformamide (1.5 mL) and tetrahydrofuran (6 mL) at room temperature, triethylamine (14.6 mg, 144 μmol) was added, and the mixture was stirred for 15 min. Sodium borohydride acetate (153 mg, 720 μmol), compound 287-3 (90.0 mg, 144 μmol) and acetic acid (17.3 mg, 288 μmol) were then added to the mixture, and the resulting mixture was stirred at 25°C for 30 min. The reaction solution was poured into sodium bicarbonate aqueous solution (20 mL), and a large amount of solid precipitated and was filtered. The filter cake was washed with petroleum ether/ethyl acetate (1/1, 5 mL). The solid was collected and dried in vacuo to obtain compound 294-5. MS m/z(ESI): 526.3$[M/2+1]^+$.

6) Step 6: Compound 294-5 (90 mg, 85.7 μmol) was dissolved in dichloromethane (2 mL) solution, and hydrogen chloride in dioxane (4 mL, 4 M) was added, and the obtained mixture was reacted under stirring at room temperature for 1 h. The reaction system was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 16%-95%, flow rate: 25 mL/min) to obtain compound 294. MS m/z(ESI): 936.8 $[M+1]^+$. $^1$H NMR (400 MHz, DMSO-$d_6$) δ 11.09 (s, 1H), 8.67 - 8.64 (m, 2H), 8.49 (s, 1H), 8.33 (s, 1H), 7.19 (t, *J* = 53.2 Hz, 1H), 6.99 - 6.94 (m, 1H), 6.92 - 6.84 (m, 2H), 6.77 (d, *J* = 8.0 Hz, 1H), 5.38 - 5.31 (m, 1H), 4.33 - 4.25 (m, 1H), 4.11 - 3.97 (m, 2H), 3.62 (s, 3H), 3.60 - 3.52 (m, 2H), 3.24 - 3.10 (m, 5H), 2.93 - 2.59 (m, 10H), 2.17 - 2.08 (m, 5H), 1.98 - 1.76 (m, 10H), 1.70 - 1.56 (m, 3H), 1.52 - 1.42 (m, 2H), 1.13 - 1.04 (m, 2H), 1.04 - 1.01 (m, 3H).

Example 295: 3-(4-((S)-2-(((1R,4S)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl)cyclohexyl)methyl)-3-methyl-2,8-diazaspiro[4.5]dec-8-yl)-3-methyl-2-oxo-2,3-dihydro-1H-benzo[d]imidazol-1-yl)piperidine-2,6-dione (295)

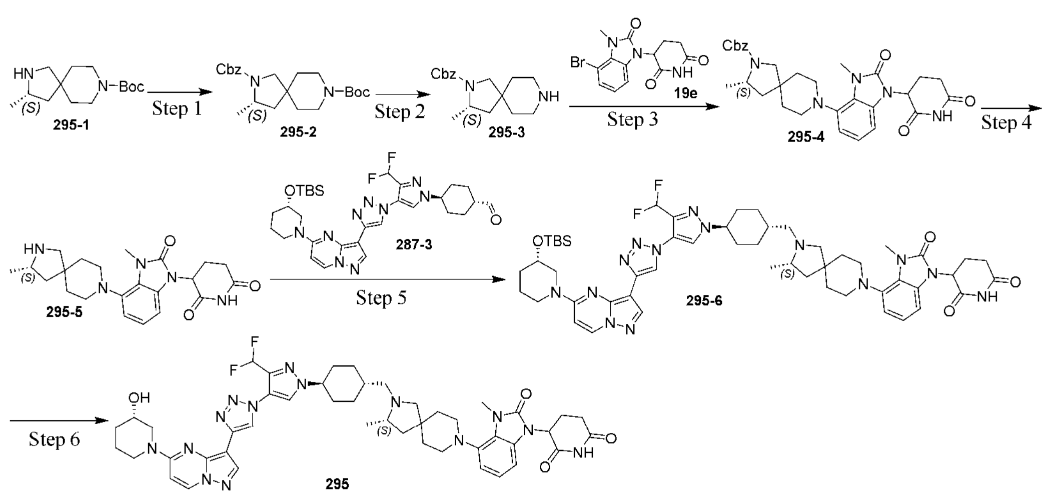


1) Step 1: Compound 295-1 (3.30 g, 13.0 mmol, prepared by the method disclosed for step R-3 product on page 225 of the specification of "WO 2021055756 A1") was dissolved in a mixed solution of water (15 mL) and acetonitrile (15 mL), sodium carbonate (4.13 g, 38.9 mmol) was added, and the obtained mixture was cooled to 0°C. Benzyl chloroformate

(3.69 mL, 26.0 mmol) was added dropwise, and the obtained mixture was reacted under stirring at room temperature for 5 h. The reaction solution was poured into water (50 mL), and the aqueous phase was extracted with ethyl acetate (30 mL × 3). The combined organic phases were washed with brine (50 mL), dried over anhydrous sodium sulfate and filtered, and the filtrate was concentrated under reduced pressure. The residue was purified on silica gel plate for thin layer chromatography (dichloromethane/methanol = 100/1 to 20/1) to obtain compound 295-2.

2) Step 2: Compound 295-2 (1.10 g, 2.83 mmol) was dissolved in dichloromethane (5 mL), hydrogen chloride/dioxane (5 mL, 4 M) was added, and the obtained mixture was reacted under stirring at room temperature for 1 h. Then the reaction solution was directly concentrated under reduced pressure to obtain compound 295-3, which was used directly in the next step. MS m/z(ESI):289.4[M+1]$^+$.

3) Step 3: Compound 19e (300 mg, 887 μmol), compound 295-3 (512 mg, 1.77 mmol), 2-dicyclohexylphosphino-2',6'-diisopropoxy-1,1'-biphenyl (41.4 mg, 88.7 μmol) and (2-dicyclohexylphosphino-2',6'-diisopropoxy-1,1'-biphenyl)(2-amino-1,1'-biphenyl-2-yl)palladium (II) methanesulfonate (74.2 mg, 88.7 μmol) were dissolved in toluene (15 mL). Lithium bis(trimethylsilyl)amide (4.44 mL, 4.44 mmol, 1.0 M solution in tetrahydrofuran, Energy Chemical) was added dropwise under nitrogen protection. The reaction solution was stirred at 80°C for 2 h. The reaction solution was cooled to room temperature, water (40 mL) was added, and ethyl acetate (30 mL × 3) was used for extraction. The combined organic phase was washed with saturated brine (15 mL), dried over anhydrous sodium sulfate and filtered, and the filtrate was concentrated under reduced pressure. The residue was purified on silica gel plate for thin layer chromatography (dichloromethane/methanol = 20/1) to obtain compound 295-4. MS m/z(ESI):546.4[M+1]$^+$.

4) Step 4: Compound 295-4 (60 mg, 110 μmol) was dissolved in methanol (5 mL), palladium carbon (10%, 60 mg) was added, and the obtained mixture was subjected to hydrogen replacement three times, and stirred for 3 h under hydrogen atmosphere (1 atm). The reaction system was filtered, and the filtrate was concentrated to obtain compound 295-5, which was directly used as the raw material for the next step. MS m/z(ESI):412.4[M+1]$^+$.

5) Step 5: Compound 295-5 (32.9 mg, 79.9 μmol) was dissolved in *N,N*-dimethylformamide (1 mL) and tetrahydrofuran (4 mL) at room temperature, triethylamine (8.08 mg, 79.9 μmol) was added, and the obtained mixture was stirred for 15 min. Then sodium borohydride acetate (84.3 mg, 400 μmol), compound 287-3 (50.0 mg, 79.9 μmol) and acetic acid (9.60 mg, 160 μmol) were added to the mixture, and the mixture was stirred at 25°C for 30 min. The reaction solution was poured into saturated sodium bicarbonate aqueous solution (20 mL), and a large amount of solid precipitated and was filtered. The filter cake was washed with petroleum ether/ethyl acetate (1/1, 5 mL) and filtered. The filter cake was collected and dried in vacuo to obtain compound 295-6 (60 mg), which was directly used as the raw material for the next step.

6) Step 6: Compound 295-6 (60 mg, 58.7 μmol) was dissolved in dichloromethane (3 mL) solution, and hydrogen chloride/dioxane (3 mL, 4 M) was added, and the obtained mixture was reacted under stirring at room temperature for 1 h. The reaction system was concentrated to obtain a residue. The reaction solution was concentrated under reduced pressure and then purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 16%-95%, flow rate: 25 mL/min) to obtain compound 295. MS m/z(ESI): 907.8[M+1]$^+$. $^1$H NMR (400 MHz, DMSO-$d_6$) δ 11.08 (s, 1H), 8.68 - 8.63 (m, 2H), 8.49 (s, 1H), 8.33 (s, 1H), 7.43 - 7.19 (m, 1H), 6.99 - 6.91 (m, 2H), 6.90 - 6.83 (m, 1H), 6.78 - 6.74 (m, 1H), 5.42 - 5.26 (m, 1H), 4.90 - 4.84 (m, 1H), 4.41 - 4.17 (m, 1H), 4.16 - 3.93 (m, 2H), 3.65 - 3.57 (m, 4H), 3.45 (s, 1H), 3.24 - 3.15 (m, 2H), 2.99 - 2.91 (m, 2H), 2.78 - 2.56 (m, 3H), 2.44 - 2.31 (m, 3H), 2.20 - 2.08 (m, 4H), 1.97 - 1.63 (m, 13H), 1.53 - 1.43 (m, 3H), 1.29 - 1.14 (m, 2H), 1.08 - 0.98 (m, 4H).

Example 296: 3-(4-((R)-2-(((1r,4R)-4-(3-(difluoromethyl)-4-(4-(5-((S)-3-hydroxypiperidin-1-yl)) pyrazolo[1,5-a]pyrimidin-3-yl)-1H-1,2,3-triazol-1-yl)-1H-pyrazol-1-yl)cyclohexyl)methyl)-3-methyl-2,8 -diazaspiro[4.5]dec-8-yl)-3-methyl-2-oxo-2,3-dihydro-1H-benzo[d]imidazol-1-yl)piperidine-2,6-dione (296)

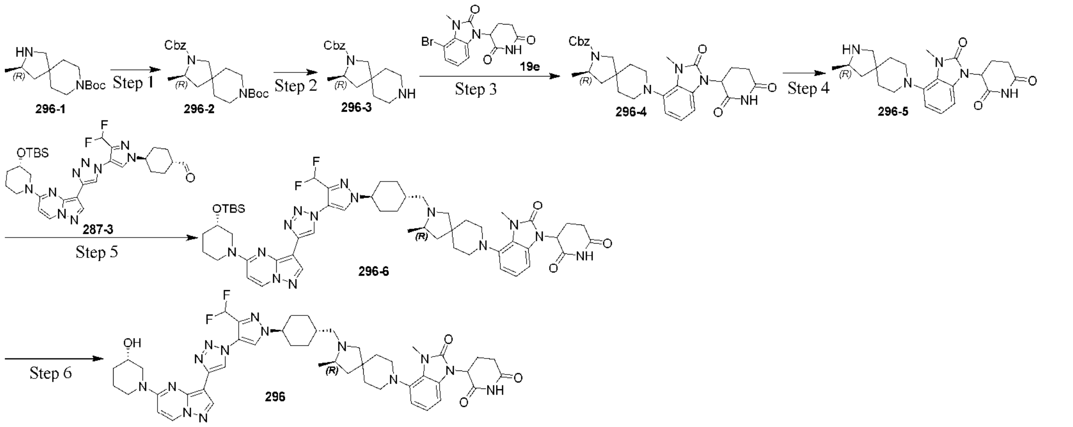


1) Step 1: Compound 296-1 (2.27 g, 8.92 mmol, using the synthesis method for compound R-3 on page 225 of the specification of the patent application "WO 2021055756 A1"), anhydrous acetonitrile (20 mL) and water (20 mL) were added to a single-neck bottle and stirred until dissolved, sodium carbonate (2.84 g, 26.8 mmol) and benzyl chloroformate (2.54 mL, 17.9 mmol) were added, and the reaction solution was stirred at room temperature for 18 h. Then, the reaction solution was extracted with ethyl acetate (30 mL × 3). The organic phases were combined, washed with saturated brine (30 mL), dried over anhydrous sodium sulfate and filtered, and the filtrate was evaporated to dryness under reduced pressure. The residue was separated by a reverse phase column (acetonitrile/0.1% formic acid aqueous solution = 0-100%) to obtain compound 296-2. MS m/z(ESI):333.2[M-55]$^+$.

2) Step 2: Compound 296-2 (2.05 g, 5.28 mmol) was dissolved in hydrogen chloride/dioxane solution (4.0 M, 20 mL) and stirred at 25°C for 1 h. The reaction mixture was concentrated under reduced pressure to obtain compound 296-3. MS m/z(ESI):289.6[M+1]$^+$.

3) Step 3: Compound 19e (1.20 g, 3.55 mmol), compound 296-3 (1.54 g, 5.32 mmol), 2-dicyclohexylphosphino-2',6'-diisopropoxy-1,1'-biphenyl (170 mg, 0.350 mmol, Bide Pharmatech Ltd.) and (2-dicyclohexylphosphino-2',6'-diisopropoxy-1,1'-biphenyl)(2-amino-1,1'-biphenyl-2-yl) palladium (II) methanesulfonate (300 mg, 0.350 mmol, Bide Pharmatech Ltd.) were dissolved in toluene (20 mL). The reaction solution was subjected to nitrogen replacement three times at 25°C, and lithium bis(trimethylsilyl)amide (17.7 mL, 17.7 mmol, 1.0 M solution in tetrahydrofuran, Bide Pharmatech Ltd.) was added under nitrogen atmosphere. The reaction solution was heated to 80°C and reacted under stirring for 2 h. The reaction solution was poured into cooled saturated ammonium chloride aqueous solution (50 mL) and diluted with dichloromethane (100 mL). The aqueous phase was subjected to liquid separation and extracted with dichloromethane (50 mL × 3). The combined organic phase was washed with saturated brine (50 mL), dried over anhydrous sodium sulfate and filtered to remove the desiccant, and then the filtrate was concentrated under reduced pressure. The residue was purified by silica gel column chromatography (dichloromethane/methanol = 100/1 to 50/1) to obtain compound 296-4. MS m/z(ESI):546.4[M+1]$^+$. $^1$H NMR (400 MHz, DMSO-$d_6$): δ 11.10 - 11.07 (m, 1H), 7.43 - 7.23 (m, 4H), 7.20 - 6.84 (m, 4H), 5.38 - 5.31 (m, 1H), 5.09 - 5.02 (m, 1H), 3.86 (s, 1H), 3.62 (s, 2H), 3.32 (s, 4H), 3.20 - 2.80 (m, 4H), 2.74 - 2.58 (m, 3H), 2.49 - 2.31 (m, 2H), 2.06 - 1.89 (m, 2H), 1.68 - 1.42 (m, 3H), 1.24 (s, 3H).

4) Step 4: Compound 296-4 (860 mg, 1.58 mmol) and wet palladium carbon (168 mg, 10%) were mixed with methanol (15 mL), and the mixture was subjected to hydrogen replacement three times at 25°C and stirred for 3 h under hydrogen atmosphere. The reaction solution was filtered through diatomaceous earth and the filtrate was concentrated under reduced pressure. The residue was purified by silica gel column chromatography (dichloromethane/ammonia in methanol (7.0 M) = 100/1 to 10/1) to obtain compound 296-5. MS m/z(ESI):412.3[M+1]$^+$. $^1$H NMR (400 MHz, $CDCl_3$) δ 7.02 - 6.96 (m, 1H), 6.93 - 6.88 (m, 1H), 6.56 (d, *J* = 7.2 Hz, 1H), 5.23 - 5.02 (m, 1H), 3.77 (s, 3H), 3.30 - 3.23 (m, 1H), 3.05 (s, 2H), 2.87 - 2.72 (m, 4H), 2.47 - 2.34 (m, 4H), 2.24 - 2.19 (m, 1H), 1.90 - 1.73 (m, 3H), 1.71 - 1.55 (m, 2H), 1.21 (s, 3H).

5) Step 5: Compound 296-5 (50.1 mg, 0.121 mmol) was dissolved in *N,N*-dimethylformamide (2 mL), and acetic acid (34.0 mg, 0.18 mmol) and sodium borohydride acetate (211.9 mg, 0.611 mmol, Bide Pharmatech Ltd.) were added. The reaction solution was stirred at 25°C for 5 min, and then compound 287-3 (83.6 mg, 0.131 mmol) was added. The reaction solution was stirred at 25°C for further 30 min. The reaction solution was concentrated under reduced pressure, and the obtained residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 296-6 (40.0 mg). MS m/z(ESI):511.7[M/2+1]$^+$.

6) Step 6: Compound 296-6 (40.0 mg, 0.039 mmol) was added to dioxane (2 mL) at room temperature, and hydrogen chloride/dioxane (2 mL, 4.0 M) was added, and the reaction solution was stirred for 1 h. The reaction solution was

concentrated under reduced pressure, and the obtained residue was purified by high performance liquid chromatography (Waters-2545, chromatographic column: SharpSil-T C18, 30×150 mm, 5 μm; mobile phase: water (containing 10 mmol/L formic acid) and acetonitrile, gradient ratio: acetonitrile 38%-45%, flow rate: 30 mL/min) to obtain compound 296. MS m/z(ESI): 907.9[M+1]$^+$. $^1$H NMR (400 MHz, DMSO-$d_6$): δ 11.09 (s, 1H), 8.69 - 8.63 (m, 2H), 8.50 (s, 1H), 8.34 (s, 1H), 7.20 (t, *J* = 53.1 Hz, 1H), 7.01 - 6.91 (m, 2H), 6.90 - 6.84 (m, 1H), 6.78 (d, *J* = 8.0 Hz, 1H), 5.41 - 5.30 (m, 1H), 4.92 - 4.83 (m, 1H), 4.38 - 4.25 (m, 1H), 4.15 - 3.96 (m, 2H), 3.68 - 3.45 (m, 6H), 3.25 - 3.19 (m, 2H), 3.03 - 2.85 (m, 4H), 2.77 - 2.61 (m, 3H), 2.46 - 2.37 (m, 2H), 2.19 - 2.11 (m, 3H), 2.04 - 1.94 (m, 3H), 1.89 - 1.66 (m, 9H), 1.54 - 1.44 (m, 3H), 1.32 - 1.24 (m, 1H), 1.22 - 1.11 (m, 1H), 1.07 - 0.95 (m, 4H).

Example 297: 3-(4-((3S)-4-(4-(((1R,4S)-4-(3-(difluoromethyl)-4-(4-(5-((S)-3-hydroxypiperidin-1-yl)pyrazolo[1,5-a]pyrimidin-3-yl)-1H-1,2,3-triazol-1-yl)-1H-pyrazol-1-yl)cyclohexyl)methyl)piperazin-1-yl)-3-fluoropiperidin-1-yl)-3-methyl-2-oxo-2,3-dihydro-1H-benzo[d]imidazol-1-yl)piperidine-2,6-dione (297)

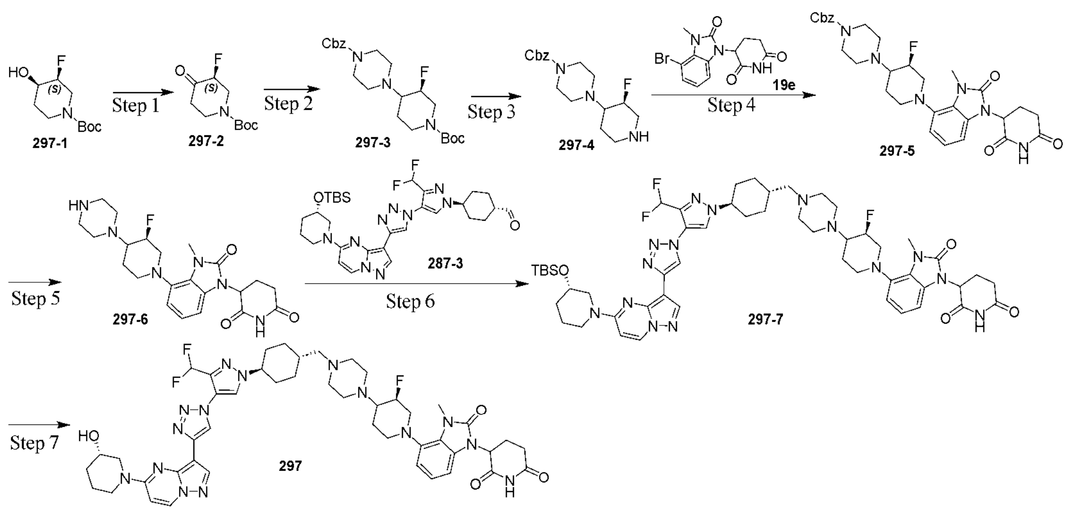


1) Step 1: Compound 297-1 (3.00 g, 13.7 mmol, Energy Chemical) was dissolved in dichloromethane (35 mL), and the obtained mixture was cooled to 0°C. Dess-Martin oxidant (8.70 g, 20.5 mmol) was added, and the obtained mixture was reacted under stirring at room temperature for 16 h. Then the reaction system was poured into sodium bicarbonate aqueous solution (100 mL), and dichloromethane (50 mL × 3) was used for extraction. The organic phases were combined, dried over anhydrous sodium sulfate and filtered, and the filtrate was concentrated under reduced pressure. The residue was purified by silica gel column chromatography (petroleum ether/ethyl acetate = 100/1 to 4/1) to obtain compound 297-2. $^1$H NMR (400 MHz, DMSO-$d_6$): δ 5.18 - 5.02 (m, 1H), 4.36 - 4.25 (m, 1H), 4.06 - 3.98 (m, 1H), 3.30 - 3.18 (m, 2H), 2.60 - 2.54 (m, 1H), 2.40 - 2.35 (m, 3H), 1.45 (s, 9H).
2) Step 2: Compound 297-2 (2.00 g, 9.21 mmol) was dissolved in 1,2-dichloroethane (40 mL), benzyl-1-piperazine carbonate (4.06 g, 18.4 mmol) and acetic acid (1.11 g, 18.4 mmol) were added, and the obtained mixture was reacted under stirring at room temperature for 1 h. Sodium triacetoxyborohydride (3.90 g, 18.4 mmol) was added, and the obtained mixture was reacted under stirring at room temperature for 16 h. The reaction system was poured into sodium bicarbonate aqueous solution (100 mL), and dichloromethane (50 mL × 3) was used for extraction. The organic phases were combined, dried over anhydrous sodium sulfate and filtered, and the filtrate was concentrated under reduced pressure. The residue was purified by silica gel column chromatography with an eluent system (petroleum ether/ethyl acetate = 100/1 to 1/1) to obtain compound 297-3. MS m/z(ESI):422.7[M+1]$^+$.
3) Step 3: Compound 297-3 (1.60 g, 3.80 mmol) was dissolved in dichloromethane (10 mL), hydrogen chloride/dioxane (4 M, 10 mL) was added, and the obtained mixture was reacted under stirring at room temperature for 1 h. Then the reaction solution was directly concentrated under reduced pressure to obtain compound 297-4, which was directly used in the next step. MS m/z(ESI):322.6[M+1]$^+$.
4) Step 4: Compound 19e (300 mg, 887 μmol), compound 297-4 (570 mg, 1.77 mmol), 2-dicyclohexylphosphino-2',6'-diisopropoxy-1,1'-biphenyl (41.4 mg, 88.7 μmol) and (2-dicyclohexylphosphino-2',6'-diisopropoxy-1,1'-biphenyl)(2-amino-1,1'-biphenyl-2-yl)palladium (II) methanesulfonate (74.2 mg, 88.7 μmol) were dissolved in toluene (15 mL). Lithium bis(trimethylsilyl)amide (4.44 mL, 4.44 mmol, 1.0 M solution in tetrahydrofuran, Energy Chemical) was added dropwise under nitrogen protection. The reaction solution was stirred at 80°C for 2 h. The reaction solution was cooled to room temperature, water (40 mL) was added, and the aqueous phase was extracted with ethyl acetate (30 mL × 3).

The combined organic phase was washed with brine (15 mL), dried over anhydrous sodium sulfate and filtered, and the filtrate was concentrated under reduced pressure. The residue was purified by silica gel column chromatography (dichloromethane/methanol = 100/1 to 20/1) to obtain compound 297-5. MS m/z(ESI):579.4[M+1]$^+$.

5) Step 5: Compound 297-5 (100 mg, 173 μmol) was dissolved in methanol (10 mL), palladium carbon (10%, 100 mg) was added, and the obtained mixture was subjected to hydrogen replacement three times and stirred for 18 h under hydrogen atmosphere (1 atm). The reaction system was filtered and the filtrate was concentrated to obtain compound 297-6, the residue was directly used as the raw material for the next step without purification. MS m/z(ESI):445.5 [M+1]$^+$.

6) Step 6: Compound 296-6 (28.4 mg, 63.9 μmol) was dissolved in *N,N*-dimethylformamide (1 mL) and tetrahydrofuran (4 mL) at room temperature, sodium borohydride acetate (67.4 mg, 320 μmol), compound 287-3 (40.0 mg, 63.9 μmol) and acetic acid (7.68 mg, 128 μmol) were added, and the mixture was stirred at 25°C for 30 min. The reaction solution was poured into sodium bicarbonate aqueous solution (20 mL), and ethyl acetate was used for extraction (20 mL × 3). The organic phases were combined, dried over anhydrous sodium sulfate and filtered, and the filtrate was concentrated under reduced pressure to obtain compound 297-7, which was directly used as the raw material for the next step. MS m/z(ESI): 528.3[M/2+1]$^+$,

7) Step 7: Compound 297-7 (40 mg, 37.9 μmol) was dissolved in dichloromethane (3 mL) solution, and hydrogen chloride/dioxane (3 mL, 4 M) was added, and the obtained mixture was reacted under stirring at room temperature for 1 h. The reaction system was concentrated and the residue was purified by high performance liquid chromatography (Gilson_306_1741, chromatographic column: Waters-SunFire-C18-10μm-19×250 mm; mobile phase: water (containing 26.5 mmol/L formic acid) and acetonitrile, gradient ratio: acetonitrile 16%-95%, flow rate: 25 mL/min) to obtain compound 297. MS m/z(ESI): 940.8[M+1]$^+$.

Other compounds of the present disclosure can be prepared by methods similar to those described in the above examples (with appropriate modifications, if necessary).

**Biological test:**

**Test example 1: Hibit fluorescence detection of proteolysis**

Utilizing HiBiT tag technology, the HiBiT tag-containing sequence (Seq. ID. No. 1, Beijing Tsingke Biotech Co., Ltd.) was connected to the IRAK4 plasmid (Seq. ID. No. 2, Beijing Tsingke Biotech Co., Ltd.), and the plasmid was transfected into HEK293T cells (ATCC). The degradation level of IRAK4 by compounds was characterized by detecting HiBiT fluorescence levels. 300,000 HEK293T cells in 1 mL of DMEM medium (Gbico, 11965-092) were inoculated into a 6-well plate (Corning 3516) and the plate was placed in a constant temperature incubator at 37°C, 5% $CO_2$ overnight. 3 μL of X-tremeGENE™ 9 DNA Transfection Reagent (Roche #06365787001) and 1 μg of IRAK4 plasmid were added to 200 μL Opti-MEM (Gibico 31985062) and mixed uniformly gently. The 6-well plate that was incubated overnight was taken out. After the plate was left at room temperature for 15 min, the obtained mixture was added dropwise to the 6-well plate. 6 h after transient transfection, cells were digested with trypsin (Gibco, 25200-056) for one minute and centrifuged at 1000 rpm for 3 min. 5000 HEK293T cells in 40 μL DMEM medium were inoculated into a 384-well plate (Corning 3765) and the plate was placed in a constant temperature incubator at 37°C and 5% $CO_2$ overnight. Then 40 nL of compound was added to the 384-well plate using Echo 655 Liquid Handler, with the highest final concentration of 10 μM, 1:3 serial dilutions, and a total of 10 concentrations. Culture was performed at 37°C and 5% $CO_2$ for 24 h. The 384-well plate was taken out and equilibrated to room temperature. 40 μL of Nano-Glo® HiBiT Lytic Detection System (Promega N3030) was added to each well and incubated at room temperature for 15 min. The fluorescence value was recorded using EnVision Xcite Multilabel Reader (PerkinElmer 2105-0020). Prism 9.3 software was used to perform fitting analysis for data, and non-linear fitting (non-liner inhibitor (Bell-shaped)) and four parameters (4 parameter) were used, and the parameters was as follows:

$$\text{Span1=Plateau1-Dip}$$

$$\text{Span2=Plateau2-Dip}$$

$$\text{Section1=Span1/(1+10^((LogEC50_1-X)*nH1))}$$

$$\text{Section2=Span2/(1+10^((X-LogEC50_2)*nH2))}$$

The equation was as follows: Y=Dip+Section1+Section2.

Seq. ID. No. 1 (gtgagcggctggcggctgttcaagaagattagc), other DNA, synthetic construct, sequence length 33; Seq. ID. No. 2

(gccaccatgaacaaacccataacaccatcaacatatgtgcgctgcctcaatgttggactaattaggaagctgtcagatttattgatcctcaagaaggatggaagaagttagctgtagctattaaaaaaccatctggtgatgatagatacaatcagtttcacataaggagatttgaagcattacttcaaactggaaaaagtcccacttctgaattactgtttgactggggcaccacaaattgcacagttggtgatcttgtggatcttttgatccaaaatgaatttttttgctcctgcgagtcttttgctcccagatgctgttcccaaaactgctaatacactaccttctaaagaagctataacagttcagcaaaaacagatgcctttctgtgacaaagacaggacattgatgacacctgtgcagaatcttgaacaaagctatatgccacctgactcctcaagtccagaaaataaaagtttagaagttagtgatacacgttttcacagtttttcattttatgaattgaagaatgtcacaaataactttgatgaacgacccatttctgttggtggtaataaaatgggagagggaggatttggagttgtatataaaggctacgtaaataacacaactgtggcagtgaagaagcttgcagcaatggttgacattactactgaagaactgaaacagcagtttgatcaagaaataaaagtaatggcaaagtgtcaacatgaaaacttagtagaactacttggtttctcaagtgatggagatgacctctgcttagtatatgtttacatgcctaatggttcattgctagacagactctcttgcttggatggtactccaccactttcttggcacatgagatgcaagattgctcagggtgcagctaatggcatcaattttctacatgaaaatcatcatattcatagagatattaaaagtgcaaatatcttactggatgaagcttttactgctaaaatatctgactttggccttgcacgggcttctgagaagtttgcccagacagtcatgactagcagaattgtgggaacaacagcttatatggcaccagaagctttgcgtggagaaataacacccaaatctgatatttacagctttggtgtggttttactagaaataataactggacttccagctgtggatgaacaccgtgaacctcagttattgctagatattaaagaagaaattgaagatgaagaaaagacaattgaagattatattgataaaaagatgaatgatgctgattccacttcagttgaagctatgtactctgttgctagtcaatgtctgcatgaaaagaaaaataagagaccagacattaagaaggttcaacagctgctgcaagagatgacagcttctgtgagcggctggcggctgttcaagaagattagctaa), other DNA, synthetic construct, sequence length 1422.

Partial Hitbit test results are as shown in Table 2.

Table 2

| Compound | DC50 (nM) | Dmax | Compound | DC50 (nM) | Dmax |
|---|---|---|---|---|---|
| 1 | A | D | 11 | A | A |
| 2 | A | D | 12 | A | B |
| 3 | A | C | 13 | A | A |
| 4 | A | A | 14 | B | D |
| 5 | A | D | 15 | C | D |
| 6 | A | D | 16 | A | C |
| 7 | A | C | 17 | C | B |
| 8 | D | D | 18 | D | A |
| 9 | A | D | 19 | B | B |
| 10 | A | A | 20 | A | A |
| 21 | A | D | 31 | D | B |
| 22 | D | D | 32 | A | B |
| 23 | A | A | 33 | A | C |
| 24 | A | D | 34 | A | A |
| 25 | A | D | 35 | A | A |
| 26 | A | B | 36 | A | A |
| 27 | A | C | 37 | A | A |
| 28 | D | C | 38 | A | D |

(continued)

| Compound | DC50 (nM) | Dmax | Compound | DC50 (nM) | Dmax |
|---|---|---|---|---|---|
| 29 | D | B | 39 | A | A |
| 30 | A | A | 40 | A | A |
| 41 | C | B | 51 | A | A |
| 42 | A | D | 52 | B | B |
| 43 | A | A | 53 | B | B |
| 44 | A | A | 54 | A | A |
| 45 | A | B | 55 | A | A |
| 46 | A | B | 56 | A | B |
| 47 | A | B | 57 | A | B |
| 48 | D | C | 58 | A | B |
| 49 | C | B | 59 | A | B |
| 50 | B | C | 60 | C | A |
| 61 | A | A | 71 | A | A |
| 62 | A | A | 72 | A | C |
| 63 | A | C | 73 | A | D |
| 64 | A | A | 74 | A | B |
| 65 | A | C | 75 | B | A |
| 66 | A | B | 76 | A | D |
| 67 | A | A | 77 | B | B |
| 68 | A | A | 78 | C | B |
| 69 | D | A | 79 | B | B |
| 70 | C | B | 80 | A | A |
| 81 | A | A | 91 | A | A |
| 82 | A | A | 92 | C | A |
| 83 | A | B | 93 | C | B |
| 84 | A | A | 94 | A | D |
| 85 | A | A | 95 | D | D |
| 86 | C | A | 96 | A | A |
| 87 | B | B | 97 | A | A |
| 88 | A | B | 98 | A | A |
| 89 | B | A | 99 | A | A |
| 90 | D | B | 100 | B | C |
| 101 | A | B | 111 | B | A |
| 102 | A | A | 112 | A | A |
| 103 | A | A | 113 | A | A |
| 104 | A | A | 114 | A | A |
| 105 | A | A | 115 | A | A |
| 106 | A | C | 116 | B | A |
| 107 | C | A | 117 | C | A |

(continued)

| Compound | DC50 (nM) | Dmax | Compound | DC50 (nM) | Dmax |
|---|---|---|---|---|---|
| 108 | A | A | 118 | A | A |
| 109 | B | A | 119 | B | A |
| 110 | A | A | 120 | B | A |
| 121 | A | A | 131 | B | A |
| 122 | A | A | 132 | B | A |
| 123 | A | A | 133 | A | A |
| 124 | A | A | 134 | A | A |
| 125 | A | A | 135 | A | A |
| 126 | A | A | 136 | A | A |
| 127 | A | A | 137 | A | A |
| 128 | A | A | 138 | A | A |
| 129 | A | A | 139 | A | A |
| 130 | B | A | 140 | A | A |
| 141 | A | A | 151 | A | A |
| 142 | A | A | 152 | A | A |
| 143 | A | A | 153 | A | A |
| 144 | A | A | 154 | A | A |
| 145 | A | A | 155 | A | A |
| 146 | A | A | 156 | A | A |
| 147 | A | A | 177 | A | A |
| 148 | A | A | 178 | A | A |
| 149 | A | A | 179 | A | A |
| 150 | A | A | 191 | B | D |
| 184 | A | C | 192 | C | C |
| 185 | B | D | 193 | B | B |
| 186 | A | B | 195 | B | A |
| 187 | A | A | 197 | C | C |
| 188 | A | A | 199 | C | C |
| 189 | B | C | 212 | A | A |
| 202 | B | D | 215 | A | B |
| 203 | A | A | 217 | A | D |
| 204 | A | B | 219 | A | D |
| 205 | A | B | 221 | D | |
| 206 | B | D | 223 | A | A |
| 207 | A | B | 224 | A | A |
| 208 | A | A | 225 | A | B |
| 209 | C | B | 226 | A | A |
| 227 | C | C | 231 | B | B |
| 228 | A | B | 232 | B | A |

(continued)

| Compound | DC50 (nM) | Dmax | Compound | DC50 (nM) | Dmax |
|---|---|---|---|---|---|
| 229 | A | C | 233 | B | B |
| 230 | B | C | 243 | A | A |
| 235 | A | A | 245 | A | A |
| 236 | A | A | 248 | A | A |
| 237 | A | B | 257 | A | B |
| 238 | A | A | 267 | A | A |
| 239 | A | A | 268 | B | B |
| 240 | A | A | 275 | C | A |
| 281 | A | A | 284 | A | B |
| 282 | A | A | 285 | A | A |
| Note: 1. DC50 (nm): A represents DC50 ≤ 0.5 nM, B represents 0.5 nM < DC50 ≤ 1 nM, C represents 1 nM < DC50 ≤ 5 nM, and D represents 5 nM < DC50;<br>2. Dmax (%): A represents Dmax ≥ 80%, B represents 70% ≤ Dmax < 80%, C represents 60% ≤ Dmax < 70%, and D represents Dmax < 60%. | | | | | |

The results of this test example showed that the compounds of the present application exhibited excellent target protein degradation effect in the IRAK4-Hibit experiment in HEK293T cells.

**Test example 2: WB detection of proteolysis**

The degradation level of IRAK4 in THP-1 cells was detected by Western Blot technology. 1,000,000 THP-1 cells (ATCC, catalog number: TIB-202) were inoculated in a 12-well plate (Corning 3513), with 1 mL per well. 1000X gradient dilution stock of the compound was prepared, with the highest concentration set at 300 μM. The dilution was carried out using DMSO in a 1:3 dilution factor, yielding a total of 9 concentrations. 1000X gradient dilutions of the compound were added to a 12-well plate, with 1 μL per well. Incubation was performed under conditions of 37°C and 5% $CO_2$ for 24 h, and the cells were collected into 2 mL of centrifuge tubes and centrifuged at 5000 rpm for 5 min, and the supernatant was removed. 100 μL of RIPA lysis buffer (Beyotime, P0013C) was added and mixed uniformly, then the cells were lysed on ice for 10 min, centrifuged at 4°C at 14000 rpm for 10 min, and the supernatant was collected. Protein content was determined with BCA (Solarbio, PC0020-10*50T). 75 μL of the supernatant was taken, and 25 μL of NuPAGE LDS Sample Buffer (4X) (Thermo B0007) was added to each tube (5% β-mercaptoethanol was added before use) to prepare samples, and the samples were boiled in a metal bath at 100°C for 5 min. 20 uL was taken and loaded into 4-12% Bis-Tris gel (Invitrogen, WG1402BOX) to prepare 1x NuPAGE™ MOPS buffer (Invitrogen, NP0001), and the electrophoresis instrument was set to constant voltage 120 V for 1.5 h. 1x NuPAGE™ MOPS SDS electrophoresis buffer (20X) (Invitrogen, NP0001) was prepared, and the electrophoresis instrument was set to a constant current of 300 mA for 100 min, and the protein was transferred to a PVDF membrane (Millipore, IPVH00010). The PVDF membrane was blocked with blocking solution (Licor, 927-60001) at room temperature for 1 h, and the IRAK4 antibody (Abcam ab32511) was diluted at 1:1000, and the GAPDH (Abcam ab8245) was diluted at 1:3000, and then the antibody was incubated at 4°C overnight. The membrane was washed three times with 1x TBST (Elabscience E-BC-R335) the next day, each time for 5 min. Secondary antibodies IR Dye 800 CW Goat anti-rabbit (Licor #926-32211) and IRDye® 680RD Goat anti-Mouse (Licor #926-68070) were diluted at 1:10,000 and incubated for 1 h at room temperature in the dark. The membrane was washed three times with TBST, each time for 5 min. The Odyssey imaging system Li-cor Odyssey CLx was used to detect signals and analyze data. GraphPad (Prism 9.3) software was used to perform analysis for data, and non-linear fitting (non-liner inhibitor (Bell-shaped)) and four parameters (4 parameter) were used, and the parameters was as follows:

$$Span1=Plateau1-Dip$$

$$Span2=Plateau2-Dip$$

$$Section1=Span1/(1+10^((LogEC50_1-X)*nH1))$$

$$Section2=Span2/(1+10^((X-LogEC50_2)*nH2))$$

The equation was as follows: Y=Dip+Section1+Section2.

Partial WB test activity results are shown in Table 3.

Table 3

| Compound | DC50 (nM) | Dmax | Compound | DC50 (nM) | Dmax |
|---|---|---|---|---|---|
| 30 | A | C | 60 | B | A |
| 34 | A | B | 61 | A | B |
| 35 | A | B | 62 | A | A |
| 36 | A | B | 64 | C | B |
| 37 | A | B | 84 | A | C |
| 39 | A | A | 85 | A | A |
| 54 | A | B | 96 | A | A |
| 55 | A | c | 97 | A | A |
| 58 | A | A | 98 | A | A |
| 59 | A | A | 99 | B | C |
| 102 | B | C | 123 | A | C |
| 103 | A | C | 124 | A | B |
| 108 | A | A | 125 | A | A |
| 113 | C | B | 126 | A | A |
| 115 | A | A | 127 | B | A |
| 116 | B | B | 128 | A | A |
| 118 | B | A | 132 | A | B |
| 119 | B | A | 135 | A | A |
| 120 | A | A | 136 | A | A |
| 122 | A | A | 139 | A | A |
| 140 | B | A | 154 | A | A |
| 141 | A | A | 155 | A | A |
| 142 | A | A | 172 | A | A |
| 144 | A | A | 177 | A | B |
| 145 | A | A | 178 | A | A |
| 146 | A | A | 179 | A | A |
| 147 | B | A | 188 | A | A |
| 148 | B | A | 204 | A | B |
| 151 | A | A | 208 | A | A |
| 153 | A | A | 238 | B | B |
| 243 | A | A | 161 | C | C |
| 245 | A | A | 166 | A | A |
| 248 | A | A | 171 | A | A |
| 281 | A | A | 173 | A | A |

(continued)

| Compound | DC50 (nM) | Dmax | Compound | DC50 (nM) | Dmax |
|---|---|---|---|---|---|
| 282 | A | A | 174 | A | A |
| 175 | A | A | 176 | A | A |
| 247 | A | A | 291 | A | A |
| 285 | C | B | 292 | A | A |
| 286 | C | C | 293 | A | A |
| 287 | A | A | 294 | A | A |
| 288 | C | C | 295 | C | A |
| 289 | A | C | 296 | B | A |
| 290 | A | A | 297 | A | A |
| Note: 1. DC50 (nM): A represents DC50 ≤ 10 nM, B represents 10 nM < DC50 ≤ 30 nM, and C represents 30 nM < DC50;<br>2. Dmax (%): A represents Dmax ≥ 70%, B represents 60% ≤ Dmax < 70%, and C represents Dmax < 60%. | | | | | |

The results of this test example showed that the compounds of the present application exhibited excellent target protein degradation effect in the experiment of detecting IRAK4 degradation based on Western Blot technology in THP-1 cells.

The results showed that the compounds of the present disclosure had good IRAK4 proteolysis ability and had the efficacy of being a proteolysis targeting chimera of Kinase IRAK4.

**Test example 3: study on effects of compounds on proteolysis level of IRAK4 in mice in vivo**

Experimental objective: The effects of KT474 (positive reference compound) and Example 146 on the proteolysis level of IRAK4 in Balb/c mice in vivo were evaluated.

Experiment materials: Female Balb/c mice, 6-8 weeks old, 18-20 grams were used in the experiment and were provided by Vital River Laboratory Animal Technology Co., Ltd.

Experimental method: The experiment started after the experimental animals were acclimated to the animal facility environment for 3 days. The experimental animals were randomly divided into groups according to their body weights, involving: blank control group (25% HP-β-CD), KT474 10 mg/kg, KT474 30 mg/kg, KT474 100 mg/kg, Example 146 10 mg/kg, Example 146 30 mg/kg and Example 146 100 mg/kg. The blank control group was orally administered with a single dose of the vehicle (25% HP-β-CD), and the other groups were orally administered with a single dose of the corresponding compound. After 24 h, the animals were euthanized, and whole blood and spleen tissue were collected. The whole blood was temporarily placed on wet ice for subsequent PBMC extraction, and the spleen tissue was quickly placed in dry ice. PBMC extraction: 1.5 ml of red blood cell lysis buffer was added to the whole blood, and the obtained mixture was tapped with fingers for mixing, placed at room temperature in the dark for 5 min, and centrifuged at 400 g for 5 min, and the supernatant was discarded. The above steps were repeated twice. Then, 1.5 ml of DPBS was added and mixed uniformly. The mixture was centrifuged at 400 g for 5 min, and the obtained precipitate was PBMC. The PBMC was stored in a -80°C refrigerator for subsequent Western blot analysis. PBMC sample processing: The PBMC samples were placed on wet ice. 50 μL of lysis buffer was separately added to lyse the samples for 30 min, and the mixture was centrifuged at 12500 rpm for 15 min. The protein was quantified by the BCA method. 35 μL of the supernatant was taken and added to 18.8 μL of the mixture solution of reducing buffer and SDS buffer, and the obtained mixture was boiled at 100°C for 10 min. The loading amount for each well of Western Blot was 20 μg. Spleen tissue processing: The spleen tissue was placed on wet ice. 400 μL of lysis buffer was added, the spleen tissue was ground, then lysed for 30 min, and centrifuged at 12500 rpm for 15 min. The protein was quantified by BCA method. 20 μL of the supernatant was taken and added to 10.769 μL of the mixture solution of reducing buffer and SDS buffer, and the obtained mixture was boiled at 100°C for 10 min. The loading amount for each well of Western Blot was 20 μg. After membrane transfer and incubation with the IRAK4 antibody (Rb mAb to IRAK4, AB300077, Abcam) and secondary antibody (Anti-rabbit IgG Antibody, 7074s, CST), the IRAK4 protein signal was read using gel imager, and the grayscale analysis of the target protein was performed using ImageJ software. In this experiment, the proteolysis level of IRAK4 in PBMC and spleen was detected to examine the effect of the positive reference compound and the compound of the present disclosure on the proteolysis level of IRAK4 in Balb/c mice in vivo.

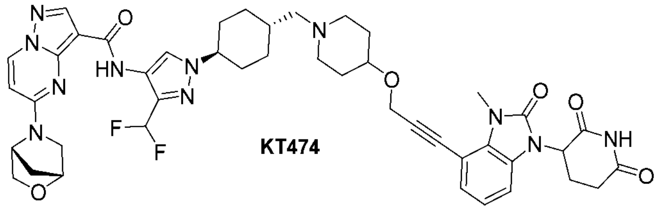

KT474

Experimental conclusion: This experiment tested the effect of KT474 (positive reference compound) and example 146 on the proteolysis level of IRAK4 in Balb/c mice in vivo, as shown in Figure 1 (proteolysis in spleen). The results after a single administration of the compound for 24 h showed that, compared with the blank control group, KT474 (positive reference compound) and example 146 were able to significantly inhibit the expression level of IRAK4 protein in the spleen and PBMC, and the proteolysis level of IRAK4 protein was dependent on the administration dosage of the compound. Among them, example 146 was significantly superior to KT474 (positive reference compound) in the proteolysis ability for spleen IRAK4 at three doses of 10, 30, and 100 mpk.

**Test example 4: in vitro evaluation of inhibitory effect on human liver microsome CYP450 enzyme**

(1) Test objective

In this experiment, the compound, liver microsomes and different CYP isoform enzyme substrates were incubated in a 37°C water bath, the amount of substrate metabolites generated was measured, and the inhibitory effect of the compound of the present disclosure on CYP enzyme was evaluated based on the IC50 value.

(2) Experimental method

i. The compound was diluted with DMSO (Aladdin, catalog number: D103273-500ml) to concentrations of 0, 0.02, 0.06, 0.2, 0.6, 2 and 6 mM. The positive reference inhibitors for each CYP isoform and their concentrations are shown in Table 4

Table 4 Positive reference inhibitors for CYP isoform enzymes and their concentrations

| CYP Isoform | Inhibitor | Work solution Concentration (μM) |
|---|---|---|
| CYP1A2 | α-naphthoflavone | 100, 30, 10,3, 1, 0.3, 0 |
| CYP2C9 | Sulfaphenazole | 1000, 300, 100, 30, 10, 3, 0 |
| CYP2C19 | S-(+)-N-3-benzyl-nirvanol | 1000, 300, 100, 30, 10, 3, 0 |
| CYP2D6 | Quinidine | 200, 60, 20,6, 2, 0.6, 0 |
| CYP 3A4 | Ketoconazole | 100, 30, 10,3, 1, 0.3, 0 |

ii. Phosphate buffer (sigma: V900050-500G) was used to formulate the substrate solution for each CYP isoform enzyme. The substrate and the concentration thereof for each CYP isoform enzyme are shown in Table 5.

Table 5 The substrate and the concentration thereof for CYP isoform enzyme

| CYP Isoform | Substrate | Conc. of stock solution (mM) |
|---|---|---|
| CYP1A2 | Phenacetin | 80 |
| CYP2C9 | Diclofenac Sodium | 16 |
| CYP2C19 | Mephenytoin | 40 |
| CYP2D6 | Dextromethorphan | 40 |
| CYP3A4-Testosterone | Testosterone | 8 |
| CYP3A4-Midazolam | Midazolam | 4 |

iii. Sample incubation. 1 μL of each concentration of the compound and positive reference inhibitor for CYP isoform enzyme were taken and placed into a 96-deep well plate (A-gen: P-1.0-RD-96-S), and 169 μL of 0.05 mg/mL liver microsomes (BioIVT, IHG) phosphate buffer solution (using 0.2 mg/mL liver microsomes for CYP2C19) was added. After uniform mixing, the plate was placed in a 37°C water bath (Shanghai Jinghong Experimental Equipment Co., Ltd.,

DK-500) with shaking for pre-incubation for 15 min. 10 μL of substrate (adding 1 μL of substrate for CYP3A4-Testosterone) and 20 μL of 10 mM NADPH phosphate buffer solution (MCE, HYF003/CS-4998) were added. After uniform mixing, the plate was incubated in a water bath with shaking for the following time: CYP1A2: 20 min, CYP2C9: 10 min, CYP2C19: 10 min, CYP2D6: 10 min, CYP3A4-Testosterone: 10 min, CYP3A4-Midazolam: 5 min. 400 μL of glacial acetonitrile/methanol (v/v, 1:1) containing an internal standard (500 nM tolbutamide, 5 nM terfenadine) was added to terminate the reaction. After centrifugation at 4000 rpm at 4°C for 40 min, the supernatant was taken and diluted with pure water according to the response of the metabolite of the substrate, and then UPLC-MS/MS (Sciex, Q-Trap 4500) was used for detection to obtain the peak area. The C18 chromatographic column was Kinetex 2.6u C18 100A (50 mm*3 mm).

(3) Data analysis

The inhibitory effect of the compound on each P450 enzyme in human liver microsomes was calculated as the percentage of reduced metabolite formation compared with the non-inhibitory control (i.e. blank DMSO). The IC50 value of the compound for each P450 enzyme in human liver microsomes was calculated with the logarithm of the remaining activity and the inhibitor concentration using Graphpad Prism software. The calculation equation was as follows:

$$\text{Peak area ratio} = \text{compound peak area}/\text{internal standard peak area}$$

Remaining activity (%) = peak area ratio of test substance in sample/peak area ratio of test substance in vehicle control group*100%

In Graphpad Prism software, the IC50 value was calculated using the standard four-parameter curve fitting algorithm.

The compounds of the present disclosure had weak inhibitory effects on the enzyme activities of CYP1A2, CYP2C9, CYP2C19, CYP2D6, and CYP3A4. The test results of representative compounds are shown in Table 6.

Table 6 Inhibitory activity data

| Sample | $IC_{50}$ (μM) | | | | | |
|---|---|---|---|---|---|---|
| | CYP1A2 | CYP2C9 | CYP2C19 | CYP2D6 | CYP3A4-T | CYP3A4-M |
| **KT474** | > 30 μM | 9.612 μM | 3.806 | > 30 μM | > 30 μM | 15.13 μM |
| **Compound 146** | > 30 μM | > 30 μM | 18.440 μM | > 30 μM | > 30 μM | > 30 μM |

**Test example 5:** Evaluation of the inhibitory effect on hERG ion channels

(1) Test objective

In this experiment, the inhibitory effect of the compound on the hERG ion channel was detected by a manual patch clamp electrophysiological method, and the cardiac safety was evaluated based on the single concentration inhibition rate or IC50 of the compound.

(2) Experimental method

The cells used in this experiment were HEK293 cell lines transfected with hERG cDNA and stably expressing hERG channels (#60187, provided by BPS, P3-P23 generations were used for experimental research). The cells was cultured in a medium containing the following components: MEM medium, 10% (v/v) inactivated fetal bovine serum, 1 mM sodium pyruvate, 500 μg geneticin, 0.1 mM non-essential amino acids, 100 U penicillin-streptomycin. HEK293 hERG cells were grown in a culture flask containing the above culture solution and cultured in an incubator at 37°C and 5% $CO_2$. The cells were passaged about three times a week and the cell confluence rate was maintained between 40% and 80%. 24 to 48 h before electrophysiological experiments, HEK293 hERG cells were transferred to glass cell slides pretreated with polylysine (PDL) at 0.05 mg/ml, seeded at the number of $1 \times 10^4$ cells in a 48-well plate, and grown in the same culture solution and conditions. The density of HEK293 hERG cells on each cell slide needed to reach the demand that the majority of cells were isolated and individual.

The components of the extracellular solution used in the hERG experiment were as follows (mM): 145 NaCl, 4

KCl, 2 $CaCl_2$, 1 $MgCl_2$, 10 glucose, and 10 HEPES (PH adjusted to 7.40 with NaOH). The components of the intracellular solution were as follows (mM): 130 KCl, 2 $MgCl_2$, 5 EGTA, 10 HEPES and 5 $Na_2ATP$ (PH adjusted to 7.25 with KOH).

The following concentrations (30, 10, 3, 1, 0.3 and 0.1 μM) were tested to obtain the IC50 of the compound. Before the experiment, stock solutions of 10, 3, 1, 0.3, and 0.1 mM were prepared by gradient dilution with DMSO, followed by further dilution with extracellular solution to achieve the final μM test concentrations. The final concentration of DMSO in the compound solutions at each concentration ranged from 0.1% to 0.3%. All compound solutions were subjected to conventional 5 to 10 min sonication and thorough shaking to ensure complete dissolution of the compound, and all test solutions were mixed uniformly by rotation for at least 10 min.

A manual patch clamp system (HEKA EPC-10 signal amplifier and digital conversion system, purchased from HEKA Electronics, Germany) was used to record whole-cell currents in electrophysiological experiments. The specific test method is described as follows: A circular glass slide with HEK293 cells stably expressing hERG channels growing on its surface was placed in an electrophysiological recording chamber under an inverted microscope. The recording chamber was continuously perfused with extracellular solution (approximately 1 milliliter per minute). Conventional whole-cell patch clamp current recording technique was used during the experiment. Unless otherwise specified, the experiments were performed at conventional room temperature (about 25°C). The cells were voltage-clamped at -80 mV. The cell clamp voltage was depolarized to +30 mV to activate hERG potassium channels and then clamped to - 50 mV after 5 seconds to eliminate inactivation and elicit tail current. The peak value of the tail current was used as the value of the magnitude of the hERG current. After the hERG current recorded in the above steps reached stabilization under the continuous perfusion of extracellular solution in the recording chamber, different concentrations of the compound to be tested could be perfused until the inhibitory effect of the drug on the hERG current reached a stable state.

(3) Data analysis

Excel software was used to calculate the inhibition rate of the compound at each concentration on the hERG ion channel, and Graphpad Prism 9.0 software was used to plot a graph based on the dose-response curve of the test compound with %hERG inhibition rate as the vertical axis and the concentration of the test compound as the horizontal axis. Curve fitting was performed to calculate IC50 using the log (inhibitor) vs. response -- Variable slope (four parameters) parameters, and the specific equation parameters were as shown below: Y=Bottom + (Top-Bottom)/(1+10^((LogIC50-X) *HillSlope)). The compound of the present disclosure have a small inhibitory effect on the hERG ion channel. The test results of some representative compounds are shown in Table 7 below.

Table 7

| Sample | hERG (IC50, manupatch) |
|---|---|
| **KT474** | 2.65 μM |
| **Compound 146** | 7.06 μM |

**Test example 6: Experiment on the inhibition of human-derived PBMC cells to secrete multiple cytokines (IFN-γ, IL-1β, IL-6, IL-10, IL-12p70, TNF-α and IL-23) by the compound**

Experiment materials: Human-derived peripheral blood mononuclear cells (hPBMC) were provided by Hemacare Donor Center.

Experimental method:

Thawing and plating PBMCs: The frozen PBMC was removed from liquid nitrogen, thawed in a 37°C water bath kettle, then resuspended with 10 mL of complete medium, and centrifuged at 350 g at room temperature for 5 min, and the supernatant was discarded. The cells were resuspended with 5 mL of complete medium, and the cells were counted. The cells were diluted to $2 \times 10^6$/mL based on the counting results, and 0.1 mL ($2 \times 10^5$ cells) was added to each well of the 96-well plate.

PBMCs were treated with the compound to be tested: 50 μL of positive reference compound KT474 and compound example 146 of the present disclosure were respectively added to the corresponding wells to a final concentration of 10 μM, 2 μM, 0.4 μM, 80 nM, 16 nM, 3.2 nM, 0.64 nM, 0.128 nM, 0.0256 nM, negative control (DMSO) and blank control, three replicate wells each. After uniform mixing, incubation was performed in an incubator at 37°C and 5% $CO_2$ for 8 h.

LPS induction: After 8 h, 50 μL of LPS was added to all wells to a final concentration of 2 ng/mL. After uniform mixing, incubation was further performed in an incubator at 37°C and 5% $CO_2$ for 16 h. After 16 h, the culture plate was

centrifuged at room temperature at 350 g for 5 min, and 150 μL of supernatant was collected from each well for subsequent MSD detection.

MSD detection: The buffer was added to the freeze-dried human inflammatory factor standard mixture to prepare a highest concentration standard (standard 1), and then serial 4-fold dilutions were performed and a standard with a concentration of 0 was prepared (a total of 10 concentrations). Corresponding 50× cytokine (including human IFN-γ, human IL-1β, human IL-6, human IL-10, human IL-12p70, human TNF-α, and human IL-23) antibodies were diluted into 1× detection antibody solution. The supernatant to be tested was diluted 10 times. 50 μL of the sample (including the standard and the supernatant to be tested) was added to the sample plate. The plate was sealed with a sealing film, and incubated at room temperature at 700 rpm for 2 h. The plate was washed 3 times with 150 μL of 0.05% Tween-20, then 25 μL of 1× detection antibody solution was added. The plate was sealed, and then incubated at room temperature at 700 rpm for 2 h. The plate was washed 3 times, and then 150 μL of 2× reading buffer was added and the plate was read using an MSD instrument.

The experimental results are shown in Fig. 2. The ability of the compound example 146 of the present disclosure to inhibit the secretion of multiple cytokines by human-derived PBMC is significantly better than that of positive reference KT474.

Various modifications of the present disclosure in addition to those described herein will be apparent to those skilled in the art from the foregoing description. Such modifications are also intended to fall within the scope of the appended claims. Each reference cited in the present application (including all patents, patent applications, journal articles, books and any other disclosures) is incorporated herein by reference in its entirety.

## Claims

1. A compound of formula (A):

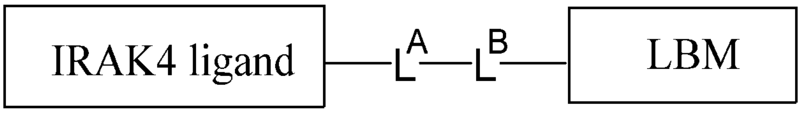

(A)

or a stereoisomer, a tautomer, a diastereomer, a racemate, a cis-trans isomer, an isotopically-labeled compound (preferably deuterated compound), an N-oxide, a metabolite, an ester, a prodrug, a crystal form, a hydrate, a solvate or a pharmaceutically acceptable salt thereof,
**characterized in that**:

$L^A$ is selected from a bond and a straight or branched $C_{1-4}$ alkylene, and the $C_{1-4}$ alkylene is optionally substituted with one or more substituents independently selected from $C_{1-4}$ alkyl, $C_{1-4}$ haloalkyl, halogen, oxo (=O), OH, CN, $NH_2$, $-NH(C_{1-4}$ alkyl) and $-N(C_{1-4}$ alkyl$)_2$;
$L^B$ is a group selected from the following (1) to (21):

(1) -CyL1-,
(2) -CyL1-La-,
(3) -CyL1-Lb-,
(4) -CyL1-La-CyL2-La-,
(5) -CyL1-$NR^{L1}$-
(6) -CyL1-C(O)-,
(7) -CyL1-C(O)-$NR^{L1}$-,
(8) -CyL1-$NR^{L1}$-C(O)-,
(9) -CyL1-CyL2-,
(10) -$NR^{L1}$-CyL1-La-,
(11) -$NR^{L1}$-CyL1-Lb-,
(12) -$NR^{L1}$-CyL3-$NR^{L2}$-,
(13) -$NR^{L1}$-CyL3-La-$NR^{L2}$-,
(14) -$NR^{L1}$-CyL1-C(O)-,
(15) -$NR^{L1}$-La-CyL1-La-,
(16) -La-CyL1-,
(17) -O-La-,
(18) -S-La-,

(19) -$NR^{L1}$-La-,
(20) -CyL1-La-CyL3-, and
(21) -CyL1-Lc-CyL4-;

wherein:

in the (1) to (21), the leftmost extending bond of each group is connected to the $L^A$ and the rightmost extending bond of each group is connected to the

LBM

moiety, or the leftmost extending bond of each group is connected to the

LBM

moiety and the rightmost extending bond of each group is connected to the $L^A$,
CyL1 and CyL2, at each occurrence, are each independently selected from 3-to 12-membered heterocycloalkylene, wherein the heterocycloalkylene preferably has 1, 2 or more nitrogen heteroatoms and 0, 1 or 2 heteroatoms selected from O and S,
CyL3, at each occurrence, is independently selected from $C_{3-12}$ cycloalkylene or 3- to 12-membered heterocycloalkylene,
CyL4, at each occurrence, is independently selected from 5- to 12-membered heteroarylene,
La, at each occurrence, is independently selected from $C_{1-4}$ hydrocarbylene,
Lb, at each occurrence, is independently selected from straight $C_{2-4}$ hydrocarbylene, wherein 1 or 2 but not all $CH_2$ in the straight $C_{2-4}$ hydrocarbylene are replaced with 1 or 2 groups selected from O, S, $NR^{L1}$ and C(O),
Lc, at each occurrence, is independently selected from a bond or $C_{1-4}$ hydrocarbylene,
CyL1, CyL2, CyL3, CyL4, La, Lb and Lc are each optionally substituted with 1 or more groups independently selected from: $C_{1-4}$ alkyl, $C_{1-4}$ haloalkyl, halogen, OH, CN, $NH_2$, -NH($C_{1-4}$ alkyl) and -N($C_{1-4}$ alkyl)$_2$, preferably methyl, ethyl, F, Cl, Br, OH, CN and $NH_2$, more preferably methyl, F, Cl and OH; and
$R^{L1}$ and $R^{L2}$, at each occurrence, are each independently selected from H and $C_{1-4}$ alkyl;
the

IRAK4 ligand

moiety has:

(I) a structure of formula (1):

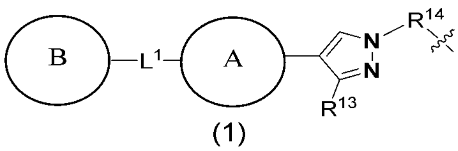

(1)

wherein:

ring A

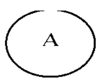

is selected from 5- to 6-membered heteroaryl, and the 5- to 6-membered heteroaryl is optionally substituted with a substituent $R^k$.

$R^k$ is selected from $C_{1-6}$ alkyl, $C_{3-7}$ cycloalkyl, $R^pR^qN$-, $C_{1-6}$ haloalkyl, $C_{1-6}$ heteroalkyl (e.g., $C_{1-6}$ alkoxy), 4- to 9-membered heterocyclyl (e.g., 5- to 6-membered saturated heterocycloalkyl), $C_{6-10}$ aryl, and 5- to 10-membered heteroaryl;

$R^p$ and $R^q$ are each independently selected from hydrogen, $C_{1-6}$ alkyl, $C_{1-6}$ alkoxy, and $C_{1-6}$ haloalkyl;

ring B

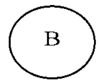

is selected from the following (1) to (3):

(1)

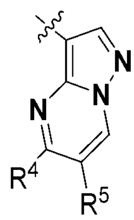

,

wherein:

$R^4$ is selected from hydrogen, $C_{1-6}$ alkyl, $C_{4-9}$ cycloalkyl, $C_{4-9}$ cycloalkyl-$C_{1-6}$ alkyl-, 4- to 9-membered saturated heterocycloalkyl, 4- to 9-membered saturated heterocycloalkyl-$C_{1-6}$ alkyl-, and -$NR_{Na}R_{Nb}$ (wherein the $R_{Na}$ and $R_{Nb}$ are each independently selected from H, $C_{1-6}$ alkyl, and $C_{3-6}$ cycloalkyl, and the $C_{1-6}$ alkyl and the $C_{3-6}$ cycloalkyl may be optionally substituted with 1-3 substituents selected from halogen, hydroxy, $C_{3-6}$ cycloalkyl, $C_{3-6}$ halocycloalkyl, and 4- to 7-membered heterocyclyl), and the $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl- are optionally substituted with 1-3 substituents selected from $C_{1-6}$ alkyl, $C_{1-6}$ alkoxy, $C_{1-6}$ haloalkyl, $C_{1-6}$ haloalkoxy, halogen, hydroxy, hydroxy-$C_{1-6}$ alkylene-, cyano, oxo, -$NH_2$, -NH($C_{1-6}$ alkyl), and -N($C_{1-6}$ alkyl)($C_{1-6}$ alkyl);

$R^5$ is selected from hydrogen, cyano, $C_{1-6}$ alkyl, -C(O)$NH_2$, and -$NR^lR^m$;

$R^l$ and $R^m$ are each independently selected from hydrogen, $C_{1-6}$ alkyl, and $C_{3-6}$ cycloalkyl;

(2)

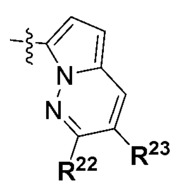

,

wherein:

$R^{22}$ is selected from hydrogen, $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl-;

$R^{23}$ is selected from hydrogen, $C_{1-6}$ alkyl, cyano, carboxyl, -C(O)$NH_2$, -$NR^lR^m$,

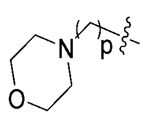, 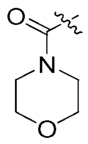, 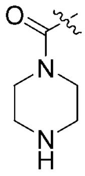, 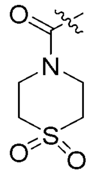, 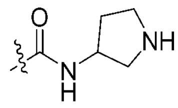, and 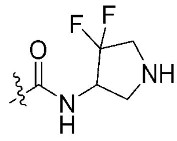;

and $R^l$ and $R^m$ are each independently selected from hydrogen, $C_{1-6}$ alkyl, and $C_{3-6}$ cycloalkyl;

(3)

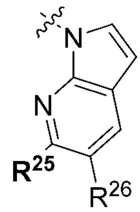,

wherein:

$R^{25}$ is selected from hydrogen, $C_{4-9}$ cycloalkyl, $C_{4-9}$ cycloalkyl-$C_{1-6}$ alkyl-, 4- to 9-membered saturated heterocycloalkyl, and 4- to 9-membered saturated heterocycloalkyl-$C_{1-6}$ alkyl-; $R^{26}$ is selected from hydrogen, $C_{1-6}$ alkyl, cyano, carboxyl, and $-C(O)NH_2$;
$L^1$ is selected from a direct bond, $C_{1-6}$ alkyl, -NH-, -O-, and -S-;
$R^{13}$ is selected from $C_{1-6}$ alkyl and $C_{1-6}$ haloalkyl;
$R^{14}$ is selected from $C_{3-6}$ cycloalkylene, 5- to 12-membered spiro heterocycloalkylene and piperidylidene, and the $C_{3-6}$ cycloalkylene, 5- to 12-membered spiro heterocycloalkylene and piperidylidene are optionally substituted with 1-2 substituents selected from hydroxy$C_{1-6}$ alkyl (e.g., hydroxymethyl), formyl, and $C_{1-6}$ alkyl;

or

(II) a structure of formula (2)

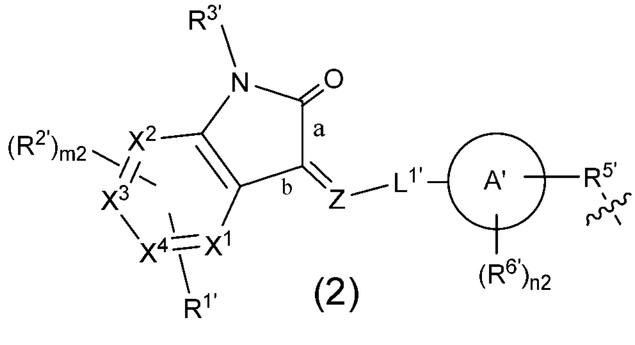

(2)

wherein:

ring A'

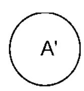

is selected from 5- to 10-membered heteroaryl;

$X^1$, $X^2$, $X^3$ and $X^4$ are each independently N or CH; and at least one of $X^1$, $X^2$, $X^3$ and $X^4$ is not N;
Z is $CR^{4'}$;
each of the letters "a" and "b" indicates the bond between the ring carbon atom connected to Z and adjacent two ring carbon atoms;

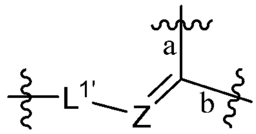

moiety is represented by a structure of the following formula (i) or formula (ii):

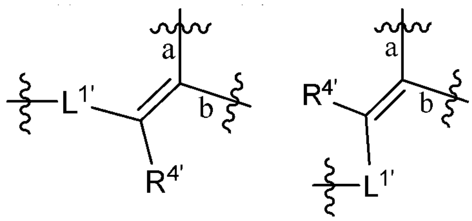

(i) or (ii) ;

$L^{1'}$ is selected from a direct bond and $NR^{7'}$; $R^{1'}$ is $-L^{2'}-R^{1a}$;
$L^{2'}$ is $-S(O)_2NR^{1b}-*$, $-C(O)-NR^{1b}-*$, $-NR^{1b}-C(O)-*$ or $-NR^{1b}-S(O)_2-*$, wherein the bond indicated with * is connected to $R^{1a}$;
$R^{1a}$ is selected from $C_{6-10}$ aryl and 5- to 10-membered heteroaryl, wherein the $C_{6-10}$ aryl and the 5- to 10-membered heteroaryl are each optionally substituted with 1 or more substituents independently selected from the following groups: halogen, OH, SH, $-NR^{1e}R^{1f}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{1e}R^{1f}$;
each $R^{2'}$ is independently selected from: H, halogen, OH, SH, $-NR^{2a}R^{2b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, $-$ $C_{1-6}$ alkylene-OH, $-C_{1-6}$ alkylene-SH, $-C_{1-6}$ alkylene-CN and $-C_{1-6}$ alkylene-$NR^{2a}R^{2b}$;
m2 is 0, 1, 2 or 3;
$R^{3'}$ and $R^{7'}$ are each independently selected from: H, $C_{1-6}$ alkyl, $C_{1-6}$ haloalkyl, $C_{2-6}$ alkenyl, $C_{2-6}$ haloalkenyl, $-C_{1-6}$ alkylene-$O-C_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{3a}R^{3b}$;
$R^{4'}$ is selected from: H, D, halogen, OH, SH, $-NR^{4a}R^{4b}$, CN, $C_{1-6}$ alkyl, $C_{1-6}$ haloalkyl, $C_{2-6}$ alkenyl, $C_{2-6}$ haloalkenyl, $-C_{1-6}$ alkylene-$O-C_{1-6}$ alkyl, $-C_{1-6}$ alkylene-OH, $-C_{1-6}$ alkylene-SH, $-C_{1-6}$ alkylene-CN and $-C_{1-6}$ alkylene-$NR^{4a}R^{4b}$;
$R^{5'}$ is selected from $C_{3-10}$ cyclohydrocarbylene and 3- to 10-membered heterocyclylene, wherein the $C_{3-10}$ cyclohydrocarbylene and the 3- to 10-membered heterocyclylene are each optionally substituted with 1 or more substituents independently selected from the following groups: halogen, OH, SH, $-NR^{5a}R^{5b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, $-$ $C_{1-6}$ alkylene-OH, $-C_{1-6}$ alkylene-SH, $-C_{1-6}$ alkylene-CN and $-C_{1-6}$ alkylene-$NR^{5a}R^{5b}$;
$R^{6'}$ is selected from: H, halogen, OH, SH, $-NR^{6a}R^{6b}$, CN, $C_{1-6}$ alkyl, $C_{1-6}$ haloalkyl, $C_{2-6}$ alkenyl, $C_{2-6}$ haloalkenyl, $-O-C_{1-6}$ alkyl, $-O-C_{1-6}$ haloalkyl, $-S-C_{1-6}$ alkyl, $-C_{1-6}$ alkylene-$O-C_{1-6}$ alkyl, $-OC_{1-6}$ alkylene-$OC_{1-6}$ alkyl, $-C_{1-6}$ alkylene-OH, $-$ $C_{1-6}$ alkylene-SH, $-C_{1-6}$ alkylene-CN and $-C_{1-6}$ alkylene-$NR^{6a}R^{6b}$;
n2 is 0, 1, 2, 3 or 4; and
$R^{1b}$, $R^{1e}$ , $R^{1f}$, $R^{2a}$, $R^{2b}$, $R^{3a}$, $R^{3b}$, $R^{4a}$, $R^{4b}$, $R^{5a}$, $R^{5b}$, $R^{6a}$, and $R^{6b}$, at each occurrence, are independently selected from H and $C_{1-6}$ alkyl; and
the

LBM

moiety is a ligase binding moiety.

**2.** The compound according to claim 1, **characterized in that** $L^A$ is selected from a bond and $C_{1-2}$ alkylene, and the $C_{1-2}$ alkylene is optionally substituted with 1 or more substituents independently selected from $C_{1-2}$ alkyl, $C_{1-2}$ haloalkyl, halogen, oxo, OH, CN, $NH_2$, -NH($C_{1-2}$ alkyl) and -N($C_{1-2}$ alkyl)$_2$;

preferably, $L^A$ is selected from a bond and $C_{1-2}$ alkylene, and the $C_{1-2}$ alkylene is optionally substituted with a substituent selected from $C_{1-2}$ alkyl, halogen and oxo;
further preferably, $L^A$ is selected from a bond, $-CH_2-$, $-CH_2-CH_2-$, $-CH(CH_3)-$ and -C(O)-;
further preferably, $L^A$ is $-CH_2-$ or $-CH_2-CH_2-$, and more preferably $-CH_2-$.

**3.** The compound according to claim 1 or 2, **characterized in that** the IRAK4 ligand moiety has the structure of formula (1).

**4.** The compound according to any one of claims 1-3, **characterized in that**:

the ring A is selected from 5-membered heteroaryl, the 5-membered heteroaryl contains at least one N atom, and the 5-membered heteroaryl is optionally substituted with a substituent $R^k$;
preferably, the ring A is selected from 5-membered heteroaryl, and the 5-membered heteroaryl contains 2-3 heteroatoms in which at least 2 heteroatoms are N atoms; the 5-membered heteroaryl is optionally substituted with a substituent $R^k$;
more preferably, the ring A is selected from 1,2,3-triazolyl, 1,2,4-triazolyl, pyrazolyl, imidazolyl, 1,3,4-thiadiazolyl and 1,3,4-oxadiazolyl;
further preferably, the ring A is selected from

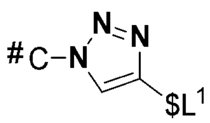,

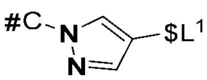, 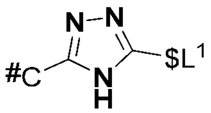, 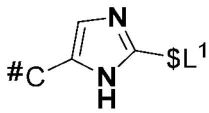, 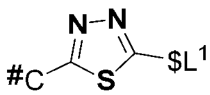

and

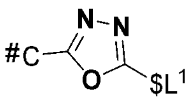,

wherein #C represents a connection site to

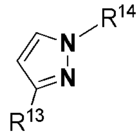

moiety, and $\$L^1$ represents a connection site to $L^1$;
further preferably, the ring A is selected from

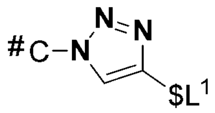

and

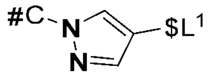,

wherein #C represents a connection site to

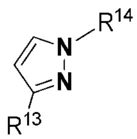

moiety, and $\$L^1$ represents a connection site to $L^1$;
and/or
$R^k$ is selected from $C_{1-6}$ alkyl, $C_{3-6}$ cycloalkyl, $R^pR^qN$-, $C_{1-6}$ haloalkyl and 4- to 9-membered saturated heterocycloalkyl (e.g., 5- to 6-membered saturated heterocycloalkyl);
preferably, $R^k$ is selected from $C_{1-6}$ alkyl, $C_{3-6}$ cycloalkyl, $R^pR^qN$-, $C_{1-6}$ haloalkyl and morpholinyl;
and/or
$R^p$ and $R^q$ are each independently selected from hydrogen and $C_{1-6}$ alkyl;
and/or
more preferably, $R^k$ is selected from isopropyl, cyclopropyl, dimethylamino, difluoromethyl, and

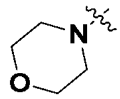.

**5.** The compound according to any one of claims 1-4, **characterized in that**:

$L^1$ is selected from a direct bond and -NH-, preferably a direct bond;
and/or
$R^{13}$ is selected from $C_{1-4}$ haloalkyl, preferably $-CHF_2$ and trifluoromethyl, and more preferably $-CHF_2$.

**6.** The compound according to any one of claims 1-5, **characterized in that** the structure of formula (1) is represented by the following formula (1-1) or (1-2):

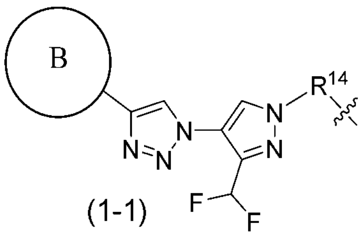
(1-1)

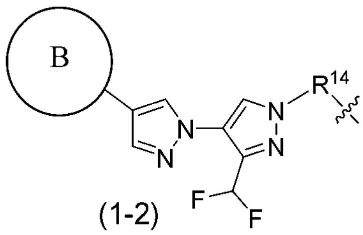
(1-2).

**7.** The compound according to any one of claims 1-6, **characterized in that** the ring B is a group selected from the following (1) to (3):

(1)

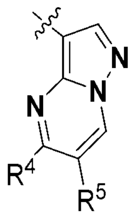,

wherein:

$R^4$ is selected from hydrogen, 4- to 7-membered saturated monocyclic heterocycloalkyl, 6- to 9-membered saturated bridged heterocycloalkyl, 6- to 9-membered saturated spiro heterocycloalkyl, 4- to 7-membered saturated monocyclic heterocycloalkyl-$C_{1-4}$ alkyl-, 6- to 9-membered saturated bridged heterocycloalkyl-$C_{1-4}$ alkyl-, $-NR_{Na}R_{Nb}$ (wherein the $R_{Na}$ and $R_{Nb}$ are each independently selected from H, $C_{1-6}$ alkyl, and $C_{3-6}$ cycloalkyl, and the $C_{1-6}$ alkyl and the $C_{3-6}$ cycloalkyl may be optionally substituted with 1-3 substituents selected from halogen, hydroxy, $C_{3-6}$ cycloalkyl, $C_{3-6}$ halocycloalkyl, and 4- to 7-membered heterocyclyl), and the 4- to 7-membered saturated monocyclic heterocycloalkyl, the 6- to 9-membered saturated bridged heterocycloalkyl, the 6- to 9-membered saturated spiro heterocycloalkyl, the 4- to 7-membered saturated monocyclic heterocycloalkyl-$C_{1-4}$ alkyl-, and the 6- to 9-membered saturated bridged heterocycloalkyl-$C_{1-4}$ alkyl- are optionally substituted with 1-3 substituents selected from $C_{1-6}$ alkyl, $C_{1-6}$ alkoxy, $C_{1-6}$ haloalkyl, $C_{1-6}$ haloalkoxy, halogen, hydroxy, hydroxy-$C_{1-6}$ alkylene-, oxo, $-NH_2$, $-NH(C_{1-6}$ alkyl), and $-N(C_{1-6}$ alkyl)($C_{1-6}$ alkyl);

preferably, $R^4$ is selected from hydrogen, 4- to 7-membered saturated monocyclic heterocycloalkyl, 6- to 9-membered saturated bridged heterocycloalkyl, 6- to 9-membered saturated spiro heterocycloalkyl, $-NR_{Na}R_{Nb}$ (wherein the $R_{Na}$ and $R_{Nb}$ are each independently selected from H, $C_{1-6}$ alkyl, and $C_{3-6}$ cycloalkyl, and the $C_{1-6}$ alkyl and the $C_{3-6}$ cycloalkyl may be optionally substituted with 1, 2 or 3 substituents selected from hydroxy and $C_{3-6}$ cycloalkyl), and the 4- to 7-membered saturated monocyclic heterocycloalkyl, the 6- to 9-membered saturated bridged heterocycloalkyl, and the 6- to 9-membered saturated spiro heterocycloalkyl are optionally substituted with 1, 2 or 3 substituents selected from $C_{1-6}$ alkyl, $C_{1-6}$ haloalkyl, hydroxy, hydroxy-$C_{1-6}$ alkylene-, $-NH_2$, $-NH(C_{1-6}$ alkyl), and $-N(C_{1-6}$ alkyl)($C_{1-6}$ alkyl);

$R^4$ is selected from hydrogen,

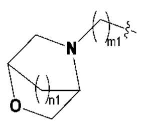, 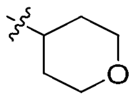, 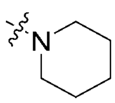, 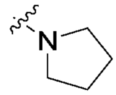,

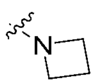, 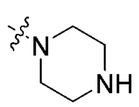, 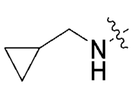, 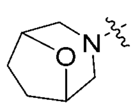, 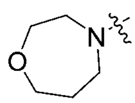, 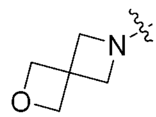, 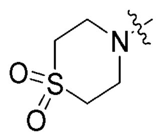,

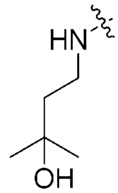 and 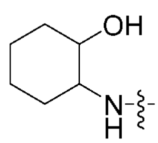,

and the

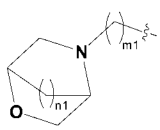, 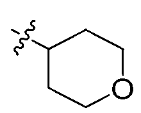, 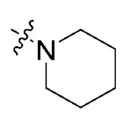, 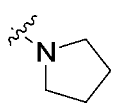,

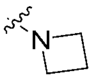, 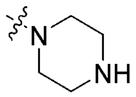, 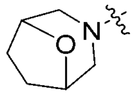, 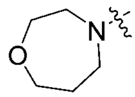 and 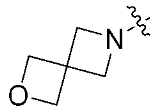

are optionally substituted with 1-3 substituents selected from $C_{1-6}$ alkyl, halogen, hydroxy, hydroxy($C_{1-6}$ alkyl)-, cyano, $-NH_2$, -N($C_{1-6}$ alkyl) and -N($C_{1-6}$ alkyl)($C_{1-6}$ alkyl),
m1 is selected from 0, 1, 2 and 3, preferably 0, and n1 is selected from 0, 1, 2 and 3, preferably 0 or 1;
preferably, $R^4$ is selected from hydrogen,

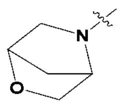, 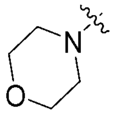, 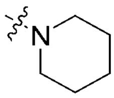,

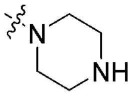, 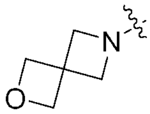, 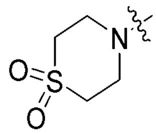, 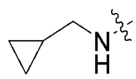, 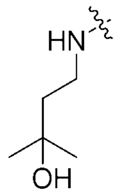, and 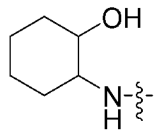,

and the

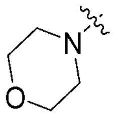, 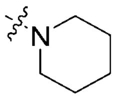, and 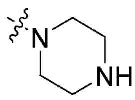

are optionally substituted with 1, 2 or 3 substituents selected from $C_{1-6}$ alkyl, hydroxy, $-NH_2$, and hydroxy($C_{1-6}$ alkyl)-;
and/or
$R^4$ is selected from hydrogen,

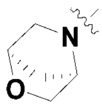, 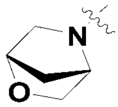, 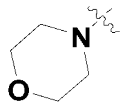, 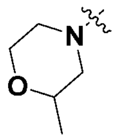

(including

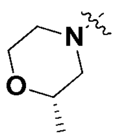 and 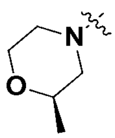

),

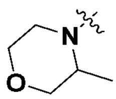

(including

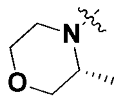 and 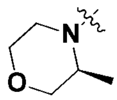

),

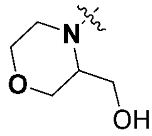

(including

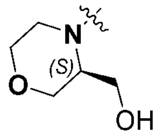

and

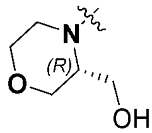

),

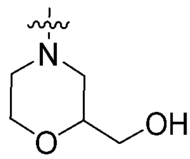

(including

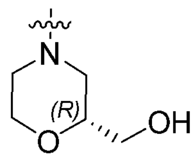 and 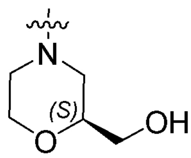

),

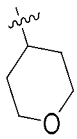, 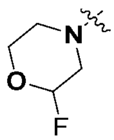, 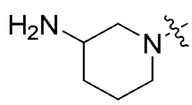

(including

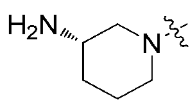 and 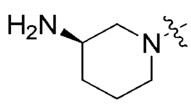

),

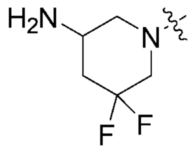, 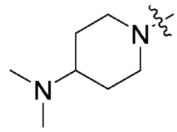, 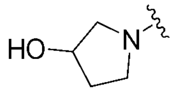,

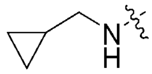, 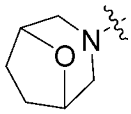, 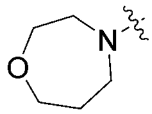, 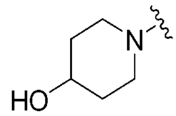, 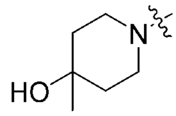, 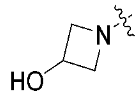,

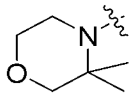, 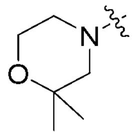, 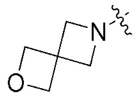, 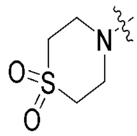, 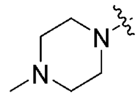, 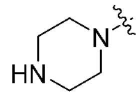, 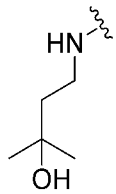,

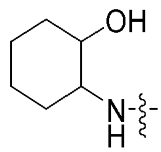

(including

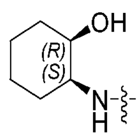, 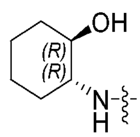, 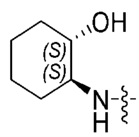, 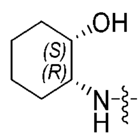,

preferably

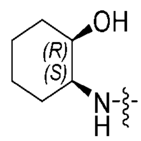 and 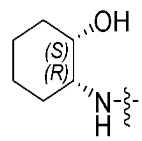

),

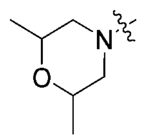

(including

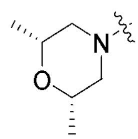, 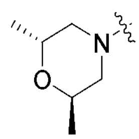,

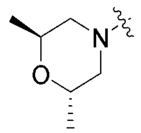 , 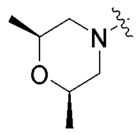 ,

preferably

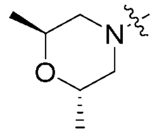

),

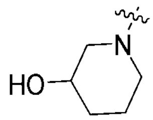

(including

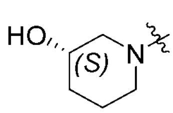 or 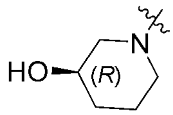

); and/or

$R^5$ is selected from hydrogen, cyano, -C(O)$NH_2$, and -$NR^lR^m$;

and/or

$R^l$ and $R^m$ are each independently selected from hydrogen and $C_{1-6}$ alkyl;

and/or

$R^5$ is selected from hydrogen and cyano;

and/or

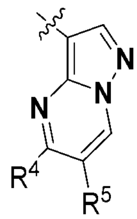

as a whole is selected from

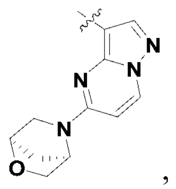 , 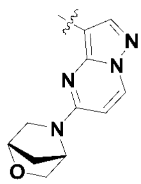 , 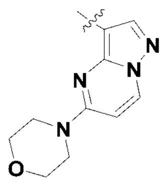 ,

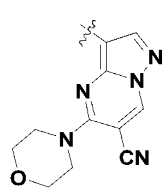 , 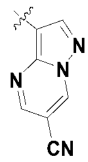 , 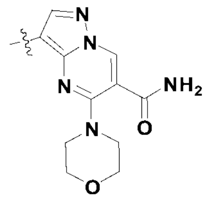 , 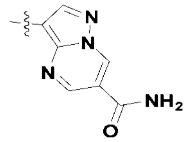 , 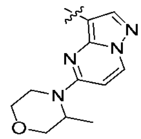

(preferably

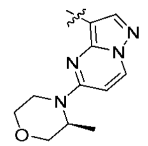

),

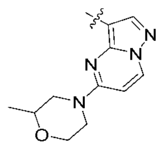

(preferably

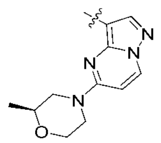 or 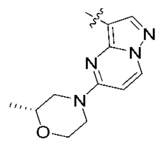

),

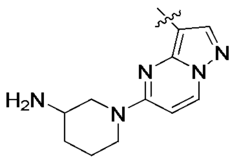

(preferably

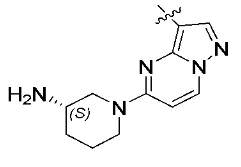 or 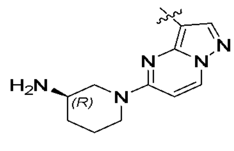

),

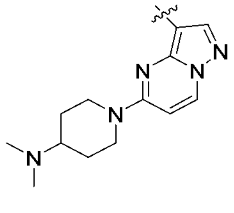 , 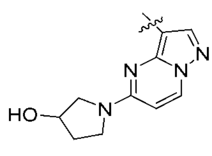 , 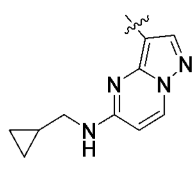 , 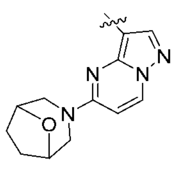 , 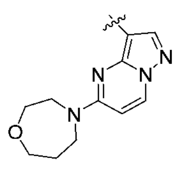 ,

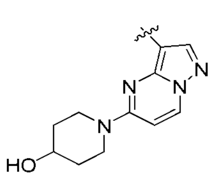 , 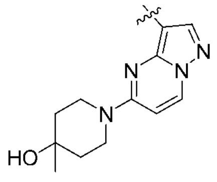 , 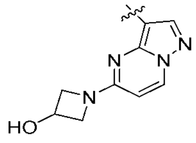 , 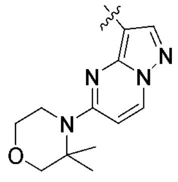 , 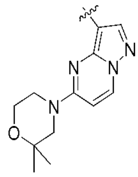 ,

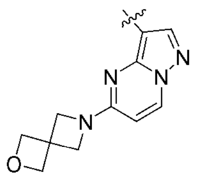 , 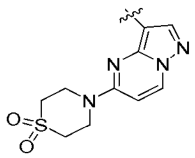 , 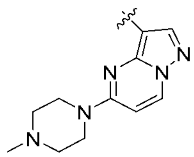 , 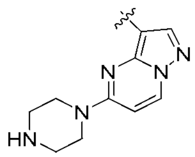 , 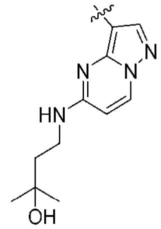 ,

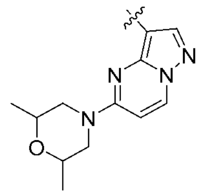

(preferably

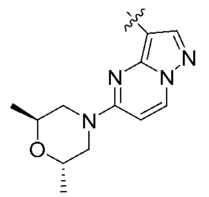

),

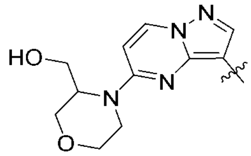

(preferably

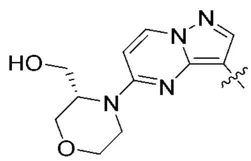

),

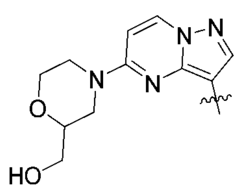

(preferably

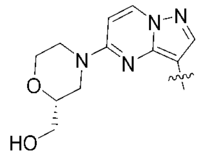

),

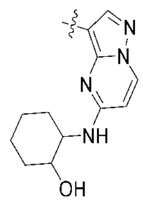

(preferably

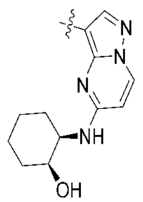

and

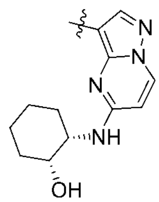

),

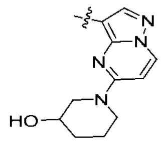

(preferably

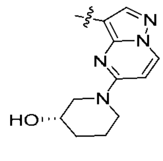

or

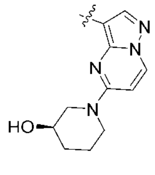

) and

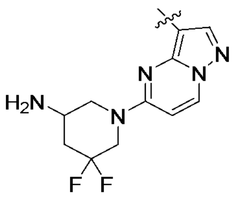

;

preferably,

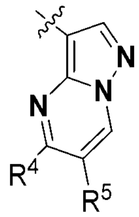

as a whole is selected from

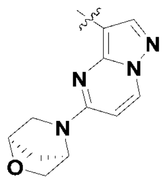

,

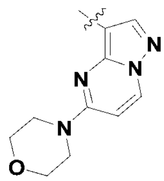

,

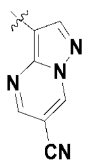

,

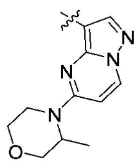

(preferably

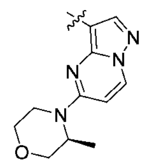

),

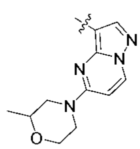

(preferably

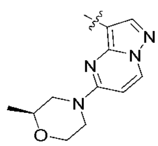

or

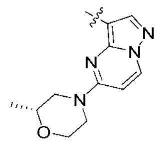

),

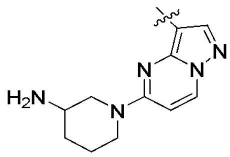

(preferably

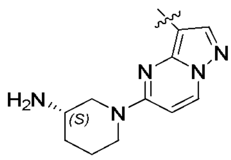

or

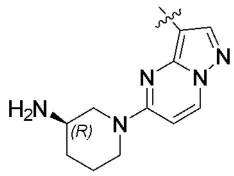

),

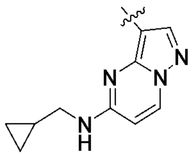

,

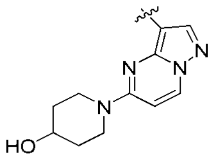

,

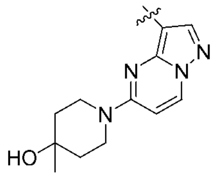

,

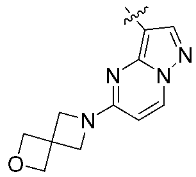

,

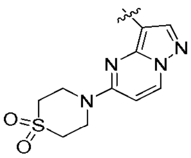

,

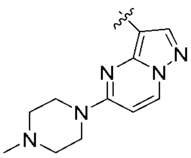

,

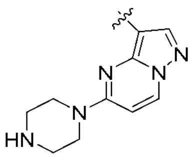

,

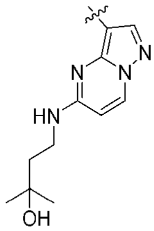

,

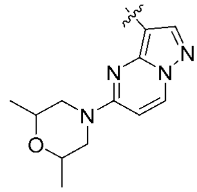

(preferably

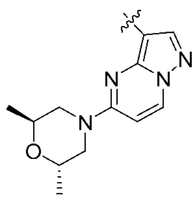

),

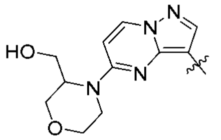

(preferably

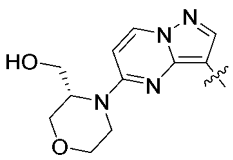

),

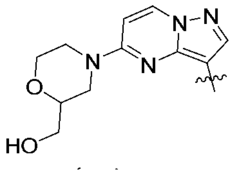

(preferably

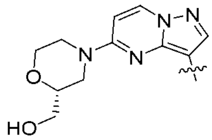

),

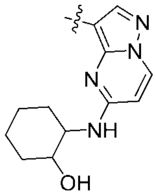

(preferably

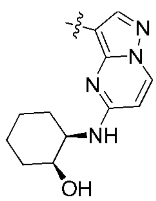

and

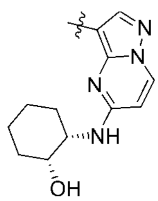

),

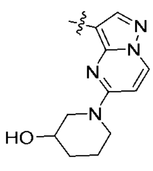

(preferably

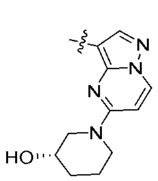

or

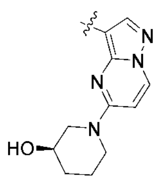

);

(2)

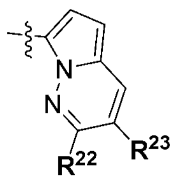,

wherein:

$R^{22}$ is selected from hydrogen and

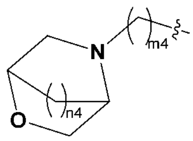,

m4 is selected from 0, 1, 2, and 3, and n4 is selected from 0, 1, 2, and 3;
preferably, $R^{22}$ is selected from hydrogen and

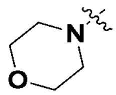;

more preferably, $R^{22}$ is hydrogen;
and/or
$R^{23}$ is selected from hydrogen, $C_{1-6}$ alkyl, cyano, carboxyl, $-C(O)NH_2$ and - $NR^lR^m$;
preferably, $R^{23}$ is selected from hydrogen, cyano, carboxyl, $-C(O)NH_2$ and - $NR^lR^m$;
preferably, $R^{23}$ is selected from hydrogen, cyano, $-C(O)NH_2$ and $-NR^lR^m$;
more preferably, $R^{23}$ is selected from $-C(O)NH_2$;
and/or
$R^l$ and $R^m$ are each independently selected from hydrogen and $C_{1-6}$ alkyl;
and/or

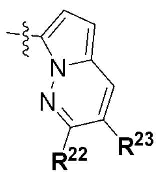

as a whole is selected from

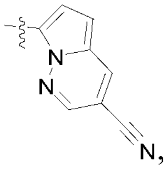, 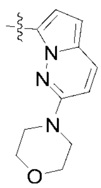, 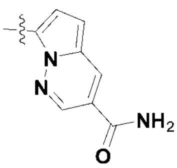,

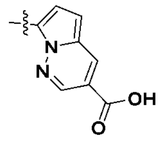, 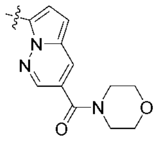, 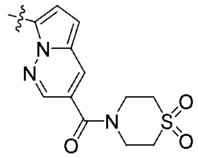, 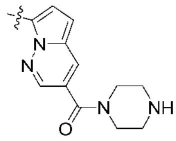, 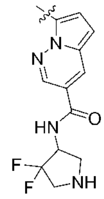 and 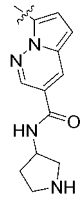;

preferably,

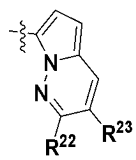

as a whole is selected from

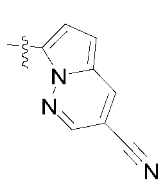, 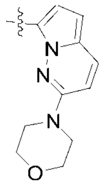

and

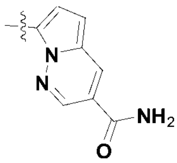;

more preferably,

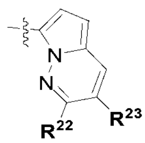

as a whole is

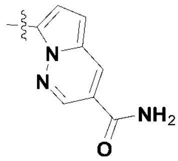;

(3)

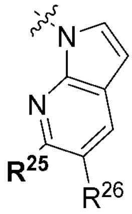,

wherein:

$R^{25}$ is selected from hydrogen,

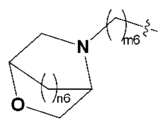 and 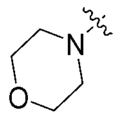,

m6 is selected from 0, 1, 2 and 3, and n6 is selected from 0, 1, 2 and 3;
preferably, $R^{25}$ is selected from hydrogen and

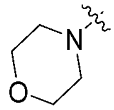;

more preferably, $R^{25}$ is hydrogen;
and/or
$R^{26}$ is selected from hydrogen, $C_{1-6}$ alkyl, cyano and -C(O)$NH_2$; preferably, $R^{26}$ is selected from hydrogen, $C_{1-6}$ alkyl and -C(O)$NH_2$; more preferably, $R^{26}$ is selected from -C(O)$NH_2$;
and/or

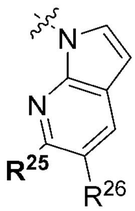

as a whole is selected from

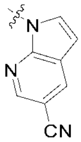, 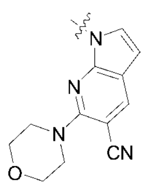, 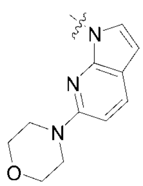, 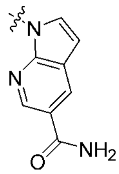

and

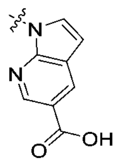;

preferably,

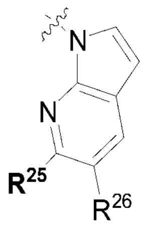

as a whole is

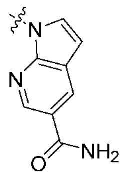
.

**8.** The compound according to any one of claims 1-7, **characterized in that**:

$R^{14}$ is selected from $C_{3-6}$ cycloalkylene, and the $C_{3-6}$ cycloalkylene is optionally substituted with 1-2 substituents selected from hydroxy$C_{1-6}$ alkyl (e.g., hydroxymethyl) and formyl;
preferably, $R^{14}$ is selected from

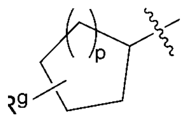
;

p is selected from 0, 1, and 2; $R^g$ is selected from hydrogen, hydroxy$C_{1-6}$ alkyl, and formyl; preferably, $R^g$ is hydrogen;
alternatively, $R^{14}$ is selected from 7- to 11-membered spiro heterocycloalkylene, and the 7- to 11-membered spiro heterocycloalkylene is optionally substituted with 1-2 substituents selected from hydroxy$C_{1-6}$ alkyl (e.g., hydroxymethyl), formyl and $C_{1-3}$ alkyl,
preferably, $R^{14}$ is selected from 9- to 11-membered spiro heterocycloalkylene, and the 9- to 11-membered spiro heterocycloalkylene has 1, 2 or more nitrogen heteroatoms and 0, 1 or 2 heteroatoms selected from O and S;
alternatively, $R^{14}$ is selected from piperidylidene, and the piperidylidene is optionally substituted with 1-2 $C_{1-3}$ alkyl (preferably methyl);
more preferably, $R^{14}$ is selected from

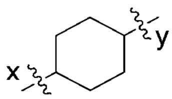
,
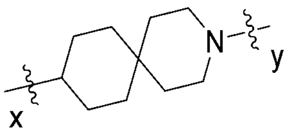
,

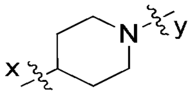
,
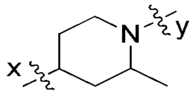
and
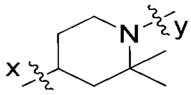
,

further more preferably

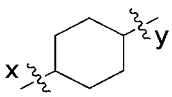
,
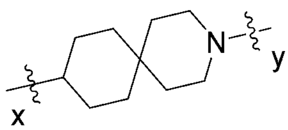
,
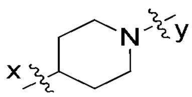
and
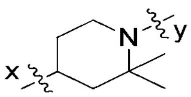
,

even more preferably

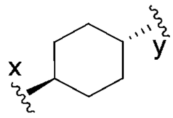, 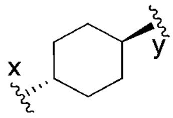, 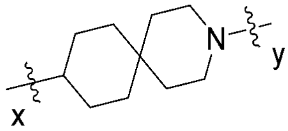, 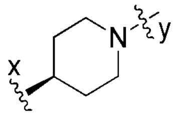

, 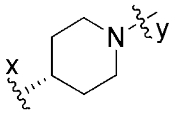, 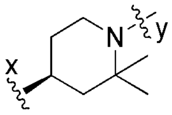 and 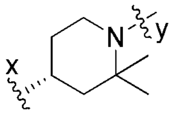,

wherein the bond labeled with x is connected to the pyrazole ring, and the bond labeled with y is connected to the $L^A$.

**9.** The compound according to any one of claims 1-8, **characterized in that** the

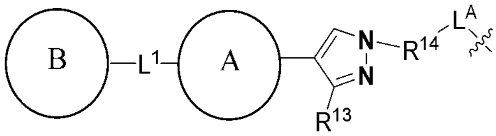

moiety is selected from:

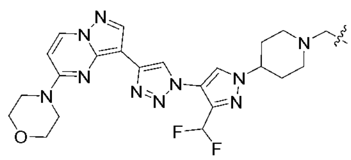, 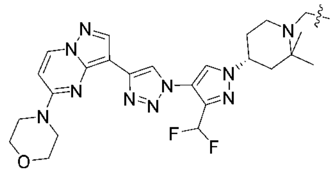,

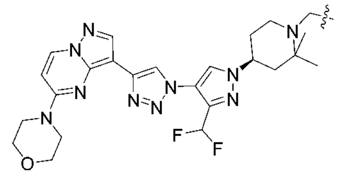, 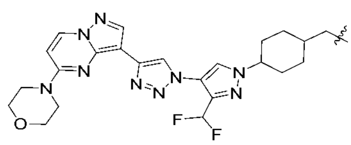, 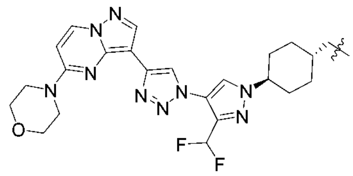,

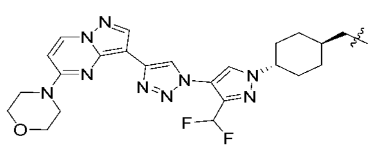, 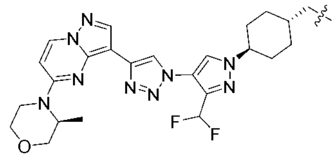,

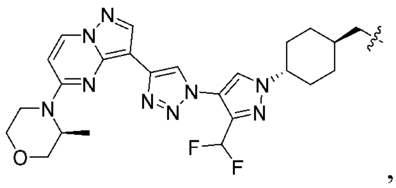, 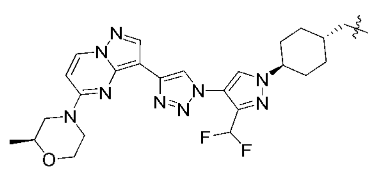,

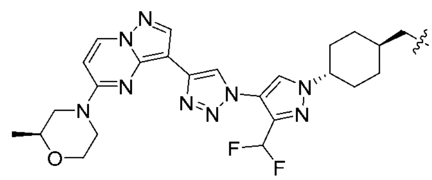
,
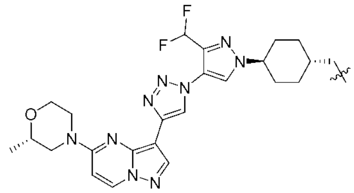
,

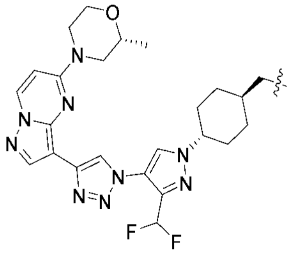
,
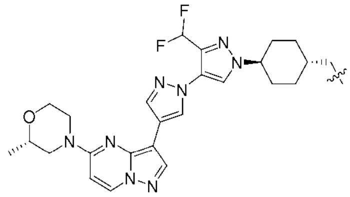
,
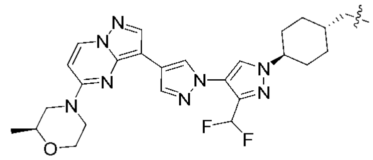
,

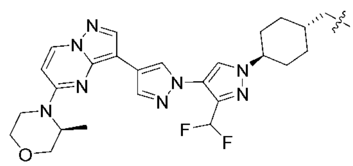
,
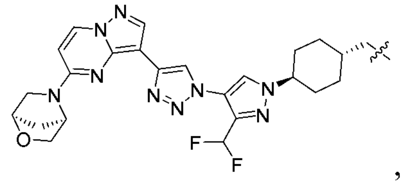
,

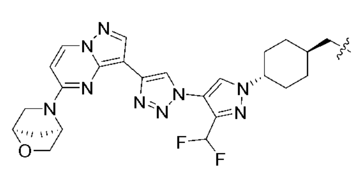
,
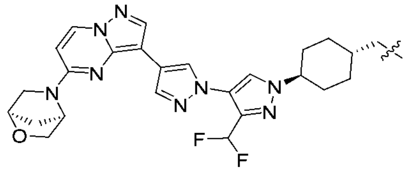
,

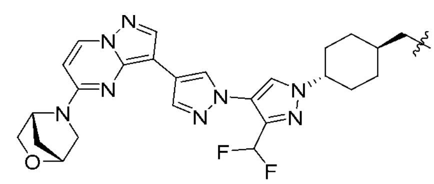
,
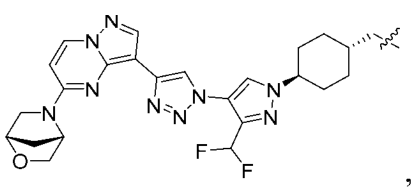
,

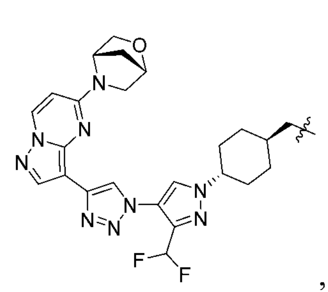
,
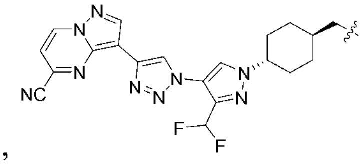
,

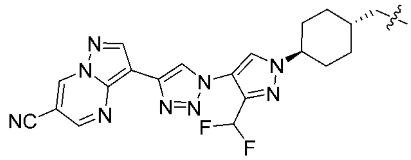
,
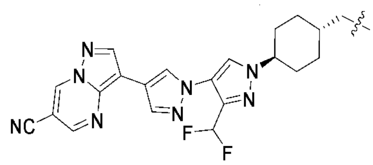
,

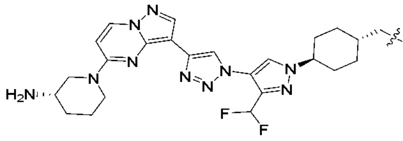,

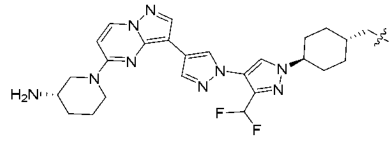,

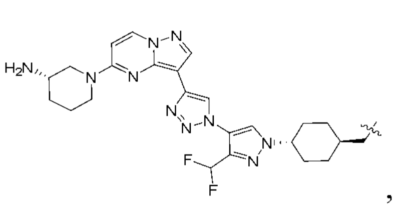,

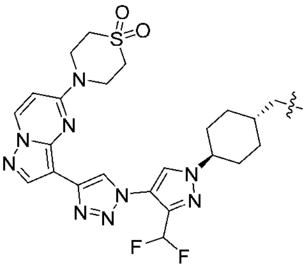,

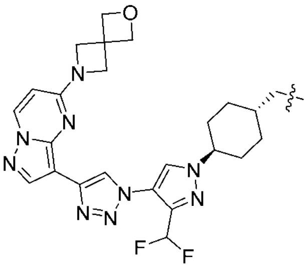,

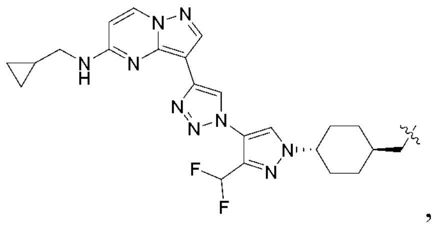,

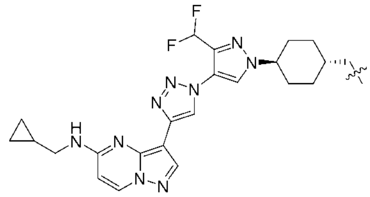,

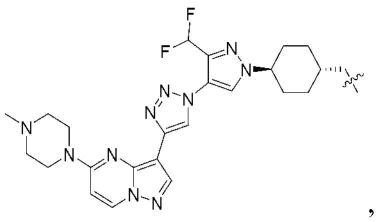,

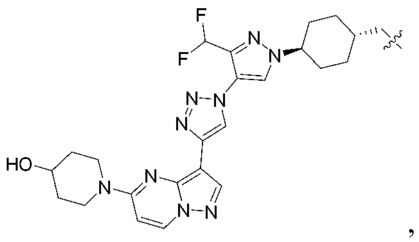,

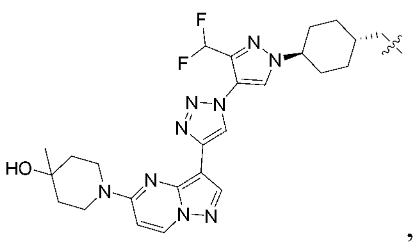,

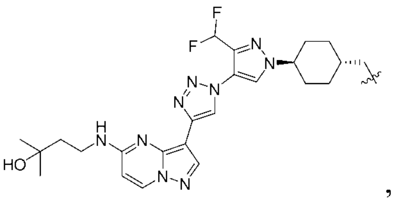,

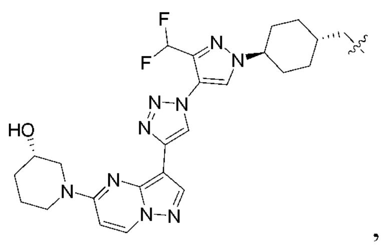,

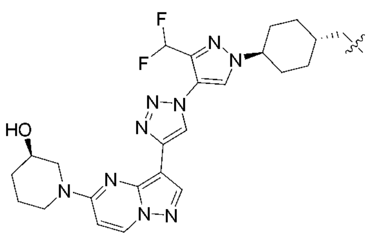,

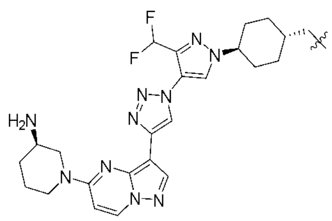,
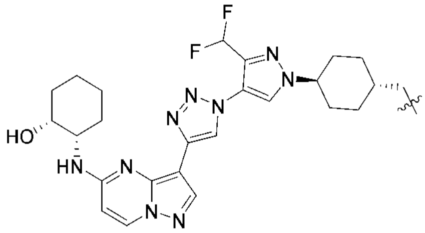,

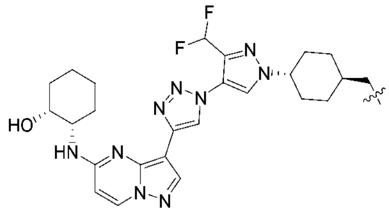,
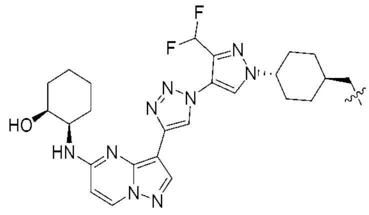,

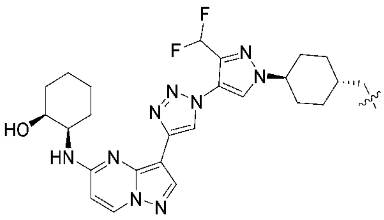,
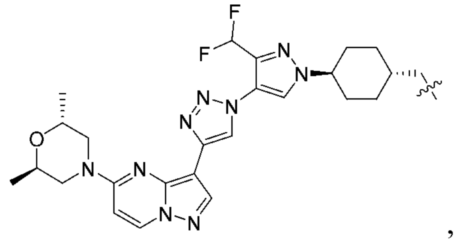,

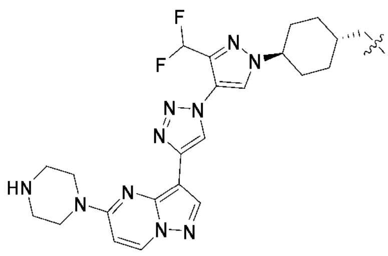,
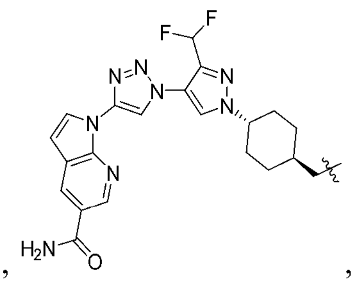,
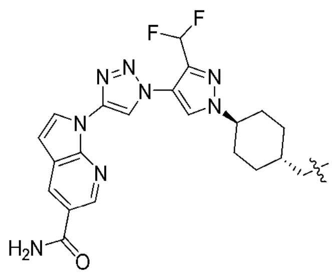

,
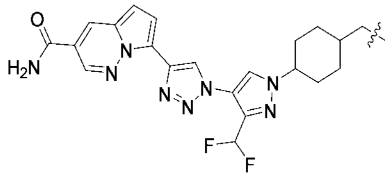,
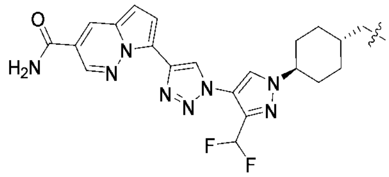,

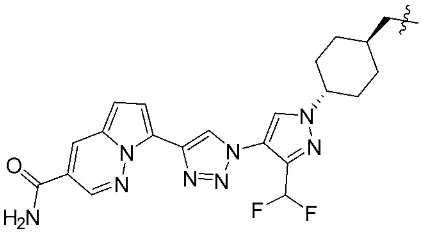,
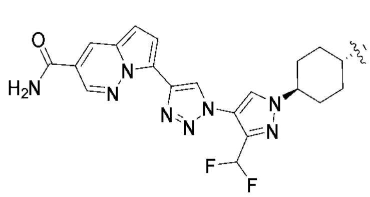,

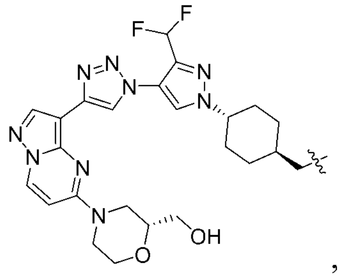, 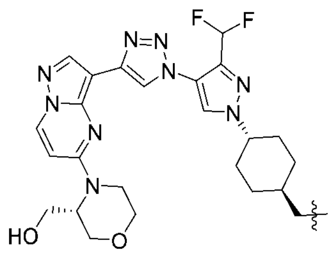,

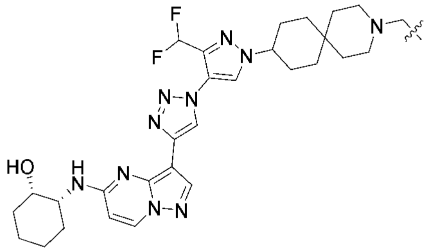, 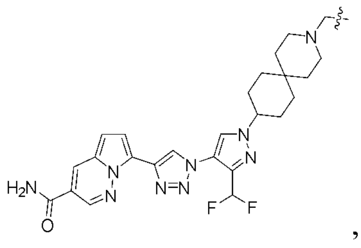,

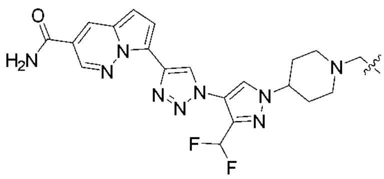, 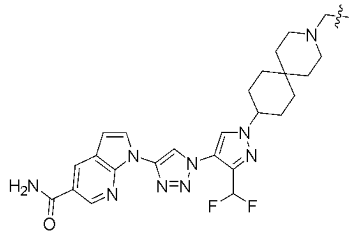

and

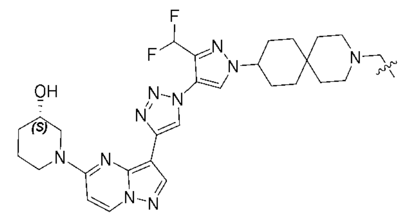.

10. The compound according to claim 1 or 2, **characterized in that** the IRAK4 ligand moiety has the structure of formula (2).

11. The compound according to any one of claims 1-2 and 10, **characterized in that** $R^{3'}$ is selected from: H, $C_{1-4}$ alkyl, $C_{1-4}$ haloalkyl, $C_{2-4}$ alkenyl, $C_{2-4}$ haloalkenyl, -$C_{1-4}$ alkylene-O-$C_{1-4}$ alkyl, -$C_{1-4}$ alkylene-OH, -$C_{1-4}$ alkylene-SH, -$C_{1-4}$ alkylene-CN and -$C_{1-4}$ alkylene-$NR^{3a}R^{3b}$; preferably H and $C_{1-4}$ alkyl, preferably H and methyl, and more preferably H.

12. The compound according to any one of claims 1-2 and 10-11, **characterized in that** the structure of formula (2) is represented by formula (2-1):

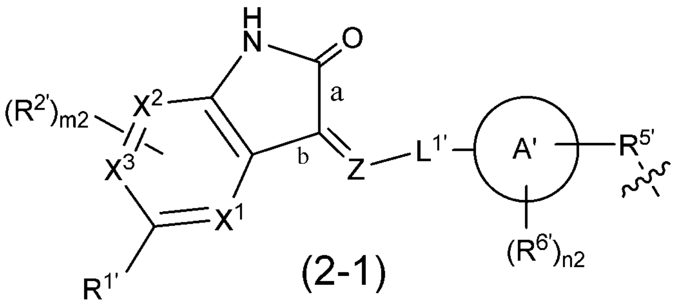

(2-1)

.

**13.** The compound according to any one of claims 1-2 and 10-12, **characterized in that**:

n2 is 0 or 1;
and/or
$R^{5'}$ is selected from: $C_{3-10}$ cyclohydrocarbylene and 3- to 10-membered heterocyclylene, wherein the $C_{3-10}$ cyclohydrocarbylene and the 3- to 10-membered heterocyclylene are each optionally substituted with 1 or more substituents independently selected from the following groups: halogen, OH, SH, $-NR^{5a}R^{5b}$, CN, $C_{1-4}$ alkyl, $C_{1-4}$ haloalkyl, $C_{2-4}$ alkenyl, $C_{2-4}$ haloalkenyl, -O-$C_{1-4}$ alkyl, -O-$C_{1-4}$ haloalkyl, -S-$C_{1-4}$ alkyl, -$C_{1-4}$ alkylene-O-$C_{1-4}$ alkyl, -O$C_{1-4}$ alkylene-O$C_{1-4}$ alkyl, - $C_{1-4}$ alkylene-OH, -$C_{1-4}$ alkylene-SH, -$C_{1-4}$ alkylene-CN and -$C_{1-4}$ alkylene-$NR^{5a}R^{5b}$;
preferably, $R^{5'}$ is selected from: $C_{3-6}$ cycloalkylene and 5- to 10-membered heterocycloalkylene, wherein the $C_{3-6}$ cycloalkylene and the 5- to 10-membered heterocycloalkylene are each optionally substituted with 1 or more substituents independently selected from the following groups: halogen, OH, SH, $-NR^{5a}R^{5b}$, CN, $C_{1-4}$ alkyl, $C_{1-4}$ haloalkyl, $C_{2-4}$ alkenyl, $C_{2-4}$ haloalkenyl, -O-$C_{1-4}$ alkyl, -O-$C_{1-4}$ haloalkyl, -S-$C_{1-4}$ alkyl, -$C_{1-4}$ alkylene-O-$C_{1-4}$ alkyl, -O$C_{1-4}$ alkylene-O$C_{1-4}$ alkyl, - $C_{1-4}$ alkylene-OH, -$C_{1-4}$ alkylene-SH, -$C_{1-4}$ alkylene-CN and -$C_{1-4}$ alkylene-$NR^{5a}R^{5b}$;
more preferably, $R^{5'}$ is selected from: $C_{3-6}$ cycloalkylene and 5- to 10-membered heterocycloalkylene, wherein the $C_{3-6}$ cycloalkylene and the 5- to 10-membered heterocycloalkylene are each optionally substituted with 1 or more substituents independently selected from the following groups: $C_{1-4}$ alkyl, $C_{1-4}$ haloalkyl, -$C_{1-4}$ alkylene-O-$C_{1-4}$ alkyl, -O$C_{1-4}$ alkylene-O$C_{1-4}$ alkyl, -$C_{1-4}$ alkylene-OH, -$C_{1-4}$ alkylene-SH, -$C_{1-4}$ alkylene-CN and -$C_{1-4}$ alkylene-$NR^{5a}R^{5b}$;
more preferably, $R^{5'}$ is selected from

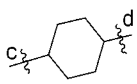, 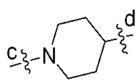, 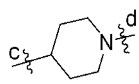,

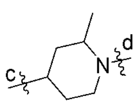 and 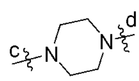,

preferably

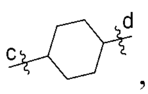, 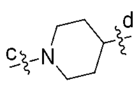 and 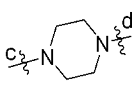,

more preferably

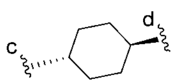, 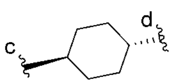, 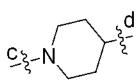 and 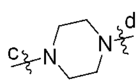,

wherein the bond labeled with "c" is connected to the $L^A$ and the bond labeled with "d" is connected to the ring A'; or preferably, the bond labeled with "c" is connected to the ring A' and the bond labeled with "d" is connected to the $L^A$;
and/or

the ring A' is selected from 5- to 10-membered monocyclic or fused bicyclic heteroaryl;

preferably, the ring A' is selected from 5- to 6-membered monocyclic heteroaryl and 9- to 10-membered fused bicyclic heteroaryl;

more preferably, the ring A' is selected from: 5- to 6-membered monocyclic heteroaryl and benzo 5- to 6-membered monocyclic heteroaryl, wherein preferably, the ring A' is connected to the $L^{1'}$ via the 5- to 6-membered monocyclic heteroaryl;

more preferably, the ring A' is selected from: 5-membered monocyclic heteroaryl and benzo 5-membered monocyclic heteroaryl, wherein preferably, the ring A' is connected to the $L^1$ via the 5-membered monocyclic heteroaryl;

and/or, wherein preferably, the 5-membered monocyclic heteroaryl is selected from furanyl, thienyl, oxazolyl, isoxazolyl, thiazolyl, isothiazolyl, oxadiazolyl, thiadiazolyl, pyrrolyl, pyrazolyl, imidazolyl, triazolyl, and tetrazolyl;

more preferably, the ring A' is selected from pyrazolyl, benzofuranyl, benzothienyl and indolyl; wherein preferably, the benzofuranyl is connected to the $L^1$ via furan ring, the benzothienyl is connected to the $L^1$ via thiophene ring, and the indolyl is connected to the $L^1$ via pyrrole ring;

more preferably, the ring A' is selected from

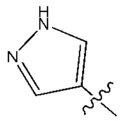, 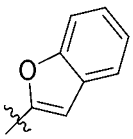, 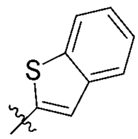

and

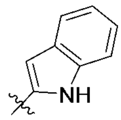;

preferably, the

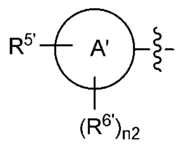

moiety is selected from

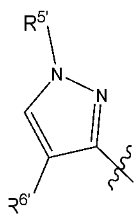, 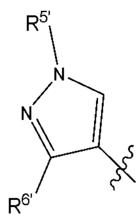,

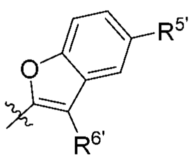, 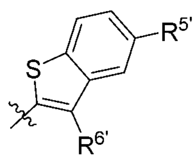 and 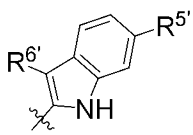,

more preferably

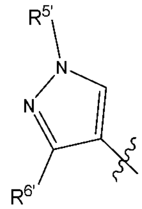

and

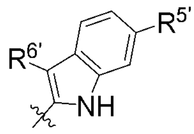

;

and/or
$X^1$, $X^2$ and $X^3$ are each CH; or $X^1$ is N, and $X^2$ and $X^3$ are each CH; or $X^2$ is N, and $X^1$ and $X^3$ are each CH; or $X^3$ is N, and $X^1$ and $X^2$ are each CH; or $X^1$ and $X^2$ are each N, and $X^3$ is CH; or $X^1$ and $X^3$ are each N, and $X^2$ is CH; or $X^2$ and $X^3$ are each N, and $X^1$ is CH; or $X^1$, $X^2$ and $X^3$ are each N.

**14.** The compound according to any one of claims 1-2 and 10-13, **characterized in that** the structure of formula (2):

is represented by formula (2-2) to (2-4):

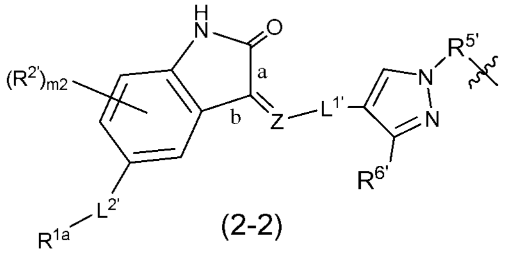

(2-2)

,

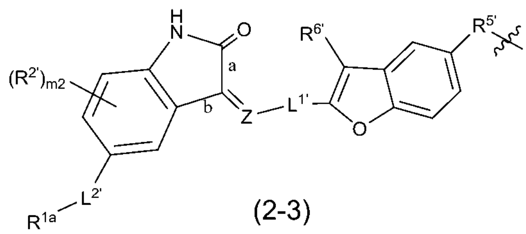

(2-3)

,

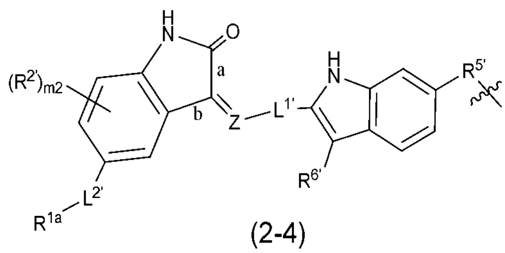

(2-4)

;

preferably, represented by formula (2-5) to (2-8):

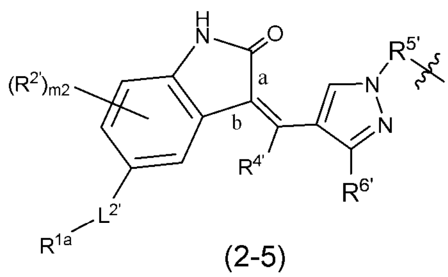

(2-5)

,

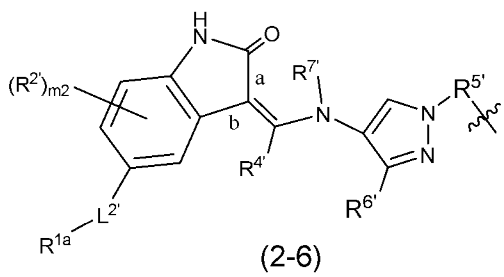

(2-6)

,

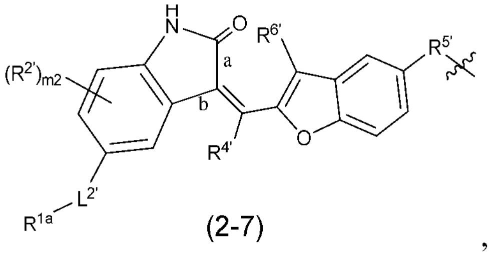
(2-7) ,

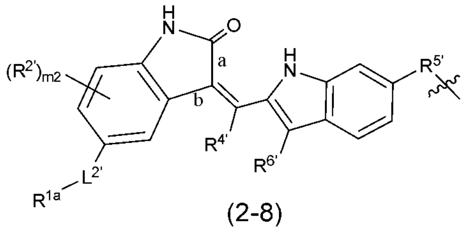
(2-8) .

**15.** The compound according to any one of claims 1-2 and 10-14, **characterized in that**:

$R^{7'}$ is selected from: H, $C_{1-4}$ alkyl, $C_{1-4}$ haloalkyl, $C_{2-4}$ alkenyl, $C_{2-4}$ haloalkenyl, -$C_{1-4}$ alkylene-O-$C_{1-4}$ alkyl, -$C_{1-4}$ alkylene-OH, -$C_{1-4}$ alkylene-SH, -$C_{1-4}$ alkylene-CN and -$C_{1-4}$ alkylene-$NR^{3a}R^{3b}$; preferably H and $C_{1-4}$ alkyl, more preferably H and methyl, further more preferably H;
and/or
$R^{4'}$ is selected from: H, D, halogen, OH, SH, -$NR^{4a}R^{4b}$, CN, $C_{1-4}$ alkyl, $C_{1-4}$ haloalkyl, $C_{2-4}$ alkenyl, $C_{2-4}$ haloalkenyl, -$C_{1-4}$ alkylene-O-$C_{1-4}$ alkyl, -$C_{1-4}$ alkylene-OH, -$C_{1-4}$ alkylene-SH, -$C_{1-4}$ alkylene-CN and -$C_{1-4}$ alkylene-$NR^{4a}R^{4b}$; preferably H, D, halogen, OH, -$NR^{4a}R^{4b}$, CN, $C_{1-4}$ alkyl and $C_{1-4}$ haloalkyl; preferably H, D, F, Cl, OH, -$NH_2$, CN, methyl, ethyl, -$CHF_2$ and -$CF_3$; more preferably H, D, F, Cl methyl and ethyl, further more preferably H;
and/or
$R^{6'}$ is selected from: H, halogen, OH, SH, -$NR^{6a}R^{6b}$, CN, $C_{1-4}$ alkyl, $C_{1-4}$ haloalkyl, $C_{2-4}$ alkenyl, $C_{2-4}$ haloalkenyl, -O-$C_{1-4}$ alkyl, -O-$C_{1-4}$ haloalkyl, -S-$C_{1-4}$ alkyl, -$C_{1-4}$ alkylene-O-$C_{1-4}$ alkyl, -$OC_{1-4}$ alkylene-$OC_{1-4}$ alkyl, -$C_{1-4}$ alkylene-OH, - $C_{1-4}$ alkylene-SH, -$C_{1-4}$ alkylene-CN and -$C_{1-4}$ alkylene-$NR^{6a}R^{6b}$;
preferably, $R^{6'}$ is selected from: H, halogen, OH, SH, -$NR^{6a}R^{6b}$, CN, $C_{1-4}$ alkyl, $C_{1-4}$ haloalkyl, -$C_{1-4}$ alkylene-OH, -$C_{1-4}$ alkylene-SH, -$C_{1-4}$ alkylene-CN and -$C_{1-4}$ alkylene-$NR^{6a}R^{6b}$;
more preferably, $R^{6'}$ is selected from: H, F, Cl, OH, -$NH_2$, -$NHCH_3$, -$N(CH_3)_2$, CN, methyl, ethyl, -$CHF_2$ and -$CF_3$; more preferably H, -$NH_2$, -$NHCH_3$, -$N(CH_3)_2$, methyl, ethyl, -$CHF_2$ and -$CF_3$, further more preferably H, methyl, ethyl and -$CHF_2$.

**16.** The compound according to any one of claims 1-2 and 10-15, **characterized in that** the structure of formula (2) is represented by formula (2-9) to (2-12):

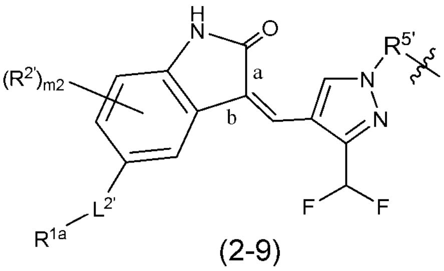
(2-9) ,

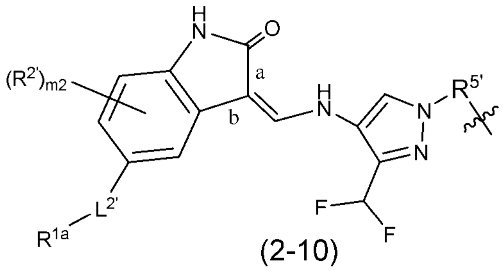
(2-10) ,

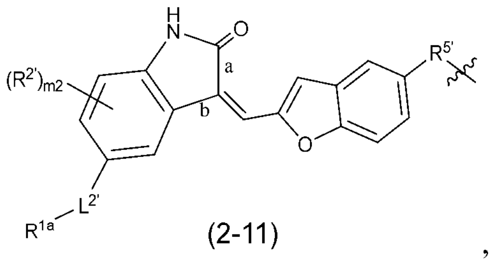
(2-11) ,

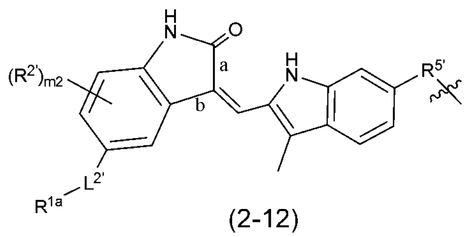
(2-12) .

**17.** The compound according to any one of claims 1-2 and 10-16, **characterized in that**:

$R^{1a}$ is $C_{6-10}$ aryl and 5- to 10-membered heteroaryl, wherein the $C_{6-10}$ aryl and the 5- to 10-membered heteroaryl are each optionally substituted with 1 or more substituents independently selected from the following groups: halogen, OH, SH, - $NR^{1e}R^{1f}$, CN, $C_{1-4}$ alkyl, $C_{1-4}$ haloalkyl, $C_{2-4}$ alkenyl, $C_{2-4}$ haloalkenyl, -O-$C_{1-4}$ alkyl, -O-$C_{1-4}$

haloalkyl, -S-$C_{1-4}$ alkyl, -$C_{1-4}$ alkylene-O-$C_{1-4}$ alkyl, -$OC_{1-4}$ alkylene-$OC_{1-4}$ alkyl, -$C_{1-4}$ alkylene-OH, -$C_{1-4}$ alkylene-SH, -$C_{1-4}$ alkylene-CN and -$C_{1-4}$ alkylene-$NR^{1e}R^{1f}$;
preferably, $R^{1a}$ is phenyl, wherein the phenyl is optionally substituted with 1 or more substituents independently selected from the following groups: halogen, OH, -$NR^{1e}R^{1f}$, CN, $C_{1-4}$ alkyl, $C_{1-4}$ haloalkyl, -O-$C_{1-4}$ alkyl and -O-$C_{1-4}$ haloalkyl, preferably F, Cl, OH, -$NH_2$, CN, methyl and ethyl;
more preferably, $R^{1a}$ is selected from

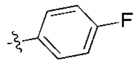 and 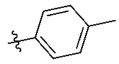 ;

and/or
$L^{2'}$ is -$S(O)_2NR^{1b}$-*, -C(O)-$NR^{1b}$-* or -$NR^{1b}$-C(O)-*, preferably -$S(O)_2NR^{1b}$-*, wherein the bond indicated with * is connected to $R^{1a}$;
and/or
each $R^{2'}$ is independently selected from: H, halogen, OH, SH, -$NR^{2a}R^{2b}$, CN, $C_{1-4}$ alkyl, $C_{1-4}$ haloalkyl, $C_{2-4}$ alkenyl, $C_{2-4}$ haloalkenyl, -O-$C_{1-4}$ alkyl, -O-$C_{1-4}$ haloalkyl, -S-$C_{1-4}$ alkyl, -$C_{1-4}$ alkylene-O-$C_{1-4}$ alkyl, -$OC_{1-4}$ alkylene-$OC_{1-4}$ alkyl, - $C_{1-4}$ alkylene-OH, -$C_{1-4}$ alkylene-SH, -$C_{1-4}$ alkylene-CN and -$C_{1-4}$ alkylene-$NR^{2a}R^{2b}$; preferably H;
and/or
$R^{1b}$, $R^{1e}$, $R^{1f}$, $R^{2a}$, $R^{2b}$, $R^{3a}$, $R^{3b}$, $R^{4a}$, $R^{4b}$, $R^{5a}$, $R^{5b}$, $R^{6a}$, and $R^{6b}$, at each occurrence, are independently selected from H and $C_{1-4}$ alkyl, preferably H, methyl and ethyl.

**18.** The compound according to any one of claims 1-2 and 10-17, **characterized in that**:

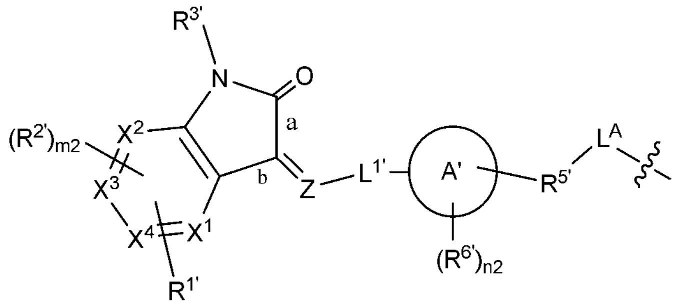

moiety is selected from:

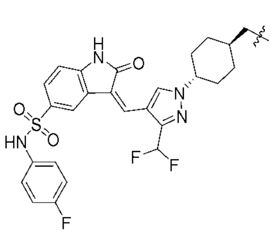 , 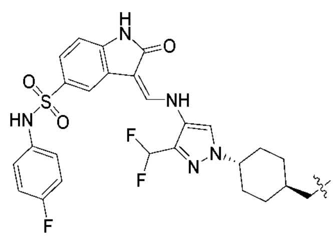 , 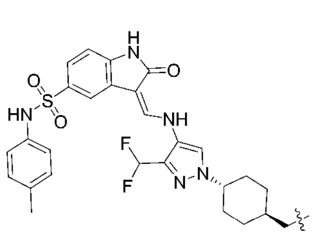 ,

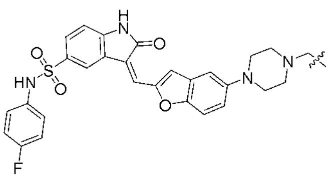 , 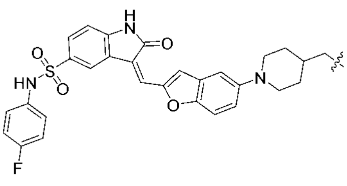

and

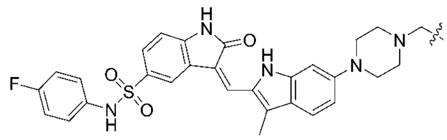
.

**19.** The compound according to any one of claims 1-18, **characterized in that**:

the CyL1 group and the CyL2 group, at each occurrence, are each independently selected from 4- to 11-membered heterocycloalkylene, preferably 4-to 7-membered monocyclic heterocycloalkylene, 6- to 10-membered fused bicyclic heterocycloalkylene, 6- to 9-membered bridged heterocycloalkylene and 5- to 12-membered spiro heterocycloalkylene, more preferably 4- to 6-membered monocyclic heterocycloalkylene, 8- to 10-membered fused bicyclic heterocycloalkylene, 6- to 8-membered bridged heterocycloalkylene and 7- to 11-membered spiro heterocycloalkylene, wherein any of the above heterocycloalkylene preferably has 1, 2 or more nitrogen heteroatoms and 0, 1 or 2 heteroatoms selected from O and S;

and/or

the CyL3 group, at each occurrence, is independently selected from $C_{4-11}$ cycloalkylene, 4- to 11-membered heterocycloalkylene, preferably $C_{4-6}$ monocyclic cycloalkylene, $C_{6-10}$ fused bicyclic cycloalkylene, $C_{6-9}$ bridged cycloalkylene, $C_{5-12}$ spiro cycloalkylene, 4- to 7-membered monocyclic heterocycloalkylene, 6- to 10-membered fused bicyclic heterocycloalkylene, 6- to 9-membered bridged heterocycloalkylene and 5- to 12-membered spiro heterocycloalkylene, more preferably $C_{5-6}$ monocyclic cycloalkylene, $C_{8-10}$ fused bicyclic cycloalkylene, $C_{6-8}$ bridged cycloalkylene, $C_{7-11}$ spiro cycloalkylene, 4- to 6-membered monocyclic heterocycloalkylene, 8- to 10-membered fused bicyclic heterocycloalkylene, 6- to 8-membered bridged heterocycloalkylene and 7- to 11-membered spiro heterocycloalkylene, more preferably $C_{5-6}$ monocyclic cycloalkylene, $C_{9-11}$ spiro cycloalkylene, wherein any of the above heterocycloalkylene preferably has 1, 2 or more nitrogen heteroatoms and 0, 1 or 2 heteroatoms selected from O and S;

and/or

the CyL4 group, at each occurrence, is independently selected from 5- to 10-membered heteroarylene, preferably 5- to 6-membered heteroarylene, more preferably 5- to 6-membered nitrogen-containing heteroarylene;

and/or

La, at each occurrence, is independently selected from $C_{1-4}$ alkylene, $C_{2-4}$ alkenylene and $C_{2-4}$ alkynylene, preferably $-CH_2-$, $-(CH_2)_2-$, $-(CH_2)_3-$, $-(CH_2)_4-$, - CH=CH-, $-CH_2-CH=CH-$, $-CH=CH-CH_2-$, -C=C-, $-CH_2-C=C-$, $-C\equiv C-CH_2-$, $-C\equiv C-CH_2CH_2-$, $-CH_2CH_2-C\equiv C-$ and $-CH_2-C\equiv C-CH_2-$, more preferably $-CH_2-$, $-(CH_2)_2-$, - $(CH_2)_3-$, $-C\equiv C-$, $-CH_2-C\equiv C-$, $-C\equiv C-CH_2-$, $-C\equiv C-CH_2CH_2-$ and $-CH_2CH_2-C\equiv C-$;

and/or

Lb, at each occurrence, is independently selected from -O-straight $C_{1-3}$ alkylene, -straight $C_{1-3}$ alkylene-O-, -O-$C_{2-3}$ alkenylene, -$C_{2-3}$ alkenylene-O-, -O-$C_{2-3}$ alkynylene, -$C_{2-3}$ alkynylene-O-, -$NR^{8'}$-straight $C_{1-3}$ alkylene-, -straight $C_{1-3}$ alkylene-$NR^{8'}$-, -straight $C_{1-2}$ alkylene-$NR^{8'}$-straight $C_{1-2}$ alkylene-, -straight $C_{1-2}$ alkylene-C(O)-$NR^{8'}$-, -$NR^{8'}$-C(O)-straight $C_{1-2}$ alkylene-, -C(O)-straight $C_{1-3}$ alkylene-, -straight $C_{1-3}$ alkylene-C(O)-, -straight $C_{1-2}$ alkylene-$NR^{8'}$-C(O)- and - C(O)-$NR^{8'}$-straight $C_{1-2}$ alkylene-; preferably -O-$C_{2-3}$ alkynylene, -$NR^{8'}$-straight $C_{1-3}$ alkylene-, -straight $C_{1-3}$ alkylene-$NR^{8'}$-, -straight $C_{1-2}$ alkylene-$NR^{8'}$-straight $C_{1-2}$ alkylene-, -straight $C_{1-2}$ alkylene-C(O)-$NR^{8'}$-, -C(O)-straight $C_{1-3}$ alkylene-; wherein $R^{8'}$, at each occurrence, is independently selected from H and $C_{1-4}$ alkyl;

and/or

Lc, at each occurrence, is independently selected from a bond or straight $C_{1-3}$ alkylene, preferably a bond, methylene or ethylene, more preferably a bond or methylene;

and/or

$R^{L1}$ $R^{L2}$, and $R^{8'}$, at each occurrence, are each independently selected from H, methyl and ethyl, more preferably H and methyl;

and/or $L^B$ is a group selected from the following (1) to (21):

(1)

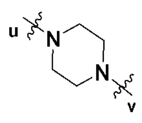, 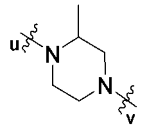

(preferably

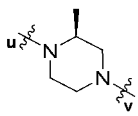 and 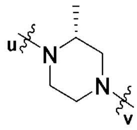), 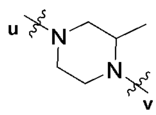

(preferably

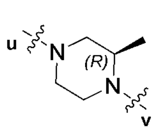 and 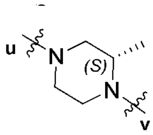), 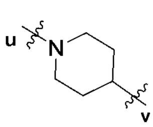, 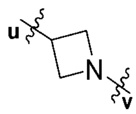, 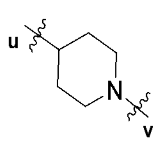,

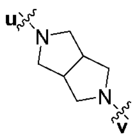, 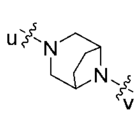, 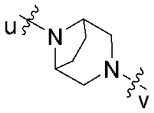, 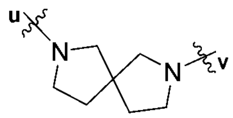, 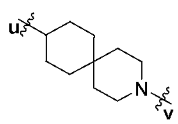,

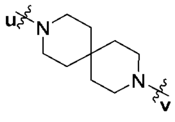, 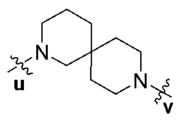, 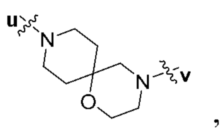, 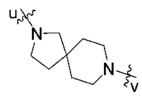, 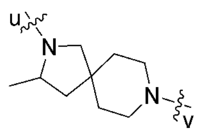

(preferably

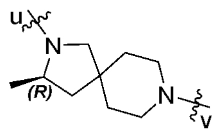 and 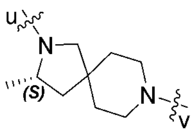), 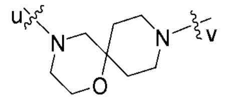,

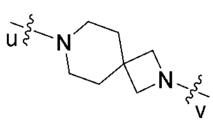, 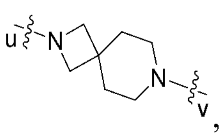, 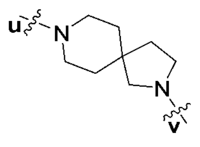 and 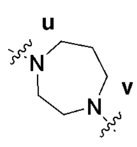,

(2)

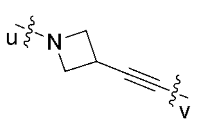, 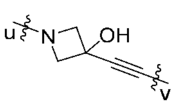, 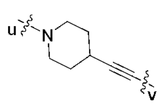, 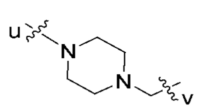,

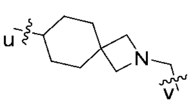, 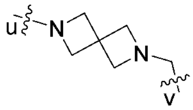, 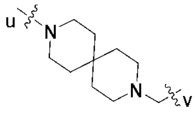, 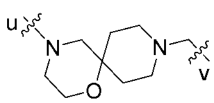, 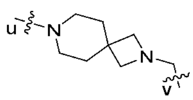

and

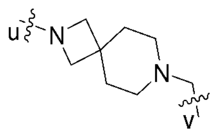,

(3)

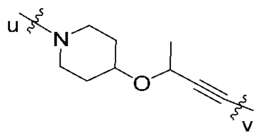

(preferably

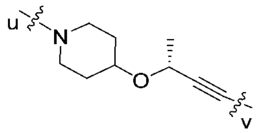

and

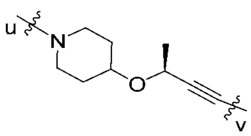), 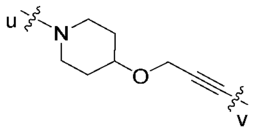, 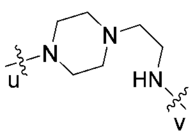, 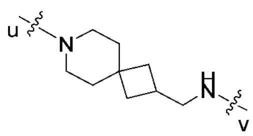,

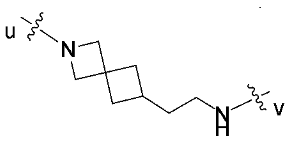, 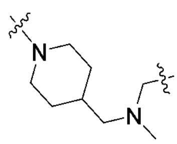, 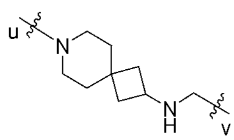, 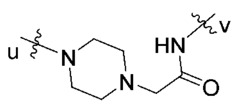,

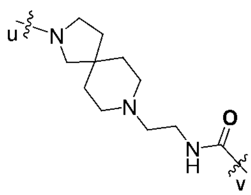 and 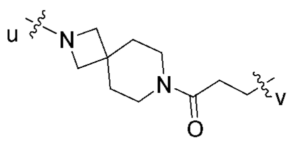,

(4)

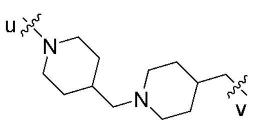,

(5)

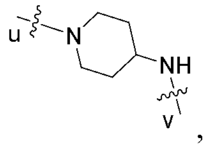

,

(6)

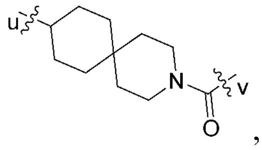

,

(7)

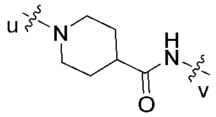 and 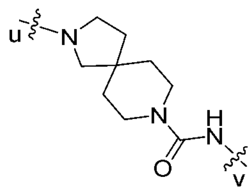

,

(8)

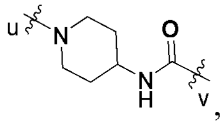

,

(9)

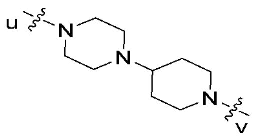 , 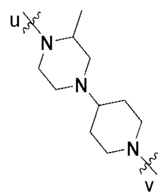

(preferably

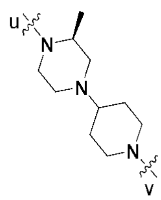

and

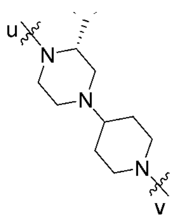

),

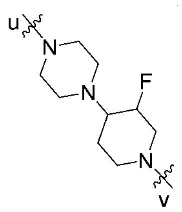

(preferably

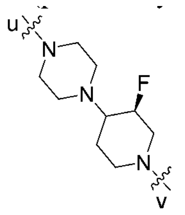 , 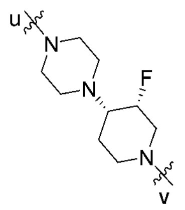 ,

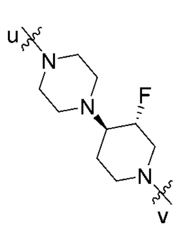 , 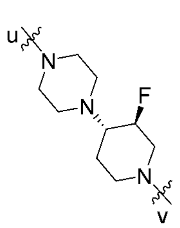 and 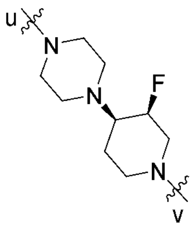

),

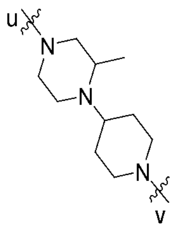

(preferably

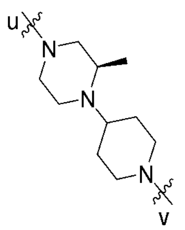 and 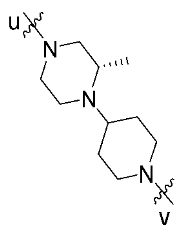

),

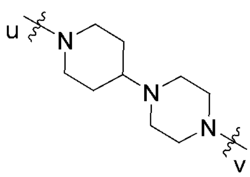 , 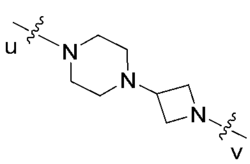 ,

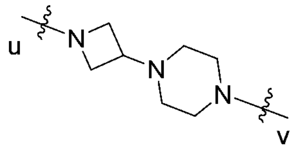, 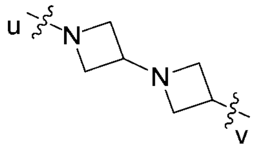,

and

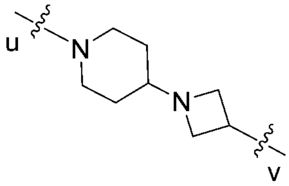;

(10)

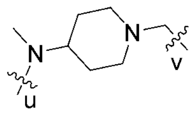 and 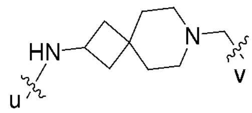,

(11)

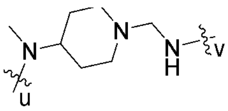 and 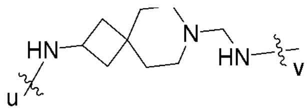,

,
(12)

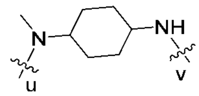

(preferably

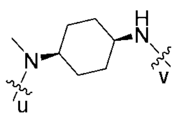, 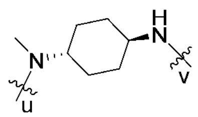,

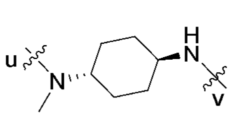 and 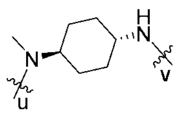

) and

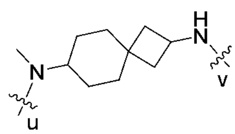,

(13)

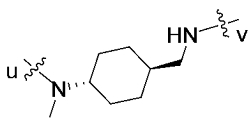 and 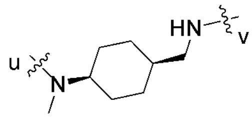 ,

(14)

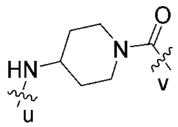 ,

(15)

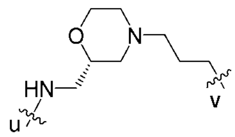 , 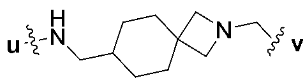 and 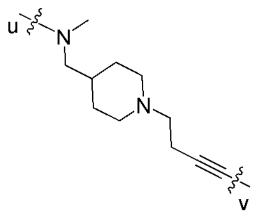 ,

(16)

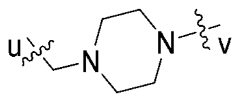 ,

(17)

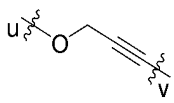 ,

(18)

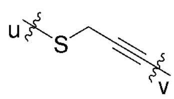 ,

(19)

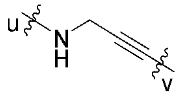 ,

(20)

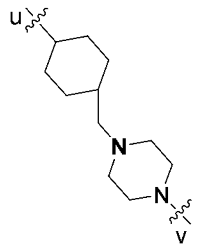

preferably (

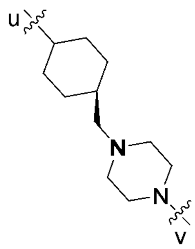 and 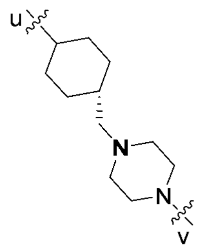

) and

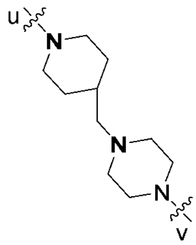

and
(21)

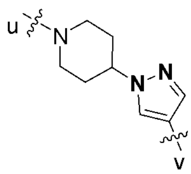 and 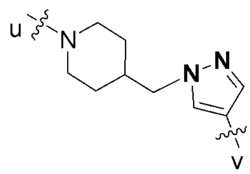 ;

wherein preferably, in any group of the above (1) to (21), the bond labeled with "u" is connected to the LA, and the bond labeled with "v" is connected to the

LBM

moiety.

**20.** The compound according to any one of claims 1-19, **characterized in that**:

the

LBM

moiety is an E3 ubiquitin ligase ligand,

preferably, the

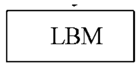

moiety is selected from:

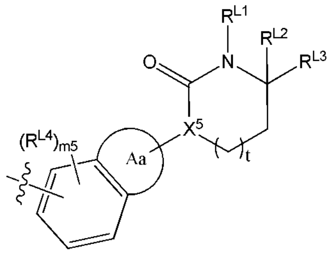

,

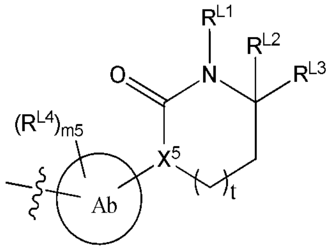

and

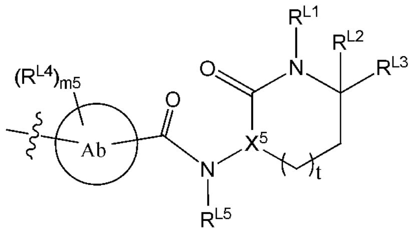

,

wherein:

ring Aa

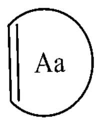

is 5-membered heterocyclyl or 5-membered heteroaryl, preferably 5-membered heterocyclyl or 5-membered heteroaryl having 1, 2 or more N heteroatoms, wherein the 5-membered heterocyclyl and the 5-membered heteroaryl are optionally substituted with one or more substituents independently selected from H, halogen, OH, $NH_2$, CN, oxo and $C_{1-4}$ alkyl,
preferably,

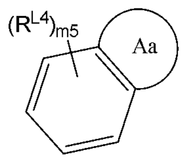

moiety is selected from

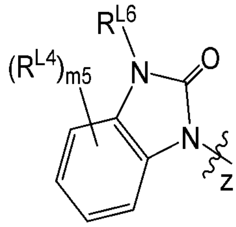

,

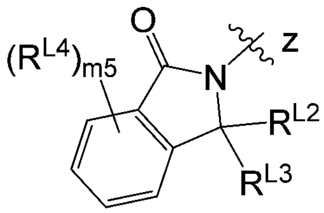 and 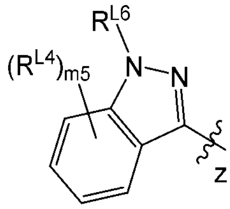,

wherein the bond labeled with "z" is connected to $X^5$;
each ring

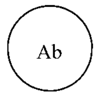

is independently phenyl or 5- to 6-membered heteroaryl, preferably phenyl;
$X^5$ is $CR^{L7}$ or N; t is 0 or 1, preferably 1;
$R^{L1}$, $R^{L5}$ and $R^{L6}$, at each occurrence, are each independently selected from H and $C_{1-4}$ alkyl, preferably H and methyl;
$R^{L2}$ and $R^{L3}$, at each occurrence, are each independently selected from H and $C_{1-4}$ alkyl, preferably H and methyl; or $R^{L2}$ and $R^{L3}$ together form oxo;
$R^{L4}$ and $R^{L7}$, at each occurrence, are each independently selected from H, halogen, OH, $NH_2$, CN and $C_{1-4}$ alkyl, preferably H, F, Cl, Br and $C_{1-2}$ alkyl, more preferably H, F, Cl and methyl;
m5 is 0, 1, 2, 3 or 4, preferably 1 or 2;
preferably, the

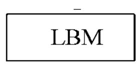

moiety is selected from

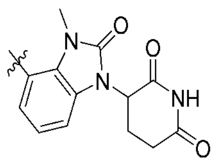, 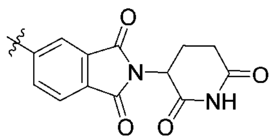, 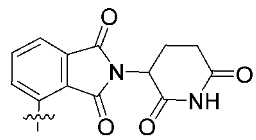,

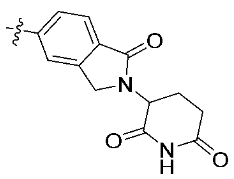, 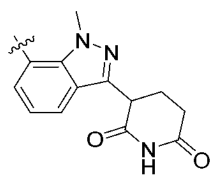, 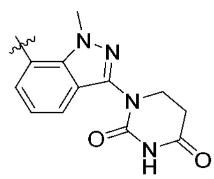, 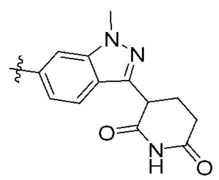,

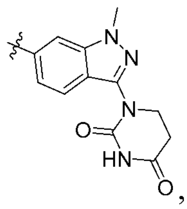, 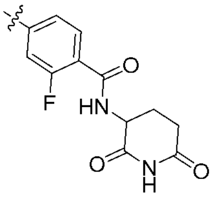 and 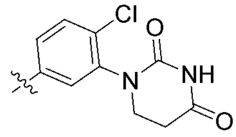.

**21.** The compound according to any one of claims 1-20, **characterized in that** the

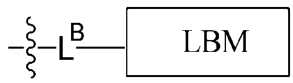

moiety is selected from:

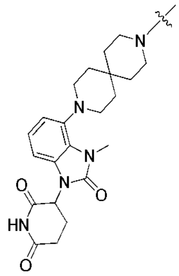, 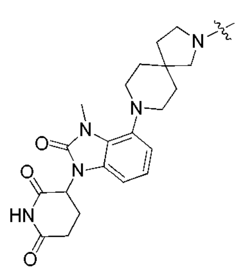, 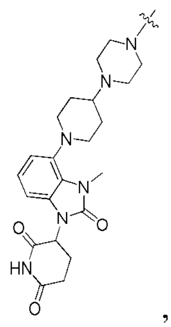, 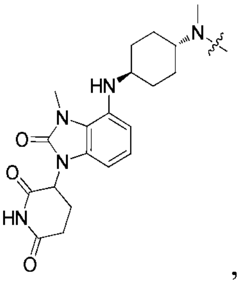, 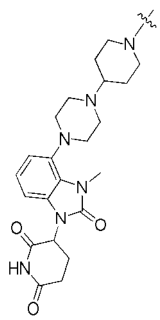, 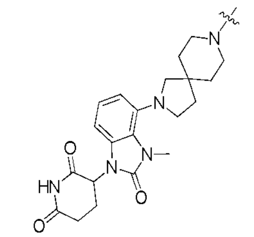, 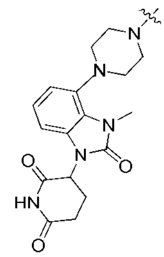, 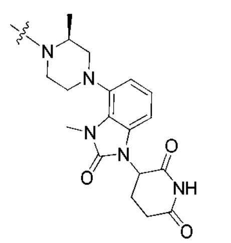, 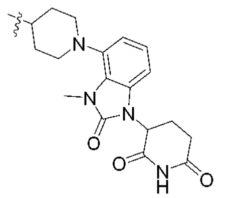, 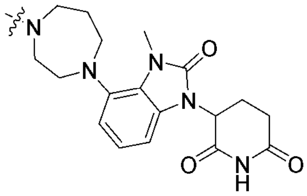, 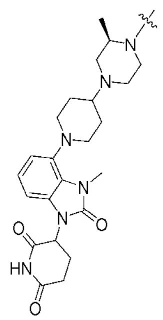, 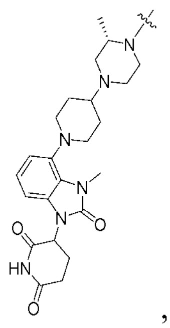, 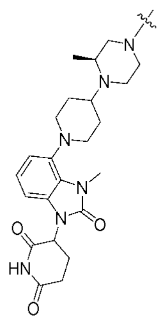, 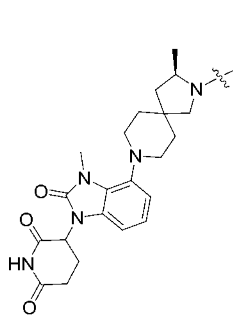

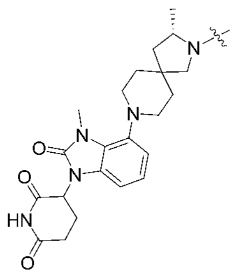, and 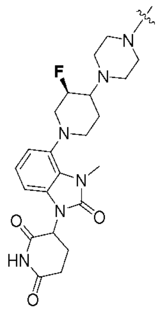.

**22.** The compound according to any one of claims 1 to 21, **characterized in that** the compound is selected from the compounds listed in Table 1 in the specification.

**23.** The compound according to claim 1, **characterized in that** the compound has a structure represented by formula (B):

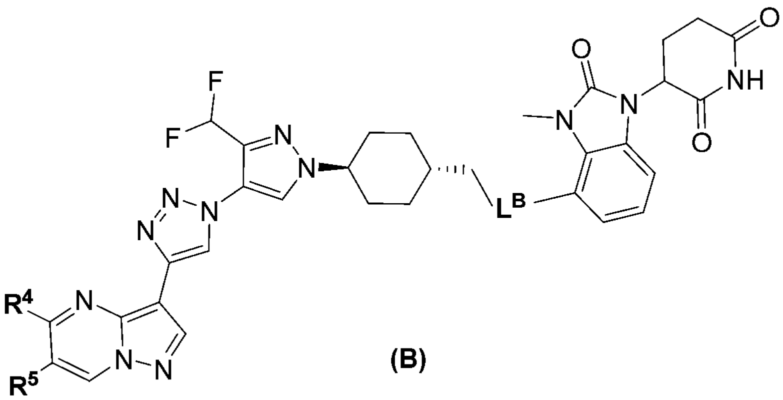

**(B)**

wherein:

$L^B$ is selected from:

(1) -CyL1-, wherein the CyL1 group here is selected from 7- to 11-membered spiro heterocycloalkylene, more preferably 9- to 11-membered spiro heterocycloalkylene, wherein the 7- to 11-membered spiro heterocycloalkylene and the 9- to 11-membered spiro heterocycloalkylene each have 1 or 2, preferably 2 nitrogen heteroatoms, and are optionally substituted with 1 or more groups independently selected from: $C_{1-4}$ alkyl and halogen, preferably methyl, F and Cl, more preferably methyl and F; or
(2) -CyL1-CyL2-, wherein the CyL1 group and the CyL2 group here are each independently selected from 4- to 7-membered monocyclic heterocycloalkylene, more preferably 4- to 6-membered monocyclic heterocycloalkylene, wherein the 4-to 7-membered monocyclic heterocycloalkylene and the 4- to 6-membered monocyclic heterocycloalkylene each have 1 or 2 nitrogen heteroatoms, and are optionally substituted with 1 or more groups independently selected from: $C_{1-4}$ alkyl and halogen, preferably methyl, F and Cl, more preferably methyl and F;

$R^4$ is selected from 4- to 6-membered saturated monocyclic heterocycloalkyl and $-NR_{Na}R_{Nb}$, where the $R_{Na}$ is selected from H and $C_{1-6}$ alkyl and the $R_{Nb}$ is selected from $C_{3-6}$ cycloalkyl, and wherein the 4- to 6-membered saturated monocyclic heterocycloalkyl and the $C_{3-6}$ cycloalkyl are substituted with 1-3 substituents selected from halogen and hydroxy; and $R^5$ is hydrogen.

**24.** The compound according to claim 23, **characterized in that** $L^B$ is selected from:

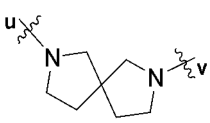, 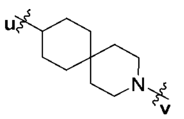, 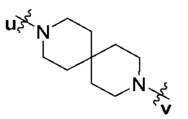, 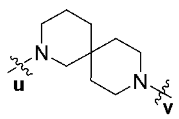, 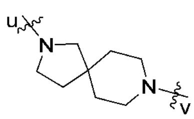,

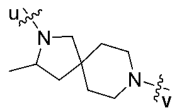

(preferably

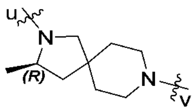 and 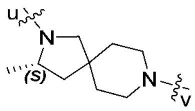 ), 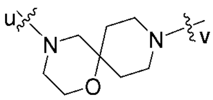 ,

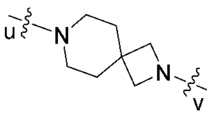 , 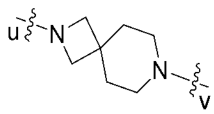 and 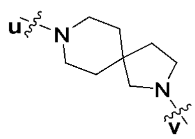 , 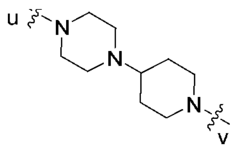 ,

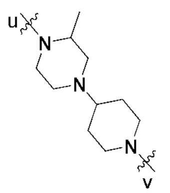

(preferably

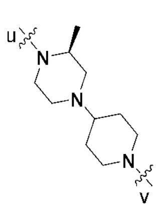 and 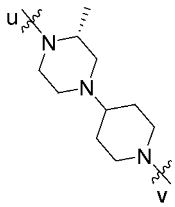 ), 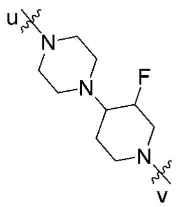

(preferably

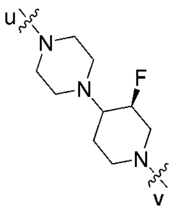 , 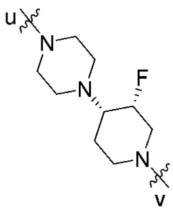 , 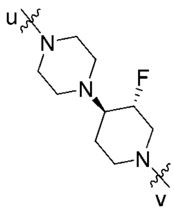 , 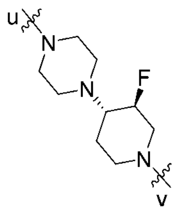 and 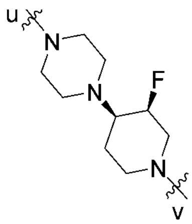

),

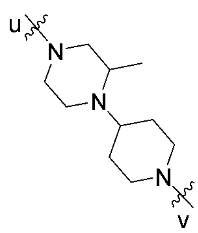

(preferably

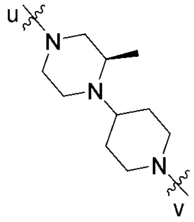 and 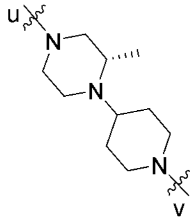

),

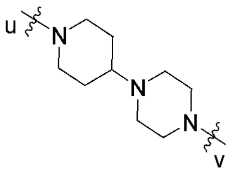,

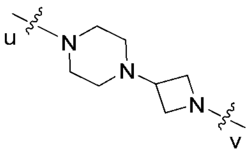, 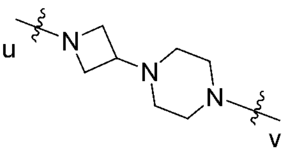 and 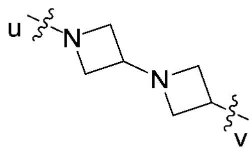,

more preferably

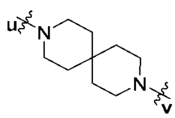 and 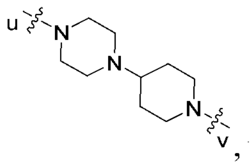,

wherein in any of the above groups, the bond labeled with "u" is connected to

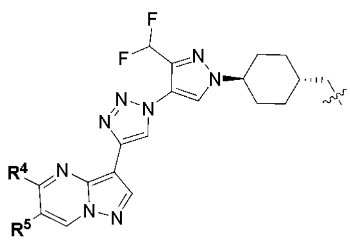

moiety, and the bond labeled with "v" is connected to

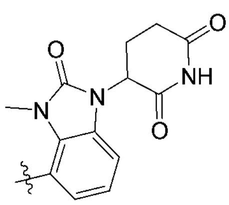

moiety;
and/or

$R^4$ is selected from 4- to 6-membered saturated monocyclic nitrogen-containing heterocycloalkyl (preferably piperidinyl) and $-NR_{Na}R_{Nb}$, wherein the $R_{Na}$ is H and the $R_{Nb}$ is selected from $C_{3-6}$ cycloalkyl (preferably cyclohexyl), and wherein the 4-to 6-membered saturated monocyclic nitrogen-containing heterocycloalkyl and the $C_{3-6}$ cycloalkyl are substituted with 1 hydroxy,
preferably, $R^4$ is selected from

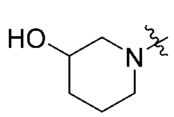
and
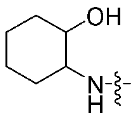
,

more preferably

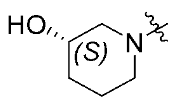
and
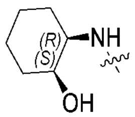
.

**25.** The compound according to claim 23 or 24, **characterized in that** the compound is selected from:

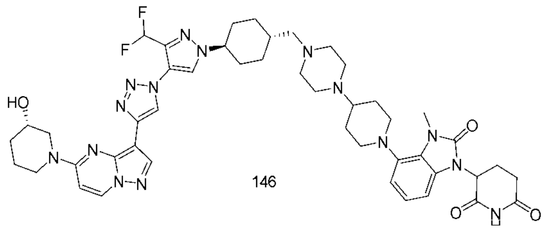
146

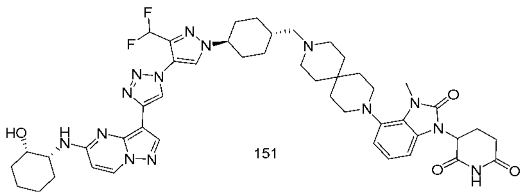
151

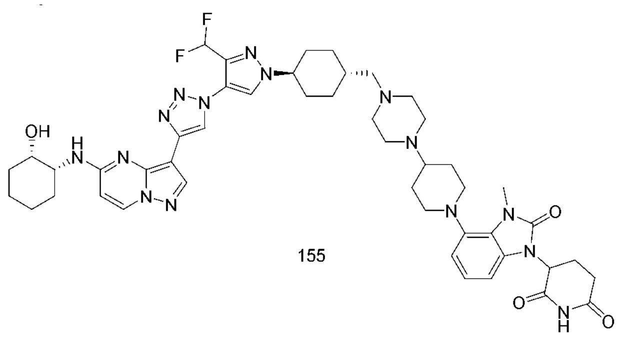
155
.

**26.** A pharmaceutical composition, **characterized in that** the pharmaceutical composition comprises the compound, or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof according to any one of claims 1 to 25, and a pharmaceutically acceptable excipient, carrier or diluent.

**27.** The compound, or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof according to any one of claims

1 to 25, or the pharmaceutical composition according to claim 26 for use in the preparation of a medicament for treating a disease, disorder or condition associated with kinase protein IRAK4.

**28.** A method for treating a disease, disorder or condition associated with kinase protein IRAK4, **characterized in that** the method comprises administering to an individual in need thereof a therapeutically effective amount of the compound, or the stereoisomer, the tautomer, the diastereomer, the racemate, the cis-trans isomer, the isotopically-labeled compound (preferably deuterated compound), the N-oxide, the metabolite, the ester, the prodrug, the crystal form, the hydrate, the solvate or the pharmaceutically acceptable salt thereof according to any one of claims 1 to 25, or the pharmaceutical composition according to claim 26.

**29.** The use according to claim 27, or the method according to claim 28, **characterized in that** the disease, disorder or condition associated with kinase protein IRAK4 is selected from: autoimmune condition, inflammatory condition, cancer, transplant rejection, thromboembolism, atherosclerosis, myocardial infarction and metabolic syndrome;

preferably, the inflammatory condition is selected from: osteoarthritis, gout, gouty arthritis, chronic obstructive pulmonary disease, periodic fever, atopic dermatitis, hidradenitis suppurativa, chronic nephritis, allergic eczema, lymphadenectasis, sepsis, irritable bowel syndrome (IBD), ulcerative colitis, asthma and allergies, preferably osteoarthritis, chronic obstructive pulmonary disease, atopic dermatitis, hidradenitis suppurativa and chronic nephritis; and/or
preferably, the autoimmune condition is selected from: Crohn's disease, rheumatoid arthritis, systemic lupus erythematosus, lupus nephritis, cutaneous lupus, psoriasis, psoriatic arthritis, multiple sclerosis, neuropathic pain, ankylosing spondylitis, reactive arthritis and systemic juvenile idiopathic arthritis, preferably psoriasis; and/or
preferably, the transplant rejection is selected from graft-versus-host disease and allogeneic transplant rejection; and/or
preferably, the cancer is selected from: brain cancer, kidney cancer, liver cancer, stomach cancer, vaginal cancer, ovarian cancer, stomach tumor, breast cancer, bladder cancer, colon cancer, prostate cancer, pancreatic cancer, lung cancer, cervical cancer, testicular cancer, skin cancer, bone cancer, thyroid cancer, sarcoma, glioblastoma, neuroblastoma, gastrointestinal cancer, neck and head tumor, adenoma, adenocarcinoma, keratoacanthoma, epidermoid carcinoma, large cell carcinoma, non-small cell lung cancer, Hodgkin and non-Hodgkin lymphoma, mastocarcinoma, follicular carcinoma, papillary carcinoma, seminoma, melanoma, acute myeloid leukemia, chronic myeloid leukemia, diffuse large B-cell lymphoma, activated B-cell-like diffuse large B-cell lymphoma, chronic lymphocytic leukemia, chronic lymphocytic lymphoma, primary effusion lymphoma, Burkitt's lymphoma/leukemia, acute lymphocytic leukemia, B-cell prolymphocytic leukemia, lymphoplasmacytic lymphoma, Waldenstrom's macroglobulinemia, splenic marginal zone lymphoma, intravascular large B-cell lymphoma, plasmacytoma, and multiple myeloma.

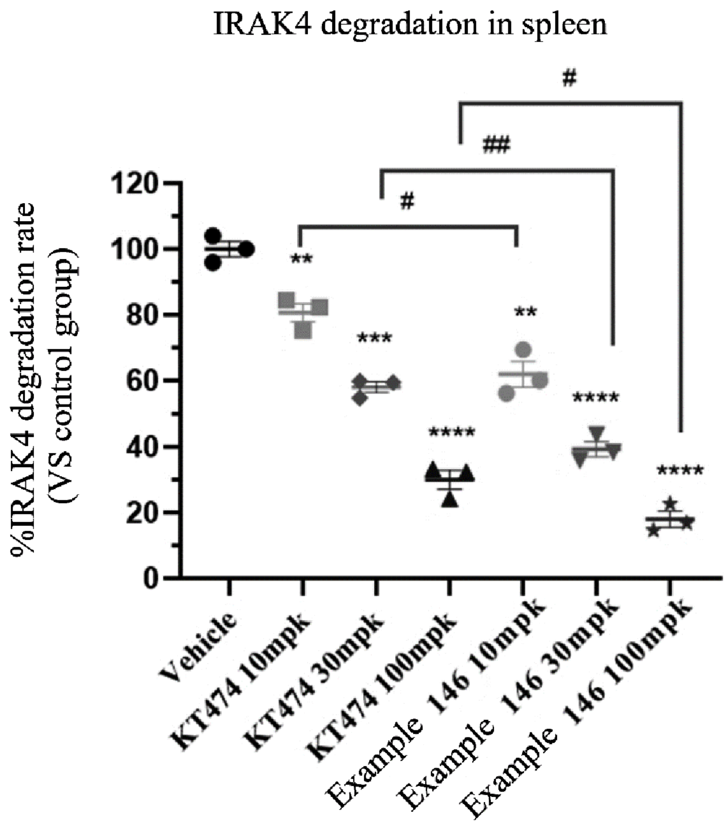
IRAK4 degradation in spleen
%IRAK4 degradation rate
(VS control group)
120
100
80
60
40
20
0
#
##
#
**
***
****
**
****
****
Vehicle
KT474 10mpk
KT474 30mpk
KT474 100mpk
Example 146 10mpk
Example 146 30mpk
Example 146 100mpk

Figure 1

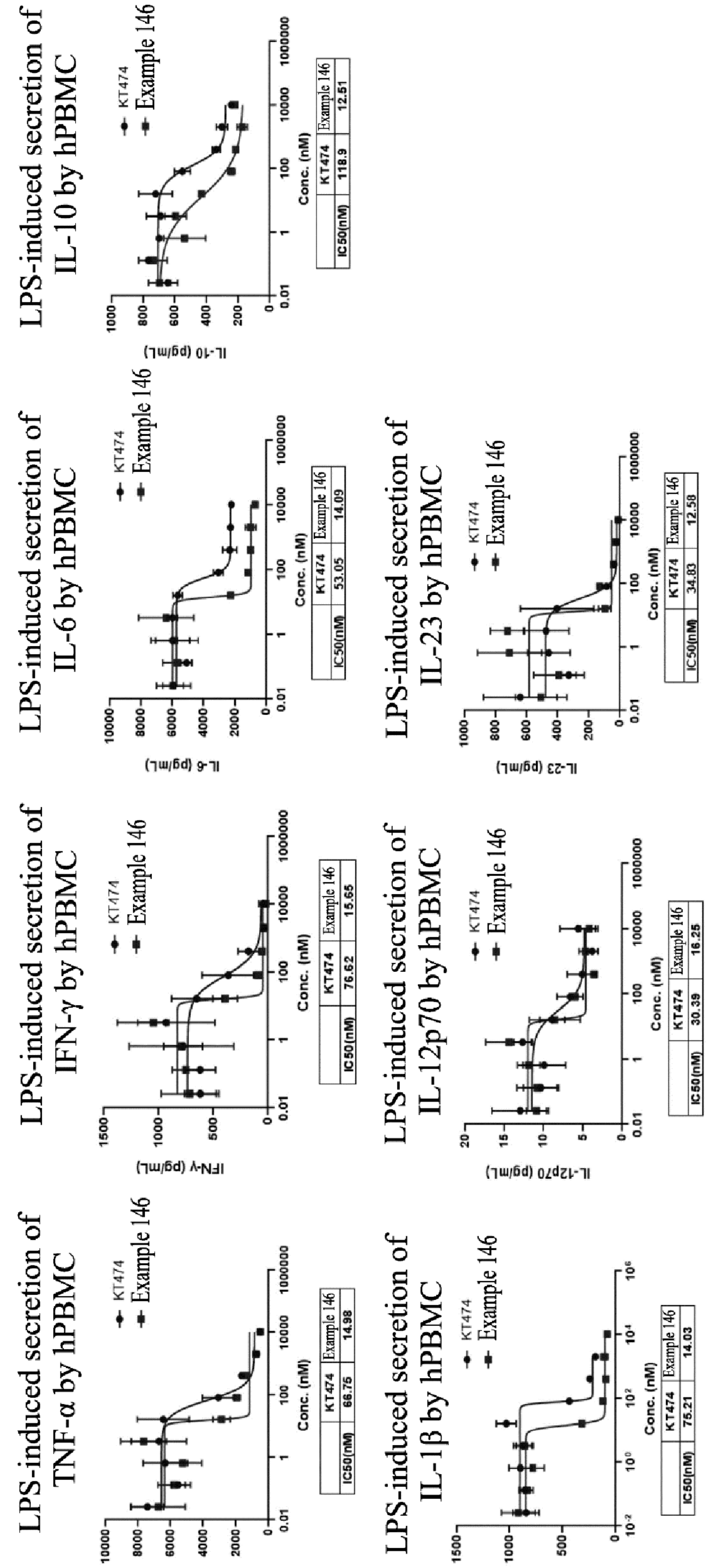
LPS-induced secretion of TNF-α by hPBMC
KT474
Example 146
TNF-α (pg/mL)
Conc. (nM)
IC50(nM) KT474 66.76 Example 146 14.98
LPS-induced secretion of IFN-γ by hPBMC
IFN-γ (pg/mL)
IC50(nM) KT474 76.62 Example 146 15.65
LPS-induced secretion of IL-6 by hPBMC
IL-6 (pg/mL)
IC50(nM) KT474 53.05 Example 146 14.09
LPS-induced secretion of IL-10 by hPBMC
IL-10 (pg/mL)
IC50(nM) KT474 118.9 Example 146 12.51
LPS-induced secretion of IL-1β by hPBMC
IL-1β (pg/ml)
IC50(nM) KT474 75.21 Example 146 14.03
LPS-induced secretion of IL-12p70 by hPBMC
IL-12p70 (pg/mL)
IC50(nM) KT474 30.39 Example 146 16.25
LPS-induced secretion of IL-23 by hPBMC
IL-23 (pg/mL)
IC50(nM) KT474 34.83 Example 146 12.58

Figure 2

# INTERNATIONAL SEARCH REPORT

International application No.
**PCT/CN2024/111013**

## A. CLASSIFICATION OF SUBJECT MATTER

C07D403/14(2006.01)i； C07D401/14(2006.01)i； C07D487/04(2006.01)i； A61P37/06(2006.01)i； A61P35/00(2006.01)i； A61K31/4192(2006.01)i； A61K31/538(2006.01)i

According to International Patent Classification (IPC) or to both national classification and IPC

## B. FIELDS SEARCHED

Minimum documentation searched (classification system followed by classification symbols)

IPC:C07D,A61P,A61K

Documentation searched other than minimum documentation to the extent that such documents are included in the fields searched

Electronic data base consulted during the international search (name of data base and, where practicable, search terms used)

CNTXT, ENTXTC, VEN, STN-Reg, STN-MARPAT: 众格生物, 白细胞介素, 抑制剂, 三唑, ZHONGGE BIOLOGICAL, IRAK, interleukin receptor associated kinase, triazole, 结构式检索, structural formula search

## C. DOCUMENTS CONSIDERED TO BE RELEVANT

| Category* | Citation of document, with indication, where appropriate, of the relevant passages | Relevant to claim No. |
|---|---|---|
| PX | WO 2024017381 A1 (SHENZHEN ZHONGGE BIOLOGICAL TECHNOLOGY CO., LTD.) 25 January 2024 (2024-01-25)<br>claims 1-22, and description, paragraph 05090 | 1-29 |
| X | CN 103429585 A (MERCK SERONO S.A.) 04 December 2013 (2013-12-04)<br>claims 1-14 | 1-29 |
| A | CN 114502158 A (KMYLAR MEDICAL COMPANY) 13 May 2022 (2022-05-13)<br>entire document | 1-29 |
| A | WO 2014202763 A1 (AB SCIENCE) 24 December 2014 (2014-12-24)<br>entire document | 1-29 |
| A | CN 106749268 A (GUANGDONG HEC PHARMACEUTICAL CO., LTD.) 31 May 2017 (2017-05-31)<br>entire document | 1-29 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 November 2024** | **29 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/111013**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/111013**

**Box No. II Observations where certain claims were found unsearchable (Continuation of item 2 of first sheet)**

This international search report has not been established in respect of certain claims under Article 17(2)(a) for the following reasons:

1. ☑ Claims Nos.:
because they relate to subject matter not required to be searched by this Authority, namely:

Claims 28-29 relate to a treatment method implemented on a human/animal, which belongs to subject matter for which no search is required by the International Searching Authority (PCT Rule 39.1(iv)). However, it is expected that the applicant may amend claims 28-29 to a pharmaceutical use, and thus the international search report is provided on this basis.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/111013**

| Patent document cited in search report | Publication date (day/month/year) | Patent family member(s) | Publication date (day/month/year) |
|---|---|---|---|
| WO 2024017381 A1 | 25 January 2024 | TW 202417451 A | 01 May 2024 |
| CN 103429585 A | 04 December 2013 | BR 122021014375 B1 | 30 November 2021 |
| | | CA 2822166 A1 | 28 June 2012 |
| | | CA 2822166 C | 29 October 2019 |
| | | HK 1191938 A1 | 08 August 2014 |
| | | US 2013274241 A1 | 17 October 2013 |
| | | US 9073892 B2 | 07 July 2015 |
| | | EA 201300730 A1 | 30 December 2013 |
| | | EA 023544 B1 | 30 June 2016 |
| | | AU 2011347711 A1 | 01 August 2013 |
| | | AU 2011347711 B2 | 02 February 2017 |
| | | MX 2013007149 A | 30 October 2013 |
| | | PT 2655357 T | 28 September 2016 |
| | | LT 2655357 T | 10 October 2016 |
| | | SI 2655357 T1 | 28 October 2016 |
| | | PL 2655357 T3 | 30 December 2016 |
| | | RS 55165 B1 | 31 January 2017 |
| | | JP 2014500286 A | 09 January 2014 |
| | | JP 6007189 B2 | 12 October 2016 |
| | | KR 20130133252 A | 06 December 2013 |
| | | KR 101842098 B1 | 26 March 2018 |
| | | HUE 029617 T2 | 28 March 2017 |
| | | SG 191205 A1 | 31 July 2013 |
| | | WO 2012084704 A1 | 28 June 2012 |
| | | EP 2655357 A1 | 30 October 2013 |
| | | EP 2655357 B1 | 22 June 2016 |
| | | ES 2592713 T3 | 01 December 2016 |
| | | IL 226912 A | 31 January 2017 |
| | | ZA 201305483 B | 25 June 2014 |
| | | AR 084507 A1 | 22 May 2013 |
| | | HRP 20161127 T1 | 18 November 2016 |
| | | BR 112013015460 A2 | 20 September 2016 |
| | | BR 112013015460 B1 | 25 January 2022 |
| CN 114502158 A | 13 May 2022 | EP 3989966 A1 | 04 May 2022 |
| | | EP 3989966 A4 | 27 September 2023 |
| | | JP 2022538192 A | 31 August 2022 |
| | | WO 2020264499 A1 | 30 December 2020 |
| | | US 2023069104 A1 | 02 March 2023 |
| | | BR 112021026517 A2 | 10 May 2022 |
| | | CO 2021017893 A2 | 17 January 2022 |
| | | CA 3144805 A1 | 30 December 2020 |
| | | KR 20220032063 A | 15 March 2022 |
| | | IL 289267 A | 01 February 2022 |
| | | MX 2021015995 A | 11 March 2022 |
| | | AU 2020302118 A1 | 24 February 2022 |
| WO 2014202763 A1 | 24 December 2014 | AR 096654 A1 | 27 January 2016 |
| | | TW 201534597 A | 16 September 2015 |
| | | HK 1222396 A1 | 30 June 2017 |
| | | US 2016152608 A1 | 02 June 2016 |
| | | US 10202370 B2 | 12 February 2019 |

Form PCT/ISA/210 (patent family annex) (July 2022)

**INTERNATIONAL SEARCH REPORT**
**Information on patent family members**

International application No.
**PCT/CN2024/111013**

| Patent document cited in search report | Publication date (day/month/year) | Patent family member(s) | Publication date (day/month/year) |
|---|---|---|---|
| | | EP 3010906 A1 | 27 April 2016 |
| | | EP 3010906 B1 | 12 June 2019 |
| CN 106749268 A | 31 May 2017 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311018650
- CN 202311038908
- CN 202311618951
- CN 202410120222
- WO 2022161414 A1
- CN 113423427
- US 20190192668 A1
- WO 2020206424 A1
- WO 2022236058 A1
- WO 2021247899 A1
- WO 2020264499 A1
- WO 2020264499 A
- WO 2022140472 A1
- WO 2021158634 A1
- WO 2023019166 A1
- WO 2020113233 A
- WO 2022068933 A1
- WO 2015117563 A1
- WO 2022012623 A
- WO 2021188948 A1
- WO 2023017446 A1
- WO 2020113233 A1
- WO 2023017442 A
- WO 2022125790 A1
- WO 2014195919 A1
- WO 2021127283 A2
- CN 1120108582020 A
- WO 2023023537 A1
- WO 2023278759 A1
- WO 2022253250 A1
- WO 2022236339 A1
- WO 2021055295 A1
- WO 2021207291 A1
- WO 2021113557 A1
- WO 2021091575 A
- WO 2015108490 A2
- WO 2023283610 A1
- WO 2013163262 A1
- WO 2019089422 A1
- WO 2021055756 A1


### Non-patent literature cited in the description


- **STAHL** ; **WERMUTH**. Handbook of Pharmaceutical Salts: Properties, Selection, and Use. Wiley-VCH, 2002
- **T. L. GILCHRIST**. Comprehensive Organic Synthesis. Academic Press, vol. 7, 748-750
- **G. W. H. CHEESEMAN** ; **E. S. G. WERSTIUK**. Advances in Heterocyclic Chemistry. Academic Press, vol. 22, 390-392
- **T. HIGUCHI** ; **V. STELLA**. Pro-drugs as Novel Delivery Systems. ACS Symposium Series, vol. 14
- Bioreversible Carriers in Drug Design. Pergamon Press, 1987
- **H. BUNDGAARD**. Design of Prodrugs. Elsevier, 1985
- Protective Groups in Organic Chemistry. Plenum Press, 1973
- **T.W. GREENE** ; **P.G.M. WUTS**. Protective Groups in Organic Synthesis. John Wiley & Sons, 1991